# EUROPEAN PATENT APPLICATION

(11) **EP 2 725 015 A1**
(43) Date of publication of application: **30.04.2014**
(21) Application number: 13193524.9
(22) Date of filing: 17.09.2008
(51) Int. Cl.: C07D 239/22, C07D 239/54, C07D 401/10, C07D 403/10, C07D 409/10, C07D 413/10, C07D 417/10, A61P 31/12, A61K 31/513

(54) **Uracil or thymine derivative for treating hepatitis c**

(30) Priority: 17.09.2007 US 972877 P; 13.09.2008 US 96791
(62) Divisional of application: 08831873.8
(71) Applicant: AbbVie Bahamas Ltd., New Providence, Nassau (BS)
(72) Inventor: Wagner, Rolf, Antioch, IL Illinois 60002 (US); Tufano, Michael D., Chicago, IL Illinois 60645 (US); Stewart, Kent D., Gurnee, IL Illinois 60031 (US); Rockway, Todd W., Grayslake, IL Illinois 60030 (US); Randolph, John T., Libertyville, IL Illinois 60048 (US); Pratt, John K., Kenosha, WI Wisconsin 53142 (US); Motter, Christopher E., Oak Creek, WI Wisconsin 53154 (US); Maring, Clarence J., Palatine, IL Illinois 60067 (US); Longenecker, Kenton L., Grayslake, IL Illinois 60030 (US); Liu, Yaya, Buffalo Grove, IL Illinois 60089 (US); Liu, Dachun, Waikegan, IL Illinois 60085 (US); Krueger, Allan C., Gurnee, IL Illinois 60031 (US); Kati, Warren M., Gurnee, IL Illinois 60031 (US); Hutchinson, Douglas K., Antioch, IL Illinois 60002-1863 (US); Huang, Peggy P., Lake Bluff, IL Illinois 60044 (US); Flentge, Charles A., Salem, WI Wisconsin 53168 (US); Donner, Pamela L., Mundelein, IL Illinois 60060 (US); Degoey, David A., Mundelein, WI Wisconsin 53168 (US); Betebenner, David A., Libertyville, IL Illinois 60048 (US); Barnes, David M., Bristol, WI Wisconsin 53104 (US); Chen, Shuang, Gurnee, IL Illinois 60031 (US); Franczyk II, Thaddeus S, Lake Villa, IL Illinois 60046 (US); Gao, Yi, Vernon Hills, IL Illinois 60061 (US); Haight, Anthony R, Wadsworth, IL Illinois 60083 (US); Hengeveld, John E, Kenosha, WI Wisconsin 53143 (US); Henry, Rodger F, Wildwood, IL Illinois 60030 (US); Kotecki, Brian J, Oak Creek, IL Illinois 53154 (US); Lou, Xiaochun, Long Grove, IL Illinois 60047 (US); Sarris, Kathy, Deefield, IL Illinois 60015 (US); Zhang, Geoff G. Z., Libertyville, IL Illinois 60048 (US)
(74) Representative: Kingsbury, Oliver William

(57) **Abstract**

This invention relates to: (a) compounds and salts thereof that, *inter alia*, inhibit HCV; (b) intermediates useful for the preparation of such compounds and salts; (c) compositions comprising such compounds and salts; (d) methods for preparing such intermediates, compounds, salts, and compositions; and (e) use of such compounds, salts, and compositions.

SEQUENCE LISTING

<110> ABBOTT LABORATORIES

<120> URACIL OR THYMINE DERIVATIVE FOR TREATING HEPATITIS C

<130> P42106EP-D1

<140>
<141> 2008-09-17

<150> EP08831873.8
<151> 2008-09-17

<150> 61/096,791
<151> 2008-09-13

<150> 60/972,877
<151> 2007-09-17

<160> 1

<170> PatentIn version 3.5

<210> 1
<211> 171
<212> RNA
<213> Artificial Sequence

<220>
<223> Description of Artificial Sequence: Synthetic
      polynucleotide

<400> 1
gggcgaauug ggcccucuag augcaugcuc gagcggccgc cagugugaug gauaucugca      60

gaauucgccc uugguggcuc caucuuagcc cuagucacgg cuagcuguga aagguccgug      120

agccgcuuga cugcagagag ugcugauacu ggccucucug cagaucaagu c              171

## Description

### CROSS-REFERENCE TO RELATED PATENT APPLICATIONS

This patent application claims priority to U.S. Provisional Patent Application No. 60/972,877 (filed September 17, 2007) and U.S. Provisional Patent Application No. 61/096,791 (filed September 13, 2008). The entire text of those applications is incorporated by reference into this application.

### FIELD OF THE INVENTION

This invention is directed to: (a) compounds and salts thereof that, *inter alia,* are useful as hepatitis C virus (HCV) inhibitors; (b) intermediates useful for the preparation of such compounds and salts; (c) compositions comprising such compounds and salts; (d) methods for preparing such intermediates, compounds, salts, and compositions; (e) methods of use of such compounds, salts, and compositions; and (f) kits comprising such compounds, salts, and compositions.

### BACKGROUND OF THE INVENTION

Hepatitis C is a blood-borne, infectious, viral disease that is caused by a hepatotropic virus called HCV. At least six different HCV genotypes (with several subtypes within each genotype) are known to date. In North America, HCV genotype 1a predominates, followed by HCV genotypes 1b, 2a, 2b, and 3a. In the United States, HCV genotypes 1, 2, and 3 are the most common, with about 80% of the hepatitis C patients having HCV genotype 1. In Europe, HCV genotype 1b is predominant, followed by HCV genotypes 2a, 2b, 2c, and 3a. HCV genotypes 4 and 5 are found almost exclusively in Africa. As discussed below, the patient's HCV genotype is clinically important in determining the patient's potential response to therapy and the required duration of such therapy.

An HCV infection can cause liver inflammation (hepatitis) that is often asymptomatic, but ensuing chronic hepatitis can result in cirrhosis of the liver (fibrotic scarring of the liver), liver cancer, and/or liver failure. The World Health Organization estimates that about 170 million persons worldwide are chronically infected with HCV, and from about three to about four million persons are newly infected globally each year. According to the Centers for Disease Control and Prevention, about four million people in the United States are infected with HCV. Co-infection with the human immunodeficiency virus (HIV) is common, and rates of HCV infection among HIV positive populations are higher.

There is a small chance of clearing the virus spontaneously, but the majority of patients with chronic hepatitis C will not clear it without treatment. Indications for treatment typically include proven HCV infection and persistent abnormal liver function tests. There are two treatment regimens that are primarily used to treat hepatitis C: monotherapy (using an interferon agent - either a "conventional" or longer-acting pegylated interferon) and combination therapy (using an interferon agent and ribavirin). Interferon, which is injected into the bloodstream, works by bolstering the immune response to HCV; and ribavirin, which is taken orally, is believed to work by preventing HCV replication. Taken alone, ribavirin does not effectively suppress HCV levels, but an interferon/ribavirin combination is more effective than interferon alone. Typically, hepatitis C is treated with a combination of pegylated interferon alpha and ribavirin for a period of 24 or 48 weeks, depending on the HCV genotype.

The goal of treatment is sustained viral response -- meaning that HCV is not measurable in the blood after therapy is completed. Following treatment with a combination of pegylated interferon alpha and ribavirin, sustained cure rates (sustained viral response) of about 75% or better occur in people with HCV genotypes 2 and 3 in 24 weeks of treatment, about 50% in those with HCV genotype 1 with 48 weeks of treatment, and about 65% in those with HCV genotype 4 in 48 weeks of treatment.

Treatment may be physically demanding, particularly for those with prior history of drug or alcohol abuse, because both interferon and ribavirin have numerous side effects. Common interferon-associated side effects include flu-like symptoms, extreme fatigue, nausea, loss of appetite, thyroid problems, high blood sugar, hair loss, and skin reactions at the injection site. Possible serious interferon-associated side effects include psychoses (*e.g.,* suicidal behavior), heart problems (*e.g.,* heart attack, low blood pressure), other internal organ damage, blood problems (*e.g.,* blood counts falling dangerously low), and new or worsening autoimmune disease (*e.g.,* rheumatoid arthritis). Ribavirin-associated side effects include anemia, fatigue, irritability, skin rash, nasal stuffiness, sinusitis, and cough. Ribavirin can also cause birth defects, so pregnancy in female patients and female partners of male patients must be avoided during treatment and for six months afterward.

Some patients do not complete treatment because of the serious side effects discussed above; other patients (non-responders) continue to have measurable HCV levels despite treatment; and yet other patients (relapsers) "clear" the virus during therapy, but the virus returns sometime after completion of the treatment regimen. Thus, there continues to be a need for alternative compounds, compositions, and methods of treatment (used either in combination with or in lieu of an interferon agent and/or ribavirin) to alleviate the symptoms of hepatitis C, thereby providing partial or complete relief. This invention provides compounds (including salts thereof), compositions, and methods of treatment that generally address such a need.

### SUMMARY OF THE INVENTION

This invention is directed to compounds that correspond in structure to formula **I:**

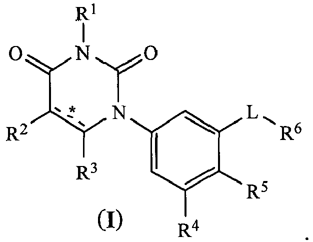

In formula **I:**
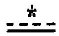 is selected from the group consisting of single carbon-carbon bond and double carbon-carbon bond;
**R¹** is selected from the group consisting of hydrogen, methyl, and nitrogen-protecting group;
**R²** is selected from the group consisting of hydrogen, halo, hydroxy, methyl, cyclopropyl, and cyclobutyl;
**R³** is selected from the group consisting of hydrogen, halo, oxo, and methyl;
**R⁴** is selected from the group consisting of halo, alkyl, alkenyl, alkynyl, nitro, cyano, azido, alkyloxy, alkenyloxy, alkynyloxy, amino, aminocarbonyl, aminosulfonyl, alkylsulfonyl, carbocyclyl, and heterocyclyl, wherein:
   (**a**) the amino, aminocarbonyl, and aminosulfonyl optionally are substituted with:
      (**1**) one or two substituents independently selected from the group consisting of alkyl, alkenyl, alkynyl, and alkylsulfonyl, or
      (**2**) two substituents that, together with the amino nitrogen, form a single-ring heterocyclyl, and
   (**b**) the alkyl, alkenyl, alkynyl, alkyloxy, alkenyloxy, alkynyloxy, and alkylsulfonyl, optionally are substituted with one or more substituents independently selected from the group consisting of halo, oxo, nitro, cyano, azido, hydroxy, amino, alkyloxy, trimethylsilyl, carbocyclyl, and heterocyclyl, wherein:
      the amino optionally is substituted with:
         (**1**) one or two substituents independently selected from the group consisting of alkyl, alkenyl, alkynyl, alkylcarbonyl, alkylsulfonyl, alkyloxycarbonyl, carbocyclyl, heterocyclyl, carbocyclylalkyl, and heterocyclylalkyl, or
         (**2**) two substituents that, together with the amino nitrogen, form a single-ring heterocyclyl, and
   (**c**) the carbocyclyl and heterocyclyl optionally are substituted with up to three substituents independently selected from the group consisting of alkyl, alkenyl, alkynyl, halo, oxo, nitro, cyano, azido, hydroxy, amino, alkyloxy, trimethylsilyl, carbocyclyl, and heterocyclyl, wherein:
      the amino optionally is substituted with:
         (**1**) one or two substituents independently selected from the group consisting of alkyl, alkenyl, alkynyl, alkylcarbonyl, alkylsulfonyl, alkyloxycarbonyl, carbocyclyl, heterocyclyl, carbocyclylalkyl, and heterocyclylalkyl, or
         (**2**) two substituents that, together with the amino nitrogen, form a single-ring heterocyclyl;
**R⁵** is selected from the group consisting of hydrogen, hydroxy, alkyl, alkenyl, alkynyl, alkyloxy, alkenyloxy, alkynyloxy, alkylsulfonyloxy, carbocyclylsulfonyloxy, haloalkylsulfonyloxy, and halo;
**L** is selected from the group consisting of bond, C(**R^{A}**)=C(**R^{B}**), C=C, C(O)N(**R^{C}**), N(**R^{D}**)C(O), C₁-C₂-alkylene, C(H)₂O, OC(H)₂, cyclopropyl-1,2-ene, C(H)₂N(**R^{L}**), N(**R^{M}**)C(H)₂, C(O)CH₂, and CH₂C(O);
**R^{A}, R^{B}, R^{L},** and **R^{M}** are independently selected from the group consisting of hydrogen, C₁-C₆-alkyl, C₁-C₆-alkyloxy, C₃-C₈-cycloalkyl, and halo, wherein:
   the C₁-C₆-alkyl optionally is substituted with one or more substituents independently selected from the group consisting of carboxy, halo, hydroxy, nitro, oxo, amino, cyano, alkyloxycarbonyl, alkylcarbonyloxy, alkyloxy, carbocyclyl, and heterocyclyl;
**R^{C}** is selected from the group consisting of hydrogen and alkyl;
**R^{D}** is selected from the group consisting of hydrogen and alkyl;
**R⁶** is selected from the group consisting of C₅-C₆-carbocyclyl, 5-6-membered heterocyclyl, fused 2-ring heterocyclyl, and fused 2-ring carbocyclyl, wherein each such substituent optionally is substituted with one or more substituents independently selected from the group consisting of **R^{E}**, **R^{F}**, **R^{G}**, **R^{H}**, **R^{I}**, **R^{J}**, and **R^{K};**
each **R^{E}** is independently selected from the group consisting of halo, nitro, hydroxy, oxo, carboxy, cyano, amino, imino, azido, and aldehydo, wherein:
   the amino optionally is substituted with one or two substituents independently selected from the group consisting of alkyl, alkenyl, and alkynyl;
each **R^{F}** is independently selected from the group consisting of alkyl, alkenyl, and alkynyl, wherein:
each such substituent optionally is substituted with one or more substituents independently selected from the group consisting of carboxy, hydroxy, halo, amino, imino, nitro, azido, oxo, aminosulfonyl, alkylsulfonyl, alkyloxycarbonyl, alkenyloxycarbonyl, alkynyloxycarbonyl, alkylcarbonyloxy, alkenylcarbonyloxy, alkynylcarbonyloxy, alkyloxy, alkenyloxy, alkynyloxy, carbocyclyl, heterocyclyl, cyano, and aminocarbonyl, wherein:
   the amino, imino, aminosulfonyl, aminocarbonyl, carbocyclyl, and heterocyclyl optionally are substituted with one or two substituents independently selected from the group consisting of alkyl, alkenyl, alkynyl, alkylsulfonyl, alkenylsulfonyl, alkynylsulfonyl, alkylsulfonylamino, hydroxy, and alkyloxy,
wherein:
   amino portion of the alkylsulfonylamino optionally is substituted with a substituent selected from the group consisting of alkyl, alkenyl, and alkynyl;
each **R^{G}** is independently selected from the group consisting of carbocyclyl and heterocyclyl, wherein:
each such substituent optionally is substituted with one or more substituents independently selected from the group consisting of alkyl, alkenyl, alkynyl, carboxy, hydroxy, halo, amino, nitro, azido, oxo, aminosulfonyl, alkyloxycarbonyl, alkenyloxycarbonyl, alkynyloxycarbonyl, alkylcarbonyloxy, alkenylcarbonyloxy, alkynylcarbonyloxy, alkyloxy, alkenyloxy, alkynyloxy, carbocyclyl, heterocyclyl, cyano, and aminocarbonyl, wherein:
   the amino, aminosulfonyl, and aminocarbonyl optionally are substituted with one or two substituents independently selected from the group consisting of alkyl, alkenyl,
alkynyl, alkylsulfonyl, alkenylsulfonyl, and alkynylsulfonyl;
each **R^{H}** is independently selected from the group consisting of alkyloxy, alkenyloxy, alkynyloxy, alkylsulfonyloxy, alkenylsulfonyloxy, and alkynylsulfonyloxy, wherein:
   each such substituent optionally is substituted with one or more substituents independently selected from the group consisting of carboxy, hydroxy, halo, amino, nitro, azido, oxo, aminosulfonyl, alkyloxycarbonyl, alkenyloxycarbonyl, alkynyloxycarbonyl, alkylcarbonyloxy, alkenylcarbonyloxy, alkynylcarbonyloxy, alkyloxy, alkenyloxy, alkynyloxy, carbocyclyl, heterocyclyl, cyano, and aminocarbonyl, wherein:
      the amino, aminosulfonyl, and aminocarbonyl optionally are substituted with one or two substituents independently selected from the group consisting of alkyl, alkenyl, alkynyl, alkylsulfonyl, alkenylsulfonyl, and alkynylsulfonyl;
each **R^{I}** is independently selected from the group consisting of alkylcarbonyl, alkenylcarbonyl, alkynylcarbonyl, aminocarbonyl, alkyloxycarbonyl, carbocyclylcarbonyl, and heterocyclylcarbonyl, wherein:
   (**a**) the alkylcarbonyl, alkenylcarbonyl, and alkynylcarbonyl optionally are substituted with one or more substituents independently selected from the group consisting of carboxy, hydroxy, halo, amino, nitro, azido, oxo, aminosulfonyl, alkyloxycarbonyl, alkenyloxycarbonyl, alkynyloxycarbonyl, alkylcarbonyloxy, alkenylcarbonyloxy, alkynylcarbonyloxy, alkyloxy, alkenyloxy, alkynyloxy, carbocyclyl, heterocyclyl, cyano, and aminocarbonyl, and
   (**b**) the aminocarbonyl optionally is substituted with one or two substituents independently selected from the group consisting of alkyl, alkenyl, alkynyl, alkyloxyalkyl, carbocyclyl, heterocyclyl, alkylsulfonyl, and alkylsulfonylamino, wherein:
      the carbocyclyl and heterocyclyl optionally are substituted with one or two substituents independently selected from the group consisting of halo, alkyl, and oxo;
each R^{J} is independently selected from the group consisting of carbocyclylsulfonylamino, heterocyclylsulfonylamino, alkylcarbonylamino, alkenylcarbonylamino, alkynylcarbonylamino, alkyloxycarbonylamino, alkenyloxycarbonylamino, alkynyloxycarbonylamino, alkylsulfonylamino, alkenylsulfonylamino, alkynylsulfonylamino, aminocarbonylamino, alkyloxycarbonylaminoimino, alkylsulfonylaminoimino, alkenylsulfonylaminoimino, and alkynylsulfonylaminoimino, wherein:
(**a**) the amino portion of such substituents optionally is substituted with a substituent independently selected from the group consisting of carbocyclylalkyl, heterocyclylalkyl, alkylcarbonyloxy, aminocarbonylalkyl, alkyl, alkenyl, alkynyl, alkylcarbonyl, alkenylcarbonyl, alkynylcarbonyl, alkyloxycarbonyl, alkyloxyalkyloxycarbonyl, alkylcarbonyloxyalkyl, and alkylsulfonyl, wherein:
   (**1**) the carbocyclyl portion of the carbocyclylalkyl and the heterocyclyl portion of the heterocyclylalkyl optionally are substituted with one or more substituents independently selected from the group consisting of alkyl, alkenyl, alkynyl, carboxy, hydroxy, alkyloxy, alkenyloxy, alkynyloxy, halo, nitro, cyano, azido, oxo, and amino, and
   (**2**) the amino portion of the aminocarbonylalkyl optionally is substituted with one or two substituents independently selected from the group consisting of alkyl, alkenyl, and alkynyl,
(**b**) the alkyl, alkenyl, and alkynyl portion of such substituents optionally is substituted with one or more substituents independently selected from the group consisting of carboxy, halo, oxo, amino, alkyloxycarbonyl, alkylcarbonyloxy, hydroxy, alkyloxy, carbocyclyl, heterocyclyl, and cyano, wherein:
   the amino optionally is substituted with one or two substituents independently selected from the group consisting of alkyl, alkenyl, alkynyl, alkyloxy, alkenyloxy, and alkynyloxy, wherein:
      the alkyl optionally is substituted with one or more hydroxy;
(**c**) the carbocyclyl and heterocyclyl portions of such substituents optionally are substituted with one or more substituents independently selected from the group consisting of alkyl, alkenyl, alkynyl, carboxy, hydroxy, alkyloxy, alkenyloxy, alkynyloxy, halo, nitro, cyano, azido, and amino, wherein:
   the amino optionally is substituted with one or two substituents independently selected from the group consisting of alkyl, alkenyl, and alkynyl; and
      each **R^{K}** is independently selected from the group consisting of aminosulfonyl, alkylsulfonyl, alkenylsulfonyl, and alkynylsulfonyl, wherein:
      (**a**) the alkylsulfonyl, alkenylsulfonyl, and alkynylsulfonyl optionally are substituted with one or more substituents independently selected from the group consisting of carboxy, hydroxy, halo, amino, nitro, azido, oxo, aminosulfonyl, alkyloxycarbonyl, alkenyloxycarbonyl, alkynyloxycarbonyl, alkylcarbonyloxy, alkenylcarbonyloxy, alkynylcarbonyloxy, alkyloxy, alkenyloxy, alkynyloxy, carbocyclyl, heterocyclyl, cyano, and aminocarbonyl, wherein:
         the amino, aminosulfonyl, and aminocarbonyl optionally are substituted with one or two substituents independently selected from the group consisting of alkyl, alkenyl, and alkynyl; and
      (**b**) the aminosulfonyl optionally is substituted with one or two substituents independently selected from the group consisting of alkyl, alkenyl, and alkynyl.

This invention also is directed to the salts (including pharmaceutically acceptable salts) of the compounds of the invention.

This invention also is directed to compositions (including pharmaceutical compositions) that comprise one or more compounds and/or salts of the invention, and, optionally, one or more additional therapeutic agents.

This invention also is directed to kits that comprise one or more compounds and/or salts of the invention, and, optionally, one or more additional therapeutic agents.

This invention also is directed to methods of use of the compounds, salts, compositions, and/or kits of the invention to, for example, inhibit replication of an RNA virus (including HCV), treat a disease treatable by inhibiting HCV ribonucleic acid (RNA) polymerase (including hepatitis C).

This invention also is directed to a use of one or more compounds and/or salts of the invention to prepare a medicament. The medicament optionally can comprise one or more additional therapeutic agents. In some embodiments, the medicament is useful for treating hepatitis C.

Further benefits of Applicants' invention will be apparent to one skilled in the art from reading this patent application.

### BRIEF DESCRIPTION OF THE DRAWINGS

Figure **1** shows an illustrative PXRD pattern for the ethanol solvate of compound **IB-L0-2.3.**
Figure **2** shows an illustrative TGA profile of the ethanol solvate of compound **IB-L0-2.3.**
Figure **3** shows an illustrative PXRD pattern for the acetonitrile solvate of compound **IB-L0-2.3.**
Figure **4** shows an illustrative PXRD pattern for the ethyl acetate solvate of compound **IB-L0-2.3.**
Figure **5** shows an illustrative PXRD pattern for the 2-propanol solvate of compound **IB-L0-2.3.**
Figure **6** shows an illustrative PXRD pattern for the methanol solvate of compound **IB-L0-2.3.**
Figure **7** shows an illustrative PXRD pattern for the 1-propanol solvate of compound **IB-L0-2.3.**
Figure **8** shows an illustrative PXRD pattern for the solvent free crystalline compound **IB-L0-2.3.**
Figure **9** shows an illustrative PXRD pattern for the hydrate of compound **IB-L0-2.3.**
Figure **10** shows an illustrative PXRD pattern for the pattern A monosodium salt of compound **IB-L0-2.3.**
Figure **11** shows an illustrative TGA profile of the pattern A monosodium salt of compound **IB-L0-2.3.**
Figure **12** shows an illustrative PXRD pattern for the pattern B monosodium salt of compound **IB-L0-2.3.**
Figure **13** shows an illustrative TGA profile of the pattern B monosodium salt of compound **IB-L0-2.3.**
Figure **14** shows an illustrative PXRD pattern for the pattern C monosodium salt of compound **IB-L0-2.3.**
Figure **15** shows an illustrative PXRD pattern for the disodium salt of compound **IB-L0-2.3.**
Figure **16** shows an illustrative TGA profile of the disodium salt of compound **IB-L0-2.3.**
Figure **17** shows an illustrative PXRD pattern for the monopotassium salt of compound **IB-L0-2.3.**
Figure **18** shows an illustrative TGA profile of the monopotassium salt of compound **IB-L0-2.3.**
Figure **19** shows an illustrative PXRD pattern for the pattern A monocholine salt of compound **IB-L0-2.3.**
Figure **20** shows an illustrative TGA profile of the pattern A monocholine salt of compound **IB-L0-2.3.**
Figure **21** shows an illustrative PXRD pattern for the pattern B monocholine salt of compound **IB-L0-2.3.**
Figure **22** shows an illustrative TGA profile of the pattern B monocholine salt of compound **IB-L0-2.3.**
Figure **23** shows an illustrative PXRD pattern for the dicholine salt of compound **IB-L0-2.3.**
Figure **24** shows an illustrative PXRD pattern for the disodium salt nonahydrate of compound **IB-L1-1.1.**
Figure **25** shows an illustrative PXRD pattern for the disodium salt tetrahydrate of compound **IB-L1-1.1.**
Figure **26** shows an illustrative TGA profile of the disodium salt tetrahydrate of compound **IB-L1-1.1.**
Figure **27** shows an illustrative PXRD pattern for the dipotassium salt tetrahydrate of compound **IB-L1-1.1.**
Figure **28** shows an illustrative PXRD pattern for the monopotassium salt trihydrate of compound **IB-L1-1.1.**
Figure **29** shows an illustrative PXRD pattern for the monopotassium salt dihydrate of compound **IB-L1-1.1.**
Figure **30** shows an illustrative TGA profile of the monopotassium salt dihydrate of compound **IB-L1-1.1.**
Figure **31** shows an illustrative PXRD pattern for the 1/7 potassium salt of compound **IB-L1-1.1.**
Figure **32** shows an illustrative PXRD pattern for the monodiethylamine salt tetrahydrate of compound **IB-L1-1.1.**
Figure **33** shows an illustrative TGA profile of the monodiethylamine salt tetrahydrate of compound **IB-L1-1.1.**
Figure **34** shows an illustrative PXRD pattern for the pattern A polymorph of compound **IB-L1-1.1.**
Figure **35** shows an illustrative DSC profile of the pattern A polymorph of compound **IB-L1-1.1.**
Figure **36** shows an illustrative PXRD pattern for the pattern B polymorph of compound **IB-L1-1.1.**
Figure **37** shows an illustrative PXRD pattern for the pattern C polymorph of compound **IB-L1-1.1.**
Figure **38** shows an illustrative PXRD pattern for the pattern D polymorph of compound **IB-L1-1.1.**
Figure **39** shows an illustrative PXRD pattern for the pattern A hydrate of compound **IB-L1-1.1.**
Figure **40** shows an illustrative TGA profile of the pattern A hydrate of compound **IB-L1-1.1.**
Figure **41** shows an illustrative PXRD pattern for the pattern B hydrate of compound **IB-L1-1.1.**
Figure **42** shows an illustrative TGA profile of the pattern B hydrate of compound **IB-L1-1.1.**
Figure **43** shows an illustrative PXRD pattern for the pattern C hydrate of compound **IB-L1-1.1.**
Figure **44** shows an illustrative TGA profile of the pattern C hydrate of compound **IB-L1-1.1.**
Figure **45** shows an illustrative PXRD pattern for the pattern D hydrate of compound **IB-L1-1.1.**
Figure **46** shows an illustrative PXRD pattern for the pattern E hydrate of compound **IB-L1-1.1.**

### DETAILED DESCRIPTION OF THE INVENTION

This detailed description is intended only to acquaint others skilled in the art with Applicants' invention, its principles, and its practical application so that others skilled in the art may adapt and apply the invention in its numerous forms, as they may be best suited to the requirements of a particular use. This description and its specific examples are intended for purposes of illustration only. This invention, therefore, is not limited to the embodiments described in this patent application, and may be variously modified.

### A. Definitions.

The term "alkyl" (alone or in combination with another term(s)) means a straight-or branched-chain saturated hydrocarbyl substituent typically containing from 1 to about 20 carbon atoms, more typically from 1 to about 8 carbon atoms, and even more typically from 1 to about 6 carbon atoms. Examples of such substituents include methyl, ethyl, n-propyl, isopropyl, n-butyl, isobutyl, sec-butyl, tert-butyl, pentyl, iso-amyl, and hexyl. As in this definition, throughout this detailed description Applicants have provided illustrative examples. The provision of such illustrative examples should not be interpreted as if the provided illustrative examples are the only options available to one skilled in the art.

The term "alkenyl" (alone or in combination with another term(s)) means a straight- or branched-chain hydrocarbyl substituent containing one or more double bonds and typically from 2 to about 20 carbon atoms, more typically from about 2 to about 8 carbon atoms, and even more typically from about 2 to about 6 carbon atoms. Examples of such substituents include ethenyl (vinyl), 2-propenyl, 3-propenyl, 1,4-pentadienyl, 1,4-butadienyl, 1-butenyl, 2-butenyl, and 3-butenyl.

The term "alkynyl" (alone or in combination with another term(s)) means a straight- or branched-chain hydrocarbyl substituent containing one or more triple bonds and typically from 2 to about 20 carbon atoms, more typically from about 2 to about 8 carbon atoms, and even more typically from about 2 to about 6 carbon atoms. Examples of such substituents include ethynyl, 2-propynyl, 3-propynyl, 2-butynyl, and 3-butynyl.

The term "carbocyclyl" (alone or in combination with another term(s)) means a saturated cyclic (*i.e.,* "cycloalkyl"), partially saturated cyclic (*i.e.,* "cycloalkenyl"), or completely unsaturated (*i.e.,* "aryl") hydrocarbyl substituent containing from 3 to 14 carbon ring atoms ("ring atoms" are the atoms bound together to form the ring or rings of a cyclic substituent). A carbocyclyl may be a single ring, which typically contains from 3 to 6 ring atoms. Examples of such single-ring carbocyclyls include cyclopropyl (cyclopropanyl), cyclobutyl (cyclobutanyl), cyclopentyl (cyclopentanyl), cyclopentenyl, cyclopentadienyl, cyclohexyl (cyclohexanyl), cyclohexenyl, cyclohexadienyl, and phenyl. A carbocyclyl alternatively may be 2 or 3 rings fused together, such as naphthalenyl, tetrahydronaphthalenyl (tetralinyl), indenyl, indanyl (dihydroindenyl), anthracenyl, phenanthrenyl, and decalinyl.

The term "cycloalkyl" (alone or in combination with another term(s)) means a saturated cyclic hydrocarbyl substituent containing from 3 to 14 carbon ring atoms. A cycloalkyl may be a single carbon ring, which typically contains from 3 to 6 carbon ring atoms. Examples of single-ring cycloalkyls include cyclopropyl, cyclobutyl, cyclopentyl, and cyclohexyl. A cycloalkyl alternatively may be 2 or 3 carbon rings fused together, such as, decalinyl.

The term "aryl" (alone or in combination with another term(s)) means an aromatic carbocyclyl containing from 6 to 14 carbon ring atoms. Examples of aryls include phenyl, naphthalenyl, and indenyl.

In some instances, the number of carbon atoms in a hydrocarbyl substituent (e.g., alkyl, alkenyl, alkynyl, or cycloalkyl) is indicated by the prefix "Cₓ-C_{y}-", wherein x is the minimum and y is the maximum number of carbon atoms in the substituent. Thus, for example, "C₁-C₆-alkyl" refers to an alkyl substituent containing from 1 to 6 carbon atoms. Illustrating further, C₃-C₆-cycloalkyl means a saturated hydrocarbyl ring containing from 3 to 6 carbon ring atoms.

The term "hydrogen" (alone or in combination with another term(s)) means a hydrogen radical, and may be depicted as -H.

The term "hydroxy" (alone or in combination with another term(s)) means -OH.

The term "nitro" (alone or in combination with another term(s)) means -NO₂.

The term "cyano" (alone or in combination with another term(s)) means -CN, which also may be depicted as -C≡N.

The term "keto" (alone or in combination with another term(s)) means an oxo radical, and may be depicted as =O.

The term "carboxy" (alone or in combination with another term(s)) means -C(O)-OH.

The term "amino" (alone or in combination with another term(s)) means -NH₂.

The term "imino" (alone or in combination with another term(s)) means =NH.

The term "aminoimino" (alone or in combination with another term(s)) means =NNH₂.

The term "halogen" or "halo" (alone or in combination with another term(s)) means a fluorine radical (which may be depicted as -F), chlorine radical (which may be depicted as -Cl), bromine radical (which may be depicted as -Br), or iodine radical (which may be depicted as -I).

A substituent is "substitutable" if it comprises at least one carbon or nitrogen atom that is bonded to one or more hydrogen atoms. Thus, for example, hydrogen, halogen, and cyano do not fall within this definition. In addition, a sulfur atom in a heterocyclyl containing such atom is substitutable with one or two oxo substituents.

If a substituent is described as being "substituted", a non-hydrogen radical is in the place of hydrogen radical on a carbon or nitrogen of the substituent. Thus, for example, a substituted alkyl substituent is an alkyl substituent in which at least one non-hydrogen radical is in the place of a hydrogen radical on the alkyl substituent. To illustrate, monofluoroalkyl is alkyl substituted with a fluoro radical, and difluoroalkyl is alkyl substituted with two fluoro radicals. It should be recognized that if there are more than one substitution on a substituent, each non-hydrogen radical may be identical or different (unless otherwise stated).

If a substituent is described as being "optionally substituted", the substituent may be either (1) not substituted or (2) substituted. If a substituent is described as being optionally substituted with up to a particular number of non-hydrogen radicals, that substituent may be either (1) not substituted; or (2) substituted by up to that particular number of non-hydrogen radicals or by up to the maximum number of substitutable positions on the substituent, whichever is less. Thus, for example, if a substituent is described as a heteroaryl optionally substituted with up to 3 non-hydrogen radicals, then any heteroaryl with less than 3 substitutable positions would be optionally substituted by up to only as many non-hydrogen radicals as the heteroaryl has substitutable positions. To illustrate, tetrazolyl (which has only one substitutable position) would be optionally substituted with up to one non-hydrogen radical. To illustrate further, if an amino nitrogen is described as being optionally substituted with up to 2 non-hydrogen radicals, then a primary amino nitrogen will be optionally substituted with up to 2 non-hydrogen radicals, whereas a secondary amino nitrogen will be optionally substituted with up to only 1 non-hydrogen radical.

This patent application uses the terms "substituent" and "radical" interchangeably.

The prefix "halo" indicates that the substituent to which the prefix is attached is substituted with one or more independently selected halogen radicals. For example, haloalkyl means an alkyl substituent in which at least one hydrogen radical is replaced with a halogen radical. Examples of haloalkyls include chloromethyl, 1-bromoethyl, fluoromethyl, difluoromethyl, trifluoromethyl, and 1,1,1-trifluoroethyl. It should be recognized that if a substituent is substituted by more than one halogen radical, those halogen radicals may be identical or different (unless otherwise stated).

The prefix "perhalo" indicates that every hydrogen radical on the substituent to which the prefix is attached is replaced with independently selected halogen radicals, *i.e.,* each hydrogen radical on the substituent is replaced with a halogen radical. If all the halogen radicals are identical, the prefix typically will identify the halogen radical. Thus, for example, the term "perfluoro" means that every hydrogen radical on the substituent to which the prefix is attached is substituted with a fluorine radical. To illustrate, the term "perfluoroalkyl" means an alkyl substituent wherein a fluorine radical is in the place of each hydrogen radical.

The term "carbonyl" (alone or in combination with another term(s)) means -C(O)-.

The term "aminocarbonyl" (alone or in combination with another term(s)) means -C(O)-NH₂.

The term "oxy" (alone or in combination with another term(s)) means an ether substituent, and may be depicted as -O-.

The term "alkoxy" (alone or in combination with another term(s)) means an alkylether substituent, i.e., -O-alkyl. Examples of such a substituent include methoxy (-O-CH₃), ethoxy, n-propoxy, isopropoxy, n-butoxy, iso-butoxy, sec-butoxy, and tert-butoxy.

The term "alkylcarbonyl" (alone or in combination with another term(s)) means -C(O)-alkyl.

The term "aminoalkylcarbonyl" (alone or in combination with another term(s)) means -C(O)-alkyl-NH₂.

The term "alkoxycarbonyl" (alone or in combination with another term(s)) means -C(O)-O-alkyl.

The term "carbocyclylcarbonyl" (alone or in combination with another term(s)) means -C(O)-carbocyclyl.

Similarly, the term "heterocyclylcarbonyl" (alone or in combination with another term(s)) means -C(O)-heterocyclyl.

The term "carbocyclylalkylcarbonyl" (alone or in combination with another term(s)) means -C(O)-alkyl-carbocyclyl.

Similarly, the term "heterocyclylalkylcarbonyl" (alone or in combination with another term(s)) means -C(O)-alkyl-heterocyclyl.

The term "carbocyclyloxycarbonyl" (alone or in combination with another term(s)) means -C(O)-O-carbocyclyl.

The term "carbocyclylalkoxycarbonyl" (alone or in combination with another term(s)) means -C(O)-O-alkyl-carbocyclyl.

The term "thio" or "thia" (alone or in combination with another term(s)) means a thiaether substituent, *i.e.,* an ether substituent wherein a divalent sulfur atom is in the place of the ether oxygen atom. Such a substituent may be depicted as -S-. This, for example, "alkyl-thio-alkyl" means alkyl-S-alkyl (alkyl-sulfanyl-alkyl).

The term "thiol" or "sulfhydryl" (alone or in combination with another term(s)) means a sulfhydryl substituent, and may be depicted as -SH.

The term "(thiocarbonyl)" (alone or in combination with another term(s)) means a carbonyl wherein the oxygen atom has been replaced with a sulfur. Such a substituent may be depicted as -C(S)-.

The term "sulfonyl" (alone or in combination with another term(s)) means -S(O)₂-.

The term "aminosulfonyl" (alone or in combination with another term(s)) means -S(O)₂-NH₂.

The term "sulfinyl" or "sulfoxido" (alone or in combination with another term(s)) means -S(O)-.

The term "heterocyclyl" (alone or in combination with another term(s)) means a saturated (*i.e.,* "heterocycloalkyl"), partially saturated *(i.e.,* "heterocycloalkenyl"), or completely unsaturated (*i.e.,* "heteroaryl") ring structure containing a total of 3 to 14 ring atoms. At least one of the ring atoms is a heteroatom (*i.e.,* oxygen, nitrogen, or sulfur), with the remaining ring atoms being independently selected from the group consisting of carbon, oxygen, nitrogen, and sulfur.

A heterocyclyl may be a single ring, which typically contains from 3 to 7 ring atoms, more typically from 3 to 6 ring atoms, and even more typically 5 to 6 ring atoms. Examples of single-ring heterocyclyls include furanyl, dihydrofuranyl, tetrahydrofuranyl, thiophenyl (thiofuranyl), dihydrothiophenyl, tetrahydrothiophenyl, pyrrolyl, pyrrolinyl, pyrrolidinyl, imidazolyl, imidazolinyl, imidazolidinyl, pyrazolyl, pyrazolinyl, pyrazolidinyl, triazolyl, tetrazolyl, oxazolyl, oxazolidinyl, isoxazolidinyl, isoxazolyl, thiazolyl, isothiazolyl, thiazolinyl, isothiazolinyl, thiazolidinyl, isothiazolidinyl, thiodiazolyl, oxadiazolyl (including 1,2,3-oxadiazolyl, 1,2,4-oxadiazolyl, 1,2,5-oxadiazolyl (furazanyl), or 1,3,4-oxadiazolyl), oxatriazolyl (including 1,2,3,4-oxatriazolyl or 1,2,3,5-oxatriazolyl), dioxazolyl (including 1,2,3-dioxazolyl, 1,2,4-dioxazolyl, 1,3,2-dioxazolyl, or 1,3,4-dioxazolyl), oxathiazolyl, oxathiolyl, oxathiolanyl, pyranyl, dihydropyranyl, thiopyranyl, tetrahydrothiopyranyl, pyridinyl (azinyl), piperidinyl, diazinyl (including pyridazinyl (1,2-diazinyl), pyrimidinyl (1,3-diazinyl), or pyrazinyl (1,4-diazinyl)), piperazinyl, triazinyl (including 1,3,5-triazinyl, 1,2,4-triazinyl, and 1,2,3-triazinyl)), oxazinyl (including 1,2-oxazinyl, 1,3-oxazinyl, or 1,4-oxazinyl)), oxathiazinyl (including 1,2,3-oxathiazinyl, 1,2,4-oxathiazinyl, 1,2,5-oxathiazinyl, or 1,2,6-oxathiazinyl)), oxadiazinyl (including 1,2,3-oxadiazinyl, 1,2,4-oxadiazinyl, 1,4,2-oxadiazinyl, or 1,3,5-oxadiazinyl)), morpholinyl, azepinyl, oxepinyl, thiepinyl, and diazepinyl.

A heterocyclyl alternatively may be 2 or 3 rings fused together, such as, for example, indolizinyl, pyranopyrrolyl, 4H-quinolizinyl, purinyl, naphthyridinyl, pyridopyridinyl (including pyrido[3,4-b]-pyridinyl, pyrido[3,2-b]-pyridinyl, or pyrido[4,3-b]-pyridinyl), and pteridinyl. Other examples of fused-ring heterocyclyls include benzo-fused heterocyclyls, such as indolyl, isoindolyl (isobenzazolyl, pseudoisoindolyl), indoleninyl (pseudoindolyl), isoindazolyl (benzpyrazolyl), benzazinyl (including quinolinyl (1-benzazinyl) or isoquinolinyl (2-benzazinyl)), phthalazinyl, quinoxalinyl, quinazolinyl, benzodiazinyl (including cinnolinyl (1,2-benzodiazinyl) or quinazolinyl (1,3-benzodiazinyl)), benzopyranyl (including chromanyl or isochromanyl), benzoxazinyl (including 1,3,2-benzoxazinyl, 1,4,2-benzoxazinyl, 2,3,1-benzoxazinyl, or 3,1,4-benzoxazinyl), and benzisoxazinyl (including 1,2-benzisoxazinyl or 1,4-benzisoxazinyl).

The term "2-fused ring" heterocyclyl (alone or in combination with another term(s)) means a saturated, partially saturated, or aryl heterocyclyl containing 2 fused rings. Examples of 2-fused-ring heterocyclyls include indolizinyl, quinolizinyl, purinyl, naphthyridinyl, pteridinyl, indolyl, isoindolyl, indoleninyl, isoindazolyl, phthalazinyl, quinoxalinyl, quinazolinyl, benzodiazinyl, benzopyranyl, benzothiopyranyl, benzoxazolyl, anthranilyl, benzodioxolyl, benzodioxanyl, benzoxadiazolyl, benzofuranyl, isobenzofuranyl, benzothiazolyl, benzothiadiazolyl, benzimidazolyl, benzotriazolyl, benzoxazinyl, and tetrahydroisoquinolinyl.

The term "heteroaryl" (alone or in combination with another term(s)) means an aromatic heterocyclyl containing from 5 to 14 ring atoms. A heteroaryl may be a single ring or 2 or 3 fused rings. Examples of heteroaryl substituents include 6-membered ring substituents such as pyridyl, pyrazyl, pyrimidinyl, pyridazinyl, and 1,3,5-, 1,2,4- or 1,2,3-triazinyl; 5-membered ring substituents such as imidazyl, furanyl, thiophenyl, pyrazolyl, oxazolyl, isoxazolyl, thiazolyl, 1,2,3-, 1,2,4-, 1,2,5-, or 1,3,4-oxadiazolyl and isothiazolyl; 6/5-membered fused ring substituents such as benzothiofuranyl, benzisoxazolyl, benzoxazolyl, purinyl, and anthranilyl; and 6/6-membered fused rings such as benzopyranyl, quinolinyl, isoquinolinyl, cinnolinyl, quinazolinyl, and benzoxazinyl.

A prefix attached to a multi-component substituent only applies to the first component. To illustrate, the term "alkylcycloalkyl" contains two components: alkyl and cycloalkyl. Thus, the C₁-C₆-prefix on C₁-C₆-alkylcycloalkyl means that the alkyl component of the alkylcycloalkyl contains from 1 to 6 carbon atoms; the C₁-C₆-prefix does not describe the cycloalkyl component. To illustrate further, the prefix "halo" on haloalkoxyalkyl indicates that only the alkoxy component of the alkoxyalkyl substituent is substituted with one or more halogen radicals. If halogen substitution may alternatively or additionally occur on the alkyl component, the substituent would instead be described as "halogen-substituted alkoxyalkyl" rather than "haloalkoxyalkyl." And finally, if the halogen substitution may only occur on the alkyl component, the substituent would instead be described as "alkoxyhaloalkyl."

If substituents are described as being "independently selected" from a group, each substituent is selected independent of the other. Each substituent therefore may be identical to or different from the other substituent(s).

When words are used to describe a substituent, the rightmost-described component of the substituent is the component that has the free valence.

When a chemical formula is used to describe a substituent, the dash on the left side of the formula indicates the portion of the substituent that has the free valence.

When a chemical formula is used to describe a linking element between two other elements of a depicted chemical structure, the leftmost dash of the substituent indicates the portion of the substituent that is bound to the left element in the depicted structure. The rightmost dash, on the other hand, indicates the portion of the substituent that is bound to the right element in the depicted structure. To illustrate, if the depicted chemical structure is X-L-Y and L is described as -C(O)-N(H)-, then the chemical would be X-C(O)-N(H)-Y.

With reference to the use of the words "comprise" or "comprises" or "comprising" in this patent application (including the claims), Applicants note that unless the context requires otherwise, those words are used on the basis and clear understanding that they are to be interpreted inclusively, rather than exclusively, and that Applicants intend each of those words to be so interpreted in construing this patent application, including the claims below.

ChemDraw software has been used to generate the compound names in this patent application.

The term "amorphous" as applied to a compound refers to a solid-state in which the compound molecules are present in a disordered arrangement and do not form a distinguishable crystal lattice or unit cell. When subjected to X-ray powder diffraction, an amorphous compound does not produce any characteristic crystalline peaks.

The term "crystalline form" as applied to a compound refers to a solid-state in which the compound molecules are arranged to form a distinguishable crystal lattice (i) comprising distinguishable unit cells, and (ii) yielding diffraction pattern peaks when subjected to X-ray radiation.

The term "purity", unless otherwise qualified, means the chemical purity of a compound according to conventional HPLC assay.

The term "phase purity" means the solid-state purity of a compound with regard to a particular crystalline or amorphous form of the compound as determined by X-ray powder diffraction analytical methods.

The term "phase pure" refers to purity with respect to other solid-state forms of the compound, and does not necessarily imply a high degree of chemical purity with respect to other compounds.

The term "PXRD" means X-ray powder diffraction.

The term "TGA" means thermogravimetric analysis.

The term "DSC" means differential scanning calorimetry.

### B. Compounds.

This invention is directed, in part, to compounds that are phenyl-uracil derivatives that correspond in structure to formula **I:**

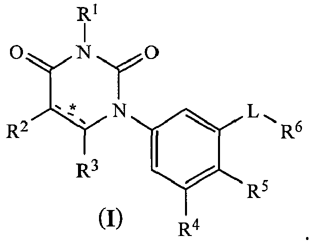

In these compounds, 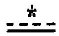 is selected from the group consisting of single carbon-carbon bond and double carbon-carbon bond.

In some embodiments, 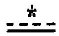 is a single carbon-carbon bond. In these embodiments, the compounds of formula I correspond in structure to the following formula *(i.e.,* formula **IA**):

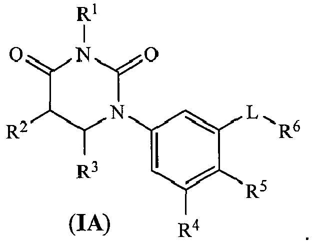

In other embodiments, 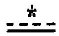 is a double carbon-carbon bond. In these embodiments, the compounds of formula I correspond in structure to the following formula *(i.e.,* formula **IB**):

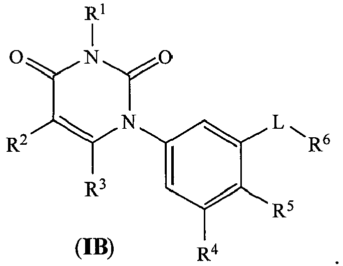

### B1. Substituent R¹.

**R¹** is selected from the group consisting of hydrogen, methyl, and nitrogen-protecting group.

In some embodiments, **R¹** is hydrogen.

In some embodiments, **R¹** is methyl.

In some embodiments, **R¹** is selected from the group consisting of hydrogen and methyl.

In some embodiments, **R¹** is a nitrogen-protecting group. In these embodiments, the compounds are useful as intermediates for the preparation of compounds of formula **I.** Nitrogen-protecting groups suitable for preparing compounds of formula **I** are known to those skilled in the art.

### B2. Substituent R².

**R²** is selected from the group consisting of hydrogen, halo, hydroxy, methyl, cyclopropyl, and cyclobutyl.

In some embodiments, **R²** is hydrogen.

In some embodiments, **R²** is halo. In some such embodiments, **R²** is selected from the group consisting of fluoro and chloro. In other such embodiments, **R²** is fluoro. In yet other such embodiments, **R²** is chloro. In yet other such embodiments, **R²** is bromo. In further such embodiments, **R²** is iodo.

In some embodiments, **R²** is hydroxy.

In some embodiments, **R²** is methyl.

In some embodiments, **R²** is cyclopropyl.

In some embodiments, **R²** is cyclobutyl.

In some embodiments, **R²** is selected from the group consisting of hydrogen, methyl, hydroxy, and halo. In some such embodiments, **R²** is selected from the group consisting of hydrogen, methyl, hydroxy, fluoro, and chloro. In other such embodiments, **R²** is selected from the group consisting of hydrogen, methyl, hydroxy, and fluoro. In yet other such embodiments, **R²** is selected from the group consisting of hydrogen, methyl, hydroxy, and chloro. In yet other such embodiments, **R²** is selected from the group consisting of hydrogen, methyl, hydroxy, and bromo. In further such embodiments, **R²** is selected from the group consisting of hydrogen, methyl, hydroxy, and iodo.

In some embodiments, **R²** is selected from the group consisting of hydrogen, methyl, and halo. In some such embodiments, **R²** is selected from the group consisting of hydrogen, methyl, fluoro, and chloro. In other such embodiments, **R²** is selected from the group consisting of hydrogen, methyl, and fluoro. In yet other such embodiments, **R²** is selected from the group consisting of hydrogen, methyl, and chloro. In yet other such embodiments, **R²** is selected from the group consisting of hydrogen, methyl, and bromo. In further such embodiments, **R²** is selected from the group consisting of hydrogen, methyl, and iodo.

In some embodiments, **R²** is selected from the group consisting of hydrogen and halo. In some such embodiments, **R²** is selected from the group consisting of hydrogen, fluoro, and chloro. In other such embodiments, **R²** is selected from the group consisting of hydrogen and fluoro. In yet other such embodiments, **R²** is selected from the group consisting of hydrogen and chloro. In yet other such embodiments, **R²** is selected from the group consisting of hydrogen and bromo. In further such embodiments, **R²** is selected from the group consisting of hydrogen and iodo.

### B3. Substituent R³.

**R³** is selected from the group consisting of hydrogen, halo, oxo, and methyl. In some such embodiments, **R³** is selected from the group consisting of hydrogen, fluoro, oxo, and methyl. In other such embodiments, **R³** is selected from the group consisting of hydrogen, chloro, oxo, and methyl. In yet other such embodiments, **R³** is selected from the group consisting of hydrogen, bromo, oxo, and methyl. In yet other such embodiments, **R³** is selected from the group consisting of hydrogen, iodo, oxo, and methyl.

In some embodiments, **R³** is selected from the group consisting of hydrogen, halo, and oxo. In some such embodiments, **R³** is selected from the group consisting of hydrogen, fluoro, and oxo. In other such embodiments, **R³** is selected from the group consisting of hydrogen, chloro, and oxo. In yet other such embodiments, **R³** is selected from the group consisting of hydrogen, bromo, and oxo. In yet other such embodiments, **R³** is selected from the group consisting of hydrogen, iodo, and oxo.

In some embodiments, **R³** is selected from the group consisting of hydrogen and methyl.

In some embodiments, **R³** is hydrogen.

In some embodiments, **R³** is methyl.

In some embodiments, **R³** is oxo.

In some embodiments, **R³** is halo. In some such embodiments, **R³** is fluoro. In other such embodiments, **R³** is chloro. In yet other such embodiments, **R³** is bromo. In further such embodiments, **R³** is iodo.

### B4. Substituent R⁴.

**R⁴** is selected from the group consisting of halo, alkyl, alkenyl, alkynyl, nitro, cyano, azido, alkyloxy, alkenyloxy, alkynyloxy, amino, aminocarbonyl, aminosulfonyl, alkylsulfonyl, carbocyclyl, and heterocyclyl, wherein:
(**a**) the amino, aminocarbonyl, and aminosulfonyl optionally are substituted with:
   (**1**) one or two substituents independently selected from the group consisting of alkyl, alkenyl, alkynyl, and alkylsulfonyl, or
   (**2**) two substituents that, together with the amino nitrogen, form a single-ring heterocyclyl,
(**b**) the alkyl, alkenyl, alkynyl, alkyloxy, alkenyloxy, alkynyloxy, and alkylsulfonyl, optionally are substituted with one or more substituents independently selected from the group consisting of halo, oxo, nitro, cyano, azido, hydroxy, amino, alkyloxy, trimethylsilyl, carbocyclyl, and heterocyclyl, wherein:
   the amino optionally is substituted with:
      (**1**) one or two substituents independently selected from the group consisting of alkyl, alkenyl, alkynyl, alkylcarbonyl, alkylsulfonyl, alkyloxycarbonyl, carbocyclyl, heterocyclyl, carbocyclylalkyl, and heterocyclylalkyl, or
      (**2**) two substituents that, together with the amino nitrogen, form a single-ring heterocyclyl, and
(**c**) the carbocyclyl and heterocyclyl optionally are substituted with up to three substituents independently selected from the group consisting of alkyl, alkenyl, alkynyl, halo, oxo, nitro, cyano, azido, hydroxy, amino, alkyloxy, trimethylsilyl, carbocyclyl, and heterocyclyl, wherein:
   the amino optionally is substituted with:
      (**1**) one or two substituents independently selected from the group consisting of alkyl, alkenyl, alkynyl, alkylcarbonyl, alkylsulfonyl, alkyloxycarbonyl, carbocyclyl, heterocyclyl, carbocyclylalkyl, and heterocyclylalkyl, or
      (**2**) two substituents that, together with the amino nitrogen, form a single-ring heterocyclyl.

In some embodiments, **R⁴** is selected from the group consisting of halo, alkyl, alkenyl, alkynyl, nitro, cyano, azido, alkyloxy, alkenyloxy, alkynyloxy, amino, aminocarbonyl, aminosulfonyl, alkylsulfonyl, carbocyclyl, and heterocyclyl, wherein:
the amino, aminocarbonyl, and aminosulfonyl optionally are substituted with:
   (**1**) one or two substituents independently selected from the group consisting of alkyl, alkenyl, alkynyl, and alkylsulfonyl, or
   (**2**) two substituents that, together with the amino nitrogen, form a single-ring heterocyclyl.

In some embodiments, **R⁴** is selected from the group consisting of halo, alkyl, alkenyl, alkynyl, nitro, cyano, azido, alkyloxy, alkenyloxy, alkynyloxy, amino, aminocarbonyl, aminosulfonyl, alkylsulfonyl, carbocyclyl, and heterocyclyl, wherein:
the alkyl, alkenyl, alkynyl, alkyloxy, alkenyloxy, alkynyloxy, and alkylsulfonyl, optionally are substituted with one or more substituents independently selected from the group consisting of halo, oxo, nitro, cyano, azido, hydroxy, amino, alkyloxy, trimethylsilyl, carbocyclyl, and heterocyclyl, wherein:
   the amino optionally is substituted with:
      (**1**) one or two substituents independently selected from the group consisting of alkyl, alkenyl, alkynyl, alkylcarbonyl, alkylsulfonyl, alkyloxycarbonyl, carbocyclyl, heterocyclyl, carbocyclylalkyl, and heterocyclylalkyl, or
      (**2**) two substituents that, together with the amino nitrogen, form a single-ring heterocyclyl.

In some embodiments, **R⁴** is selected from the group consisting of halo, alkyl, alkenyl, alkynyl, nitro, cyano, azido, alkyloxy, alkenyloxy, alkynyloxy, amino, aminocarbonyl, aminosulfonyl, alkylsulfonyl, carbocyclyl, and heterocyclyl, wherein:
the carbocyclyl and heterocyclyl optionally are substituted with up to three substituents independently selected from the group consisting of alkyl, alkenyl, alkynyl, halo, oxo, nitro, cyano, azido, hydroxy, amino, alkyloxy, trimethylsilyl, carbocyclyl, and heterocyclyl, wherein:
   the amino optionally is substituted with:
      (**1**) one or two substituents independently selected from the group consisting of alkyl, alkenyl, alkynyl, alkylcarbonyl, alkylsulfonyl, alkyloxycarbonyl, carbocyclyl, heterocyclyl, carbocyclylalkyl, and heterocyclylalkyl, or
      (**2**) two substituents that, together with the amino nitrogen, form a single-ring heterocyclyl.

In some embodiments, **R⁴** is selected from the group consisting of halo, alkyl, alkenyl, alkynyl, nitro, cyano, azido, alkyloxy, alkenyloxy, alkynyloxy, amino, aminocarbonyl, aminosulfonyl, alkylsulfonyl, carbocyclyl, and heterocyclyl, wherein:
(**a**) the amino, aminocarbonyl, and aminosulfonyl optionally are substituted with:
   (**1**) one or two substituents independently selected from the group consisting of alkyl, alkenyl, and alkynyl, or,
   (**2**) two substituents that, together with the amino nitrogen, form a single-ring heterocyclyl; and
(**b**) the alkyl, alkenyl, alkynyl, alkyloxy, alkenyloxy, alkynyloxy, alkylsulfonyl, carbocyclyl, and heterocyclyl optionally are substituted with up to three substituents independently selected from the group consisting of halo, oxo, nitro, cyano, azido, hydroxy, amino, alkyloxy, carbocyclyl, and heterocyclyl, wherein the amino optionally is substituted with:
   (**1**) one or two substituents independently selected from the group consisting of alkyl, alkenyl, alkynyl, alkylcarbonyl, alkylsulfonyl, alkyloxycarbonyl, carbocyclyl, heterocyclyl, carbocyclylalkyl, and heterocyclylalkyl, or,
   (**2**) two substituents that, together with the amino nitrogen, form a single-ring heterocyclyl.

In some embodiments, **R⁴** is selected from the group consisting of halo, alkyl, alkenyl, alkynyl, nitro, cyano, azido, alkyloxy, alkenyloxy, alkynyloxy, amino, aminocarbonyl, aminosulfonyl, alkylsulfonyl, carbocyclyl, and heterocyclyl, wherein:
the amino, aminocarbonyl, and aminosulfonyl optionally are substituted with:
   (**1**) one or two substituents independently selected from the group consisting of alkyl, alkenyl, and alkynyl, or,
   (**2**) two substituents that, together with the amino nitrogen, form a single-ring heterocyclyl.

In some embodiments, **R⁴** is selected from the group consisting of halo, alkyl, alkenyl, alkynyl, nitro, cyano, azido, alkyloxy, alkenyloxy, alkynyloxy, amino, aminocarbonyl, aminosulfonyl, alkylsulfonyl, carbocyclyl, and heterocyclyl, wherein:
the alkyl, alkenyl, alkynyl, alkyloxy, alkenyloxy, alkynyloxy, alkylsulfonyl, carbocyclyl, and heterocyclyl optionally are substituted with up to three substituents independently selected from the group consisting of halo, oxo, nitro, cyano, azido, hydroxy, amino, alkyloxy, carbocyclyl, and heterocyclyl, wherein the amino optionally is substituted with:
   (**1**) one or two substituents independently selected from the group consisting of alkyl, alkenyl, alkynyl, alkylcarbonyl, alkylsulfonyl, alkyloxycarbonyl, carbocyclyl, heterocyclyl, carbocyclylalkyl, and heterocyclylalkyl, or,
   (**2**) two substituents that, together with the amino nitrogen, form a single-ring heterocyclyl.

In some embodiments, **R⁴** is selected from the group consisting of halo, C₁-C₄-alkyl, C₂-C₄-alkenyl, C₂-C₄-alkynyl, amino, C₁-C₄-alkylsulfonyl, C₃-C₆-carbocyclyl, and 5-6-membered heterocyclyl, wherein:
(**a**) the amino optionally is substituted with one or two substituents independently selected from the group consisting of alkyl, alkenyl, alkynyl, and alkylsulfonyl,
(**b**) the C₁-C₄-alkyl, C₂-C₄-alkenyl, and C₂-C₄-alkynyl optionally are substituted with one or more substituents independently selected from the group consisting of halo, oxo, hydroxy, alkyloxy, and trimethylsilyl, and
(**c**) the C₃-C₆-carbocyclyl and 5-6-membered heterocyclyl optionally are substituted with up to three substituents independently selected from the group consisting of alkyl, alkenyl, alkynyl, halo, and amino, wherein:
   the amino optionally is substituted with one or two substituents independently selected from the group consisting of alkyl, alkenyl, alkynyl, and alkylsulfonyl.

In some embodiments, **R⁴** is selected from the group consisting of C₁-C₄-alkyl, C₂-C₄-alkenyl, C₂-C₄-alkynyl, amino, C₁-C₄-alkylsulfonyl, C₃-C₆-carbocyclyl, and 5-6-membered heterocyclyl, wherein:
(**a**) the amino optionally is substituted with one or two substituents independently selected from the group consisting of alkyl, alkenyl, alkynyl, and alkylsulfonyl,
(**b**) the C₁-C₄-alkyl, C₂-C₄-alkenyl, and C₂-C₄-alkynyl optionally are substituted with one or more substituents independently selected from the group consisting of halo, oxo, hydroxy, alkyloxy, and trimethylsilyl, and
(**c**) the C₃-C₆-carbocyclyl and 5-6-membered heterocyclyl optionally are substituted with up to three substituents independently selected from the group consisting of alkyl, alkenyl, alkynyl, halo, and amino, wherein:
   the amino optionally is substituted with one or two substituents independently selected from the group consisting of alkyl, alkenyl, alkynyl, and alkylsulfonyl.

In some embodiments, **R⁴** is selected from the group consisting of halo, C₁-C₄-alkyl, C₃-C₆-carbocyclyl, and 5-6-membered heterocyclyl, wherein:
(**a**) the C₁-C₄-alkyl optionally is substituted with up to three substituents independently selected from the group consisting of halo, oxo, hydroxy, alkyloxy, and trimethylsilyl, and
(**b**) the C₃-C₆-carbocyclyl and 5-6-membered heterocyclyl optionally are substituted with one or two substituents independently selected from the group consisting of alkyl, halo, and alkylsulfonylamino.

In some embodiments, **R⁴** is selected from the group consisting of halo, C₁-C₄-alkyl, C₃-C₆-carbocyclyl, and 5-6-membered heterocyclyl, wherein:
(**a**) the C₁-C₄-alkyl optionally is substituted with one or two substituents independently selected from the group consisting of halo, oxo, hydroxy, alkyloxy, and trimethylsilyl, and
(**b**) the C₃-C₆-carbocyclyl and 5-6-membered heterocyclyl optionally are substituted with a substituent selected from the group consisting of alkyl, halo, and alkylsulfonylamino.

In some embodiments, **R⁴** is selected from the group consisting of C₁-C₄-alkyl, C₃-C₆-carbocyclyl, and 5-6-membered heterocyclyl, wherein:
(**a**) the C₁-C₄-alkyl optionally is substituted with up to three substituents independently selected from the group consisting of halo, oxo, hydroxy, alkyloxy, and trimethylsilyl, and
(**b**) the C₃-C₆-carbocyclyl and 5-6-membered heterocyclyl optionally are substituted with one or two substituents independently selected from the group consisting of alkyl, halo, and alkylsulfonylamino.

In some embodiments, **R⁴** is selected from the group consisting of halo, tert-butyl, C₃-C₆-carbocyclyl, and 5-6-membered heterocyclyl, wherein:
the C₃-C₆-carbocyclyl and 5-6-membered heterocyclyl optionally are substituted with a substituent selected from the group consisting of alkyl, halo, and alkylsulfonylamino.

In some embodiments, **R⁴** is selected from the group consisting of tert-butyl, C₃-C₆-carbocyclyl, and 5-6-membered heterocyclyl, wherein:
the C₃-C₆-carbocyclyl and 5-6-membered heterocyclyl optionally are substituted with a substituent selected from the group consisting of alkyl, halo, and alkylsulfonylamino.

In some embodiments, **R⁴** is selected from the group consisting of halo, alkyl, haloalkyl, carboxyalkyl, hydroxyalkyl, alkyloxyalkyl, trimethylsilylalkynyl, alkylcarbocyclyl, carbocyclyl, alkylheterocyclyl, heterocyclyl, halocarbocyclyl, alkylsulfonylamino, and alkylsulfonyl.

In some embodiments, **R⁴** is selected from the group consisting of halo, alkyl, alkenyl, alkynyl, nitro, cyano, azido, alkyloxy, alkenyloxy, alkynyloxy, amino, aminocarbonyl, aminosulfonyl, alkylsulfonyl, carbocyclyl, and heterocyclyl.

In some embodiments, **R⁴** is selected from the group consisting of halo, C₁-C₄-alkyl, C₂-C₄-alkenyl, C₂-C₄-alkynyl, amino, C₁-C₄-alkylsulfonyl, C₃-C₆-carbocyclyl, and 5-6-membered heterocyclyl. In some such embodiment, **R⁴** is selected from the group consisting of halo, C₁-C₄-alkyl, C₂-C₄-alkenyl, C₂-C₄-alkynyl, amino, C₁-C₄-alkylsulfonyl, C₆-carbocyclyl, and 5-6-membered heterocyclyl. In other such embodiment, **R⁴** is selected from the group consisting of halo, C₁-C₄-alkyl, C₂-C₄-alkenyl, C₂-C₄-alkynyl, amino, C₁-C₄-alkylsulfonyl, phenyl, and 5-6-membered heteroaryl.

In some embodiments, **R⁴** is selected from the group consisting of C₁-C₄-alkyl, C₂-C₄-alkenyl, C₂-C₄-alkynyl, amino, C₁-C₄-alkylsulfonyl, C₃-C₆-carbocyclyl, and 5-6-membered heterocyclyl. In some such embodiment, **R⁴** is selected from the group consisting of C₁-C₄-alkyl, C₂-C₄-alkenyl, C₂-C₄-alkynyl, amino, C₁-C₄-alkylsulfonyl, C₆-carbocyclyl, and 5-6-membered heterocyclyl. In other such embodiment, **R⁴** is selected from the group consisting of C₁-C₄-alkyl, C₂-C₄-alkenyl, C₂-C₄-alkynyl, amino, C₁-C₄-alkylsulfonyl, phenyl, and 5-6-membered heteroaryl.

In some embodiments, **R⁴** is selected from the group consisting of halo, C₁-C₄-alkyl, C₃-C₆-carbocyclyl, and 5-6-membered heterocyclyl. In some such embodiments, **R⁴** is selected from the group consisting of halo, C₁-C₄-alkyl, C₆-carbocyclyl, and 5-6-membered heterocyclyl. In other such embodiments, **R⁴** is selected from the group consisting of halo, C₁-C₄-alkyl, phenyl, and 5-6-membered heteroaryl.

In some embodiments, **R⁴** is selected from the group consisting of C₁-C₄-alkyl, C₃-C₆-carbocyclyl, and 5-6-membered heterocyclyl. In some such embodiments, **R⁴** is selected from the group consisting of C₁-C₄-alkyl, C₆-carbocyclyl, and 5-6-membered heterocyclyl. In other such embodiments, **R⁴** is selected from the group consisting of C₁-C₄-alkyl, phenyl, and 5-6-membered heteroaryl.

In some embodiments, **R⁴** is selected from the group consisting of halo, tert-butyl, C₃-C₆-carbocyclyl, and 5-6-membered heterocyclyl. In some such embodiments, **R⁴** is selected from the group consisting of halo, tert-butyl, C₆-carbocyclyl, and 5-6-membered heterocyclyl. In other such embodiments, **R⁴** is selected from the group consisting of halo, tert-butyl, phenyl, and 5-6-membered heteroaryl.

In some embodiments, **R⁴** is selected from the group consisting of tert-butyl, C₃-C₆-carbocyclyl, and 5-6-membered heterocyclyl. In some such embodiments, **R⁴** is selected from the group consisting of tert-butyl, C₆-carbocyclyl, and 5-6-membered heterocyclyl. In other such embodiments, **R⁴** is selected from the group consisting of tert-butyl, phenyl, and 5-6-membered heteroaryl.

In some embodiments, **R⁴** is selected from the group consisting of C₃-C₆-carbocyclyl and 5-6-membered heterocyclyl. In some such embodiments, **R⁴** is selected from the group consisting of C₆-carbocyclyl, and 5-6-membered heterocyclyl. In other such embodiments, **R⁴** is selected from the group consisting of phenyl and 5-6-membered heteroaryl.

Suitable carbocyclyls for the above embodiments include, for example, cyclopropyl and phenyl.

Suitable heterocyclyls for the above embodiments include, for example, furanyl, thienyl, and pyridinyl.

In some embodiments, **R⁴** is selected from the group consisting of halo, alkyl, and alkyloxy.

In some embodiments, **R⁴** is alkyl.

In some embodiments, **R⁴** is tert-butyl.

### B5. Substituent R⁵.

**R⁵** is selected from the group consisting of hydrogen, hydroxy, alkyl, alkenyl, alkynyl, alkyloxy, alkenyloxy, alkynyloxy, alkylsulfonyloxy, carbocyclylsulfonyloxy, haloalkylsulfonyloxy, and halo.

In some embodiments, **R⁵** is selected from the group consisting of hydrogen, hydroxy, alkyloxy, and halo. In some such embodiments, **R⁵** is selected from the group consisting of hydrogen, hydroxy, alkyloxy, and fluoro. In other such embodiments, **R⁵** is selected from the group consisting of hydrogen, hydroxy, alkyloxy, and fluoro. In yet other such embodiments, **R⁵** is selected from the group consisting of hydrogen, hydroxy, alkyloxy, and chloro. In yet other such embodiments, **R⁵** is selected from the group consisting of hydrogen, hydroxy, alkyloxy, and bromo. In further such embodiments, **R⁵** is selected from the group consisting of hydrogen, hydroxy, alkyloxy, and iodo.

In some embodiments, **R⁵** is selected from the group consisting of hydrogen, hydroxy, methoxy, and halo. In some such embodiments, **R⁵** is selected from the group consisting of hydrogen, hydroxy, methoxy, and fluoro. In other such embodiments, **R⁵** is selected from the group consisting of hydrogen, hydroxy, methoxy, and chloro. In yet other such embodiments, **R⁵** is selected from the group consisting of hydrogen, hydroxy, methoxy, and bromo. In further such embodiments, **R⁵** is selected from the group consisting of hydrogen, hydroxy, methoxy, and iodo.

In some embodiments, **R⁵** is selected from the group consisting of hydrogen, hydroxy, and alkyloxy. In some such embodiments, **R⁵** is selected from the group consisting of hydrogen, hydroxy, methoxy, and ethoxy.

In some embodiments, **R⁵** is s hydrogen.

In some embodiments, **R⁵** is hydroxy.

In some embodiments, **R⁵** is alkyloxy.

In some embodiments, **R⁵** is methoxy.

In some embodiments, **R⁵** is ethoxy.

### B6. Substituent L.

**L** is selected from the group consisting of bond, C(**R^{A}**)=C(**R^{B}**), C≡C, C(O)N(**R^{C}**), N(**R^{D}**)C(O), C₁-C₂-alkylene, C(H)₂O, OC(H)₂, cyclopropyl-1,2-ene, C(H)₂N(**R^{L}**), N(**R^{M}**)C(H)₂, C(O)CH₂, and CH₂C(O), wherein **R^{A}**, **R^{B}**, **R^{C}**, **R^{D}**, **R^{L}**, and **R^{M}** are as discussed below.

In some embodiments, **L** is selected from the group consisting of bond, C(**R^{A}**)=C(**R^{B}**), C≡C, C(O)N(**R^{C}**), N(**R^{D}**)C(O), C₁-C₂-alkylene, C(H)₂O, OC(H)₂, cyclopropyl-1,2-ene, C(H)₂N(**R^{L}**), and N(**R^{M}**)C(H)₂.

In some embodiments, **L** is selected from the group consisting of C(**R^{A}**)=C(**R^{B}**), ethylene, and cyclopropyl-1,2-ene.

In some embodiments, **L** is selected from the group consisting of C(**R^{A}**)=C(**R^{B}**), C≡C, C(O)N(**R^{C}**), N(**R^{D}**)C(O), C₁-C₂-alkylene, C(H)₂O, OC(H)₂, cyclopropyl-1,2-ene, C(H)₂N(**R^{L}**), N(**R^{M}**)C(H)₂, C(O)CH₂, and CH₂C(O).

In some embodiments, **L** is selected from the group consisting of C=C, C(O)N(**R^{C}**), N(**R^{D}**)C(O), C(H)₂O, OC(H)₂, C(H)₂N(**R^{L}**), and N(**R^{M}**)C(H)₂.

In some embodiments, **L** is a bond. In these embodiments, the compounds of formula **I** correspond in structure to formula **I-L0:**

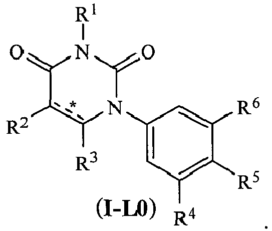

In some such embodiments, the compounds correspond in structure to the following formula (*i.e.,* formula **IA-L0**):

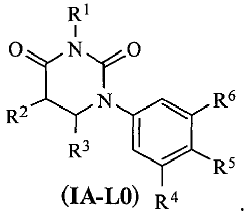

In other such embodiments, the compounds correspond in structure to the following formula (*i.e.,* formula **IB-L0**):

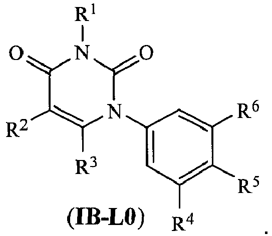

In some embodiments, **L** is C(**R^{A}**)=C(**R^{B}**), wherein **R^{A}** and **R^{B}** are as discussed below. In these embodiments, the compounds of formula **I** correspond in structure to formula **I-L1:**

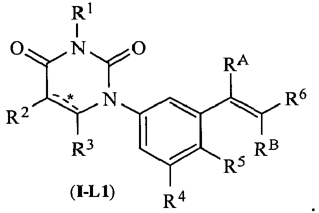

In some such embodiments, the compounds correspond in structure to formula **IA-L1:**

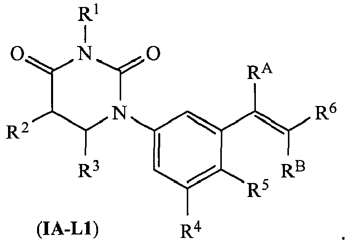

In other such embodiments, the compounds correspond in structure to formula **IB-L1:**

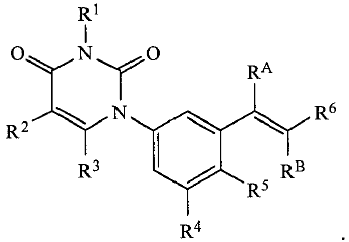

Typically, the compounds of formula **I-L1** are more potent if **R⁶** and the phenyl-uracil are on opposite sides of the double bond (*i.e.,* in trans configuration in relation to the double bond).

In some embodiments, **L** is C≡C. In these embodiments, the compounds of formula **I** correspond in structure to formula **I-L2:**

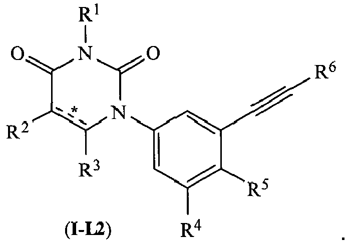

In some such embodiments, the compounds correspond in structure to **IA-L2:**

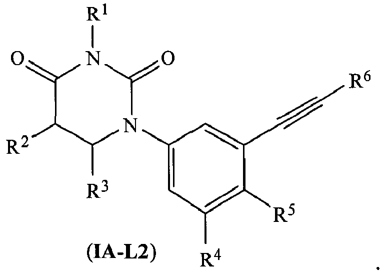

In other such embodiments, the compounds correspond in structure to formula **IB-L2:**

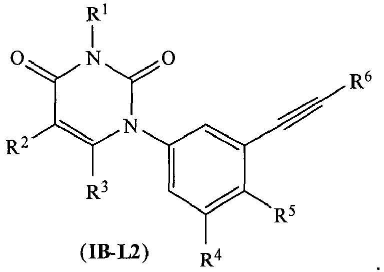

In some embodiments, **L** is C(O)N(**R^{C}**), wherein **R^{C}** is as discussed below. In these embodiments, the compounds of formula **I** correspond in structure to formula **I-L3:**

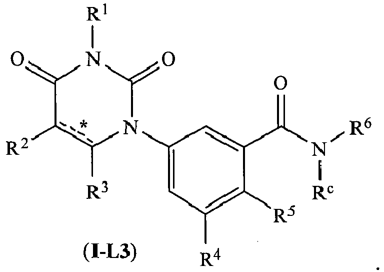

In some such embodiments, the compounds correspond in structure to formula **IA-L3:**

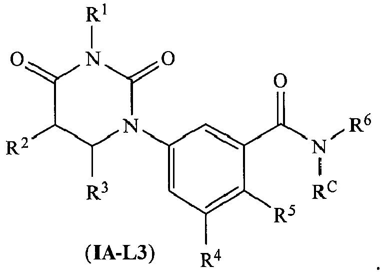

In other such embodiments, the compounds correspond in structure to formula **IB-L3:**

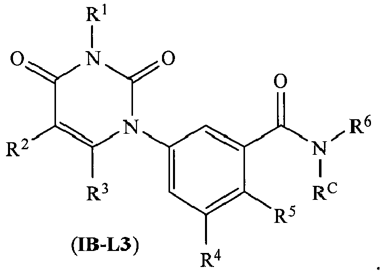

In some embodiments, **L** is N(**R^{D}**)C(O), wherein **R^{D}** is as discussed below. In these embodiments, the compounds of formula **I** correspond in structure to formula **I-L4:**

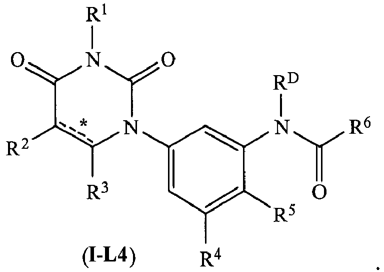

In some such embodiments, the compounds correspond in structure to formula **IA-L4:**

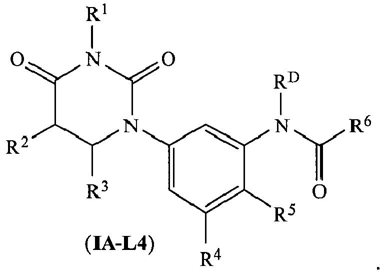

In other such embodiments, the compounds correspond in structure to formula **IB-L4:**

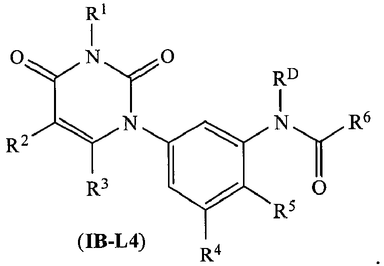

In some embodiments, **L** is C₁-C₂-alkylene. In these embodiments, the compounds of formula **I** correspond in structure to formula **I-L5-1** (if **L** is methylene) or **I-L5-2** (if L is ethylene):

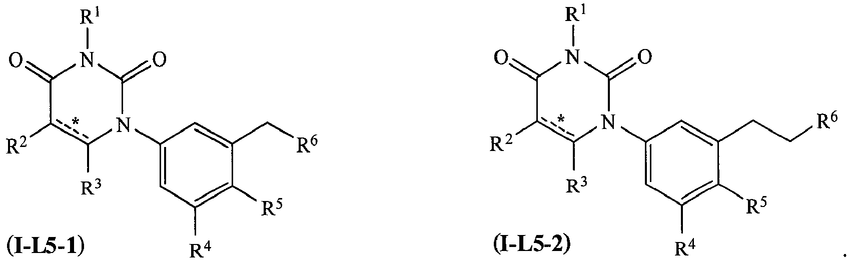

In some such embodiments, compounds correspond in structure to formula **IA-L5-1** (if **L** is methylene) or **IA-L5-2** (if **L** is ethylene):

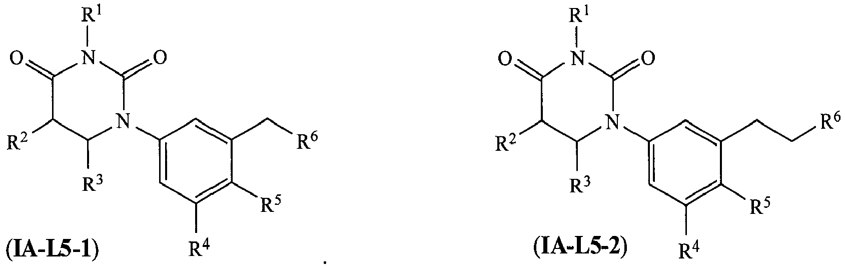

In other such embodiments, the compounds correspond in structure to formula **IB-L5-1** (if **L** is methylene) or **IB-L5-2** (if **L** is ethylene):

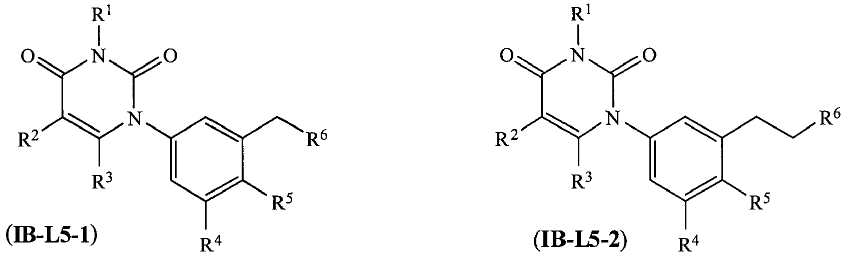

In some embodiments, **L** is C(H)₂O. In these embodiments, the compounds of formula **I** correspond in structure to formula **I-L6:**

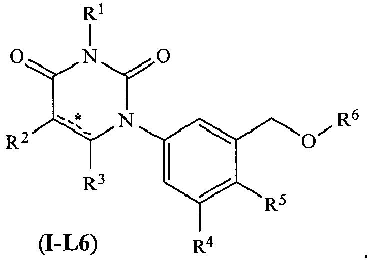

In some such embodiments, the compounds correspond in structure to formula **IA-L6:**

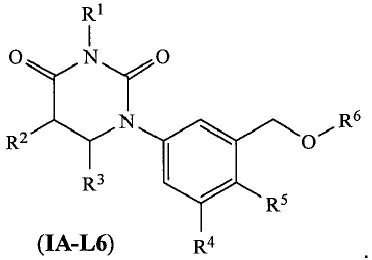

In other such embodiments, the compounds correspond in structure to formula **IB-L6:**

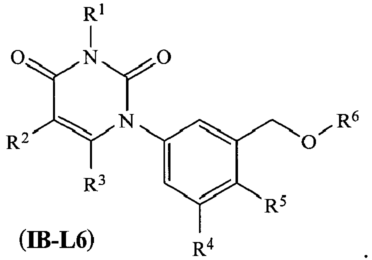

In some embodiments, **L** is OC(H)₂. In these embodiments, the compounds of formula **I** correspond in structure to formula **I-L7:**

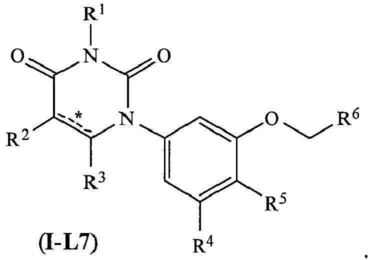

In some such embodiments, the compounds correspond in structure to formula **IA-L7:**

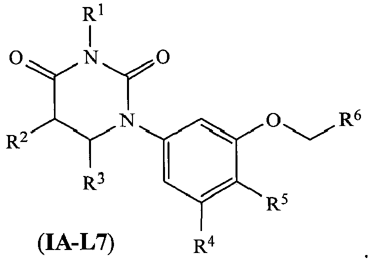

In other such embodiments, the compounds correspond in structure to formula **IB-L7:**

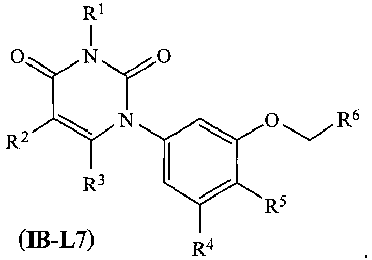

In some embodiments, **L** is cyclopropyl-1,2-ene. In these embodiments, the compounds of formula **I** correspond in structure to formula **I-L8:**

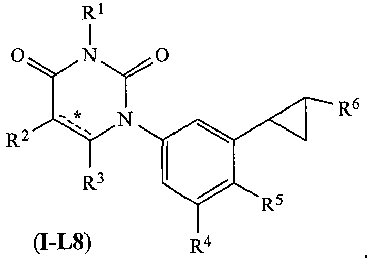

In some such embodiments, the compounds correspond in structure to formula **IA-L8:**

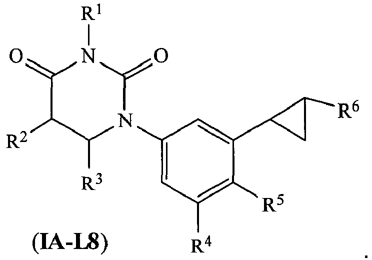

In other such embodiments, the compounds correspond in structure to formula **IB-L8:**

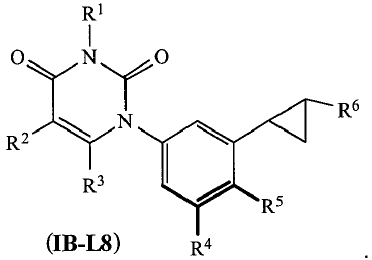

In some embodiments, L is selected from the group consisting of C=C, ethylene, and cyclopropyl-1,2-ene.

In some embodiments, **L** is C(H)₂N(**R^{L}**). In these embodiments, the compounds of formula **I** correspond in structure to formula **I-L9:**

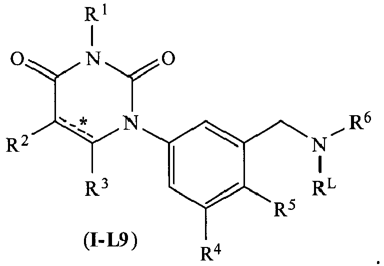

In some such embodiments, the compounds correspond in structure to formula **IA-L9:**

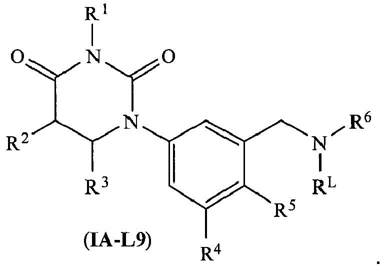

In other such embodiments, the compounds correspond in structure to formula **IB-L9:**

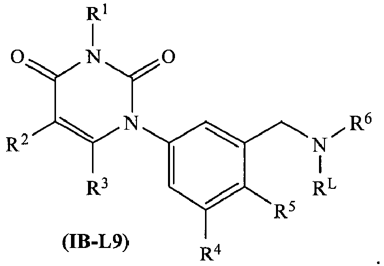

In some embodiments, **L** is N(**R^{M}**)C(H)₂. In these embodiments, the compounds of formula **I** correspond in structure to formula **I-L10:**

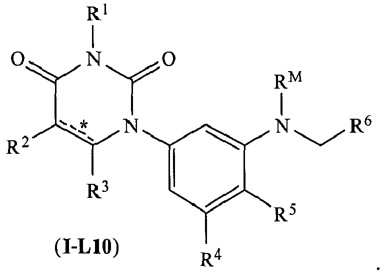

In some such embodiments, the compounds correspond in structure to formula **IA-L10:**

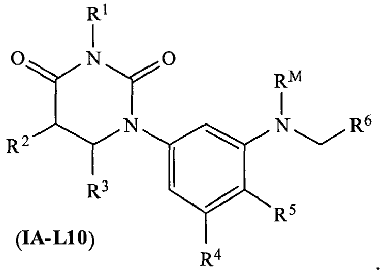

In other such embodiments, the compounds correspond in structure to formula **IB-L10:**

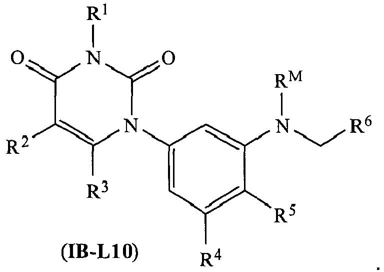

In some embodiments, **L** is C(O)C(H)₂. In these embodiments, the compounds of formula **I** correspond in structure to formula **I-L11:**

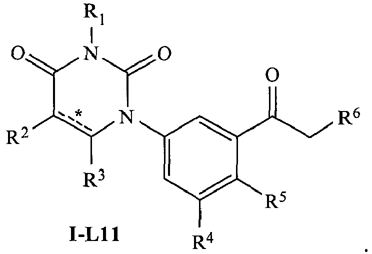

In some such embodiments, the compounds correspond in structure to formula **IA-L11:**

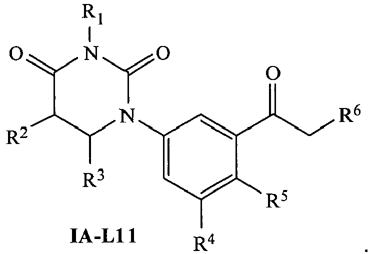

In other such embodiments, the compounds correspond in structure to formula **IB-L11:**

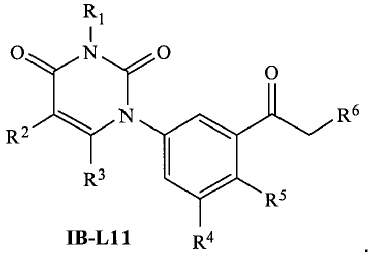

In some embodiments, **L** is C(H)₂C(O). In these embodiments, the compounds of formula **I** correspond in structure to formula **I-L12:**

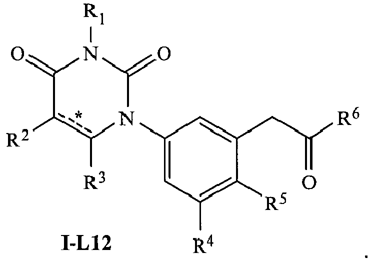

In some such embodiments, the compounds correspond in structure to formula **IA-L12:**

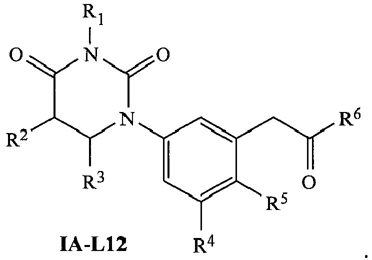

In other such embodiments, the compounds correspond in structure to formula **IB-L12:**

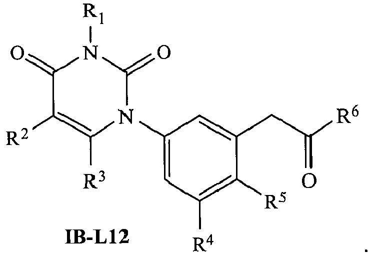

### B7. Substituents R^{A} and R^{B}.

**R^{A}** and **R^{B}** are independently selected from the group consisting of hydrogen, C₁-C₆-alkyl, C₁-C₆-alkyloxy, C₃-C₈-cycloalkyl, and halo, wherein:
the C₁-C₆-alkyl optionally is substituted with one or more substituents independently selected from the group consisting of carboxy, halo, hydroxy, nitro, oxo, amino, cyano, alkyloxycarbonyl, alkylcarbonyloxy, alkyloxy, carbocyclyl, and heterocyclyl.

In some embodiments, one of **R^{A}** and **R^{B}** is hydrogen, and the other is selected from the group consisting of C₁-C₆-alkyl, C₁-C₆-alkyloxy, C₃-C₈-cycloalkyl, and halo, wherein:
the C₁-C₆-alkyl optionally is substituted with one or more substituents independently selected from the group consisting of carboxy, halo, hydroxy, nitro, oxo, amino, cyano, alkyloxycarbonyl, alkylcarbonyloxy, alkyloxy, carbocyclyl, and heterocyclyl.

In some embodiments, **R^{A}** and **R^{B}** are independently selected from the group consisting of hydrogen, C₁-C₆-alkyl, C₁-C₆-alkyloxy, C₃-C₈-cycloalkyl, and halo.

In some of the above embodiments, **R^{A}** is hydrogen. In other of the above embodiments, **R^{B}** is hydrogen.

In some embodiment, one of **R^{A}** and **R^{B}** is hydrogen, and the other is selected from the group consisting of hydrogen, methyl, methoxy, and halo.

In some embodiments, **R^{A}** is hydrogen, and **R^{B}** is selected from the group consisting of methyl, methoxy, and halo. In some such embodiments, **R^{B}** is selected from the group consisting of methyl, methoxy, and fluoro. In other such embodiments, **R^{B}** is selected from the group consisting of methyl, methoxy, and chloro. In yet other such embodiments, **R^{B}** is selected from the group consisting of methyl, methoxy, and bromo. In further such embodiments, **R^{B}** is selected from the group consisting of methyl, methoxy, and iodo. In yet further such embodiments, **R^{B}** is selected from the group consisting of methyl, methoxy, chloro, and fluoro.

In some embodiments, **R^{B}** is hydrogen, and **R^{A}** is selected from the group consisting of methyl, methoxy, and halo. In some such embodiments, **R^{A}** is selected from the group consisting of methyl, methoxy, and fluoro. In other such embodiments, **R^{A}** is selected from the group consisting of methyl, methoxy, and chloro. In yet other such embodiments, **R^{A}** is selected from the group consisting of methyl, methoxy, and bromo. In further such embodiments, **R^{A}** is selected from the group consisting of methyl, methoxy, and iodo. In yet further such embodiments, **R^{A}** is selected from the group consisting of methyl, methoxy, chloro, and fluoro.

In some embodiments, **R^{A}** is hydrogen, and **R^{B}** is hydrogen.

### B8. Substituent R^{C}.

**R^{C}** is selected from the group consisting of hydrogen and alkyl. In some such embodiments, **R^{C}** is selected from the group consisting of hydrogen and methyl.

In some embodiments, **R^{C}** is hydrogen.

In some embodiments, **R^{C}** is alkyl. In some such embodiments, **R^{C}** is methyl.

### B9. Substituent R^{D}.

**R^{D}** is selected from the group consisting of hydrogen and alkyl. In some such embodiments, **1R^{D}** is selected from the group consisting of hydrogen and methyl.

In some embodiments, **R^{D}** is hydrogen.

In some embodiments, **R^{D}** is alkyl. In some such embodiments, **R^{D}** is methyl.

### B10. Substituent R^{L}.

**R^{L}** is selected from the group consisting of hydrogen, C₁-C₆-alkyl, C₁-C₆-alkyloxy, C₃-C₈-cycloalkyl, and halo, wherein:
the C₁-C₆-alkyl optionally is substituted with one or more substituents independently selected from the group consisting of carboxy, halo, hydroxy, nitro, oxo, amino, cyano, alkyloxycarbonyl, alkylcarbonyloxy, alkyloxy, carbocyclyl, and heterocyclyl.

In some embodiments, **R^{L}** is selected from the group consisting of hydrogen, C₁-C₆-alkyl, C₁-C₆-alkyloxy, C₃-C₈-cycloalkyl, and halo.

In some embodiments, **R^{L}** is selected from the group consisting of hydrogen, C₁-C₆-alkyl, C₁-C₆-alkyloxy, and halo, wherein:
the C₁-C₆-alkyl optionally is substituted with one or more substituents independently selected from the group consisting of carboxy, halo, hydroxy, nitro, oxo, amino, cyano, alkyloxycarbonyl, alkylcarbonyloxy, alkyloxy, carbocyclyl, and heterocyclyl.

In some embodiments, **R^{L}** is selected from the group consisting of hydrogen, C₁-C₆-alkyl, C₁-C₆-alkyloxy, and halo.

In some of the above embodiments, **R^{L}** is halo. In some such embodiments, the halo is fluoro. In other such embodiments, the halo is chloro. In yet other such embodiments, the halo is bromo. In further such embodiments, the halo is iodo.

In some of the above embodiments, **R^{L}** is hydrogen.

In some of the above embodiments, **R^{L}** is C₁-C₆-alkyl.

In some of the above embodiments, **R^{L}** is C₁-C₆-alkyloxy.

### B11. Substituent R^{M}.

**R^{M}** is selected from the group consisting of hydrogen, C₁-C₆-alkyl, C₁-C₆-alkyloxy, C₃-C₈-cycloalkyl, and halo, wherein:
the C₁-C₆-alkyl optionally is substituted with one or more substituents independently selected from the group consisting of carboxy, halo, hydroxy, nitro, oxo, amino, cyano, alkyloxycarbonyl, alkylcarbonyloxy, alkyloxy, carbocyclyl, and heterocyclyl.

In some embodiments, **R^{M}** is selected from the group consisting of hydrogen, C₁-C₆-alkyl, C₁-C₆-alkyloxy, C₃-C₈-cycloalkyl, and halo.

In some embodiments, **R^{M}** is selected from the group consisting of hydrogen, C₁-C₆-alkyl, C₁-C₆-alkyloxy, and halo, wherein:
the C₁-C₆-alkyl optionally is substituted with one or more substituents independently selected from the group consisting of carboxy, halo, hydroxy, nitro, oxo, amino, cyano, alkyloxycarbonyl, alkylcarbonyloxy, alkyloxy, carbocyclyl, and heterocyclyl.

In some embodiments, **R^{M}** is selected from the group consisting of hydrogen, C₁-C₆-alkyl, C₁-C₆-alkyloxy, and halo.

In some of the above embodiments, **R^{M}** is halo. In some such embodiments, the halo is fluoro. In other such embodiments, the halo is chloro. In yet other such embodiments, the halo is bromo. In further such embodiments, the halo is iodo.

In some of the above embodiments, **R^{M}** is hydrogen.

In some of the above embodiments, **R^{M}** is C₁-C₆-alkyl.

In some of the above embodiments, **R^{M}** is C₁-C₆-alkyloxy.

### B12. Substituent R⁶.

**R⁶** is selected from the group consisting of C₅-C₆-carbocyclyl, 5-6-membered heterocyclyl, fused 2-ring carbocyclyl, and fused 2-ring heterocyclyl, wherein each such substituent optionally is substituted with one or more substituents independently selected from the group consisting of **R^{E}**, **R^{F}**, **R^{G}**, **R^{H}**, **R^{I}**, **R^{J}**, and **R^{K}**, wherein **R^{E}**, **R^{F}**, **R**^{G}, **R^{H}**, **R^{I}**, **R^{J}**, and **R^{K}** are as described below. In some such embodiments, the C₅-C₆-carbocyclyl, 5-6-membered heterocyclyl, fused 2-ring carbocyclyl, and fused 2-ring heterocyclyl are not substituted. In other such embodiments, the C₅-C₆-carbocyclyl, 5-6-membered heterocyclyl, fused 2-ring carbocyclyl, and fused 2-ring heterocyclyl are substituted with a substituent selected from the group consisting of **R^{E}**, **R^{F}**, **R^{G}**, **R^{H}**, **R^{I}**, **R^{J}**, and **R^{K}.** In other such embodiments, the C₅-C₆-carbocyclyl, 5-6-membered heterocyclyl, fused 2-ring carbocyclyl, and fused 2-ring heterocyclyl are substituted with a substituent selected from the group consisting of **R^{E}**, **R^{F}**, **R^{I}**, **R^{J}**, and **R^{K}**. In other such embodiments, the C₅-C₆-carbocyclyl, 5-6-membered heterocyclyl, fused 2-ring carbocyclyl, and fused 2-ring heterocyclyl are substituted with a substituent selected from the group consisting of **R^{E}**, **R^{F}**, and **R^{J}.** In other such embodiments, the C₅-C₆-carbocyclyl, 5-6-membered heterocyclyl, fused 2-ring carbocyclyl, and fused 2-ring heterocyclyl are substituted with a substituent selected from the group consisting of **R^{F}** and **R^{J}.** In other such embodiments, the C₅-C₆-carbocyclyl, 5-6-membered heterocyclyl, fused 2-ring carbocyclyl, and fused 2-ring heterocyclyl are substituted with **R^{J}.** In yet other such embodiments, the C₅-C₆-carbocyclyl, 5-6-membered heterocyclyl, fused 2-ring carbocyclyl, and fused 2-ring heterocyclyl are substituted with two substituents independently selected from the group consisting of **R^{E}**, **R^{F}**, **R^{G}**, **R^{H}**, **R^{I}**, **R^{J}**, and **R^{K}**. In yet other such embodiments, the C₅-C₆-carbocyclyl, 5-6-membered heterocyclyl, fused 2-ring carbocyclyl, and fused 2-ring heterocyclyl are substituted with two substituents independently selected from the group consisting of **R^{E}**, **R^{F}**, **R^{I}**, **R^{J}**, and **R^{K}**. In yet other such embodiments, the C₅-C₆-carbocyclyl, 5-6-membered heterocyclyl, fused 2-ring carbocyclyl, and fused 2-ring heterocyclyl are substituted with two substituents independently selected from the group consisting of **R^{E}**, **R^{F}**, and **R^{J}**. In yet other such embodiments, the C₅-C₆-carbocyclyl, 5-6-membered heterocyclyl, fused 2-ring carbocyclyl, and fused 2-ring heterocyclyl are substituted with two substituents independently selected from the group consisting of **R^{F}** and **R^{J}.** In further such embodiments, the C₅-C₆-carbocyclyl, 5-6-membered heterocyclyl, fused 2-ring carbocyclyl, and fused 2-ring heterocyclyl are substituted with three substituents independently selected from the group consisting of **R^{E}**, **R^{F}**, **R^{G}**, **R^{H}**, **R^{I}**, **R^{J}**, and **R^{K}.** In further such embodiments, the C₅-C₆-carbocyclyl, 5-6-membered heterocyclyl, fused 2-ring carbocyclyl, and fused 2-ring heterocyclyl are substituted with three substituents independently selected from the group consisting of **R^{E}**, **R^{F}**, **R^{I}**, **R^{J}**, and **R^{K}.** In further such embodiments, the C₅-C₆-carbocyclyl, 5-6-membered heterocyclyl, fused 2-ring carbocyclyl, and fused 2-ring heterocyclyl are substituted with three substituents independently selected from the group consisting of **R^{E}**, **R^{F}**, and **R^{J}.** In further such embodiments, the C₅-C₆-carbocyclyl, 5-6-membered heterocyclyl, fused 2-ring carbocyclyl, and fused 2-ring heterocyclyl are substituted with three substituents independently selected from the group consisting of **R^{F}** and **R^{J}**. In further such embodiments, the C₅-C₆-carbocyclyl, 5-6-membered heterocyclyl, fused 2-ring carbocyclyl, and fused 2-ring heterocyclyl are substituted with one, two, or three substituents independently selected from the group consisting of **R^{E}**, **R^{F}**, **R^{G}**, **R^{H}**, **R^{I}**, **R^{J}**, and **R^{K}.** In further such embodiments, the C₅-C₆-carbocyclyl, 5-6-membered heterocyclyl, fused 2-ring carbocyclyl, and fused 2-ring heterocyclyl are substituted with one, two, or three substituents independently selected from the group consisting of **R^{E}**, **R^{F}**, **R^{I}**, **R^{J}**, and **R^{K}.** In further such embodiments, the C₅-C₆-carbocyclyl, 5-6-membered heterocyclyl, fused 2-ring carbocyclyl, and fused 2-ring heterocyclyl are substituted with one, two, or three substituents independently selected from the group consisting of **R^{E}**, **R^{F}**, and **R^{J}**. In further such embodiments, the C₅-C₆-carbocyclyl, 5-6-membered heterocyclyl, fused 2-ring carbocyclyl, and fused 2-ring heterocyclyl are substituted with one, two, or three substituents independently selected from the group consisting of **R^{F}** and **R^{J}.**

In some embodiments, **R⁶** is selected from the group consisting of C₅-C₆-carbocyclyl and 5-6-membered heterocyclyl, wherein each such substituent optionally is substituted with one or more substituents independently selected from the group consisting of **R^{E}**, **R^{F}**, **R^{G}**, **R^{H}**, **R^{I}**, **R^{J}**, and **R^{K}**. In some such embodiments, the C₅-C₆-carbocyclyl and 5-6-membered heterocyclyl are not substituted. In other such embodiments, the C₅-C₆-carbocyclyl and 5-6-membered heterocyclyl are substituted with a substituent selected from the group consisting of **R^{E}**, **R^{F}**, **R^{G}**, **R^{H}**, **R^{I}**, **R^{J}**, and **R^{K}**. In yet other such embodiments, the C₅-C₆-carbocyclyl and 5-6-membered heterocyclyl are substituted with two substituents independently selected from the group consisting of **R^{E}**, **R^{F}**, **R^{G}**, **R^{H}**, **R^{I}**, **R^{J}**, **and R^{K}.** In further such embodiments, the C₅-C₆-carbocyclyl and 5-6-membered heterocyclyl are substituted with three substituents independently selected from the group consisting of **R^{E}**, **R^{F}**, **R^{G}**, **R^{H}**, **R^{I}**, **R^{J}**, and **R^{K}.** In further such embodiments, the C₅-C₆-carbocyclyl and 5-6-membered heterocyclyl are substituted with one, two, or three substituents independently selected from the group consisting of **R^{E}**, **R^{F}**, **R^{G}**, **R^{H}**, **R^{I}**, **R^{J}**, and **R^{K}**.

In some embodiments, **R⁶** is C₅-C₆-carbocyclyl optionally substituted with one or more substituents independently selected from the group consisting of **R^{E}**, **R^{F}**, **R^{G}**, **R^{H}**, **R^{I}**, **R^{J}**, and **R^{K}**. In some such embodiments, the C₅-C₆-carbocyclyl is not substituted. In other such embodiments, the C₅-C₆-carbocyclyl is substituted with a substituent selected from the group consisting of **R^{E}, R^{F}, R^{G}, R^{H}, R^{I}, R^{J}**, and **R^{K}.** In yet other such embodiments, the C₅-C₆-carbocyclyl is substituted with two substituents independently selected from the group consisting of **R^{E}**, **k^{F}**, **R^{G}**, **R^{H}**, **R^{I}**, **R^{J}**, and **R^{K}.** In further such embodiments, the C₅-C₆-carbocyclyl is substituted with three substituents independently selected from the group consisting of **R^{E}**, **R^{F}**, **R^{G}**, **R^{H}**, **R^{I}**, **R^{J}**, and **R^{K}.** In further such embodiments, the C₅-C₆-carbocyclyl is substituted with one, two, or three substituents independently selected from the group consisting of **R^{E}**, **R^{F}**, **^{R}G**, **R^{H}**, **R^{I}**, **R^{J}**, and **R^{K}.**

In some embodiments, **R⁶** is 5-6-membered heterocyclyl optionally substituted with one or more substituents independently selected from the group consisting of **R^{E}**, **R^{F}**, **R^{G}**, **R^{H}**, **R^{I}**, **R^{J}**, and **R^{K}**. In some such embodiments, the 5-6-membered heterocyclyl is not substituted. In other such embodiments, the 5-6-membered heterocyclyl is substituted with a substituent selected from the group consisting of **R^{E}**, **R^{F}**, **R^{G}**, **R^{H}**, **R^{I}**, **R^{J}**, and **R^{K}.** In yet other such embodiments, the 5-6-membered heterocyclyl is substituted with two substituents independently selected from the group consisting of **R^{E}**, **R^{F}**, **R^{G}**, **R^{H}**, **R^{I}**, **R^{J}**, and **R^{K}.** In further such embodiments, the 5-6-membered heterocyclyl is substituted with three substituents independently selected from the group consisting of **R^{E}**, **R^{F}**, **R^{G}**, **R^{H}**, **R¹**, **R^{J}**, and **R^{K}.** In further such embodiments, the 5-6-membered heterocyclyl is substituted with one, two, or three substituents independently selected from the group consisting of **R^{E}**, **R^{F}**, **R^{G}**, **R^{H}**, **R^{X}**, **R^{J}**, and **R^{K}.**

In some embodiments, **R⁶** is selected from the group consisting of fused 2-ring carbocyclyl and fused 2-ring heterocyclyl, wherein each such substituent optionally is substituted with one or more substituents independently selected from the group consisting of **R^{E}**, **R^{F}**, **R^{G}**, **R^{H}**, **R^{I}**, **R^{J}**, and **R^{K}.** In some such embodiments, the fused 2-ring carbocyclyl and fused 2-ring heterocyclyl are not substituted. In other such embodiments, the fused 2-ring carbocyclyl and fused 2-ring heterocyclyl are substituted with a substituent selected from the group consisting of **R^{E}**, **R^{F}**, **R^{G}**, **R^{H}**, **R^{I}**, **R^{J}**, and **R^{K}.** In yet other such embodiments, the fused 2-ring carbocyclyl and fused 2-ring heterocyclyl are substituted with two substituents independently selected from the group consisting of **R^{E}**, **R^{F}**, **R^{G}**, **R^{H}**, **R^{I}**, **R^{J}**, and **R^{K}**. In further such embodiments, the fused 2-ring carbocyclyl and fused 2-ring heterocyclyl are substituted with three substituents independently selected from the group consisting of **R^{E}**, **R^{F}**, **R^{G}**, **R^{H}**, **R^{I}**, **R^{J}**, and **R^{K}**. In further such embodiments, the fused 2-ring carbocyclyl and fused 2-ring heterocyclyl are substituted with one, two, or three substituents independently selected from the group consisting of **R^{E}**, **R^{F}**, **R^{G}**, **R^{H}**, **R^{I}**, **R^{J}**, and **R^{K}**.

In some embodiments, **R⁶** is fused 2-ring carbocyclyl optionally substituted with one or more substituents independently selected from the group consisting of **R^{E}**, **R^{F}**, **R^{G}**, **R^{H}**, **R^{I}**, **R^{J}**, and **R^{K}**. In some such embodiments, the fused 2-ring carbocyclyl is not substituted. In other such embodiments, the fused 2-ring carbocyclyl is substituted with a substituent selected from the group consisting of **R^{E}**, **R^{F}**, **R^{G}**, **R^{H}**, **R^{I}**, **R^{J}**, and **R^{K}**. In yet other such embodiments, the fused 2-ring carbocyclyl is substituted with two substituents independently selected from the group consisting of **R^{E}**, **R^{F}**, **R^{G}**, **R^{H}**, **R^{I}**, **R^{J}**, and **R^{K}**. In further such embodiments, the fused 2-ring carbocyclyl is substituted with three substituents independently selected from the group consisting of **R^{E}**, **R^{F}**, **R^{G}**, **R^{H}**, **R^{I}**, **R^{J}**, and **R^{K}**. In further such embodiments, the fused 2-ring carbocyclyl is substituted with one, two, or three substituents independently selected from the group consisting of **R^{E}**, **R^{F}**, **R^{G}**, **R^{H}**, **R^{I}**, **R^{J}**, and **R^{K}**.

In some embodiments, **R⁶** is fused 2-ring heterocyclyl optionally substituted with one or more substituents independently selected from the group consisting of **R^{E}**, **R^{F}**, **R^{G}**, **R^{H}**, **R^{I}**, **R^{J}**, and **R^{K}**. In some such embodiments, the fused 2-ring heterocyclyl is not substituted. In other such embodiments, the fused 2-ring heterocyclyl is substituted with a substituent selected from the group consisting of **R^{E}**, **R^{F}**, **R^{G}**, **R^{H}**, **R^{I}**, **R^{J}**, and **R^{K}.** In yet other such embodiments, the fused 2-ring heterocyclyl is substituted with two substituents independently selected from the group consisting of **R^{E}**, **R^{F}**, **R^{G}**, **R^{H}**, **R^{I}**, **R^{J}**, and **R^{K}.** In further such embodiments, the fused 2-ring heterocyclyl is substituted with three substituents independently selected from the group consisting of **R^{E}**, **R^{F}**, **R^{G}**, **R^{H}**, **R¹**, **R^{J}**, and **R^{K}.** In further such embodiments, the fused 2-ring heterocyclyl is substituted with one, two, or three substituents independently selected from the group consisting of **R^{E}**, **R^{F}**, **R^{G}**, **R^{H}**, **R^{I}**, **R^{J}**, and **R^{K}**.

In some of the above embodiments, the optionally substituted C₅-C₆-carbocyclyl is selected from the group consisting of cyclopentyl, cyclopentenyl, cyclopentadienyl, cyclohexyl, cyclohexenyl, cyclohexadienyl, and phenyl. In some such embodiments, the optionally substituted C₅-C₆-carbocyclyl is phenyl.

In some of the above embodiments, the optionally substituted C₅-C₆-carbocyclyl is C₅-carbocyclyl. Examples of C₅-carbocyclyls include cyclopentyl, cyclopentenyl, and cyclopentadienyl.

In other of the above embodiments, the optionally substituted C₅-C₆-carbocyclyl is C₆-carbocyclyl. Examples of C₆-carbocyclyls include cyclohexyl, cyclohexenyl, cyclohexadienyl, and phenyl.

In some of the above embodiments, the optionally substituted 5-6-membered-heterocyclyl is selected from the group consisting of furanyl, dihydrofuranyl, tetrahydrofuranyl, thiophenyl (thiofuranyl), dihydrothiophenyl, tetrahydrothiophenyl, pyrrolyl, pyrrolinyl, pyrrolidinyl, oxazolyl, dihydrooxazolyl, isoxazolyl, dihydroisoxazolyl, oxazolidinyl, isoxazolidinyl, thiazolyl, isothiazolyl, thiazolinyl, isothiazolinyl, thiazolidinyl, isothiazolidinyl, imidazolyl, imidazolidinyl, pyrazolyl, pyrazolinyl, pyrazolidinyl, oxathiolyl, oxathiolanyl, triazolyl, oxadiazolyl, furazanyl, tetrazolyl, oxatriazolyl, dioxazolyl, oxathiazolyl, oxathiazolidinyl, dihydrooxadiazolyl, dioxazolidinyl, pyranyl, dihydropyranyl, tetrahydropyranyl, pyridinyl, dihydropyridinyl, tetrahydropyridinyl, piperidinyl, diazinyl, pyrazinyl, pyridazinyl, pyrimidinyl, dihydropyrazinyl, tetrahydropyrazinyl, piperazinyl, triazinyl, dihydrotriazinyl, tetrahydrotriazinyl, triazinanyl, oxazinyl, dihydrooxazinyl, morpholinyl, oxathiazinyl, dihydrooxathiazinyl, oxathiazinanyl, oxadiazinyl, dihydrooxadiazinyl, oxadiazinanyl, thiopyranyl, dihydrothiopyranyl, and tetrahydrothiopyranyl.

In some of the above embodiments, the optionally substituted 5-6-membered-heterocyclyl is 5-membered heterocyclyl. Examples of such 5-membered heterocyclyl include furanyl, dihydrofuranyl, tetrahydrofuranyl, thiophenyl (thiofuranyl), dihydrothiophenyl, tetrahydrothiophenyl, pyrrolyl, pyrrolinyl, pyrrolidinyl, oxazolyl, dihydrooxazolyl, isoxazolyl, dihydroisoxazolyl, oxazolidinyl, isoxazolidinyl, thiazolyl, isothiazolyl, thiazolinyl, isothiazolinyl, thiazolidinyl, isothiazolidinyl, imidazolyl, imidazolidinyl, pyrazolyl, pyrazolinyl, pyrazolidinyl, oxathiolyl, oxathiolanyl, triazolyl, oxadiazolyl, furazanyl, tetrazolyl, oxatriazolyl, dioxazolyl, oxathiazolyl, oxathiazolidinyl, dihydrooxadiazolyl, and dioxazolidinyl.

In other of the above embodiments, the optionally substituted 5-6-membered-heterocyclyl is 6-membered heterocyclyl. Examples of 6-membered heterocyclyls include pyranyl, dihydropyranyl, tetrahydropyranyl, pyridinyl, dihydropyridinyl, tetrahydropyridinyl, piperidinyl, diazinyl, pyrazinyl, pyridazinyl, pyrimidinyl, dihydropyrazinyl, tetrahydropyrazinyl, piperazinyl, triazinyl, dihydrotriazinyl, tetrahydrotriazinyl, triazinanyl, oxazinyl, dihydrooxazinyl, morpholinyl, oxathiazinyl, dihydrooxathiazinyl, oxathiazinanyl, oxadiazinyl, dihydrooxadiazinyl, oxadiazinanyl, thiopyranyl, dihydrothiopyranyl, and tetrahydrothiopyranyl.

In some of the above embodiments, the optionally substituted fused 2-ring carbocyclyl is selected from the group consisting of naphthalenyl, dihydronaphthalenyl, tetrahydronaphthalenyl, hexahydronaphthalenyl, octahydronaphthalenyl, decahydronaphthalenyl, indenyl, dihydroindenyl, hexahydroindenyl, octahydroindenyl, pentalenyl, octahydropentalenyl, and hexahydropentalenyl. In some such embodiments, the optionally substituted fused 2-ring carbocyclyl is selected from the group consisting of naphthalenyl and dihydroindenyl. In some such embodiments, the optionally substituted fused 2-ring carbocyclyl is naphthalenyl. In other such embodiments, the optionally substituted fused 2-ring carbocyclyl is dihydroindenyl. In further such embodiments, the optionally substituted fused 2-ring carbocyclyl is indenyl.

In some of the above embodiments, the optionally substituted fused 2-ring heterocyclyl is selected from the group consisting of 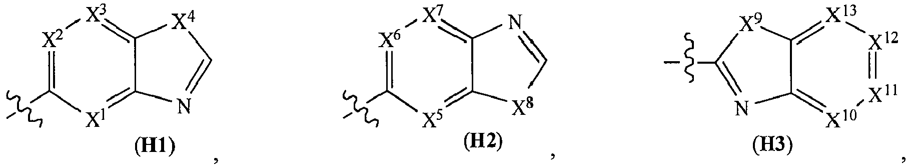 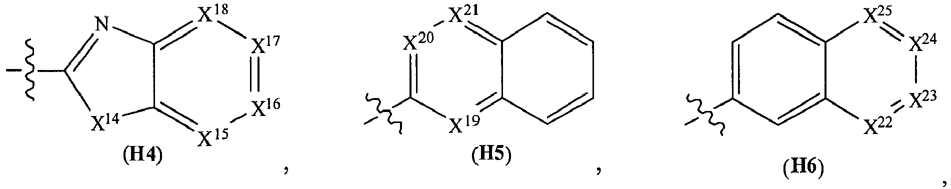 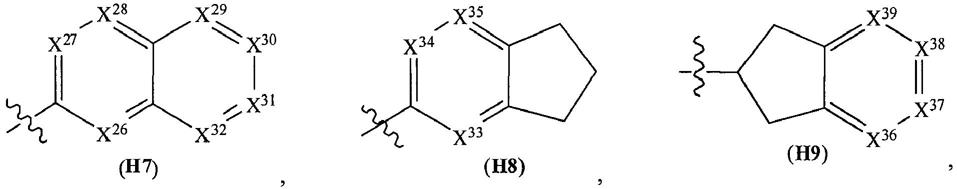 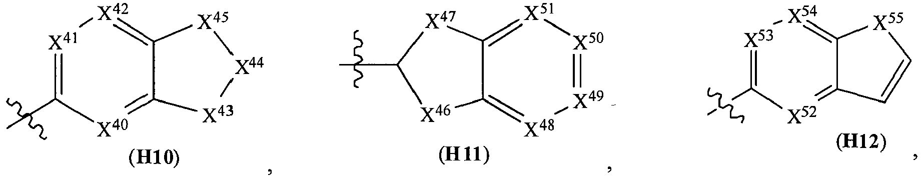 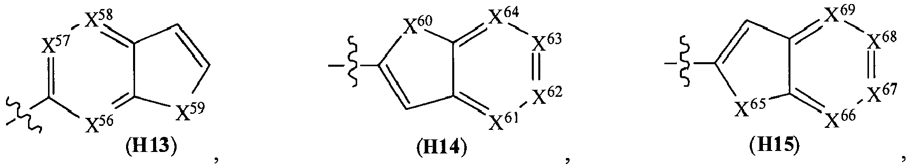 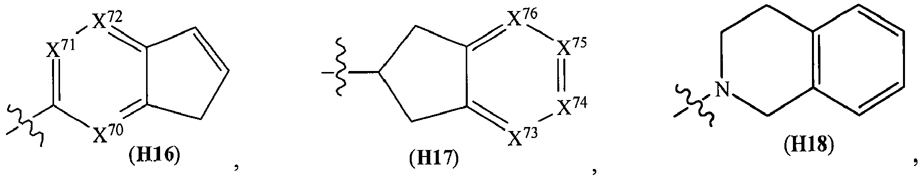 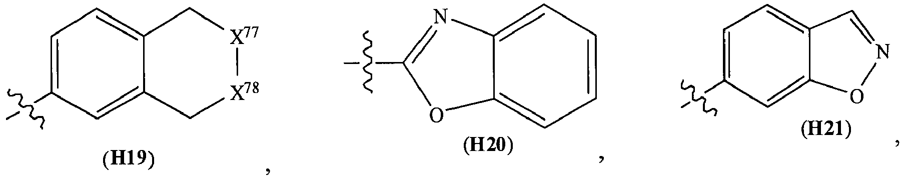 and 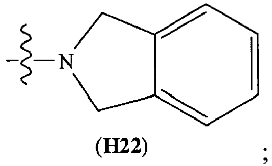
**X¹**, **X²**, and **X³** are independently selected from the group consisting of N and C(H);
**X⁴** is selected from the group consisting of N(H), O, and S;
**X⁵**, **X⁶**, and **X⁷** are independently selected from the group consisting of N and C(H);
**X⁸** is selected from the group consisting ofN(H), O, and S;
**X⁹** is selected from the group consisting of N(H), O, and S;
**X¹⁰**, **X¹¹**, **X¹²**, and **X¹³** are independently selected from the group consisting of N and C(H);
**X¹⁴** is selected from the group consisting ofN(H), O, and S;
**X¹⁵**, **X¹⁶**, **X¹⁷**, and **X¹⁸** are independently selected from the group consisting of N and C(H);
one or more of **X¹⁹**, **X²⁰**, and **X²¹** is N, and the remaining one(s) is/are C(H);
one or more of **X²²**, **X²³**, **X²⁴**, and **X²⁵** is N, and the remaining one(s) is/are C(H);
one or more of **X²⁶**, **X²⁷**, and **X²⁸** is N, and the remaining one(s) is/are C(H);
one or more of **X²⁹**, **X³⁰**, **X³¹**, and **X³²** is N, and the remaining one(s) is/are C(H);
one or more of **X³³**, **X³⁴**, and **X³⁵** is N, and the remaining one(s) is/are C(H);
one or more of **X³⁶**, **X³⁷**, **X³⁸**, and **X³⁹** is N, and the remaining one(s) is/are C(H);
**X⁴⁰**, **X⁴¹**, and **X⁴²** are independently selected from the group consisting of N and C(H);
one of **X⁴³**, **X⁴⁴**, and **X⁴⁵** is selected from the group consisting ofN(H), O, and S, and the remaining two are C(H)₂;
one of **X⁴⁶** and **X⁴⁷** is selected from the group consisting of N(H), O, and S, and the other one is C(H)₂;
**X⁴⁸**, **X⁴⁹**, **X⁵⁰**, and **X⁵¹** are independently selected from the group consisting of N and C(H);
**X⁵²**, **X⁵³**, and **X⁵⁴** are independently selected from the group consisting of N and C(H);
**X⁵⁵** is selected from the group consisting ofN(H), O, and S;
**X⁵⁶**, **X⁵⁷**, and **X⁵⁸** are independently selected from the group consisting of N and C(H);
**X⁵⁹** is selected from the group consisting ofN(H), O, and S;
**X⁶⁰** is selected from the group consisting ofN(H), O, and S;
**X⁶¹**, **X⁶²**, **X⁶³**, and **X⁶⁴** are independently selected from the group consisting of N and C(H);
**X⁶⁵** is selected from the group consisting ofN(H), O, and S;
**X⁶⁶**, **X⁶⁷**, **X⁶⁸**, and **X⁶⁹** are independently selected from the group consisting of N and C(H);
one or more of **X⁷⁰**, **X⁷¹**, and **X⁷²** is N, and the remaining one(s) is/are C(H);
one or more of **X⁷³**, **X⁷⁴**, **X⁷⁵**, and **X⁷⁶** is N, and the remaining one(s) is/are C(H); and
one of **X⁷⁷** and **X⁷⁸** is N(H), and the remaining one is C(H)₂.

In some of the above embodiments, the optionally substituted fused 2-ring heterocyclyl is selected from the group consisting of

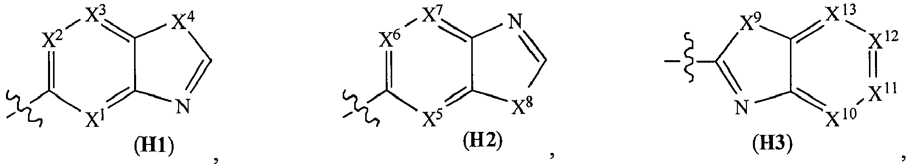

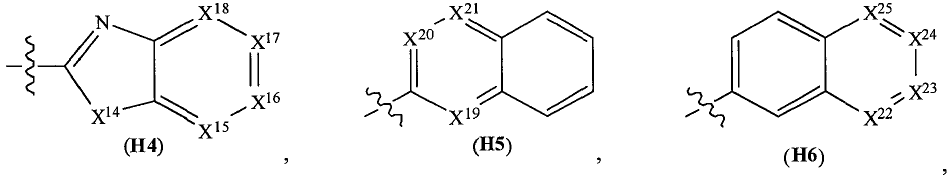

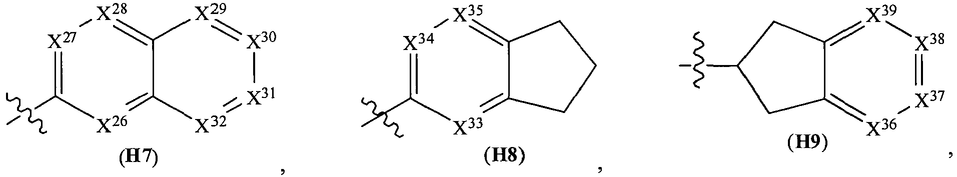

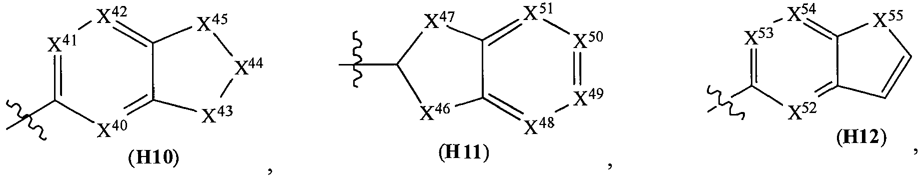

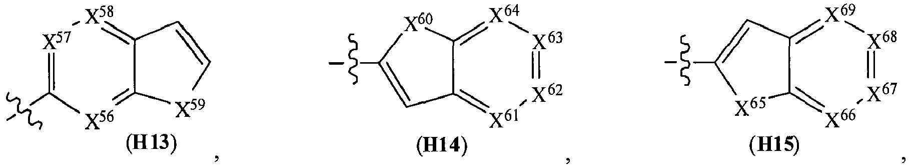

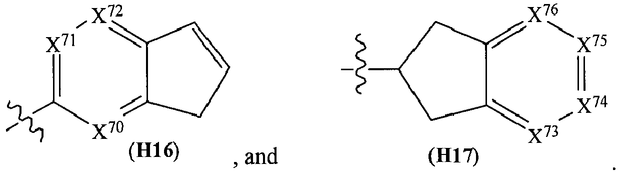

In some of the above embodiments, the optionally substituted fused 2-ring heterocyclyl is selected from the group consisting of:

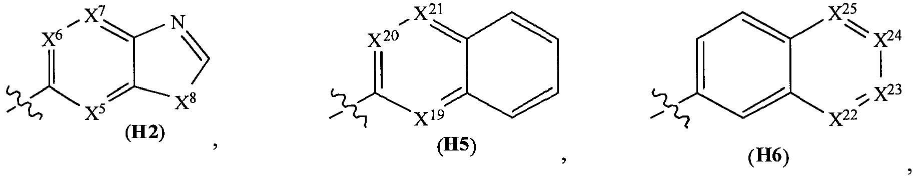

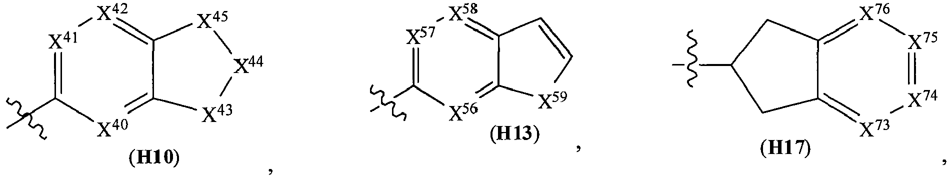

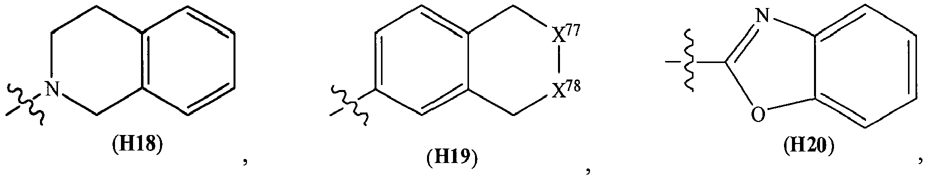

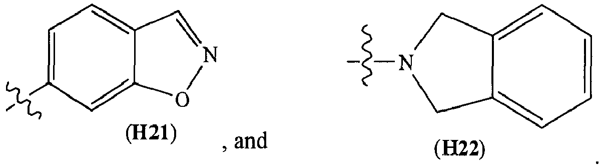

In some of the above embodiments, **X¹, X²,** and **X³** are C(H).

In some of the above embodiments, **X⁵, X⁶,** and **X⁷** are C(H).

In some of the above embodiments, **X¹⁰**, **X¹¹**, **X¹²**, and **X¹³** are C(H).

In some of the above embodiments, **X¹⁵**, **X¹⁶**, **X¹⁷**, and **X¹⁸** are C(H).

In some of the above embodiments, one of **X¹⁹**, **X²⁰**, and **X²¹** is N.

In some of the above embodiments, one of **X²²**, **X²³**, **X²⁴**, and **X²⁵** is N.

In some of the above embodiments, one of **X²⁶**, **X²⁷**, and **X²⁸** is N, and one of **X²⁹**, **X³⁰**, **X³¹**, and **X³²** is N.

In some of the above embodiments, **X⁴⁰**, **X⁴¹**, and **X⁴²** are C(H).

In some of the above embodiments, **X⁴⁸**, **X⁴⁹**, **X⁵⁰**, and **X⁵¹** are C(H).

In some of the above embodiments, **X⁵²**, **X⁵³**, and **X⁵⁴** are C(H).

In some of the above embodiments, **X⁵⁶**, **X⁵⁷**, and **X⁵⁸** are C(H).

In some of the above embodiments, **X⁶¹**, **X⁶²**, **X⁶³**, and **X⁶⁴** are C(H).

In some of the above embodiments, **X⁶⁶**, **X⁶⁷**, **X⁶⁸**, and **X⁶⁹** are C(H).

In some of the above embodiments, one or more of **X⁷⁰**, **X⁷¹**, and **X⁷²** is N, and the remaining one(s) is/are C(H).

In some of the above embodiments, one or more of **X⁷³**, **X⁷⁴**, **X⁷⁵**, and **X⁷⁶** is N, and the remaining one(s) is/are C(H).

### B13. Substituent R^{E}.

Each **R^{E}** is independently selected from the group consisting of halo, nitro, hydroxy, oxo, carboxy, cyano, amino, imino, azido, and aldehydo, wherein the amino optionally is substituted with one or two substituents independently selected from the group consisting of alkyl, alkenyl, and alkynyl.

In some embodiment, each **R^{E}** is independently selected from the group consisting of halo, nitro, hydroxy, oxo, carboxy, amino, imino, and aldehydo, wherein the amino optionally is substituted with one or two independently selected alkyl.

In some embodiment, each **R^{E}** is independently selected from the group consisting of halo, nitro, hydroxy, oxo, carboxy, amino, imino, aldehydo, and alkylamino.

In some embodiment, each **R^{E}** is independently selected from the group consisting of chloro, fluoro, nitro, hydroxy, oxo, carboxy, amino, imino, aldehydo, and alkylamino.

In some embodiment, each **R^{E}** is independently selected from the group consisting of halo, nitro, hydroxy, oxo, carboxy, cyano, amino, imino, and azido. In some such embodiments, each **R^{E}** is halo. In other such embodiments, each **R^{E}** is nitro. In yet other such embodiments, each **R^{E}** is hydroxy. In yet other such embodiments, each **R^{E}** is oxo. In yet other such embodiments, each **R^{E}** is carboxy. In yet other such embodiments, each **R^{E}** is cyano. In yet other such embodiments, each **R^{E}** is amino. In further such embodiments, each **R^{E}** is imino. In yet further such embodiments, each **R^{E}** is and azido.

In some embodiments, each **R^{E}** is independently selected from the group consisting of halo, nitro, hydroxy, oxo, carboxy, cyano, amino, and imino.

### B14. Substituent R^{F}.

Each **R^{F}** is independently selected from the group consisting of alkyl, alkenyl, and alkynyl, wherein:
each such substituent optionally is substituted with one or more substituents independently selected from the group consisting of carboxy, hydroxy, halo, amino, imino, nitro, azido, oxo, aminosulfonyl, alkylsulfonyl, alkyloxycarbonyl, alkenyloxycarbonyl, alkynyloxycarbonyl, alkylcarbonyloxy, alkenylcarbonyloxy, alkynylcarbonyloxy, alkyloxy, alkenyloxy, alkynyloxy, carbocyclyl, heterocyclyl, cyano, and aminocarbonyl, wherein:
   the amino, imino, aminosulfonyl, aminocarbonyl, carbocyclyl, and heterocyclyl optionally are substituted with one or two substituents independently selected from the group consisting of alkyl, alkenyl, alkynyl, alkylsulfonyl, alkenylsulfonyl, alkynylsulfonyl, alkylsulfonylamino, hydroxy, and alkyloxy,
wherein:
   amino portion of the alkylsulfonylamino optionally is substituted with a substituent selected from the group consisting of alkyl, alkenyl, and alkynyl.

In some embodiment, each **R^{F}** is independently selected from the group consisting of alkyl, alkenyl, and alkynyl, wherein:
each such substituent optionally is substituted with one or more substituents independently selected from the group consisting of carboxy, hydroxy, halo, amino, imino, nitro, azido, oxo, aminosulfonyl, alkylsulfonyl, alkyloxycarbonyl, alkenyloxycarbonyl, alkynyloxycarbonyl, alkylcarbonyloxy, alkenylcarbonyloxy, alkynylcarbonyloxy, alkyloxy, alkenyloxy, alkynyloxy, carbocyclyl, heterocyclyl, cyano, and aminocarbonyl, wherein:
   the amino, imino, aminosulfonyl, and aminocarbonyl optionally are substituted with one or two substituents independently selected from the group consisting of alkyl, alkenyl, alkynyl, alkylsulfonyl, alkenylsulfonyl, alkynylsulfonyl, and alkylsulfonylamino, wherein:
      amino portion of the alkylsulfonylamino optionally is substituted with a substituent selected from the group consisting of alkyl, alkenyl, and alkynyl.

In some of the above embodiments, each **R^{F}** is independently selected from the group consisting of the alkyl, alkynyl, and alkynyl, wherein such substituents are not substituted.

In some embodiments, each **R^{F}** is independently selected from the group consisting of alkyl, alkenyl, and alkynyl, wherein:
each such substituent optionally is substituted with one or two substituents independently selected from the group consisting of carboxy, hydroxy, halo, amino, imino, nitro, oxo, aminosulfonyl, alkylsulfonyl, alkyloxycarbonyl, alkylcarbonyloxy, alkyloxy, carbocyclyl, heterocyclyl, cyano, and aminocarbonyl, wherein:
   the amino, imino, aminosulfonyl, and aminocarbonyl optionally are substituted with one or two substituents independently selected from the group consisting of alkyl, alkylsulfonyl, and alkylsulfonylamino,
wherein:
   amino portion of the alkylsulfonylamino optionally is substituted with alkyl.

In some embodiments, each **R^{F}** is an independently selected alkyl optionally substituted with a substituent selected from the group consisting of carboxy, hydroxy, halo, amino, imino, nitro, oxo, aminosulfonyl, alkylsulfonyl, alkyloxycarbonyl, alkylcarbonyloxy, alkyloxy, carbocyclyl, heterocyclyl, cyano, and aminocarbonyl, wherein:
the amino, imino, aminosulfonyl, and aminocarbonyl optionally are substituted with one or two substituents independently selected from the group consisting of alkyl, alkylsulfonyl, and alkylsulfonylamino, wherein:
   amino portion of the alkylsulfonylamino optionally is substituted with alkyl.

In some embodiments, each **R^{F}** is an independently selected alkyl optionally substituted with a substituent selected from the group consisting of carboxy, halo, amino, imino, and aminosulfonyl, wherein:
the amino, imino, and aminosulfonyl optionally are substituted with one or two substituents independently selected from the group consisting of alkyl, alkylsulfonyl, and alkylsulfonylamino.

In some embodiments, each **R^{F}** is an independently selected alkyl optionally substituted with amino, wherein the amino optionally is substituted with alkylsulfonyl.

In some embodiments, each **R^{F}** is an independently selected alkyl substituted with amino, wherein the amino is substituted with alkylsulfonyl. In some such embodiments, each **R^{F}** is methylsulfonylaminomethyl.

In some embodiments, each **R^{F}** is independently selected from the group consisting of alkyl, alkenyl, and alkynyl, wherein:
each such substituent optionally is substituted with one, two, or three substituents independently selected from the group consisting of carboxy, hydroxy, halo, amino, imino, nitro, azido, oxo, aminosulfonyl, alkylsulfonyl, alkyloxycarbonyl, alkenyloxycarbonyl, alkynyloxycarbonyl, alkylcarbonyloxy, alkenylcarbonyloxy, alkynylcarbonyloxy, alkyloxy, alkenyloxy, alkynyloxy, carbocyclyl, heterocyclyl, cyano, and aminocarbonyl.

In some embodiments, each **R^{F}** is independently selected alkyl substituted with one or more substituents independently selected from the group consisting of carboxy, hydroxy, halo, amino, imino, nitro, azido, oxo, aminosulfonyl, alkylsulfonyl, alkyloxycarbonyl, alkenyloxycarbonyl, alkynyloxycarbonyl, alkylcarbonyloxy, alkenylcarbonyloxy, alkynylcarbonyloxy, alkyloxy, alkenyloxy, alkynyloxy, carbocyclyl, heterocyclyl, cyano, and aminocarbonyl.

### B15. Substituent R^{G}.

Each **R^{G}** is independently selected from the group consisting of carbocyclyl and heterocyclyl, wherein:
each such substituent optionally is substituted with one or more substituents independently selected from the group consisting of alkyl, alkenyl, alkynyl, carboxy, hydroxy, halo, amino, nitro, azido, oxo, aminosulfonyl, alkyloxycarbonyl, alkenyloxycarbonyl, alkynyloxycarbonyl, alkylcarbonyloxy, alkenylcarbonyloxy, alkynylcarbonyloxy, alkyloxy, alkenyloxy, alkynyloxy, carbocyclyl, heterocyclyl, cyano, and aminocarbonyl, wherein:
   the amino, aminosulfonyl, and aminocarbonyl optionally are substituted with one or two substituents independently selected from the group consisting of alkyl, alkenyl, alkynyl, alkylsulfonyl, alkenylsulfonyl, and alkynylsulfonyl.

In some of the above embodiments, each **R^{G}** is independently selected from the group consisting of carbocyclyl and heterocyclyl, wherein such substituents are not substituted.

In some embodiments, each **R^{G}** is independently selected from the group consisting of carbocyclyl and heterocyclyl, wherein:
each such substituent optionally is substituted with one or two substituents independently selected from the group consisting of alkyl, carboxy, hydroxy, halo, amino, nitro, oxo, aminosulfonyl, alkyloxycarbonyl, alkylcarbonyloxy, alkyloxy, carbocyclyl, heterocyclyl, cyano, and aminocarbonyl, wherein:
   the amino, aminosulfonyl, and aminocarbonyl optionally are substituted with one or two substituents independently selected from the group consisting of alkyl and alkylsulfonyl.

In some of the above embodiments, the carbocyclyl is C₃-C₆-carbocyclyl.

In some of the above embodiments, the heterocyclyl is 5-6-membered heterocyclyl.

### B16. Substituent R^{H}.

Each **R^{H}** is independently selected from the group consisting of alkyloxy, alkenyloxy, alkynyloxy, alkylsulfonyloxy, alkenylsulfonyloxy, and alkynylsulfonyloxy, wherein:
each such substituent optionally is substituted with one or more substituents independently selected from the group consisting of carboxy, hydroxy, halo, amino, nitro, azido, oxo, aminosulfonyl, alkyloxycarbonyl, alkenyloxycarbonyl, alkynyloxycarbonyl, alkylcarbonyloxy, alkenylcarbonyloxy, alkynylcarbonyloxy, alkyloxy, alkenyloxy, alkynyloxy, carbocyclyl, heterocyclyl, cyano, and aminocarbonyl, wherein:
   the amino, aminosulfonyl, and aminocarbonyl optionally are substituted with one or two substituents independently selected from the group consisting of alkyl, alkenyl, alkynyl, alkylsulfonyl, alkenylsulfonyl, and alkynylsulfonyl.

In some of the above embodiments, each **R^{H}** is independently selected from the group consisting of alkyloxy, alkenyloxy, alkynyloxy, alkylsulfonyloxy, alkenylsulfonyloxy, and alkynylsulfonyloxy, wherein such substituents are not substituted.

In some embodiments, each **R^{H}** is independently selected from the group consisting of alkyloxy and alkylsulfonyloxy, wherein:
each such substituent optionally is substituted with one or two substituents independently selected from the group consisting of carboxy, hydroxy, halo, amino, nitro, oxo, aminosulfonyl, alkyloxycarbonyl, alkylcarbonyloxy, alkyloxy, carbocyclyl, heterocyclyl, cyano, and aminocarbonyl, wherein:
   the amino, aminosulfonyl, and aminocarbonyl optionally are substituted with one or two substituents independently selected from the group consisting of alkyl and alkylsulfonyl.

In some embodiments, each **R^{H}** is independently selected from the group consisting of alkyloxy and alkylsulfonyloxy, wherein:
each such substituent optionally is substituted with one or two substituents independently selected from the group consisting of carboxy, hydroxy, halo, amino, nitro, oxo, aminosulfonyl, alkyloxycarbonyl, alkylcarbonyloxy, alkyloxy, cyano, and aminocarbonyl, wherein:
   the amino, aminosulfonyl, and aminocarbonyl optionally are substituted with one or two substituents independently selected from the group consisting of alkyl and alkylsulfonyl.

In some embodiments, each **R^{H}** is independently selected from the group consisting of alkyloxy and alkylsulfonyloxy, wherein:
each such substituent optionally is substituted with one or two substituents independently selected from the group consisting of carboxy, hydroxy, halo, amino, nitro, oxo, aminosulfonyl, alkyloxycarbonyl, alkylcarbonyloxy, alkyloxy, cyano, and aminocarbonyl.

In some embodiments, each **R^{H}** is independently selected alkyloxy.

In some embodiments, each **R^{H}** is independently selected alkylsulfonyloxy.

### B17. Substituent R^{I}.

Each **R^{I}** is independently selected from the group consisting of alkylcarbonyl, alkenylcarbonyl, alkynylcarbonyl, aminocarbonyl, alkyloxycarbonyl, carbocyclylcarbonyl, and heterocyclylcarbonyl, wherein:
(**a**) the alkylcarbonyl, alkenylcarbonyl, and alkynylcarbonyl optionally are substituted with one or more substituents independently selected from the group consisting of carboxy, hydroxy, halo, amino, nitro, azido, oxo, aminosulfonyl, alkyloxycarbonyl, alkenyloxycarbonyl, alkynyloxycarbonyl, alkylcarbonyloxy, alkenylcarbonyloxy, alkynylcarbonyloxy, alkyloxy, alkenyloxy, alkynyloxy, carbocyclyl, heterocyclyl, cyano, and aminocarbonyl, and
(**b**) the aminocarbonyl optionally is substituted with one or two substituents independently selected from the group consisting of alkyl, alkenyl, alkynyl, alkyloxyalkyl, carbocyclyl, heterocyclyl, alkylsulfonyl, and alkylsulfonylamino, wherein:
   the carbocyclyl and heterocyclyl optionally are substituted with one or two substituents independently selected from the group consisting of halo, alkyl, and oxo.

In some embodiments, each **R^{I}** is independently selected from the group consisting of alkylcarbonyl, alkenylcarbonyl, alkynylcarbonyl, aminocarbonyl, alkyloxycarbonyl, carbocyclylcarbonyl, and heterocyclylcarbonyl, wherein such substituents are not substituted.

In some embodiments, each **R^{I}** is independently selected from the group consisting of alkylcarbonyl, aminocarbonyl, alkyloxycarbonyl, carbocyclylcarbonyl, and heterocyclylcarbonyl, wherein:
(**a**) the alkylcarbonyl optionally is substituted with a substituent selected from the group consisting of carboxy, hydroxy, halo, amino, nitro, oxo, aminosulfonyl, alkyloxycarbonyl, alkylcarbonyloxy, alkyloxy, and aminocarbonyl, and
(**b**) the aminocarbonyl optionally is substituted with a substituent selected from the group consisting of alkyl, alkyloxyalkyl, alkylsulfonyl, and alkylsulfonylamino.

In some embodiments, each **R^{I}** is independently selected from the group consisting of alkylcarbonyl and aminocarbonyl, wherein:
the aminocarbonyl optionally is substituted with a substituent selected from the group consisting of alkyl, alkyloxyalkyl, alkylsulfonyl, and alkylsulfonylamino.

In some embodiment, each **R^{I}** is independently selected from the group consisting of alkylcarbonyl, alkenylcarbonyl, alkynylcarbonyl, and aminocarbonyl, wherein:
(**a**) the alkylcarbonyl, alkenylcarbonyl, and alkynylcarbonyl optionally are substituted with one or more substituents independently selected from the group consisting of carboxy, hydroxy, halo, amino, nitro, azido, oxo, aminosulfonyl, alkyloxycarbonyl, alkenyloxycarbonyl, alkynyloxycarbonyl, alkylcarbonyloxy, alkenylcarbonyloxy, alkynylcarbonyloxy, alkyloxy, alkenyloxy, alkynyloxy, carbocyclyl, heterocyclyl, cyano, and aminocarbonyl, and
(**b**) the aminocarbonyl optionally is substituted with one or two substituents independently selected from the group consisting of alkyl, alkenyl, alkynyl, and alkylsulfonylamino.

In some of the above embodiments, each **R^{I}** is independently selected from the group consisting of alkylcarbonyl, alkenylcarbonyl, alkynylcarbonyl, and aminocarbonyl, wherein such substituents are not substituted.

In some embodiments, each **R^{I}** is independently selected from the group consisting of alkylcarbonyl and aminocarbonyl, wherein:
(**a**) the alkylcarbonyl optionally is substituted with one or two substituents independently selected from the group consisting of carboxy, hydroxy, halo, amino, nitro, azido, oxo, aminosulfonyl, alkyloxycarbonyl, alkylcarbonyloxy, alkyloxy, carbocyclyl, heterocyclyl, cyano, and aminocarbonyl, and
(**b**) the aminocarbonyl optionally is substituted with one or two substituents independently selected from the group consisting of alkyl and alkylsulfonylamino.

In some embodiments, each **R^{I}** is independently selected from the group consisting of alkylcarbonyl and aminocarbonyl, wherein:
(**a**) the alkylcarbonyl optionally is substituted with one or two substituents independently selected from the group consisting of carboxy, hydroxy, halo, amino, nitro, oxo, aminosulfonyl, alkyloxycarbonyl, alkylcarbonyloxy, alkyloxy, cyano, and aminocarbonyl, and
(**b**) the aminocarbonyl optionally is substituted with one or two substituents independently selected from the group consisting of alkyl and alkylsulfonylamino.

In some embodiments, each **R^{I}** is independently selected from the group consisting of alkylcarbonyl and aminocarbonyl, wherein:
the alkylcarbonyl optionally is substituted with one or two substituents independently selected from the group consisting of carboxy, hydroxy, halo, amino, nitro, azido, oxo, aminosulfonyl, alkyloxycarbonyl, alkylcarbonyloxy, alkyloxy, carbocyclyl, heterocyclyl, cyano, and aminocarbonyl.

In some embodiments, each **R^{I}** is independently selected alkylcarbonyl.

In some embodiments, each **R^{I}** is independently selected aminocarbonyl.

### B18. Substituent R^{J}.

Each **R^{J}** is independently selected from the group consisting of carbocyclylsulfonylamino, heterocyclylsulfonylamino, alkylcarbonylamino, alkenylcarbonylamino, alkynylcarbonylamino, alkyloxycarbonylamino, alkenyloxycarbonylamino, alkynyloxycarbonylamino, alkylsulfonylamino, alkenylsulfonylamino, alkynylsulfonylamino, aminocarbonylamino, alkyloxycarbonylaminoimino, alkylsulfonylaminoimino, alkenylsulfonylaminoimino, and alkynylsulfonylaminoimino, wherein:
(**a**) the amino portion of such substituents optionally is substituted with a substituent independently selected from the group consisting of carbocyclylalkyl, heterocyclylalkyl, alkylcarbonyloxy, aminocarbonylalkyl, alkyl, alkenyl, alkynyl, alkylcarbonyl, alkenylcarbonyl, alkynylcarbonyl, alkyloxycarbonyl, alkyloxyalkyloxycarbonyl, alkylcarbonyloxyalkyl, and alkylsulfonyl, wherein:
   (**1**) the carbocyclyl portion of the carbocyclylalkyl and the heterocyclyl portion of the heterocyclylalkyl optionally are substituted with one or more substituents independently selected from the group consisting of alkyl, alkenyl, alkynyl, carboxy, hydroxy, alkyloxy, alkenyloxy, alkynyloxy, halo, nitro, cyano, azido, oxo, and amino, and
   (**2**) the amino portion of the aminocarbonylalkyl optionally is substituted with one or two substituents independently selected from the group consisting of alkyl, alkenyl, and alkynyl,
(**b**) the alkyl, alkenyl, and alkynyl portion of such substituents optionally is substituted with one or more substituents independently selected from the group consisting of carboxy, halo, oxo, amino, alkyloxycarbonyl, alkylcarbonyloxy, hydroxy, alkyloxy, carbocyclyl, heterocyclyl, and cyano, wherein:
   the amino optionally is substituted with one or two substituents independently selected from the group consisting of alkyl, alkenyl, alkynyl, alkyloxy, alkenyloxy, and alkynyloxy, wherein:
      the alkyl optionally is substituted with one or more hydroxy;
(**c**) the carbocyclyl and heterocyclyl portions of such substituents optionally are substituted with one or more substituents independently selected from the group consisting of alkyl, alkenyl, alkynyl, carboxy, hydroxy, alkyloxy, alkenyloxy, alkynyloxy, halo, nitro, cyano, azido, and amino, wherein:
   the amino optionally is substituted with one or two substituents independently
   selected from the group consisting of alkyl, alkenyl, and alkynyl.

In some embodiment, each **R^{J}** is independently selected from the group consisting of carbocyclylsulfonylamino, heterocyclylsulfonylamino, alkylcarbonylamino, alkenylcarbonylamino, alkynylcarbonylamino, alkyloxycarbonylamino, alkenyloxycarbonylamino, alkynyloxycarbonylamino, alkylsulfonylamino, alkenylsulfonylamino, alkynylsulfonylamino, aminocarbonylamino, alkylsulfonylaminoimino, alkenylsulfonylaminoimino, and alkynylsulfonylaminoimino, wherein:
(**a**) the amino portion of such substituents optionally is substituted with a substituent independently selected from the group consisting of carbocyclylalkyl, heterocyclylalkyl, alkylcarbonyloxy, aminocarbonylalkyl, alkyl, alkenyl, alkynyl, alkylcarbonyl, alkenylcarbonyl, alkynylcarbonyl, alkyloxycarbonyl, alkyloxyalkyloxycarbonyl, alkylcarbonyloxyalkyl, and alkylsulfonyl, wherein:
   (**1**) the carbocyclyl portion of the carbocyclylalkyl and the heterocyclyl portion of the heterocyclylalkyl optionally are substituted with one or more substituents independently selected from the group consisting of alkyl, alkenyl, alkynyl, carboxy, hydroxy, alkyloxy, alkenyloxy, alkynyloxy, halo, nitro, cyano, azido, oxo, and amino, and
   (**2**) the amino portion of the aminocarbonylalkyl optionally is substituted with one or two substituents independently selected from the group consisting of alkyl, alkenyl, and alkynyl,
(**b**) the alkyl, alkenyl, and alkynyl portion of such substituents optionally is substituted with one or more substituents independently selected from the group consisting of carboxy, halo, oxo, amino, alkyloxycarbonyl, alkylcarbonyloxy, hydroxy, alkyloxy, carbocyclyl, heterocyclyl, and cyano, wherein:
   the amino optionally is substituted with one or two substituents independently selected from the group consisting of alkyl, alkenyl, alkynyl, alkyloxy, alkenyloxy, and alkynyloxy, wherein:
      the alkyl optionally is substituted with one or more hydroxy;
(**c**) the carbocyclyl and heterocyclyl portions of such substituents optionally are substituted with one or more substituents independently selected from the group consisting of alkyl, alkenyl, alkynyl, carboxy, hydroxy, alkyloxy, alkenyloxy, alkynyloxy, halo, nitro, cyano, azido, and amino, wherein:
   the amino optionally is substituted with one or two substituents independently selected from the group consisting of alkyl, alkenyl, and alkynyl; and

In some of the above embodiments, each **R^{J}** is independently selected from the group consisting of carbocyclylsulfonylamino, heterocyclylsulfonylamino, alkylcarbonylamino, alkenylcarbonylamino, alkynylcarbonylamino, alkyloxycarbonylamino, alkenyloxycarbonylamino, alkynyloxycarbonylamino, alkylsulfonylamino, alkenylsulfonylamino, alkynylsulfonylamino, aminocarbonylamino, alkylsulfonylaminoimino, alkenylsulfonylaminoimino, and alkynylsulfonylaminoimino, wherein such substituents are not substituted.

In some embodiments, each **R^{J}** is independently selected from the group consisting of carbocyclylsulfonylamino, heterocyclylsulfonylamino, alkylcarbonylamino, alkyloxycarbonylamino, alkylsulfonylamino, aminocarbonylamino, and alkylsulfonylaminoimino, wherein:
(**a**) the amino portion of such substituents optionally is substituted with a substituent independently selected from the group consisting of carbocyclylalkyl, heterocyclylalkyl, alkylcarbonyloxy, aminocarbonylalkyl, alkyl, alkylcarbonyl, alkyloxycarbonyl, alkyloxyalkyloxycarbonyl, alkylcarbonyloxyalkyl, and alkylsulfonyl, wherein:
   (**1**) the carbocyclyl portion of the carbocyclylalkyl and the heterocyclyl portion of the heterocyclylalkyl optionally are substituted with one or two substituents independently selected from the group consisting of alkyl, carboxy, hydroxy, alkyloxy, halo, nitro, cyano, oxo, and amino, and
   (**2**) the amino portion of the aminocarbonylalkyl optionally is substituted with one or two substituents independently selected from the group consisting of alkyl, alkenyl, and alkynyl,
(**b**) the alkyl portion of such substituents optionally is substituted with one or two substituents independently selected from the group consisting of carboxy, halo, oxo, amino, alkyloxycarbonyl, alkylcarbonyloxy, hydroxy, alkyloxy, carbocyclyl, heterocyclyl, and cyano, wherein:
   the amino optionally is substituted with one or two substituents independently selected from the group consisting of alkyl and alkyloxy, wherein:
      the alkyl optionally is substituted with one or more hydroxy;
(**c**) the carbocyclyl and heterocyclyl portions of such substituents optionally are substituted with one or two substituents independently selected from the group consisting of alkyl, carboxy, hydroxy, alkyloxy, halo, nitro, cyano, and amino, wherein:
   the amino optionally is substituted with one or two substituents independently selected alkyl.

In some embodiments, each **R^{J}** is independently selected from the group consisting of carbocyclylsulfonylamino, heterocyclylsulfonylamino, alkylsulfonylamino, and alkylsulfonylaminoimino, wherein:
(**a**) the amino portion of such substituents optionally is substituted with a substituent independently selected from the group consisting of carbocyclylalkyl, heterocyclylalkyl, alkylcarbonyloxy, aminocarbonylalkyl, alkyl, alkylcarbonyl, alkyloxycarbonyl, alkyloxyalkyloxycarbonyl, alkylcarbonyloxyalkyl, and alkylsulfonyl, wherein:
   (**1**) the carbocyclyl portion of the carbocyclylalkyl and the heterocyclyl portion of the heterocyclylalkyl optionally are substituted with one or two substituents independently selected from the group consisting of alkyl, carboxy, hydroxy, alkyloxy, halo, nitro, cyano, oxo, and amino, and
   (**2**) the amino portion of the aminocarbonylalkyl optionally is substituted with one or two substituents independently selected from the group consisting of alkyl, alkenyl, and alkynyl,
(**b**) the alkyl portion of such substituents optionally is substituted with one or two substituents independently selected from the group consisting of carboxy, halo, oxo, amino, alkyloxycarbonyl, alkylcarbonyloxy, hydroxy, alkyloxy, carbocyclyl, heterocyclyl, and cyano, wherein:
   the amino optionally is substituted with one or two substituents independently selected from the group consisting of alkyl and alkyloxy, wherein:
      the alkyl optionally is substituted with one or more hydroxy;
(**c**) the carbocyclyl and heterocyclyl portions of such substituents optionally are substituted with one or two substituents independently selected from the group consisting of alkyl, carboxy, hydroxy, alkyloxy, halo, nitro, cyano, and amino, wherein:
   the amino optionally is substituted with one or two substituents independently selected alkyl.

In some embodiments, each **R^{J}** is independently selected from the group consisting of carbocyclylsulfonylamino, heterocyclylsulfonylamino, alkylsulfonylamino, and alkylsulfonylaminoimino, wherein:
the amino portion of such substituents optionally is substituted with a substituent independently selected from the group consisting of carbocyclylalkyl, heterocyclylalkyl, alkylcarbonyloxy, aminocarbonylalkyl, alkyl, alkylcarbonyl, alkyloxycarbonyl, alkyloxyalkyloxycarbonyl, alkylcarbonyloxyalkyl, and alkylsulfonyl, wherein:
   (**1**) the carbocyclyl portion of the carbocyclylalkyl and the heterocyclyl portion of the heterocyclylalkyl optionally are substituted with one or two substituents independently selected from the group consisting of alkyl, carboxy, hydroxy, alkyloxy, halo, nitro, cyano, oxo, and amino, and
   (**2**) the amino portion of the aminocarbonylalkyl optionally is substituted with one or two substituents independently selected from the group consisting of alkyl, alkenyl, and alkynyl.

In some embodiments, each **R^{J}** is independently selected from the group consisting of carbocyclylsulfonylamino, heterocyclylsulfonylamino, alkylsulfonylamino, and alkylsulfonylaminoimino, wherein:
the alkyl portion of the alkylsulfonylamino and alkylsulfonylaminoimino optionally is substituted with one or two substituents independently selected from the group consisting of carboxy, halo, oxo, amino, alkyloxycarbonyl, alkylcarbonyloxy, hydroxy, alkyloxy, carbocyclyl, heterocyclyl, and cyano, wherein:
   the amino optionally is substituted with one or two substituents independently selected from the group consisting of alkyl and alkyloxy, wherein:
      the alkyl optionally is substituted with one or more hydroxy.

In some embodiments, each **R^{J}** is independently selected from the group consisting of carbocyclylsulfonylamino, heterocyclylsulfonylamino, alkylsulfonylamino, and alkylsulfonylaminoimino, wherein:
the carbocyclyl and heterocyclyl portions of such substituents optionally are substituted with one or two substituents independently selected from the group consisting of alkyl, carboxy, hydroxy, alkyloxy, halo, nitro, cyano, and amino.

In some embodiments, each **R^{J}** is independently selected from the group consisting of carbocyclylsulfonylamino and heterocyclylsulfonylamino, wherein:
the carbocyclyl and heterocyclyl portions of such substituents optionally are substituted with one or two substituents independently selected from the group consisting of alkyl, carboxy, hydroxy, alkyloxy, halo, nitro, cyano, and amino.

In some embodiments, each **R^{J}** is independently selected from the group consisting of alkylsulfonylamino, alkenylsulfonylamino, alkynylsulfonylamino, and alkylsulfonylaminoimino, wherein:
(**a**) the amino portion of such substituents optionally is substituted with a substituent independently selected from the group consisting of carbocyclylalkyl, heterocyclylalkyl, alkylcarbonyloxy, aminocarbonylalkyl, alkyl, alkylcarbonyl, alkyloxycarbonyl, alkyloxyalkyloxycarbonyl, alkylcarbonyloxyalkyl, and alkylsulfonyl, wherein:
   (**1**) the carbocyclyl portion of the carbocyclylalkyl and the heterocyclyl portion of the heterocyclylalkyl optionally are substituted with one or two substituents independently selected from the group consisting of alkyl, carboxy, hydroxy, alkyloxy, halo, nitro, cyano, oxo, and amino, and
   (**2**) the amino portion of the aminocarbonylalkyl optionally is substituted with one or two substituents independently selected from the group consisting of alkyl, alkenyl, and alkynyl,
(**b**) the alkyl, alkenyl, and alkynyl portion of such substituents optionally is substituted with one or two substituents independently selected from the group consisting of carboxy, halo, oxo, amino, alkyloxycarbonyl, alkylcarbonyloxy, hydroxy, alkyloxy, carbocyclyl, heterocyclyl, and cyano, wherein:
   the amino optionally is substituted with one or two substituents independently selected from the group consisting of alkyl and alkyloxy, wherein:
      the alkyl optionally is substituted with one or more hydroxy.

In some embodiments, each **R^{J}** is an independently selected alkylsulfonylamino, wherein:
(**a**) the amino portion of the alkylsulfonylamino optionally is substituted with a substituent independently selected from the group consisting of carbocyclylalkyl, heterocyclylalkyl, alkylcarbonyloxy, aminocarbonylalkyl, alkyl, alkylcarbonyl, alkyloxycarbonyl, alkyloxyalkyloxycarbonyl, alkylcarbonyloxyalkyl, and alkylsulfonyl, wherein:
   (**1**) the carbocyclyl portion of the carbocyclylalkyl and the heterocyclyl portion of the heterocyclylalkyl optionally are substituted with one or two substituents independently selected from the group consisting of alkyl, carboxy, hydroxy, alkyloxy, halo, nitro, cyano, oxo, and amino, and
   (**2**) the amino portion of the aminocarbonylalkyl optionally is substituted with one or two substituents independently selected from the group consisting of alkyl, alkenyl, and alkynyl,
(**b**) the alkyl portion of the alkylsulfonylamino optionally is substituted with one or two substituents independently selected from the group consisting of carboxy, halo, oxo, amino, alkyloxycarbonyl, alkylcarbonyloxy, hydroxy, alkyloxy, carbocyclyl, heterocyclyl, and cyano, wherein:
   the amino optionally is substituted with one or two substituents independently selected from the group consisting of alkyl and alkyloxy, wherein:
      the alkyl optionally is substituted with one or more hydroxy.

In some embodiments, each **R^{J}** is an independently selected alkylsulfonylamino, wherein:
the amino portion of the alkylsulfonylamino optionally is substituted with a substituent independently selected from the group consisting of carbocyclylalkyl, heterocyclylalkyl, alkylcarbonyloxy, aminocarbonylalkyl, alkyl, alkylcarbonyl, alkyloxycarbonyl, alkyloxyalkyloxycarbonyl, alkylcarbonyloxyalkyl, and alkylsulfonyl, wherein:
   (**1**) the carbocyclyl portion of the carbocyclylalkyl and the heterocyclyl portion of the heterocyclylalkyl optionally are substituted with one or two substituents independently selected from the group consisting of alkyl, carboxy, hydroxy, alkyloxy, halo, nitro, cyano, oxo, and amino, and
   (**2**) the amino portion of the aminocarbonylalkyl optionally is substituted with one or two substituents independently selected from the group consisting of alkyl, alkenyl, and alkynyl.

In some embodiments, each **R^{J}** is an independently selected alkylsulfonylamino, wherein:
the amino portion of the alkylsulfonylamino optionally is substituted with a substituent independently selected from the group consisting of carbocyclylalkyl, heterocyclylalkyl, alkylcarbonyloxy, aminocarbonylalkyl, alkyl, alkylcarbonyl, alkyloxycarbonyl, alkyloxyalkyloxycarbonyl, alkylcarbonyloxyalkyl, and alkylsulfonyl.

In some embodiments, each **R^{J}** is an independently selected alkylsulfonylamino, wherein:
the alkyl portion of the alkylsulfonylamino optionally is substituted with one or two substituents independently selected from the group consisting of carboxy, halo, oxo, amino, alkyloxycarbonyl, alkylcarbonyloxy, hydroxy, alkyloxy, carbocyclyl, heterocyclyl, and cyano, wherein:
   the amino optionally is substituted with one or two substituents independently selected from the group consisting of alkyl and alkyloxy, wherein:
      the alkyl optionally is substituted with one or more hydroxy.

In some embodiments, each **R^{J}** is an independently selected alkylsulfonylamino, wherein:
the alkyl portion of the alkylsulfonylamino optionally is substituted with one or two substituents independently selected from the group consisting of carboxy, halo, oxo, amino, alkyloxycarbonyl, alkylcarbonyloxy, hydroxy, alkyloxy, carbocyclyl, heterocyclyl, and cyano.

In some embodiments, each **R^{J}** is an independently selected alkylsulfonylamino. In some such embodiments, each **R^{J}** is methylsulfonylamino.

In some embodiments, each **R^{J}** is an independently selected alkylsulfonylaminoimino, wherein:
(**a**) the amino portion of the alkylsulfonylaminoimino optionally is substituted with a substituent independently selected from the group consisting of carbocyclylalkyl, heterocyclylalkyl, alkylcarbonyloxy, aminocarbonylalkyl, alkyl, alkylcarbonyl, alkyloxycarbonyl, alkyloxyalkyloxycarbonyl, alkylcarbonyloxyalkyl, and alkylsulfonyl, wherein:
   (**1**) the carbocyclyl portion of the carbocyclylalkyl and the heterocyclyl portion of the heterocyclylalkyl optionally are substituted with one or two substituents independently selected from the group consisting of alkyl, carboxy, hydroxy, alkyloxy, halo, nitro, cyano, oxo, and amino, and
   (**2**) the amino portion of the aminocarbonylalkyl optionally is substituted with one or two substituents independently selected from the group consisting of alkyl, alkenyl, and alkynyl,
(**b**) the alkyl portion of the alkylsulfonylaminoimino optionally is substituted with one or two substituents independently selected from the group consisting of carboxy, halo, oxo, amino, alkyloxycarbonyl, alkylcarbonyloxy, hydroxy, alkyloxy, carbocyclyl, heterocyclyl, and cyano, wherein:
   the amino optionally is substituted with one or two substituents independently selected from the group consisting of alkyl and alkyloxy, wherein:
      the alkyl optionally is substituted with one or more hydroxy.

In some embodiments, each **R^{J}** is an independently selected alkylsulfonylaminoimino, wherein:
the amino portion of the alkylsulfonylaminoimino optionally is substituted with a substituent independently selected from the group consisting of carbocyclylalkyl, heterocyclylalkyl, alkylcarbonyloxy, aminocarbonylalkyl, alkyl, alkylcarbonyl, alkyloxycarbonyl, alkyloxyalkyloxycarbonyl, alkylcarbonyloxyalkyl, and alkylsulfonyl, wherein:
   (**1**) the carbocyclyl portion of the carbocyclylalkyl and the heterocyclyl portion of the heterocyclylalkyl optionally are substituted with one or two substituents independently selected from the group consisting of alkyl, carboxy, hydroxy, alkyloxy, halo, nitro, cyano, oxo, and amino, and
   (**2**) the amino portion of the aminocarbonylalkyl optionally is substituted with one or two substituents independently selected from the group consisting of alkyl, alkenyl, and alkynyl.

In some embodiments, each **R^{J}** is an independently selected alkylsulfonylaminoimino, wherein:
the amino portion of the alkylsulfonylaminoimino optionally is substituted with a substituent independently selected from the group consisting of carbocyclylalkyl, heterocyclylalkyl, alkylcarbonyloxy, aminocarbonylalkyl, alkyl, alkylcarbonyl, alkyloxycarbonyl, alkyloxyalkyloxycarbonyl, alkylcarbonyloxyalkyl, and alkylsulfonyl.

In some embodiments, each **R^{J}** is an independently selected alkylsulfonylaminoimino, wherein:
the alkyl portion of the alkylsulfonylaminoimino optionally is substituted with one or two substituents independently selected from the group consisting of carboxy, halo, oxo, amino, alkyloxycarbonyl, alkylcarbonyloxy, hydroxy, alkyloxy, carbocyclyl, heterocyclyl, and cyano, wherein:
   the amino optionally is substituted with one or two substituents independently selected from the group consisting of alkyl and alkyloxy, wherein:
      the alkyl optionally is substituted with one or more hydroxy.

In some embodiments, each **R^{J}** is an independently selected alkylsulfonylaminoimino, wherein:
the alkyl portion of the alkylsulfonylaminoimino optionally is substituted with one or two substituents independently selected from the group consisting of carboxy, halo, oxo, amino, alkyloxycarbonyl, alkylcarbonyloxy, hydroxy, alkyloxy, carbocyclyl, heterocyclyl, and cyano.

In some embodiments, each **R^{J}** is an independently selected alkylsulfonylaminoimino. In some such embodiments, each **R^{J}** is methylsulfonylaminoimino.

In some embodiments, each **R^{J}** is independently selected from the group consisting of alkylcarbonylamino and alkyloxycarbonylamino, wherein:
the alkyl portion of such substituents optionally is substituted with one or two substituents independently selected from the group consisting of carboxy, halo, oxo, amino, alkyloxycarbonyl, alkylcarbonyloxy, hydroxy, alkyloxy, carbocyclyl, heterocyclyl, and cyano.

### B19. Substituent R^{K}.

Each **R^{K}** is independently selected from the group consisting of aminosulfonyl, alkylsulfonyl, alkenylsulfonyl, and alkynylsulfonyl, wherein:
(**a**) the alkylsulfonyl, alkenylsulfonyl, and alkynylsulfonyl optionally are substituted with one or more substituents independently selected from the group consisting of carboxy, hydroxy, halo, amino, nitro, azido, oxo, aminosulfonyl, alkyloxycarbonyl, alkenyloxycarbonyl, alkynyloxycarbonyl, alkylcarbonyloxy, alkenylcarbonyloxy, alkynylcarbonyloxy, alkyloxy, alkenyloxy, alkynyloxy, carbocyclyl, heterocyclyl, cyano, and aminocarbonyl, wherein:
   the amino, aminosulfonyl, and aminocarbonyl optionally are substituted with one or two substituents independently selected from the group consisting of alkyl, alkenyl, and alkynyl; and
(**b**) the aminosulfonyl optionally is substituted with one or two substituents independently selected from the group consisting of alkyl, alkenyl, and alkynyl.

In some of the above embodiments, each **R^{K}** is independently selected from the group consisting of aminosulfonyl, alkylsulfonyl, alkenylsulfonyl, and alkynylsulfonyl, wherein such substituents are not substituted.

In some embodiments, each **R^{K}** is independently selected from the group consisting of aminosulfonyl and alkylsulfonyl, wherein:
(**a**) the alkylsulfonyl optionally is substituted with one or two substituents independently selected from the group consisting of carboxy, hydroxy, halo, amino, nitro, oxo, aminosulfonyl, alkyloxycarbonyl, alkylcarbonyloxy, alkyloxy, carbocyclyl, heterocyclyl, cyano, and aminocarbonyl; and
(**b**) the aminosulfonyl optionally is substituted with one or two substituents independently selected alkyl.

In some embodiments, each **R^{K}** is independently selected from the group consisting of aminosulfonyl and alkylsulfonyl.

### C. Embodiments of Compounds of Formula I.

Various embodiments of substituents **R¹**, **R²**, **R³**, **R⁴**, **R⁵**, **L**, **R^{A}**, **R^{B}**, **R^{C}**, **R^{D}**, **R⁶**, **R^{E}**, **R^{F}**, **R^{G}**, **R^{H}**, **R^{I}**, **R^{J}**, and **R^{K}** have been discussed above. These substituent embodiments can be combined to form various embodiments of compounds of formula **I**. All embodiments of compounds of formula **I** formed by combining the substituent embodiments discussed above are within the scope of Applicants' invention, and some illustrative embodiments of the compounds of formula **I** are provided below.

In some embodiments, the compounds of formula **I** correspond in structure to formula **I-L0**: 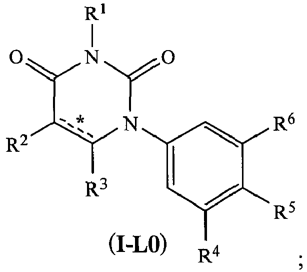 is selected from the group consisting of single carbon-carbon bond and double carbon-carbon bond;
**R¹** is selected from the group consisting of hydrogen and methyl;
**R²** is selected from the group consisting of hydrogen and halo
**R³** is selected from the group consisting of hydrogen and halo;
**R⁴** is selected from the group consisting of C₁-C₄-alkyl, C₃-C₆-carbocyclyl, and 5-6-membered heterocyclyl, wherein:
   (**a**) the C₁-C₄-alkyl optionally is substituted with up to three substituents independently selected from the group consisting of halo, oxo, hydroxy, alkyloxy, and trimethylsilyl, and
   (**b**) the C₃-C₆-carbocyclyl and 5-6-membered heterocyclyl optionally are substituted with one or two substituents independently selected from the group consisting of alkyl, halo, and alkylsulfonylamino;
**R⁵** is selected from the group consisting of hydrogen, hydroxy, alkyloxy, and halo;
**R⁶** is selected from the group consisting of C₅-C₆-carbocyclyl, 5-6-membered heterocyclyl, fused 2-ring heterocyclyl, and fused 2-ring carbocyclyl, wherein each such substituent is substituted with one, two, or three substituents independently selected from the group consisting of R^{E}, **R^{F}, R^{I}, R^{J}**, and **R^{K}**;
each **R^{E}** is independently selected from the group consisting of chloro, fluoro, nitro, hydroxy, oxo, carboxy, amino, imino, aldehydo, and alkylamino;
each **R^{F}** is an independently selected alkyl optionally substituted with a substituent selected from the group consisting of carboxy, halo, amino, imino, and aminosulfonyl, wherein:
   the amino, imino, and aminosulfonyl optionally are substituted with one or two substituents independently selected from the group consisting of alkyl, alkylsulfonyl, and alkylsulfonylamino;
each **R^{I}** is independently selected from the group consisting of alkylcarbonyl and aminocarbonyl, wherein:
   the aminocarbonyl optionally is substituted with a substituent selected from the group consisting of alkyl, alkyloxyalkyl, alkylsulfonyl, and alkylsulfonylamino;
each **R^{J}** is independently selected from the group consisting of alkylsulfonylamino, alkenylsulfonylamino, alkynylsulfonylamino, and alkylsulfonylaminoimino, wherein:
   (**a**) the amino portion of such substituents optionally is substituted with a substituent independently selected from the group consisting of carbocyclylalkyl, heterocyclylalkyl, alkylcarbonyloxy, aminocarbonylalkyl, alkyl, alkylcarbonyl, alkyloxycarbonyl, alkyloxyalkyloxycarbonyl, alkylcarbonyloxyalkyl, and alkylsulfonyl, wherein:
      (**1**) the carbocyclyl portion of the carbocyclylalkyl and the heterocyclyl portion of the heterocyclylalkyl optionally are substituted with one or two substituents independently selected from the group consisting of alkyl, carboxy, hydroxy, alkyloxy, halo, nitro, cyano, oxo, and amino, and
      (**2**) the amino portion of the aminocarbonylalkyl optionally is substituted with one or two substituents independently selected from the group consisting of alkyl, alkenyl, and alkynyl,
   (**b**) the alkyl, alkenyl, and alkynyl portion of such substituents optionally is substituted with one or two substituents independently selected from the group consisting of carboxy, halo, oxo, amino, alkyloxycarbonyl, alkylcarbonyloxy, hydroxy, alkyloxy, carbocyclyl, heterocyclyl, and cyano, wherein:
      the amino optionally is substituted with one or two substituents independently selected from the group consisting of alkyl and alkyloxy, wherein:
         the alkyl optionally is substituted with one or more hydroxy; and
each **R^{K}** is independently selected from the group consisting of aminosulfonyl and alkylsulfonyl, wherein:
   (**a**) the alkylsulfonyl optionally is substituted with one or two substituents independently selected from the group consisting of carboxy, hydroxy, halo, amino, nitro, oxo, aminosulfonyl, alkyloxycarbonyl, alkylcarbonyloxy, alkyloxy, carbocyclyl, heterocyclyl, cyano, and aminocarbonyl; and
   (**b**) the aminosulfonyl optionally is substituted with one or two substituents independently selected alkyl.

In some embodiments, in the compounds of formula **I**:
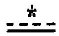 is selected from the group consisting of single carbon-carbon bond and double carbon-carbon bond;
**R¹** is selected from the group consisting of hydrogen and methyl;
**R²** is selected from the group consisting of hydrogen and halo;
**R³** is selected from the group consisting of hydrogen and halo;
**R⁴** is selected from the group consisting of C₁-C₄-alkyl, C₃-C₆-carbocyclyl, and 5-6-membered heterocyclyl, wherein:
   (**a**) the C₁-C₄-alkyl optionally is substituted with up to three substituents independently selected from the group consisting of halo, oxo, hydroxy, alkyloxy, and trimethylsilyl, and
   (**b**) the C₃-C₆-carbocyclyl and 5-6-membered heterocyclyl optionally are substituted with one or two substituents independently selected from the group consisting of alkyl, halo, and alkylsulfonylamino;
**R⁵** is selected from the group consisting of hydrogen, hydroxy, alkyloxy, and halo;
**L** is selected from the group consisting of C(**R^{A}**)=C(**R^{B}**), ethylene, and cyclopropyl-1,2-ene; one of **R^{A}** and **R^{B}** is hydrogen, and the other is selected from the group consisting of hydrogen, methyl, methoxy, and halo;
**R⁶** is selected from the group consisting of C₅-C₆-carbocyclyl and 5-6-membered heterocyclyl, wherein each such substituent is substituted with one, two, or three substituents independently selected from the group consisting of **R^{E}**, **R^{F}**, and **R^{J}**;
   each **R^{E}** is independently selected from the group consisting of chloro, fluoro, nitro, hydroxy, oxo, carboxy, amino, imino, aldehydo, and alkylamino;
   each **R^{F}** is an independently selected alkyl optionally substituted with a substituent selected from the group consisting of carboxy, halo, amino, imino, and aminosulfonyl, wherein:
   the amino, imino, and aminosulfonyl optionally are substituted with one or two substituents independently selected from the group consisting of alkyl, alkylsulfonyl, and alkylsulfonylamino;
   each **R^{I}** is independently selected from the group consisting of alkylcarbonyl and aminocarbonyl, wherein:
   the aminocarbonyl optionally is substituted with a substituent selected from the group consisting of alkyl, alkyloxyalkyl, alkylsulfonyl, and alkylsulfonylamino; and
   each **R^{J}** is independently selected from the group consisting of alkylsulfonylamino, alkenylsulfonylamino, alkynylsulfonylamino, and alkylsulfonylaminoimino, wherein:
   (**a**) the amino portion of such substituents optionally is substituted with a substituent independently selected from the group consisting of carbocyclylalkyl, heterocyclylalkyl, alkylcarbonyloxy, aminocarbonylalkyl, alkyl, alkylcarbonyl, alkyloxycarbonyl, alkyloxyalkyloxycarbonyl, alkylcarbonyloxyalkyl, and alkylsulfonyl, wherein:
      (**1**) the carbocyclyl portion of the carbocyclylalkyl and the heterocyclyl portion of the heterocyclylalkyl optionally are substituted with one or two substituents independently selected from the group consisting of alkyl, carboxy, hydroxy, alkyloxy, halo, nitro, cyano, oxo, and amino, and
      (**2**) the amino portion of the aminocarbonylalkyl optionally is substituted with one or two substituents independently selected from the group consisting of alkyl, alkenyl, and alkynyl,
   (**b**) the alkyl, alkenyl, and alkynyl portion of such substituents optionally is substituted with one or two substituents independently selected from the group consisting of carboxy, halo, oxo, amino, alkyloxycarbonyl, alkylcarbonyloxy, hydroxy, alkyloxy, carbocyclyl, heterocyclyl, and cyano, wherein:
      the amino optionally is substituted with one or two substituents independently selected from the group consisting of alkyl and alkyloxy, wherein the alkyl optionally is substituted with one or more hydroxy.

In some embodiments, the compounds of formula **I** correspond in structure to formula **I-L0**: 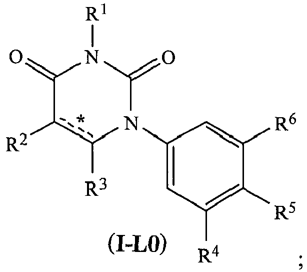 is selected from the group consisting of single carbon-carbon bond and double carbon-carbon bond;
**R¹** is hydrogen;
**R²** is selected from the group consisting of hydrogen and halo
**R³** is hydrogen;
**R⁴** is tert-butyl;
**R⁵** is selected from the group consisting of hydrogen, hydroxy, methoxy, and halo;
**R⁶** is a fused 2-ring carbocyclyl selected from the group consisting of naphthalenyl, dihydronaphthalenyl, tetrahydronaphthalenyl, hexahydronaphthalenyl, octahydronaphthalenyl, decahydronaphthalenyl, indenyl, dihydroindenyl, hexahydroindenyl, octahydroindenyl, pentalenyl, octahydropentalenyl, and hexahydropentalenyl, wherein each such substituent is substituted with a substituent selected from the group consisting of **R^{F}** and **R^{J}**;
**R^{F}** is alkylsulfonylaminoalkyl; and
**R^{J}** is alkylsulfonylamino.

Examples of compounds of formula **I** (and salts thereof) are shown in Tables 1 through 38 below. The synthesis examples below provide step-by-step preparation instructions for some of these compounds. The remaining compounds were prepared utilizing the general method-of-preparation discussion, specific synthesis examples below, and/or the discussion throughout this application.

**TABLE 1**

| | |
|---|---|
| 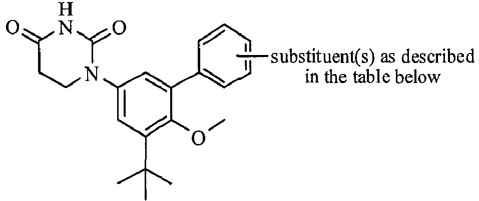 | |

| **compound** | **substituent(s)** |
|---|---|
| | |
| **IA-L0-1.1** | -4-C(H)=NN(H)S(O)₂CH₃ |
| **IA-L0-1.2** | -4-C(CH₃)=NN(H)S(O)₂CH₃ |
| **IA-L0-1.3** | -3-F and -4-C(H)=NN(H)S(O)₂CH₃ and -5-F |
| **IA-L0-1.4** | -3-F and -4-C(H)=NN(H)S(O)₂CH₃ |
| **IA-L0-1.5** | -3-C(H)=NN(H)S(O)₂CH₃ and -4-OCH₃ |
| **IA-L0-1.6** | -2-F and -3-F and -4-C(H)=NN(H)S(O)₂CH₃ |
| **IA-L0-1.7** | -3-C(H)=NN(H)S(O)₂CH₃ |
| **IA-L0-1.8** | -3-C(CH₃)=NN(H)S(O)₂CH₃ |

**TABLE 2**

| | |
|---|---|
| 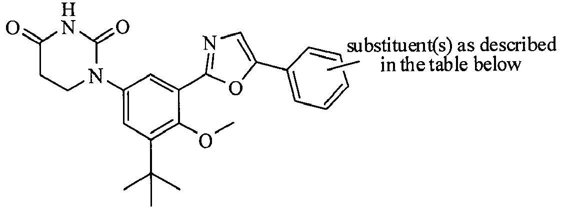 | |

| **compound** | **substituent(s)** |
|---|---|
| | |
| **IA-L0-1.9** | -3-N(H)S(O)₂CH₃ |
| **IA-L0-1.10** | -3-NO₂ |
| **IA-L0-1.11** | -4-NO₂ |

**TABLE 3**

| | |
|---|---|
| 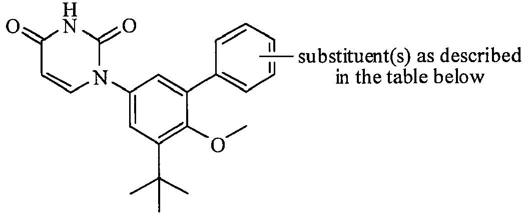 | |

| **compound** | **substituent(s)** |
|---|---|
| | |
| **IB-L0-1.1** | -4-C(H)=NN(H)S(O)₂CH₃ |
| **IB-L0-1.2** | -4-N(H)S(O)₂CH₃ |
| **IB-L0-1.3** | -3-F and -4-C(H)=NN(H)S(O)₂CH₃ |
| **IB-L0-1.4** | -4-C(H)₂C(H)₂N(H)S(O)₂CH₃ |
| **IB-L0-1.5** | -4-C(CH₃)=NN(H)S(O)₂CH₃ |
| **IB-L0-1.6** | -3-C(H)=NN(H)S(O)₂CH₃ and -4-OCH₃ |
| **IB-L0-1.7** | -4-N(H)C(O)N(H)S(O)₂CH₃ |
| **IB-L0-1.8** | -4-C(O)N(H)N(H)S(O)₂CH₃ |
| **IB-L0-1.9** | -3-C(CH₃)=NN(H)S(O)₂CH₃ |
| **IB-L0-1.10** | -3-C(H)=NN(H)S(O)₂CH₃ |

**TABLE 4**

| | | |
|---|---|---|
| 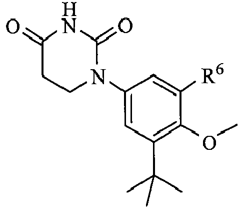 | | |

| **compound** | **R⁶** | |
|---|---|---|
| | **ring/ring structure** | **substituent(s)** |
| | | |
| **IA-L0-2.1** | benzimidazol-2-yl | -5-N(H)S(O)₂CH₃ |
| **IA-L0-2.2** | benzthiazol-2-yl | -6-N(H)S(O)₂CH₃ |
| **IA-L0-2.3** | benzthiazol-2yl | --- |
| **IA-L0-2.4** | benzthiazol-2-yl | -5-N(H)S(O)₂CH₃ |
| **IA-L0-2.5** | benzoxazol-2-yl | -6-N(H)S(O)₂CH₃ |
| **IA-L0-2.6** | benzoxazol-2-yl | -6-NO₂ |
| **IA-L0-2.7** | benzoxazol-2-yl | -5-NO₂ |
| **IA-L0-2.8** | benzoxazol-2-yl | -5-N(H)S(O)₂CH₃ |
| **IA-L0-2.9** | naphthalen-2-yl | -6-N(H)S(O)₂CH₃ |
| **IA-L0-2.10** | benzimidazol-2-yl | -5-N[S(O)₂CH₃]₂ |

**TABLE 5**

| | | | |
|---|---|---|---|
| 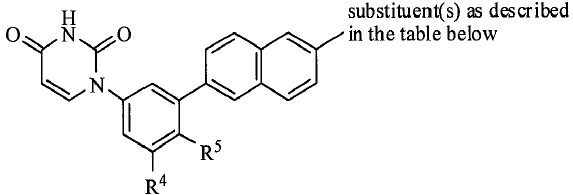 | | | |

| **compound** | **R⁴** | **R⁵** | **substituent(s)** |
|---|---|---|---|
| | | | |
| **IB-L0-2.1** | -C(CH₃)₃ | -OCH₃ | -H |
| **IB-L0-2.2** | -C(CH₃)₃ | -OCH₃ | -OCH₃ |
| **IB-L0-2.3** | -C(CH₃)₃ | -OCH₃ | -N(H)S(O)₂CH₃ |
| **IB-L0-2.8** | -C(CH₃)₃ | -H | -N(H)S(O)₂CH₃ |
| **IB-L0-2.14** | -C(CH₃)₃ | -Cl | -N(H)S(O)₂CH₃ |
| **IB-L0-2.23** | -C(CH₃)₃ | -OC(H)₂CH₃ | -N(H)S(O)₂CH₃ |
| **IB-L0-2.52** | -C(CH₃)₂C(H)₂C(H)₃ | -OCH₃ | -N(H)S(O)₂CH₃ |
| **IB-L0-2.53** | 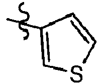 | -OCH₃ | -N(H)S(O)₂CH₃ |
| **IB-L0-2.54** | -C(CH₃)₂C(H)₂OH | -OCH₃ | -N(H)S(O)₂CH₃ |
| **IB-L0-2.56** | -CF₃ | -OCH₃ | -N(H)S(O)₂CH₃ |
| **IB-L0-2.57** | -I | -OCH₃ | -N(H)S(O)₂CH₃ |
| **IB-L0-2.58** | 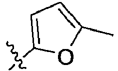 | -OCH₃ | -N(H)S(O)₂CH₃ |
| **IB-L0-2.59** | furan-2-yl | -OCH₃ | -N(H)S(O)₂CH₃ |
| **IB-L0-2.60** | -C(F)₂CF₃ | -OCH₃ | -N(H)S(O)₂CH₃ |
| **IB-L0-2.61** | 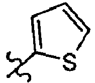 | -OCH₃ | -N(H)S(O)₂CH₃ |
| **IB-L0-2.64** | furan-3-yl | -OCH₃ | -N(H)S(O)₂CH₃ |
| **IB-L0-2.66** | -C(CH₃)₂C(H)₂OCH₃ | -OCH₃ | -N(H)S(O)₂CH₃ |
| **IB-L0-2.68** | -S(O)₂CH₃ | -OCH₃ | -N(H)S(O)₂CH₃ |
| **IB-L0-2.69** | -Br | -OCH₃ | -N(H)S(O)₂CH₃ |
| **IB-L0-2.70** | -C(CH₃)₂C(O)OCH₃ | -OCH₃ | -N(H)S(O)₂CH₃ |
| **IB-L0-2.71** | phenyl | -OCH₃ | -N(H)S(O)₂CH₃ |
| **IB-L0-2.72** | -C(O)OCH₃ | -OCH₃ | -N(H)S(O)₂CH₃ |
| **IB-L0-2.73** | 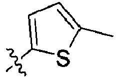 | -OCH₃ | -N(H)S(O)₂CH₃ |
| **IB-L0-2.74** | 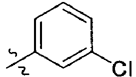 | -OCH₃ | -N(H)S(O)₂CH₃ |
| **IB-L0-2.75** | -N(H)S(O)₂CH₃ | -OCH₃ | -N(H)S(O)₂CH₃ |
| **IB-L0-2.76** | 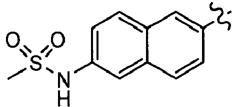 | -OCH₃ | -N(H)S(O)₂CH₃ |
| **IB-L0-2.77** | -C(CH₃)₂C(O)OH | -OCH₃ | -N(H)S(O)₂CH₃ |
| **IB-L0-2.78** | -C≡CSi(CH₃)₃ | -OCH₃ | -N(H)S(O)₂CH₃ |

**TABLE 6**

| | | |
|---|---|---|
| 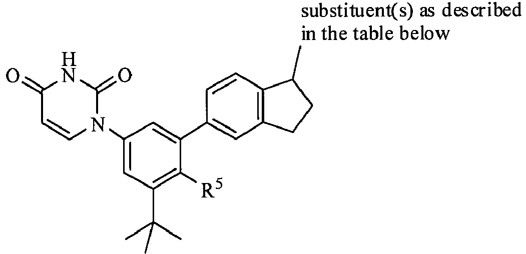 | | |

| **compound** | **R⁵** | **substituent(s)** |
|---|---|---|
| | | |
| **IB-L0-2.4** | -OCH₃ | =NN(H)S(O)₂CH₃ |
| **IB-L0-2.7** | -H | =NN(H)S(O)₂CH₃ |
| **IB-L0-2.9** | -OCH₃ | (S) -C(H)₂N(H)S(O)₂CH₃ |
| **IB-L0-2.10** | -OCH₃ | (R) -F and -C(H)₂N(H)S(O)₂CH₃ |
| **IB-L0-2.12** | -OCH₃ | -F and -C(H)₂N(H)S(O)₂CH₃ |
| **IB-L0-2.15** | -OCH₃ | (R) -C(H)₂N(H)S(O)₂CH₃ |
| **IB-L0-2.17** | -OCH₃ | -C(H)₂N(H)S(O)₂CH₃ |
| **IB-L0-2.20** | -OCH₃ | (S) -F and -C(H)₂N(H)S(O)₂CH₃ |
| **IB-L0-2.22** | -OCH₃ | (S) -C(CH₃)₂N(H)S(O)₂CH₃ |
| **IB-L0-2.24** | -OCH₃ | =NN(H)C(O)OCH₃ |
| **IB-L0-2.25** | -OCH₃ | -CH₃ and -C(H)₂N(H)S(O)₂CH₃ |
| **IB-L0-2.29** | -OCH₃ | -C(CH₃)₂N(H)S(O)₂CH₃ |
| **IB-L0-2.31** | -OCH₃ | -N(H)N(H)S(O)₂CH₃ |
| **IB-L0-2.34** | -OCH₃ | -C(O)N(H)S(O)₂CH₃ |
| **IB-L0-2.36** | -OCH₃ | -OH |
| **IB-L0-2.37** | -OCH₃ | (R) -C(CH₃)₂N(H)S(O)₂CH₃ |
| **IB-L0-2.44** | -OCH₃ | -N(H)S(O)₂CH₃ |
| **IB-L0-2.50** | -OCH₃ | =O |

**TABLE 7**

| | | | |
|---|---|---|---|
| 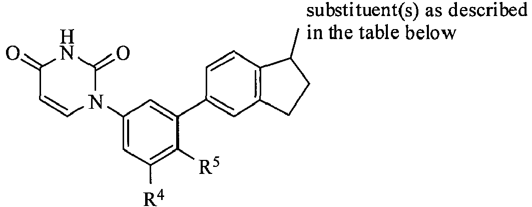 | | | |

| **compound** | **R⁴** | **R⁵** | **substituent(s)** |
|---|---|---|---|
| | | | |
| **IB-L0-2.51** | 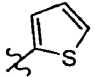 | -OCH₃ | =NN(H)S(O)₂CH₃ |
| **IB-L0-2.55** | furan-2-yl | -OCH₃ | =NN(H)S(O)₂CH₃ |

**TABLE 8**

| | | |
|---|---|---|
| 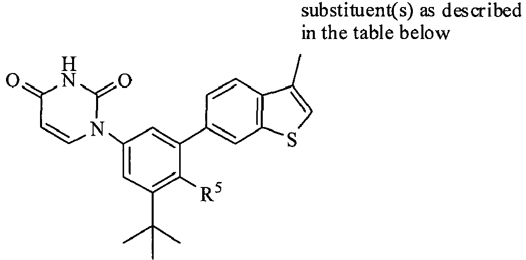 | | |

| **compound** | **R⁵** | **substituent(s)** |
|---|---|---|
| | | |
| **IB-L0-2.11** | -OCH₃ | C(H)₂N(H)S(O)₂CH₃ |
| **IB-L0-2.21** | -OCH₃ | -C(H)₂N(CH₃)S(O)₂CH₃ |
| **IB-L0-2.35** | -Cl | -C(H)₂N(H)S(O)₂CH₃ |

**TABLE 9**

| | | |
|---|---|---|
| 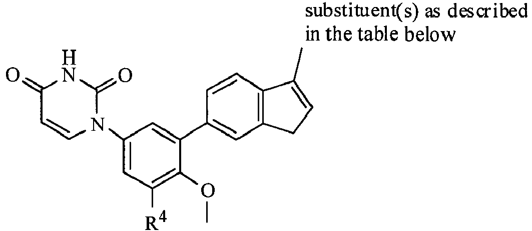 | | |

| **compound** | **R⁴** | **substituent(s)** |
|---|---|---|
| | | |
| **IB-L0-2.13** | -C(CH₃)₃ | -C(H)₂N(H)S(O)₂CH₃ |
| **IB-L0-2.16** | -C(CH₃)₃ | -C(H)₂N(CH₃)S(O)₂CH₃ |
| **IB-L0-2.41** | -C(CH₃)₃ | -C(CH₃)₂N(H)S(O)₂CH₃ |
| **IB-L0-2.62** | 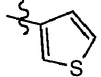 | -C(H)₂N(H)S(O)₂CH₃ |
| **IB-L0-2.63** | 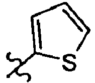 | -C(H)₂N(H)S(O)₂CH₃ |
| **IB-L0-2.65** | furan-2-yl | -C(H)₂N(H)S(O)₂CH₃ |
| **IB-L0-2.67** | furan-3-yl | -C(H)₂N(H)S(O)₂CH₃ |

**TABLE 10**

| | |
|---|---|
| 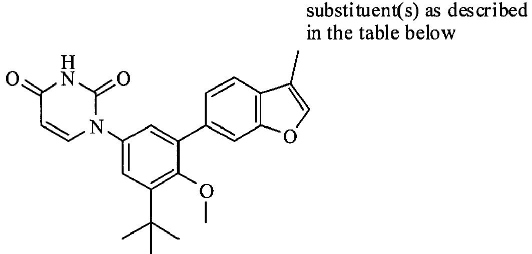 | |

| **compound** | **substituent(s)** |
|---|---|
| | |
| **IB-L0-2.18** | -C(H)₂N(H)S(O)₂CH₃ |
| **IB-L0-2.42** | -CH₃ |

**TABLE 11**

| | |
|---|---|
| 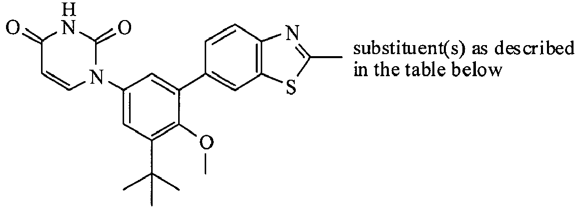 | |

| **compound** | **substituent(s)** |
|---|---|
| | |
| **IB-L0-2.27** | -NH₂ |
| **IB-L0-2.28** | -N(H)S(O)₂CH₃ |
| **IB-L0-2.33** | -H |
| **IB-L0-2.38** | -Cl |
| **IB-L0-2.39** | -NH₂ |
| **IB-L0-2.46** | -N(H)C(H)₂ C(H)₂CH₃ |
| **IB-L0-2.47** | 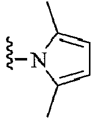 |
| **IB-L0-2.49** | -N(H)C(O)CH₃ |

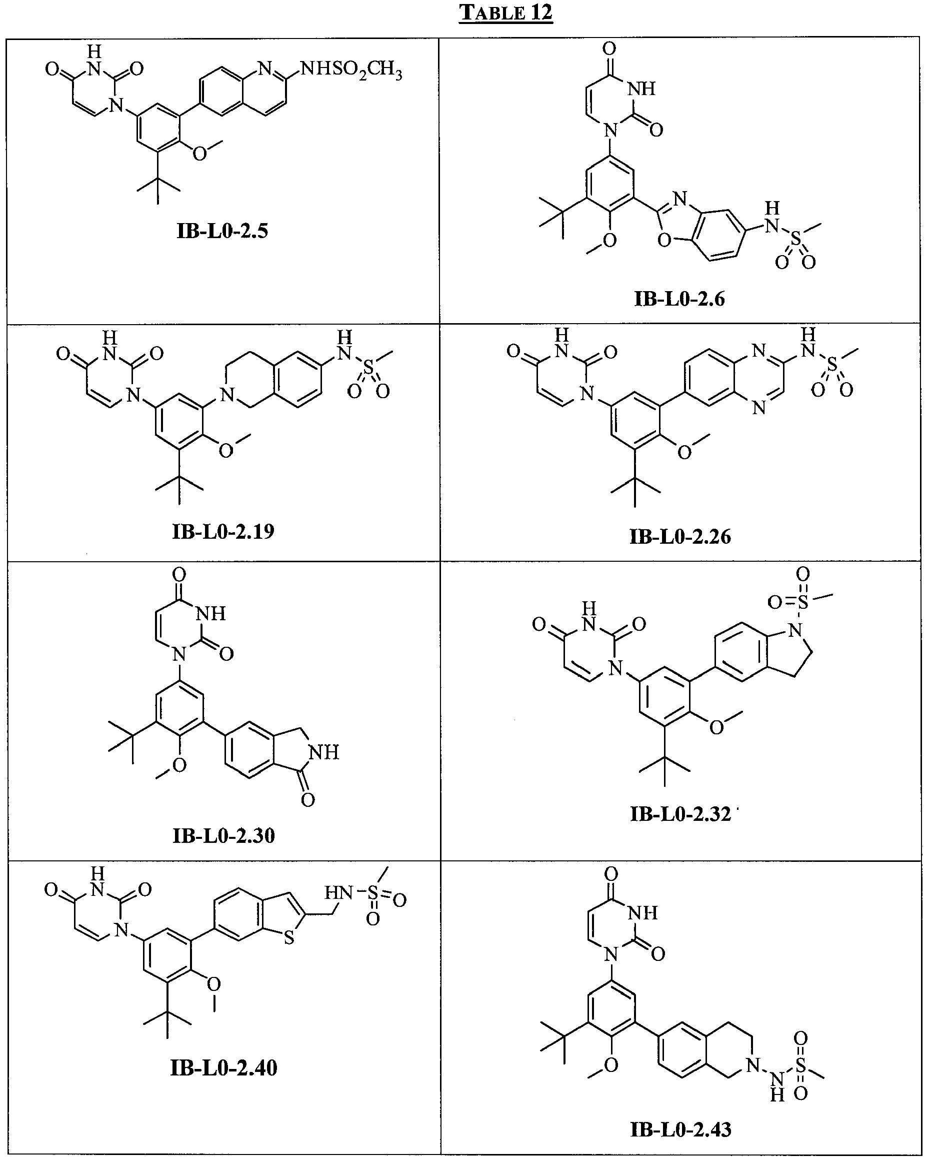

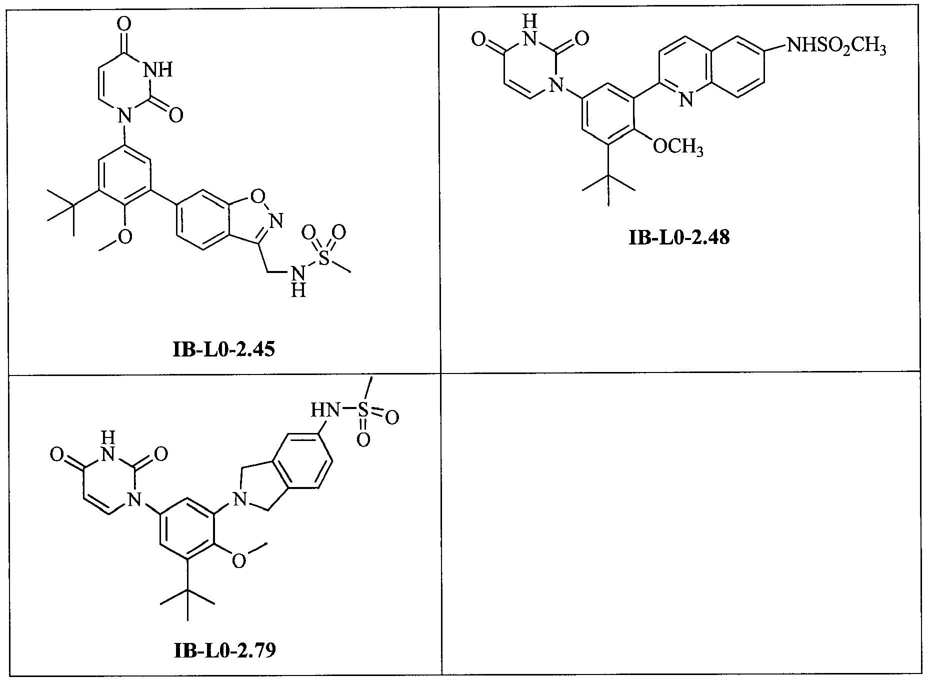

**TABLE 13**

| | | | |
|---|---|---|---|
| 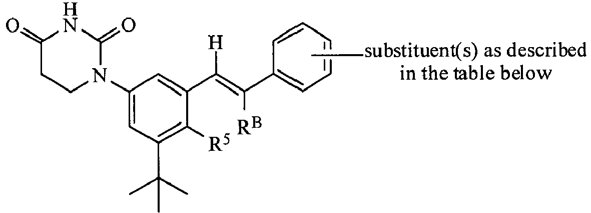 | | | |

| **compound** | **R⁵** | **R^{B}** | **substituent(s)** |
|---|---|---|---|
| | | | |
| **IA-L1-1.3** | -OCH₃ | -Cl | -4-N(H)S(O)₂CH₃ [Z] |
| **IA-L1-1.4** | -OCH₃ | -F | -4-N(H)S(O)₂CH₃ [Z] |
| **IA-L1-1.5** | -OCH₃ | -F | -4-N(H)S(O)₂CH₃ [E] |
| **IA-L1-1.6** | -OCH₃ | -CH₃ | -4-N(H)S(O)₂CH₃ [E] |
| **IA-L1-1.9** | -OCH₃ | -H | -4-N(H)S(O)₂CH₃ [E] |
| **IA-L1-1.10** | -OCH₃ | -H | -4-N(H)S(O)₂CH₃ [Z] |
| **IA-L1-1.11** | -OCH₃ | -H | -4-N[C(O)CH₃]S(O)₂CH₃ [E] |
| **IA-L1-1.12** | -OCH₃ | -H | -4-F [E] |
| **IA-L1-1.13** | -OCH₃ | -H | -4-NH₂ [E] |
| **IA-L1-1.14** | -OCH₃ | -H | -4-OCH₃ [E] |
| **IA-L1-1.16** | -H | -H | -4-N(H)S(O)₂CH₃ [E] |
| **IA-L1-1.17** | -OCH₃ | -OCH₃ | -4-N(H)S(O)₂CH₃ [Z] |
| **IA-L1-1.18** | -OCH₃ | -H | --- [E] |
| **IA-L1-1.20** | -OCH₃ | -H | -4-N(H)S(O)₂CH₃ [Z] |
| **IA-L1-1.21** | -OCH₃ | -F | -4-N(H)S(O)₂CH₃ [Z]:[E] (1:1) |
| **IA-L1-1.22** | -OCH₃ | -H | -4-NO₂ [E] |
| **IA-L1-1.23** | -OCH₃ | -Cl | -4-NO₂ [Z] |
| **IA-L1-1.24** | -OCH₃ | -CH₃ | -4-NO₂ [E] |
| **IA-L1-1.25** | -H | -H | -4-NO₂ [E] |
| **IA-L1-1.26** | -OCH₃ | -H | -3-F and -4-N(H)S(O)₂CH₃ [E] |
| **IA-L1-1.27** | -OCH₃ | -H | -2-OCH₃ and -4-N(H)S(O)₂CH₃ [E] |

**TABLE 14**

| | | |
|---|---|---|
| 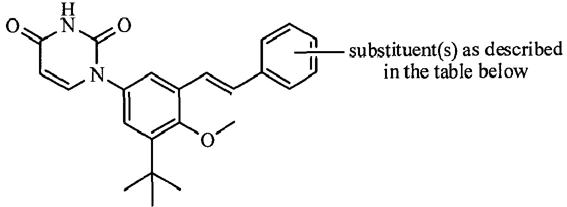 | | |

| **compound** | **substituent(s)** | |
|---|---|---|
| | | |
| **IB-L1-1.1** | -4-N(H)S(O)₂CH₃ [E] | |
| **IB-L1-1.4** | -2-C(O)OH and -4-N(H)S(O)₂CH₃ [E] | |
| **IB-L1-1.5** | -3-F and -4-N(H)S(O)₂CH₃ [E] | |
| **IB-L1-1.6** | -2-C(O)H and -4-N(H)S(O)₂CH₃ [E] | |
| **IB-L1-1.7** | -2-C(O)OCH₃ and -4-N(H)S(O)₂CH₃ [E] | |
| **IB-L1-1.8** | -2-C(H)=N(OH) and -4-N(H)S(O)₂CH₃ [E] | |
| **IB-L1-1.9** | -2-C(O)N(H)CH₂CH₂OCH₃ and -4-N(H)S(O)₂CH₃ [E] | |
| **IB-L1-1.10** | -2-CH₂OH and -4-N(H)S(O)₂CH₃ [E] | |
| **IB-L1-1.11** | -2-C(O)OC(H)₂CH₃ and -4-N(H)S(O)₂CH₃ [E] | |
| **IB-L1-1.13** | -2-C(H)₂OCH₃ and -4-N(H)S(O)₂CH₃ [E] | |
| **IB-L1-1.14** | -2-C(O)N(CH₃)₂ and -4-N(H)S(O)₂CH₃ [E] | |
| **IB-L1-1.15** | -2-CH₃ and -4-N(H)S(O)₂CH₃ and -5-F [E] | |
| **IB-L1-1.16** | imidazol-2-yl and -4-N(H)S(O)₂CH₃ [E] | |
| **IB-L1-1.17** | -2-C(O)N(H)CH₃ and -4-N(H)S(O)₂CH₃ [E] | |
| **IB-L1-1.18** | 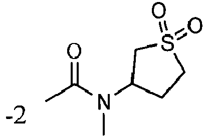 | and -4-N(H)S(O)₂CH₃ [E] |
| **IB-L1-1.19** | -2-C(H)=NOCH₃ and -4-N(H)S(O)₂CH₃ [E] | |
| **IB-L1-1.21** | -2-C(O)NH₂ and -4-N(H)S(O)₂CH₃ [E] | |
| **IB-L1-1.22** | 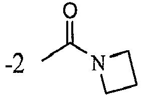 | and -4-N(H)S(O)₂CH₃ [E] |
| **IB-L1-1.23** | 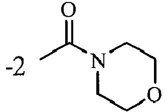 | and -4-N(H)S(O)₂CH₃ [E] |
| **IB-L1-1.24** | -2-C(O)N(CH₃)C(H)₂C(H)₂OCH₃ and -4-N(H)S(O)₂CH₃ [E] | |
| **IB-L1-1.25** | -2-C(H)₂OC(H)(CH₃)₂ and -4-N(H)S(O)₂CH₃ [E] | |
| **IB-L1-1.26** | 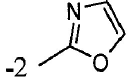 | and -4-N(H)S(O)₂CH₃ [E] |
| **IB-L1-1.27** | 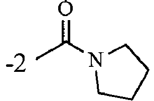 | and -4-N(H)S(O)₂CH₃ [E] |
| **IB-L1-1.28** | -2-NH₂ and -4-N(H)S(O)₂CH₃ [E] | |
| **IB-L1-1.29** | 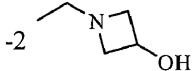 | and -4-N(H)S(O)₂CH₃ [E] |
| **IB-L1-1.31** | -2-C(H)₂N(H)C(H)₂C(H)₂C(H)(CH₃)₂ and -4-N(H)S(O)₂CH₃ [E] | |
| **IB-L1-1.32** | -2-N(H)C(O)OC(CH₃)₃ and -4-N(H)S(O)₂CH₃ [E] | |
| **IB-L1-1.33** | 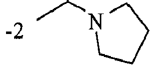 | and -4-N(H)S(O)₂CH₃ [E] |
| **IB-L1-1.34** | -4-N(H)S(O)₂CH₃ [Z] | |

**TABLE 15**

| | |
|---|---|
| 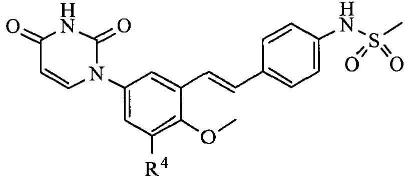 | |

| **compound** | **R⁴** |
|---|---|
| | |
| **IB-L1-1.45** | -C(CH₃)₂C(H)₂OH [E] |
| **IB-L1-1.46** | furan-2-yl [E] |
| **IB-L1-1.47** | 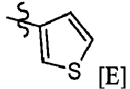 |
| **IB-L1-1.48** | 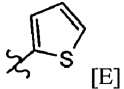 |
| **IB-L1-1.49** | -S(O)₂CH₃ [E] |
| **IB-L1-1.50** | furan-3-yl [E] |
| **IB-L1-1.51** | -I [E] |
| **IB-L1-1.52** | -Br [E] |
| **IB-L1-1.53** | pyridin-3-yl [E] |
| **IB-L1-1.55** | pyridin-4-yl [E] |

**TABLE 16**

| | | |
|---|---|---|
| 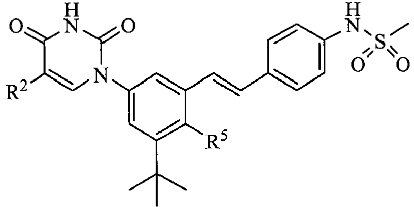 | | |

| **compound** | **R²** | **R⁵** |
|---|---|---|
| | | |
| **IB-L1-1.2** | -F | -OCH₃ [E] |
| **IB-L1-1.12** | -H | -Cl [E] |
| **IB-L1-1.20** | -Cl | -OCH₃ [E] |
| **IB-L1-1.30** | -H | -OCH₂CH₃ [E] |

**TABLE 17**

| | | | |
|---|---|---|---|
| 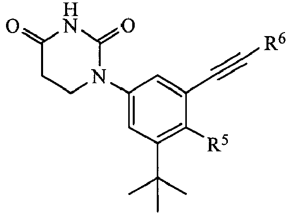 | | | |

| **compound** | **R⁵** | **R⁶** | |
|---|---|---|---|
| | | **ring/ring structure** | **substituent(s)** |
| | | | |
| **IA-L2-1.1** | -H | phenyl | -4-N(H)S(O)₂CH₃ |
| **IA-L2-1.2** | -H | phenyl | -2-CH₃ and -4-N(H)S(O)₂CH₃ |
| **IA-L2-1.3** | -H | phenyl | -2-Cl and -4-N(H)S(O)₂CH₃ |
| **IA-L2-1.4** | -OCH₃ | phenyl | --- |
| **IA-L2-1.26** | -OCH₃ | pyridin-3-yl | -6-N(H)S(O)₂CH₃ |
| **IA-L2-1.7** | -OCH₃ | phenyl | -3-N(H)S(O)₂CH₃ |
| **IA-L2-1.8** | -OCH₃ | phenyl | -4-N(H)S(O)₂CH₃ |
| **IA-L2-1.9** | -OCH₃ | phenyl | -2-CH₃ and -4-N(H)S(O)₂CH₃ |
| **IA-L2-1.10** | -OCH₃ | phenyl | -3-CH₃ and -4-N(H)S(O)₂CH₃ |
| **IA-L2-1.11** | -OCH₃ | phenyl | 2-C(H)₂C(H)₃ and -4-N(H)S(O)₂CH₃ |
| **IA-L2-1.12** | -OCH₃ | phenyl | -2-F and -4-N(H)S(O)₂CH₃ |
| **IA-L2-1.13** | -OCH₃ | phenyl | -3-F and -4-N(H)S(O)₂CH₃ |
| **IA-L2-1.14** | -OCH₃ | phenyl | -2-Cl and -4-N(H)S(O)₂CH₃ |
| **IA-L2-1.15** | -OCH₃ | phenyl | -3-Cl and -4-N(H)S(O)₂CH₃ |
| **IA-L2-1.16** | -OCH₃ | phenyl | -2-OCH₃ and -4-N(H)S(O)₂CH₃ |
| **IA-L2-1.17** | -OCH₃ | phenyl | -3-OCF₃ and -4-N(H)S(O)₂CH₃ |
| **IA-L2-1.18** | -OCH₃ | phenyl | -2-CF₃ and -4-N(H)S(O)₂CH₃ |
| **IA-L2-1.19** | -OCH₃ | phenyl | -3-CF₃ and -4-N(H)S(O)₂CH₃ |
| **IA-L2-1.20** | -OCH₃ | phenyl | -2-CH₃ and -4-N(H)S(O)₂CH₃ and -5-F |
| **IA-L2-1.21** | -OCH₃ | phenyl | -2-Cl and -3-F and -4-N(H)S(O)₂CH₃ |
| **IA-L2-1.22** | -OCH₃ | phenyl | -2-CF₃ and -4-N(H)S(O)₂CH₃ and -5-F |
| **IA-L2-1.24** | -OCH₃ | pyridin-2-yl | -3-CH₃ and -5-N(H)S(O)₂CH₃ |
| **IA-L2-1.25** | -OCH₃ | pyridin-2-yl | -5-N(H)S(O)₂CH₃ |
| **IA-L2-1.26** | -OCH₃ | pyridin-3-yl | -6-N(H)S(O)₂CH₃ [C(F)₃C(O)OH salt] |

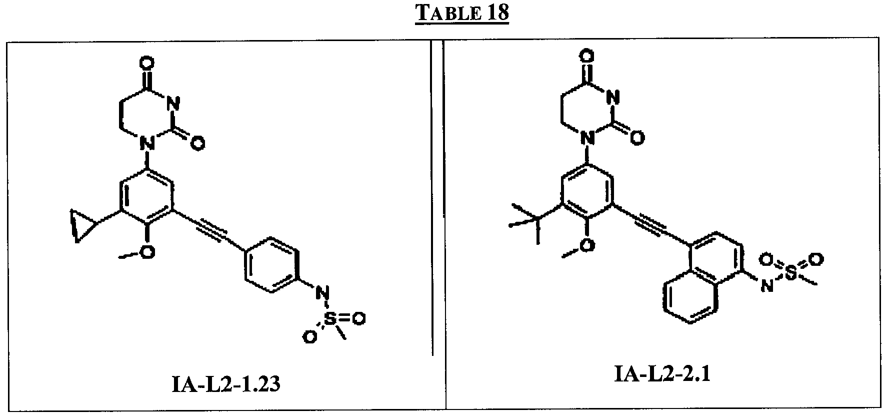

**TABLE 19**

| | | | |
|---|---|---|---|
| 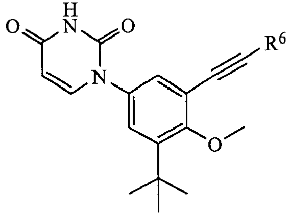 | | | |

| **compound** | **R⁶** | | |
|---|---|---|---|
| | **ring/ring structure** | **substituent(s)** | |
| | | | |
| **IB-L2-1.1** | phenyl | -2-CH₃ and -4-N(H)S(O)₂CH₃ | |
| **IB-L2-1.2** | phenyl | -2-Cl and -4-N(H)S(O)₂CH₃ | |
| **IB-L2-1.3** | phenyl | -2-CH₃ and -4-N(H)S(O)₂CH₃ and -5-F | |
| **IB-L2-1.4** | phenyl | -3-F and -4-N(H)S(O)₂CH₃ and -5-F | |
| **IB-L2-1.5** | phenyl | -2-CF₃ and -4-N(H)S(O)₂CH₃ and -5-F | |
| **IB-L2-1.6** | phenyl | -2-OH and -4-N(H)S(O)₂CH₃ | |
| **IB-L2-1.7** | phenyl | -2-C(O)OCH₃ and -4-N(H)S(O)₂CH₃ | |
| **IB-L2-1.8** | pyridin-2-yl | -5-N(H)S(O)₂CH₃ | |
| **IB-L2-1.9** | pyrazin-2-yl | -5-N(H)S(O)₂CH₃ | |
| **IB-L2-1.10** | phenyl | -2-C(CH₃)₃ and -4-N(H)S(O)₂CH₃ | |
| **IB-L2-1.11** | phenyl | 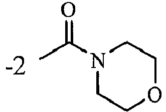 | and -4-N(H)S(O)₂CH₃ |
| **IB-L2-1.12** | phenyl | -2-N(H)C(O)CH₃ and -4-N(H)S(O)₂CH₃ | |

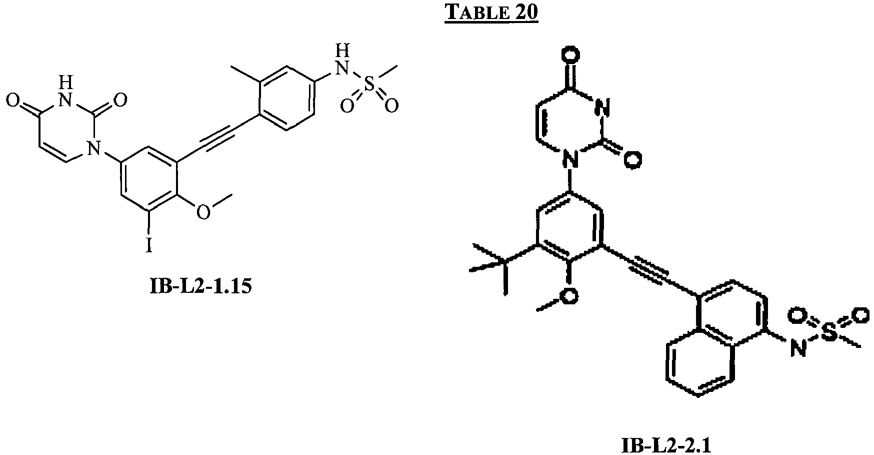

**TABLE 21**

| | | |
|---|---|---|
| 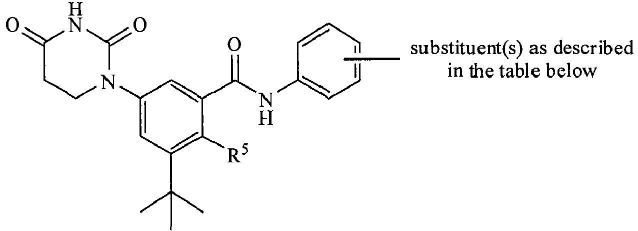 | | |

| **compound** | **R⁵** | **substituent(s)** |
|---|---|---|
| | | |
| **IA-L3-1.3** | -OH | --- |
| **IA-L3-1.4** | -OH | -4-N(CH₃)S(O)₂CH₃ |
| **IA-L3-1.5** | -OH | -3-N(H)S(O)₂CH₃ |
| **IA-L3-1.6** | -OH | -4-N(H)S(O)₂CH₃ |
| **IA-L3-1.7** | -OH | -4-N(H)S(O)₂CH₂CH(CH₃)₂ |
| **IA-L3-1.8** | -OH | -4-N(H)S(O)₂CH₂CH₂OCH₃ |
| **IA-L3-1.9** | -OH | -4-N(H)S(O)₂CH₂CF₃ |
| **IA-L3-1.10** | -OH | -4-N(H)S(O)₂phenyl |
| **IA-L3-1.11** | -OH | -3-C(H)₂N(H)S(O)₂CH₃ |
| **IA-L3-1.12** | -OH | -4-N(H)S(O)₂CH₂phenyl |
| **IA-L3-1.13** | -OH | 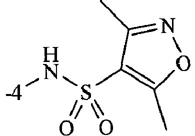 |
| **IA-L3-1.14** | -OH | 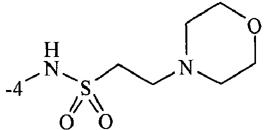 |
| **IA-L3-1.15** | -OH | -4-C(H)₂C(O)OH |
| **IA-L3-1.16** | -OH | -4-C(H)₂S(O)₂N(H)CH₃ |
| **IA-L3-1.17** | -OH | -4-OC(H)₂C≡N |
| **IA-L3-1.18** | -OH | -4-OC(H)₂C(O)NH₂ |
| **IA-L3-1.19** | -OH | -4-OS(O)₂CH₃ |
| **IA-L3-1.20** | -OH | -2-S(O)₂CH₃ |
| **IA-L3-1.21** | -OH | -4-S(O)₂CH₃ |
| **IA-L3-1.22** | -OH | -2-S(O)₂NH₂ |
| **IA-L3-1.24** | -OCH₃ | --- |
| **IA-L3-1.28** | -OCH₃ | -2-C(H)₂CH₃ |
| **IA-L3-1.30** | -OCH₃ | -4-C(H)₂C(O)OH |
| **IA-L3-1.31** | -OCH₃ | -4-C(H)₂S(O)₂N(H)CH₃ |
| **IA-L3-1.32** | -OCH₃ | -4-CF₃ |
| **IA-L3-1.33** | -OCH₃ | -4-OH |
| **IA-L3-1.34** | -OCH₃ | -2-OCH₃ |
| **IA-L3-1.35** | -OCH₃ | -3-OCH₃ |
| **IA-L3-1.36** | -OCH₃ | -4-OCH₃ |
| **IA-L3-1.37** | -OCH₃ | -2-OC(H)₂CH₃ |
| **IA-L3-1.38** | -OCH₃ | -4-OC(H)₂C≡N |
| **IA-L3-1.39** | -OCH₃ | -4-OC(H)₂C(O)NH₂ |
| **IA-L3-1.40** | -OCH₃ | -4-OCF₃ |
| **IA-L3-1.41** | -OCH₃ | -4-OS(O)₂CH₃ |
| **IA-L3-1.42** | -OCH₃ | -4-C(O)CH₃ |
| **IA-L3-1.43** | -OCH₃ | -4-C(O)C(H)₂C(O)OC(H)₂CH₃ |
| **IA-L3-1.44** | -OCH₃ | -3-C(O)NH₂ |
| **IA-L3-1.45** | -OCH₃ | -4-F |
| **IA-L3-1.46** | -OCH₃ | -4-Cl |
| **IA-L3-1.47** | -OCH₃ | -4-N(H)C(O)CH₃ |
| **IA-L3-1.48** | -OCH₃ | -4-N(H)C(O)C(H)₂OC(O)CH₃ |
| **IA-L3-1.49** | -OCH₃ | -4-N(H)C(O)OCH₃ |
| **IA-L3-1.50** | -OCH₃ | -4-N(H)C(O)OC(CH₃)₃ |
| **IA-L3-1.51** | -OCH₃ | -4-N(H)S(O)₂CH₃ |
| **IA-L3-1.52** | -OCH₃ | -4-N(H)S(O)₂C(H)₂CH(CH₃)₂ |
| **IA-L3-1.53** | -OCH₃ | -4-N(H)S(O)₂C(H)₂C(H)₂OH |
| **IA-L3-1.54** | -OCH₃ | -4-N(H)S(O)₂C(H)₂C(H)₂OCH₃ |
| **IA-L3-1.55** | -OCH₃ | -4-N(H)S(O)₂C(H)₂CF₃ |
| **IA-L3-1.56** | -OCH₃ | -4-N(H)S(O)₂C(H)₂C(H)₂N[C(H)₂C(H)₂OH]₂ |
| **IA-L3-1.57** | -OCH₃ | -4-N(H)S(O)₂C(H)₂phenyl |
| **IA-L3-1.58** | -OCH₃ | 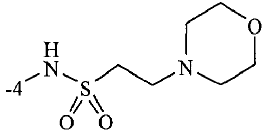 |
| **IA-L3-1.59** | -OCH₃ | 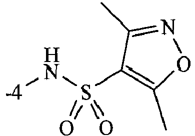 |
| **IA-L3-1.60** | -OCH₃ | -4-N(H)S(O)₂phenyl |
| **IA-L3-1.62** | -OCH₃ | -4-N(CH₃)S(O)₂CH₃ |
| **IA-L3-1.63** | -OCH₃ | -4-N[C(H)₂CH₃]S(O)₂CH₃ |
| **IA-L3-1.64** | -OCH₃ | -4-N[C(H)₂OC(O)CH₃]S(O)₂CH₃ |
| **IA-L3-1.65** | -OCH₃ | -4-N[C(H)₂OC(O)C(CH₃)₃]S(O)₂CH₃ |
| **IA-L3-1.66** | -OCH₃ | 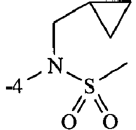 |
| **IA-L3-1.67** | -OCH₃ | 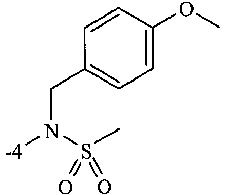 |
| **IA-L3-1.69** | -OCH₃ | -4-N[C(O)CH₃]S(O)₂CH₃ |
| **IA-L3-1.70** | -OCH₃ | -4-N[C(O)C(H)₂CH₃]S(O)₂CH₃ |
| **IA-L3-1.71** | -OCH₃ | -4-N[C(O)C(H)₂C(H)₂CH₃]S(O)₂CH₃ |
| **IA-L3-1.72** | -OCH₃ | -4-N[C(O)C(H)(CH₃)₂]S(O)₂CH₃ |
| **IA-L3-1.73** | -OCH₃ | -4-N[C(O)OCH₃]S(O)₂CH₃ |
| **IA-L3-1.74** | -OCH₃ | -4-N[C(O)OC(H)₂CH₃]S(O)₂CH₃ |
| **IA-L3-1.76** | -OCH₃ | -4-N[C(O)OC(H)₂C(H)(CH₃)₂]S(O)₂CH₃ |
| **IA-L3-1.77** | -OCH₃ | -4-N[C(O)OC(H)₂C(H)₂OCH₃]S(O)₂CH₃ |
| **IA-L3-1.78** | -OCH₃ | -4-S(O)₂CH₃ |
| **IA-L3-1.79** | -OCH₃ | -2-S(O)₂CH₃ |
| **IA-L3-1.80** | -OCH₃ | -2-S(O)₂NH₂ |
| **IA-L3-1.81** | -OCH₃ | -2-CH₃ and 3-OH |
| **IA-L3-1.82** | -OCH₃ | -2-CH₃ and -4-F |
| **IA-L3-1.83** | -OCH₃ | -2-CH₃ and -4-N(H)S(O)₂CH₃ |
| **IA-L3-1.84** | -OCH₃ | -2-CF₃ and -4-N(H)S(O)₂CH₃ |
| **IA-L3-1.85** | -OCH₃ | -2-OCH₃ and -4-N(H)S(O)₂CH₃ |
| **IA-L3-1.86** | -OCH₃ | -2-OCH₃ and -5-N(H)C(O)CH₃ |
| **IA-L3-1.87** | -OCH₃ | -3-NO₂ and -4-N(H)S(O)₂CH₃ |
| **IA-L3-1.105** | -H | -4-N(H)S(O)₂CH₃ |
| **IA-L3-1.107** | -OCH₃ | -4-N(H)₂ [Cl-] |
| **IA-L3-1.108** | -OCH₃ | -4-N[C(H)₂C(H)=C(H)₂]S(O)₂CH₃ |
| **IA-L3-1.119** | -OH | -2-OCH₃ and -4-N(H)S(O)₂CH₃ |
| **IA-L3-1.120** | -OCH₃ | -3-C(H)₂N(H)S(O)₂CH₃ |
| **IA-L3-1.121** | -OCH₃ | 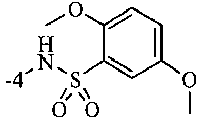 |
| **IA-L3-1.122** | -OCH₃ | -4-N(H)S(O)₂C(H)₂C(H)₂OH |
| **IA-L3-1.123** | -OCH₃ | -4-N[C(H)₂OC(O)C(H)₂C(H)₂CH₃]S(O)₂CH₃ |

**TABLE 22**

| | | | |
|---|---|---|---|
| 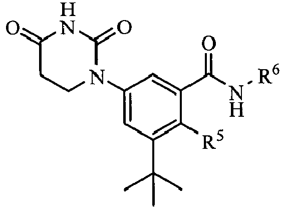 | | | |

| **compound** | **R⁵** | | **R⁶** |
|---|---|---|---|
| | | **ring/ring structure** | **substituent(s)** |
| **IA-L3-1.89** | -OH | thiophen-2-yl | -3-S(O)₂NH₂ and -4-C(H)₂N(H)S(O)₂CH₃ |
| **IA-L3-1.90** | -OCH₃ | thiophen-2-yl | -3-S(O)₂NH₂ and -4-C(H)₂N(H)S(O)₂CH₃ |
| **IA-L3-1.91** | -OH | thiophen-2-yl | -4-C(H)₂N(H)S(O)₂CH₃ |
| **IA-L3-1.94** | -OCH₃ | thiophen-2-yl | -4-C(H)₂N(H)S(O)₂CH₃ |
| **IA-L3-1.95** | -OH | thiophen-2-yl | -4-C(H)₂N(CH₃)S(O)₂CH₃ |
| **IA-L3-1.96** | -OCH₃ | thiophen-2-yl | -4-C(H)₂N[C(O)OC(CH₃)₃]S(O)₂CH₃ |
| **IA-L3-1.97** | -OCH₃ | thiophen-2-yl | 4-C(H)₂N(CH₃)S(O)₂CH₃ |
| **IA-L3-1.98** | -OH | thiophen-2-yl | -4-C(H)₂N[C(O)OC(CH₃)₃] S(O)₂CH₃ |
| **IA-L3-1.99** | -OCH₃ | pyridin-3-yl | -6-N(H)S(O)₂CH₃ |
| **IA-L3-1.100** | -OCH₃ | pyridin-3-yl | -6-N(H)₂ [Cl⁻] |
| **IA-L3-1.101** | -OCH₃ | pyridin-2-yl | -5-N[S(O)₂CH₃]₂ |
| **IA-L3-1.102** | -OCH₃ | pyridin-2-yl | -5-N(H)S(O)₂CH₃ |
| **IA-L3-1.103** | -OCH₃ | cyclohexyl | -4-N(H)S(O)₂CH₃ |
| **IA-L3-1.104** | -OH | thiazol-2-yl | |

**TABLE 23**

| | | | | |
|---|---|---|---|---|
| 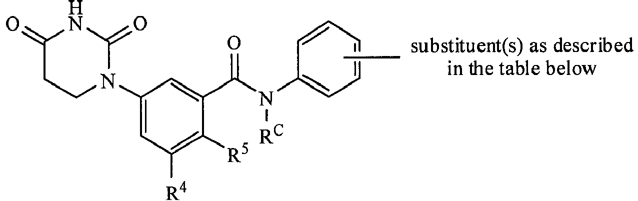 | | | | |

| **compound** | **R⁴** | **R⁵** | **R^{C}** | **substituent(s)** |
|---|---|---|---|---|
| | | | | |
| **IA-L3-1.1** | -C(CH₃)₃ | -OH | -CH₃ | ---- |
| **IA-L3-1.2** | -C(CH₃)₃ | -OH | -CH₃ | -4-N(H)S(O)₂CH₃ |
| **IA-L3-1.27** | -C(CH₃)₃ | -OCH₃ | -CH₃ | -4-N(H)S(O)₂CH₃ |
| **IA-L3-1.111** | -C(H)(CH₃)₂ | -OCH₃ | -H | -4-N(H)S(O)₂CH₃ |
| **IA-L3-1.112** | -CH₂CH₃ | -OCH₃ | -H | -4-N(H)S(O)₂CH₃ |
| **IA-L3-1.113** | -CH₂CH₃ | -OH | -H | -4-N(H)S(O)₂CH₃ |
| **IA-L3-1.114** | -H | -OH | -H | -4-N(H)S(O)₂CH₃ |

**TABLE 24**

| | | |
|---|---|---|
| 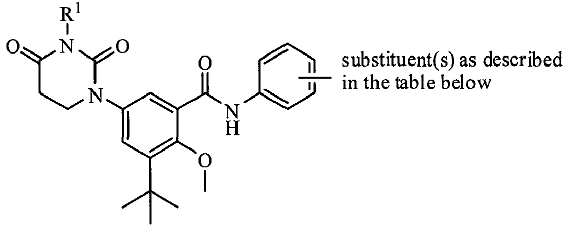 | | |

| **compound** | **R¹** | **substituent(s)** |
|---|---|---|
| | | |
| **IA-L3-1.88** | -C(H)₂OC(O)C(H)₂C(H)₂CH₃ | -4-N[C(H)₂OC(O)CH₂CH₂CH₃]S(O)₂CH₃ |
| **IA-L3-1.25** | -CH₃ | -4-N(H)S(O)₂CH₃ |
| **IA-L3-1.26** | -CH₃ | -4-N(CH₃)S(O)₂CH₃ |
| **IA-L3-1.115** | -C(H)₂C(O)N(H)CH₂CH₃ | -4-N[C(H)₂C(O)N(H)CH₂CH₃]S(O)₂CH₃ |
| **IA-L3-1.116** | -C(H)₂OC(O)C(CH₃)₃ | -4-N[C(H)₂OC(O)C(CH₃)₃]S(O)₂CH₃ |
| **IA-L3-1.117** | 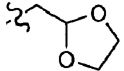 | -4-N(H)S(O)₂CH₃ |
| **IA-L3-1.118** | -CH₂C=CH₂ | -4-N[C(H)₂C=CH₂]S(O)₂CH₃ |

**TABLE 25**

| | | |
|---|---|---|
| 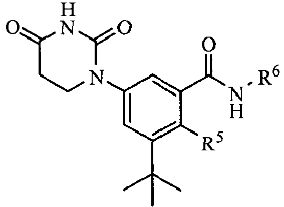 | | |

| **compound** | **R⁵** | **R⁶** |
|---|---|---|
| | | |
| **IA-L3-2.1** | -OH | 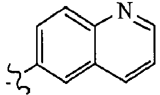 |
| **IA-L3-2.2** | -OCH₃ | 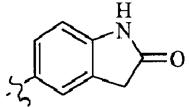 |
| **IA-L3-2.3** | -OCH₃ | 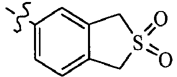 |
| **IA-L3-2.4** | -OCH₃ | 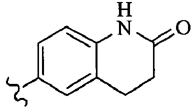 |
| **IA-L3-2.5** | -OH | 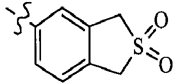 |

**TABLE 26**

| | | |
|---|---|---|
| 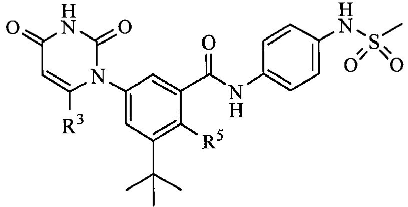 | | |

| **compound** | **R³** | **R⁵** |
|---|---|---|
| | | |
| **IB-L3-1.1** | -H | -OCH₃ |
| **IB-L3-1.2** | -H | -OH |
| **IB-L3-1.3** | -CH₃ | -OCH₃ |
| **IB-L3-1.4** | -CH₃ | -OH |

**TABLE 27**

| | | | |
|---|---|---|---|
| 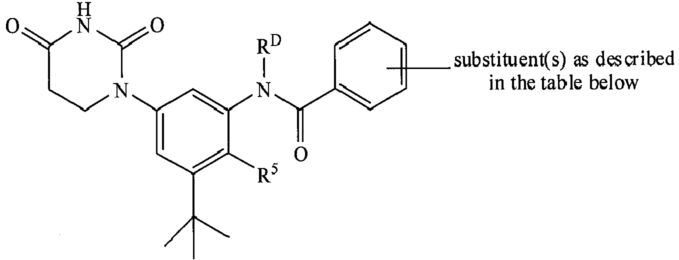 | | | |

| **compound** | **R⁵** | **R^{D}** | **substituent(s)** |
|---|---|---|---|
| | | | |
| **IA-L4-1.1** | -H | -H | -4-N(H)S(O)₂CH₃ |
| **IA-L4-1.2** | -OH | -H | --- |
| **IA-L4-1.3** | -OH | -H | -4-N(H)S(O)₂CH₃ |
| **IA-L4-1.4** | -OH | -H | -4-CH₂S(O)₂CH₃ |
| **IA-L4-1.5** | -OH | -H | -4-NO₂ |
| **IA-L4-1.6** | -OH | -H | -4-NH₂ |
| **IA-L4-1.7** | -OCH₃ | -CH₃ | -4-N(H)S(O)₂CH₃ |
| **IA-L4-1.8** | -OCH₃ | -H | --- |
| **IA-L4-1.9** | -OCH₃ | -H | -4-N(H)S(O)₂CH₃ |
| **IA-L4-1.10** | -OCH₃ | -H | -4-CH₂S(O)₂CH₃ |
| **IA-L4-1.11** | -OCH₃ | -H | -2-OCH₃ and -4-N(H)S(O)₂CH₃ |
| **IA-L4-1.12** | -OCH₃ | -H | -2-Cl and -4-N(H)S(O)₂CH₃ |

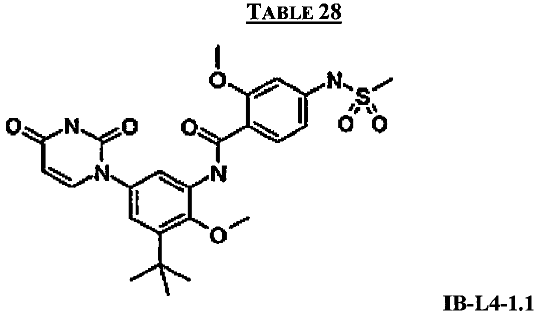

**TABLE 29**

| | | |
|---|---|---|
| 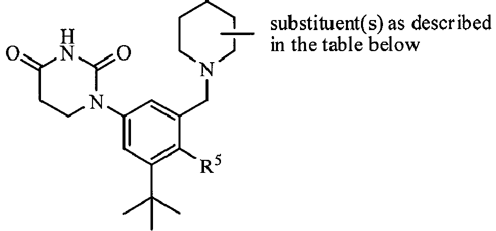 | | |

| **compound** | **R⁵** | **substituent(s)** |
|---|---|---|
| | | |
| **IA-L5-1-1.1** | -OH | -4-N(H)C(O)OC(CH₃)₃ |
| **IA-L5-1-1.2** | -OH | -3-C(H)₂N(H)S(O)₂CH₃ |
| **IA-L5-1-1.3** | -OH | -3-C(O)OC(H)₂CH₃ |
| **IA-L5-1-1.4** | -OH | -3-CH₃ [C(F)₃C(O)O⁻] |
| **IA-L5-1-1.5** | -OH | -4-C(O)OCH₃ |
| **IA-L5-1-1.6** | -OH | -3-OH [C(F)₃C(O)O⁻] |
| **IA-L5-1-1.7** | -OH | -3-C(O)N[C(H)₂CH₃]₂ [C(F)₃C(O)O⁻] |
| **IA-L5-1-1.8** | -OH | -3-C(O)NH₂ [C(F)₃C(O)O⁻] |

**TABLE 30**

| | | | |
|---|---|---|---|
| 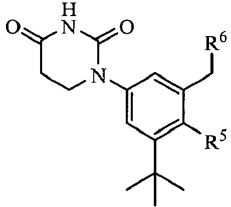 | | | |

| **compound** | **R⁵** | **R⁶** | |
|---|---|---|---|
| | | **ring/ring structure** | **substituent(s)** |
| | | | |
| **IA-L5-1-1.10** | -OH | piperazin-1-yl | -4-C(O)OC(CH₃)₃ |
| **IA-L5-1-1.11** | -OH | pyrrolidin-1-yl | -3-N(H)S(O)₂CH₃ |
| **IA-L5-1-1.12** | -OH | pyrrolidin-1-yl | -3-N(H)C(O)OC(CH₃)₃ |
| **IA-L5-1-1.13** | -OH | morpholin-4-yl | -2-C(H)₃ and -6-C(H)₃ [C(F)₃C(O)O⁻] |
| **IA-L5-1-1.14** | -OH | morpholin-4-yl | ---- [C(F)₃C(O)O⁻] |

**TABLE 31**

| | | |
|---|---|---|
| 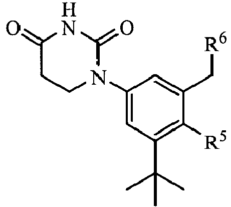 | | |

| **compound** | **R⁵** | **R⁶** |
|---|---|---|
| | | |
| **IA-L5-1-2.1** | -OCH₃ | 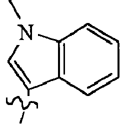 |
| **IA-L5-1-2.2** | -OH | 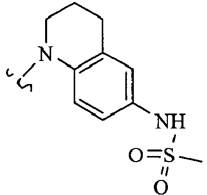 |

**TABLE 32**

| | |
|---|---|
| 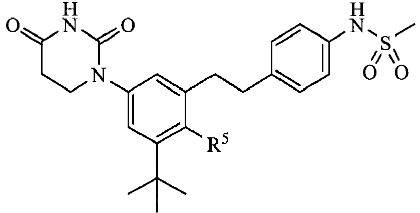 | |

| **compound** | **R⁵** |
|---|---|
| | |
| **IA-L5-2-1.1** | -OCH₃ |
| **IA-L5-2-1.2** | -H |

**TABLE 33**

| | |
|---|---|
| 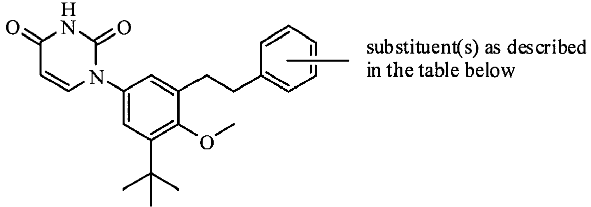 | |

| **compound** | **substituent(s)** |
|---|---|
| **IB-L5-2-1.1** | -2-C(O)OCH₃ and -4-N(H)S(O)₂CH₃ |
| **IB-L5-2-1.2** | -4-N(H)S(O)₂CH₃ |

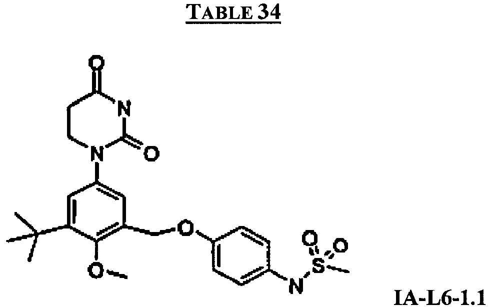

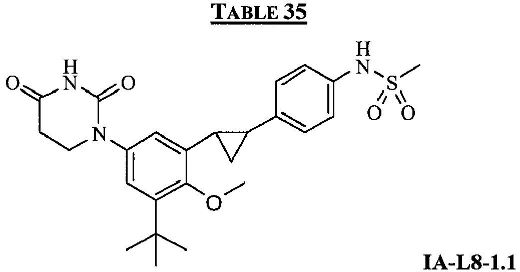

**TABLE 36**

| | | | | |
|---|---|---|---|---|
| 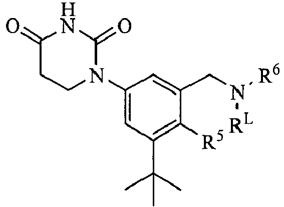 | | | | |

| **compound** | **R^{L}** | **R⁵** | **R⁶** | |
|---|---|---|---|---|
| | | | **ring/ring structure** | **substituent(s)** |
| | | | | |
| **IA-L9-1.1** | -CH₂CH₃ | -OH | cyclohexyl [C(F)₃C(O) O⁻] | ---- |
| **IA-L9-1.2** | -CH₃ | -OH | cyclohexyl [C(F)₃C(O)O⁻] | ---- |
| **IA-L9-1.3** | -H | -OH | phenyl | -4-N(H)S(O)₂CH₃ |
| **IA-L9-1.4** | -H | -OCH₃ | phenyl | -4-N(H)S(O)₂CH₃ |

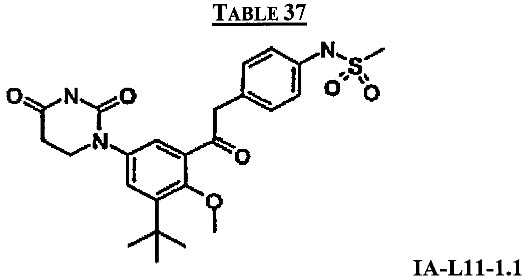

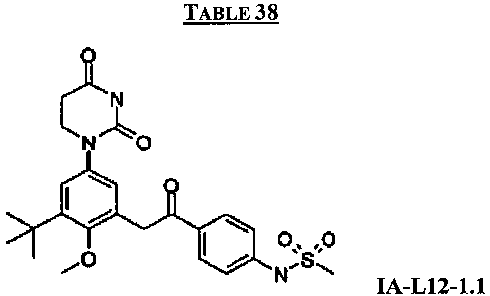

### D. Isomers.

This invention also is directed, in part, to all isomers of the compounds of formula **I** (and their salts) (*i.e.*, structural and stereoisomers). Structural isomers include chain and position isomers. Stereoisomers include E/Z isomers (*i.e.*, isomers with regard to one or more double bonds), enantiomers (*i.e.*, stereo- isomers that have opposite configurations at all stereogenic centers), and diastereoisomers (*i.e.*, stereo- isomers that have the same configuration at one or more stereogenic centers, but differ at other stereogenic centers).

### E. Salts.

This invention also is directed, in part, to all salts of the compounds of formula **I**. A salt of a compound may be advantageous due to one or more of the salt's properties, such as, for example, enhanced pharmaceutical stability in differing temperatures and humidities, or a desirable solubility in water or other solvents. Where a salt is intended to be administered to a patient (as opposed to, for example, being in use in an *in vitro* context), the salt preferably is pharmaceutically acceptable and/or physiologically compatible. The term "pharmaceutically acceptable" is used adjectivally in this patent application to mean that the modified noun is appropriate for use as a pharmaceutical product or as a part of a pharmaceutical product. Pharmaceutically acceptable salts include salts commonly used to form alkali metal salts and to form addition salts of free acids or free bases. In general, these salts typically may be prepared by conventional means by reacting, for example, the appropriate acid or base with a compound of the invention.

Pharmaceutically acceptable acid addition salts of the compounds of formula **I** can be prepared from an inorganic or organic acid. Examples of often suitable inorganic acids include hydrochloric, hydrobromic, hydroiodic, nitric, carbonic, sulfuric, and phosphoric acid. Suitable organic acids generally include, for example, aliphatic, cycloaliphatic, aromatic, araliphatic, heterocyclic, carboxylic, and sulfonic classes of organic acids. Specific examples of often suitable organic acids include acetate, trifluoroacetate, formate, propionate, succinate, glycolate, gluconate, digluconate, lactate, malate, tartaric acid, citrate, ascorbate, glucuronate, maleate, fumarate, pyruvate, aspartate, glutamate, benzoate, anthranilic acid, mesylate, stearate, salicylate, p-hydroxybenzoate, phenylacetate, mandelate, embonate (pamoate), ethanesulfonate, benzenesulfonate, pantothenate, 2-hydroxyethanesulfonate, sulfanilate, cyclohexylaminosulfonate, algenic acid, beta-hydroxybutyric acid, galactarate, galacturonate, adipate, alginate, bisulfate, butyrate, camphorate, camphorsulfonate, cyclopentanepropionate, dodecylsulfate, glycoheptanoate, glycerophosphate, heptanoate, hexanoate, nicotinate, oxalate, palmoate, pectinate, 2-naphthalesulfonate, 3-phenylpropionate, picrate, pivalate, thiocyanate, tosylate, and undecanoate.

Pharmaceutically acceptable base addition salts of the compounds of formula **I** include, for example, metallic salts and organic salts. Preferred metallic salts include alkali metal (group Ia) salts, alkaline earth metal (group IIa) salts, and other physiologically acceptable metal salts. Such salts may be made from aluminum, calcium, lithium, magnesium, potassium, sodium, and zinc. Preferred organic salts can be made from amines, such as tromethamine, diethylamine, N,N'-dibenzylethylenediamine, chloroprocaine, choline, diethanolamine, ethylenediamine, meglumine (N-methylglucamine), and procaine. Basic nitrogen-containing groups can be quaternized with agents such as lower alkyl (C₁-C₆) halides (*e.g.*, methyl, ethyl, propyl, and butyl chlorides, bromides, and iodides), dialkyl sulfates (*e.g.*, dimethyl, diethyl, dibutyl, and diamyl sulfates), long chain halides (*e.g.*, decyl, lauryl, myristyl, and stearyl chlorides, bromides, and iodides), arylalkyl halides (*e.g.*, benzyl and phenethyl bromides), and others.

In some embodiments, the salt is sodium salt of N-(6-(3-tert-butyl-5-(2,4-dioxo-3,4-dihydro-pyrimidin-1(2H)-yl)-2-methoxyphenyl)naphthalen-2-yl)methanesulfonamide.

In some embodiments, the salt is monosodium salt of N-(6-(3-tert-butyl-5-(2,4-dioxo-3,4-dihydropyrimidin-1(2H)-yl)-2-methoxyphenyl)naphthalen-2-yl)methanesulfonamide.

In some embodiments, the salt is disodium salt of N-(6-(3-tert-butyl-5-(2,4-dioxo-3,4-dihydro-pyrimidin-1 (2H)-yl)-2-methoxyphenyl)naphthalen-2-yl)methanesulfonamide.

In some embodiments, the salt is potassium salt of N-(6-(3-tert-butyl-5-(2,4-dioxo-3,4-dihydro-pyrimidin-1 (2H)-yl)-2-methoxyphenyl)naphthalen-2-yl)methanesulfonamide.

In some embodiments, the salt is monopotassium salt of N-(6-(3-tert-butyl-5-(2,4-dioxo-3,4-dihydropyrimidin- (2H)-yl)-2-methoxyphenyl)naphthalen-2-yl)methanesulfonamide:

In some embodiments, the salt is choline salt of N-(6-(3-tert-butyl-5-(2,4-dioxo-3,4-dihydro-pyrimidin-1 (2H)-yl)-2-methoxyphenyl)naphthalen-2-yl)methanesulfonamide.

In some embodiments, the salt is monocholine salt of N-(6-(3-tert-butyl-5-(2,4-dioxo-3,4-dihydropyrimidin-1 (2H)-yl)-2-methoxyphenyl)naphthalen-2-yl)methanesulfonamide.

In some embodiments, the salt is sodium salt of (E)-N-(4-(3-tert-butyl-5-(2,4-dioxo-3,4-dihydro-pyrimidin-1 (2H)-yl)-2-methoxystyryl)phenyl)methanesulfonamide.

In some embodiments, the salt is disodium salt of (E)-N-(4-(3-tert-butyl-5-(2,4-dioxo-3,4-dihydropyrimidin-1(2H)-yl)-2-methoxystyryl)phenyl)methanesulfonamide.

In some embodiments, the salt is potassium salt of (E)-N-(4-(3-tert-butyl-5-(2,4-dioxo-3,4-dihydropyrimidin-1(2H)-yl)-2-methoxystyryl)phenyl)methanesulfonamide.

In some embodiments, the salt is monopotassium salt of (E)-N-(4-(3-tert-butyl-5-(2,4-dioxo-3,4-dihydropyrimidin-1(2H)-yl)-2-methoxystyryl)phenyl)methanesulfonamide.

### F. Purity.

Compounds of formula **I** (and salts thereof) with any level of purity (including pure and substantially pure) are within the scope of Applicants' invention. The term "substantially pure" in reference to a compound/salt/isomer, means that the preparation/composition containing the compound/salt/isomer contains more than about 85% by weight of the compound/salt/isomer, preferably more than about 90% by weight of the compound/salt/isomer, preferably more than about 95% by weight of the compound/salt/isomer, preferably more than about 97% by weight of the compound/salt/isomer, and preferably more than about 99% by weight of the compound/salt/isomer.

### G. Crystalline Forms of Some Specific Compounds and Salts of The Invention.

### G1. Crystalline Forms of N-(6-(3-Tert-butyl-5-(2,4-dioxo-3,4-dihydropyrimidin-1(2H)-yl)-2-methoxyphenyl)naphthalen-2-yl)methanesulfonamide (compound IB-L0-2.3).

This invention also relates, in part, to crystalline forms of N-(6-(3-tert-butyl-5-(2,4-dioxo-3,4-dihydropyrimidin-1(2H)-yl)-2-methoxyphenyl)naphthalen-2-yl)methanesulfonamide (compound **IB-L0-2.3**), namely the solvate, hydrate, and solvent-free crystalline forms discussed below.

### G1A. IB-L0-2.3 Solvates.

This invention also relates, in part, to an ethanol solvate of compound **IB-L0-2.3**.

In some embodiments, the ethanol solvate has an X-ray powder diffraction pattern comprising one or more peaks selected from the group consisting of 8.3±0.2,9.7±0.2, 10.6±0.2, 13.6±0.2, 17.2±0.2, 19.2±0.2, 22.7±0.2, 26.9±0.2, and 29.4±0.2 degrees two theta (20). In some such embodiments, the ethanol solvate has an X-ray powder diffraction pattern comprising three or more peaks selected from the group consisting of 8.3±0.2, 9.7±0.2, 10.6±0.2, 13.6±0.2, 17.2±0.2, 19.2±0.2, 22.7±0.2, 26.9±0.2, and 29.4±0.2 degrees 2θ. In other such embodiments, the ethanol solvate has an X-ray powder diffraction pattern comprising five or more peaks selected from the group consisting of 8.3±0.2, 9.7±0.2, 10.6±0.2, 13.6±0.2, 17.2±0.2, 19.2±0.2, 22.7±0.2, 26.9±0.2, and 29.4±0.2 degrees 2θ.

In some embodiments, the ethanol solvate has an X-ray powder diffraction pattern comprising one or more peaks selected from the group consisting of 8.3±0.2, 9.7±0.2, 10.0±0.2, 10.6±0.2, 13.6±0.2, 17.2±0.2, 17.5±0.2, 19.2±0.2, 19.4±0.2, 22.7±0.2, 26.9±0.2, and 29.4±0.2 degrees 2θ. In some such embodiments, the ethanol solvate has an X-ray powder diffraction pattern comprising three or more peaks selected from the group consisting of 8.3±0.2, 9.7±0.2, 10.0±0.2, 10.6±0.2, 13.6±0.2, 17.2±0.2, 17.5±0.2, 19.2±0.2, 19.4±0.2, 22.7±0.2, 26.9±0.2, and 29.4±0.2 degrees 2θ. In other embodiments, the ethanol solvate has an X-ray powder diffraction pattern comprising five or more peaks selected from the group consisting of 8.3±0.2, 9.7±0.2, 10.0±0.2, 10.6±0.2, 13.6±0.2, 17.2±0.2, 17.5±0.2, 19.2±0.2, 19.4±0.2, 22.7±0.2, 26.9±0.2, and 29.4±0.2 degrees 2θ.

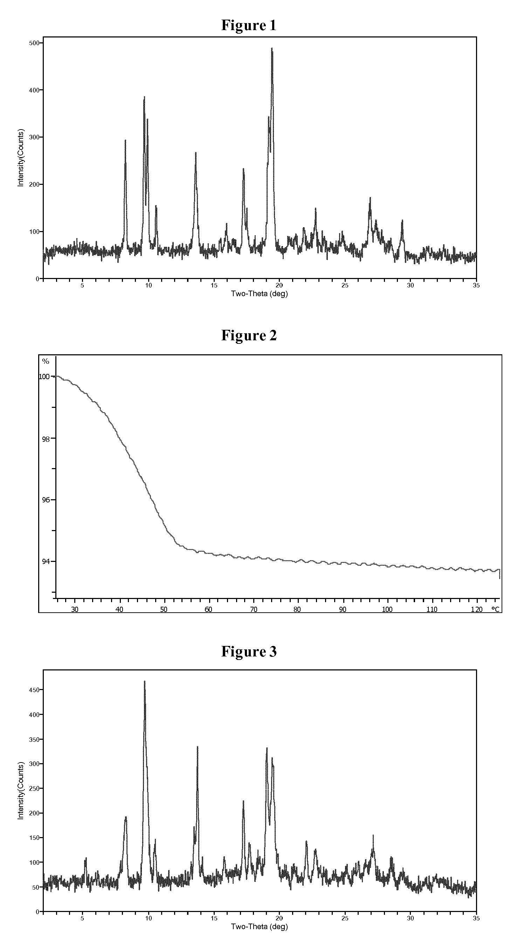

In some embodiments, the ethanol solvate has an X-ray powder diffraction pattern substantially as shown in Figure **1**. The 2θ values for the peaks in Figure **1** (and their intensities) are as follows: 8.25 (54), 9.67 (74), 9.92 (63), 10.59 (21), 13.64 (49), 17.25 (40), 17.51 (20), 19.19 (66), 19.43 (100), 22.75 (19), 26.92 (25), and 29.39 (18).

This invention also relates, in part, to an acetonitrile solvate of compound **IB-L0-2.3**.

In some embodiments, the acetonitrile solvate has an X-ray powder diffraction pattern comprising one or more peaks selected from the group consisting of 5.3±0.2, 8.3±0.2, 9.7±0.2, 10.5±0.2, 13.8±0.2, 17.2±0.2, 19.1±0.2, and 19.5±0.2 degrees 2θ. In some such embodiments, the acetonitrile solvate has an X-ray powder diffraction pattern comprising three or more peaks selected from the group consisting of 5.3±0.2, 8.3±0.2, 9.7±0.2, 10.5±0.2, 13.8±0.2, 17.2±0.2, 19.1±0.2, and 19.5±0.2 degrees 2θ. In other such embodiments, the acetonitrile solvate has an X-ray powder diffraction pattern comprising five or more peaks selected from the group consisting of 5.3±0.2, 8.3±0.2, 9.7±0.2, 10.5±0.2, 13.8±0.2, 17.2±0.2, 19.1±0.2, and 19.5±0.2 degrees 2θ.

In some embodiments, the acetonitrile solvate has an X-ray powder diffraction pattern comprising one or more peaks selected from the group consisting of 5.3±0.2, 8.3±0.2, 9.7±0.2, 10.5±0.2, 13.8±0.2, 17.2±0.2, 17.7±0.2, 19.1±0.2, 19.5±0.2, 22.0±0.2, 22.8±0.2, and 27.2±0.2 degrees 2θ. In some such embodiments, the acetonitrile solvate has an X-ray powder diffraction pattern comprising three or more peaks selected from the group consisting of 5.3±0.2, 8.3±0.2, 9.7±0.2, 10.5±0.2, 13.8±0.2, 17.2±0.2, 17.7±0.2, 19.1±0.2, 19.5±0.2, 22.0±0.2, 22.8±0.2, and 27.2±0.2 degrees 2θ. In other such embodiments, the acetonitrile solvate has an X-ray powder diffraction pattern comprising five or more peaks selected from the group consisting of 5.3±0.2, 8.3±0.2, 9.7±0.2, 10.5±0.2, 13.8±0.2, 17.2±0.2, 17.7±0.2, 19.1±0.2, 19.5±0.2, 22.0±0.2, 22.8±0.2, and 27.2±0.2 degrees 2θ.

In some embodiments, the acetonitrile solvate has an X-ray powder diffraction pattern substantially as shown in Figure **3**. The 2θ values for the peaks in Figure **3** (and their intensities) are as follows: 5.27 (14), 8.29 (33), 9.72 (100), 10.53 (20), 13.77 (67), 17.25 (38), 17.69 (17), 19.05 (63), 19.47 (58), 22.05 (19), 22.75 (16), and 27.17 (21).

This invention also relates, in part, to an ethyl acetate solvate of compound **IB-L0-2.3**.

In some embodiments, the ethyl acetate solvate has an X-ray powder diffraction pattern comprising one or more peaks selected from the group consisting of 7.9±0.2, 9.3±0.2, 9.7±0.2, 10.6±0.2, 18.7±0.2, 38.5±0.2, and 44.7±0.2 degrees 2θ. In some such embodiments, the ethyl acetate solvate has an X-ray powder diffraction pattern comprising three or more peaks selected from the group consisting of 7.9±0.2, 9.3±0.2, 9.7±0.2, 10.6±0.2, 18.7±0.2, 38.5±0.2, and 44.7±0.2 degrees 2θ. In other such embodiments, the ethyl acetate solvate has an X-ray powder diffraction pattern comprising five or more peaks selected from the group consisting of 7.9±0.2, 9.3±0.2, 9.7±0.2, 10.6±0.2, 18.7±0.2, 38.5±0.2, and 44.7±0.2 degrees 2θ.

In some embodiments, the ethyl acetate solvate has an X-ray powder diffraction pattern comprising one or more peaks selected from the group consisting of 7.9±0.2, 9.3±0.2, 9.7±0.2, 10.6±0.2, 13.7±0.2, 17.4±0.2, 18.7±0.2, 21.7±0.2, 22.0±0.2, 28.2±0.2, 38.5±0.2, and 44.7±0.2 degrees 2θ. In some such embodiments, the ethyl acetate solvate has an X-ray powder diffraction pattern comprising three or more peaks selected from the group consisting of 7.9±0.2, 9.3±0.2, 9.7±0.2, 10.6±0.2, 13.7±0.2, 17.4±0.2, 18.7±0.2, 21.7±0.2, 22.0±0.2, 28.2±0.2, 38.5±0.2, and 44.7±0.2 degrees 2θ. In other such embodiments, the ethyl acetate solvate has an X-ray powder diffraction pattern comprising five or more peaks selected from the group consisting of 7.9±0.2, 9.3±0.2, 9.7±0.2, 10.6±0.2, 13.7±0.2, 17.4±0.2, 18.7±0.2, 21.7±0.2, 22.0±0.2, 28.2±0.2, 38.5±0.2, and 44.7±0.2 degrees 2θ.

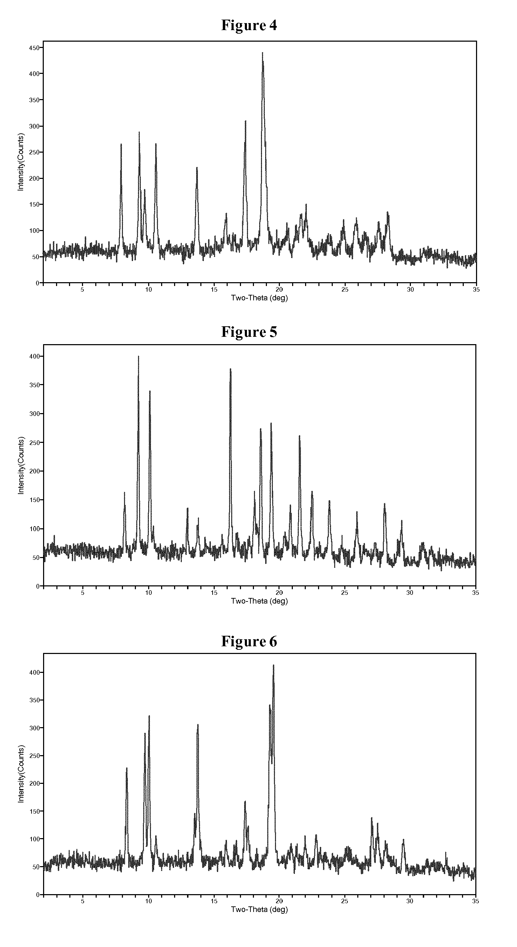

In some embodiments, the ethyl acetate has an X-ray powder diffraction pattern substantially as shown in Figure **4**. The 2θ values for the peaks in Figure **4** (and their intensities) are as follows: 7.94 (24), 9.33 (26), 9.72 (13), 10.58 (23), 13.71 (19), 17.40 (28), 18.72 (44), 21.69 (8), 22.04 (10), 28.23 (8), 38.45 (100), and 44.66 (95).

This invention also relates, in part, to a 2-propanol solvate of compound **IB-L0-2.3**.

In some embodiments, the 2-propanol solvate has an X-ray powder diffraction pattern comprising one or more peaks selected from the group consisting of 8.2±0.2, 9.3±0.2, 10.1±0.2, 16.3±0.2, 18.1±0.2, 18.6±0.2, 19.4±0.2, 21.6±0.2, and 22.5±0.2 degrees 2θ. In some such embodiments, the 2-propanol solvate has an X-ray powder diffraction pattern comprising three or more peaks selected from the group consisting of 8.2±0.2, 9.3±0.2, 10.1±0.2, 16.3±0.2, 18.1±0.2, 18.6±0.2, 19.4±0.2, 21.6±0.2, and 22.5±0.2 degrees 2θ. In other such embodiments, the 2-propanol solvate has an X-ray powder diffraction pattern comprising five or more peaks selected from the group consisting of 8.2±0.2, 9.3±0.2, 10.1±0.2, 16.3±0.2, 18.1±0.2, 18.6±0.2, 19.4±0.2, 21.6±0.2, and 22.5±0.2 degrees 2θ.

In some embodiments, the 2-propanol solvate has an X-ray powder diffraction pattern comprising one or more peaks selected from the group consisting of 8.2±0.2, 9.3±0.2, 10.1±0.2, 16.3±0.2, 18.1±0.2, 18.6±0.2, 19.4±0.2, 21.6±0.2, 22.5±0.2, 23.8±0.2, 26.0±0.2, and 28.0±0.2 degrees 2θ. In some such embodiments, the 2-propanol solvate has an X-ray powder diffraction pattern comprising three or more peaks selected from the group consisting of 8.2±0.2, 9.3±0.2, 10.1±0.2, 16.3±0.2, 18.1±0.2, 18.6±0.2, 19.4±0.2, 21.6±0.2, 22.5±0.2, 23.8±0.2, 26.0±0.2, and 28.0±0.2 degrees 2θ. In other such embodiments, the 2-propanol solvate has an X-ray powder diffraction pattern comprising five or more peaks selected from the group consisting of 8.2±0.2, 9.3±0.2, 10.1±0.2, 16.3±0.2, 18.1±0.2, 18.6±0.2, 19.4±0.2, 21.6±0.2, 22.5±0.2, 23.8±0.2, 26.0±0.2, and 28.0±0.2 degrees 2θ.

In some embodiments, the 2-propanol solvate has an X-ray powder diffraction pattern substantially as shown in Figure **5**. The 2θ values for the peaks in Figure **5** (and their intensities) are as follows: 8.18 (32), 9.26 (100), 10.12 (81), 16.28 (93), 18.11 (30), 18.59 (63), 19.40 (67), 21.57 (60), 22.51 (31), 23.82 (29), 25.94 (24), and 28.05 (29).

This invention also relates, in part, to a methanol solvate of compound **IB-L0-2.3**.

In some embodiments, the methanol solvate has an X-ray powder diffraction pattern comprising one or more peaks selected from the group consisting of 8.4±0.2, 9.7±0.2, 10.1±0.2, 13.8±0.2, 17.4±0.2, 19.3±0.2, and 19.6±0.2 degrees 2θ. In some such embodiments, the methanol solvate has an X-ray powder diffraction pattern comprising three or more peaks selected from the group consisting of 8.4±0.2, 9.7±0.2, 10.1±0.2, 13.8±0.2, 17.4±0.2, 19.3±0.2, and 19.6±0.2 degrees 2θ. In other such embodiments, the methanol solvate has an X-ray powder diffraction pattern comprising five or more peaks selected from the group consisting of 8.4±0.2, 9.7±0.2, 10.1±0.2, 13.8±0.2, 17.4±0.2, 19.3±0.2, and 19.6±0.2 degrees 2θ.

In some embodiments, the methanol solvate has an X-ray powder diffraction pattern comprising one or more peaks selected from the group consisting of 8.4±0.2, 9.7±0.2, 10.1±0.2, 13.5±0.2, 13.8±0.2, 17.4±0.2, 19.3±0.2, 19.6±0.2, and 27.1±0.2 degrees 2θ. In some such embodiments, the methanol solvate has an X-ray powder diffraction pattern comprising three or more peaks selected from the group consisting of 8.4±0.2, 9.7±0.2, 10.1±0.2, 13.5±0.2, 13.8±0.2, 17.4±0.2, 19.3±0.2, 19.6±0.2, and 27.1±0.2 degrees 2θ. In other such embodiments, the methanol solvate has an X-ray powder diffraction pattern comprising five or more peaks selected from the group consisting of 8.4±0.2, 9.7±0.2, 10.1±0.2, 13.5±0.2, 13.8±0.2, 17.4±0.2, 19.3±0.2, 19.6±0.2, and 27.1±0.2 degrees 2θ.

In some embodiments, the methanol solvate has an X-ray powder diffraction pattern substantially as shown in Figure **6**. The 2θ values for the peaks in Figure **6** (and their intensities) are as follows: 8.36 (48), 9.74 (65), 10.05 (74), 13.55 (24), 13.79 (69), 17.40 (32), 19.30 (80), 19.58 (100), and 27.08 (24).

This invention also relates, in part, to a 1-propanol solvate of compound **IB-L0-2.3**.

In some embodiments, the 1-propanol solvate has an X-ray powder diffraction pattern comprising one or more peaks selected from the group consisting of 8.2±0.2, 9.3±0.2, 10.1±0.2, 15.7±0.2, 16.2 ±0.2, 18.4±0.2, 19.3±0.2, 21.6±0.2, and 22.8±0.2 degrees 2θ. In some such embodiments, the 1-propanol solvate has an X-ray powder diffraction pattern comprising three or more peaks selected from the group consisting of 8.2±0.2, 9.3±0.2, 10.1±0.2, 15.7±0.2, 16.2 ±0.2, 18.4±0.2, 19.3±0.2, 21.6±0.2, and 22.8±0.2 degrees 2θ. In other such embodiments, the 1-propanol solvate has an X-ray powder diffraction pattern comprising five or more peaks selected from the group consisting of 8.2±0.2, 9.3±0.2, 10.1±0.2, 15.7±0.2, 16.2 ±0.2, 18.4±0.2, 19.3±0.2, 21.6±0.2, and 22.8±0.2 degrees 2θ.

In some embodiments, the 1-propanol solvate has an X-ray powder diffraction pattern comprising one or more peaks selected from the group consisting of 8.2±0.2, 9.3±0.2, 10.1±0.2, 10.5±0.2, 15.7±0.2, 16.2 ±0.2, 18.4±0.2, 18.6±0.2, 19.3±0.2, 21.0±0.2, 21.6±0.2, and 22.8±0.2 degrees 2θ. In some such embodiments, the 1-propanol solvate has an X-ray powder diffraction pattern comprising three or more peaks selected from the group consisting of 8.2±0.2, 9.3±0.2, 10.1±0.2, 10.5±0.2, 15.7±0.2, 16.2 ±0.2, 18.4±0.2, 18.6±0.2, 19.3±0.2, 21.0±0.2, 21.6±0.2, and 22.8±0.2 degrees 2θ. In other such embodiments, the 1-propanol solvate has an X-ray powder diffraction pattern comprising five or more peaks selected from the group consisting of 8.2±0.2, 9.3±0.2, 10.1±0.2, 10.5±0.2, 15.7±0.2, 16.2 ±0.2, 18.4±0.2, 18.6±0.2, 19.3±0.2, 21.0±0.2, 21.6±0.2, and 22.8±0.2 degrees 2θ.

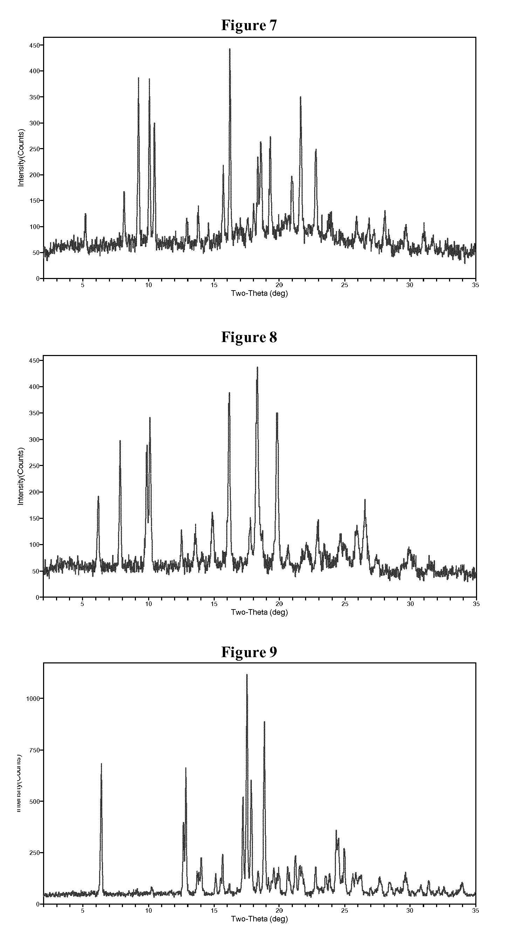

In some embodiments, the 1-propanol solvate has an X-ray powder diffraction pattern substantially as shown in Figure **7**. The 2θ values for the peaks in Figure **7** (and their intensities) are as follows: 8.15 (27), 9.26 (87), 10.08 (84), 10.47 (62), 15.73 (40), 16.24 (100), 18.37 (41), 18.59 (49), 19.33 (50), 20.97 (28), 21.65 (71), and 22.81 (44).

This invention also relates, in part, to a process for preparing the above solvates by suspending compound **IB-L0-2.3** in the corresponding solvent.

### GIB. Solvent Free IB-L0-2.3.

This invention also relates, in part, to a solvent free crystalline form of compound **IB-L0-2.3**.

In some embodiments, the solvent free compound **IB-L0-2.3** has an X-ray powder diffraction pattern comprising one or more peaks selected from the group consisting of 6.2±0.2, 7.9±0.2, 9.9±0.2, 16.2±0.2, and 18.3±0.2 degrees two theta (20). In some such embodiments, the solvent free compound **IB-L0-2.3** has an X-ray powder diffraction pattern comprising three or more peaks selected from the group consisting of 6.2±0.2, 7.9±0.2, 9.9±0.2, 16.2±0.2, and 18.3±0.2 degrees 2θ. In other such embodiments, the solvent free compound **IB-L0-2.3** has an X-ray powder diffraction pattern comprising five or more peaks selected from the group consisting of 6.2±0.2, 7.9±0.2, 9.9±0.2, 16.2±0.2, and 18.3±0.2 degrees 2θ.

In some embodiments, the solvent free compound **IB-L0-2.3** has an X-ray powder diffraction pattern comprising one or more peaks selected from the group consisting of 6.2±0.2, 7.9±0.2, 9.9±0.2, 10.1±0.2, 14.9±0.2, 16.2±0.2, 18.3±6.2, 19.8±0.2, and 26.5±0.2 degrees 2θ. In some such embodiments, the solvent free compound **IB-L0-2.3** has an X-ray powder diffraction pattern comprising three or more peaks selected from the group consisting of 6.2±0.2, 7.9±0.2, 9.9±0.2, 10.1±0.2, 14.9±0.2, 16.2±0.2, 18.3±0.2, 19.8±0.2, and 26.5±0.2 degrees 2θ. In other such embodiments, the solvent free compound **IB-L0-2.3** has an X-ray powder diffraction pattern comprising five or more peaks selected from the group consisting of 6.2±0.2, 7.9±0.2, 9.9±0.2, 10.1±0.2, 14.9±0.2, 16.2±0.2, 18.3±0.2, 19.8±0.2, and 26.5±0.2 degrees 2θ. In yet other such embodiments, the solvent free compound **IB-L0-2.3** has an X-ray powder diffraction pattern comprising eight or more peaks selected from the group consisting of 6.2±0.2, 7.9±6.2, 9.9±0.2, 10.1±0.2, 14.9±6.2, 16.2±0.2, 18.3±0.2, 19.8±6.2, and 26.5±6.2 degrees 2θ.

In some embodiments, the solvent free compound **IB-L0-2.3** has an X-ray powder diffraction pattern substantially as shown in Figure **8****.** The 2θ values for the peaks in Figure **8** (and their intensities) are as follows: 6.20 (36), 7.85 (66), 9.89 (61), 10.12 (75), 14.87 (27), 16.19 (89), 18.32 (100), 19.82 (77), and 26.53 (34).

This invention also relates, in part, to a process for preparing the solvent free crystalline form of compound **IB-L0-2.3** by desolvating one of **IB-L0-2.3** solvates discussed above. A solvate can be desolvated by heating the solvate solid for about 10min at ~125°C.

### G1C. IB-L0-2.3 Hydrate.

This invention also relates, in part, to a hydrate of compound **IB-L0-2.3**.

In some embodiments, the hydrate has an X-ray powder diffraction pattern comprising one or more peaks selected from the group consisting of 6.4±0.2, 12.9±0.2, 17.9±0.2, and 18.9±0.2 degrees 2θ. In some such embodiments, the hydrate has an X-ray powder diffraction pattern comprising three or more peaks selected from the group consisting of 6.4±0.2, 12.9±0.2, 17.9±0.2, and 18.9±0.2 degrees 2θ.

In some embodiments, the hydrate has an X-ray powder diffraction pattern comprising one or more peaks selected from the group consisting of 6.4±0.2, 12.9±0.2, 17.5±0.2, 17.9±0.2, 18.9±0.2, and 24.4±0.2 degrees 2θ. In some such embodiments, the hydrate has an X-ray powder diffraction pattern comprising three or more peaks selected from the group consisting of 6.4±0.2, 12.9±0.2, 17.5±0.2, 17.9±0.2, 18.9±0.2, and 24.4±0.2 degrees 2θ. In other such embodiments, the hydrate has an X-ray powder diffraction pattern comprising five or more peaks selected from the group consisting of 6.4±0.2, 12.9±0.2, 17.5±0.2, 17.9±0.2, 18.9±0.2, and 24.4±0.2 degrees 2θ.

In some embodiments, the hydrate has an X-ray powder diffraction pattern comprising one or more peaks selected from the group consisting of 6.4±0.2, 12.7±0.2, 12.9±0.2, 14.1±0.2, 15.7±0.2, 17.2±0.2, 17.5±0.2, 17.9±0.2, 18.9±0.2, 21.2±0.2, 24.4±0.2, and 25.0±0.2 degrees 2θ. In some such embodiments, the hydrate has an X-ray powder diffraction pattern comprising three or more peaks selected from the group consisting of 6.4±0.2, 12.7±0.2, 12.9±0.2, 14.1±0.2, 15.7±0.2, 17.2±0.2, 17.5±0.2, 17.9±0.2, 18.9±0.2, 21.2±0.2, 24.4±0.2, and 25.0±0.2 degrees 2θ. In other such embodiments, the hydrate has an X-ray powder diffraction pattern comprising five or more peaks selected from the group consisting of 6.4+0.2, 12.7±0.2, 12.9±0.2, 14.1±0.2, 15.7±0.2, 17.2±0.2, 17.5±0.2, 17.9±0.2, 18.9±0.2, 21.2±0.2, 24.4±0.2, and 25.0±0.2 degrees 2θ.

In some embodiments, the hydrate has an X-ray powder diffraction pattern substantially as shown in Figure **9**. The 2θ values for the peaks in Figure **9** (and their intensities) are as follows: 6.42 (60), 12.71 (33), 12.89 (58), 14.05 (17), 15.68 (18), 17.22 (44), 17.53 (100), 17.86 (51), 18.87 (77), 21.25 (17), 24.35 (28), and 24.95 (20).

This invention also relates, in part, to a process for preparing the hydrate by suspending the above-described solvent free crystalline compound in water. The hydrate was prepared by suspending 300mg of the solvent free crystalline compound in 2ml of water at 45⁰C for four days.

### G2. Crystalline Forms of N-(6-(3-Tert-butyl-5-(2,4-dioxo-3,4-dihydropyrimidin-1(2H)-yl)-2-methoxyphenyl)naphthalen-2-yl)methanesulfonamide, Monosodium Salt.

This invention also relates, in part, to crystalline forms of N-(6-(3-tert-butyl-5-(2,4-dioxo-3,4-dihydropyrimidin-1(2H)-yl)-2-methoxyphenyl)naphthalen-2-yl)methanesulfonamide, monosodium salt, namely the pattern A, pattern B, and pattern C crystalline forms discussed below.

This invention relates, in part, to a pattern A crystalline monosodium salt.

In some embodiments, the pattern A monosodium salt has an X-ray powder diffraction pattern comprising one or more peaks selected from the group consisting of 4.6±0.2, 10.4±0.2, 12.0±0.2, 15.6±0.2, 18.6±0.2, 22.8±0.2, and 23.9±0.2 degrees 2θ. In some such embodiments, the pattern A monosodium salt has an X-ray powder diffraction pattern comprising three or more peaks selected from the group consisting of 4.6±0.2, 10.4±0.2, 12.0±0.2, 15.6±0.2, 18.6±0.2, 22.8±0.2, and 23.9±0.2 degrees 2θ. In other such embodiments, the pattern A monosodium salt has an X-ray powder diffraction pattern comprising five or more peaks selected from the group consisting of 4.6±0.2, 10.4±0.2, 12.0±0.2, 15.6±0.2, 18.6±0.2, 22.8±0.2, and 23.9±0.2 degrees 2θ.

In some embodiments, the pattern A monosodium salt has an X-ray powder diffraction pattern comprising one or more peaks selected from the group consisting of 4.6±0.2, 10.4±0.2, 12.0±0.2, 15.6±0.2, 18.6±0.2, 22.8±0.2, 23.3±0.2, and 23.9±0.2 degrees 2θ. In some such embodiments, the pattern A monosodium salt has an X-ray powder diffraction pattern comprising three or more peaks selected from the group consisting of 4.6±0.2, 10.4±0.2, 12.0±0.2, 15.6±0.2, 18.6±0.2, 22.8±0.2, 23.3±0.2, and 23.9±0.2 degrees 2θ. In other such embodiments, the pattern A monosodium salt has an X-ray powder diffraction pattern comprising five or more peaks selected from the group consisting of 4.6±0.2, 10.4±0.2, 12.0±0.2, 15.6±0.2, 18.6±0.2, 22.8±0.2, 23.3±0.2, and 23.9±0.2 degrees 2θ.

In some embodiments, the pattern A monosodium salt has an X-ray powder diffraction pattern comprising one or more peaks selected from the group consisting of 4.6±0.2, 10.4±0.2, 12.0±0.2, 15.6±0.2, 16.0±0.2, 18.6±0.2, 22.8±0.2, 23.3±0.2, 23.9±0.2, and 28.3±0.2 degrees 2θ. In some such embodiments, the pattern A monosodium salt has an X-ray powder diffraction pattern comprising three or more peaks selected from the group consisting of 4.6±0.2, 10.4±0.2, 12.0±0.2, 15.6±0.2, 16.0±0.2, 18.6±0.2, 22.8±0.2, 23.3±0.2, 23.9±0.2, and 28.3±0.2 degrees 2θ. In other such embodiments, the pattern A monosodium salt has an X-ray powder diffraction pattern comprising five or more peaks selected from the group consisting of 4.6±0.2, 10.4±0.2, 12.0±0.2, 15.6±0.2, 16.0±0.2, 18.6±0.2, 22.8±0.2, 23.3±0.2, 23.9±0.2, and 28.3±0.2 degrees 2θ. In other such embodiments, the pattern A monosodium salt has an X-ray powder diffraction pattern comprising eight or more peaks selected from the group consisting of 4.6±0.2, 10.4±0.2, 12.0±0.2, 15.6±0.2, 16.0±0.2, 18.6±0.2, 22.8±0.2, 23.3±0.2, 23.9±0.2, and 28.3±0.2 degrees 2θ.

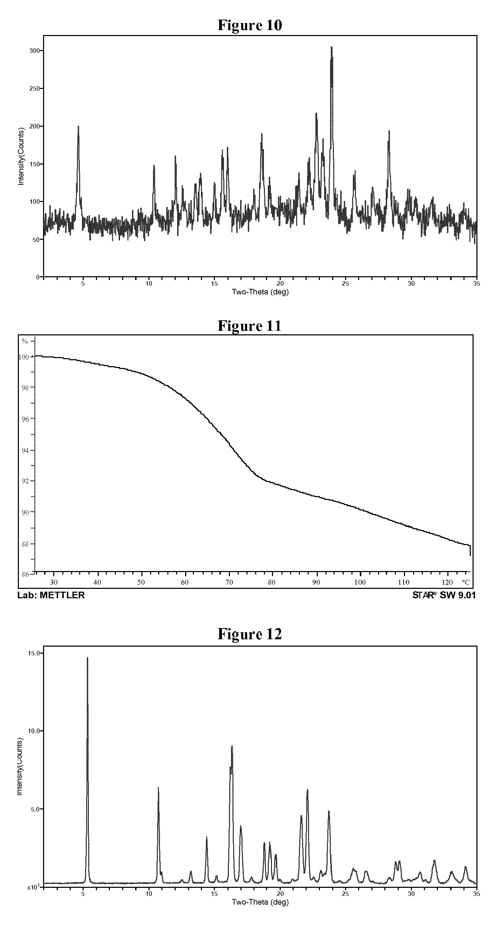

In some embodiments, the pattern A monosodium salt has an X-ray powder diffraction pattern substantially as shown in Figure **10**. The 2θ values for the peaks in Figure **10** (and their intensities) are as follows: 4.64 (62), 10.41 (38), 12.04 (38), 15.62 (44), 15.99 (44), 18.63 (49), 22.77 (60), 23.29 (40), 23.93 (100), and 28.31 (56).

This invention also relates, in part, to a process for preparing the pattern A monosodium salt. The pattern A monosodium salt was prepared by adding 1M aqueous NaOH (0.548ml) to compound **IB-L0-2.3** (225.72mg), seeding the resulting suspension with crystalline N-(6-(3-tert-butyl-5-(2,4-dioxo-3,4-dihydropyrimidin-1(2H)-yl)-2-methoxyphenyl)naphthalen-2-yl)methanesulfonamide, disodium salt (prepared as discussed below), and equilibrating the resulting suspension at ambient conditions. The pattern A monosodium salt was formed on the following day through a solution-mediated process. The stoichiometry of the salt was presumed to be 1:1 based on the crystallization procedure. This invention also relates, in part, to a pattern B crystalline monosodium salt.

In some embodiments, the pattern B monosodium salt has an X-ray powder diffraction pattern comprising one or more peaks selected from the group consisting of 5.4±0.2, 10.8±0.2, 14.4±0.2, 16.3±0.2, 17.0±0.2, 21.6±0.2, 22.1±0.2, and 23.7±0.2 degrees 2θ. In some such embodiments, the pattern B monosodium salt has an X-ray powder diffraction pattern comprising three or more peaks selected from the group consisting of 5.4±0.2, 10.8±0.2, 14.4±0.2, 16.3±0.2, 17.0±0.2, 21.6±0.2, 22.1±0.2, and 23.7±0.2 degrees 2θ. In other such embodiments, the pattern B monosodium salt has an X-ray powder diffraction pattern comprising five or more peaks selected from the group consisting of 5.4±0.2, 10.8±0.2, 14.4±0.2, 16.3±0.2, 17.0±0.2, 21.6±0.2, 22.1±0.2, and 23.7±0.2 degrees 2θ.

In some embodiments, the pattern B monosodium salt has an X-ray powder diffraction pattern comprising one or more peaks selected from the group consisting of 5.4±0.2, 10.8±0.2, 14.4±0.2, 16.3±0.2, 17.0±0.2, 18.8±0.2, 19.2±0.2, 19.6±0.2, 21.6±0.2, 22.1±0.2, 23.7±0.2, 28.8±0.2, 29.1±0.2, and 31.8±0.2 degrees 2θ. In some such embodiments, the pattern B monosodium salt has an X-ray powder diffraction pattern comprising three or more peaks selected from the group consisting of 5.4±0.2, 10.8±0.2, 14.4±0.2, 16.3±0.2, 17.0±0.2, 18.8±0.2, 19.2±0.2, 19.6±0.2, 21.6±0.2, 22.1±0.2, 23.7±0.2, 28.8±0.2, 29.1±0.2, and 31.8±0.2 degrees 2θ. In other such embodiments, the pattern B monosodium salt has an X-ray powder diffraction pattern comprising five or more peaks selected from the group consisting of 5.4±0.2, 10.8±0.2, 14.4±0.2, 16.3±0.2, 17.0±0.2, 18.8±0.2, 19.2±0.2, 19.6+0.2, 21.6±0.2, 22.1±0.2, 23.7±0.2, 28.8±0.2, 29.1±0.2, and 31.8±0.2 degrees 2θ. In other such embodiments, the pattern B monosodium salt has an X-ray powder diffraction pattern comprising eight or more peaks selected from the group consisting of 5.4±0.2, 10.8±0.2, 14.4±0.2, 16.3±0.2, 17.0±0.2, 18.8±0.2, 19.2±0.2, 19.6±0.2, 21.6±0.2, 22.1±0.2, 23.7±0.2, 28.8±0.2, 29.1±0.2, and 31.8±0.2 degrees 2θ.

In some embodiments, the pattern B monosodium salt has an X-ray powder diffraction pattern comprising one or more peaks selected from the group consisting of 5.4±0.2, 10.8±0.2, 14.4±0.2, 16.3±0.2, 17.0±0.2, 18.8±0.2, 19.2±0.2, 19.6±0.2, 21.6±0.2, 22.1±0.2, 23.7±0.2, 28.8±0.2, 29.1±0.2, and 31.8±0.2 degrees 2θ. In some such embodiments, the pattern B monosodium salt has an X-ray powder diffraction pattern comprising two or more peaks selected from the group consisting of 5.4±0.2, 10.8±0.2, 14.4±0.2, 16.3±0.2, 17.0±0.2, 18.8±0.2, 19.2±0.2, 19.6±0.2, 21.6±0.2, 22.1±0.2, 23.7±0.2, 29.1±0.2, and 31.8±0.2 degrees 2θ. In other such embodiments, the pattern B monosodium salt has an X-ray powder diffraction pattern comprising two or more peaks selected from the group consisting of 5.4±0.2, 10.8±0.2, 14.4±0.2, 16.3±0.2, 17.0±0.2, 18.8±0.2, 19.2±0.2,19.6±0.2, 21.6±0.2, 22.1±0.2, 23.7±0.2, 28.8±0.2, and 31.8±0.2 degrees 2θ. In yet other such embodiments, the pattern B monosodium salt has an X-ray powder diffraction pattern comprising three or more peaks selected from the group consisting of 5.4±0.2, 10.8±0.2, 14.4±0.2, 16.3±0.2, 17.0±0.2, 18.8±0.2, 19.2±0.2, 19.6±0.2, 21.6±0.2, 22.1±0.2, 23.7±0.2, and 31.8±0.2 degrees 2θ. In yet other such embodiments, the pattern B monosodium salt has an X-ray powder diffraction pattern comprising three or more peaks selected from the group consisting of 5.4±0.2, 10.8±0.2, 14.4±0.2, 16.3±0.2, 17.0±0.2, 18.8±0.2, 19.2±0.2, 21.6±0.2, 22.1±0.2, and 23.7±0.2 degrees 2θ. In yet other such embodiments, the pattern B monosodium salt has an X-ray powder diffraction pattern comprising three or more peaks selected from the group consisting of 5.4±0.2, 10.8±0.2, 14.4±0.2, 16.3±0.2, 17.0±0.2, 19.2±0.2, 21.6±0.2, 22.1±0.2, and 23.7±0.2 degrees 2θ. In yet other such embodiments, the pattern B monosodium salt has an X-ray powder diffraction pattern comprising three or more peaks selected from the group consisting of 5.4±0.2, 10.8±0.2, 14.4±0.2, 16.3±0.2, 17.0±0.2, 18.8±0.2, 21.6±0.2, 22.1±0.2, and 23.7±0.2 degrees 2θ. In yet other such embodiments, the pattern B monosodium salt has an X-ray powder diffraction pattern comprising three or more peaks selected from the group consisting of 5.4±0.2, 10.8±0.2, 14.4±0.2, 16.3±0.2, 17.0±0.2, 21.6±0.2, 22.1±0.2, and 23.7±0.2 degrees 2θ. In yet other such embodiments, the pattern B monosodium salt has an X-ray powder diffraction pattern comprising three or more peaks selected from the group consisting of 5.4±0.2, 10.8±0.2, 16.3±0.2, 22.1±0.2, and 23.7±0.2 degrees 2θ.

In some embodiments, the pattern B monosodium salt has an X-ray powder diffraction pattern comprising peaks at 5.4±0.2, 10.8±0.2, and 16.3±0.2 degrees 2θ. In some such embodiments, the pattern B monosodium salt has an X-ray powder diffraction pattern comprising peaks at 5.4±0.2, 10.8±0.2, 16.3±0.2, and 22.1±0.2 degrees 2θ. In other such embodiments, the pattern B monosodium salt has an X-ray powder diffraction pattern comprising peaks at 5.4±0.2, 10.8±0.2, 16.3±0.2, 22.1±0.2, and 23.7±0.2 degrees 2θ. In yet other such embodiments, the pattern B monosodium salt has an X-ray powder diffraction pattern comprising peaks at 5.4±0.2, 10.8±0.2, 14.4±0.2, 16.3±0.2, 17.0±0.2, 21.6±0.2, 22.1±0.2, and 23.7±0.2 degrees 2θ. In yet other such embodiments, the pattern B monosodium salt has an X-ray powder diffraction pattern comprising peaks at 5.4±0.2, 10.8±0.2, 14.4±0.2, 16.3±0.2, 17.0±0.2, 18.8±0.2, 21.6±0.2, 22.1±0.2, and 23.7±0.2 degrees 2θ. In yet other such embodiments, the pattern B monosodium salt has an X-ray powder diffraction pattern comprising peaks at 5.4±0.2, 10.8±0.2, 14.4±0.2, 16.3±0.2, 17.0±0.2, 19.2±0.2, 21.6±0.2, 22.1±0.2, and 23.7±0.2 degrees 2θ. In yet other such embodiments, the pattern B monosodium salt has an X-ray powder diffraction pattern comprising peaks at 5.4±0.2, 10.8±0.2, 14.4±0.2, 16.3±0.2, 17.0±0.2, 18.8±0.2, 19.2±0.2, 21.6±0.2, 22.1±0.2, and 23.7±0.2 degrees 2θ. In further such embodiments, the pattern B monosodium salt has an X-ray powder diffraction pattern comprising peaks at 5.4±0.2, 10.8±0.2, 14.4±0.2, 16.3±0.2, 17.0±0.2, 18.8±0.2, 19.2±0.2, 19.6±0.2, 21.6±0.2, 22.1±0.2, 23.7±0.2, and 31.8±0.2 degrees 2θ. In yet further such embodiments, the pattern B monosodium salt has an X-ray powder diffraction pattern comprising peaks at 5.4±0.2, 10.8±0.2, 14.4±0.2, 16.3±0.2, 17.0±0.2, 18.8±0.2, 19.2±0.2, 19.6±0.2, 21.6±0.2, 22.1±0.2, 23.7±0.2, 28.8±0.2, and 31.8±0.2 degrees 2θ. In yet further such embodiments, the pattern B monosodium salt has an X-ray powder diffraction pattern comprising peaks at 5.4±0.2, 10.8±0.2, 14.4±0.2, 16.3±0.2, 17.0±0.2, 18.8±0.2, 19.2±0.2, 19.6±0.2, 21.6±0.2, 22.1±0.2, 23.7±0.2, 29.1±0.2, and 31.8±0.2 degrees 2θ. In yet further such embodiments, the pattern B monosodium salt has an X-ray powder diffraction pattern comprising peaks at 5.4±0.2, 10.8±0.2, 14.4±0.2, 16.3±0.2, 17.0±0.2, 18.8±0.2, 19.2±0.2, 19.6±0.2, 21.6±0.2, 22.1±0.2, 23.7±0.2, 28.8±0.2, 29.1±0.2, and 31.8±0.2 degrees 2θ.

In some embodiments, the pattern B monosodium salt has an X-ray powder diffraction pattern substantially as shown in Figure **12**. The 2θ values for the peaks in Figure **12** (and their intensities) are as follows: 5.36 (100), 10.75 (42), 14.43 (20), 16.34 (60), 17.00 (25), 18.83 (18), 19.24 (18), 19.66 (12), 21.64 (29), 22.12 (41), 23.73 (32), 28.83 (9), 29.10 (9), and 31.78 (10).

This invention also relates, in part, to a process for preparing the pattern B monosodium salt. The pattern B monosodium salt can prepared by suspending the pattern A monosodium salt (for example, ∼ 30mg) in various organic solvents (e.g., ∼ 125ul acetonitrile, ethanol, 1-propanol, or 2-propanol) at room temperature. The pattern B monosodium salt was also prepared by seeding a solution with pattern B monosodium salt. Compound **IB-L0-2.3** (12.5g) was dissolved in DMSO (37.5ml) at ∼68⁰C. 1.04g NaOH dissolved in 6.3ml of water, 6.3ml 2-propanol, and 12.5ml 35.2:1 v/v 2-propanol/water was added. The solution was seeded with 125mg of pattern B seeds slurried in 12.5ml of 35.2:1 v/v 2-propanol/water, and the crystallization slurry was incubated at ∼68⁰C for ∼1.5h. 175ml 35.2:1 v/v 2-propanol/water at ∼68⁰C was added over ∼7h, and the crystallization slurry was cooled to ∼0⁰C over no less than 7h. The crystals were isolated by filtration and analyzed by PXRD. The crystals were then dried at ∼50⁰C under vacuum (approximately 3 inches of mercury). The dried crystals were analyzed by PXRD, which showed no change in comparison to the pre-drying sample. The stoichiometry of the pattern B monosodium salt was confirmed by ion chromatography.

This invention also relates, in part, to a pattern C crystalline monosodium salt.

In some embodiments, the pattern C monosodium salt has an X-ray powder diffraction pattern comprising one or more peaks selected from the group consisting of 5.0±0.2, 12.0±0.2, 17.5±0.2, 18.8±0.2, and 22.7±0.2 degrees 2θ. In some such embodiments, the pattern C monosodium salt has an X-ray powder diffraction pattern comprising three or more peaks selected from the group consisting of 5.0±0.2, 12.0±0.2, 17.5±0.2, 18.8±0.2, and 22.7±0.2 degrees 2θ.

In some embodiments, the pattern C monosodium salt has an X-ray powder diffraction pattern comprising one or more peaks selected from the group consisting of 5.0±0.2, 12.0±0.2, 17.5±0.2, 17.8±0.2, 18.8±0.2, and 22.7±0.2 degrees 2θ. In some such embodiments, the pattern A monosodium salt has an X-ray powder diffraction pattern comprising three or more peaks selected from the group consisting of 5.0±0.2, 12.0±0.2, 17.5±0.2, 17.8±0.2, 18.8±0.2, and 22.7±0.2 degrees 2θ. In other such embodiments, the pattern A monosodium salt has an X-ray powder diffraction pattern comprising five or more peaks selected from the group consisting of 5.0±0.2, 12.0±0.2, 17.5±0.2, 17.8±0.2, 18.8±0.2, and 22.7±0.2 degrees 2θ.

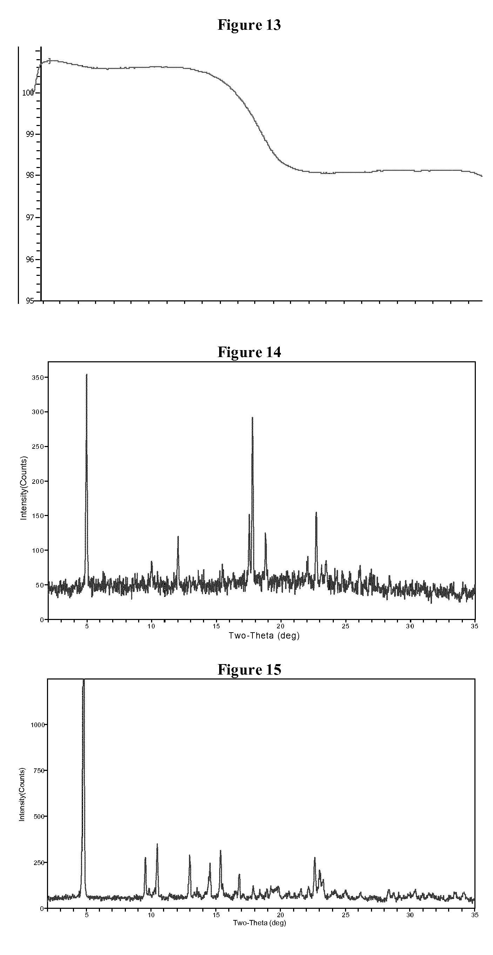

In some embodiments, the pattern C monosodium salt has an X-ray powder diffraction pattern substantially as shown in Figure **14**. The 2θ values for the peaks in Figure **14** (and their intensities) are as follows: 4.97 (100), 12.03 (24), 17.55 (32), 17.80 (77), 18.79 (23), and 22.74 (33).

This invention also relates, in part, to a process for preparing the pattern C monosodium salt. The pattern C monosodium salt was prepared as follows. Pattern B monosodium salt (100mg) was dissolved in 400ul DMSO and 2ml 12:1 v/v 2-propanol/H₂O at 70°C. Pattern B monosodium salt seed crystals were added to the solution, and the solution was then cooled to ambient temperature over 20min. Filtration yielded crystals of the pattern C monosodium salt.

### G3. Crystalline Form of N-(6-(3-Tert-butyl-5-(2,4-dioxo-3,4-dihydropyrimidin-1(2H)-yl)-2-methoxyphenyl)naphthalen-2 yl)methanesulfonamide, Disodium Salt.

This invention also relates, in part, to a crystalline form of N-(6-(3-tert-butyl-5-(2,4-dioxo-3,4-dihydropyrimidin-1(2H)-yl)-2-methoxyphenyl)naphthalen-2-yl)methanesulfonamide, disodium salt.

In some embodiments, the disodium salt has an X-ray powder diffraction pattern comprising one or more peaks selected from the group consisting of 4.8±0.2, 9.6±0.2, 10.5±0.2, 13.0±0.2, 14.6±0.2, 15.4±0.2, 16.8 ±0.2, and 23.0±0.2 degrees 2θ. In some such embodiments, the disodium salt has an X-ray powder diffraction pattern comprising three or more peaks selected from the group consisting of 4.8±0.2, 9.6±0.2, 10.5±0.2, 13.0±0.2, 14.6±0.2, 15.4±0.2, 16.8 ±0.2, and 23.0±0.2 degrees 2θ. In other such embodiments, the disodium salt has an X-ray powder diffraction pattern comprising five or more peaks selected from the group consisting of 4.8±0.2, 9.6±0.2, 10.5±0.2, 13.0±0.2, 14.6±0.2, 15.4±0.2, 16.8 ±0.2, and 23.0±0.2 degrees 2θ.

In some embodiments, the disodium salt has an X-ray powder diffraction pattern comprising one or more peaks selected from the group consisting of 4.8±0.2, 9.6±0.2, 10.5±0.2, 13.0±0.2, 14.6±0.2, 15.4±0.2, 16.8 ±0.2, 22.7±0.2, 23.0±0.2, and 23.3±0.2 degrees 2θ. In some such embodiments, the disodium salt has an X-ray powder diffraction pattern comprising three or more peaks selected from the group consisting of 4.8±0.2, 9.6±0.2, 10.5±0.2, 13.0±0.2, 14.6±0.2, 15.4±0.2, 16.8 ±0.2, 22.7±0.2, 23.0±0.2, and 23.3±0.2 degrees 2θ. In other such embodiments, the disodium salt has an X-ray powder diffraction pattern comprising five or more peaks selected from the group consisting of 4.8±0.2, 9.6±0.2, 10.5±0.2, 13.0±0.2, 14.6±0.2, 15.4±0.2, 16.8 ±0.2, 22.7±0.2, 23.0±0.2, and 23.3±0.2 degrees 2θ.

In some embodiments, the disodium salt has an X-ray powder diffraction pattern substantially as shown in Figure **15**. The 2θ values for the peaks in Figure **15** (and their intensities) are as follows: 4.80 (100), 9.59 (10), 10.51 (13), 12.98 (11), 14.56 (8), 15.38 (12), 16.84 (6), 22.68 (10), 23.04 (6), and 23.33 (4).

This invention also relates, in part, to a process for preparing the disodium salt. The disodium salt was prepared by suspending compound **IB-L0-2.3** (52.83mg) in 1M aqueous NaOH (1.1ml) (the molar ratio compound:NaOH was 1:10). The solution was heated to 36⁰C, and the solid dissolved completely to yield a clear solution. The solution was naturally cooled to ambient temperature, and the salt crystallized in 24h. Alternatively, the disodium salt was prepared by suspending compound **IB-L0-2.3** (51mg) in EtOH (1ml). NaOH in 1.2ml of 5:1 v/v EtOWH₂O (2.1 molar equivalent) was added. The reaction mixture was concentrated and 2ml acetonitrile was added to induce crystallization. The stoichiometry of this solid was determined by ion chromatography.

### G4. Crystalline Form of N-(6-(3-Tert-butyl-5-(2,4-dioxo-3,4-dihydropyrimidin-1(2H)-yl)-2-methoxyphenyl)naphthalen-2-yl)methanesulfonamide, Monopotassium Salt.

This invention also relates, in part, to a crystalline form of N-(6-(3-tert-butyl-5-(2,4-dioxo-3,4-dihydropyrimidin-1(2H)-yl)-2-methoxyphenyl)naphthalen-2-yl)methanesulfonamide, monopotassium salt.

In some embodiments, the monopotassium salt has an X-ray powder diffraction pattern comprising one or more peaks selected from the group consisting of 5.0±0.2, 9.9±0.2, 11.3±0.2, 13.3±0.2, 16.9±0.2, 18.1±0.2, 19.1±0.2, 20.0±0.2, 21.1±0.2, 23.5±0.2, 24.8±0.2, and 25.7±0.2 degrees 2θ. In some such embodiments, the monopotassium salt has an X-ray powder diffraction pattern comprising three or more peaks selected from the group consisting of 5.0±0.2, 9.9±0.2, 11.3±0.2, 13.3±0.2, 16.9±0.2, 18.1±0.2, 19.1±0.2, 20.0±0.2, 21.1±0.2, 23.5±0.2, 24.8±0.2, and 25.7±0.2 degrees 2θ. In other such embodiments, the monopotassium salt has an X-ray powder diffraction pattern comprising five or more peaks selected from the group consisting of 5.0±0.2, 9.9±0.2, 11.3±0.2, 13.3±0.2, 16.9±0.2, 18.1±0.2, 19.1±0.2, 20.0±0.2, 21.1±0.2, 23.5±0.2, 24.8±0.2, and 25.7±0.2degrees 2θ.

In some embodiments, the monopotassium salt has an X-ray powder diffraction pattern comprising one or more peaks selected from the group consisting of 5.0±0.2, 9.9±0.2, 11.3±0.2, 13.3±0.2, 16.9±0.2, 18.1±0.2, 19.1±0.2, 20.0±0.2, 21.1±0.2, 21.5±0.2, 23.5±0.2, 24.8±0.2, and 25.7±0.2 degrees 2θ. In some such embodiments, the monopotassium salt has an X-ray powder diffraction pattern comprising three or more peaks selected from the group consisting of 5.0±0.2, 9.9±0.2, 11.3±0.2, 13.3±0.2, 16.9±0.2, 18.1±0.2, 19.1±0.2, 20.0±0.2, 21.1±0.2, 21.5±0.2, 23.5±0.2, 24.8±0.2, and 25.7±0.2 degrees 2θ. In other such embodiments, the monopotassium salt has an X-ray powder diffraction pattern comprising five or more peaks selected from the group consisting of 5.0±0.2, 9.9±0.2, 11.3±0.2, 13.3±0.2, 16.9±0.2, 18.1±0.2, 19.1±0.2, 20.0±0.2, 21.1±0.2, 21.5±0.2, 23.5±0.2, 24.8±0.2, and 25.7±0.2degrees 2θ.

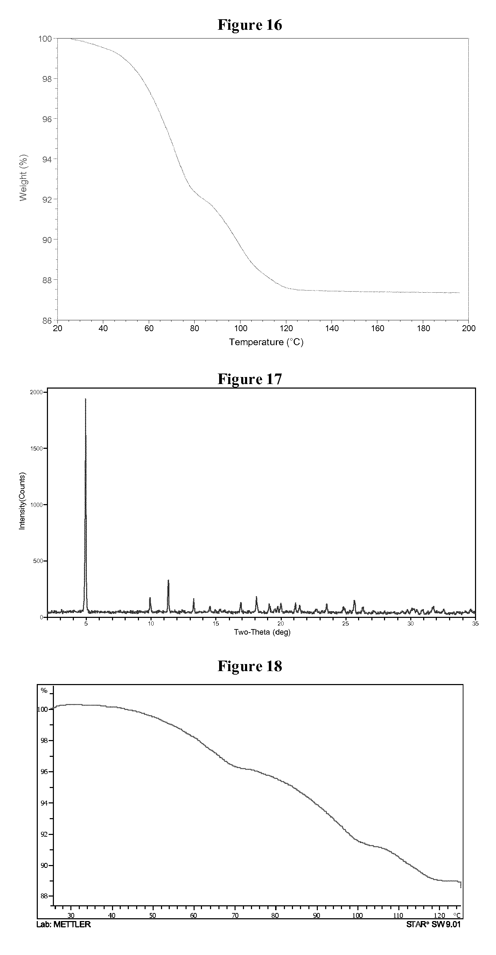

In some embodiments, the monopotassium salt has an X-ray powder diffraction pattern substantially as shown in Figure **17**. The 2θ values for the peaks in Figure **17** (and their intensities) are as follows: 4.97 (100), 9.94 (7), 11.33 (15), 13.28 (7), 16.91 (5), 18.13 (7), 19.14 (4), 20.00 (4), 21.13 (4), 21.45 (4), 23.54 (4), 24.84 (3), and 25.67 (6).

This invention also relates, in part, to a process for preparing the monopotassium salt. The monopotassium salt was prepared in aqueous medium. 0.366ml of 1M aqueous KOH was added to 150.56mg of compound **IB-L0-2.3** (molar ratio 1:1.2). The resulting suspension was equilibrated at ambient conditions. The monopotassium salt was formed on the following day through a solution-mediated process. Alternatively, the monopotassium salt was prepared by suspending compound **IB-L0-**2.3 (300mg) in 3ml acetonitrile. KOH in 1.3mL of H₂O (2.1 molar equivalent) was added. Additional 1ml H₂O was added to dissolve all solids. Afterwards, 12ml acetonitrile was added to induce crystallization. The stoichiometry of the salt was confirmed by ion chromatograph.

### G5. Crystalline Forms of N-(6-(3-Tert-butyl-5-(2,4-dioxo-3,4-dihydropyrimidin-1(2H)-yl)-2-methoxyphenyl)naphthalen-2-yl)methanesulfonamide, Monocholine Salt.

This invention also relates, in part, to crystalline forms of N-(6-(3-tert-butyl-5-(2,4-dioxo-3,4-dihydropyrimidin-1(2H)-yl)-2-methoxyphenyl)naphthalen-2-yl)methanesulfonamide, monocholine salt, namely the pattern A and pattern B crystalline forms discussed below.

This invention relates, in part, to a pattern A crystalline monocholine salt.

In some embodiments, the pattern A monocholine salt has an X-ray powder diffraction pattern comprising one or more peaks selected from the group consisting of 10.9±0.2, 12.1±0.2, 13.4±0.2, 15.5±0.2, 17.0±0.2, 17.8±0.2, 18.3±0.2, 19.5±0.2, and 21.9±0.2 degrees 2θ. In some such embodiments, the pattern A monocholine salt has an X-ray powder diffraction pattern comprising three or more peaks selected from the group consisting of 10.9±0.2, 12.1±0.2, 13.4±0.2, 15.5±0.2, 17.0±0.2, 17.8±0.2, 18.3±0.2, 19.5±0.2, and 21.9±0.2 degrees 2θ. In other such embodiments, the pattern A monocholine salt has an X-ray powder diffraction pattern comprising five or more peaks selected from the group consisting of 10.9±0.2, 12.1±0.2, 13.4±0.2, 15.5±0.2, 17.0±0.2, 17.8±0.2, 18.3±0.2, 19.5±0.2, and 21.9±0.2 degrees 2θ.

In some embodiments, the pattern A monocholine salt has an X-ray powder diffraction pattern comprising one or more peaks selected from the group consisting of 10.9±0.2, 12.1±0.2, 13.0±0.2, 13.4±0.2, 13.6±0.2, 15.5±0.2, 17.0±0.2, 17.8±0.2, 18.3±0.2, 19.5±0.2, 19.7±0.2, and 21.9±0.2 degrees 2θ. In some such embodiments, the pattern A monocholine salt has an X-ray powder diffraction pattern comprising three or more peaks selected from the group consisting of degrees 2θ. In other such embodiments, the pattern A monocholine salt has an X-ray powder diffraction pattern comprising five or more peaks selected from the group consisting of degrees 2θ.

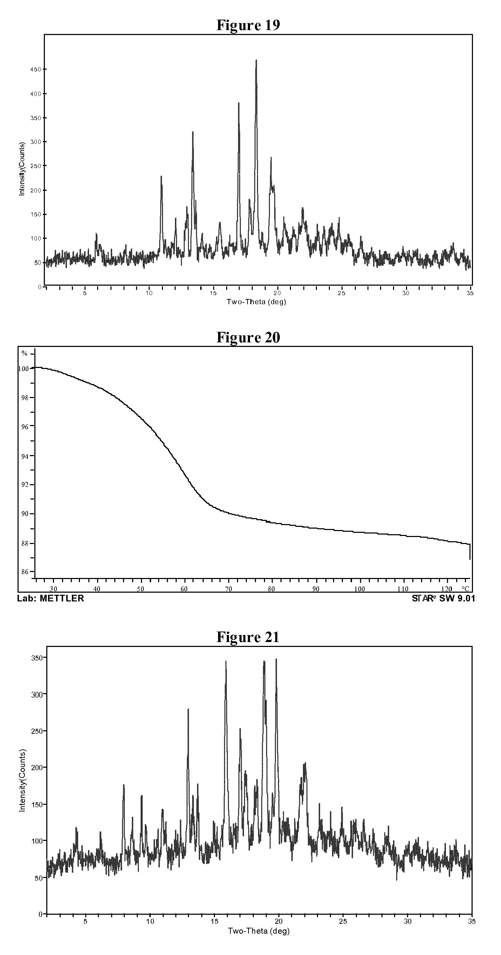

In some embodiments, the pattern A monocholine salt has an X-ray powder diffraction pattern substantially as shown in Figure **19**. The 2θ values for the peaks in Figure **19** (and their intensities) are as follows: 10.94 (42), 12.06 (20), 12.96 (26), 13.42 (64), 13.64 (27), 15.51 (18), 16.98 (78), 17.81 (26), 18.32 (100), 19.49 (48), 19.70 (33), and 21.91 (22).

This invention also relates, in part, to a process for preparing the pattern A monocholine salt. It was prepared in a solvent mixture of tetrahydrofuran (THF) and methanol. Compound **IB-L0-2.3** (56.79mg) was dissolved in THF at 60°C, 40.01mg of choline hydroxide solution (45wt% in methanol) was added resulting in a molar ratio of 1:1.2. The crystals formed upon natural cooling to ambient temperature.

This invention also relates, in part, to a pattern B crystalline monocholine salt.

In some embodiments, the pattern B monocholine salt has an X-ray powder diffraction pattern comprising one or more peaks selected from the group consisting of 8.0±0.2, 9.4±0.2, 11.0±0.2, 13.0±0.2, 13.7±0.2, 15.9±0.2, 17.0±0.2, 18.3±0.2, 18.9±0.2, 19.8±0.2, and 22.1±0.2 degrees 2θ. In some such embodiments, the pattern B monocholine salt has an X-ray powder diffraction pattern comprising three or more peaks selected from the group consisting of 8.0±0.2, 9.4±0.2, 11.0±0.2, 13.0±0.2, 13.7±0.2, 15.9±0.2, 17.0±0.2, 18.3±0.2, 18.9±0.2, 19.8±0.2, and 22.1±0.2 degrees 2θ. In other such embodiments, the pattern B monocholine salt has an X-ray powder diffraction pattern comprising five or more peaks selected from the group consisting of 8.0±0.2, 9.4±0.2, 11.0±0.2, 13.0±0.2, 13.7±0.2, 15.9±0.2, 17.0±0.2, 18.3±0.2, 18.9±0.2, 19.8±0.2, and 22.1±0.2 degrees 2θ.

In some embodiments, the pattern B monocholine salt has an X-ray powder diffraction pattern comprising one or more peaks selected from the group consisting of 8.0±0.2, 9.4±0.2, 11.0±0.2, 13.0±0.2, 13.3±0.2, 13.7±0.2, 15.9±0.2, 17.0±0.2, 17.4±0.2, 18.3±0.2, 18.9±0.2, 19.8±0.2, 21.8±0.2, and 22.1±0.2 degrees 2θ. In some such embodiments, the pattern B monocholine salt has an X-ray powder diffraction pattern comprising three or more peaks selected from the group consisting of 8.0±0.2, 9.4±0.2, 11.0±0.2, 13.0±0.2, 13.3±0.2, 13.7±0.2, 15.9±0.2, 17.0±0.2, 17.4±0.2, 18.3±0.2, 18.9±0.2, 19.8±0.2, 21.8±0.2, and 22.1±0.2 degrees 2θ. In other such embodiments, the pattern B monocholine salt has an X-ray powder diffraction pattern comprising five or more peaks selected from the group consisting of 8.0±0.2, 9.4±0.2, 11.0±0.2, 13.0±0.2, 13.3±0.2, 13.7±0.2, 15.9±0.2, 17.0±0.2, 17.4±0.2, 18.3±0.2, 18.9±0.2, 19.8±0.2, 21.8±0.2, and 22.1±0.2 degrees 2θ.

In some embodiments, the pattern B monocholine salt has an X-ray powder diffraction pattern substantially as shown in Figure **21**. The 2θ values for the peaks in Figure **21** (and their intensities) are as follows: 7.96 (41), 9.38 (34), 10.96 (24), 12.98 (76), 13.34 (33), 13.72 (37), 15.90 (100), 17.03 (60), 17.42 (37), 18.30 (31), 18.85 (93), 19.82 (90), 21.76 (38), and 22.06 (46).

This invention also relates, in part, to a process for preparing the pattern B monocholine salt. It was prepared by suspending amorphous choline salt in ethyl acetate for seven days.

### G6. Crystalline Form of N-(6-(3-Tert-butyl-5-(2,4-dioxo-3,4-dihydropyrimidin-1(2H)-yl)-2-methoxyphenyl)naphthalen-2-yl)methanesulfonamide, Dicholine Salt.

This invention also relates, in part, to a crystalline form of N-(6-(3-tert-butyl-5-(2,4-dioxo-3,4-dihydropyrimidin-1 (2H)-yl)-2-methoxyphenyl)naphthalen-2-yl)methanesulfonamide, dicholine salt.

In some embodiments, the dicholine salt has an X-ray powder diffraction pattern comprising one or more peaks selected from the group consisting of 8.6±0.2, 11.0±0.2, 12.9±0.2, 17.0±0.2, 17.5±0.2, 18.9±0.2, 19.8±0.2, and 21.9±0.2 degrees 2θ. In some such embodiments, the dicholine salt has an X-ray powder diffraction pattern comprising three or more peaks selected from the group consisting of 8.6±0.2, 11.0±0.2, 12.9±0.2, 17.0±0.2, 17.5±0.2, 18.9±0.2, 19.8±0.2, and 21.9±0.2 degrees 2θ. In other such embodiments, the dicholine salt has an X-ray powder diffraction pattern comprising five or more peaks selected from the group consisting of 8.6±0.2, 11.0±0.2, 12.9±0.2, 17.0±0.2, 17.5±0.2, 18.9±0.2, 19.8±0.2, and 21.9±0.2 degrees 2θ.

In some embodiments, the dicholine salt has an X-ray powder diffraction pattern comprising one or more peaks selected from the group consisting of 8.6±0.2, 11.0±0.2, 12.9±0.2, 17.0±0.2, 17.5±0.2, 18.9±0.2, 19.8±0.2, 21.9±0.2, and 22.1±0.2 degrees 2θ. In some such embodiments, the dicholine salt has an X-ray powder diffraction pattern comprising three or more peaks selected from the group consisting of 8.6±0.2, 11.0±0.2, 12.9±0.2, 17.0±0.2, 17.5±0.2, 18.9±0.2, 19.8±0.2, 21.9±0.2, and 22.1±0.2 degrees 2θ. In other such embodiments, the dicholine salt has an X-ray powder diffraction pattern comprising five or more peaks selected from the group consisting of 8.6±0.2, 11.0±0.2, 12.9±0.2, 17.0±0.2, 17.5±0.2, 18.9±0.2, 19.8±0.2, 21.9±0.2, and 22.1±0.2 degrees 2θ.

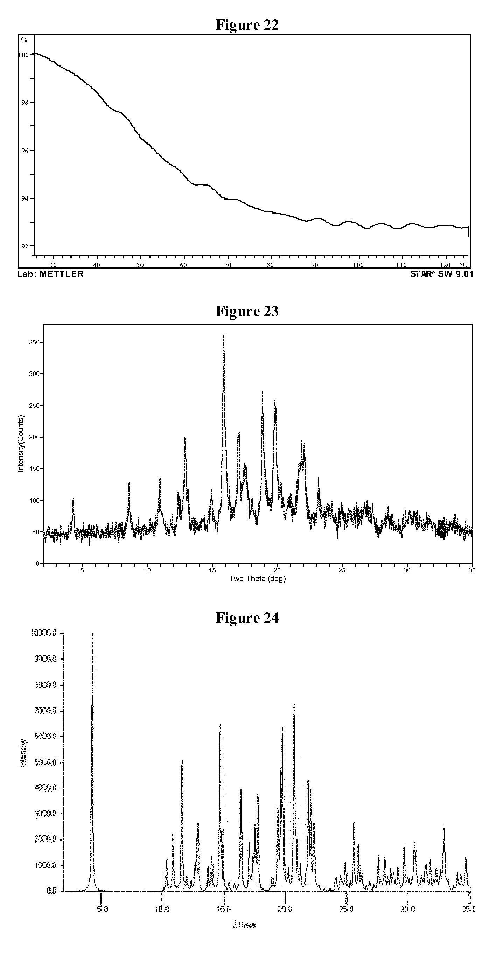

In some embodiments, the dicholine salt has an X-ray powder diffraction pattern substantially as shown in Figure **23**. The 2θ values for the peaks in Figure **23** (and their intensities) are as follows: 8.62 (28), 10.98 (29), 12.93 (50), 15.88 (100), 17.03 (42), 17.47 (29), 18.88 (66), 19.82 (57), 21.89 (42), 2.07 (41).

This invention also relates, in part, to a process for preparing the dicholine salt. It was prepared by suspending compound **IB-L0-2.3** (200mg) in 0.75ml MeOH. Choline hydroxide in MeOH (210ml, 45wt%, 2.10 molar equivalent) was added. The reaction mixture was concentrated, and 4ml acetonitrile and 6ml isopropyl acetate were added. The reaction mixture was then seeded with trace amount of the compound **IB-L0-2.3** monopotassium salt seed crystals (discussed above). The reaction mixture started to crystallize shortly after. The stoichiometry of the salt was determined by solution ¹H NMR.

### G7. Crystalline Forms of (E)-N-(4-(3-tert-butyl-5-(2,4-dioxo-3,4-dihydropyrimidin-1(2H)-yl)-2-methoxystyryl)phenyl)methanesulfonamide Disodium Salt.

This invention also relates, in part, to crystalline forms of (E)-N-(4-(3-tert-butyl-5-(2,4-dioxo-3,4-dihydropyrimidin-1(2H)-yl)-2-methoxystyryl)phenyl)methanesulfonamide disodium salt, namely the nonahydrate and tetrahydrate crystalline forms discussed below.

This invention relates, in part, to a nonahydrate crystalline disodium salt. The crystallographic unit cell parameters of the nonahydrate crystalline disodium salt have been determined to be as follows: a is 8.9Å, b is 9.4Å, and c is 20.7Å (more precisely, a is 8.926(2)Å, b is 9.415(2)Å, and c is 20.674(5)Å); the cell angles are: α - 94.8⁰, β - 93.3⁰, and γ - 107.0⁰ (more precisely, α is 94.796(4)⁰, β is 93.345(4)⁰, and γ is 107.013(4)⁰); and the cell volume is 1649Å³ (more precisely, 1649.3(7)Å³). The salt crystallizes in the P-1 space group.

In some embodiments, the disodium salt nonahydrate has an X-ray powder diffraction pattern comprising one or more peaks selected from the group consisting of 4.3±0.2, 10.4±0.2, 10.9±0.2,, 11.6±0.2, 12.9±0.2, 14.7±0.2, 16.4±0.2, 17.8±0.2, 19.4±0.2,19.8±0.2, 20.8±0.2, 21.9±0.2, and 23.5±0.2 degrees 2θ. In some such embodiments, the disodium salt nonahydrate has an X-ray powder diffraction pattern comprising three or more peaks selected from the group consisting of 4.3±0.2, 10.4±0.2, 10.9±0.2,, 11.6±0.2, 12.9±0.2, 14.7±0.2, 16.4±0.2, 17.8±0.2, 19.4±0.2, 19.8±0.2, 20.8±0.2, 21.9±0.2, and 23.5±0.2 degrees 2θ. In other such embodiments, the disodium salt nonahydrate has an X-ray powder diffraction pattern comprising five or more peaks selected from the group consisting of 4.3±0.2, 10.4±0.2, 10.9±0.2,, 11.6±0.2, 12.9±0.2, 14.7±0.2, 16.4±0.2, 17.8±0.2, 19.4±0.2, 19.8±0.2, 20.8±0.2, 21.9±0.2, and 23.5±0.2 degrees 20.

In some embodiments, the disodium salt nonahydrate has an X-ray powder diffraction pattern comprising one or more peaks selected from the group consisting of 4.3±0.2, 10.4±0.2, 10.9±0.2,, 11.6±0.2, 12.9±0.2, 14.7±0.2, 14.9±0.2, 16.4±0.2, 17.8±0.2, 19.4±0.2, 19.7±0.2, 19.8±0.2, 20.8±0.2, 20.9±0.2, 21.9±0.2, 22.1±0.2, and 23.5±0.2 degrees 2θ. In some such embodiments, the disodium salt nonahydrate has an X-ray powder diffraction pattern comprising three or more peaks selected from the group consisting of 4.3±0.2, 10.4±0.2, 10.9±0.2,, 11.6±0.2, 12.9±0.2, 14.7±0.2, 14.9±0.2, 16.4±0.2, 17.8±0.2, 19.4±0.2, 19.7±0.2, 19.8±0.2, 20.8±0.2, 20.9±0.2, 21.9±0.2, 22.1±0.2, and 23.5±0.2 degrees 20. In other such embodiments, the disodium salt nonahydrate has an X-ray powder diffraction pattern comprising five or more peaks selected from the group consisting of 4.3±0.2, 10.4±0.2, 10.9±0.2,, 11.6±0.2, 12.9±0.2, 14.7±0.2, 14.9±0.2, 16.4±0.2, 17.8±0.2, 19.4±0.2, 19.7±0.2, 19.8±0.2, 20.8±0.2, 20.9±0.2, 21.9±0.2, 22.1±0.2, and 23.5±0.2 degrees 2θ.

In some embodiments, the disodium salt nonahydrate has an X-ray powder diffraction pattern substantially as shown in Figure **24**. The 2θ values for the peaks in Figure **24** (and their intensities) are as follows: 4.31 (100), 10.36 (12), 10.91 (23), 11.61 (52), 12.93 (24), 14.73 (65), 14.89 (20), 16.44 (41), 17.80 (38), 19.44 (26), 19.67 (37), 19.83 (59), 20.75 (69), 20.89 (21), 21.92 (43), 22.13 (40), and 22.42 (24).

This invention also relates, in part, to a process for preparing the disodium salt nonahydrate. It was prepared in aqueous medium. Aqueous NaOH (1M, 1.18ml) was added to (E)-N-(4-(3-tert-butyl-5-(2,4-dioxo-3,4-dihydropyrimidin-1(2H)-yl)-2-methoxystyryl)phenyl)methanesulfonamide (compound **IB-L1-1.1**) (27.82mg) (molar ratio 1:20 acid:base). The resulting suspension was equilibrated at ambient conditions. The disodium salt nonahydrate formed seven days later through a solution-mediated process. Alternatively, the disodium salt nonahydrate was prepared by suspending 278.8mg of compound **IB-L1-1.1** in 1.25ml THF while heated to about 50°C. Aqueous NaOH (1N, 1.5ml, 2.2molar equivalents) was added. The solid dissolved completely to yield a clear solution, which was naturally cooled to ambient temperatures. The salt crystallized spontaneously. The molecular structure was determined by single crystal diffractometry.

This invention relates, in part, to a tetrahydrate crystalline disodium salt.

In some embodiments, the disodium salt tetrahydrate has an X-ray powder diffraction pattern comprising one or more peaks selected from the group consisting of 4.8±0.2, 12.1±0.2, 14.0±0.2, 17.0±0.2, 17.5±0.2, 20.9±0.2, 21.6±0.2, 25.0±0.2, and 29.5±0.2 degrees 2θ. In some such embodiments, the disodium salt tetrahydrate has an X-ray powder diffraction pattern comprising three or more peaks selected from the group consisting of 4.8±0.2, 12.1±0.2, 14.0±0.2, 17.0±0.2, 17.5±0.2, 20.9±0.2, 21.6±0.2, 25.0±0.2, and 29.5±0.2 degrees 20. In other such embodiments, the disodium salt tetrahydrate has an X-ray powder diffraction pattern comprising five or more peaks selected from the group consisting of 4.8±0.2, 12.1±0.2, 14.0±0.2, 17.0±0.2, 17.5±0.2, 20.9±0.2, 21.6±0.2, 25.0±0.2, and 29.5±0.2 degrees 20.

In some embodiments, the disodium salt tetrahydrate has an X-ray powder diffraction pattern comprising one or more peaks selected from the group consisting of 4.8±0.2, 12.1±0.2, 14.0±0.2, 14.4±0.2, 17.0±0.2, 17.5±0.2, 20.9±0.2, 21.6±0.2, 25.0±0.2, 29.5±0.2, and 34.2±0.2 degrees 20. In some such embodiments, the disodium salt tetrahydrate has an X-ray powder diffraction pattern comprising three or more peaks selected from the group consisting of 4.8±0.2, 12.1±0.2, 14.0±0.2, 14.4±0.2, 17.0±0.2, 17.5±0.2, 20.9±0.2, 21.6±0.2, 25.0±0.2, 29.5±0.2, and 34.2±0.2 degrees 20. In other such embodiments, the disodium salt tetrahydrate has an X-ray powder diffraction pattern comprising five or more peaks selected from the group consisting of 4.8±0.2, 12.1±0.2, 14.0±0.2, 14.4±0.2, 17.0±0.2, 17.5±0.2, 20.9±0.2, 21.6±0.2, 25.0±0.2, 29.5±0.2, and 34.2±0.2 degrees 20.

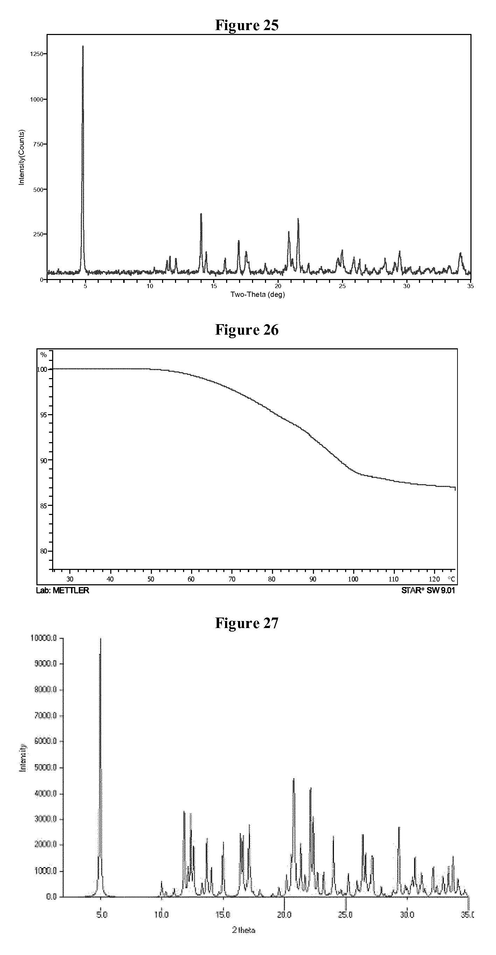

In some embodiments, the disodium salt tetrahydrate has an X-ray powder diffraction pattern substantially as shown in Figure **25**. The 20 values for the peaks in Figure **25** (and their intensities) are as follows: 4.81 (100), 12.07 (7), 14.01 (27), 14.41 (8), 16.96 (18), 17.53 (11), 20.87 (18), 21.58 (22), 24.99 (11), 29.47 (9), and 34.20 (9).

This invention also relates, in part, to a process for preparing the disodium salt tetrahydrate by suspending the nonahydrate disodium salt in an organic solvent (*e.g.*, ethanol, 1-propanol, or 2-propanol).

### G8. Crystalline Form of (E)-N-(4-(3-tert-butyl-5-(2,4-dioxo-3,4-dihydropyrimidin-1(2H)yl)-2-methoxystyryl)phenyl)methanesulfonamide Dipotassium Salt.

This invention also relates, in part, to a crystalline (E)-N-(4-(3-tert-butyl-5-(2,4-dioxo-3,4-dihydropyrimidin-1(2H)-yl)-2-methoxystyryl)phenyl)methanesulfonamide dipotassium salt tetrahydrate.

The crystallographic unit cell parameters of the dipotassium salt tetrahydrate have been determined to be as follows: a is 14.5Å, b is 10.8Å, and c is 35.8Å (more precisely, a is 14.454(14)Å, b is 10.763(14)Å, and c is 35.75(4)Å); the cell angle is: β - 98.8⁰ (more precisely, β is 98.82(3)⁰); and the cell volume is 5 499Å³ (more precisely, 5499(11)Å³) The salt crystallizes in the C2/c space group.

In some embodiments, the dipotassium salt tetrahydrate has an X-ray powder diffraction pattern comprising one or more peaks selected from the group consisting of 5.0±0.2, 11.9±0.2, 12.4±0.2, 13.7±0.2, 15.0±0.2, 16.5±0.2, 17.1±0.2, 20.8±0.2, 21.3±0.2, 22.2±0.2, 24.0±0.2, 26.4±0.2, and 29.3±0.2 degrees 20. In some such embodiments, the dipotassium salt tetrahydrate has an X-ray powder diffraction pattern comprising three or more peaks selected from the group consisting of 5.0±0.2, 11.9±0.2, 12.4±0.2, 13.7±0.2, 15.0±0.2, 16.5±0.2, 17.1±0.2, 20.8±0.2, 21.3±0.2, 22.2±0.2, 24.0±0.2, 26.4±0.2, and 29.3±0.2 degrees 2θ. In other such embodiments, the dipotassium salt tetrahydrate has an X-ray powder diffraction pattern comprising five or more peaks selected from the group consisting of 5.0±0.2, 11.9±0.2, 12.4±0.2, 13.7±0.2, 15.0±0.2, 16.5±0.2, 17.1±0.2, 20.8±0.2, 21.3±0.2, 22.2±0.2, 24.0±0.2, 26.4±0.2, and 29.3±0.2 degrees 2θ.

In some embodiments, the dipotassium salt tetrahydrate has an X-ray powder diffraction pattern comprising one or more peaks selected from the group consisting of 5.0±0.2, 11.9±0.2, 12.4±0.2, 12.6±0.2, 13.7±0.2, 15.0±0.2, 16.5±0.2, 16.7±0.2, 17.1±0.2, 20.7±0.2, 20.8±0.2, 21.3±0.2, 22.2±0.2, 22.4±0.2, 24.0±0.2, 26.4±0.2, and 29.3±0.2 degrees 2θ. In some such embodiments, the dipotassium salt tetrahydrate has an X-ray powder diffraction pattern comprising three or more peaks selected from the group consisting of 5.0±0.2, 11.9±0.2, 12.4±0.2, 12.6±0.2, 13.7±0.2, 15.0±0.2, 16.5±0.2, 16.7±0.2, 17.1±0.2, 20.7±0.2, 20.8±0.2, 21.3±0.2, 22.2±0.2, 22.4±0.2, 24.0±0.2, 26.4±0.2, and 29.3±0.2 degrees 2θ. In other such embodiments, the dipotassium salt tetrahydrate has an X-ray powder diffraction pattern comprising five or more peaks selected from the group consisting of 5.0±0.2, 11.9±0.2, 12.4±0.2, 12.6±0.2, 13.7±0.2, 15.0±0.2, 16.5±0.2, 16.7±0.2, 17.1±0.2, 20.7±0.2, 20.8±0.2, 21.3±0.2, 22.2±0.2, 22.4±0.2, 24.0±0.2, 26.4±0.2, and 29.3±0.2 degrees 20.

In some embodiments, the dipotassium salt tetrahydrate has an X-ray powder diffraction pattern substantially as shown in Figure **27**. The 2θ values for the peaks in Figure **27** (and their intensities) are as follows: 5.00 (100), 11.86 (34), 12.39 (32), 12.64 (19), 13.70 (23), 15.03 (21), 16.47 (24), 16.66 (24), 17.12 (28), 20.75 (29), 20.81 (33), 21.34 (22), 22.15 (46), 22.38 (31), 24.02 (24), 26.44 (24), and 29.32 (21).

This invention also relates, in part, to a process for preparing the dipotassium salt tetrahydrate by suspending compound **IB-L1-1.1** (261.13mg) in 1.25ml THF while heated to about 50°C. Aqueous KOH (1N, 1.3ml, 2.2 molar equivalent) was added. The solid dissolved completely to yield a clear solution, which was naturally cooled to ambient temperatures. Crystallization occurred during the slow evaporation process.

### G9. Crystalline Forms of (E)-N-(4-(3-tert-butyl-5-(2,4-dioxo-3,4-dihydropyrimidin-1-(2H)-yl)-2-methoxystyryl)phenyl)methanesulfonamide Monopotassium Salt.

This invention also relates, in part, to crystalline forms of (E)-N-(4-(3-tert-butyl-5-(2,4-dioxo-3,4-dihydropyrimidin-1(2H)-yl)-2-methoxystyryl)phenyl)methanesulfonamide monopotassium salt, namely the trihydrate and dihydrate crystalline forms discussed below.

This invention relates, in part, to a monopotassium salt trihydrate. The crystallographic unit cell parameters of the trihydrate crystalline monopotassium salt have been determined to be as follows: a is 9.0Å, b is 8.3Å, and c is 18.6Å (more precisely, a is 9.0393(16)Å, b is 8.3332(15)Å, and c is 18.582(3)Å); the cell angles are: α - 80.5°, β - 85.1°, and γ - 80.5° (more precisely, α is 80.511(2)⁰, β is 85.134(3)⁰, and γ is 80.531(2)⁰); and the cell volume is 1359Å³ (more precisely, 1359.3(4)Å³). The salt crystallizes in the P-1 space group.

In some embodiments, the monopotassium salt trihydrate has an X-ray powder diffraction pattern comprising one or more peaks selected from the group consisting of 4.8±0.2, 10.8±0.2, 11.3±0.2, 13.4±0.2, 15.3±0.2, 16.9±0.2, 21.2±0.2, 21.7±0.2, 22.1±0.2, 22.5±0.2, and 23.0±0.2 degrees 20. In some such embodiments, the monopotassium salt trihydrate has an X-ray powder diffraction pattern comprising three or more peaks selected from the group consisting of 4.8±0.2, 10.8±0.2, 11.3±0.2, 13.4±0.2, 15.3±0.2, 16.9±0.2, 21.2±0.2, 21.7±0.2, 22.1±0.2, 22.5±0.2, and 23.0±0.2 degrees 20. In other such embodiments, the monopotassium salt trihydrate has an X-ray powder diffraction pattern comprising five or more peaks selected from the group consisting of 4.8±0.2, 10.8±0.2, 11.3±0.2, 13.4±0.2, 15.3±0.2, 16.9±0.2, 21.2±0.2, 21.7±0.2, 22.1±0.2, 22.5±0.2, and 23.0±0.2 degrees 20.

In some embodiments, the monopotassium salt trihydrate has an X-ray powder diffraction pattern comprising one or more peaks selected from the group consisting of 4.8±0.2, 10.8±0.2, 11.3±0.2, 13.4±0.2, 13.6±0.2, 15.3±0.2, 16.9±0.2, 21.2±0.2, 21.7±0.2, 21.7±0.2, 22.1±0.2, 22.5±0.2, 22.6±0.2, and 23.0±0.2 degrees 20. In some such embodiments, the monopotassium salt trihydrate has an X-ray powder diffraction pattern comprising three or more peaks selected from the group consisting of 4.8±0.2, 10.8±0.2, 11.3±0.2, 13.4±0.2, 13.6±0.2, 15.3±0.2, 16.9±0.2, 21.2±0.2, 21.7±0.2, 21.7±0.2, 22.1±0.2, 22.5±0.2, 22.6±0.2, and 23.0±0.2 degrees 20. In other such embodiments, the monopotassium salt trihydrate has an X-ray powder diffraction pattern comprising five or more peaks selected from the group consisting of 4.8±0.2, 10.8±0.2, 11.3±0.2, 13.4±0.2, 13.6±0.2, 15.3±0.2, 16.9±0.2, 21.2±0.2, 21.7±0.2, 21.7±0.2, 22.1±0.2, 22.5±0.2, 22.6±0.2, and 23.0±0.2 degrees 20.

In some embodiments, the monopotassium salt trihydrate has an X-ray powder diffraction pattern comprising one or more peaks selected from the group consisting of 4.8±0.2, 10.8±0.2, 11.3±0.2, 13.4±0.2, 13.6±0.2, 15.3±0.2, 16.9±0.2, 21.2±0.2, 21.7±0.2, 21.7±0.2, 22.1±0.2, 22.5±0.2, 22.6±0.2, and 23.0±0.2 degrees 20. In some such embodiments, the monopotassium salt trihydrate has an X-ray powder diffraction pattern comprising three or more peaks selected from the group consisting of 4.8±0.2, 10.8±0.2, 11.3±0.2, 13.4±0.2, 15.3±0.2, 16.9±0.2, 21.2±0.2, 21.7±0.2, 22.1±0.2, 22.5±0.2, and 23.0±0.2 degrees 20. In other such embodiments, the monopotassium salt trihydrate has an X-ray powder diffraction pattern comprising five or more peaks selected from the group consisting of 4.8±0.2, 10.8±0.2, 11.3±0.2, 13.4±0.2, 15.3±0.2, 16.9±0.2, 21.2±0.2, 21.7±0.2, 22.1±0.2, 22.5±0.2, and 23.0±0.2 degrees 20.

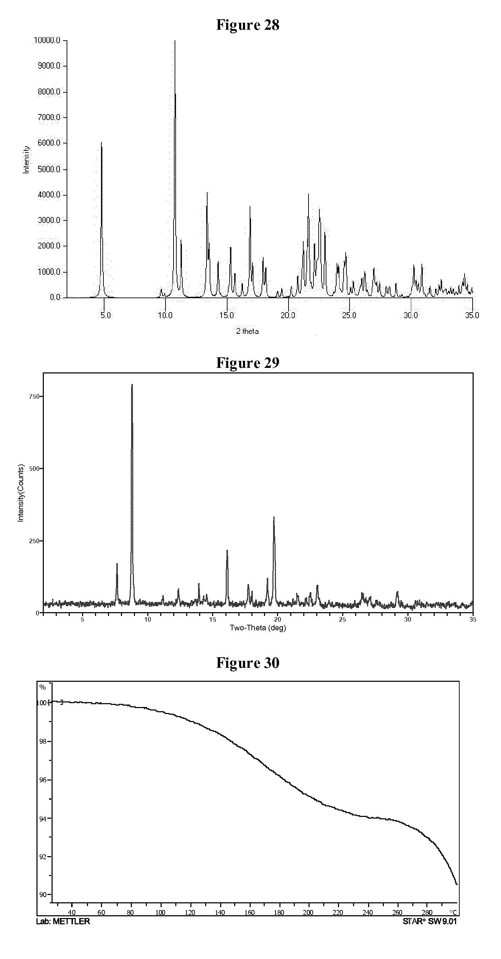

In some embodiments, the monopotassium salt trihydrate has an X-ray powder diffraction pattern substantially as shown in Figure **28**. The 2θ values for the peaks in Figure **28** (and their intensities) are as follows: 4.83 (60), 10.79 (100), 11.31 (22), 13.42 (41), 13.59 (18), 15.32 (21), 16.90 (38), 21.24 (22), 21.68 (20), 21.68 (21), 22.15 (22), 22.55 (29), 22.63 (23), and 23.02 (27).

This invention also relates, in part, to a process for preparing the monopotassium salt trihydrate. It was prepared by suspending compound **IB-L1-1.1** (108.81mg) in 0.4ml THF while heated to about 50°C. Aqueous KOH solution (1N, 0.278ml, 1.2 molar equivalent) was added. The solid dissolved completely to yield a clear solution. Additional 1.6ml THF was added to the solution, which was then naturally cooled to ambient temperatures and crystallization was observed. Alternatively, the monopotassium salt trihydrate was prepared by suspending compound **IB-L1-1.1** (343.89mg) in 1.0ml THF while heated to 50°C. Aqueous KOH (1 N, 0.878ml, 1.2 molar equivalent) was added. The solid dissolved completely to yield a clear solution. Ethanol was added to the solution dropwise to a total volume of 4.0ml. The solution was then naturally cooled to ambient temperature and crystallization was observed.

This invention relates, in part, to a monopotassium salt dihydrate.

In some embodiments, the monopotassium salt dihydrate has an X-ray powder diffraction pattern comprising one or more peaks selected from the group consisting of 7.7±0.2, 8.8±0.2, 16.1±0.2, and 19.7±0.2 degrees 20. In some such embodiments, the monopotassium salt dihydrate has an X-ray powder diffraction pattern comprising three or more peaks selected from the group consisting of degrees 20.

In some embodiments, the monopotassium salt dihydrate has an X-ray powder diffraction pattern comprising one or more peaks selected from the group consisting of 7.7±0.2, 8.8±0.2, 12.4±0.2, 14.0±0.2, 16.1±0.2, 17.7±0.2, 19.2±0.2, 19.7±0.2, 23.1±0.2, and 29.2±0.2 degrees 20. In some such embodiments, the monopotassium salt dihydrate has an X-ray powder diffraction pattern comprising three or more peaks selected from the group consisting of 7.7±0.2, 8.8±0.2, 12.4±0.2, 14.0±0.2, 16.1±0.2, 17.7±0.2, 19.2±0.2, 19.7±0.2, 23.1±0.2, and 29.2±0.2 degrees 20. In other such embodiments, the monopotassium salt dihydrate has an X-ray powder diffraction pattern comprising five or more peaks selected from the group consisting of 7.7±0.2, 8.8±0.2, 12.4±0.2, 14.0±0.2, 16.1±0.2, 17.7±0.2, 19.2±0.2, 19.7±0.2, 23.1±0.2, and 29.2±0.2 degrees 20.

In some embodiments, the monopotassium salt dihydrate has an X-ray powder diffraction pattern substantially as shown in Figure **29**. The 20 values for the peaks in Figure **29** (and their intensities) are as follows: 7.68 (19), 8.83 (100), 12.40 (7), 13.97 (10), 16.12 (25), 17.75 (9), 19.22 (12), 19.73 (40), 23.05 (9), and 29.21 (7).

This invention also relates, in part, to a process for preparing the monopotassium salt dihydrate. It was prepared by suspending the monopotassium salt trihydrate in media of low water activity, such as an ethanol/H₂O mixture (50/1 v/v). Alternatively, the monopotassium salt dihydrate was prepared by dissolving potassium trihydrate solid (1.8g) in 36mL of IPA and 4ml water at 80°C. The resulting solution was cooled to 55°C over 1h. The solution was then seeded with 7.5mg of dihydrate crystals at 55°C and maintained at 55°C for 1h. Heptane (36ml) was then added over 3h. The reaction mixture was cooled to 0°C, and filtration yielded a material containing both di- and trihydrate crystals. The solid was then reslurried in 20mL of 10:1 v/v EtOH/H₂O at 50°C for 3h and cooled to 25°C over 5h. The slurry was then mixed at 25°C for additional 3 days and cooled to 0°C over 3h and held at this temperature for 2h. The resulting crystals were filtered and air-dried on filter funnel for 1h to give dihydrate. The dihydrate monopotassium salt was also prepared by slurrying a mixture of dihydrate and trihydrate crystals in 10:1 v/v EtOH/H₂O at 80°C for 2 days. The potassium content was confirmed by ion chromatography.

### G10. Crystalline Form of (E)-N-(4-(3-tert-butyl-5-(2,4-dioxo-3,4-dihydropyrimidin-1(2H)-yl)-2-methoxystyryl)phenyl)methanesulfonamide 1/7 Potassium Salt.

This invention also relates, in part, to a crystalline form of (E)-N-(4-(3-tert-butyl-5-(2,4-dioxo-3,4-dihydropyrimidin-1(2H)-yl)-2-methoxystyryl)phenyl)methanesulfonamide 1/7 potassium salt.

In some embodiments, the 1/7 potassium salt has an X-ray powder diffraction pattern comprising one or more peaks selected from the group consisting of 7.7±0.2, 8.3±0.2, 10.1±0.2, 10.6±0.2, 11.4±0.2, 12.0±0.2, 13.4±0.2, 15.6±0.2, 16.3±0.2, 16.7±0.2, 17.2±0.2, 18.3±0.2, 18.8±0.2, 19.4±0.2, 19.9±0.2, 20.2±0.2, 20.5±0.2, 21.2±0.2, 22.1±0.2, and 22.9±0.2 degrees 2θ. In some such embodiments, the 1/7 potassium salt has an X-ray powder diffraction pattern comprising three or more peaks selected from the group consisting of 7.7±0.2, 8.3±0.2, 10.1±0.2, 10.6±0.2, 11.4±0.2, 12.0±0.2, 13.4±0.2, 15.6±0.2, 16.3±0.2, 16.7±0.2, 17.2±0.2, 18.3±0.2, 18.8±0.2, 19.4±0.2, 19.9±0.2, 20.2±0.2, 20.5±0.2, 21.2±0.2, 22.1±0.2, and 22.9±0.2 degrees 2θ. In other such embodiments, the 1/7 potassium salt has an X-ray powder diffraction pattern comprising five or more peaks selected from the group consisting of 7.7±0.2, 8.3±0.2, 10.1±0.2, 10.6±0.2, 11.4±0.2, 12.0±0.2, 13.4±0.2, 15.6±0.2, 16.3±0.2, 16.7±0.2, 17.2±0.2, 18.3±0.2, 18.8±0.2, 19.4±0.2, 19.9±0.2, 20.2±0.2, 20.5±0.2, 21.2±0.2, 22.1±0.2, and 22.9±0.2 degrees 2θ.

In some embodiments, the 1/7 potassium salt has an X-ray powder diffraction pattern comprising one or more peaks selected from the group consisting of 7.7±0.2, 8.3±0.2, 10.1±0.2, 10.6±0.2, 11.4±0.2, 12.0±0.2, 13.4±0.2, 15.6±0.2, 16.3±0.2, 16.7±0.2, 17.2±0.2, 18.3±0.2, 18.8±0.2, 19.4±0.2, 19.9±0.2, 20.2±0.2, 20.5±0.2, 20.8±0.2, 21.2±0.2, 22.1±0.2, 22.9±0.2, 24.3±0.2, 24.9±0.2, and 25.1±0.2 degrees 2θ. In some such embodiments, the 1/7 potassium salt has an X-ray powder diffraction pattern comprising three or more peaks selected from the group consisting of 7.7±0.2, 8.3±0.2, 10.1±0.2, 10.6±0.2, 11.4±0.2, 12.0±0.2, 13.4±0.2, 15.6±0.2, 16.3±0.2, 16.7±0.2, 17.2±0.2, 18.3±0.2, 18.8±0.2, 19.4±0.2, 19.9±0.2, 20.2±0.2, 20.5±0.2, 20.8±0.2, 21.2±0.2, 22.1±0.2, 22.9±0.2, 24.3±0.2, 24.9±0.2, and 25.1±0.2 degrees 2θ. In other such embodiments, the 1/7 potassium salt has an X-ray powder diffraction pattern comprising five or more peaks selected from the group consisting of 7.7±0.2, 8.3±0.2, 10.1±0.2, 10.6±0.2, 11.4±0.2, 12.0±0.2, 13.4±0.2, 15.6±0.2, 16.3±0.2, 16.7±0.2, 17.2±0.2, 18.3±0.2, 18.8±0.2, 19.4±0.2, 19.9±0.2, 20.2±0.2, 20.5±0.2, 20.8±0.2, 21.2±0.2, 22.1±0.2, 22.9±0.2, 24.3±0.2, 24.9±0.2, and 25.1±0.2 degrees 2θ.

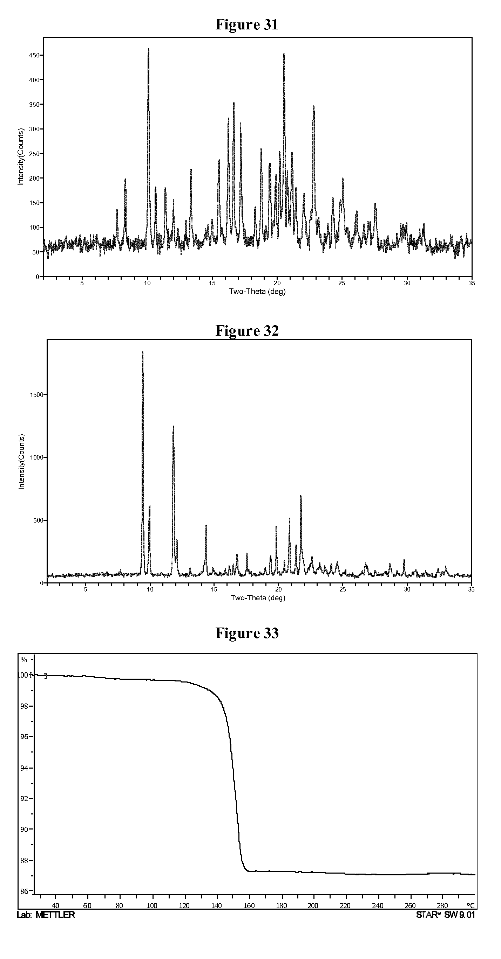

In some embodiments, the 1/7 potassium salt has an X-ray powder diffraction pattern substantially as shown in Figure **31**. The 2θ values for the peaks in Figure **31** (and their intensities) are as follows: 7.71 (19), 8.33 (34), 10.10 (100), 10.66 (29), 11.39 (27), 12.04 (22), 13.39 (39), 15.56 (41), 16.27 (62), 16.69 (70), 17.22 (59), 18.31 (18), 18.78 (47), 19.44 (36), 19.89 (28), 20.19 (33), 20.54 (87), 20.80 (33), 21.15 (47), 22.05 (24), 22.82 (67), 24.32 (22), 24.87 (22), and 25.07 (33).

This invention also relates, in part, to a process for preparing the 1/7 potassium salt. It was prepared by suspending compound **IB-LI-1.1** (2g) 6ml THF at 50°C. One molar equivalent of KOH dissolved in 4.3ml water was added, and the reaction mixture was heated to 65°C to dissolve all solids. The solution was then cooled to ambient temperatures over 2h and spontaneous crystallization took place. The slurry was then cooled to 5°C and held at that temperature for 2h. The pale yellow crystals were filtered and air-dried for 24h at ambient conditions. The potassium content was determined by ion chromatography.

### G11. Crystalline Form of (E)-N-(4-(3-tert-butyl-5-(2,4-dioxo-3,4-dihydropyrimidin-1(2H)yl)-2-methoxystyryl)phenyl)methanesulfonamide Monodiethylamine Salt Tetrahydrate.

This invention also relates, in part, to crystalline (E)-N-(4-(3-tert-butyl-5-(2,4-dioxo-3,4-dihydropyrimidin-1(2H)-yl)-2-methoxystyryl)phenyl)methanesulfonamide monodiethylamine salt tetrahydrate.

In some embodiments, the monodiethylamine salt tetrahydrate has an X-ray powder diffraction pattern comprising one or more peaks selected from the group consisting of 9.5±0.2, 10.0±0.2, 11.8±0.2, 12.1±0.2, 14.4±0.2, 16.8±0.2, 17.6±0.2, 19.8±0.2, 20.8±0.2, 21.4±0.2, 21.8±0.2, and 29.8±0.2 degrees 20. In some such embodiments, the monodiethylamine salt tetrahydrate has an X-ray powder diffraction pattern comprising three or more peaks selected from the group consisting of 9.5±0.2, 10.0±0.2, 11.8±0.2, 12.1±0.2, 14.4±0.2, 16.8±0.2, 17.6±0.2, 19.8±0.2, 20.8±0.2, 21.4±0.2, 21.8±0.2, and 29.8±0.2 degrees 20. In other such embodiments, the monodiethylamine salt tetrahydrate has an X-ray powder diffraction pattern comprising five or more peaks selected from the group consisting of 9.5±0.2, 10.0±0.2, 11.8±0.2, 12.1±0.2, 14.4±0.2, 16.8±0.2, 17.6±0.2, 19.8±0.2, 20.8±0.2, 21.4±0.2, 21.8±0.2, and 29.8±0.2 degrees 20.

In some embodiments, the monodiethylamine salt tetrahydrate has an X-ray powder diffraction pattern comprising one or more peaks selected from the group consisting of 9.5±0.2, 10.0±0.2, 11.8±0.2, 12.1±0.2, 14.4±0.2, 16.8±0.2, 17.6±0.2, 19.4±0.2, 19.8±0.2, 20.8±0.2, 21.4±0.2, 21.8±0.2, 21.9±0.2, and 29.8±0.2 degrees 20. In some such embodiments, the monodiethylamine salt tetrahydrate has an X-ray powder diffraction pattern comprising three or more peaks selected from the group consisting of 9.5±0.2, 10.0±0.2, 11.8±0.2, 12.1±0.2, 14.4±0.2, 16.8±0.2, 17.6±0.2, 19.4±0.2, 19.8±0.2, 20.8±0.2, 21.4±0.2, 21.8±0.2, 21.9±0.2, and 29.8±0.2 degrees 2θ. In other such embodiments, the monodiethylamine salt tetrahydrate has an X-ray powder diffraction pattern comprising five or more peaks selected from the group consisting of 9.5±0.2, 10.0±0.2, 11.8±0.2, 12.1±0.2, 14.4±0.2, 16.8±0.2, 17.6±0.2, 19.4±0.2, 19.8±0.2, 20.8±0.2, 21.4±0.2, 21.8±0.2, 21.9±0.2, and 29.8±0.2 degrees 20.

In some embodiments, the monodiethylamine salt tetrahydrate has an X-ray powder diffraction pattern substantially as shown in Figure **32**. The 2θ values for the peaks in Figure **32** (and their intensities) are as follows: 9.45 (100), 9.97 (31), 11.85 (67), 12.09 (16), 14.38 (22), 16.80 (9), 17.59 (10), 19.39 (8), 19.83 (21), 20.85 (25), 21.37 (12), 21.75 (34), 21.87 (8), and 29.78 (7).

This invention also relates, in part, to a process for preparing the monodiethylamine salt tetrahydrate. It was prepared in aqueous medium. Compound **IB-L1-1.1** was slowly added to 500ul of 1M diethylamine until no more solid can be dissolved into the solution. The solution was then evaporated slowly at ambient temperatures and the salt crystallized 2 days later. Alternatively, the monodiethylamine salt tetrahydrate was prepared by suspending 64.15mg of compound **IB-L1-1.1** in 400ul 1M diethylamine while heated to 50°C. About 5 drops of THF (∼ 20ul) was added. The solid dissolved completely upon addition to yield a clear solution. The solution was then evaporated at ambient temperature, and the salt crystallized 4 days later. The stoichiometry of the salt was confirmed by solution ¹H NMR.

### G12. Crystalline Forms of (E)-N-(4-(3-tert-butyl-5-(2,4-dioxo-3,4-dihydropyrimidin-1(2H)yl)-2-methoxystyryl)phenyl)methahesulfonamide (compound IB-L1-1.1).

This invention also relates, in part, to crystalline forms of (E)-N-(4-(3-tert-butyl-5-(2,4-dioxo-3,4-dihydropyrimidin-1(2H)-yl)-2-methoxystyryl)phenyl)methanesulfonamide (compound **IB-L1-1.1**), namely the true polymorphs (pattern A, pattern B, pattern C, and pattern D) and hydrate (pattern AH, pattern BH, pattern CH, and pattern DH) crystalline forms discussed below.

### G12A. IB-L1-1.1 True Polymorphs.

This invention relates, in part, to pattern A crystalline (E)-N-(4-(3-tert-butyl-5-(2,4-dioxo-3,4-dihydropyrimidin-1(2H)-yl)-2-methoxystyryl)phenyl)methanesulfonamide.

In some embodiments, the pattern A polymorph has an X-ray powder diffraction pattern comprising one or more peaks selected from the group consisting of 5.8±0.2, 9.9±0.2, 11.8±0.2, 12.4±0.2, 14.5±0.2, 18.8±0.2, 22.7±0.2, and 29.2±0.2 degrees 20. In some such embodiments, the pattern A polymorph has an X-ray powder diffraction pattern comprising three or more peaks selected from the group consisting of 5.8±0.2, 9.9±0.2, 11.8±0.2, 12.4±0.2, 14.5±0.2, 18.8±0.2, 22.7±0.2, and 29.2±0.2 degrees 20. In other such embodiments, the pattern A polymorph has an X-ray powder diffraction pattern comprising five or more peaks selected from the group consisting of 5.8±0.2, 9.9±0.2, 11.8±0.2, 12.4±0.2, 14.5±0.2, 18.8±0.2, 22.7±0.2, and 29.2±0.2 degrees 20.

In some embodiments, the pattern A polymorph has an X-ray powder diffraction pattern comprising one or more peaks selected from the group consisting of 5.8±0.2, 9.9±0.2, 11.8±0.2, 12.4±0.2, 14.0±0.2, 14.5±0.2, 15.3±0.2, 18.5±0.2, 18.8±0.2, 22.2±0.2, 22.7±0.2, 23.8±0.2, 26.0±0.2, and 29.2±0.2 degrees 2θ. In some such embodiments, the pattern A polymorph has an X-ray powder diffraction pattern comprising three or more peaks selected from the group consisting of 5.8±0.2, 9.9±0.2, 11.8±0.2, 12.4±0.2, 14.0±0.2, 14.5±0.2, 15.3±0.2, 18.5±0.2, 18.8±0.2, 22.2±0.2, 22.7±0.2, 23.8±0.2, 26.0±0.2, and 29.2±0.2 degrees 2θ. In other such embodiments, the pattern A polymorph has an X-ray powder diffraction pattern comprising five or more peaks selected from the group consisting of 5.8±0.2, 9.9±0.2, 11.8±0.2, 12.4±0.2, 14.0±0.2, 14.5±0.2, 15.3±0.2, 18.5±0.2, 18.8±0.2, 22.2±0.2, 22.7±0.2, 23.8±0.2, 26.0±0.2, and 29.2±0.2 degrees 2θ.

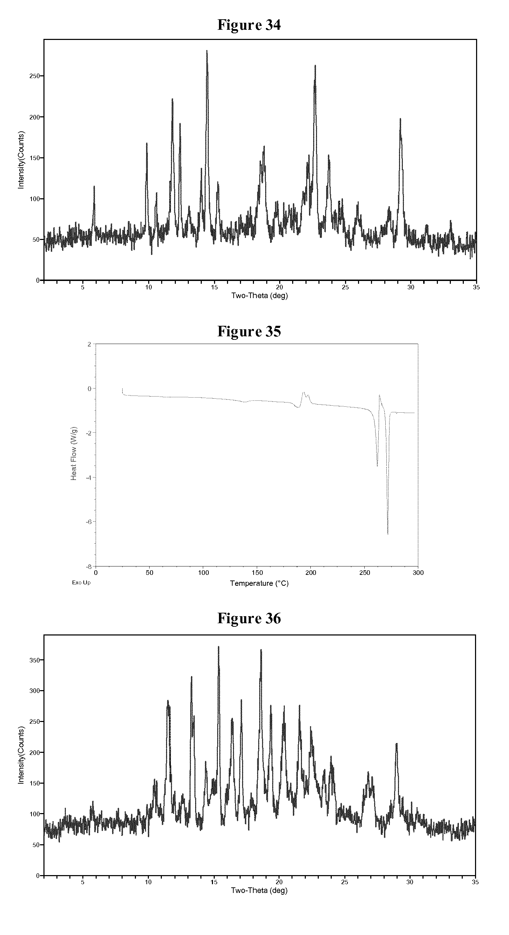

In some embodiments, the pattern A polymorph has an X-ray powder diffraction pattern substantially as shown in Figure **34**. The 2θ values for the peaks in Figure **34** (and their intensities) are as follows: 5.85 (28), 9.88 (51), 11.79 (73), 12.38 (56), 14.03 (38), 14.45 (100), 15.27 (29), 18.52 (39), 18.80 (47), 22.24 (40), 22.72 (77), 23.76 (39), 25.98 (22), and 29.21 (64).

This invention also relates, in part, to a process for preparing pattern A polymorph. Pattern A polymorph was prepared as discussed in **Example F** below.

This invention relates, in part, to pattern B crystalline (E)-N-(4-(3-tert-butyl-5-(2,4-dioxo-3,4-dihydropyrimidin-1(2H)-yl)-2-methoxystyryl)phenyl)methanesulfonamide.

In some embodiments, the pattern B polymorph has an X-ray powder diffraction pattern comprising one or more peaks selected from the group consisting of 11.5±0.2, 13.3±0.2, 15.4±0.2, 16.4±0.2, 17.1±0.2, 18.6±0.2, 19.4±0.2, 20.4±0.2, 21.6±0.2, 22.4±0.2, 24.0±0.2, 26.8±0.2, and 29.0±0.2 degrees 20. In some such embodiments, the pattern B polymorph has an X-ray powder diffraction pattern comprising three or more peaks selected from the group consisting of 11.5±0.2, 13.3±0.2, 15.4±0.2, 16.4±0.2, 17.1±0.2, 18.6±0.2, 19.4±0.2, 20.4±0.2, 21.6±0.2, 22.4±0.2, 24.0±0.2, 26.8±0.2, and 29.0±0.2 degrees 2θ. In other such embodiments, the pattern B polymorph has an X-ray powder diffraction pattern comprising five or more peaks selected from the group consisting of 11.5±0.2, 13.3±0.2, 15.4±0.2, 16.4±0.2, 17.1±0.2, 18.6±0.2, 19.4±0.2, 20.4±0.2, 21.6±0.2, 22.4±0.2, 24.0±0.2, 26.8±0.2, and 29.0±0.2 degrees 2θ.

In some embodiments, the pattern B polymorph has an X-ray powder diffraction pattern substantially as shown in Figure **36**. The 2θ values for the peaks in Figure **36** (and their intensities) are as follows: 11.52 (71), 13.30 (87), 15.37 (100), 16.42 (60), 17.13 (69), 18.60 (97), 19.37 (56), 20.40 (62), 21.55 (55), 22.41 (39), 23.99 (33), 26.81 (31), and 28.98 (50).

This invention relates, in part, to pattern C crystalline (E)-N-(4-(3-tert-butyl-5-(2,4-dioxo-3,4-dihydropyrimidin-1(2H)-yl)-2-methoxystyryl)phenyl)methanesulfonamide.

In some embodiments, the pattern C polymorph has an X-ray powder diffraction pattern comprising one or more peaks selected from the group consisting of 7.7±0.2, 10.1±0.2, 10.6±0.2, 12.0 ±0.2, 13.4±0.2, 16.2±0.2, 19.4±0.2, 20.5±0.2, 21.4±0.2, 22.0±0.2, 22.6±0.2, 24.3±0.2, and 27.6±0.2 degrees 20. In some such embodiments, the pattern C polymorph has an X-ray powder diffraction pattern comprising three or more peaks selected from the group consisting of 7.7±0.2, 10.1±0.2, 10.6±0.2, 12.0 ±0.2, 13.4±0.2, 16.2±0.2, 19.4±0.2, 20.5±0.2, 21.4±0.2, 22.0±0.2, 22.6±0.2, 24.3±0.2, and 27.6±0.2 degrees 20. In other such embodiments, the pattern C polymorph has an X-ray powder diffraction pattern comprising five or more peaks selected from the group consisting of 7.7±0.2, 10.1±0.2, 10.6±0.2, 12.0 ±0.2, 13.4±0.2, 16.2±0.2, 19.4±0.2, 20.5±0.2, 21.4±0.2, 22.0±0.2, 22.6±0.2, 24.3±0.2, and 27.6±0.2 degrees 20.

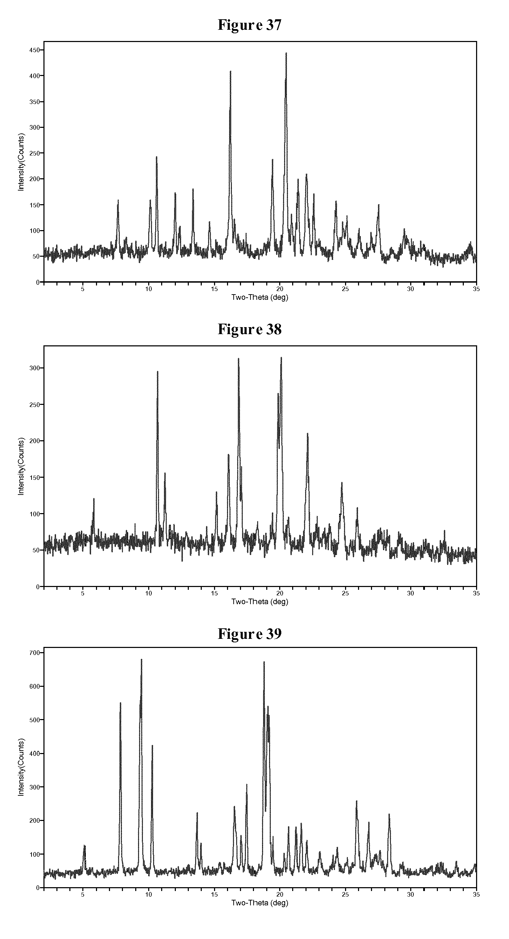

In some embodiments, the pattern C polymorph has an X-ray powder diffraction pattern substantially as shown in Figure **37**. The 20 values for the peaks in Figure **37** (and their intensities) are as follows: 7.69 (27), 10.13 (27), 10.64 (49), 12.01 (31), 13.39 (33), 16.25 (91), 19.44 (46), 20.49 (100), 21.40 (35), 22.03 (37), 22.60 (30), 24.32 (23), and 27.55 (27).

This invention relates, in part, to pattern D crystalline (E)-N-(4-(3-tert-butyl-5-(2,4-dioxo-3,4-dihydropyrimidin-1(2H)-yl)-2-methoxystyryl)phenyl)methanesulfonamide.

In some embodiments, the pattern D polymorph has an X-ray powder diffraction pattern comprising one or more peaks selected from the group consisting of 5.8±0.2, 10.7±0.2, 11.2±0.2, 15.2±0.2, 16.1±0.2, 16.9±0.2, 19.9±0.2, 22.1±0.2, 24.7±0.2, and 26.0±0.2 degrees 20. In some such embodiments, the pattern D polymorph has an X-ray powder diffraction pattern comprising three or more peaks selected from the group consisting of 5.8±0.2, 10.7±0.2, 11.2±0.2, 15.2±0.2, 16.1±0.2, 16.9±0.2, 19.9±0.2, 22.1±0.2, 24.7±0.2, and 26.0±0.2 degrees 2θ. In other such embodiments, the pattern D polymorph has an X-ray powder diffraction pattern comprising five or more peaks selected from the group consisting of 5.8±0.2, 10.7±0.2, 11.2±0.2, 15.2±0.2, 16.1±0.2, 16.9±0.2, 19.9±0.2, 22.1±0.2, 24.7±0.2, and 26.0±0.2 degrees 20.

In some embodiments, the pattern D polymorph has an X-ray powder diffraction pattern comprising one or more peaks selected from the group consisting of 5.8±0.2, 10.7±0.2, 11.2±0.2, 15.2±0.2, 16.1±0.2, 16.9±0.2, 17.1±0.2, 19.9±0.2, 20.1±0.2, 22.1±0.2, 24.7±0.2, and 26.0±0.2 degrees 20. In some such embodiments, the pattern D polymorph has an X-ray powder diffraction pattern comprising three or more peaks selected from the group consisting of 5.8±0.2, 10.7±0.2, 11.2±0.2, 15.2±0.2, 16.1±0.2, 16.9±0.2, 17.1±0.2, 19.9±0.2, 20.1±0.2, 22.1±0.2, 24.7±0.2, and 26.0±0.2 degrees 2θ. In other such embodiments, the pattern D polymorph has an X-ray powder diffraction pattern comprising five or more peaks selected from the group consisting of 5.8±0.2, 10.7±0.2, 11.2±0.2, 15.2±0.2, 16.1±0.2, 16.9±0.2, 17.1±0.2, 19.9±0.2, 20.1±0.2, 22.1±0.2, 24.7±0.2, and 26.0±0.2 degrees 20.

In some embodiments, the pattern D polymorph has an X-ray powder diffraction pattern substantially as shown in Figure **38**. The 2θ values for the peaks in Figure **38** (and their intensities) are as follows: 5.81 (24), 10.70 (91), 11.23 (60), 15.17 (28), 16.10 (48), 16.89 (100), 17.10 (42), 19.88 (81), 20.12 (100), 22.12 (59), 24.72 (37), and 25.91 (24).

This invention also relates, in part, to a process for preparing pattern B, C, and D polymorphs by heating pattern A polymorph to about 160, about 225, and about 268⁰C, respectively using DSC.

### G12B. IB-L1-1.1 Hydrates.

This invention also relates, in part, to hydrates of compound **IB-L1-1.1**, namely to hydrates A, B, C, D, and E discussed below.

This invention relates, in part, to a pattern A (E)-N-(4-(3-tert-butyl-5-(2,4-dioxo-3,4-dihydro-pyrimidin-1(2H)-yl)-2-methoxystyryl)phenyl)methanesulfonamide hydrate.

In some embodiments, the pattern A hydrate has an X-ray powder diffraction pattern comprising one or more peaks selected from the group consisting of 5.1±0.2, 7.9±0.2, 9.5±0.2, 10.3±0.2, 13.7±0.2, 16.5±0.2, 17.1±0.2, 17.5±0.2, 18.8±0.2, 19.2±0.2, 20.7±0.2, 21.3±0.2, 21.6±0.2, 25.8±0.2, 26.8±0.2, and 28.4±0.2 degrees 2θ. In some such embodiments, the pattern A hydrate has an X-ray powder diffraction pattern comprising three or more peaks selected from the group consisting of 5.1±0.2, 7.9±0.2, 9.5±0.2, 10.3±0.2, 13.7±0.2, 16.5±0.2, 17.1±0.2, 17.5±0.2, 18.8±0.2, 19.2±0.2, 20.7±0.2, 21.3±0.2, 21.6±0.2, 25.8±0.2, 26.8±0.2, and 28.4±0.2 degrees 2θ. In other such embodiments, the pattern A hydrate has an X-ray powder diffraction pattern comprising five or more peaks selected from the group consisting of 5.1±0.2, 7.9±0.2, 9.5±0.2, 10.3±0.2, 13.7±0.2, 16.5±0.2, 17.1±0.2, 17.5±0.2, 18.8±0.2, 19.2±0.2, 20.7±0.2, 21.3±0.2, 21.6±0.2, 25.8±0.2, 26.8±0.2, and 28.4±0.2 degrees 2θ.

In some embodiments, the pattern A hydrate has an X-ray powder diffraction pattern substantially as shown in Figure **39**. The 2θ values for the peaks in Figure **39** (and their intensities) are as follows: 5.13 (13), 7.87 (80), 9.45 (100), 10.29 (60), 13.7 (28), 16.54 (30), 17.07 (17), 17.51 (40), 18.80 (99), 19.18 (74), 20.69 (21), 21.25 (21), 21.63 (23), 25.85 (32), 26.81 (20), and 28.35 (27).

This invention also relates, in part, to a process for preparing the pattern A hydrate by suspending pattern A polymorph (discussed above) in ethyl acetate. The recovered pattern A hydrate contains ∼1 water molecules per molecule of compound **IB-L1-1.1**.

This invention also relates, in part, to a pattern B (E)-N-(4-(3-tert-butyl-5-(2,4-dioxo-3,4-dihydro-pyrimidin-1(2H)-yl)-2-methoxystyryl)phenyl)methanesulfonamide hydrate.

In some embodiments, the pattern B hydrate has an X-ray powder diffraction pattern comprising one or more peaks selected from the group consisting of 6.3±0.2, 7.7±0.2, 10.4±0.2, 12.7±0.2, 13.3±0.2, 14.9±0.2, 15.4±0.2, 16.4±0.2, 18.6±0.2, 18.9±0.2, 19.4±0.2, 22.5±0.2, 23.5±0.2, 24.0±0.2, 26.8±0.2, and 29.0±0.2 degrees 2θ. In some such embodiments, the pattern B hydrate has an X-ray powder diffraction pattern comprising three or more peaks selected from the group consisting of 6.3±0.2, 7.7±0.2, 10.4±0.2, 12.7±0.2, 13.3±0.2, 14.9±0.2, 15.4±0.2, 16.4±0.2, 18.6±0.2, 18.9±0.2, 19.4±0.2, 22.5±0.2, 23.5±0.2, 24.0±0.2, 26.8±0.2, and 29.0±0.2 degrees 2θ. In other such embodiments, the pattern B hydrate has an X-ray powder diffraction pattern comprising five or more peaks selected from the group consisting of 6.3±0.2, 7.7±0.2, 10.4±0.2, 12.7±0.2, 13.3±0.2, 14.9±0.2, 15.4±0.2, 16.4±0.2, 18.6±0.2, 18.9±0.2, 19.4±0.2, 22.5±0.2, 23.5±0.2, 24.0±0.2, 26.8±0.2, and 29.0±0.2 degrees 2θ.

In some embodiments, the pattern B hydrate has an X-ray powder diffraction pattern comprising one or more peaks selected from the group consisting of 6.3±0.2, 7.7±0.2, 10.4±0.2, 12.7±0.2, 13.3±0.2, 13.5±0.2, 14.9±0.2, 15.4±0.2, 16.4±0.2, 18.5±0.2, 18.6±0.2, 18.9:1:0.2, 19.4±0.2, 22.5±0.2, 23.5±0.2, 24.0±0.2, 26.8±0.2, and 29.0±0.2 degrees 2θ. In some such embodiments, the pattern B hydrate has an X-ray powder diffraction pattern comprising three or more peaks selected from the group consisting of 6.3±0.2, 7.7±0.2, 10.4±0.2, 12.7±0.2, 13.3±0.2, 13.5±0.2, 14.9±0.2, 15.4±0.2, 16.4±0.2, 18.5±1:0.2, 18.6±0.2, 18.9±0.2, 19.4±0.2, 22.5±0.2, 23.5±0.2, 24.0±0.2, 26.8±0.2, and 29.0±0.2 degrees 2θ. In other such embodiments, the pattern B hydrate has an X-ray powder diffraction pattern comprising five or more peaks selected from the group consisting of 6.3±0.2, 7.7±0.2, 10.4±0.2, 12.7±0.2, 13.3±0.2, 13.5±0.2, 14.9±0.2, 15.4±0.2, 16.4±0.2, 18.5±0.2, 18.6±0.2, 18.9±0.2, 19.4±0.2, 22.5±0.2, 23.5±0.2, 24.0±0.2, 26.8±0.2, and 29.0±0.2 degrees 2θ.

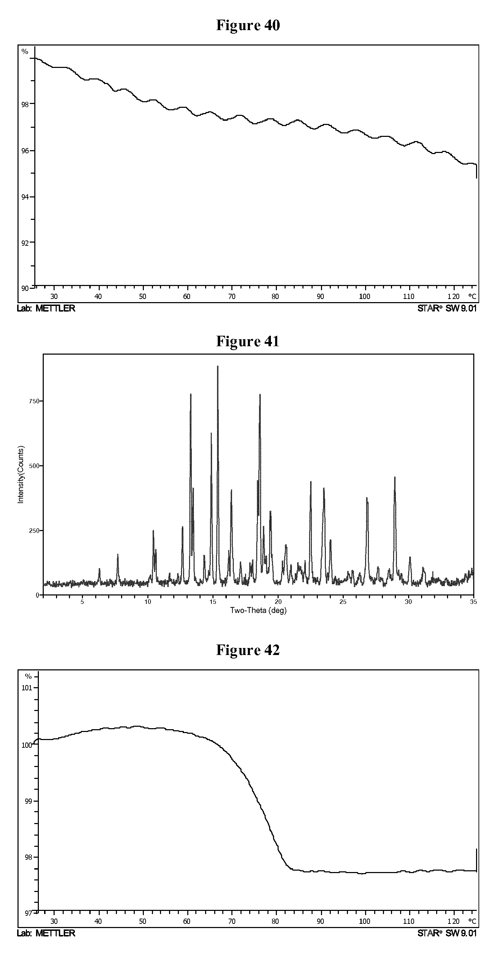

In some embodiments, the pattern B hydrate has an X-ray powder diffraction pattern substantially as shown in Figure **41**. The 2θ values for the peaks in Figure **41**(and their intensities) are as follows: 6.31 (7), 7.72 (14), 10.45 (24), 12.67 (26), 13.30 (88), 13.50 (44), 14.89 (70), 15.40 (100), 16.43 (43), 18.46 (47), 18.63 (86), 18.91 (26), 19.42 (33), 22.52 (47), 23.52 (44), 24.02 (20), 26.82 (40), and 28.97 (49).

This invention also relates, in part, to a process for preparing the pattern B hydrate by suspending pattern A polymorph (discussed above) in acetonitrile/water (9/1 v/v). The recovered pattern B hydrate contains ∼0.7 water molecules per molecule of compound **IB-L1-1.1**.

This invention also relates, in part, to a pattern C (E)-N-(4-(3-tert-butyl-5-(2,4-dioxo-3,4-dihydropyrimidin-1(2H)-yl)-2-methoxystyryl)phenyl)methanesulfonamide hydrate.

In some embodiments, the pattern C hydrate has an X-ray powder diffraction pattern comprising one or more peaks selected from the group consisting of 10.5±0.2, 13.3±0.2, 14.9±0.2, 15.4±0.2, 16.4±0.2, 18.6±0.2, 19.0±0.2, 19.4±0.2, 22.5±0.2, 23.5±0.2, 26.9±0.2, and 29.0±0.2 degrees 2θ. In some such embodiments, the pattern C hydrate has an X-ray powder diffraction pattern comprising three or more peaks selected from the group consisting of 10.5±0.2, 13.3±0.2, 14.9±0.2, 15.4±0.2, 16.4±0.2, 18.6±0.2, 19.0±0.2, 19.4±0.2, 22.5±0.2, 23.5±0.2, 26.9±0.2, and 29.0±0.2 degrees 2θ. In other such embodiments, the pattern C hydrate has an X-ray powder diffraction pattern comprising five or more peaks selected from the group consisting of 10.5±0.2, 13.3±0.2, 14.9±0.2, 15.4±0.2, 16.4±0.2, 18.6±0.2, 19.0±0.2, 19.4±0.2, 22.5±0.2, 23.5±0.2, 26.9±0.2, and 29.0±0.2 degrees 2θ.

In some embodiments, the pattern C hydrate has an X-ray powder diffraction pattern comprising one or more peaks selected from the group consisting of 10.5±0.2, 13.3±0.2, 13.5±0.2, 14.9±0.2, 15.4±0.2, 16.4±0.2, 18.6±0.2, 19.0±0.2, 19.4±0.2, 22.5±0.2, 23.5±0.2, 26.9±0.2, and 29.0±0.2 degrees 2θ. In some such embodiments, the pattern C hydrate has an X-ray powder diffraction pattern comprising three or more peaks selected from the group consisting of 10.5±0.2, 13.3±0.2, 13.5±0.2, 14.9±0.2, 15.4±0.2, 16.4±0.2, 18.6±0.2, 19.0±0.2, 19.4±0.2, 22.5±0.2, 23.5±0.2, 26.9±0.2, and 29.0±0.2 degrees 2θ. In other such embodiments, the pattern C hydrate has an X-ray powder diffraction pattern comprising five or more peaks selected from the group consisting of 10.5±0.2, 13.3±0.2, 13.5±0.2, 14.9±0.2, 15.4±0.2, 16.4±0.2, 18.6±0.2, 19.0±0.2, 19.4±0.2, 22.5±0.2, 23.5±0.2, 26.9±0.2, and 29.0±0.2 degrees 2θ.

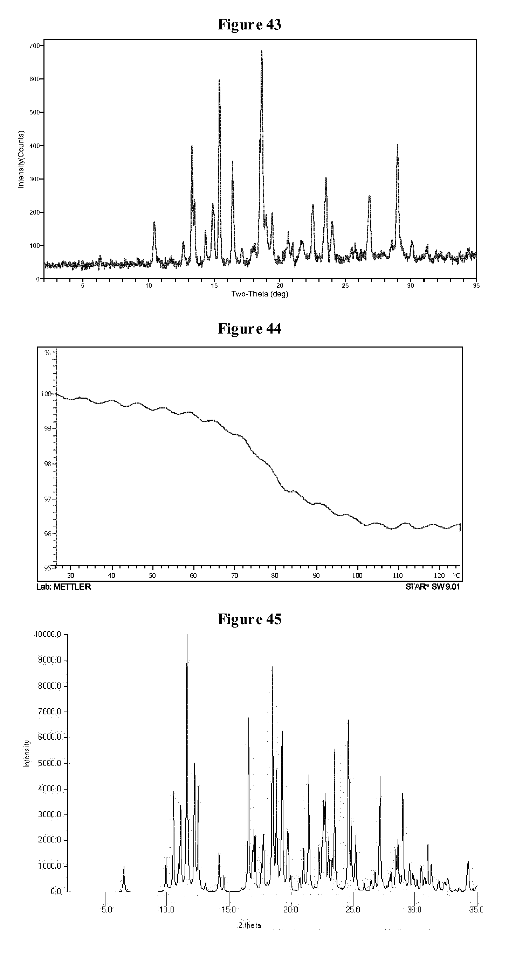

In some embodiments, the pattern C hydrate has an X-ray powder diffraction pattern substantially as shown in Figure **43**. The 2θ values for the peaks in Figure **43** (and their intensities) are as follows: 10.47 (21), 13.31 (56), 13.49 (31), 14.91 (28), 15.40 (86), 16.43 (48), 18.61 (100), 18.96 (20), 19.44 (19), 22.55 (26), 23.54 (39), 26.84 (29), and 28.99 (54).

This invention also relates, in part, to a process for preparing the pattern C hydrate by suspending pattern A polymorph (discussed above) in water. The recovered pattern C hydrate contains ∼1 water molecules per molecule of compound **IB-L1-1.1**.

This invention also relates, in part, to a pattern D (E)-N-(4-(3-tert-butyl-5-(2,4-dioxo-3,4-dihydropyrimidin-1(2H)-yl)-2-methoxystyryl)phenyl)methanesulfonamide hydrate.

The crystallographic unit cell parameters of the pattern D hydrate salt have been determined to be as follows: a is 17.8Å, b is 9.6Å, and c is 27.0Å (more precisely, a is 17.783(2)Å, b is 9.5651(12)Å, and c is 27.014(4)Å); the cell angle is: β - 93.3⁰ (more precisely, β is 93.256(2)⁰); and the cell volume is 4588Å³ (more precisely, 4587.5(10)Å³). The salt crystallizes in the C2/c space group.

In some embodiments, the pattern D hydrate has an X-ray powder diffraction pattern comprising one or more peaks selected from the group consisting of 6.6±0.2, 10.0±0.2, 10.5±0.2, 11.1±0.2, 11.6±0.2, 12.2±0.2, 14.2±0.2, 16.6±0.2, 17.1±0.2, 17.7±0.2, 18.5±0.2, 18.8±0.2, 19.3±0.2, 21.4±0.2, 22.7±0.2, 23.1±0.2, 23.6±0.2, 24.6±0.2, 25.2±0.2, 27.2±0.2, 29.1±0.2, and 31.0±0.2 degrees 2θ. In some such embodiments, the pattern D hydrate has an X-ray powder diffraction pattern comprising three or more peaks selected from the group consisting of 6.6±0.2, 10.0±0.2, 10.5±0.2, 11.1±0.2, 11.6±0.2, 12.2±0.2, 14.2±0.2, 16.6±0.2, 17.1±0.2, 17.7±0.2, 18.5±0.2, 18.8±0.2, 19.3±0.2, 21.4±0.2, 22.7±0.2, 23.1±0.2, 23.6±0.2, 24.6±0.2, 25.2±0.2, 27.2±0.2, 29.1±0.2, and 31.0±0.2 degrees 2θ. In other such embodiments, the pattern D hydrate has an X-ray powder diffraction pattern comprising five or more peaks selected from the group consisting of 6.6±0.2, 10.0±0.2, 10.5±0.2, 11.1±0.2, 11.6±0.2, 12.2±0.2, 14.2±0.2, 16.6±0.2, 17.1±0.2, 17.7±0.2, 18.5±0.2, 18.8±0.2, 19.3±0.2, 21.4±0.2, 22.7±0.2, 23.1±0.2, 23.6±0.2, 24.6±0.2, 25.2±0.2, 27.2±0.2, 29.1±0.2, and 31.0±0.2 degrees 2θ.

In some embodiments, the pattern D hydrate has an X-ray powder diffraction pattern comprising one or more peaks selected from the group consisting of 6.6±0.2, 10.0±0.2, 10.5±0.2, 11.1±0.2, 11.6±0.2, 12.2±0.2, 12.5±0.2, 14.2±0.2, 16.6±0.2, 17.1±0.2, 17.7±0.2, 18.5±0.2, 18.8±0.2, 19.3±0.2, 21.4±0.2, 22.7±0.2, 22.8±0.2, 23.1±0.2, 23.6±0.2, 24.6±0.2, 24.9±0.2, 25.2±0.2, 27.2±0.2, 29.1±0.2, and 31.0±0.2 degrees 2θ. In some such embodiments, the pattern D hydrate has an X-ray powder diffraction pattern comprising three or more peaks selected from the group consisting of 6.6±0.2, 10.0±0.2, 10.5±0.2, 11.1±0.2, 11.6±0.2, 12.2±0.2, 12.5±0.2, 14.2±0.2, 16.6±0.2, 17.1±0.2, 17.7±0.2, 18.5±0.2, 18.8±0.2, 19.3±0.2, 21.4±0.2, 22.7±0.2, 22.8±0.2, 23.1±0.2, 23.6±0.2, 24.6±0.2, 24.9±0.2, 25.2±0.2, 27.2±0.2, 29.1±0.2, and 31.0±0.2 degrees 2θ. In other such embodiments, the pattern D hydrate has an X-ray powder diffraction pattern comprising five or more peaks selected from the group consisting of 6.6±0.2, 10.0±0.2, 10.5±0.2, 11.1±0.2, 11.6±0.2, 12.2±0.2, 12.5±0.2, 14.2±0.2, 16.6±0.2, 17.1±0.2, 17.7±0.2, 18.5±0.2, 18.8±0.2, 19.3±0.2, 21.4±0.2, 22.7±0.2, 22.8±0.2, 23.1±0.2, 23.6±0.2, 24.6±0.2, 24.9±0.2, 25.2±0.2, 27.2±0.2, 29.1±0.2, and 31.0±0.2 degrees 2θ.

In some embodiments, the pattern D hydrate has an X-ray powder diffraction pattern substantially as shown in Figure **45**. The 2θ values for the peaks in Figure **45** (and their intensities) are as follows: 6.55 (10), 9.96 (12), 10.51 (37), 11.09 (31), 11.62 (100), 12.24 (44), 12.54 (40), 14.22 (15), 16.62 (68), 17.07 (22), 17.77 (21), 18.52 (82), 18.84 (47), 19.30 (63), 21.45 (34), 22.67 (30), 22.80 (34), 23.08 (20), 23.57 (58), 24.63 (73), 24.88 (26), 25.24 (21), 27.23 (36), 29.06 (41), and 31.04 (21).

This invention also relates, in part, to a process for preparing the pattern D hydrate. It was prepared by suspending pattern A polymorph (discussed above) in ethanol. Alternatively, it was prepared by suspending compound **IB-L1-1.1** (103.03mg) in 400ul THF while heated to about 55°C. Aqueous NaOH (1M, 264ul, 1.2 molar equivalent) was added. The solid dissolved completely to yield a clear solution. Ethanol (1.6ml) was added to the solution. The solution was allowed to cool naturally to ambient temperatures. Crystals were formed during the slow evaporation process. Although it appears that the lattice can accommodate as much as 0.5 water molecules per molecule of compound **IB-L1-1.1**, the recovered pattern D hydrate contained ∼0.2 water molecules per molecule of compound **IB-L1-1.1**.

This invention also relates, in part, to a pattern E (E)-N-(4-(3-tert-butyl-5-(2,4-dioxo-3,4-dihydropyrimidin-1(2H)-yl)-2-methoxystyryl)phenyl)methanesulfonamide hydrate.

The crystallographic unit cell parameters of the pattern E hydrate crystalline disodium salt have been determined to be as follows: a is 9.5Å, b is 14.5Å, and c is 17.3Å (more precisely, a is 9.462(2)Å, b is 14.462(3)Å, and c is 17.281(4)Å); the cell angles are: α - 84.9⁰, β - 80.8⁰, and γ - 81.8⁰ (more precisely, α is 84.863(4)⁰, β is 80.760(4)⁰, and γ is 81.751(4)⁰); and the cell volume is 2304Å³ (more precisely, 2304.4(9)Å³). The salt crystallizes in the P-1 space group.

In some embodiments, the pattern E hydrate has an X-ray powder diffraction pattern comprising one or more peaks selected from the group consisting of 6.2±0.2, 7.8±0.2, 10.2±0.2, 10.7±0.2, 12.1±0.2, 16.3±0.2, 19.7±0.2, 20.9±0.2, 21.8±0.2, 24.5±0.2, and 28.0±0.2 degrees 2θ. In some such embodiments, the pattern E hydrate has an X-ray powder diffraction pattern comprising three or more peaks selected from the group consisting of 6.2±0.2, 7.8±0.2, 10.2±0.2, 10.7±0.2, 12.1±0.2, 16.3±0.2, 19.7±0.2, 20.9±0.2, 21.8±0.2, 24.5±0.2, and 28.0±0.2 degrees 2θ. In other such embodiments, the pattern E hydrate has an X-ray powder diffraction pattern comprising five or more peaks selected from the group consisting of 6.2±0.2, 7.8±0.2, 10.2±0.2, 10.7±0.2, 12.1±0.2, 16.3±0.2, 19.7±0.2, 20.9±0.2, 21.8±0.2, 24.5±0.2, and 28.0±0.2 degrees 2θ.

In some embodiments, the pattern E hydrate has an X-ray powder diffraction pattern comprising one or more peaks selected from the group consisting of 6.2±0.2, 7.8±0.2, 10.2±0.2, 10.4±0.2, 10.7±0.2, 12.1±0.2, 16.3±0.2, 19.7±0.2, 20.9±0.2, 21.8±0.2, 24.5±0.2, and 28.0±0.2 degrees 2θ. In some such embodiments, the pattern E hydrate has an X-ray powder diffraction pattern comprising three or more peaks selected from the group consisting of 6.2±0.2, 7.8±0.2, 10.2±0.2, 10.4±0.2, 10.7±0.2, 12.1±0.2, 16.3±0.2, 19.7±0.2, 20.9±0.2, 21.8±0.2, 24.5±0.2, and 28.0±0.2 degrees 2θ. In other such embodiments, the pattern E hydrate has an X-ray powder diffraction pattern comprising five or more peaks selected from the group consisting of 6.2±0.2, 7.8±0.2, 10.2±0.2, 10.4±0.2, 10.7±0.2, 12.1±0.2, 16.3±0.2, 19.7±0.2, 20.9±0.2, 21.8±0.2, 24.5±0.2, and 28.0±0.2 degrees 2θ.

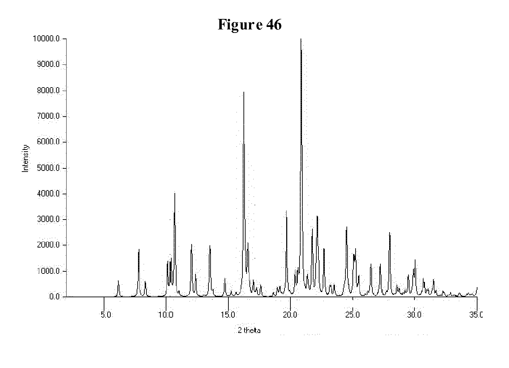

In some embodiments, the pattern E hydrate has an X-ray powder diffraction pattern substantially as shown in Figure **46**. The 2θ values for the peaks in Figure **46** (and their intensities) are as follows: 6.19 (6), 7.81 (18), 10.17 (13), 10.40 (14), 10.68 (39), 12.06 (20), 16.29 (78), 19.72 (32), 20.88 (100), 21.77 (27), 24.52 (25), and 28.01 (27).

This invention also relates, in part, to a process for preparing the pattern E hydrate. It was prepared by suspending compound **IB-L1-1.1** (56.76mg) in 200ul THF while heated. Aqueous NaOH (1M, 146uL, 1.2 molar equivalent) was added, which yielded a clear solution. Ethanol (800ul) was added to the solution. The solution was allowed to cool naturally to ambient temperatures. Crystals were formed during the slow evaporation process. Although it appears that the lattice can accommodate as much as one water molecule per molecule of compound **IB-L1-1.1**, the recovered pattern D hydrate contained ∼0.25 water molecules per molecule of compound **IB-L1-1.1**.

### H. Compositions.

This invention also is directed, in part, to compositions comprising one or more compounds and/or salts of the invention (including the crystalline compounds and salts discussed in section *G* above). In some embodiments, the compositions comprise one or more substantially phase pure crystalline forms (compounds/salts/solvates/hydrates) discussed in section *G* above. The compositions can be pharmaceutical compositions.

In some embodiments, the compositions further comprise one or more additional therapeutic agents. Such therapeutic agents can, but need not be, additional HCV inhibitors.

The preferred composition depends on the method of administration, and typically comprises one or more conventional pharmaceutically acceptable carriers, adjuvants, and/or vehicles (together referred to as "excipients"). Formulation of drugs is generally discussed in, for example, Hoover, J., Remington's Pharmaceutical Sciences (Mack Publishing Co., 1975) and Ansel's Pharmaceutical Dosage Forms and Drug Delivery Systems (Lippincott Williams & Wilkins, 2005).

Solid dosage forms for oral administration include, for example, capsules, tablets, pills, powders, and granules. In such solid dosage forms, the compounds or salts are ordinarily combined with one or more excipients. If administered per os, the compounds or salts can be mixed with, for example, lactose, sucrose, starch powder, cellulose esters of alkanoic acids, cellulose alkyl esters, talc, stearic acid, magnesium stearate, magnesium oxide, sodium and calcium salts of phosphoric and sulfuric acids, gelatin, acacia gum, sodium alginate, polyvinylpyrrolidone, and/or polyvinyl alcohol, and then tableted or encapsulated for convenient administration. Such capsules or tablets can contain a controlled-release formulation, as can be provided in, for example, a dispersion of the compound or salt in hydroxypropylmethyl cellulose. In the case of capsules, tablets, and pills, the dosage forms also can comprise buffering agents, such as sodium citrate, or magnesium or calcium carbonate or bicarbonate. Tablets and pills additionally can be prepared with enteric coatings.

Liquid dosage forms for oral administration include, for example, pharmaceutically acceptable emulsions (including both oil-in-water and water-in-oil emulsions), solutions (including both aqueous and non-aqueous solutions), suspensions (including both aqueous and non-aqueous suspensions), syrups, and elixirs containing inert diluents commonly used in the art (*e.g*., water). Such compositions also can comprise, for example, wetting, emulsifying, suspending, flavoring (*e.g*., sweetening), and/or perfuming agents.

Parenteral administration includes subcutaneous injections, intravenous injections, intramuscular injections, intrasternal injections, and infusion. Injectable preparations (*e.g*., sterile injectable aqueous or oleaginous suspensions) can be formulated according to the known art using suitable dispersing, wetting agents, and/or suspending agents. Acceptable vehicles and solvents include, for example, water, 1,3-butanediol, Ringer's solution, isotonic sodium chloride solution, bland fixed oils (*e.g*., synthetic mono- or diglycerides), fatty acids (*e.g.,* oleic acid), dimethyl acetamide, surfactants (*e.g*., ionic and non-ionic detergents), and/or polyethylene glycols.

Formulations for parenteral administration may, for example, be prepared from sterile powders or granules having one or more of the excipients mentioned for use in the formulations for oral administration. A compound or salt of the invention can be dissolved in water, polyethylene glycol, propylene glycol, ethanol, corn oil, cottonseed oil, peanut oil, sesame oil, benzyl alcohol, sodium chloride, and/or various buffers. The pH may be adjusted, if necessary, with a suitable acid, base, or buffer.

Suppositories for rectal administration can be prepared by, for example, mixing a compound or salt of the invention with a suitable nonirritating excipient that is solid at ordinary temperatures, but liquid at the rectal temperature, and will therefore melt in the rectum to release the drug. Suitable excipients include, for example, cocoa butter; synthetic mono-, di-, or triglycerides, fatty acids, and/or polyethylene glycols.

Topical administration includes the use of transdermal administration, such as transdermal patches or iontophoresis devices.

Other excipients and modes of administration known in the pharmaceutical art also may be used.

Applicants have discovered that some **I-L1** compounds in which **R⁶** and the phenyluracil are in trans-position relative to the double bond, when in solution, tend to convert into the corresponding cis-isomer upon exposure to light; thus, it may be desirable to store such solutions under conditions that reduce exposure to light (*e.g*., in an amber bottle or in a dark place).

The preferred total daily dose of the compound or salt (administered in single or divided doses) is typically from about 0.001 to about 100mg/kg, more preferably from about 0.001 to about 30mg/kg, and even more preferably from about 0.01 to about 10mg/kg (*i.e*.,mg of the compound or salt per kg body weight). Dosage unit compositions can contain such amounts or submultiples thereof to make up the daily dose. In many instances, the administration of the compound or salt will be repeated a plurality of times. Multiple doses per day typically may be used to increase the total daily dose, if desired.

Factors affecting the preferred dosage regimen include the type, age, weight, sex, diet, and condition of the patient; the severity of the pathological condition; the severity of the pathological condition; the route of administration; pharmacological considerations, such as the activity, efficacy, pharmacokinetic, and toxicology profiles of the particular compound or salt used; whether a drug delivery system is utilized; and whether the compound or salt is administered as part of a drug combination. Thus, the dosage regimen actually employed can vary widely, and therefore, can derive from the preferred dosage regimen set forth above.

### I. Kits.

This invention also is directed, in part, to a kit comprising one or more compounds and/or salts of the in invention. The kit can optionally contain one or more additional therapeutic agents and/or instructions for, for example, using the kit.

### J. Methods of Use.

This invention also is directed, in part, to a method for inhibiting replication of an RNA virus. The method comprises exposing the virus to one or more compounds and/or salts of this invention. In some embodiments, replication of the RNA virus is inhibited *in vitro.* In other embodiments, replication of the RNA virus is inhibited *in vivo.* In some embodiments, the RNA virus whose replication is being inhibited is a single-stranded, positive sense RNA virus. In some such embodiments, the RNA virus whose replication is being inhibited is a virus from the *Flaviviridae* family. In some such embodiments, the RNA virus whose replication is being inhibited is HCV.

This invention also is directed, in part, to a method for inhibiting HCV RNA polymerase. The method comprises exposing the polymerase with one or more compounds and/or salts of this invention. In some embodiments, HCV RNA polymerase activity is inhibited *in vitro.* In other embodiments, HCV RNA polymerase activity is inhibited *in vivo.*

The term "inhibiting" means reducing the level of RNA virus replication/HCV polymerase activity either *in vitro* or *in vivo.* For example, if a compound/salt of the invention reduces the level of RNA virus replication by at least about 10% compared to the level of RNA virus replication before the virus was exposed to the compound/salt, then the compound/salt inhibits RNA virus replication. In some embodiments, the compound/salt can inhibit RNA virus replication by at least about 20%, at least about 30%, at least about 40%, at least about 50%, at least about 60%, at least about 70%, at least about 80%, at least about 90%, or at least about 95%.

This invention also is directed, in part, to a method for treating a disease that can be treated by inhibiting HCV RNA polymerase. Thus, this invention also is directed, in part, to a method for treating hepatitis C in an animal in need of such treatment. These methods comprise administering to the animal one or more compounds and/or salts of the invention, and, optionally, one or more additional therapeutic agents. In some embodiments, a therapeutically effective amount of the compound(s) and/or salt(s) is administered to the animal. "Treating" means ameliorating, suppressing, eradicating, preventing, reducing the risk of, and/or delaying the onset of the disease being treated. Applicants specifically intend that the term "treating" encompass administration of the compounds and/or salts of the invention to an HCV-negative patient that is a candidate for an organ transplant. The methods of treatment are particularly suitable for use with humans, but may be used with other animals, particularly mammals. A "therapeutically-effective amount" or "effective amount" is an amount that will achieve the goal of treating the targeted condition.

In some embodiments, the methods comprise combination therapy, wherein the compound(s) and/or salt(s) of the invention is/are co-administered with a second (or even a third, fourth, etc.) compound, such as, for example, another therapeutic agent used to treat hepatitis C (*e.g*., interferon or interferon/ribavirin combination, or an HCV inhibitor such as, for example, an HCV polymerase inhibitr or an HCV protease inhibitor). The compound(s) and/or salt(s) of this invention can also be co-administered with therapeutic agents other than therapeutic agents used to treat hepatitis C (*e.g*., anti-HIV agents). In these co-administration embodiments, the compound(s) and/or salt(s) of the invention and the second, etc. therapeutic agent(s) may be administered in a substantially simultaneous manner (e.g., or within about 5 minutes of each other), in a sequential manner, or both. It is contemplated that such combination therapies may include administering one therapeutic agent multiple times between the administrations of the other. The time period between the administration of each agent may range from a few seconds (or less) to several hours or days, and will depend on, for example, the properties of each composition and active ingredient (*e.g*., potency, solubility, bioavailability, half-life, and kinetic profile), as well as the condition of the patient. The compound(s) and/or salt(s) of this invention and the second, etc. therapeutic agent may also be administered in a single formulation.

This invention also is directed, in part, to a use of one or more compounds and/or salts of the invention, and, optionally one or more additional therapeutic agents to prepare a medicament. In some embodiments, the medicament is for co-administration with one or more additional therapeutic agents.

In some embodiments, the medicament is for inhibiting replication of an RNA virus.

In some embodiments, the medicament is for treating hepatitis C.

This invention also is directed, in part, to one or more compounds and/or salts of the invention, and, optionally one or more additional therapeutic agents, for use as a medicament. In some embodiments, the medicament is for inhibiting replication of an RNA virus. In other embodiments, the medicament is for treating hepatitis C.

### K. Intermediate Compounds.

This invention also is directed, in part, to intermediates that correspond in structure to formula **II** that can be used to prepare the compounds of formula **I** (and their salts)(although some intermediates can also be used, just like the compounds of formula **I**, as HCV inhibitors, and one skilled in the art can determine such ability of the compounds of formula **II** by utilizing, for example, the methods discussed below):

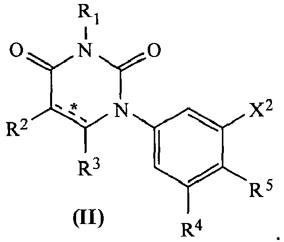

In formula **II:**
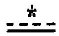 , **R¹**, **R²**, **R³**, **R⁴**, and **R⁵** are as discussed above for the compounds of formula **I**; and
**X²** is halo.

The various embodiments for 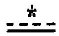 , **R¹**, **R²**, **R³**, **R⁴**, and **R⁵** (as well as their combinations) discussed above apply to the compounds of formula **II**. As to **X²**, in some embodiments, **X²** is selected from the group consisting of chloro, bromo, and iodo. In other embodiments, **X²** is selected from the group consisting of chloro and bromo. In yet other embodiments, **X²** is selected from the group consisting of chloro and iodo. In yet other embodiments, **X²** is selected from the group consisting of iodo and bromo. In further embodiments, **X²** is fluoro. In yet further embodiments, **X²** is chloro. In yet further embodiments, **X²** is bromo. And in yet further embodiments, **X²** is iodo.

The various embodiments for 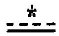 , **R²**, **R³**, **R⁴**, **R⁵**, and **X²** discussed above can be combined to form various embodiments of compounds of formula **II**, and all embodiments of compounds of formula **II** so formed are within the scope of Applicants' invention. Some exemplary embodiments of the compounds (and salts thereof) of formula **II** are discussed below.

In some embodiments, the compounds of formula **II** correspond in structure to formula **IIA**:

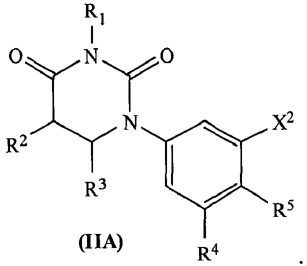

In other embodiments, the compounds of formula **II** correspond in structure to formula **IIB**:

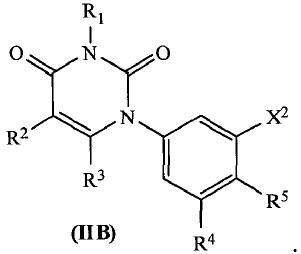

In some embodiments of the compounds of formula **II**:
**R¹** is selected from the group consisting of hydrogen, methyl, and nitrogen-protecting group;
**R²** is selected from the group consisting of hydrogen and halo;
**R³** is selected from the group consisting of hydrogen and halo;
**R⁴** is selected from the group consisting of C₁-C₄-alkyl, C₃-C₆-carbocyclyl, and 5-6-membered heterocyclyl, wherein:
   (**a**) the C₁-C₄-alkyl optionally is substituted with up to three substituents independently selected from the group consisting of halo, oxo, hydroxy, alkyloxy, and trimethylsilyl, and
   (**b**) the C₃-C₆-carbocyclyl and 5-6-membered heterocyclyl optionally are substituted with one or two substituents independently selected from the group consisting of alkyl, halo, and alkylsulfonylamino;
**R⁵** is selected from the group consisting of hydrogen, hydroxy, alkyloxy, and halo; and
**X²** is selected from the group consisting of chloro, bromo, and iodo.

In some embodiments of the compounds of formula **II**:
is a double carbon-carbon bond;
**R¹** is hydrogen;
**R²** is selected from the group consisting of hydrogen and halo;
**R³** is hydrogen;
**R⁴** is tert-butyl;
**R⁵** is selected from the group consisting of hydrogen, hydroxy, and methoxy; and
**X²** is selected from the group consisting of bromo and iodo.

In some embodiments of the compounds of formula **II**:
**R¹** is selected from the group consisting of hydrogen and methyl;
**R²** is selected from the group consisting of hydrogen and methyl;
**R³** is selected from the group consisting of hydrogen and methyl;
**R⁴** is tert-butyl;
**R⁵** is selected from the group consisting of hydroxy and methoxy; and
**X²** is selected from the group consisting of chloro, bromo, and iodo.

In some embodiments of the compounds of formula **II**:
is a double carbon-carbon bond;
**R¹** is hydrogen;
**R²** is hydrogen;
**R³** is hydrogen;
**R⁴** is tert-butyl;
**R⁵** is selected from the group consisting of hydroxy and methoxy; and
**X²** is selected from the group consisting of chloro, bromo, and iodo.

In some embodiments, the compound of formula **II** is selected from the group consisting of

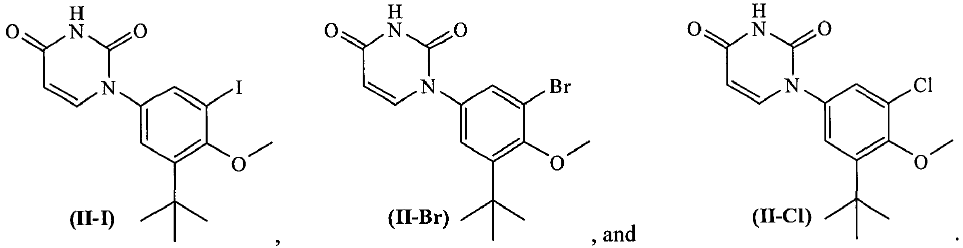

The discussion below provides instructions for the preparation of intermediate compounds of formula **II** (and salts thereof).

### L. Starting Compounds.

This invention also is directed, in part, to starting compounds that correspond in structure to formula **III** that can be used to prepare the compounds of formulas **II** and **I** (and their salts):

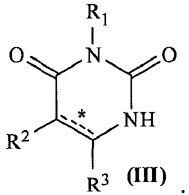

In formula **III**, 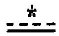 , **R¹**, **R²**, and **R³** are as discussed above for the compounds of formula **I** and **II**. The various embodiments for 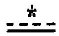 , **R¹**, **R²**, and **R³** (as well as their combinations) discussed above apply to the compounds of formula **III.** The various embodiments for 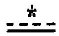 , **R¹**, **R²**, and **R³** discussed above can be combined to form various embodiments of compounds of formula **III**, and all embodiments of compounds of formula **III** so formed are within the scope of Applicants' invention. Some exemplary embodiments of the compounds (and salts thereof) of formula **III** are discussed below.

In some embodiments of the compounds of formula **III**:
**R¹** is selected from the group consisting of hydrogen, methyl, and nitrogen-protecting group;
**R²** is selected from the group consisting of hydrogen and halo; and
**R³** is selected from the group consisting of hydrogen and halo.

In some embodiments of the compounds of formula **III**:
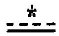 is a double carbon-carbon bond;
**R¹** is selected from the group consisting of hydrogen;
**R²** is selected from the group consisting of hydrogen and halo; and
**R³** is selected from the group consisting of hydrogen.

In some embodiments of the compounds of formula **III**:
**R¹** is selected from the group consisting of hydrogen and methyl;
**R²** is selected from the group consisting of hydrogen and methyl; and
**R³** is selected from the group consisting of hydrogen and methyl.

In some embodiments, the compound of formula **III** is uracil.

This invention also is directed, in part, to starting compounds that correspond in structure to formula **IV** that can be used to prepare the compounds of formulas **II** and **I** (and their salts):

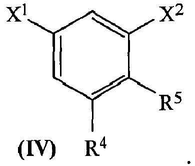

In formula **IV**:
**R⁴**, **R⁵**, and **X²** are as discussed above for the compounds of formula **I** and **II**; and
**X¹ is** halo.

The various embodiments for **R⁴**, **R⁵**, and **X²** (as well as their combinations) discussed above apply to the compounds of formula **IV**. As to **X¹**, in some embodiments, **X¹** is selected from the group consisting of chloro, bromo, and iodo. In other embodiments, **X¹** is selected from the group consisting of chloro and bromo. In yet other embodiments, **X¹** is selected from the group consisting of chloro and iodo. In yet other embodiments, **X¹** is selected from the group consisting of iodo and bromo. In further embodiments, **X¹** is fluoro. In yet further embodiments, **X¹** is chloro. In yet further embodiments, **X¹** is bromo. And in yet further embodiments, **X¹** is iodo. As to **X¹** and **X²**, in some embodiments, **X¹** and **X²** are identical.

The various embodiments for **R⁴**, **R⁵**, **X¹**, and **X²** discussed above can be combined to form various embodiments of compounds of formula **IV**, and all embodiments of compounds of formula **III** so formed are within the scope of Applicants' invention. Some exemplary embodiments of the compounds (and salts thereof) of formula **IV** are discussed below.

In some embodiments of the compounds of formula **IV**:
**R⁴** is selected from the group consisting of C₁-C₄-alkyl, C₃-C₆-carbocyclyl, and 5-6-membered heterocyclyl, wherein:
   (**a**) the C₁-C₄-alkyl optionally is substituted with up to three substituents independently selected from the group consisting of halo, oxo, hydroxy, alkyloxy, and trimethylsilyl, and
   (**b**) the C₃-C₆-carbocyclyl and 5-6-membered heterocyclyl optionally are substituted with one or two substituents independently selected from the group consisting of alkyl, halo, and alkylsulfonylamino;
**R⁵** is selected from the group consisting of hydrogen, hydroxy, and alkyloxy;
**X¹** is selected from the group consisting of chloro, bromo, and iodo; and
**X²** is selected from the group consisting of chloro, bromo, and iodo.

In some embodiments of the compounds of formula **IV**:
**R⁴** is selected from the group consisting of tert-butyl;
**R⁵** is selected from the group consisting of hydrogen, hydroxy, and methoxy;
**X¹** is selected from the group consisting of bromo and iodo; and
**X²** is selected from the group consisting of bromo and iodo.

In some embodiments of the compounds of formula **IV**:
**R⁴** is selected from the group consisting oftert-butyl;
**R⁵** is selected from the group consisting of hydroxy and methoxy;
**X¹** is selected from the group consisting of chloro, bromo, and iodo; and
**X²** is selected from the group consisting of chloro, bromo, and iodo.

In some embodiments of the compounds of formula **IV**:
**R⁴** is tert-butyl;
**R⁵** is selected from the group consisting of hydroxy and methoxy;
**X¹** is selected from the group consisting of chloro, bromo, and iodo; and
**X²** is selected from the group consisting of chloro, bromo, and iodo.

In some embodiments, the compound of formula **IV** is selected from the group consisting of

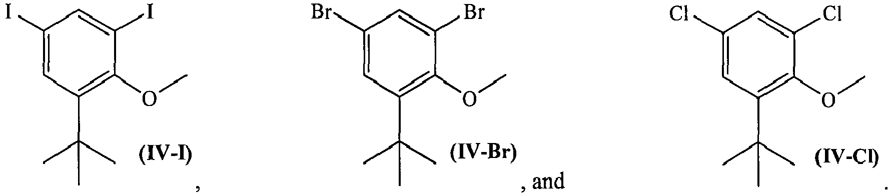

The discussion below provides instructions for the preparation of starting compounds of formula **IV** (and salts thereof).

### L. Methods for Preparation.

This invention also is directed, in part, to a process for preparing compounds of formula **II.** The process comprises reacting a compound of formula **III** with a compound of formula **IV** in the presence of (**i**) copper (I) salt catalyst and (**ii**) nitrogenous heteroaryl ligand:

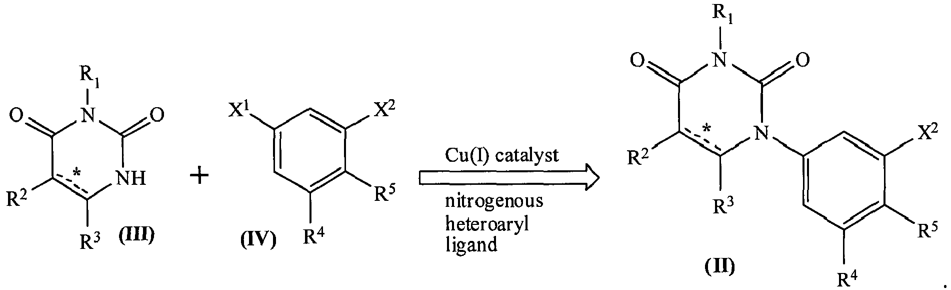

In the above process, **R¹**, **R²**, **R³**, **R⁴**, **R⁵**, **X¹**, and **X²** are as discussed above.

Applicants have discovered that the process generally results in the substitution of the N1 hydrogen of uracil derivative compound **III** thus resulting in intermediate compound **II**. When **X²** in intermediate compound **II** is chloro, bromo, or iodo, then compound **II** is is suitable for subsequent reaction (*e.g*., Suzuki coupling with an appropriate boronic acid or boronate ester) to provide compound of formula **I.** In other words, when **X²** in intermediate compound **II** is chloro, bromo, or iodo, the above process is suitable for preparing compounds of formula **I** as well.

In some embodiments, compound **III** is uracil, and compound **IV** corresponds in structure to a compound selected from the group consisting of compound **IV-I, IV-Br**, and **IV-CI**, with compounds **IV-I** and **IV-Br** typically resulting in better yield than compound **IV-CI**.

Suitable Cu(I) catalysts include, for example, CuI, CuBr, CuCl, Cu₂O, and CH₃C(O)OCu. In some embodiments, the catalyst is selected from the group consisting of CuI and CuBr. In some such embodiments, the catalyst is CuI. In other such embodiments, the catalyst is CuBr.

In some embodiments, the process is conducted in the presence of a base. In some such embodiments, the base is an inorganic base. Suitable inorganic bases include, for example, potassium, sodium, and cesium salts (*e.g.*, K₂CO₃, K₃PO₄, Cs₂CO₃, Na₂CO₃). In some embodiments, the base is selected from the group consisting of potassium salt and cesium salt. In some such embodiments, the salt is selected from the group consisting of K₃PO₄ and Cs₂CO₃. In some embodiments, the base comprises a potassium salt. In some such embodiments, the potassium salt is K₂CO₃. In other such embodiments, the potassium salt is K₃PO₄. In some embodiments, the base comprises a cesium salt. In some such embodiments, the potassium salt is Cs₂CO₃.

Typically, the process is conducted in the presence of a solvent. Suitable solvents include, for example, dimethylsulfoxide (DMSO), dimethylformamide (DMF), and acetonitrile (MeCN). In some embodiments, the solvent is DMSO.

Typically, the process is conducted at a temperature of from about 40 to about 130⁰C.

In some embodiments, the nitrogenous heteroaryl ligand comprises 8-hydroxyquinoline. In other embodiments, the ligand comprises 2-(2-pyridyl)-benzimidazole. In yet other embodiments, the ligand comprises a picolinamide compound corresponding in structure to formula **V**:

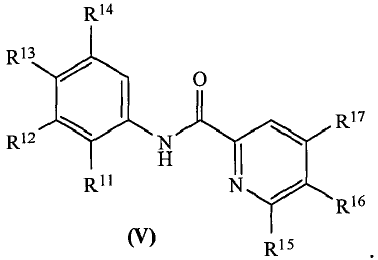

In formula **V**, **R¹¹**, **R¹²**, **R¹³**, **R¹⁴**, **R¹⁵**, **R¹⁶**, and **R¹⁷** are independently selected from the group consisting of hydrogen, C₁₋₄-perfluoroalkyl, C₁₋₄-alkyloxy, C₁₋₄-haloalkyl, chloro, or cyano. In some embodiments, **R¹¹**, **R¹²**, **R¹³**, **R¹⁴**, **R¹⁵**, **R¹⁶**, and **R¹⁷** are independently selected from the group consisting of hydrogen, methyl, methoxy, trifluoromethyl, chloro, and cyano. In some embodiments, the ligand of formula **V** comprises N-(4-cyanophenyl)picolinamide. In other embodiments, the ligand of formula **V** comprises N-(2-cyanophenyl)picolinamide.

In some embodiments, the process comprises (**a**) preparing a compound of formula **IV**; and (**b**) reacting a compound of formula **III** with a compound of formula **IV** in the presense of (**i**) copper (I) salt catalyst and (**ii**) nitrogenous heteroaryl ligand, optionally in the presence of inorganic base.

Compound of formula **IV-I** can be prepared by, for example, converting 2-tert-butylphenol into 2-tert-butyl-4,6-diiodophenol (by, for example, reacting it with NaI and NaOCl), and then converting the 2-tert-butyl-4,6-diiodophenol into 1-tert-butyl-3,5-diiodo-2-methoxybenzene (by, for example, treating it with CH₃I in the presence of a base, such as, for example, NaOH).

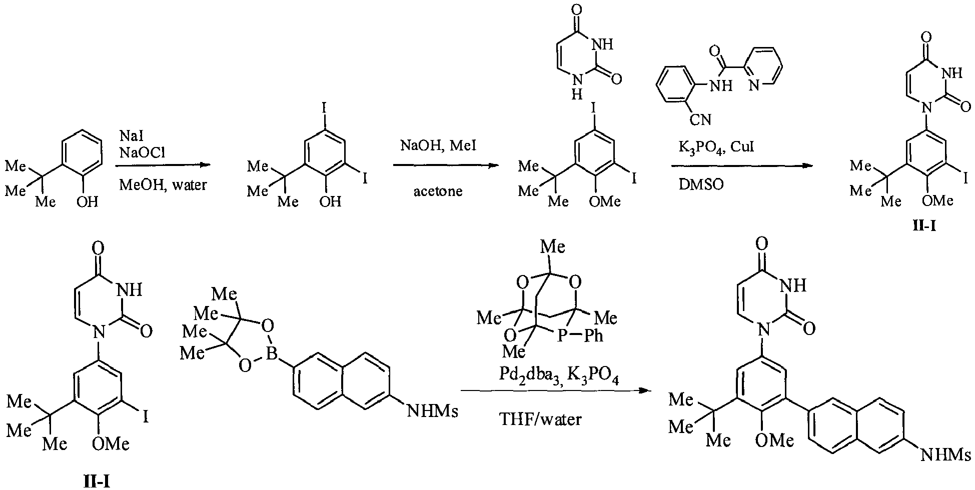

Compound of formula **IV-Br** can be prepared by, for example, converting 2-tert-butylphenol into 2,4-dibromo-6-tert-butylphenol (by, for example, reacting it with 1,3-dibromo-5,5-dimethylimidazo-lidine-2,4-dione), and then converting the 2,4-dibromo-6-tert-butylphenol into 1,5-dibromo-3-tert-butyl-2-methoxybenzene (by, for example, treating it with CH₃I in the presence of KOtBu).

Additional information about the preparation of compounds of formulas **I** and **II** (and their salts) is provided in the general discussion and/or specific synthesis examples below. In the discussion below, **R¹**, **R²**, **R³**, **R⁴**, **R⁵**, **L**, **R^{A}**, **R^{B}**, **R^{C}**, **R^{D}**, **R⁶**, **R^{E}**, **R^{F}**, **R^{G}**, **R^{H}**, **R^{I}**, **R^{J}**, **R^{K}**, **X¹**, and **X²** have the meaning discussed above unless otherwise stated.

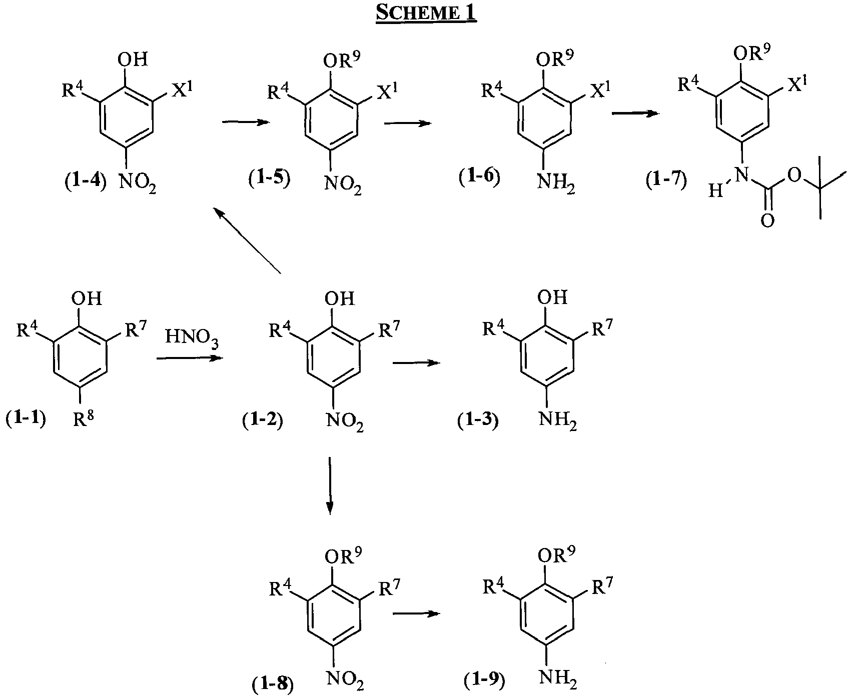

Compound (**1-1**), wherein R⁷ is, for example, hydrogen or -CO₂Me, and R⁸ is, for example, hydrogen or t-butyl, may be treated with nitric acid in solvents such as, for example, acetic acid or water in a temperature range of about 0 to about 35°C over about 1 to about 5h to provide compound (**1-2**). Compound (**1-2**) may then be reduced using conditions known to those skilled in the art to furnish the corresponding aniline (**1-3**). Typical conditions for this reduction include using hydrogen at a pressure of about 1 to about 5 atmospheres in the presence of a catalyst such as, for example, palladium or platinum on charcoal in a solvent such as, for example, tetrahydrofuran, ethyl acetate, ethanol, or hexane at or near ambient temperature over a period of about 1 to about 12h. Dependent on the functional groups present, an alternative reduction procedure may be more appropriate such as, for example, using iron powder in the presence of a mild acid such as, for example, ammonium chloride or dilute hydrochloric acid at reflux temperatures in a mixture of solvents containing, for example, methanol, water, and/or tetrahydrofuran over about 1 to about 12h. Another set of reduction conditions includes the use of sodium borohydride in a solvent mixture such as, for example, water and tetrahydrofuran. Yet another set of reduction conditions includes the use of tin(II) chloride in the presence of hydrochloric acid in such solvents as, for example, water and methanol or mixtures thereof.

Compound (**1-2**) may be modified prior to reduction. For example, treatment of compound (**1-2**), wherein R⁷ is hydrogen, with iodine monochloride in a mixture of methanol and water at or near ambient temperature over a period of about 8 to about 24h supplies compound (**1-4**), wherein X¹ is iodine. Alternatively, compound (**1-2**) can be treated with pyridinium hydrobromide perbromide in a solvent such as, for example, acetic acid at or near ambient temperature over a period of about 2 to about 16h to provide compound (**1-4**), wherein X¹ is bromine. Modifications may be introduced at the phenol moiety in compound (**1-4**). For example, the phenol may be alkylated with alkyl halides (e.g., methyl iodide), alkyl sulfates (e.g., methyl sulfate), alkenyl halides (e.g., allyl bromide), alkynyl halides (e.g., propargyl bromide) in the presence of a base such as, for example, potassium carbonate in acetone, sodium hydride in dimethylformamide, or potassium t-butoxide in tetrahydrofuran, at temperatures from about 0 to about 35°C over a period of about 1 to about 24h to provide compound (**1-5**), wherein R⁹ is, for example, alkyl, alkenyl, or alkynyl. Alternatively, alkylation may be achieved by using a reagent such as (trimethylsilyl) diazomethane in solvents such as, for example, methanol or t-butyl methyl ether, or mixtures thereof in a sealed tube at or near room temperature over about 8 to about 24h. Compound (**1-5**) may subsequently be reduced to compound (**1-6**) using the iron powder or tin(II) chloride conditions described above. An alternative reduction procedure employs hydrogenation at approximately 1 atmosphere pressure with a catalyst such as 5% platinum on sulfided carbon in a solvent such as methanol. Protection of the resultant aniline of compound (**1-6**) with, for example, a t-butyl carbamate can be achieved by treatment with di-tert-butyl dicarbonate in a solvent such as, for example, tetrahydrofuran or dioxane at a temperature of about 50 to about 65°C for about 1 to about 8h provides compound (**1-7**).

Modifications may also occur at the phenol moiety in compound (**1-2**). One skilled in the art may alkylate the phenol of compound (**1-2**) using, for example, the conditions described above to obtain compound (**1-8**). Compound (**1-8**) is transformed into compound (**1-9**) using, for example, one or more of the appropriate reduction conditions described above.

Another modification of the phenol group in compound (**1-2**) is sulfonylation to furnish compound (**1-8**), wherein R⁹ is alkylsulfonyl, carbocyclylsulfonyl, or haloalkylsulfonyl. Such a compound may be prepared by exposing compound (**1-2**) to sulfonyl chlorides such as, for example, methanesulfonyl chloride, cyclohexanesulfonyl chloride, benzenesulfonyl chloride, or 3-chloropropane sulfonyl chloride in the presence of a base such as, for example, triethylamine, diisopropylethylamine, or pyridine in a solvent such as, for example, dichloromethane at or near ambient temperature for a period of about 1 to about 24h. One skilled in the art can then transform compound (**1-8**) into compound (**1-9**) with an appropriate set of reduction conditions.

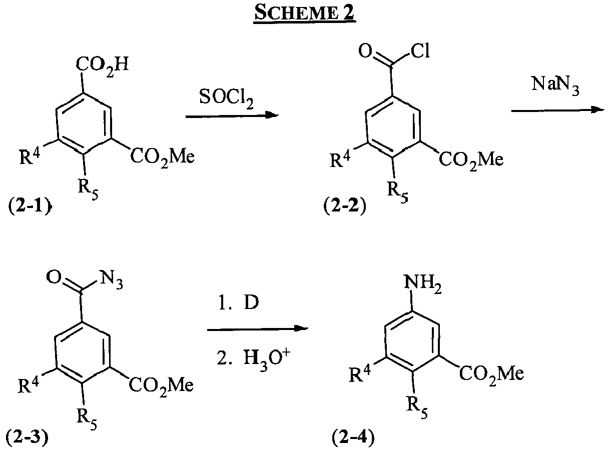

Aniline (**2-4**) can be prepared through use of the Curtius rearrangement. To this end, compound (**2-1**), wherein R⁴ is not amino, can be treated in refluxing thionyl chloride with a catalytic amount of dimethylformamide for about 1 to about 4h to obtain acid chloride (**2-2**). Treatment with thionyl chloride at the reflux temperature in solvents such as, for example, chloroform or toluene also furnishes compound (**2-2**). Compound (**2-2**) can be reacted with an aqueous solution of sodium azide in a solvent such as, for example, acetone over about 1 to about 8h to provide acyl azide (**2-3**). Compound (**2-3**) can then undergo a Curtius rearrangement in refluxing solvents such as dioxane or toluene. The intermediate isocyanate is hydrolyzed with an aqueous acid such as dilute hydrochloric acid in a solvent such as dimethoxyethane to provide compound (**2-4**).

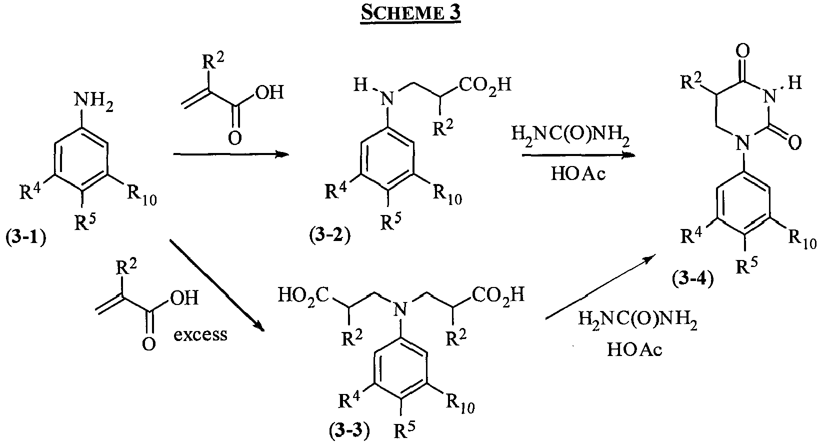

Compound (**3-1**), wherein R¹⁰ is, for example, hydrogen, bromine, iodine, or -CO₂Me, can be treated with an acrylic acid either neat at or near ambient temperature in a solvent such as, for example, toluene and heated to reflux over a period of about 15 to about 48h to supply compound (**3-2**). When excess of an acrylic acid is used, compound (**3-3**) is produced. Compound (**3-2**) or (**3-3**) can be treated with urea in a solvent such as, for example, acetic acid at about 100 to about 120°C over about 2 to about 48h to supply compound (**3-4**).

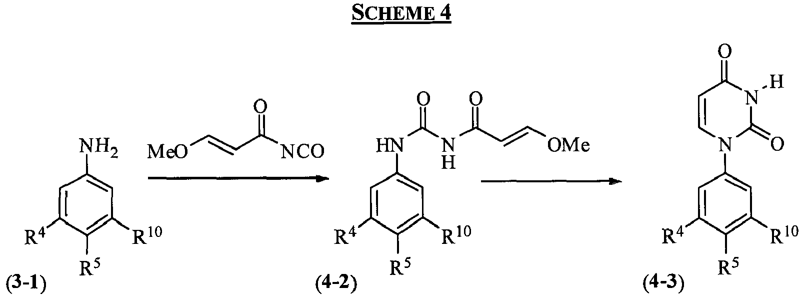

Compound (**4-2**) can be prepared from compound (**3-1**) dissolved in solvents such as, for example, dimethylformamide or dimethylacetamide by the addition of a benzene solution of (E)-3-methoxyacryloyl isocyanate (prepared as described by Santana, L.; et al. J. Heterocyclic Chem. 1999, 36, 293-295.) at a temperature of about -40 to about -15°C under an inert atmosphere and then warming to ambient temperature for from about 30 min to about 4h. Compound (**4-2**) can be treated with an acid such as, for example, sulfuric acid in mixtures of water and ethanol in a temperature range of from about 90 to about 110°C for about 1 to about 8h to supply compound (**4-3**). Alternatively, compound (**4-2**) can be cyclized to uracil (**4-3**) under the basic conditions described by Ueno, Y.; et al. J. Org. Chem. 70:7925-7935 (2005).

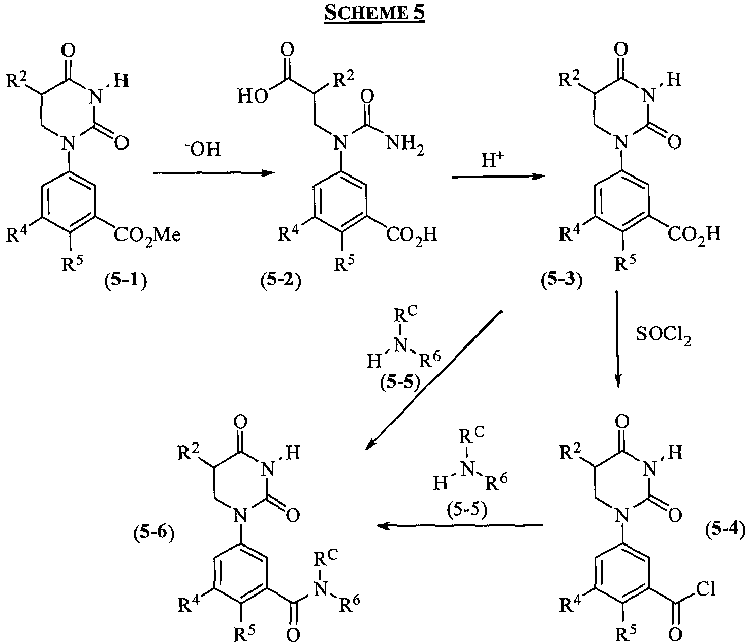

Compound (**5-1**) can be hydrolyzed with a base such as, for example, sodium hydroxide, lithium hydroxide, or potassium hydroxide in a solvent such as, for example, methanol, ethanol, or tetrahydrofuran, or mixtures thereof. The resultant reaction mixture can be stirred for a period of about 6 to about 48h at ambient temperature. Acidification with a dilute aqueous acid supplies compound (**5-2**) where the ester has been hydrolyzed and the tetrahyrdopyrimidine ring opened.

Cyclization of compound (**5-2**) to compound (**5-3**) can be accomplished by treatment with a strong acid such as, for example, concentrated hydrochloric acid in a temperature range of about 90 to about 120 °C over a period of about 1 to about 3h. Compound (**5-3**) can be treated in refluxing thionyl chloride, with or without a catalytic amount of dimethylformamide, for about 1 to about 4h to deliver acid chloride (**5-4**). Treatment with thionyl chloride at the reflux temperature in solvents such as, for example, chloroform or toluene also furnishes compound (**5-4**).

Compound (**5-4**) can be treated with an amine or the corresponding salt (**5-5**) in solvents such as, for example, dioxane, dimethylformamide, dimethylacetamide, or dichloromethane optionally in the presence of a base such as, for example, pyridine, triethylamine, or diisopropylethylamine at temperatures ranging from at or near ambient to about 100 °C for between about 1 and about 24h to provide compound (**5-6**).

Alternatively, compound (**5-3**) can be converted directly to compound (**5-6**) by reacting with an equimolar amount of amine (**5-5**) with a coupling reagent such as, for example, bis(2-oxo-3-oxazolidinyl)phosphinic chloride (BOPCI), O-(7-azabenzotriazol-1-yl)-N,N,N',N'-tetramethyluronium hexafluorophosphate (HATU), or O-benzotriazol-1-yl-N,N,N',N'-tetramethyluronium tetrafluoroborate (TBTU) along with a coupling auxiliary such as, for example, 1-Hydroxy-7-azabenzotriazole (HOAT) or 1-hydroxybenzotriazole hydrate (HOBT) in the presence or absence of a base such as, for example, N-methyl morpholine, diisopropylethylamine in solvents such as, for example, tetrahydrofuran, N,N-dimethylacetamide, N,N-dimethylformamide, pyridine, and chloroform. Typical reactions can be carried out at between about 0 to about 65°C or may be carried out in a microwave reactor to facilitate coupling.

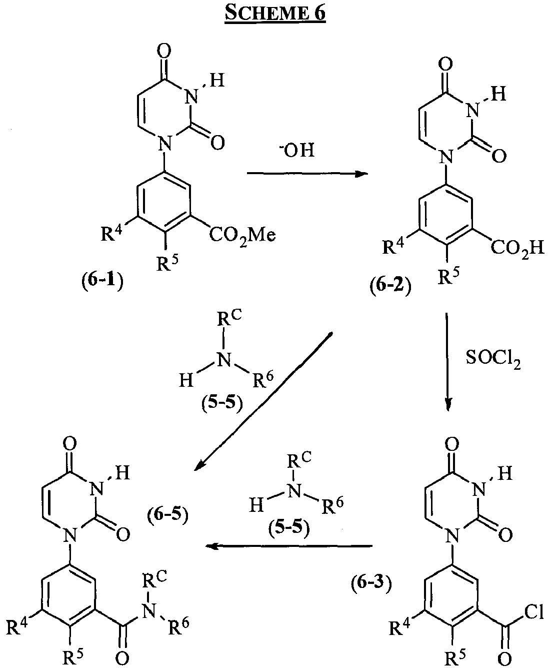

Compound (**6-1**) can be converted to compound (**6-5**) using the transformations described in Scheme 5 above.

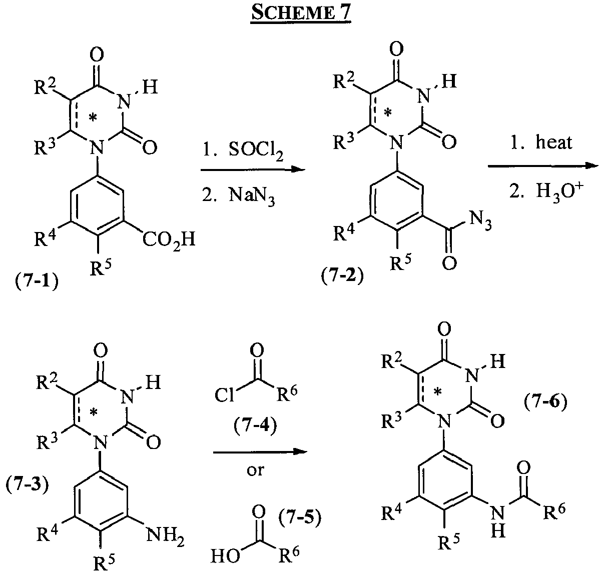

Compound (**7-1**) may be converted to acyl azide (**7-2**) in a two-step process. First, compound (**7-1**) can be treated in refluxing thionyl chloride with or without a catalytic amount of dimethyl-formamide for about 1 to about 4h to deliver the corresponding acid chloride. Treatment with thionyl chloride at the reflux temperature in solvents such as, for example, chloroform or toluene also furnishes the desired acid chloride. The acid chloride can be reacted with an aqueous solution of sodium azide in a solvent such as, for example, acetone over about 1 to about 8h to provide acyl azide (**7-2**). Compound (**7-2**) can then undergo a Curtius rearrangement in refluxing solvents such as, for example, dioxane or toluene. The intermediate isocyanate is hydrolyzed with an aqueous acid such as, for example, dilute hydrochloric acid in a solvent such as, for example, dimethoxyethane to provide compound (**7-3**). Compound (**7-3**) may be converted to compound (**7-6**) using either acid chloride (**7-4**) or carboxylic acid (**7-5**) and the amide bond forming conditions described in Schemes 5 and 6.

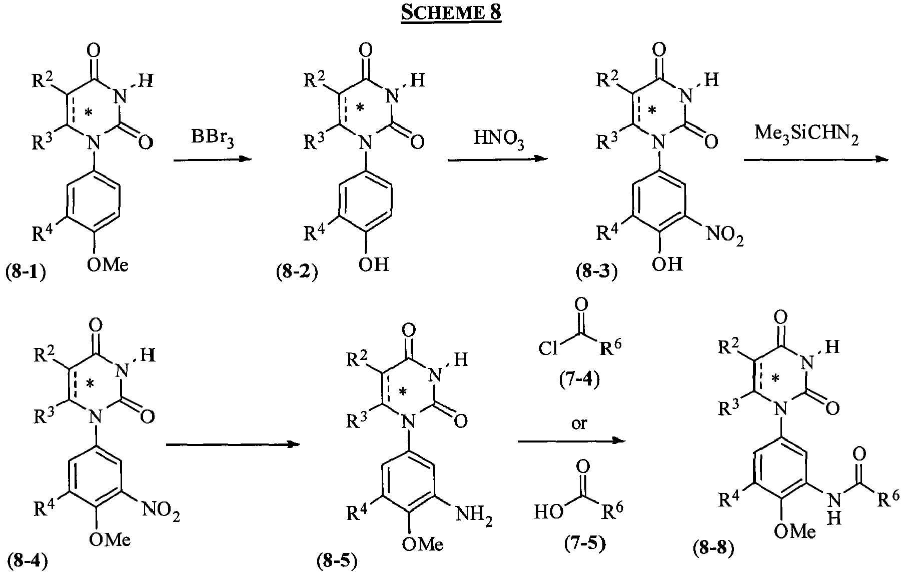

Compound (**8-1**) can be activated for nitration by removal of the methyl group with BBr₃ initially added at about 0 °C and then refluxed for about 10 to about 24h in a solvent such as, for example, dichloromethane to provide compound (**8-2**). The phenol (**8-2**) can be treated with nitric acid in acetic acid over a period of about 1 to about 10h at or near ambient temperature to supply compound (**8-3**). Compound (**8-3**) is then converted to the corresponding methyl ether (**8-4**) by treatment with a solution of (trimethylsilyl)diazomethane in tetrahydrofuran in a solvent such as, for example, methanol or a mixture of methanol and tetrahydrofuran at or near ambient temperature over about 8 to about 24h. Compound (**8-4**) may be reduced to compound (**8-5**) using a set of reduction conditions described in Scheme 1 and suitable for the functional groups present. Compound (**8-5**) can be converted into compound (**8-8**) by coupling with acid chloride (**7-4**) or carboxylic acid (**7-5**) using conditions for amide bond formation described in Schemes 5 and 6.

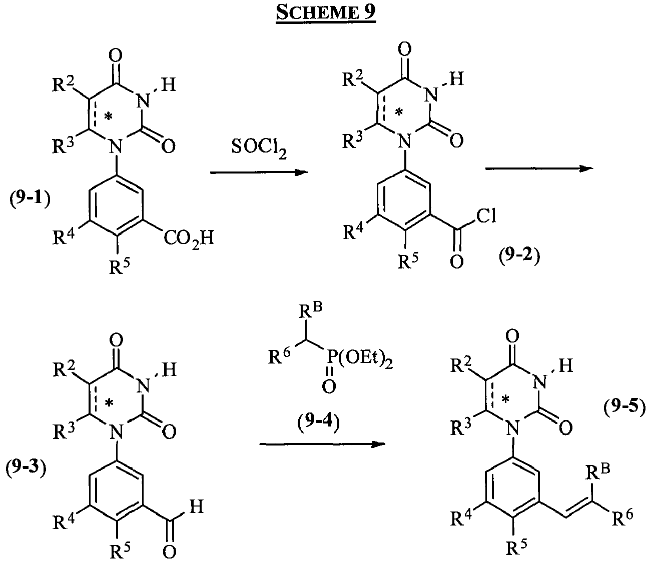

Compound (**9-1**) can be treated in refluxing thionyl chloride for about 1 to about 4h to obtain acid chloride (**9-2**). Treatment with thionyl chloride at the reflux temperature in solvents such as, for example, chloroform or toluene also furnishes compound (**9-2**). Compound (**2**) is converted to the corresponding aldehyde (**9-3**) by reduction with lithium tri-t-butoxyaluminum hydride in a solvent such as, for example, tetrahydrofuran at about -78 °C over from about 1 to about 8h. The reduction can also be achieved by treatment with indium chloride and tributyltin hydride in the presence of triphenylphosphine in a solvent such as tetrahydrofuran or toluene at temperatures from about -40 to about 0°C. Compound (**9-3**) can be treated with compound (**9-4**) in the presence of a base such as potassium t-butoxide in a solvent such as dichloromethane at or near room temperature over a period of about 1 to about 8h to provide compound (**9-5**).

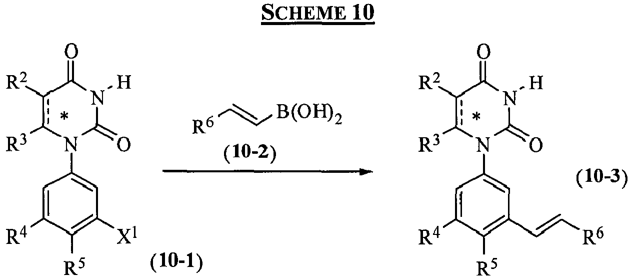

Compound (**10-1**), wherein X¹ is halo (e.g., bromine, iodine) can undergo a Suzuki reaction with vinyl boronic acid (**10-2**) to provide compound (**10-3**). The reaction typically requires the use of a base and a catalyst. Examples of bases include, for example, potassium carbonate, potassium phosphate, potassium *t*-butoxide, sodium carbonate, cesium carbonate, and cesium fluoride. Examples of catalysts include, for example, tris(dibenzylidineacetone)dipalladium (0), palladium acetate, bis(triphenyl phosphine)palladium (II) chloride, tetrakis(triphenylphosphine)palladium, dichloro[1,1'-bis(di-tert-butylphosphino)ferrocene] palladium (II), or dichloro[1,1'-bis(diphenylphosphino)ferrocene] palladium (II) dichloromethane adduct. The reaction may be conducted in a solvent such as, for example, water, dioxane, dimethoxyethane, dimethylformamide, toluene, ethanol, tetrahydrofuran and the like or mixtures thereof. The reaction may be conducted at ambient or elevated temperatures.

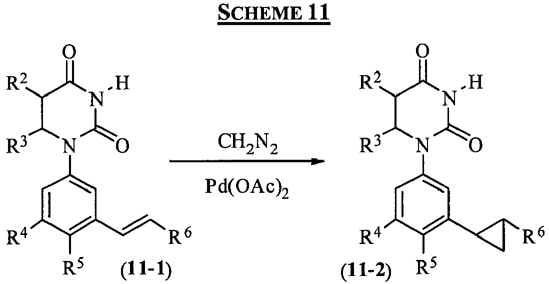

Compound (**11-1**) can be converted to compound (11-2) by treatment with diazomethane in a solvent such as, for example, tetrahydrofuran in the presence of palladium acetate at or near room temperature over a period of about 30 min to about 4h.

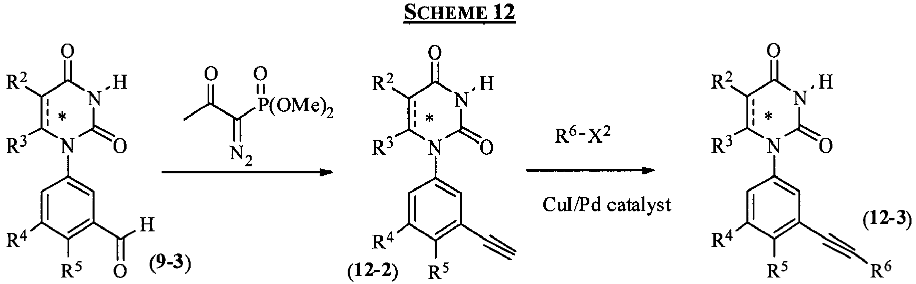

Compound (**9-3**) can be treated with dimethyl 1-diazo-2-oxopropylphosphonate (prepared as described by Ohira, S., Syn. Comm. 19:561-564 (1989)) in the presence of a base like potassium carbonate in a solvent such as, for example, methanol for about 8 to about 24h at or near room temperature to supply alkyne (**12-2**). Compound (**12-2**) is then treated with R⁶-X², wherein X² is iodine, bromine, or -O-triflate, in the presence of copper (I) iodide, palladium catalyst, base, and optionally additional triphenylphosphine in an inert atmosphere to provide compound (**12-3**). Suitable palladium catalysts include, for example, tris(dibenzylidineacetone)palladium (0), palladium acetate, bis(triphenylphosphine)palladium (II) chloride, or tetrakis(triphenylphosphine)palladium. Bases, which can be used, include, for example, triethylamine, diethylamine, diisopropylethylamine, potassium carbonate optionally in the presence of tetrabutylammonium bromide, and sodium bicarbonate. Solvents that may be used include, for example, acetonitrile, dimethylformamide, water, dioxane, and tetrahydrofuran, or mixtures thereof. The reaction can be conducted from room temperature to the reflux temperature of the solvents for about 1 to about 48h. Heating at about 50 to about 120°C in a microwave reactor from between about 5 and about 15 min also provides compound (**12-3**).

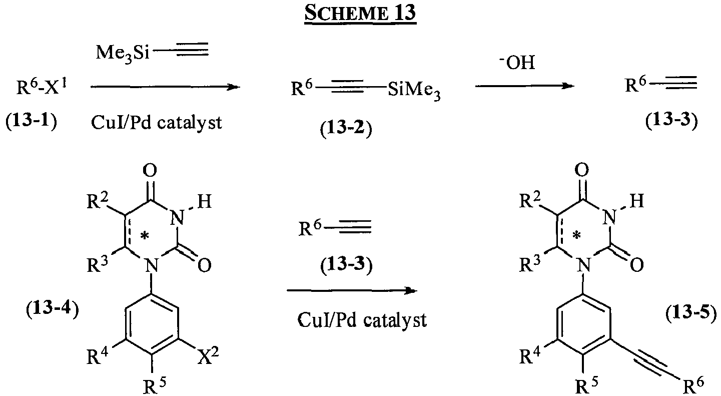

Compound (**13-1**), wherein X¹ is bromine or iodine, can be reacted in an inert atmosphere with (trimethylsilyl)acetylene in the presence of a catalyst such as, for example, palladium acetate/triphenylphosphine or copper iodide/bis(triphenylphosphine)palladium (II) chloride and a base such as, for example, triethylamine in a solvent such as, for example, toluene or acetonitrile to furnish compound (**13-2**). The reaction can be heated to about 70°C to about 100°C optionally in a sealed tube over a period of about 30 min to about 48h. Compound (**13-2**) are converted to compound (**13-3**) by treatment with a base such as, for example, potassium carbonate or sodium hydroxide in a solvent such as, for example, methanol at ambient temperature. Compound (**13-3**) are reacted with compound (**13-4**), wherein X2 is bromine or iodine, in the presence of copper (I) iodide, palladium catalyst, base, and optionally additional triphenylphosphine in an inert atmosphere to provide compound (**13-5**). Suitable palladium catalysts include, for example, tris(dibenzylidineacetone)dipalladium (0), palladium acetate, bis(triphenylphosphine)palladium (II) chloride, or tetrakis(triphenylphosphine)palladium. Bases, which can be used, include, for example triethylamine, diethylamine, diisopropylethylamine, potassium carbonate optionally in the presence of tetrabutylammonium bromide, and sodium bicarbonate. Solvents that may be used include, for example, acetonitrile, dimethylformamide, water, dioxane, and tetrahydrofuran, or mixtures thereof. The reaction can be conducted from about 40°C to the reflux temperature of the solvents for about 15 min to about 48h. Microwave heating at about 50°C to about 120°C from between about 5 and about 15 min is an alternative heating process to provide compound (**13-5**).

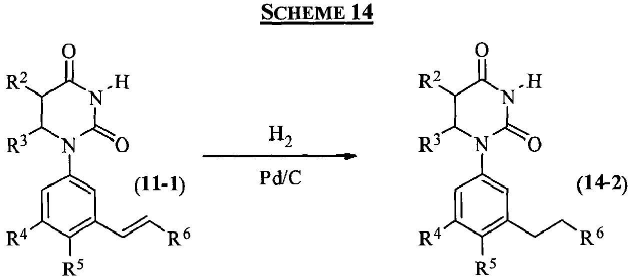

Compound (**11-1**) is reduced to supply compound (**14-2**). Typical conditions for this reduction include using hydrogen at a pressure of about 1 to about 5 atmospheres in the presence of a catalyst such as, for example, palladium or platinum on charcoal in a solvent such as, for example, tetrahydrofuran, ethyl acetate, ethanol, or hexane at or near ambient temperature over a period of about 1 to about 12h.

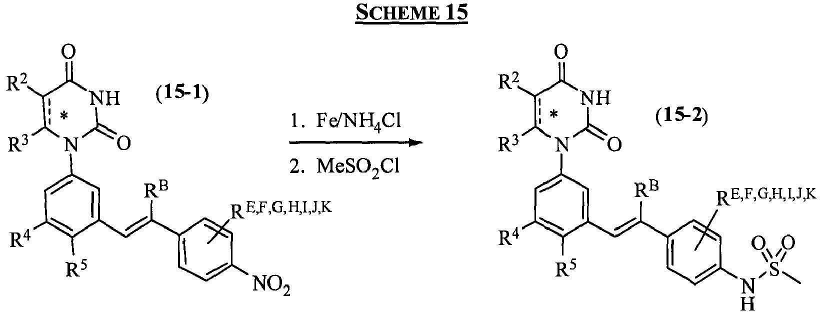

Compound (**15-1**) can be converted in a two-step sequence to compound (**15-2**). The initial step involves reduction of the aromatic nitro moiety with iron powder in the presence of a mild acid such as, for example, ammonium chloride or dilute hydrochloric acid at temperatures from about 60 to about 80°C in a mixture of solvents containing, for example, methanol, water, and tetrahydrofuran over about 1 to about 12h. The second step consists of exposure of the aniline, prepared in the first step, to methanesulfonyl chloride in the presence of a base such as pyridine in a solvent such as dichloromethane at or near ambient temperature.

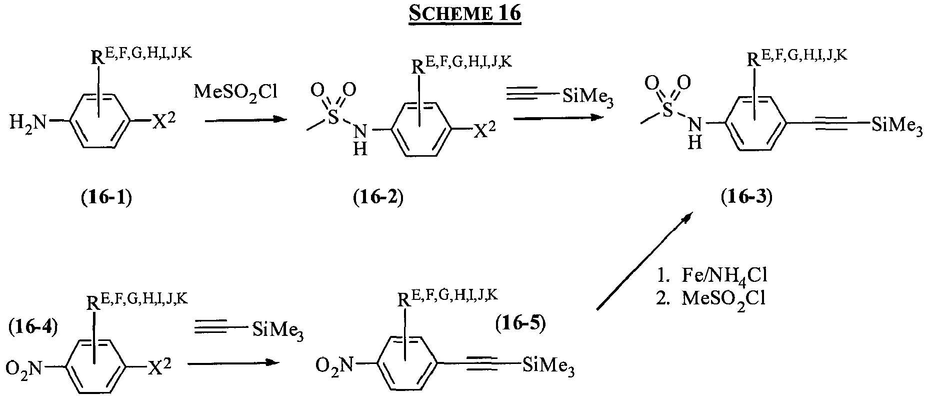

Aniline (**16-1**) and aromatic nitro compound (**16-4**), wherein X² is, for example, bromine, iodine, or triflate, can be converted to compound (**16-3**). Compound (**16-1**) can be transformed to compound (**16-2**) by treatment with methanesulfonyl chloride in the presence of a base such as pyridine in a solvent such as dichloromethane. Then compound (**16-2**) is converted to compound (**16-3**) by treatment in an inert atmosphere with (trimethylsilyl)acetylene in the presence of a catalyst such as, for example, palladium acetate, bis(triphenylphosphine)palladium (II) chloride, bis(triphenylphosphine)palladium (II) chloride in combination with copper(I) iodide and, when X² is bromine, triphenylphosphine, and a base such as, for example, triethylamine in a solvent such as toluene or acetonitrile at approximately 80°C.

Compound of formula (**16-4**) can be reacted in an inert atmosphere with (trimethylsilyl) acetylene in the presence of a catalyst such as, for example, bis(triphenylphosphine)palladium (II) chloride/ copper(I) iodide and a base such as, for example, triethylamine in a solvent such as, for example, acetonitrile at approximately 80°C to give compound (**16-5**). Compound (**16-5**) can be converted to compound (**16-3**) in a two-step sequence. The initial step involves reduction of the aromatic nitro moiety with iron powder in the presence of a mild acid such as, for example, ammonium chloride or dilute hydrochloric acid at temperatures from about 60 to about 80°C in a mixture of solvents containing, for example, methanol, water, and tetrahydrofuran over about 1 to about 12h. The second step consists of exposure of the aniline, prepared in the first step, to methanesulfonyl chloride in the presence of a base such as pyridine in a solvent such as dichloromethane at or near ambient temperature.

Removal of the trimethylsilyl group of compound (**16-3**) is accomplished as described for the production of compound (**13-3**) in Scheme 13 above.

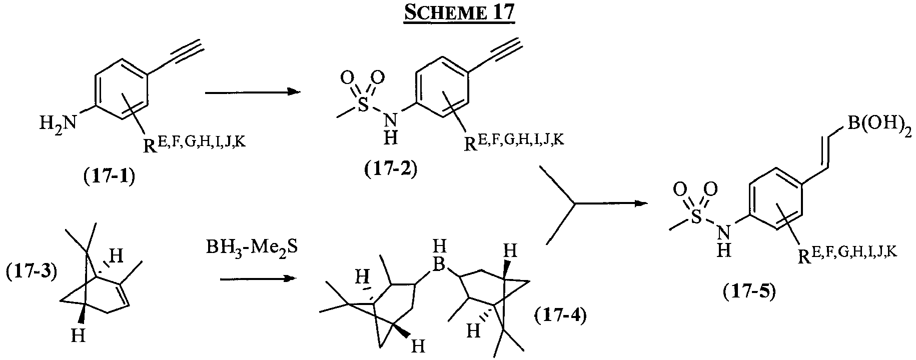

Compound (**17-1**) can be mesylated to provide compound (**17-2**) by treatment with methanesulfonyl chloride in the presence of a base such as, for example, pyridine in a solvent such as, for example, dichloromethane. Compound (**17-3**) can be exposed to borane dimethyl sulfide complex in a solvent such as, for example, tetrahydrofuran at approximately about 0 to about 10°C to supply compound (**17-4**). Compounds (**17-2**) and (**17-4**) can be combined with acetaldehyde in refluxing tetrahydrofuran. Subsequent treatment with water at room temperature yields compound **(17-5**).

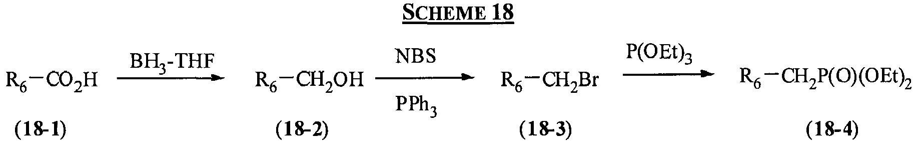

Carboxylic acid (**18-1**) can be reduced with boron tetrahydrofuran complex with heating to provide alcohol (**18-2**). Compound (**18-2**) is converted to the corresponding bromide (**18-3**) with N-bromosuccinimide and triphenylphosphine in solvents such as, for example, dichloromethane at room temperature in several hours. Treatment of compound (**18-3**) with triethyl phosphite at about 120°C for about 1 to about 3h supplies compound (**18-4**). Compound (**18-4**) can be used for example to make compound (**9-5**) as described in Scheme 9.

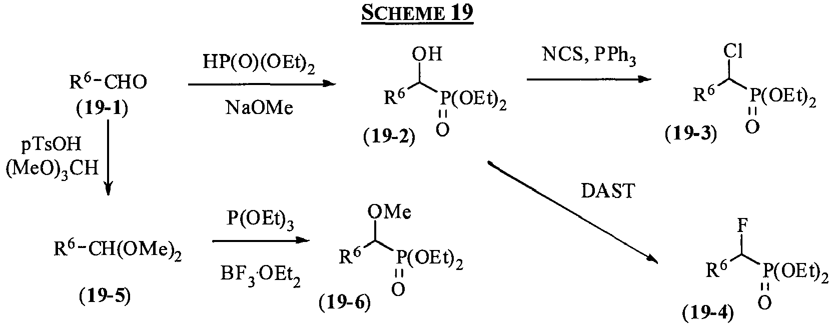

Benzaldehyde (**19-1**) can be treated with diethyl phosphonate in the presence of a base such as, for example, sodium methoxide in a solvent such as, for example, methanol at room temperature to provide compound (**19-2**). Compound (**19-2**) can be treated with N-chlorosuccinimide and triphenylphosphine in dichloromethane at room temperature to yield compound (**19-3**). Compound (**19-2**) can also be reacted with (diethylamino)sulfur trifluoride (DAST) to supply compound (**19-4**).

Compound (**19-1**) can also be treated with p-toluenesulfonic acid and trimethyl orthoformate in methanol at about 50°C to provide acetal (**19-5**). Compound (19-5) can be converted to compound (**19-6**) by exposure to triethyl phosphite and boron trifluoride diethyl etherate at about -20°C to about ambient temperature.

Compounds (**19-3**), (**19-4**), and (**19-6**) can be used for example to make compound (**9-5**) as described in Scheme 9.

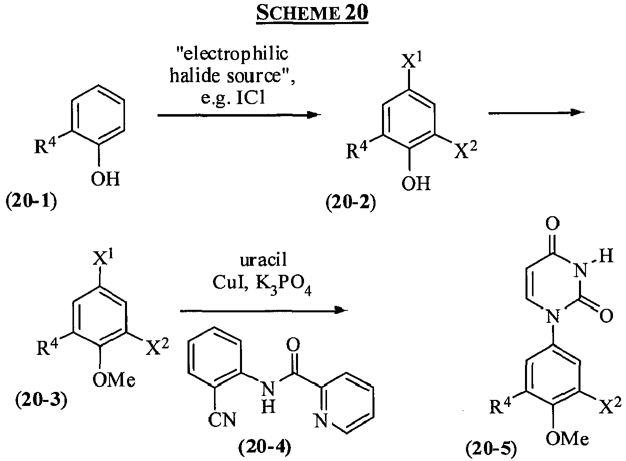

Phenol (**20-1**), wherein R⁴ is other than amino, is treated with a source of electrophilic halide, such as, for example, iodine monochloride to provide dihalogenated compound (**20-2**), wherein X¹ and X² are independently bromine or iodine. Compound (**20-2**) is transformed to compound (**20-3**) by reaction of an alkylating agent such as, for example, methyl sulfate with a base such as, for example, potassium carbonate in refluxing acetone. Alternatively, methyl iodide in the presence of a base such as, for example, potassium t-butoxide in a solvent such as, for example, tetrahydrofuran, or dimethylformamide also furnish compound (**20-3**). In yet another alternative, compound (**20-2**) can be methylated with (trimethylsilyl)diazomethane in a solvent such as, for example, t-butyl methyl ether. Compound (**20-3**) can be reacted with uracil, ligand (**20-4**), copper (I) iodide, and potassium phosphate in dimethyl sulfoxide at about 40°C to about 100°C to supply compound (**20-5**).

For example, when in compound (**20-3**), R⁴ is tert-butyl, X¹ is iodo, and X² is iodo or bromo, compound (20-3) can be stirred with uracil and compound (**20-4**) in the presence of CuI and K₂PO₄ in DMSO for about 15 to about 24h at about 60°C to supply compound (**20-5**). Alternatives to ligand (**20-4**) for making (**20-5**) are 8-hydroxyquinoline and 2-(2-pyridyl)-benzimidazole.

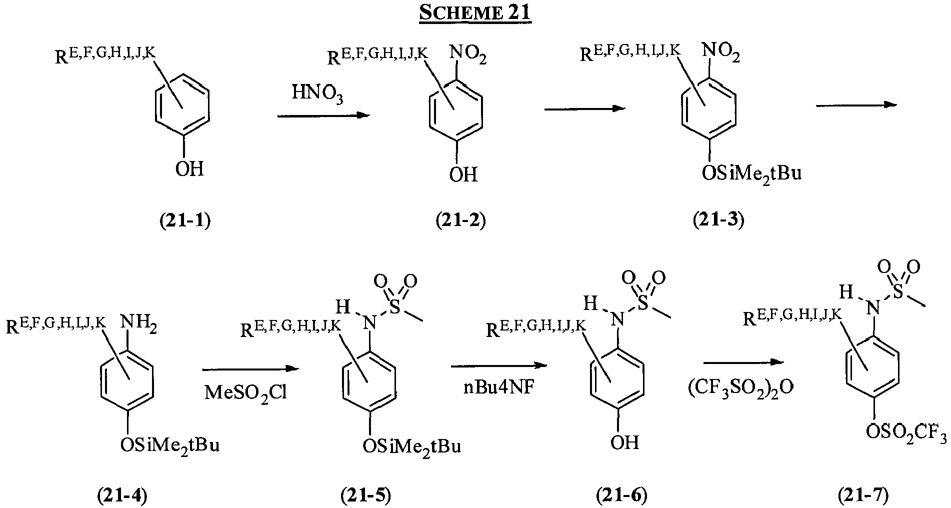

Compound (**21-1**) can be nitrated with nitric acid in acetic acid in a temperature range of about 10 to about 15°C to give compound (**21-2**). The phenol moiety of compound (**21-2**) can be protected as a silyl ether, e.g. t-butyldimethylsilyl ether, by treatment with a silyl chloride such as, for example, t-butyl dimethylsilyl chloride and imidazole in a solvent such as, for example, dimethyl formamide at ambient temperature to furnish compound (**21-3**). Compound (**21-3**) may then be reduced using conditions known to those skilled in the art to furnish the corresponding aniline (**21-4**).

Typical conditions for this reduction include using hydrogen at a pressure of about 1 to about 5 atmospheres in the presence of a catalyst such as, for example, palladium or platinum on charcoal in a solvent such as, for example, tetrahydrofuran, ethyl acetate, ethanol, methanol, or hexane at or near ambient temperature over a period of about 1 to about 12h. Dependent on the functional groups present, an alternative reduction procedure may be more appropriate such as, for example, using iron powder in the presence of a mild acid such as, for example, ammonium chloride or dilute hydrochloric acid at reflux temperatures in a mixture of solvents containing, for example, methanol, water, and tetrahydrofuran over about 1 to about 12h.

Aniline (**21-4**) can then by sulfonylated with methanesulfonyl chloride in the presence of pyridine in a solvent such as, for example, dichloromethane. The starting material and reagents are combined at about 0°C and then allowed to gradually warm to ambient temperature over the course of the reaction to supply compound (**21-5**). The silyl ether protecting group is removed under conditions familiar to one skilled in the art. For example, tetrabutylammonium fluoride in tetrahydrofuran at room temperature transforms compound (**21-5**) to compound (**21-6**). The phenol group of compound (**21-6**) may be sulfonylated with trifluoromethanesulfonic anhydride in the presence of a base such as, for example, pyridine in a solvent such as, for example, dichloromethane at room temperature to provide compound (**21-7**). Compound (**21-7**) can be used as described in Scheme 12 to make compound (**12-3**).

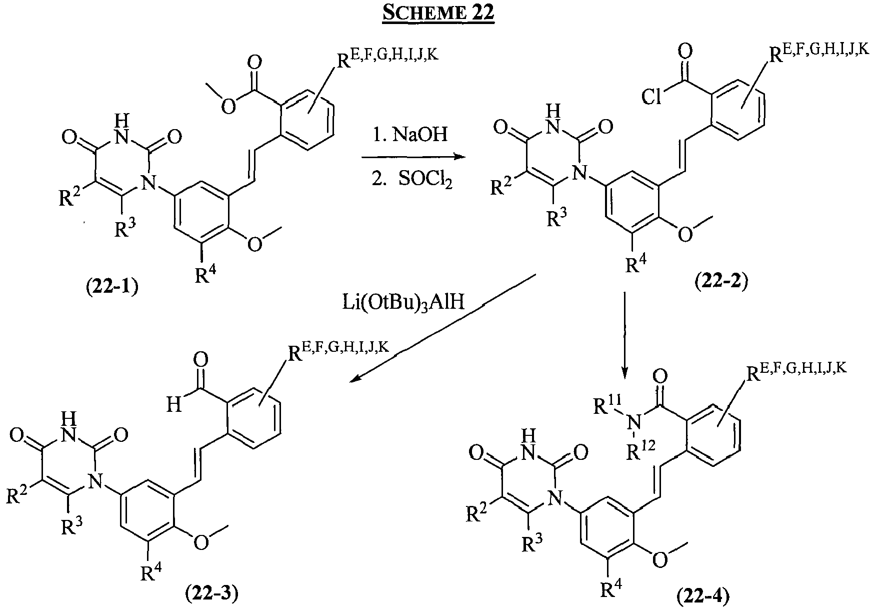

Compound (**22-1**) is converted to compound (**22-2**) in a two-step sequence. First, compound (**22-1**) can be hydrolyzed with a base such as, for example, sodium hydroxide, lithium hydroxide, or potassium hydroxide in a solvent such as, for example, methanol, ethanol, or tetrahydrofuran, or mixtures thereof. The resultant reaction mixture can be stirred for a period of about 6 to about 48h at ambient temperature. Second, the intermediate carboxylic acid is treated in refluxing thionyl chloride with or without a catalytic amount of dimethylformamide for about 1 to about 4h to deliver acid chloride (**22-2**). Treatment with thionyl chloride at reflux temperature in solvents such as, for example, chloroform or toluene also furnishes compound (**22-2**). Treatment of the carboxylic acid with oxalyl chloride in dichloromethane with a catalytic amount of dimethylformamide also furnishes compound (**22-2**).

Compound (**22-2**) can be treated with an amine or the corresponding salt in a solvent such as, for example, dioxane, dimethylformamide, dimethylacetamide, or dichloromethane optionally in the presence of a base such as, for example, pyridine, triethylamine or diisopropylethylamine at temperatures ranging from at or near ambient to about 100°C for between about 1 and about 24h to provide compound (**22-4**) wherein R¹¹ and R¹² are independently hydrogen or R^{F}, or taken together with the nitrogen to which they are attached form a 5-6-membered heterocyclyl or a fused 2-ring heterocyclyl.

Compound (**22-2**) is converted to the corresponding aldehyde (**22-3**) by reduction with lithium tri-*t*-butoxyaluminum hydride in a solvent such as, for example, tetrahydrofuran at about -60°C to about - 78°C.

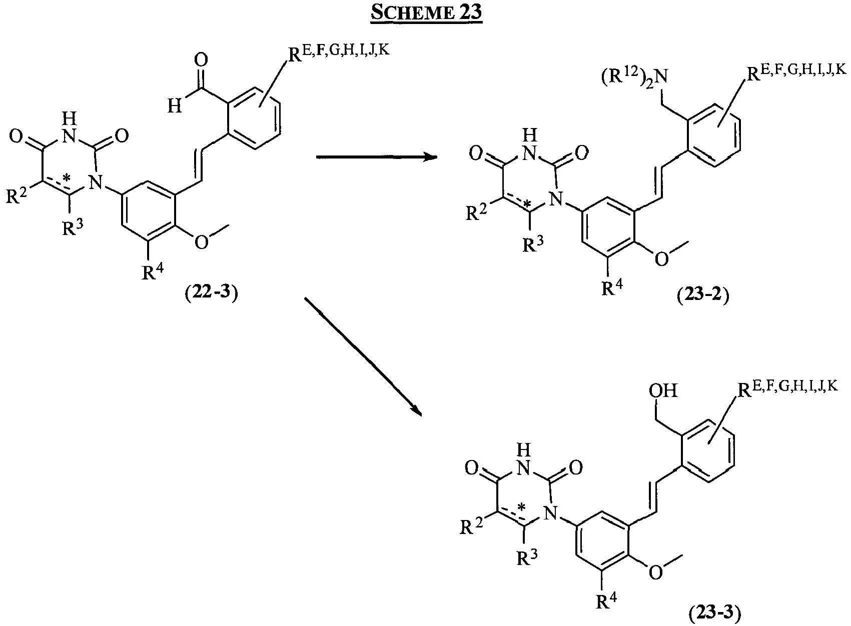

Compound (**22-3**) can be converted to compound (**23-2**) wherein R¹¹ and R¹² are independently hydrogen or R^{F}, or taken together with the nitrogen to which they are attached form a 5-6-membered heterocyclyl or a fused 2-ring heterocyclyl by treatment with an amine, N(R¹¹)(R¹²), in the presence of a reductant such as, for example, sodium triacetoxyborohydride or sodium cyanoborohydride in a solvent such as, for example, methanol, ethanol, dichloromethane, dimethylacetamide, or dimethylformamide over a period of about 1 to about 24h. The reaction often proceeds best at an acidic pH that can be maintained by the addition of acetic acid or hydrochloric acid.

Compound (**22-3**) can also be converted to compound (**23-3**) by reduction with lithium tri-*t-*butoxyaluminum hydride in a solvent such as tetrahydrofuran at room temperature.

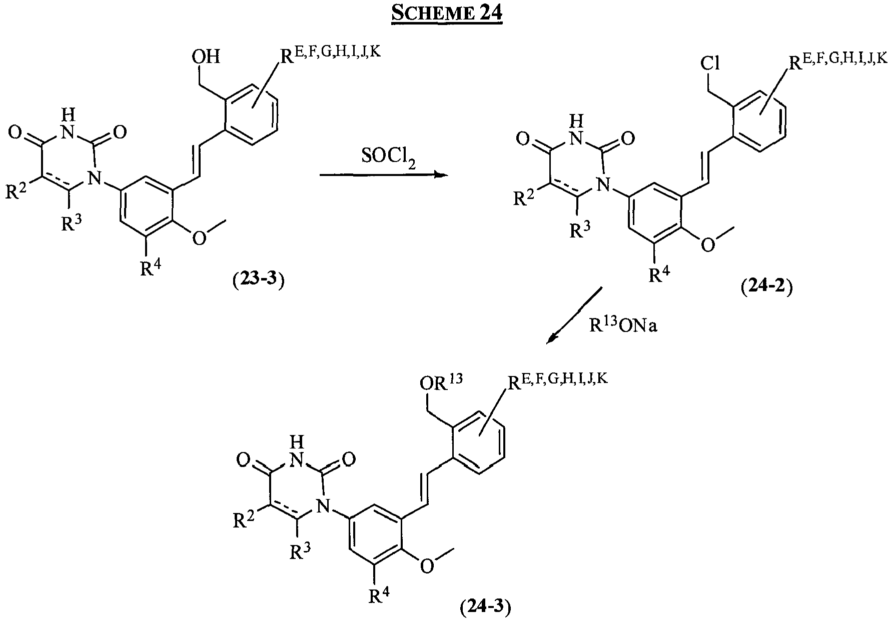

Compound (**23-3**) can be converted to compound of formula (**24-2**) by treatment with thionyl chloride in dichloromethane at room temperature. Compound (**24-2**) can be treated with a sodium alkoxide, R¹³ ONa, in a heated solution of the corresponding alcohol to provide compound (**24-3**), wherein R¹³ is hydrogen or R^{F}.

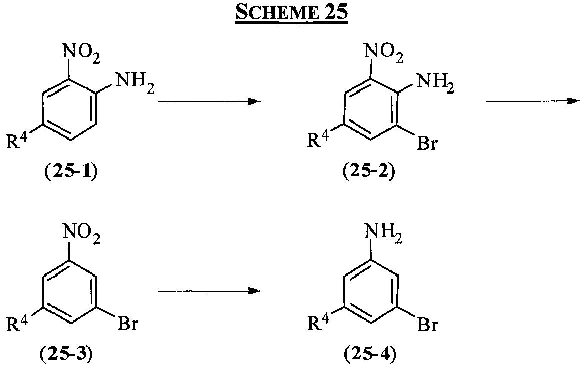

Compound (**25-1**) can be brominated by treatment with, for example, pyridinium hydrobromide perbromide in a solvent such as, for example, acetic acid at or near ambient temperature over a period of about 1 to about 8h to give compound (**25-2**). The amino group of compound (**25-2**) can be removed by exposure to t-butyl nitrite in a solvent such as, for example, dimethylformamide at a temperature initially at ambient temperature and then increased to the range of about 50 to about 65 °C to give compound (**25-3**). Additional aliquots of t-butyl nitrite can be added at ambient temperature followed by heating until the transformation is complete. Compound (**25-3**) can be reduced to compound (**25-4**) by, for example, treatment with iron and ammonium chloride.

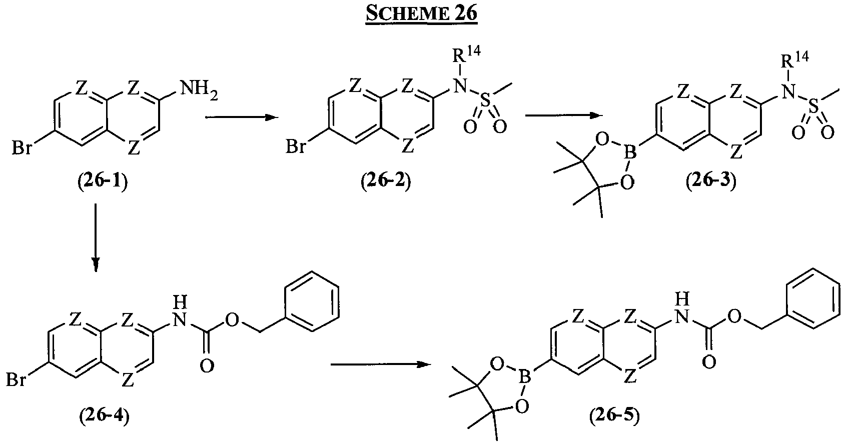

Compound (**26-1**), wherein each Z is independently N or CH can be converted to a boronic acid ester for use in Suzuki reactions. For example, compound of formula (**26-1**) can be converted to compound (**26-2**), wherein R¹⁴ is hydrogen or methanesulfonyl (when excess methanesulfonyl chloride is used) by treatment with methanesulfonyl chloride in pyridine at approximately ambient temperature in about 1 to about 8h.

Compound (**26-2**) can be transformed to compound of (**26-3**) by treatment with pinacol-borane in the presence of a catalyst such as, for example, tris(dibenzylidineacetone)dipalladium (0), ligand such as, for example, tri-t-butylphosphine, and a base such as triethylamine in solvents such as, for example, tetrahydrofuran, dioxane, or toluene at temperatures ranging from ambient to about 130°C.

Alternatively, compound (**26-2**) can be reacted with bis(pinacolato)diboron in the presence of a catalyst such as, for example, Combiphos® Pd6, dichloro[1,1'-bis(diphenylphosphino)ferrocene] palladium (II) dichloromethane adduct, or palladium acetate in the presence of a ligand such as, for example, 2-dicyclohexylphosphino-2',4',6'-triisopropylbiphenyl (XPhos), and a base such as, for example, potassium acetate in solvents such as, for example, toluene, dioxane, tetrahydrofuran, dimethylformamide or dimethyl sulfoxide in temperatures from about 60 to about 130°C to give compound (**26-3**).

Compound (**26-3**) can be converted to protected compound (**26-4**) by treatment with benzyl chloroformate initially at about 0°C in the presence of saturated aqueous sodium bicarbonate in a mixture of acetone and water. This can be warmed to ambient temperature and maintained at that temperature for about 12 to about 24h. Subsequently, compound (**26-4**) can be converted to the boronic acid pinacol ester (**26-5**) using the reaction conditions described above.

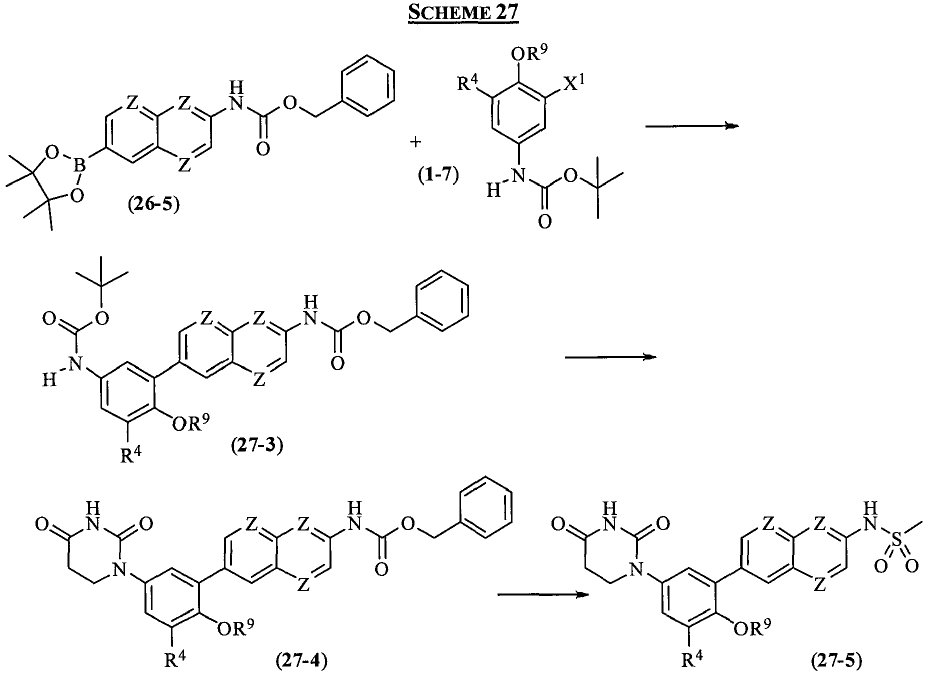

Compound (**26-5**), wherein each Z is independently N or CH, can be coupled with compound (**1-7**) under Suzuki reaction conditions to provide compound (**27-3**). Such conditions include, for example, use of a palladium catalyst such as, for example, tris(dibenzylidineacetone)palladium (0), palladium acetate, bis(triphenylphosphine)palladium (II) chloride, tetrakis(triphenylphosphine)palladium, or dichloro[1,1'-bis(diphenylphosphino)ferrocene] palladium (II) dichloromethane adduct; base such as, for example, potassium carbonate, potassium phosphate, potassium *t*-butoxide, sodium carbonate, cesium carbonate, or cesium fluoride; and solvent such as, for example, toluene, ethanol, water, or tetrahydrofuran, or mixtures thereof heated in the temperature range from about 40 to about 130°C.

Compound (**27-3**) can be transformed to compound (**27-4**) in a three-step process. The initial step involves removal of the t-butoxycarbonyl protecting group with an acid such as, for example, trifluoroacetic acid in solvent such as, for example, dichloromethane or hydrochloric acid in dioxane at room temperature over about 1 to about 24h. Subsequently, the dihydropyrimidinedione can be introduced as described in Scheme 3.

Compound (**27-5**) can be obtained from compound (**27-4**) in a two-step sequence. First, the protecting group is removed from the naphthyl amine under reductive conditions. Typically, hydrogenation (∼1 atmosphere pressure) in the presence of a catalyst such as, for example, 10% palladium on charcoal in a solvent such as, for example, ethyl acetate at or near ambient temperature over a period of about 8 to about 24h. Second, the naphthyl amine can now be sulfonylated by treatment with methanesulfonyl chloride in the presence of a base such as triethylamine in a solvent (e.g., dichloromethane) at room temperature over about 20 min to about 4h.

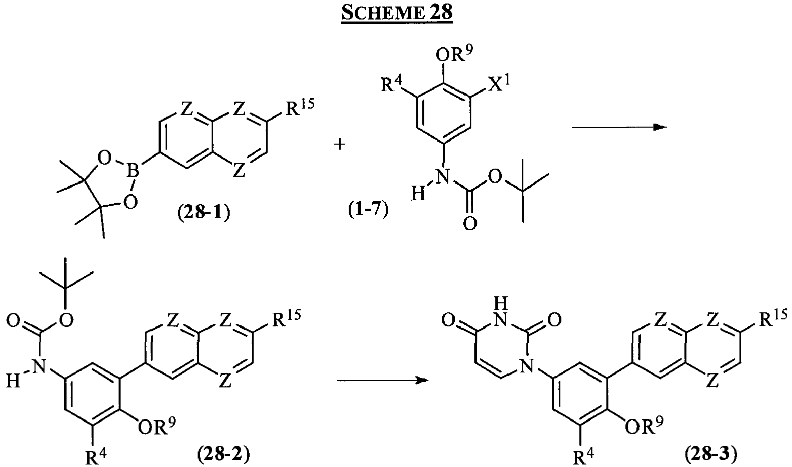

Compound (**28-1**), wherein each Z is independently N or CH, and R¹⁵ is, for example, hydrogen, -NHSO₂Me, -N(SO₂Me)₂ or methoxy can be coupled with compound (**1-7**) under Suzuki reaction conditions to provide compound (**28-2**). Such conditions include, for example, use of palladium catalyst such as, for example, tris(dibenzylidineacetone) palladium (0), palladium acetate, bis(triphenylphosphine)palladium (II) chloride, tetrakis(triphenylphosphine)palladium, or dichloro[1,1'-bis(diphenylphosphino)ferrocene]palladium (II) dichloromethane adduct; a base such as potassium carbonate, potassium phosphate, potassium *t*-butoxide, sodium carbonate, cesium carbonate, or cesium fluoride; and solvent such as, for example, toluene, ethanol, water or tetrahydrofuran, or mixtures thereof heated in the temperature range from about 40 to about 130°C. The reaction is typically deoxygenated with an inert gas such as nitrogen prior to heating. The heating may occur in conventional glassware, a sealed tube, or in a microwave reactor over about 1 to about 24h.

Compound (**28-2**) can be transformed to compound (**28-3**) in a three-step process. The initial step involves removal of the t-butoxycarbonyl protecting group with an acid such as, for example, trifluoroacetic acid in solvent such as, for example, dichloromethane or hydrochloric acid in dioxane at room temperature over about 1 to about 24h. Subsequently, the uracil can be introduced as described in Scheme 4.

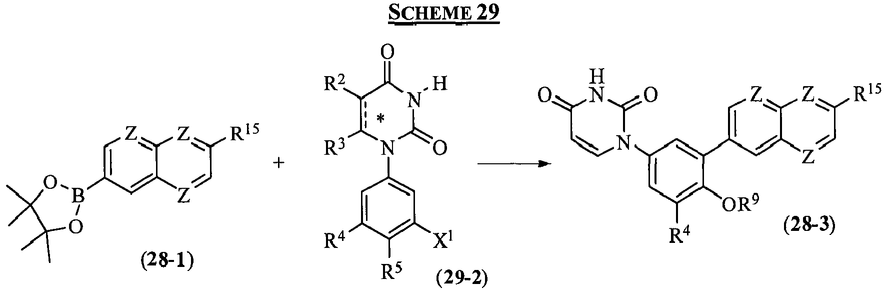

Compound (**28-1**), wherein each Z is independently N or CH, and R¹⁵ is, for example, hydrogen, -NHSO₂Me, -N(SO₂Me)₂, or methoxy can be coupled with compound of formula (**29-2**), wherein X¹ is, for example, bromine or iodine, under Suzuki reaction conditions to provide compound of formula (**28-3**). Such conditions include, for example, use of palladium catalyst such as, for example, tris(dibenzylidineacetone)palladium (0), palladium acetate, bis(triphenylphosphine)palladium (II) chloride, tetrakis(triphenylphosphine) palladium , dichloro[1,1'-bis(diphenylphosphino)ferrocene] palladium (II) dichloromethane adduct, or bis(diphenylphosphino)ferrocene]palladium (II) dichloromethane; base such as, for example, potassium carbonate, potassium phosphate, potassium *t-*butoxide, sodium carbonate, cesium carbonate, or cesium fluoride; and solvent such as, for example, toluene, ethanol, water, or tetrahydrofuran, or mixtures thereof heated in the temperature range from about 40 to about 130°C. The reaction is typically deoxygenated with an inert gas such as nitrogen prior to heating. The heating may occur in conventional glassware, a sealed tube, or in a microwave reactor over about 1 to about 24h.

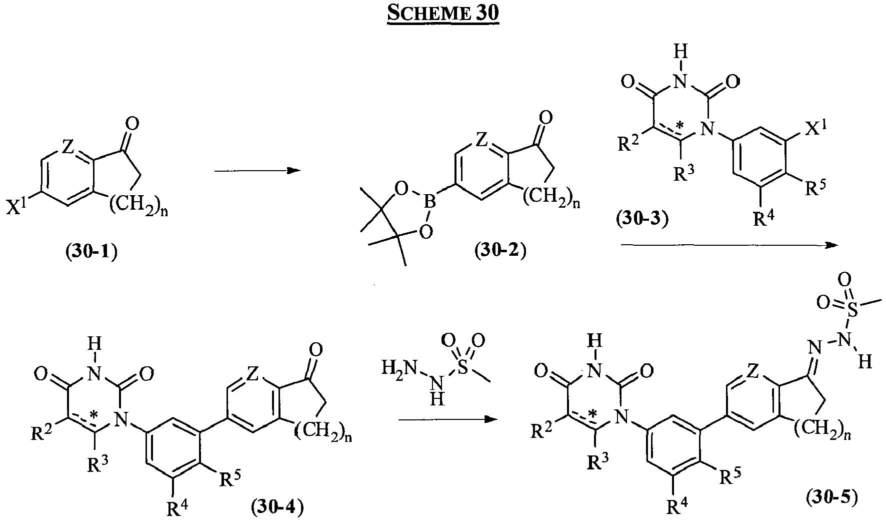

Compound (**30-1**), wherein X¹ is bromine or iodine, n is 1 or 2, and Z is CH or N, can be reacted with bis(pinacolato)diboron in the presence of a catalyst such as, for example, Combiphos® Pd6, dichloro[1,1'-bis(diphenylphosphino)ferrocene]palladium (II) dichloromethane adduct, or palladium acetate in the presence of a ligand such as 2-dicyclohexylphosphino-2',4',6'-triisopropylbiphenyl (XPhos), and a base such as potassium acetate in solvents such as, for example, toluene, dioxane, tetrahydrofuran, dimethylformamide or dimethyl sulfoxide in temperatures from 60-130°C to give compound (**30-2**). The reaction is typically deoxygenated with an inert gas such as nitrogen prior to heating. The heating may occur in conventional glassware, a sealed tube, or in a microwave reactor over 1 to 24h. Compound (**30-3**) can be reacted with compound (**30-2**) to give compound (**30-4**) employing the conditions described in Scheme 29.

Treatment of compound (**30-4**) with methanesulfonylhydrazide in solvent such as, for example, tetrahydrofuran, methanol, or ethanol, or a mixture thereof at ambient temperature to about 100°C over a period of 8 to 48h provides compound (**30-5**).

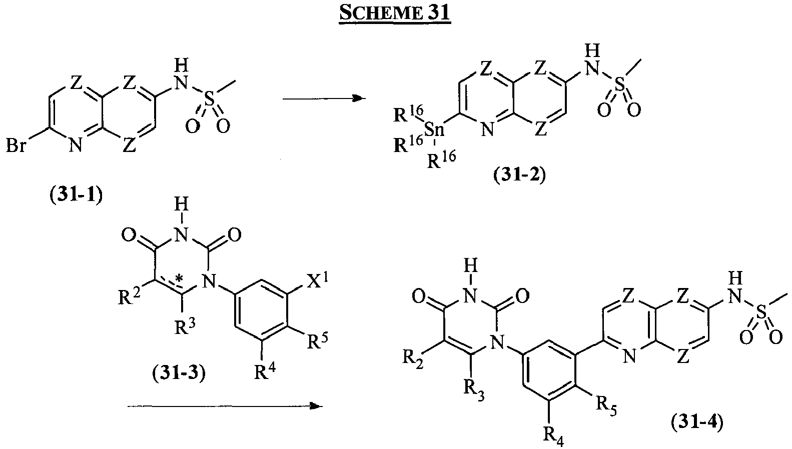

Compound (**31-1**) can be treated with hexamethylditin or hexabutylditin in the presence of a catalyst such as, for example, bis(triphenylphosphine)palladium (II) chloride in a solvent such as, for example, toluene or dioxane heated to about 50 to about 130°C to supply compound (**31-2**). Compound (**31-2**) can be treated with compound (**31-3**) in presence of catalyst such as, for example, tris(dibenzy-lidine acetone)palladium (0) and ligand such as tri(2-furyl)phosphine in solvent such as, for example, toluene, dioxane, or tetrahydrofuran heated to about 40 to about 130°C to give compound (**31-4**).

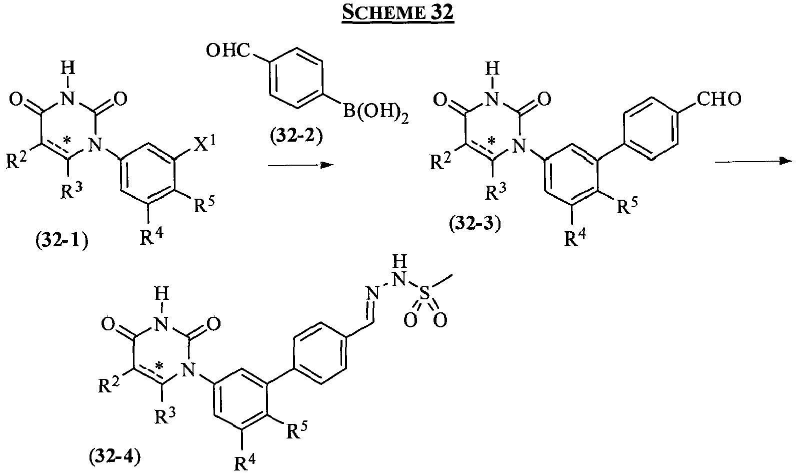

Compound (**32-1**) can be reacted with compound (**32-2**) under the Suzuki reaction conditions to give compound (**32-3**). Treatment with methanesulfonylhydrazide as described in Scheme 30 provides compound (**32-4**).

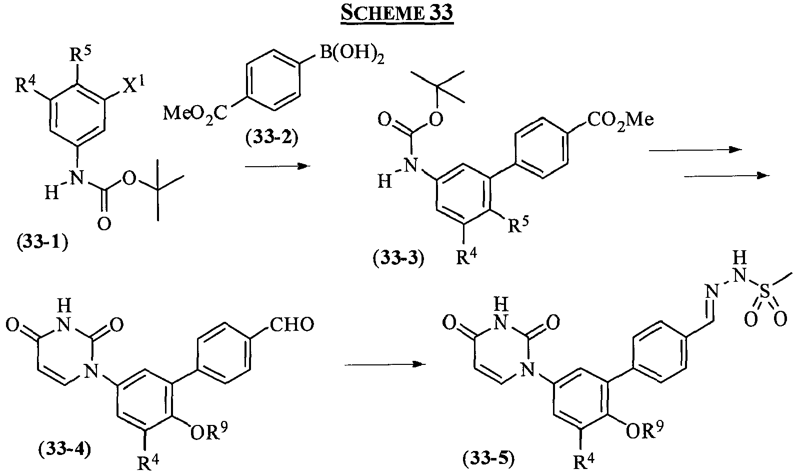

Compound (**33-1**) can be reacted with compound (**33-2**) under Suzuki reaction conditions to give compound (**33-3**). Compound (**33-3**) can be converted to compound (**33-4**) by first constructing the uracil ring. Then, the methyl carboxylate can be converted into the corresponding aldehyde. Compound (**33-4**) can be treated with methanesulfonylhydrazide to provide compound (**33-5**).

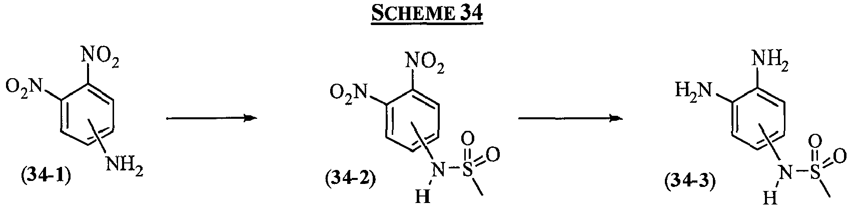

Dinitroaniline (**34-1**) can be sulfonylated with methanesulfonyl chloride in the presence of a base like, for example, pyridine in a solvent such as, for example, dichloromethane at room temperature over a period of about 8 to about 36h to give compound (**34-2**). Compound (**34-2**) can be converted to compound (**34-3**) using iron powder in the presence of a mild acid such as, for example, ammonium chloride or dilute hydrochloric acid at reflux temperatures in a mixture of solvents, such as, for example, methanol, water, and tetrahydrofuran over about 1 to about 12h.

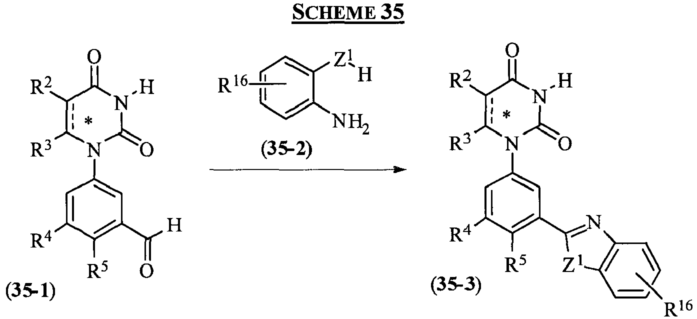

Compound (**35-1**) can be reacted with compound (**35-2**), wherein Z¹ is O, S, or NH and R¹⁶ is hydrogen, -NHSO₂Me, or NO₂, in the presence of charcoal exposed to air in solvent such as, for example, toluene heated from about 90 to about 110°C for about 24 to about 72h to give compound (**35-3**).

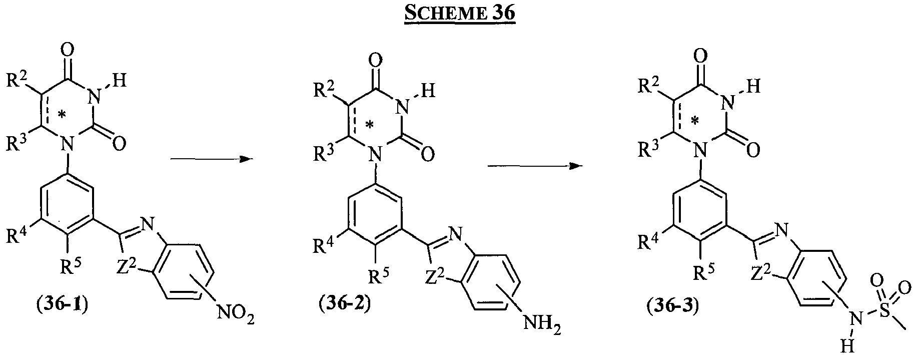

Compound (**36-1**), wherein Z² is O or S, can be reduced to compound (**36-2**) using iron powder in the presence of a mild acid such as, for example, ammonium chloride or dilute hydrochloric acid at temperatures of about 60 to about 90°C in solvents such as, for example, methanol, ethanol, water, and tetrahydrofuran, or mixtures thereof over about 30 min to about 12h. Compound (**39-2**) can be sulfonylated with methanesulfonyl chloride in the presence of a base like, for example, pyridine in a solvent such as, for example, dichloromethane at room temperature over a period of about 8 to about 36h.

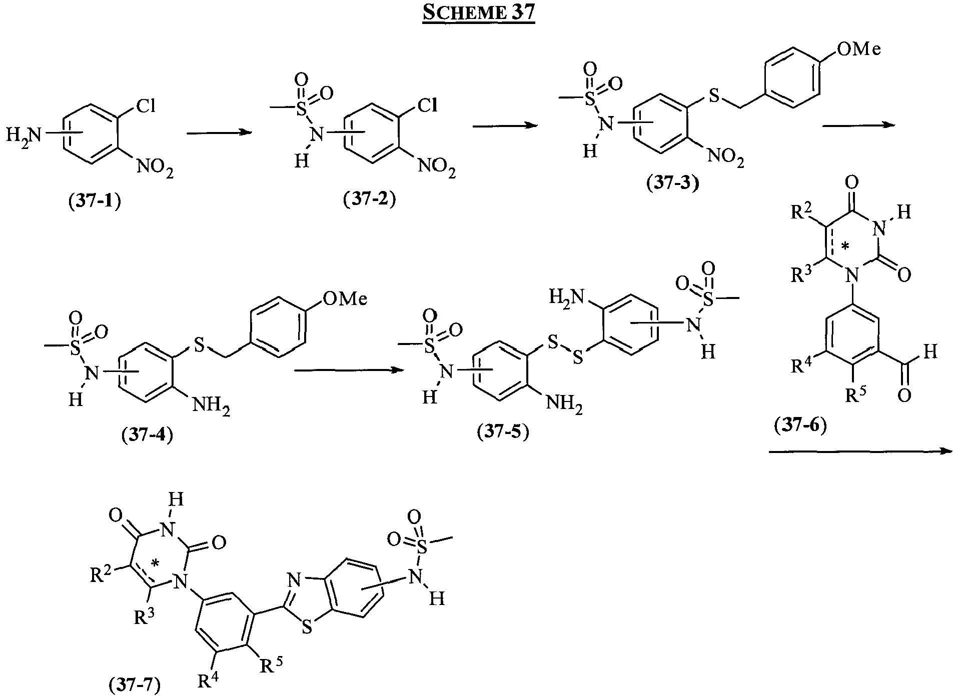

Compound (**37-1**) can be sulfonylated with methanesulfonyl chloride in the presence of a base like, for example, pyridine in a solvent such as, for example, dichloromethane at room temperature over a period of about 8 to about 36h to give compound (**37-2**). Compound (**37-2**) can be reacted with (4-methoxyphenyl)methanethiol in the presence of a base such as, for example, potassium carbonate in a solvent such as, for example, dimethylformamide heated to about 90 to about 110°C for about 8 to about 24h to give compound (**37-3**). Compound (**37-3**) can be reduced to compound (**37-4**) using iron powder in the presence of a mild acid such as, for example, ammonium chloride or dilute hydrochloric acid at temperatures of about 60 to about 90°C in solvent such as, for example, methanol, ethanol, water, and tetrahydrofuran, or mixtures thereof over about 30 min to about 12h. Compound (**37-4**) can be transformed to compound (**37-5**) in the presence of mercury(II) acetate, anisole, and trifluoroacetic acid at about 0 °C for about 30 to about 90 min and subsequently bubbling hydrogen sulfide through the mixture. Compound (**37-5**) can be treated with compound (**37-6**) in the presence of p-toluenesulfonic acid and triphenylphosphine in a solvent such as, for example, toluene heated to reflux for about 2 to about 16h to supply compound (**37-7**).

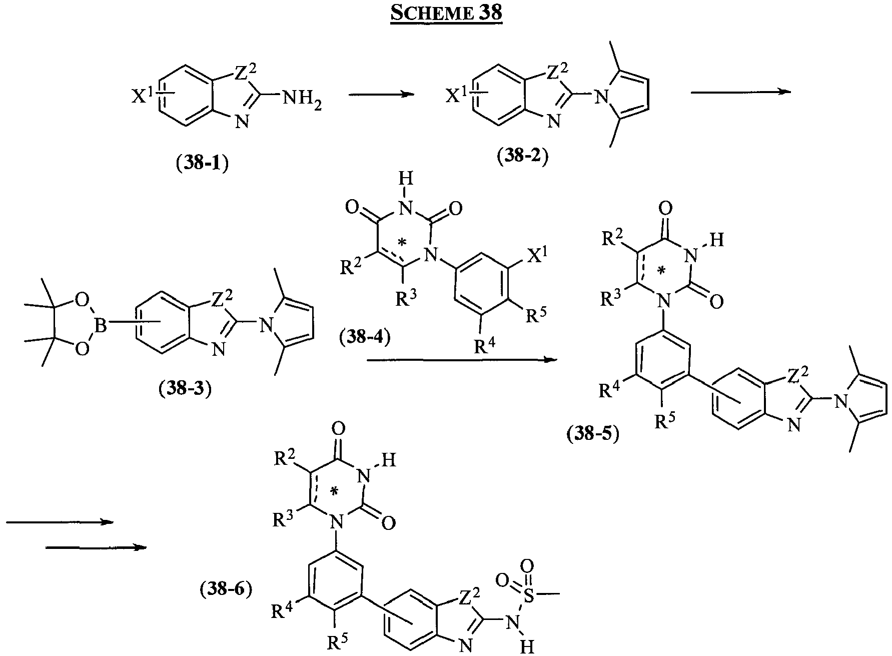

Compound (**38-1**), wherein X¹ is bromine or iodine and Z² is O or S, can be reacted with 2,5-hexanedione in the presence of a p-toluenesulfonic acid and pyridine heated in benzene to give compound of formula (**38-2**). Compound (**38-2**) can be reacted with bis(pinacolato)diboron in the presence of a catalyst such as, for example, Combiphos® Pd6, dichloro[1,1'-bis(diphenylphosphino)ferrocene] palladium (II) dichloromethane adduct, or palladium acetate in the presence of a ligand such as, for example, 2-dicyclohexylphosphino-2',4',6'-triisopropylbiphenyl (XPhos), and a base such as, for example, potassium acetate in a solvent such as, for example, toluene, dioxane, tetrahydrofuran, dimethylformamide or dimethyl sulfoxide at a temperature from about 60 to about 130°C to give compound (**38-3**). Compound (**38-3**) can be reacted with compound (**38-4**) to give compound (**38-5**) under Suzuki reaction conditions. Such conditions include, for example, use of a palladium catalyst such as, for example, dihydrogen dichlorobis(di-t-butylphosphinito-KP)palladate(2-), tris(dibenzylidineacetone) palladium (0), palladium acetate, bis(triphenylphosphine)palladium (II) chloride, tetrakis (triphenylphosphine)palladium, or dichloro[1,1'-bis(diphenylphosphino)ferrocene] palladium (II) dichloromethane adduct; a base such as, for example, potassium acetate, potassium carbonate, potassium phosphate, potassium t-butoxide, sodium carbonate, cesium carbonate, or cesium fluoride; and solvent such as, for example, toluene, ethanol, water or tetrahydrofuran, or mixtures thereof heated in the temperature range from about 40 to about 130°C.

Compound (**38-5**) can be treated with hydroxylamine hydrochloride in heated ethanol to remove the pyrrole-protecting group. Then treatment with methanesulfonyl chloride in the presence of a base such as, for example, pyridine in a solvent such as, for example, dichloromethane at or near ambient temperature supplies compound (**38-6**).

### EXAMPLES

The following examples are merely illustrative, and not limiting to this disclosure in any way.

### Example A. Preparation of (E)-N-(3-tert-butyl-5-iodo-4-methoxyphenylcarbamoyl)-3-methoxy acrylamide.

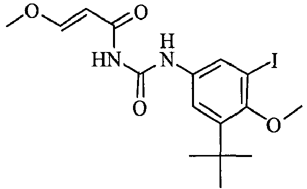

### Part A. Preparation of 2-tert-butyl-4-nitrophenol.

To a vigorously stirred solution of 2-tert-butylphenol (10g, 66.6mmol) in heptane (67ml) was added at a fast drip a solution of 70% nitric acid (4.25ml, 66.6mmol) diluted with water (4.25ml). The resulting dark red/brown mixture was stirred vigorously for 2h. The suspended solid was collected by filtration washed with hexane (300mL), water (200mL) and once again with hexane (200mL) to give a cocoa colored powder that was dried to constant mass (4.65g, 35.6%).

### Part B. Preparation of 2-tert-butyl-6-iodo-4-nitrophenol.

To the product from **Part A** (4.5g, 23.05mmol) dissolved in MeOH (120ml) and water (30mL) was added iodine monochloride (1.155ml, 23.05mmol) drop wise over a period of 10min The mixture was stirred for 2h and diluted into 1L of water and allowed to stand overnight. The solid material was collected by filtration and washed 3x50mL with water and dried under vacuum overnight to give a tan solid (7.14g, 96%).

### Part C. Preparation of 1-tert-butyl-3-iodo-2-methoxy-5-nitrobenzene.

To an ice bath cooled solution of the product from **Part B** (5.5g, 17.13mmol) in MTBE (15ml) in a 50mL pressure vessel was added 2.0M TMS diazomethane (12.85ml, 25.7mmol) followed by drop-wise addition of methanol (1.0mL) resulting in calm bubbling. The vessel was sealed and stirred at room temperature for 16h, cooled and the pressure was released. The solution was partitioned between EtOAc and water. The organic layer was washed with 1.0M HCl, saturated potassium carbonate solution, and saturated NaCl. The organic layer was dried over sodium sulfate, filtered and concentrated to give a red oil that was used without purification (5.4g, 84%).

### Part D. Preparation of 3-tert-butyl-5-iodo-4-methoxyaniline.

A mixture of the product from **Part C** (5.80g, 17.31mmol), ammonium chloride (1.389g, 26.0mmol), and iron (4.83g, 87mmol) in THF/MeOH/water (200mL total, 2/2/1) was refluxed for 2h, cooled and filtered through Celite. The filtrate was evaporated and the residue was partitioned between water and EtOAc. The organic layer was washed with saturated brine, dried with sodium sulfate, filtered and evaporated to give a brown oil (5.28g, 100% yield).

### Part E. Preparation of (E)-N-(3-tert-butyl-5-iodo-4-methoxyphenylcarbamoyl)-3-methoxy acrylamide.

To a solution of the product from **Part E** (3.05g, 10mmol) in DMF (50ml) at -20°C under N₂ was added at a fast drip a 0.4M solution in benzene of (E)-3-methoxyacryloyl isocyanate (50.0ml, 20.00mmol, prepared by the method of Santana et al., J. Heterocyclic Chem. 36:293 (1999). The solution was stirred for 15min at -20°C, warmed to room temperature for 45min and diluted into EtOAc. The EtOAc layer was washed 4 x 300mL with water, 2 x 100mL with brine, dried (Na₂SO₄) and concentrated to a brown solid. The residue was triturated in Et₂O/hexane to give a fine powder that was collected by filtration and dried to give a tan powder (2.46g, 57%).

### Example B. Preparation of 1-(3-tert-butyl-5-iodo-4-methoxyphenyl)dihydropyrimidine-2,4(1H,3H)-dione.

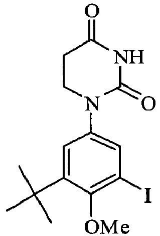

To a suspension of the product from **Example A** (2.46g, 5.69mmol) in ethanol (50ml) was added a solution of 5.5mL of H₂SO₄ in 50mL water and the mixture was heated at 110°C for 2.5h to give a clear solution. The solution was cooled and diluted with 50mL of water while stirring to give an off-white solid that was collected by filtration, washed with water and dried (2.06g, 90%).

### Example C. Preparation of 1-(3-tert-butyl-5-iodo-4-methoxyphenyl)pyrimidine-2,4(1H,3H)-dione.

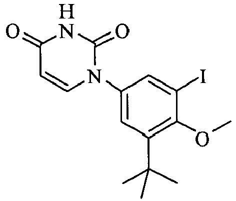

### Part A. Preparation of 2-tert-butyl-4,6-diiodophenol.

A solution of 2-tert-butylphenol (20.0g, 133mmol) in methanol (266mL) was treated with sodium hydroxide pellets (6.39g, 160mmol). The mixture was stirred until all the sodium hydroxide had dissolved and was then cooled in an ice-salt bath to -2°C. Sodium iodide (15.0g, 100mmol) was added and then 10 % sodium hypochlorite solution (45mL, 73.3mmol) was added drop wise at a rate such that the solution temperature rose no higher than 1.3°C. This sequence of events was repeated (3x) until a total of 60g (400mmol) of sodium iodide had been added and the sodium hypochlorite solution was added until the solution color changed from a light green-yellow color to the color of weak iced tea. This required all but 16mL of the 180mL total sodium hypochlorite solution measured out. With continued cooling at ca. 2°C, a solution of sodium thiosulfate pentahydrate (20g) in water (100mL) was added drop wise over 20min. After addition, the solution was acidified to pH 3 by drop wise addition of concentrated hydrochloric acid (ca. 35mL required of 40mL placed in the addition funnel). The precipitate was collected by filtration and washed with >1 liter of water. The salmon-colored solid was sucked as dry as possible, and dried in a vacuum oven at 50°C for 18h. These procedures afforded the product (49.61g, 93%) as a tan solid.

### Part B. Preparation of 1-tert-butyl-3,5-diiodo-2-methoxybenzene.

A solution of the product from **Part A** (20.0g, 49.7mmol) in acetone (140mL) was treated with methyl iodide (3.9mL, 8.83g, 62.2mmol) and 50 % (w/w) sodium hydroxide solution (3.02mL, 4.58g, 57.2mmol) followed by stirring at ambient temperature for 48h. The mixture was concentrated in vacuo to a volume of ca. 50-60mL, followed by dilution with heptane (80mL) and water (50mL). The layers were separated and the organic layer was extracted with saturated sodium chloride solution. Drying (Na₂SO₄) and concentration in vacuo afforded the product (20.59g, 99%) as a light yellow oil.

### Part C. Preparation of 1-(3-tert-butyl-5-iodo-4-methoxyphenyl)pyrimidine-2,4(1H,3H)-dione.

A suspension of the product from **Part B** (12.04g, 28.9mmol), uracil (3.89g, 34.7mmol), N-(2-cyanophenyl)picolinamide (1.29g, 5.79mmol) and tribasic potassium phosphate (12.9g, 60.8mmol) in DMSO (181mL) was degassed by nitrogen sparge for 1 h. The mixture was then treated with copper (I) iodide (551mg, 2.89mmol) and degassing was continued for another 10min. The mixture was then warmed at 60°C for 18h. The mixture was then poured into water (600mL) and acidified to pH 3 by addition of 4N hydrochloric acid solution. The mixture was diluted with ethyl acetate, and the organic layer was extracted with water (3x), saturated ammonium chloride solution (1x) and saturated sodium chloride solution. The solution was dried and treated with (3-mercaptopropyl) silica gel, followed by stirring for 2h. The mixture was filtered and concentrated in vacuo. The solid obtained was triturated with ether-ethyl acetate (>10:1) and collected by filtration and washed with ether. After drying in a vacuum oven at 50°C for 2h, these procedures afforded the product (2.75 g) as a white solid. The mother liquors were concentrated in vacuo to afford an amber solid. This material was chromatographed over a Flash 65 silica gel cartridge, eluting with 20-100 % ethyl acetate in hexanes. These procedures afforded a nearly white solid, which was triturated with ether-hexanes and collected by filtration. After drying in a vacuum oven for 3h, these procedures afforded another 4.31 g of the product as a white solid. Total yield: 7.06g (61 %).

### Example D. Preparation of 1-(3-tert-Butyl-5-iodo-4-methoxyphenyl)pyrimidine-2,4(1H,3H)-dione.

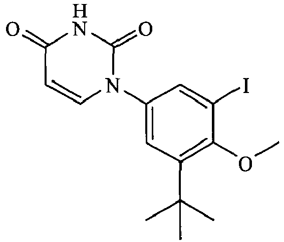

### Part A. Preparation of 2-tert-butyl-4,6-diiodophenol.

2-*tert*-Butylphenol (99.95g, 665.36mmol) was dissolved in 1250mL methanol and converted to the corresponding phenoxide with 31.96g (799.0mmol, 1.2equiv.) of sodium hydroxide by stirring the sodium hydroxide pellets at room temperature, and then cooling the reaction mixture in an ice/salt bath. Sodium iodide (299.34g, 1997.07mmol, 3.0equiv.) and 8.3% bleach (1265.83g, 1411.39mmol, 2.1equiv.) were added to the cold reaction solution in four equal portions, the bleach being added while keeping the reaction mixture at <0°C. 500mL of 20% (w/w) sodium thiosulfate solution was added over an 18-minute period, with the temperature rising from -0.6°C to 2.5°C. The pH of the reaction mixture was adjusted to approximately 3 by adding 197.5mL of conc. HCl over a period of 97min with the reaction temperature going from 1.2°C to 4.1°C. The resulting slurry was filtered, and the wet cake washed with ~ 2L of water. The wet cake was left on the Buchner funnel under vacuum overnight (approximately 15h) to yield 289.33g (potency adjusted yield = 254.61g) of the title product.

### Part B. Preparation of 1-tert-butyl-3,5-diiodo-2-methoxybenzene.

The product from **Part A** (93%assay, 21.6g, 50mmol) was dissolved in 140mL of acetone. Methyl iodide (4.2mL, 67.5mmol, 1.35equiv.) was added, followed by 50% aqueous sodium hydroxide (5.0g, 62.5mmol, 1.25equiv.). The reaction was stirred overnight, then concentrated to approximately 50-60mL. 80mL of heptanes was added followed by 50mL of water, and the layers were shaken and separated, and the aqueous layer was back extracted with 20mL of heptanes. The organic layers were combined and washed twice with 50mL each of 10% aqueous NaCl to afford 91.1grams of a heptane solution, which assayed to 19.1 g of the title compound.

### Part C. Preparation of 1-(3-tert-Butyl-5-iodo-4-methoxyphenyl)pyrimidine-2,4(1H,3H)-dione.

Uracil (33.3g, 297mmol, 1.2equiv.), K₃PO₄ (106g, 500mmol, 2.1equiv.), CuI (4.6g, 24.2mmol, 0.1equiv.), and N-(2-cyanophenyl)picolinamide (6.4g , 28.7mmol, 0.12equiv.) were charged to a flask and inerted with argon. The 1-tert-butyl-3,5-diiodo-2-methoxybenzene was solvent switched into MeCN, dissolved in 1L DMSO and sparged with argon and added to the solids. The reaction was heated to 60°C for 16h. After cooling, the reaction was diluted with 2L EtOAc and washed with 2.6L water (back extracted with 3 x 1L EtOAc). The combined organic layers were washed with 2 x 1L of 0.25M (CuOAc)₂ then 2 x 830mL 15% NH₄Cl then 800mL brine. The organic layer was then concentrated and chased with 1L heptane, then triturated with refluxing 85:15 (v/v) heptane:iPrOAc for 4h. After cooling, the product was collected by filtration and washed with an additional 330mL of 85:15 v/v heptanes:EtOAc to yield after drying 66.9g (70% yield) of the product as a white solid.

### Example E. Preparation of N-(6-(3-tert-Butyl-5-(2,4-dioxo-3,4-dihydropyrimidin-1-(2H)-yl)-2-methoxyphenyl)naphthalen-2-yl)methanesulfonamide.

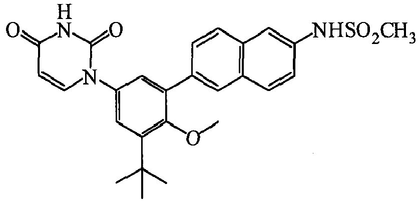

A solution of 100mL of water and 300mL of THF was sparged with nitrogen and then transferred via canula and nitrogen pressure to a flask containing 19.9965g (49.96mmol) of the product from **Example D,** 20.8234g (59.97mmol, 1.20equivalents) of *N*-(6-(4,4,5,5-tetramethyl-1,3,2-dioxaborolan-2-yl)naphthalen-2-yl)methanesulfonamide, and 21.8711g (103.03mmol, 2.06equivalents) of potassium phosphate which had been purged with nitrogen. The resulting solution was again sparged with nitrogen.

THF (100mL) was sparged with nitrogen and then transferred via canula and nitrogen pressure to a flask containing 462.8mg (0.51mmol, 0.01equivalents) of Pd₂dba₃ and 735.8mg (2.52mmol, 0.05 equivalents) of 1,3,5,7-tetramethyl-6-phenyl-2,4,8-trioxa-6-phosphaadamantane, which had been purged with nitrogen. The resulting solution was again sparged with nitrogen.

The initial THF/water solution was transferred via canula and nitrogen pressure to the flask containing the catalyst and ligand in THF. The reaction was warmed to 50°C and stirred overnight under positive nitrogen pressure. A sample of the reaction was taken the following morning. HPLC of the sample showed 0.28 PA% iodouracil starting material, 76.8 PA% product, and 5.2 PA% boronate.

The reaction was cooled to room temperature and washed, in three portions, with a solution of 5.84g of L-cysteine and 81.4g of sodium chloride in 550mL of water which had been sparged with nitrogen. The THF solution was filtered through a celite pad. The pad was rinsed with 100mL of THF, which was combined with the original THF solution. The THF solution was concentrated on the rotary evaporator to 136g. To the white slurry was added 405mL of ethyl acetate with good agitation. The slurry was filtered after stirring overnight. The wet cake was washed with 2X50mL of ethyl acetate. The solid, an ethyl acetate solvate, was dried in the vacuum oven at 50°C. It weighed 25.49g.

The solid and 8.7g of 3-mercaptopropyl derivatized silica gel was stirred in 500mL of THF then filtered through a celite pad. The filtrate was concentrated on the rotary evaporator to give 13.08g of white solid. The solid that had been filtered off on the celite pad was extracted with 500mL of THF at 60°C. The THF solution was concentrated to 66g and treated with 206mL of ethyl acetate. The solid which precipitated was filtered and dried, yielding 9.13g of product. This solid was combined with the original solid and slurried in 100mL of 200 proof 3A ethanol. It was filtered and dried in the vacuum oven at 50°C to give 20.74g of product.

### Example F. Preparation of (E)-N-(4-(3-tert-butyl-5-(2,4-dioxo-3,4-dihydropyrimidin-1(2H)-yl)-2-methoxystyryl)phenyl)methanesulfonamide.

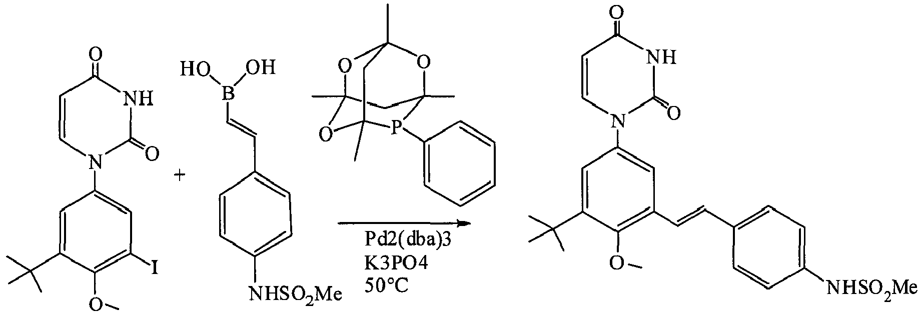

The boronic acid (96% potency) (3.75g, 15.6mmol, 1.2eq), product from **Example D** (5.0g, 12.5mmol), Cytec ligand (175mg, 5mol%), Pd2(dba)3 (46mg, 0.4mol%) and potassium phosphate (5.25g, 25.0mmol, 2eq.) were charged to a 3 neck RB flask. The solids were purged with nitrogen for 10min. 75mL 4:1 THF:water was sparged 10min and charged to the flask. The mixture was stirred to dissolve the solids followed by heating the mixture at 50°C in darkness overnight. HPLC showed the reaction was not complete after stirring overnight (~ 2% iodouracil remained). The reaction mixture was diluted with 375mL DCM and 250ml 10% citric acid. The mixture was shaken in a sep funnel and the layers were separated. The DCM layer was washed with a solution of 0.6g L-cysteine in 250ml 5% NaHCO₃ for 30min which changed the DCM layer color from orange to yellow. Repeated the 0.6g L-cysteine in 250ml 5% NaHCO3 for 30min treatment followed by a 250ml 5% NaHCO₃ wash, and a 250ml 10% NaCl wash. The DCM layer was treated with 2gm thiourea silica for 30min. Added 1gm carbon to decolorize mixed 5min and filtered through hy-flo. The wet cake was washed with DCM. The DCM solution was then stripped to give 6.74g of a light yellow solid. The solids were ~92% pure. The solids were heated in a mixture of 192ml DCM and 9mlL MeOH. They never completely dissolved. Cooled to room temp with mixing. 80ml heptane was added and more product began to crystallize. The slurry stirred over the weekend. Added 50ml heptane in portions until a total of 230ml heptane was added. The product was filtered. Filtrate was measured at 1.21mg/mL at 210nm and 1.35 at 220nm, which equals a 522-582mg loss in the liquors or 9-10% loss vs. theoretical. The wet cake was washed with 50ml of a 27ml Heptane:22ml DCM: 1ml MeOH mixture. The wash contained 0.5mg/mL product or 25mg (0.4% vs. theoretical). Product yield 5.22gm (88.9%), purity 99.2%PA. Iodouracil was removed in the crystallization. Samples were submitted to solid state for analysis and analytical for Pd determination. NMR did not show any residual solvent.

### Example 1. Preparation of (E)-N'-((3'-tert-butyl-5'-(2,4-dioxo-3,4-dihydropyrimidin-1(2H)-yl)-2'-methoxybiphenyl-4-yl)methylene)methanesulfonohydrazide (compound IB-L0-1.1).

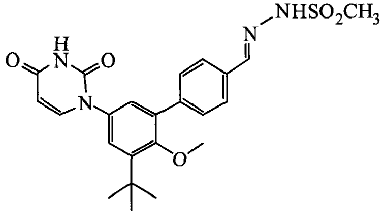

### Part A. Preparation of 2-tert-butyl-4-nitrophenol.

To a vigorously stirred solution of 2-tert-butylphenol (10g, 66.6mmol) in heptane (67ml) was added at a fast drip a solution of 70% nitric acid (4.25ml, 66.6mmol) diluted with water (4.25ml). The resulting dark red/brown mixture was stirred vigorously for 2h. The suspended solid was collected by filtration washed with hexane (300mL), water (200mL) and once again with hexane (200mL) to give a cocoa colored powder that was dried to constant mass (4.65g, 35.6%).

### Part B. Preparation of 2-bromo-6-tert-butyl-4-nitrophenol.

A solution of the product from **Part A** (1.0g, 5.12mmol) in glacial acetic acid (10.25mL) was treated portion wise with pyridine hydrobromide perbromide (1.80g, 5.63mmol) followed by stirring at room temperature for 2h. Additional pyridinium hydrobromide perbromide (3.80g) was added in two portions and after another 3h of stirring, the reaction was complete. The mixture was poured into ice water, and the mixture treated with a small amount of sodium sulfite. The resulting solid was filtered and dried under vacuum to give the title compound as a brown solid (1.40g, 100 %).

### Part C. Preparation of 1-bromo-3-tert-butyl-2-methoxy-5-nitrobenzene.

A solution of the product from **Part B** (1.40g, 5.11mmol) in 10:1 t-butylmethylether-methanol (25.5mL) was treated with 2.0M trimethylsilyldiazomethane in ether (5.1mL, 10.21mmol), followed by stirring at room temperature for 18h. The mixture was concentrated under vacuum to afford a yellow oil, which was purified by silica gel column chromatography eluting with EtOAc/hexanes to give the title compound as a yellow oil (1.36g, 92 %).

### Part D. Preparation of tert-butyl 3-bromo-5-tert-butyl-4-methoxyphenylcarbamate.

A solution of the product from **Part C** (960mg, 3.33mmol) in methanol (17mL) was treated with 5 % platinum on sulfided carbon (100mg), followed by hydrogenation under balloon pressure for 3h, and then filtered through celite and concentrated under vacuum to afford the 3-bromo-5-tert-butyl-4-methoxyaniline as a yellow oil (860mg, 3.33mmol, 100%). A solution of this material in THF (17mL) was treated with di-tert-butyl dicarbonate (800mg, 3.66mmol) followed by warming at reflux for 2h. Concentration under vacuum afforded a beige solid, which was purified by silica gel column chromatography eluting with EtOAc/hexanes. Solid was triturated with hexanes, collected by filtration, and dried under vacuum to give the title compound as a nearly white solid (890mg, 75 %).

### Part E. Preparation of methyl 5'-(tert-butoxycarbonylamino)-3'-tert-butyl-2'-methoxy biphenyl-4-carboxylate.

Toluene (2ml) and ethanol (2ml) were combined with the product from **Part E** (281mg, 0.78mmol), methyl 4-(4,4,5,5-tetramethyl-1,3,2-dioxaborolan-2-yl)benzoate (411mg, 1.57mmol) and 1M sodium carbonate (0.78ml, 0.78mmol) and de-gassed for 20min with N₂. Tetrakis(triphenyl-phosphine)palladium(0) (18mg, 0.016mmol) was added and de-gassing continued for 5-10min. Heated at 100°C in a sealed tube for 18h, cooled and concentrated under vacuum. Purification by silica gel column chromatography eluting with EtOAc/hexanes gave the title compound (182mg, 56%).

### Part F. Preparation of methyl 5'-amino-3'-tert-butyl-2'-methoxybiphenyl-4-carboxylate.

To a solution of the product from **Part E** (180mg, 0.43mmol) in CH₂Cl₂ (4ml) was added trifluoroacetic acid (2ml). Stirred for 30min and concentrated under vacuum. Dissolved in EtOAc and washed with 10% NaHCO₃ and brine. Dried over Na₂SO₄, filtered and concentrated under vacuum to give title compound (136mg, 100%).

### Part G. Preparation of 3'-tert-butyl-5'-(2,4-dioxo-3,4-dihydropyrimidin-1(2H)-yl)-2'-methoxy biphenyl-4-carboxylate.

To a solution of the product from **Part F** (120mg, 0.38mmol) in DMF (2.5ml) at-25°C was added drop wise, (E)-3-methoxyacryloyl isocyanate (1.34ml, 0.76mmol) keeping the temperature below-10°C until completion. The mixture was warmed to room temperature, stirred for 4h and poured into ether. Washed with water and brine. Dried over Na₂SO₄, filtered and concentrated under vacuum. Purification by silica gel column chromatography eluting with EtOAc/hexanes gave (E)-methyl-3'-tert-butyl-2'-methoxy-5'-(3-(3-methoxyacryloyl)ureido)biphenyl-4-carboxylate (105mg, 62%). Added ethanol (3ml), H₂O (3ml) and conc. H₂SO₄ (0.3ml) and heated at 100°C for 1h. Cooled, poured into H₂O and extracted with EtOAc. Dried over Na₂SO₄, filtered and concentrated under vacuum. Purification by silica gel column chromatography eluting with 2% CH₃OH/CHCl₃ gave the title compound (73mg, 79%).

### Part H. Preparation of 3'-tert-butyl-5'-(2,4-dioxo-3,4-dihydropyrimidin-1(2H)-yl)-2'-methoxy biphenyl-4-carbaldehyde.

A solution of the product from **Part G** (73mg, 0.18mmol) in dioxane (1ml) was treated with 0.5M LiOH (1ml, 0.36mmol) at room temperature for 1h, poured into 1N HCl and extracted with EtOAc. Dried over Na₂SO₄, filtered and concentrated under vacuum to give 3'-tert-butyl-5'-(2,4-dioxo-3,4-dihydropyrimidin-1(2H)-yl)-2'-methoxybiphenyl-4-carboxylic acid (69mg, 98%). Dissolved in thionyl chloride (2ml) and refluxed for 3h, cooled and concentrated under vacuum. Azeotroped twice with toluene to give 3'-tert-butyl-5'-(2,4-dioxo-3,4-dihydropyrimidin-1(2H)-yl)-2'-methoxybiphenyl-4-carbonyl chloride (72mg, 100%) which was dissolved in THF (1.7ml) and cooled to -78°C. 1M lithium tri-tert-butoxyaluminum hydride (THF) (0.19ml, 0.19mmol) was added drop wise and stirring was continued at-78°C for 2h. Quenched with 1N HCl (1ml) and warmed to room temperature. Added water and extracted with EtOAc. Washed with 10% NaHCO₃, dried over Na₂SO₄, filtered and concentrated under vacuum. Purification by silica gel column chromatography eluting with 1:1 EtOAc/hexanes gave the title compound (23mg, 35%).

### Part I. Preparation of (E)-N'-((3'-tert-butyl-5'-(2,4-dioxo-3,4-dihydropyrimidin-1(2H)-yl)-2'-methoxybiphenyl-4-yl)methylene)methanesulfonohydrazide.

A solution of the product from **Part H** (23mg, 0.061mmol) in CH₃OH (0.8ml) was treated with methanesulfonohydrazide (7.7mg, 0.07mmol) at room temperature for 1h, warmed to 35°C for 2h, cooled and concentrated under vacuum. Purification by silica gel column chromatography eluting with 5% CH₃OH/CHCl₃ gave title compound (14.8mg, 52%). 1H NMR (300 MHz CDCl3) ppm 1.44 (s, 9 H) 3.21 (s, 3 H) 3.32 (s, 3 H) 5.82 (d, *J*=8.09 Hz, 1 H) 7.14 - 7.24 (m, 1 H) 7.35 (d, *J*=8.09 Hz, 1 H) 7.61 (d, *J*=8.46 Hz, 2 H) 7.75 (d, *J*=8.46 Hz, 2 H) 7.79 (s, 1 H) 7.87 (s, 1 H) 8.21 (br s, 1 H). MS (ESI+) *m*/*z* 471 (M+H)+.

### Example 2. Preparation of (E)-N'-((3'-tert-butyl-5'-(2,4-dioxotetrahydropyrimidin-1(2H)-yl)-2'-methoxybiphenyl-4-yl)methylene)methanesulfonohydrazide (compound IA-L0-1.1).

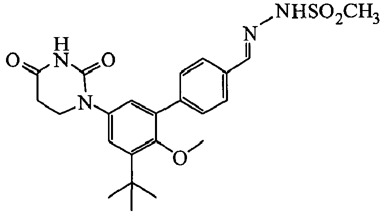

### Part A. Preparation of 2-tert-butyl-6-iodo-4-nitrophenol.

To the product from **Example 1**, **Part A** (4.5g, 23.05mmol) dissolved in MeOH (120ml) and water (30mL) was added iodine monochloride (1.155ml, 23.05mmol) drop wise over a period of 10min. The mixture was stirred for 2h and diluted into 1L of water and allowed to stand overnight. The solid material was collected by filtration and washed 3 x 50mL with water and dried under vacuum overnight to give a tan solid (7.14g, 96%).

### Part B. Preparation of 1-tert-butyl-3-iodo-2-methoxy-5-nitrobenzene.

To an ice bath cooled solution of the product from **Part A** (5.5g, 17.13mmol) in MTBE (15ml) in a 50mL pressure vessel was added 2.0M TMS diazomethane (12.85ml, 25.7mmol) followed by drop-wise addition of methanol (1.0mL) resulting in calm bubbling. The vessel was sealed and stirred at room temperature for 16h, cooled and the pressure was released. The solution was partitioned between EtOAc and water. The organic layer was washed with 1.0M HCl, saturated potassium carbonate solution, and saturated NaCl. The organic layer was dried over sodium sulfate, filtered and concentrated to give a red oil that was used without purification (5.4g, 84%).

### Part C. Preparation of 3-tert-butyl-5-iodo-4-methoxyaniline.

A mixture of the product from **Part B** (5.80g, 17.31mmol), ammonium chloride (1.389g, 26.0mmol), and iron (4.83g, 87mmol) in THF/MeOH/water (200mL total, 2/2/1) was refluxed for 2h, cooled and filtered through Celite. The filtrate was evaporated and the residue was partitioned between water and EtOAc. The organic layer was washed with saturated brine, dried with sodium sulfate, filtered and evaporated to give a brown oil (5.28g, 100% yield).

### Part D. Preparation of 1-(3-tert-butyl-5-iodo-4-methoxyphenyl)dihydropyrimidine-2,4(1H,3H)-dione.

The product from **Part C** (8.2g, 26.9mmol) was treated with acrylic acid (5.53ml, 81mmol) and stirred overnight to give an extremely viscous mixture. The mixture was treated with acetic acid (60mL) and urea (7.3g 120mmol), heated at 120°C for 24h, cooled and concentrated. The residue was azeotroped 3 x 100mL with toluene to give a brown/tan solid. The solid was suspended in a mixture of 50mL EtOAc and 100mL of saturated NaHCO₃ and stirred for thirty min to neutralize any remaining acetic acid. The solid was collected by filtration and washed repeatedly with 50mL portions of water and finally with 3:1 hexane/EtOAc (50mL) to give an off-white solid that was dried to constant mass (7.1g, 66%).

### Part E. Preparation of 3'-tert-butyl-5'-(2,4-dioxotetrahydropyrimidin-1(2H)-yl)-2'-methoxy biphenyl-4-carbaldehyde.

A mixture of the product from **Part D** (101mg, 0.25mmol), 4-formylphenylboronic acid (56.2mg, 0.38mmol), 1M sodium carbonate (0.25mL, 0.25mmol) and 1,1'-bis(diphenylphosphino) ferrocene-palladium(II)dichloride dichloromethane complex (10.2mg, 0.013mmol) in toluene/ethanol (2mL, 1/1) was purged with bubbling N₂ for 5min and microwaved at 100°C for 15min. Extracted with EtOAc and washed with brine. Dried over Na₂SO₄, filtered and concentrated under vacuum. Purification by silica gel column chromatography eluting with MeOH/CH₂Cl₂ (1% to 5%) gave the title compound (92mg, 97%).

### Part F. Preparation of (E)-N'-((3'-tert-butyl-5'-(2,4-dioxotetrahydropyrimidin-1(2H)-yl)-2'-methoxybiphenyl-4-yl)methylene)methanesulfonohydrazide.

A mixture of the product from **Part E** (90mg, 0.24mmol) and methanesulfonohydrazide (29mg, 0.26mmol) in methanol (4mL) was heated at 40°C for 2h. Evaporated and purified by silica gel column chromatography eluting with MeOH/CH₂Cl₂ (1% to 4%) to give the title compound (80mg, 72%). m.p. 209-211°C. ¹H NMR (300 MHz, DMSO-D6) δ 1.39 (s, 9 H) 2.70 (t, J=6.62 Hz, 2 H) 3.08 (s, 3 H) 3.24 (s, 3 H) 3.80 (t, J=6.62 Hz, 2 H) 7.17 (d, J=2.57 Hz, 1 H) 7.24 (d, J=2.94 Hz, 1 H) 7.59 (d, J=8.46 Hz, 2 H) 7.77 (d, J=8.46 Hz, 2 H) 8.04 (s, 1 H) 10.33 (s, 1 H) 11.10 (s, 1 H).

### Example 3. Preparation of N-(6-(3-tert-butyl-5-(2,4-dioxotetrahydropyrimidin-1(2H)-yl)-2-methoxyphenyl)naphthalen-2-yl)methanesulfonamide (compound IA-L0-2.9).

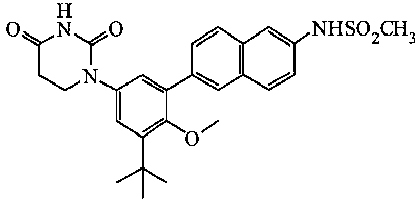

### Part A. Preparation of 6-bromo-2-naphthoic acid.

A solution of methyl 6-bromo-2-naphthoate (7.70g, 29.0mmol) in 2:1 THF:water (150mL) was treated with lithium hydroxide hydrate (2.44g, 58.1mmol) followed by stirring at room temperature for 48h. Concentrated under vacuum, diluted with water and cooled to 0°C. Acidified to pH3 with 4N HCl. Solids were collected by filtration, dissolved in toluene-EtOAc (ca. 2L) and washed with brine. Dried over Na₂SO₄, filtered and concentrated under vacuum. Brown solid was triturated with ether, collected by filtration, and dried under vacuum to give the title compound as a nearly white solid (5.07g, 70%).

### Part B. Preparation of 6-bromonaphthalen-2-amine.

A solution of the product **Part A** (5.07g, 20.19mmol) and triethylamine (4.22mL, 3.07g, 30.3mmol) in dry DMF (155mL) was treated with the diphenylphosphoroyl azide (6.55mL, 8.34g, 30.3mmol) followed by stirring at room temperature for 3h. The solution was then treated with water (20mL) followed by warming at 100°C for 1h. The solution was cooled and the flask fitted with a short-path distillation head and the DMF removed by distillation under high vacuum. The solid residue was dissolved in EtOAc and washed with saturated sodium bicarbonate solution. Filtered through celite and the filtrate was washed with water (3x) and then with brine. Dried over Na₂SO₄, filtered and concentrated under vacuum to give the title compound as a beige solid (4.48g, 100 %).

### Part C. Preparation of benzyl 6-bromonaphthalen-2-ylcarbamate.

A mixture of the product from **Part B** (1.79g, 8.06mmol) and saturated sodium bicarbonate solution (18mL) in acetone (40mL) at 0 °C was treated drop wise with benzyl chloroformate. The mixture was stirred at 0°C for 1h, and then allowed to gradually warm to room temperature over 18h. The mixture was diluted with EtOAc and water and the layers separated. The organic layer was extracted with water and washed with brine. Dried over Na₂SO₄, filtered and concentrated under vacuum. Purification by silica gel column chromatography eluting with EtOAc/hexanes gave the title compound as a pink solid (1.5g, 52%).

### Part D. Preparation of benzyl 6-(4,4,5,5-tetramethyl-1,3,2-dioxaborolan-2-yl)naphthalen-2-yl carbamate.

A resealable Schlenk tube containing a solution of the product from **Part C** (1.42g, 3.99mmol), bis(pinacolato)diboron (1.11g, 4.39mmol), and potassium acetate (1.17g, 11.96mmol) in DMF (28mL) was degassed by three freeze-thaw cycles. The solution was treated with 1,1'-bis(diphenyl phosphino)ferrocene palladium (II) chloride dichloromethane complex (98mg, 0.12mmol), followed by degassing by two additional freeze-thaw cycles. The Schlenk tube was then sealed and the mixture warmed at 80°C for 18h. Cooled and diluted with ethyl acetate and water. The mixture was treated with Darco G-60 and then filtered through celite. The filtrate was extracted with water (4x) and saturated sodium chloride solution. Dried over Na₂SO₄, filtered and concentrated under vacuum afforded a light brown oil. Purification by silica gel column chromatography eluting with EtOAc/hexane gave the title compound as a colorless oil (910mg, 57 %).

### Part E. Preparation of 2-tert-butyl-4-nitrophenol.

To a vigorously stirred solution of 2-tert-butylphenol (10g, 66.6mmol) in heptane (67ml) was added at a fast drip a solution of 70% nitric acid (4.25ml, 66.6mmol) diluted with water (4.25ml). The resulting dark red/brown mixture was stirred vigorously for 2h. The suspended solid was collected by filtration washed with hexane (300mL), water (200mL) and once again with hexane (200mL) to give a cocoa colored powder that was dried to constant mass (4.65g, 35.6%).

### Part F. Preparation of 2-bromo-6-tert-butyl-4-nitrophenol.

A solution of the product from **Part E** (1.0g, 5.12mmol) in glacial acetic acid (10.25mL) was treated portion wise with pyridine hydrobromide perbromide (1.80g, 5.63mmol) followed by stirring at room temperature for 2h. Additional pyridinium hydrobromide perbromide (3.6g) was added in two portions and after another 3h of stirring, the reaction was complete. The mixture was poured into ice water, and the mixture treated with a small amount of sodium sulfite. The resulting solid was filtered and dried under vacuum to give the title compound as a brown solid (1.40g, 100 %).

### Part G. Preparation of 1-bromo-3-tert-butyl-2-methoxy-5-nitrobenzene.

A solution of the product from **Part F** (1.40g, 5.11mmol) in 10:1 t-butylmethylether-methanol (25.5mL) was treated with 2.0M trimethylsilyldiazomethane in ether (5.1mL, 10.21mmol), followed by stirring at room temperature for 18h. The mixture was concentrated under vacuum to afford a yellow oil, which was purified by silica gel column chromatography eluting with EtOAc/hexanes to give the title compound as a yellow oil (1.36g, 92 %).

### Part H. Preparation of tert-butyl 3-bromo-5-tert-butyl-4-methoxyphenylcarbamate.

A solution of the product from **Part G** (960mg, 3.33mmol) in methanol (17mL) was treated with 5 % platinum on sulfided carbon (100mg), followed by hydrogenation under balloon pressure for 3h, and then filtered through celite and concentrated under vacuum to afford the 3-bromo-5-tert-butyl-4-methoxyaniline as a yellow oil (860mg, 3.33mmol, 100%). A solution of this material in THF (17mL) was treated with di-tert-butyl dicarbonate (800mg, 3.66mmol) followed by warming at reflux for 2h. Concentration under vacuum afforded a beige solid, which was purified by silica gel column chromatography eluting with EtOAc/hexanes. Solid was triturated with hexanes, collected by filtration, and dried under vacuum to give the title compound as a nearly white solid (890mg, 75 %).

### Part I. Preparation of benzyl 6-(3-tert-butyl-5-(tert-butylcarbamoyl)-2-methoxyphenyl) naphthalen-2-yl carbamate.

Toluene (928ul) and EtOH (928ul) were combined with the product from **Part H** (133mg, 0.37mmol), the product from **Part D** (299mg, 0.74mmol) and 1M sodium carbonate (371ul, 0.37mmol) and de-gassed for 20min with nitrogen. Tetrakis(triphenylphosphine)palladium(0) (8.6mg, 7.4umol) was added and de-gassing continued 5-10min. Heated at 85-90°C for 18h, cooled and concentrated under vacuum. Purification by silica gel column chromatography eluting with EtOAc/hexanes gave the title compound (102mg, 49%).

### Part J. Preparation of benzyl 6-(3-tert-butyl-5-(2,4-dioxotetrahydropyrimidin-1(2H)-yl)-2-methoxyphenyl)naphthalen-2-ylcarbamate.

A solution of the product from **Part I** (100mg, 0.18mmol) in CH₂Cl₂ (1.0ml) was treated with trifluoroacetic acid (0.5ml, 6.5mmol) at room temperature for 1h. Concentrated under vacuum. Dissolved in ethyl acetate, washed with 10% NaHCO₃, brine. Dried over Na₂SO₄, filtered and concentrated under vacuum. Dissolved in toluene (1.0ml) and added Et₃N (25ul, 0.18mmol) and acrylic acid (13ul, 0.19mmol) and the mixture was refluxed for 16h. Concentrated under vacuum. Dissolved in acetic acid (1.0ml, 17.5mmol) and added urea (11.9mg, 0.20mmol) and refluxed for 72h. Cooled and poured into ice water, extracted three times with CHCl₃, combined extracts, dried over Na₂SO₄, filtered and concentrated under vacuum. Purification by silica gel column chromatography eluting with EtOAc/hexanes gave title compound (57.5mg, 58%).

### Part K. Preparation of N-(6-(3-tert-butyl-5-(2,4-dioxotetrahydropyrimidin-1(2H)-yl)-2-methoxyphenyl)naphthalen-2-yl)methanesulfonamide.

Combined the product from **Part J** (56mg, 0.10mmol) and EtOAc (1.0ml) and added 10% palladium on carbon (10mg) Stirred under a balloon of H₂ gas for 16h. Filtered through Celite and concentrated under vacuum. Dissolved in CH₂Cl₂ (1.0ml), added Et₃N (16ul, 0.115mmol) and methanesulfonyl chloride (8.7ul, 0.112mmol) and stirred at room temperature for 30min. Concentrated under vacuum and purification by silica gel column chromatography eluting with EtOAc/hexanes gave the title compound (10mg, 20%). ¹H NMR (300 MHz, DMSO-*d6*) δ 1.34 - 1.48 (m, 9 H) 2.71 (t, *J*=6.62 Hz, 2 H) 3.08 (s, 3 H) 3.21 (s, 3 H) 3.82 (t, *J*=6.62 Hz, 2 H) 7.26 (s, 2 H) 7.41 (dd, *J*=8.82, 1.84 Hz, 1 H) 7.59 - 7.76 (m, 2 H) 7.89 - 8.04 (m, 3 H) 10.03 (s, 1 H) 10.34 (s, 1 H); MS (ESI+) *m*/*z* 496 (M+H)⁺; (ESI-) *m*/*z* 494 (M-H)⁻.

### Example 4A. Preparation of N-(6-(3-tert-butyl-5-(2,4-dioxo-3,4-dihydropyrimidin-1(2H)-yl)-2-methoxyphenyl)naphthalen-2-yl)methanesulfonamide (compound IB-L0-2.3).

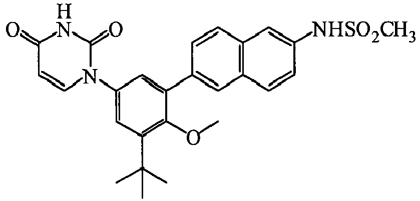

### Part A. Preparation of N-(6-bromonaphthalen-2-yl)methanesulfonamide.

A solution of the product from **Example 3**, **Part B** (4.48g, 20.17mmol) in pyridine (100mL) was treated drop wise with methanesulfonyl chloride (1.97mL, 2.89 g, 25.2mmol) followed by stirring at room temperature for 1h. Diluted with toluene and concentrated under vacuum twice. The residue was extracted with EtOAc and washed with water, 1M citric acid and brine. Treated with Darco G-60, dried over Na₂SO₄, filtered through celite and concentrated under vacuum. Solid was triturated with ether-hexane, collected by filtration and dried under vacuum to give the title compound as a faint pink solid (3.32g, 55 %).

### Part B. Preparation of N-(6-(4,4,5,5-tetramethyl-1,3,2-dioxaborolan-2-yl)naphthalen-2-yl) methanesulfonamide.

A mixture of the product from **Part A** (1.00g, 3.33mmol), bis(pincolato)diboron (1.27g, 5.00mmol), potassium acetate (0.98 g, 9.99mmol) and Combiphos Pd6 (84mg, 0.17mmol) in toluene (22mL) was heated at reflux for 3h. Cooled and diluted with ethyl acetate and water. The mixture was treated with Darco G-60 and filtered through celite. The filtrate was washed with water and brine. Dried over Na₂SO₄, filtered and concentrated under vacuum. Oil was dissolved in ether and precipitated by addition of hexanes. The product was collected by filtration and washed with hexanes. Evaporation of the filtrate and purification by silica gel column chromatography eluting with EtOAc/hexanes. The title compound from crystallization and chromatography was obtained as a white solid (927mg, 80%).

### Part C. Preparation of tert-butyl 3-tert-butyl-4-methoxy-5-(6-(methylsulfonamido) naphthalen-2-yl)phenylcarbamate.

Combined the product from **Example 3**, **Part H** (87mg, 0.243mmol), the product from **Part B** (169mg, 0.486mmol), toluene (1.0ml), ethanol (1.02ml) and sodium carbonate (0.243ml, 0.243mmol) in a sealed tube and de-gassed with N₂ gas for 20min. Tetrakis(triphenylphosphine)palladium(0) (5.61mg, 4.86µmol) was added and de-gassing was continued another 5-10 min. Heated at 90-95°C for 16h. Cooled and concentrated under vacuum. Purification by silica gel column chromatography eluting with EtOAc/hexanes gave the title compound (92.2mg, 76 %).

### Part D. Preparation of N-(6-(3-tert-butyl-5-(2,4-dioxo-3,4-dihydropyrimidin-1(2H)-yl)-2-methoxyphenyl)naphthalen-2-yl)methanesulfonamide.

A solution of the product from **Part C** (90mg, 0.180mmol) in CH₂Cl₂ (2.0ml) was treated with trifluoroacetic acid (1.0ml, 12.98mmol) at room temperature for 1h. Concentrated under vacuum, dissolved residue in EtOAc, washed with 10% NaHCO₃, and brine. Dried over Na₂SO₄, filtered and concentrated under vacuum. Dissolved in DMF (1.4ml) and cooled to -25°C and added (E)-3-methoxy-acryloyl isocyanate (0.633ml, 0.361mmol) drop wise while maintaining the temperature below -10°C. Warmed to room temperature and stirred for 2h. Poured into ether, washed with water, and brine. Dried over Na₂SO₄, filtered and concentrated under vacuum. Added a mixture of H₂SO₄ (0.1ml, 1.876mmol), water (1.0ml) and EtOH (1.0ml) and stirred at 100°C 16h. Cooled and concentrated under vacuum. Poured into water, extracted with EtOAc, combined extracts and washed with brine. Dried over Na₂SO₄, filtered and concentrated under vacuum. Purification by silica gel column chromatography eluting with MeOH/CHCl₃ gave the title compound (53mg, 59%). ¹H NMR (300 MHz DMSO-*d6*) δ 1.42 (s, 9 H) 3.08 (s, 3 H) 3.25 (s, 3 H) 5.65 (d, *J*=7.72 Hz, 1 H) 7.34 (dd, *J*=15.81, 2.57 Hz, 2 H) 7.42 (dd, *J*=8.82, 1.84 Hz, 1 H) 7.65 - 7.76 (m, 2 H) 7.80 (d, *J*=8.09 Hz, 1 H) 7.96 (t, *J*= 8.27 Hz, 2 H) 8.02 (s, 1 H) 10.04 (s, 1 H) 11.41 (s, 1 H); MS (ESI+) *m*/*z* 494 (M+H)⁺; (ESI-) *m*/*z* 492 (M-H)⁻.

### Example 4B. Preparation of N-(6-(3-tert-butyl-5-(2,4-dioxo-3,4-dihydropyrimidin-1(2H)-yl)-2-methoxyphenyl)naphthalen-2-yl)methanesulfonamide (compound IB-L0-2.3).

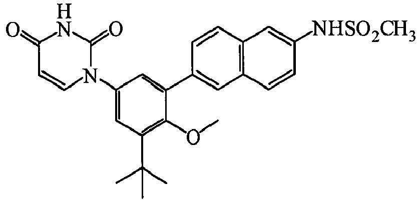

### Part A. Preparation of 2-tert-butyl-6-iodo-4-nitrophenol.

To the product from **Example 3**, **Part E** (4.5g, 23.05mmol) dissolved in MeOH (120ml) and water (30mL) was added iodine monochloride (1.155ml, 23.05mmol) drop wise over a period of 10min. The mixture was stirred for 2h and diluted into 1L of water and allowed to stand overnight. The solid material was collected by filtration and washed 3 x 50mL with water and dried under vacuum overnight to give a tan solid (7.14g, 96%).

### Part B. Preparation of 1-tert-butyl-3-iodo-2-methoxy-5-nitrobenzene.

To an ice bath cooled solution of the product from **Part A** (5.5g, 17.13mmol) in MTBE (15ml) in a 50mL pressure vessel was added 2.0M trimethylsilyl diazomethane (12.85ml, 25.7mmol) followed by drop-wise addition of methanol (1.0mL) resulting in calm bubbling. The vessel was sealed and stirred at room temperature for 16h, cooled and the pressure was released. The solution was partitioned between EtOAc and water. The organic layer was washed with 1.0M HCl, saturated potassium carbonate solution, and saturated NaCl. The organic layer was dried over sodium sulfate, filtered and concentrated to give a red oil that was used without purification (5.4g, 84%).

### Part C. Preparation of 3-tert-butyl-5-iodo-4-methoxyaniline.

A mixture of the product from **Part B** (5.80g, 17.31mmol), ammonium chloride (1.389g, 26.0mmol), and iron (4.83g, 87mmol) in THF/MeOH/water (200mL total, 2/2/1) was refluxed for 2h, cooled and filtered through Celite. The filtrate was evaporated and the residue was partitioned between water and EtOAc. The organic layer was washed with saturated brine, dried with sodium sulfate, filtered and evaporated to give a brown oil (5.28g, 100% yield).

### Part D. Preparation of (E)-N-(3-tert-butyl-5-iodo-4-methoxyphenylcarbamoyl)-3-methoxy acrylamide.

To a solution of the product from **Part C** (3.05g, 10mmol) in DMF (50ml) at -20 °C under N₂ was added at a fast drip a 0.4M solution in benzene of (E)-3-methoxyacryloyl isocyanate (50.0ml, 20.00mmol, prepared by the method of Santana et al., J. Heterocyclic. Chem. 36:293 (1999). The solution was stirred for 15min at -20 °C, warmed to room temperature for 45min and diluted with EtOAc. The organic was washed with water and brine. Dried over Na₂SO₄, filtered and concentrated to a brown solid. The residue was triturated in Et₂O/hexane to give a fine powder that was collected by filtration and dried under vacuum to give the title compound as a tan powder (2.46g, 57%).

### Part E. Preparation of 1-(3-tert-butyl-5-iodo-4-methoxyphenyl)dihydropyrimidine-2,4(1H,3H)-dione.

To a suspension of the product from **Part D** (2.46g, 5.69mmol) in ethanol (50ml) was added a solution of 5.5mL of H₂SO₄ in 50mL water and the mixture was heated at 110°C for 2.5h to give a clear solution. Cooled and diluted with 50mL of water while stirring to give an off-white solid that was collected by filtration, washed with water and dried under vacuum to give the title compound (2.06g, 90%).

### Part F. Preparation of N-(6-(3-tert-butyl-5-(2,4-dioxo-3,4-dihydropyrimidin-1(2H)-yl)-2-methoxyphenyl)naphthalen-2-yl)methanesulfonamide.

In a microwave tube, the product from **Part E** (104mg, 0.26mmol), the product from **Example 4A**, **Part B** (108mg, 0.31mmol), and 1.0M sodium carbonate solution (312µL, 0.31mmol) in 1:1 ethanol-toluene (1.7mL) was degassed by nitrogen sparge for 15min. 1,1'-Bis(diphenylphosphino) ferrocene palladium (II) chloride dichloromethane complex (9mg, 0.011mmol) was added, and degassing was continued for another 5min. The tube was sealed and heated in the microwave at 100°C for 1h. Diluted with dichloromethane and washed with 1M citric acid solution and brine. The organic layer was then stirred with (3-mercaptopropyl) silica gel for 1h. Filtered through celite and concentrated under vacuum. Triturated with ether, methanol, and then again with ether to give the title compound as a nearly white solid (32mg, 25 %). ¹H NMR (300 MHz, DMSO-*d₆*): δ 11.41 (d, J=1.84 Hz, 1 H) 10.04 (s, 1 H) 8.03 (s, 1 H) 7.96 (t, J=8.09 Hz, 2 H) 7.80 (d, J=8.09 Hz, 1 H) 7.63 - 7.79 (m, 2 H) 7.35 - 7.45 (m, 1 H) 7.37 (d, J=2.57 Hz, 1 H) 7.32 (d, J=2.57 Hz, 1 H) 5.65 (dd, J=8.09, 2.21 Hz, 1 H) 3.25 (s, 3 H) 3.09 (s, 3 H) 1.43 (s, 9 H). MS (+ESI) m/z (rel abundance): 494 (100, M+H), 511 (90, M+NH4), 987 (20, 2M+H), 1009 (8, 2M+Na).

### Example 5. Preparation of N-(6-(3-tert-butyl-5-(2,4-dioxo-3,4-dihydropyrimidin-1-(2H)-yl)-2-methoxyphenyl)quinolin-2-yl)methanesulfonamide (compound IB-L0-2.5).

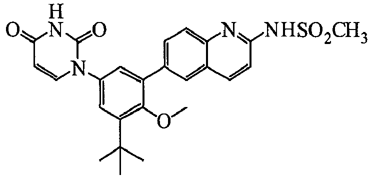

### Part A. Preparation of (E)-N-(4-bromophenyl)-3-methoxyacrylamide.

Combined 4-bromoaniline (285mg, 1.659mmol), CH₂Cl₂ (2.0ml) and pyridine (0.25ml, 3.09mmol) and slowly added (E)-3-methoxyacryloyl chloride (200mg, 1.659mmol) and stirred at room temperature for 2h. The resulting yellow solid was filtered off and washed with water. The solid was dried under vacuum to give the title compound (406mg, 96 %).

### Part B. Preparation of 6-Bromoquinolin-2(1H)-one.

The product from **Part A** (395mg, 1.542mmol) was added in portions to H₂SO₄ (4.5ml). Stirred for 3h at room temperature, poured onto crushed ice. Solid filtered, washed with water and dried under vacuum to give the title compound (203mg, 59 %).

### Part C. Preparation of 6-bromo-2-chloroquinoline.

To phosphorus oxychloride (2.5ml, 26.8mmol) was added, in portions, the product from **Part B** (200mg, 0.893mmol). Refluxed for 1h, cooled to room temperature and poured onto crushed ice. Extracted with CHCl₃, extracts combined, dried overmgSO₄, filtered and concentrated under vacuum to give the title compound (173mg, 80%).

### Part D. Preparation of 6-bromo-2-aminoquinoline.

The product from **Part C** (173mg, 0.713mmol), acetamide (843mg, 14.27mmol) and potassium carbonate (493mg, 3.57mmol) were combined and heated at 200 °C for 2h. Cooled to room temperature, whereupon it solidified. Dissolved in a mixture of CHCl₃ and water. Aqueous layer was extracted twice more with CHCl₃, extracts were combined, washed with brine, dried over Na₂SO₄, filtered and concentrated under vacuum. Purification by silica gel column chromatography eluting with MeOH/CHCl₃ gave title compound (92mg, 58 %).

### Part E. Preparation of N-(6-bromoquinolin-2-yl)-N-(methylsulfonyl)methanesulfonamide.

Combined the product from **Part D** (90mg, 0.403 mmol) and CH₂Cl₂ (2.0ml) and added triethylamine (0.062ml, 0.444mmol) and methanesulfonyl chloride (0.035ml, 0.444mmol). Stirred at room temperature 16h. Added triethylamine (0.062ml, 0.444mmol) and methanesulfonyl chloride (0.035ml, 0.444mmol) and stirred at room temperature for 1h. Diluted with EtOAc, washed with 10% citric acid, 10% NaHCO₃ and brine. Dried over Na₂SO₄, filtered and concentrated under vacuum. Dissolved in EtOAc and poured into excess hexane. Solid collected by filtration to give the title compound (94mg, 61%).

### Part F. Preparation ofN-(methylsulfonyl)-N-(6-(4,4,5,5-tetramethyl-1,3,2-dioxaborolan-2-yl) quinolin-2-yl)methanesulfonamide.

Combined the product from **Part E** (94mg, 0.248mmol), bis(pinacolato)diboron (94mg, 0.372mmol), potassium acetate (73.0mg, 0.744mmol), Combi-Phos^{®}PD6 (6.22mg, 0.012mmol) and toluene (1.5ml) and refluxed 18h. Cooled to room temperature, diluted with EtOAc and water, filtered through Celite, separated the phases, washed the organic phase with brine. Dried over Na₂SO₄, filtered and concentrated under vacuum. Purification by silica gel column chromatography eluting with EtOAc/hexanes gave title compound (67mg, 63%).

### Part G. Preparation of N-(6-(3-tert-butyl-5-(2,4-dioxo-3,4-dihydropyrimidin-1-(2H)-yl)-2-methoxyphenyl)quinolin-2-yl)methanesulfonamide.

Combined in a microwave tube the product from **Example 4B**, **Part E** (27mg, 0.067mmol), the product from **Part F** (37.4mg, 0.088mmol), ethanol (1.0ml), toluene (1.0ml) and 1M sodium carbonate (0.067ml, 0.067mmol) and the solution was degassed using N₂ gas for 20min. Tetrakis- (triphenyl-phosphine)palladium(0) (1.559mg, 1.349µmol) was added and the solution was degassed an additional 5min. The tube was sealed and heated in the microwave at 100°C for 45min. Cooled solution diluted with 1:1 EtOAc:water and filtered through Celite. Aqueous layer was extracted twice more with EtOAc, combined organic extracts and washed with brine. Dried over Na₂SO₄, filtered and concentrated under vacuum. Purification by silica gel column chromatography eluting with MeOH/CHCl₃ gave title compound (13.7mg, 41%). ¹H NMR (300 MHz, CDCl₃) δ 1.45 (s, 9 H) 3.18 (s, 3 H) 3.30 (s, 3 H) 5.83 (dd, *J*=7.91, 2.02 Hz, 1 H) 6.99 (d, *J*= 8.82 Hz, 1 H) 7.21 (d, *J*=2.57 Hz, 1 H) 7.36 (d, *J*=7.72 Hz, 1 H) 7.52 (d, *J*=8.46 Hz, 1 H) 7.82 - 7.91 (m, 2 H) 7.98 (d, *J*=9.19 Hz, 1 H) 8.29 (s, 1 H); MS (ESI+) *m*/*z* 495 (M+H)⁺; (ESI-) *m*/*z* 493 (M-H)⁻.

### Example 6. Preparation of (E)-N'-(5-(3-tert-butyl-5-(2,4-dioxo-3,4-dihydropyrimidin-1(2H)-yl)-2-methoxyphenyl)-2,3-dihydro-1H-inden-1-ylidene)methanesulfonohydrazide (compound IB-L0-2.4).

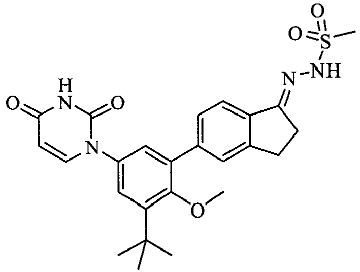

### Part A. Preparation of 5-(4,4,5,5-tetramethyl-1,3,2-dioxaborolan-2-yl)-2,3-dihydro-1H-inden-1-one.

A mixture of 5-bromo-2,3-dihydro-1H-inden-1-one (2.50g, 11.85mmol), bis(pinacolato) diboron (3.61g, 14.21mmol), potassium acetate (3.49g, 35.5mmol) and Combiphos Pd6 (178mg, 0.36mmol) in toluene (60mL) was heated at reflux for 8h. Cooled, diluted with EtOAc and extracted with water (2 x) and washed with brine. Dried over Na₂SO₄ and stirred for 1h with (3-mercaptopropyl) silica gel. Filtered and concentrated under vacuum to afford a yellow solid. Purification by silica gel column chromatography eluting with EtOAc/hexanes gave a yellow solid. Triturated with cold hexanes, filtered and dried under vacuum to give the title compound as a fine nearly white solid (1.99g, 65%). A second crop of crystals (140mg) was obtained from the mother liquors, bringing the yield to 70%.

### Part B. Preparation of 1-(3-tert-butyl-4-methoxy-5-(1-oxo-2,3-dihydro-1H-inden-5-yl)phenyl) pyrimidine-2,4(1H,3H)-dione.

In a microwave tube, a suspension of the product from **Example 4B**, **Part E** (130mg, 0.33mmol), the product from **Part A** (101mg, 0.39mmol), and 1.0M sodium carbonate solution (390µL, 0.39mmol) in 1:1 ethanol-toluene (1.20mL) was degassed by nitrogen sparge for 15min. The mixture was treated with 1,1'-bis(diphenylphosphino)ferrocene palladium (II) chloride dichloromethane complex (13mg, 0.016mmol) and degassing was continued for another 5min and heated at 100°C in the microwave for 1h. Cooled, diluted with EtOAc and extracted with 1M citric acid solution and brine. The organic layer was then stirred with (3-mercaptopropyl) silica gel for 1h. Filtered and concentrated under vacuum. Purification by silica gel column chromatography eluting with EtOAc/hexanes gave the title compound as a white solid (80mg, 61 %).

### Part C. Preparation of (E)-N'-(5-(3-tert-butyl-5-(2,4-dioxo-3,4-dihydropyrimidin-1(2H)-yl)-2-methoxyphenyl)-2,3-dihydro-1H-inden-1-ylidene)methanesulfonohydrazide.

A suspension of the product from **Part B** (77mg, 0.19mmol) and methanesulfonylhydrazide (22mg, 0.20mmol) in 3:1 THF:MeOH (1.9mL) was warmed at 60°C for 24h. The mixture was concentrated under vacuum and the residue was purified by silica gel column chromatography eluting with EtOAc/hexanes to give the title compound as a white solid (62mg, 66 %). ¹H NMR (300 MHz, DMSO-*d₆*): δ 11.40 (d, J=1.84 Hz, 1 H) 9.94 (s, 1 H) 7.76 (dd, J=13.97, 8.09 Hz, 2 H) 7.52 - 7.59 (m, 1 H) 7.51 (d, J=8.46 Hz, 1 H) 7.11 - 7.40 (m, 2 H) 3.28 (s, 3 H) 2.96 - 3.19 (m, 5 H), 2.85 (m, 2 H), 1.40 (s, 9 H). MS (+ESI) m/z (rel abundance): 497 (100, M+H), 1015 (5, 2M+Na).

### Example 7. Preparation of N-(2-(3-tert-butyl-5-(2,4-dioxo-3,4-dihydropyrimidin-1(2H)-yl)-2-methoxyphenyl)benzo[d]oxazol-5-yl)methanesulfonamide (compound IB-L0-2.6).

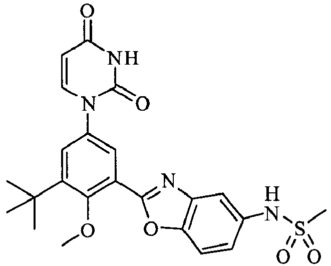

### Part A. Preparation of methyl 3-tert-butyl-2-hydroxy-5-nitrobenzoate.

Methyl 3,5-di-tert-butyl-2-hydroxybenzoate (28.66g, 108.4mmol) was dissolved with stirring in 430mL glacial acetic acid and the resulting mixture was treated drop wise with fuming nitric acid (90%, 179.26mL). When the addition was complete, the resulting mixture was stirred for 2.5h. The reaction mixture was poured into a 2.0L of crushed ice and allowed to stand 30min. Afterwards, 1.0L of water was added and the ice water mixture was allowed to melt. The mixture was then filtered, washed with water and dried to provide the title compound (24.57g, 89%).

### Part B. Preparation of methyl 3-tert-butyl-2-methoxy-5-nitrobenzoate.

Methyl 3-tert-butyl-2-hydroxy-5-nitrobenzoate (11.41g, 45.0mmol), potassium carbonate (9.34g, 67.6mmol), acetone (200mL), and dimethyl sulfate (6.46g, 67.6mmol) were added together. The resultant mixture was then heated to reflux for 16h. The mixture was then filtered and the solid was washed with ethyl acetate. The resulting organic liquid was then concentrated under vacuum to an oil and redissolved in ethyl acetate (600mL). The organic solution was then washed with water, dried, filtered and concentrated under vacuum to an oil that was then subjected to purification via column chromatography (gradient of 5% to 40% EtOAc/Hexanes) to yield the title compound as an oil (10.42, 87%).

### Part C. Preparation of methyl 5-amino-3-tert-butyl-2-methoxybenzoate.

Methyl 3-tert-butyl-2-methoxy-5-nitrobenzoate (10.42g, 39.0mmol), iron powder (325mesh, 10.89g, 195mmol), ammonium chloride (3.13g, 58.5mmol), water (30mL), and methanol (150mL) were added together. The resultant mixture was then refluxed for 1h. The mixture was then cooled to room temperature, filtered through celite, and the celite washed with methanol. The filtrate was then concentrated under vacuum and dissolved in ethyl acetate (600mL). The resultant solution was then washed with water and brine. The organic extract was then dried, filtered and concentrated under vacuum to yield the title compound as an oil (9.25g, 100%).

### Part D. Preparation of (E)-methyl 3-tert-butyl-2-methoxy-5-(3-(3-methoxyacryloyl)ureido) benzoate.

The product obtained as described in **Part C** (2.0g, 8.43mmol) was dissolved in 30mL of N,N-dimethylacetamide and cooled to -25°C. A 0.5Molar solution of E-3-methoxyacryloyl isocyanate in benzene (21.9mL, 10.96mmol) was added drop wise and the resulting solution was stirred at ambient temperature for 4h, and then poured into water. The product was extracted into dichloromethane, washed with brine, dried over sodium sulfate, filtered and evaporated under vacuum to give 100% yield.

### Part E. Preparation of methyl 3-tert-butyl-5-(2,4-dioxo-3,4-dihydropyrimidin-1(2H)-yl)-2-methoxybenzoate.

The product from **Part D** (3.1g, 8.51mmol)was dissolved in ethanol (60mL). Sulfuric acid (6mL) was added to water (60mL) then this solution was added in one portion to the ethanol. The heterogeneous mixture was heated at 100°C for 3h. The ethanol was removed under vacuum, and then the aqueous solution was extracted with dichloromethane and evaporated to dryness. This residue was purified by flash chromatography, eluting with 1% methanol/dichloromethane to yield 1.23g (44%).

### Part F. Preparation of 3-tert-butyl-5-(2,4-dioxo-3,4-dihydropyrimidin-1(2H)-yl)-2-methoxybenzoic acid.

The product from **Part E** (1.23g, 3.7mmol) was taken up in ethanol (5mL) and 1Molar sodium hydroxide solution (10mL) and stirred at ambient temperature for 18h. The solution was diluted with 1M HCl and the resulting solid was filtered and dried to give 0.945 g (80%).

### Part G. Preparation of 3-tert-butyl-5-(2,4-dioxo-3,4-dihydropyrimidin-1(2H)-yl)-2-methoxy benzaldehyde.

The product from **Part F** (0.945g, 2.97mmol) was taken up in thionyl chloride (4.5mL) and the mixture was heated at 80°C for 40min. After evaporation to dryness, the acid chloride was dissolved in dry THF (8mL) and cooled to -78°C. A 1Molar solution of lithium tri-tert-butoxyaluminum hydride in THF (3.0mL, 3.0mmol) was added drop wise. After 45min the cold reaction was quenched with 1M HCl (5mL), extracted into ethyl acetate, and purified by flash column, eluting with dichloromethane followed by 1% methanol/dichloromethane to give 0.635 g (71%).

### Part H. Preparation of 1-(3-tert-butyl-4-methoxy-5-(5-nitrobenzo[d]oxazol-2-yl)phenyl) pyrimidine-2,4(1 H,3H)-dione.

The product from **Part G** (400mg, 1.323mmol), 2-amino-4-nitrophenol (204mg, 1.323mmol), Charcoal (Darco KB, 191mg, 15.88mmol) and toluene (50mL) were added to a flask and the mixture was heated to 120°C, and stirred open to the air for 48h. Filtered through Celite and concentrated under vacuum. Purification by silica gel column chromatography eluting with CH₂Cl₂/MeOH gave the title compound (300mg, 52%).

### Part I. Preparation of N-(2-(3-tert-butyl-5-(2,4-dioxo-3,4-dihydropyrimidin-1(2H)-yl)-2-methoxyphenyl)benzo[d]oxazol-5-yl)methanesulfonamide.

To the product from **Part H** (300mg, 0.687mmol), iron (192mg, 3.44mmol), and ammonium chloride (55mg, 1.031mmol) was added to a mixture of THF (15mL), EtOH (15mL) and water (4.5mL). The resultant solution was heated to 90°C for 45min, and cooled. Filtered through Celit, washed with ethanol, and concentrated under vacuum. The solid was dissolved in ethyl acetate, and washed with water. Dried over Na₂SO₄, filtered and concentrated under vacuum. Purification by silica gel column chromatography eluting with CH₂Cl₂/MeOH provided the aniline. The solid (75mg, 0.185mmol) was dissolved in CH₂Cl₂ (5mL), and pyridine (0.045mL, 0.554mmol) and methanesulfonyl chloride (0.025mL, 0.323mmol) were added and stirred at room temperature for 16h. CH₂Cl₂ was added followed by washing with a 1N HCl. Dried over Na₂SO₄, filtered and concentrated under vacuum. Purification by silica gel column chromatography eluting with CH₂Cl₂/MeOH provided the title compound as a solid (9.8mg, 3%, two steps). 1H NMR (300MHz, DMSO-d6): δ 11.46 (s,1H), 9.85 (s,1H), 7.91 (d, J=2.2Hz, 1H), 7.81 (dd, J=9.9,8.8Hz, 2H), 7.68 (d, J=2.2Hz, 1H), 7.56 (d, J=2.6Hz, 1H), 7.33 (dd, J=8.8,1.8Hz, 1H), 5.68 (d, J=7.7Hz, 1H), 3.64 (s, 3H), 3.00 (s, 3H), 1.42 (s, 9H). MS: m/z 485 (M+H)+.

### Example 8. Preparation of 1-(3-tert-butyl-4-methoxy-5-(6-nitrobenzo[d]oxazol-2-yl)phenyl) dihydropyrimidine-2,4(1H,3H)-dione (compound IA-L0-2.6).

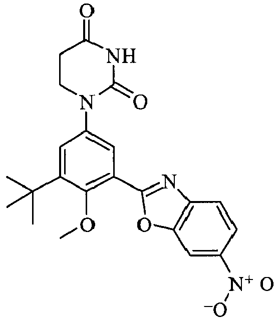

### Part A. Preparation of 3-(3-tert-butyl-4-methoxy-5-(methoxycarbonyl)phenylamino) propanoic acid.

The product from **Example 7**, **Part C** (16.44g, 69.3mmol) was dissolved in toluene (200mL). This mixture was heated to reflux and acrylic acid added over time (1mL of acrylic acid added every 3h, 5.23mL total, 76.2mmol). The mixture was then refluxed for 24h. The mixture was then cooled and concentrated under vacuum to dryness to yield an oil as the crude title compound that was used directly in the next reaction.

### Part B. Preparation of methyl 3-tert-butyl-5-(2,4-dioxotetrahydropyrimidin-1(2H)-yl)-2-methoxybenzoate.

The product from **Part A** (21.43g, 69.3mmol), urea (10.4g, 173mmol) and acetic acid (glacial, 200mL) were added together. The mixture was then heated to 120°C for 18.5h followed by concentration under vacuum to dryness to an oil. To this oil was added methanol (13mL), and ethyl acetate (350mL). The resultant mixture was allowed to stand for 24-48h whereby a precipitate formed. The resulting solid was filtered off and washed with a small amount of methanol (10mL) and then air dried to yield the title compound as a solid (15.26g, 66%).

### Part C. Preparation of 3-tert-butyl-5-(2,4-dioxotetrahydropyrimidin-1(2H)-yl)-2-methoxy benzoic acid.

The product from **Part B** (4.52g, 13.52mmol), methanol (70mL), and tetrahydrofuran (70mL) were added together. The mixture was then stirred vigorously until a homogenous solution resulted. Once homogenous, a solution of aqueous sodium hydroxide (1.0M, 68mL) was added. The mixture was then stirred for 12h, the mixture was then concentrated under vacuum to remove the organic solvent, followed by the addition of aqueous hydrochloric acid (1.0M, 80mL) that resulted in solid formation. The mixture was then concentrated under vacuum. To this material was added hydrochloric acid (12M, 100mL) and the resultant material heated to 100°C for 1.5h. The reaction was then cooled and water added. The resulting solid was filtered, washed with water, and dried to yield the title compound as a solid (3.55g, 82%).

### Part D. Preparation of 3-tert-butyl-5-(2,4-dioxotetrahydropyrimidin-1(2H)-yl)-2-methoxy benzaldehyde.

The product obtained in **Part C** (4.07g, 12.71mmol) and thionyl chloride (40.82mL, 559mmol) were combined and the mixture was refluxed for 2h, followed by concentration under vacuum to provide a light yellow colored solid product. The solid was dissolved in tetrahydrofuran (125mL), the solution cooled to -78°C and LiAl(O*t*Bu)₃ (1M, 14mL) was added slowly over 10min while maintaining the temperature at -78°C. The mixture was stirred at 78°C for 2h. The reaction was quenched with hydrochloric acid (aq., 1M, 25mL) at -78°C. The mixture was warmed to room temperature and ethyl acetate was added. The layers were separated and the aqueous layer was washed with ethyl acetate. The organic extracts were combined and washed with half saturated sodium bicarbonate solution. The organic layer was dried, filtered and concentrated under vacuum to yield a solid as the title compound (3.73g, 96%).

### Part E. Preparation of 1-(3-tert-butyl-4-methoxy-5-(6-nitrobenzo[d]oxazol-2-yl)phenyl)dihydro pyrimidine-2,4(1H,3H)-dione.

A mixture of the product from **Part D** (75mg, 0.246mmol), 2-amino-5-nitrophenol (38mg, 0.0246mmol) and Darco KB charcoal (excess) was refluxed in toluene (10mL) for 24h under exposure to atmospheric of oxygen. Cooled, filtered and purified by reverse phase HPLC chromatography eluting with a 40-100% gradient of acetonitrile in water (0.1% TFA) to provide the title compound as a solid (96mg, 64%). ¹H NMR (300 MHz, DMSO-d₆): δ 1.42 (s, 9 H) 2.74 (t, J=6.80 Hz, 2 H) 3.66 (s, 3 H) 3.82 - 3.88 (m, 2 H) 7.56 (d, J=2.57 Hz, 1 H) 7.91 (d, J=2.57 Hz, 1 H) 8.09 (d, J=8.82 Hz, 1 H) 8.37 (dd, J=8.82, 2.21 Hz, 1 H) 8.84 (d, J=2.21 Hz, 1 H) 10.44 (s, 1 H). MS ESI+ (439) (M+H)+.

### Example 9. Preparation of N-(2-(3-tert-butyl-5-(2,4-dioxotetrahydropyrimidin-1(2H)-yl)-2-methoxyphenyl)benzo[d]oxazol-6-yl)methanesulfonamide (compound IA-L0-2.5).

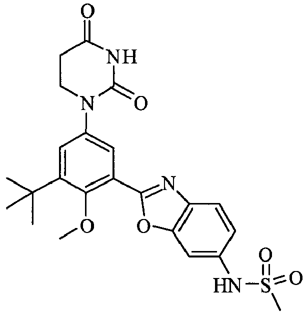

The product from **Example 8** (96mg. 0.219mmol) was reacted a iron (0.614g, 1.10mmol), and ammonium chloride (0.176g, 0.329mmol) in the presence of a mixture of tetrahydrofuran (5mL), ethanol (5mL) and water (3mL). The slurry was heated to 90°C for 45min, cooled to ambient temperature. Filtered through a pad of celite (10g), washed with ethanol (20mL), and the filtrate was concentrated under vacuum to a solid. The resulting solid was dissolved in ethyl acetate and washed with water. Dried over Na₂SO₄, filtered and concentrated under vacuum to a yellow solid, providing the corresponding aniline. The solid was dissolved in dichloromethane (10mL), pyridine (0.670mL, 0.657mmol) and methanesulfonyl chloride (0.221mL, 0.329mmol) were added and the solution stirred at room temperature 16h. CH₂Cl₂ was added followed by washing with a 1N aq. HCl solution. Dried over Na₂SO₄, filtered and concentrated under vacuum. Purification by silica gel column chromatography eluting with 98:2 CH₂Cl₂:MeOH gave the title compound as a solid (25mg, 21%, two steps). ¹H NMR (300 MHz, DMSO-d₆): δ 1.41 (s, 9 H) 2.73 (t, J=6.62 Hz, 2 H) 3.06 (s, 3 H) 3.61 (s, 3 H) 3.83 (t, J=6.62 Hz, 2 H) 7.28 (dd, J=8.46, 1.84 Hz, 1 H) 7.48 (d, J=2.57 Hz, 1 H) 7.65 (d, J=1.84 Hz, 1 H) 7.80 (d, J=1.47 Hz, 1 H) 7.82 (d, J=4.04 Hz, 1 H) 10.03 (s, 1 H) 10.41 (s, 1 H). MS ESI+ (487) (M+H)+.

### Example 10. Preparation of 1-(3-tert-butyl-4-methoxy-5-(5-nitrobenzo[d]oxazol-2-yl)phenyl) dihydropyrimidine-2,4(1H,3H)-dione (compound IA-L0-2.7).

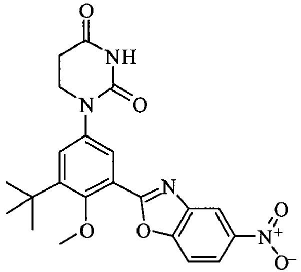

The product from **Example 8**, **Part D** (150mg, 0.493mmol) was reacted with 2-amino-4-nitrophenol (76mg, 0.493mmol) according to the procedures from **Example 8**, **Part E** to provide the title compound as a solid (70mg, 32%). ¹H NMR (300 MHz, DMSO-d₆): δ 1.42 (s, 9 H) 2.74 (t, J=6.80 Hz, 2 H) 3.65 (s, 3 H) 3.85 (t, J=6.62 Hz, 2 H) 7.55 (d, J=2.57 Hz, 1 H) 7.89 (d, J=2.94 Hz, 1 H) 8.12 (d, J=8.82 Hz, 1 H) 8.40 (dd, J=9.01, 2.39 Hz, 1 H) 8.76 (d, J=2.21 Hz, 1 H) 10.43 (s, 1 H). MS ESI+ (439) (M+H)+.

### Example 11. Preparation of N-(2-(3-tert-butyl-5-(2,4-dioxotetrahydropyrimidin-1(2H)-yl)-2-methoxyphenyl)benzo[d]oxazol-5-yl)methanesulfonamide (compound IA-L0-2.8).

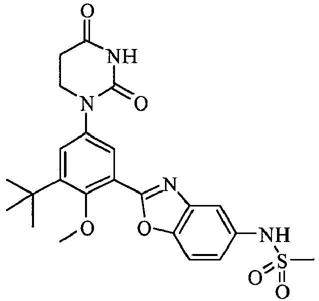

The product from **Example 10** (65mg, 0.148mmol) was reacted according to the procedures from **Example 9** to provide the title compound as a solid (42mg, 44%). ¹H NMR (300 MHz, DMSO-d₆): δ 1.41 (s, 9 H) 2.73 (t, J=6.43 Hz, 2 H) 3.01 (s, 3 H) 3.60 (s, 3 H) 3.83 (t, J=6.43 Hz, 2 H) 7.31 (dd, J=8.64, 2.02 Hz, 1 H) 7.49 (d, J=2.94 Hz, 1 H) 7.56 (d, J=2.21 Hz, 1 H) 7.67 (d, J=2.21 Hz, 1 H) 7.81 (s, 1 H) 9.82 (s, 1 H) 10.41 (s, 1 H). MS ESI+ (487) (M+H)+.

### Example 12. Preparation of 1-(3-(benzo[d]thiazol-2-yl)-5-tert-butyl-4-methoxyphenyl)dihydro pyrimidine-2,4(1H,3H)-dione (compound IA-L0-2.3).

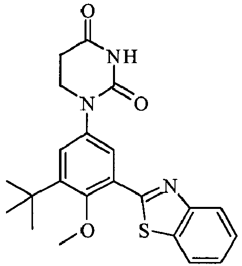

The product from **Example 8**, **Part** D (75mg, 0.246mmol) was reacted with 2-aminobenzene thiol (0.026mL, 0.246mmol) according to the procedures from **Example 8**, **Part E** to provide the title compound as a solid (25mg, 25%). ¹H NMR (300 MHz, DMSO-d₆): δ 1.44 (s, 9 H) 2.73 (t, J=6.43 Hz, 2 H) 3.62 (s, 3 H) 3.84 (t, J=6.62 Hz, 2 H) 7.46 (d, J=2.57 Hz, 1 H) 7.48 - 7.60 (m, 2 H) 7.86 (d, J=2.57 Hz, 1 H) 8.13 (dd, J=17.28, 7.72 Hz, 2 H) 10.40 (s, 1 H). MS ESI+ (410) (M+H)+.

### Example 13. Preparation of N-(2-(3-tert-butyl-5-(2,4-dioxotetrahydropyrimidin-1(2H)-yl)-2-methoxyphenyl)-1H-benzo[d]imidazol-5-yl)methanesulfonamide (compound IA-L0-2.1).

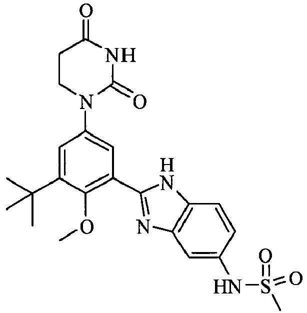

### Part A. Preparation of N-(3,4-dinitrophenyl)methanesulfonamide.

A mixture of 3,4-dinitroaniline (5.27g, 28.8mmol), methanesulfonyl chloride (3.36mL, 43.1mmol) and pyridine (5.82mL, 71.9mmol) in CH₂Cl₂ (100mL) was stirred for 24h. Mixture was concentrated under vacuum to provide a crude semi-solid title compound that was used without further purification.

### Part B. Preparation of N-(3,4-diaminophenyl)methanesulfonamide.

The product from **Part A** (7.51g, 28.8mmol) was reacted with iron (16g, 288mmol) and NH₄Cl (3.84g, 71.9mmol) in refluxing CH₃OH (100mL) and water (20mL) for 2 h. Filtered through celite and concentrated under vacuum. Purification by silica gel column chromatography eluting with MeOH/CH₂Cl₂ provided the title compound as a dark semi-solid (0.5g, 8%).

### Part C. Preparation of N-(2-(3-tert-butyl-5-(2,4-dioxotetrahydropyrimidin-1(2H)-yl)-2-methoxy phenyl)-1H-benzo[d]imidazol-5-yl)methanesulfonamide.

A mixture of the product from **Example 8**, **Part D** (200mg, 0.657mmol) was reacted with the product from **Part B** (132mg, 0.657mmol) according to the procedures from **Example 8**, **Part E** to provide the title compound as a solid (112mg, 34%). ¹H NMR (300 MHz, DMSO-d₆): δ 1.43 (s, 9 H) 2.72 (t, J=6.62 Hz, 2 H) 2.93 (s, 3 H) 3.44 (s, 3 H) 3.82 (t, J=6.43 Hz, 2 H) 7.07 - 7.14 (m, 1 H) 7.38 (d, J=2.57 Hz, 1 H) 7.48 - 7.64 (m, 2 H) 7.72 (d, J=2.57 Hz, 1 H) 9.57 (s, 1 H) 10.38 (s, 1 H) 12.55 (s, 1 H). MS ESI+ (486) (M+H)+.

### Example 14. Preparation of N-(2-(3-tert-butyl-5-(2,4-dioxotetrahydropyrimidin-1(2H)-yl)-2-methoxyphenyl)benzo[d]thiazol-6-yl)methanesulfonamide (compound IA-L0-2.2).

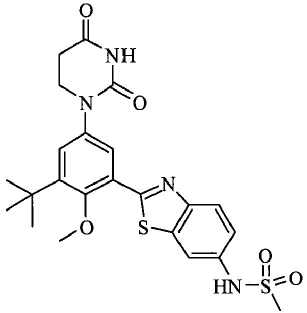

### Part A. Preparation of N-(3-chloro-4-nitrophenyl)methanesulfonamide.

A mixture of 3-chloro-4-nitroaniline (4.85g, 28.1mmol), methanesulfonyl chloride (3.29mL, 42.2mmol) and pyridine (6.82mL, 84mmol) in THF (100mL) was stirred for 24h. Poured in 1M HCl (500mL). The resulting precipitate was filtered and air-dried to provide the title compound as a solid (7.03g, 100%).

### Part B. Preparation of N-(3-(4-methoxybenzylthio)-4-nitrophenyl)methanesulfonamide.

A mixture of the product from **Part A** (7.0g, 27.9mmol), (4-methoxyphenyl)methanethiol (3.89mL, 27.9mmol) and K₂CO₃ (11.58g, 84mmol) in DMF was heated at 100°C for 12h. Cooled and poured into 1M HCl (800mL). The resulting precipitate was filtered and air-dried to provide the title compound as a yellow solid (6.98g, 68%).

### Part C. Preparation of N-(4-amino-3-(4-methoxybenzylthio)phenyl)methanesulfonamide.

The product from **Part B** (6.98g, 19.0mmol) was reacted according to the procedures from **Example 13, Part B** to provide the title compound as a yellow semi-solid (4.44 g, 69%).

### Part D. Preparation of N,N'-(3,3'-disulfanediylbis(4-amino-3,1-phenylene))dimethane-sulfonamide.

The product from **Part C** (708mg, 2.09mmol) was reacted with mercuric (II) acetate (667mg, 2.09mmol), anisole (0.457mL, 4.18mmol) and TFA (10mL) at 0°C for 45min. Concentrated under vacuum and dissolved in MeOH. Hydrogen sulfide gas was bubbled into solution for 1h followed by filtration and concentration under vacuum. Purification by silica gel chromatography eluting with EtOAc/hexane gave the title compound as a yellowish solid (340mg, 75%).

### Part E. Preparation of N-(2-(3-tert-butyl-5-(2,4-dioxotetrahydropyrimidin-1(2H)-yl)-2-methoxyphenyl)benzo[d]thiazol-6-yl)methanesulfonamide.

The product from **Part D** (100mg, 0.23mmol) was reacted with the product from **Example 8**, **Part D** (140mg, 0.46mmol), triphenylphosphine (60.4mg, 0.23mmol) and 4-methylbenzene- sulfonic acid (0.0054mL, 0.046mmol) in refluxing toluene for 3h. Concentrated under vacuum and purified by reverse phase HPLC chromatography eluting a 40-100% gradient of acetonitrile in water (0.1% TFA) to give the title compound as a solid (99mg, 43%). ¹H NMR (300 MHz, DMSO-d₆): δ 1.43 (s, 9 H) 2.73 (t, J=6.62 Hz, 2 H) 3.07 (s, 3 H) 3.63 (s, 3 H) 3.83 (t, J=6.62 Hz, 2 H) 7.39 (dd, J=8.82, 2.21 Hz, 1 H) 7.45 (d, J=2.57 Hz, 1 H) 7.83 (d, J=2.57 Hz, 1 H) 7.95 (d, J=2.21 Hz, 1 H) 8.05 (d, J=8.82 Hz, 1 H) 10.03 (s, 1 H) 10.39 (s, 1 H). MS ESI+ (503) (M+H)+.

### Example 15. Preparation of N-(2-(3-tert-butyl-5-(2,4-dioxotetrahydropyrimidin-1(2H)-yl)-2-methoxyphenyl)benzo[d]thiazol-5-yl)methanesulfonamide (compound IA-L0-2.4).

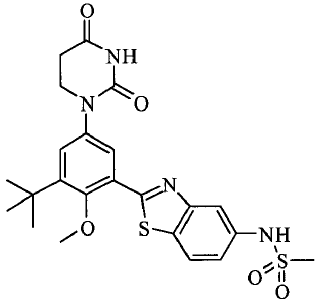

### Part A. Preparation of N-(4-chloro-3-nitrophenyl)methanesulfonamide.

A mixture of 4-chloro-3-nitroaniline (5.0g, 29mmol), methanesulfonyl chloride (2.37mL, 30.4mmol) and pyridine (5.9mL, 72.4mmol) in THF (100mL) was stirred for 24h. Poured in 1M HCl (500mL). The resulting precipitate was filtered and air-dried to provide the title compound as a solid (6.7g, 92%).

### Part B. Preparation of N-(4-(4-methoxybenzylthio)-3-nitrophenyl)methanesulfonamide.

A mixture of the product from **Part A** (3.0g, 12mmol), (4-methoxyphenyl)methanethiol (1.67mL, 12mmol) and K₂CO₃ (4.96g, 36mmol) in DMF was heated at 100°C for 12h. Cooled and poured into 1M HCl (800mL). The resulting precipitate was filtered and air-dried to provide the title compound as a yellow solid (1.95g, 44.2%).

### Part C. Preparation of N-(3-amino-4-(4-methoxybenzylthio)phenyl)methanesulfonamide.

The product from **Part B** (1.43g, 3.88mmol) was reacted according to the procedures from **Example 13**, **Part B** to provide the title compound as a white solid (1.31g, 100%).

### Part D. Preparation of N,N'-(4,4'-disulfanediylbis(3-amino-4,1-phenylene))dimethane-sulfonamide.

The product from **Part C** (75mg, 0.222mmol) was reacted with mercuric (II) acetate (70.6mg, 0.222mmol), anisole (0.048mL, 0.443mmol) and TFA (10mL) at 0°C for 45min. Concentrated under vacuum and dissolved in MeOH. Hydrogen sulfide gas was bubbled into solution for 1h followed by filtration and concentration under vacuum. Purification by silica gel column chromatography eluting with EtOAc/Hexane gave the title compound as a yellowish solid (34mg, 71%).

### Part E. Preparation of N-(2-(3-tert-butyl-5-(2,4-dioxotetrahydropyrimidin-1(2H)-yl)-2-methoxy phenyl)benzo[d]thiazol-5-yl)methanesulfonamide.

The product from **Part D** (50mg, 0.115mmol) was reacted with the product from **Example 8**, **Part D** (70mg, 0.230mmol), triphenylphosphine (30.2mg, 0.115mmol) and 4-methylbenzenesulfonic acid (0.00267mL, 0.023mmol) in refluxing toluene for 3h. Concentrated under vacuum and purified by reverse phase HPLC chromatography eluting with a 40-100% gradient of acetonitrile in water (0.1% TFA) to give the title compound as a solid (40mg, 33%). ¹H NMR (300 MHz, DMSO-d₆): δ 1.43 (s, 9 H) 2.73 (t, J=6.80 Hz, 2 H) 3.05 (s, 3 H) 3.63 (s, 3 H) 3.84 (t, J=6.62 Hz, 2 H) 7.35 (dd, J=8.64, 2.02 Hz, 1 H) 7.46 (d, J=2.94 Hz, 1 H) 7.86 (d, J=2.94 Hz, 1 H) 7.92 (d, J=1.84 Hz, 1 H) 8.10 (d, J=8.46 Hz, 1 H) 9.98 (s, 1 H) 10.40 (s, 1 H). MS ESI+ (503) (M+H)+.

### Example 16. Preparation of 1-(3-tert-butyl-4-methoxy-5-(naphthalen-2-yl)phenyl) pyrimidine-2,4(1H,3H)-dione (compound IB-L0-2.1).

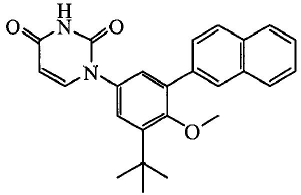

### Part A. Preparation of tert-butyl 3-tert-butyl-4-methoxy-5-(naphthalen-2-yl)phenyl carbamate.

In a resealable Schlenk tube, a solution of the product from **Example 3**, **Part H** (200mg, 0.56mmol), naphthalene-2-boronic acid (144mg, 0.84mmol), and 1.0M sodium carbonate solution (558µL, 0. 56mmol) in toluene (2.8mL) was degassed by nitrogen sparge for 10min. The mixture was treated with 1,1'-bis(diphenylphosphino)ferrocene palladium (II) chloride dichloromethane complex (14mg, 0.017mmol) and degassing was continued for another 5min. The Schlenk tube was sealed and warmed at 95°C for 18h. Cooled and diluted with ethyl acetate and water. Treated with Darco G-60 and filtered through celite. Filtrate was extracted with water (2 x) and with brine. Dried over Na₂SO₄, filtered and concentrated. Purification by silica gel column chromatography eluting with 10-75 % EtOAc in hexanes gave the title compound as an oil (210mg, 93 %).

### Part B. Preparation of 3-tert-butyl-4-methoxy-5-(naphthalen-2-yl)aniline.

The product from **Part A** (210mg, 0.52mmol) was dissolved in 4N HCl in dioxane (4.0mL) and stirred at room temperature for 1h. Concentration under vacuum afforded a solid, which was suspended in ethyl acetate and stirred with saturated sodium bicarbonate solution. The organic layer was dried over Na₂SO₄, filtered and concentrated under vacuum to give the title compound, as a brown oil (111mg, 70 %).

### Part C. Preparation of (E)-N-(3-tert-butyl-4-methoxy-5-(naphthalen-2-yl)phenylcarbamoyl)-3-methoxyacrylamide.

A solution of the product from **Part B** (111mg, 0.36mmol) in dry DMF (2.9mL) at -20°C was treated with (E)-3-methoxyacryloyl isocyanate solution (0.66mL, of 0.55M in benzene, 0.36mmol) followed by gradual warming to room temperature. After stirring for 30min, the mixture was cooled again to -20°C and more (E)-3-methoxyacryloyl isocyanate solution (1.0mL, 0.55mmol) was added. After warming again to room temperature for 30min, the reaction was complete. Diluted with EtOAc and extracted with water and brine. Dried over Na₂SO₄, filtered and concentrated under vacuum. Purification by silica gel column chromatography eluting with 10-100 % EtOAc in hexane gave the title compound as a light yellow oil (144mg, 92%).

### Part D. Preparation of 1-(3-tert-butyl-4-methoxy-5-(naphthalen-2-yl)phenyl)pyrimidine-2,4(1H,3H)-dione.

A suspension of the product from **Part C** (144mg, 0.33mmol) in 2:2:1 ethanol-water-THF (15mL) was treated with 1N sulfuric acid solution (3.0mL) followed by warming at 100°C for 24h. Cooled and diluted with EtOAc and extracted with water and brine. Dried over Na₂SO₄, filtered and concentrated under vacuum. Purification by silica gel column chromatography eluting with 10-100% EtOAc in hexane gave the title compound as a white solid (62mg, 47 %). ¹H NMR (300 MHz, DMSO-*d₆*): δ 11.42 (s, 1 H), 8.08 (s, 1 H), 7.90 - 8.04 (m, 3 H), 7.81 (d, J=7.72 Hz, 1 H), 7.72 (d, J=8.46 Hz, 1 H), 7.56 (dd, J=6.25, 3.31 Hz, 2 H), 7.39 (d, J=2.57 Hz, 1 H), 7.33 (d, J=2.57 Hz, 1 H), 5.65 (d, J=7.72 Hz, 1 H), 3.24 (s, 3 H), 1.43 (s, 9 H). MS +ESI m/z (rel abundance): 401 (100, M+H), 418 (30, M+NH4).

### Example 17. Preparation of 1-(3-tert-butyl-4-methoxy-5-(6-methoxynaphthalen-2-yl)phenyl) pyrimidine-2,4(1H,3H)-dione (compound IB-L0-2.2).

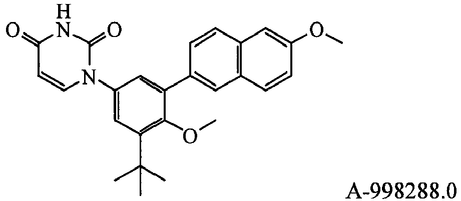

### Part A. Preparation of tert-butyl 3-tert-butyl-4-methoxy-5-(6-methoxynaphthalen-2-yl)phenyl carbamate.

The product from **Example 3, Part H** (158mg, 0.44mmol) was reacted with 6-methoxynaphthalen-2-ylboronic acid (107mg, 0.52mmol) according to the procedures from **Example 16, Part A** to provide the title compound as a white solid (92mg, 47 %).

### Part B. Preparation of 3-tert-butyl-4-methoxy-5-(6-methoxynaphthalen-2-yl)aniline.

The product from **Part A** (92mg, 0.21mmol) was reacted according to the procedures from **Example 16, Part B** to provide the title compound as a pink solid (71mg, 99%).

### Part C. Preparation of (E)-N-(3-tert-butyl-4-methoxy-5-(6-methoxynaphthalen-2-yl)phenyl carbamoyl)-3-methoxyacrylamide.

The product from **Part B** (71mg, 0.21mmol) was reacted according to the procedures from **Example 16, Part C** to provide the title compound as a buff-colored solid (58mg, 59 %).

### Part D. Preparation of 1-(3-tert-butyl-4-methoxy-5-(6-methoxynaphthalen-2-yl)phenyl) pyrimidine-2,4(1H,3H)-dione.

A solution of the product from **Part C** (58mg, 0.13mmol) in 2:1:1 ethanol-THF-water (4.0mL) was treated with 1.0M sulfuric acid solution (3.0mL) followed by warming at 95°C for 24h. Cooled and diluted with EtOAc. Extracted with water and brine. Dried over Na₂SO₄, filtered and concentrated under vacuum. Purification by silica gel column chromatography eluting with 10-100 % EtOAc in hexanes gave the product as a faint pink solid (28mg, 52%). ¹H NMR (300 MHz, DMSO-*d₆*): δ 11.41 (s, 1 H), 8.00 (s, 1 H), 7.91 (dd, J=8.64, 4.60 Hz, 2 H), 7.80 (d, J=7.72 Hz, 1 H), 7.67 (d, J=8.82 Hz, 1 H), 7.34 - 7.47 (m, 2 H), 7.21 - 7.32 (m, 1 H), 7.20 (dd, J=9.01, 2.39 Hz, 1 H), 5.65 (d, J=7.72 Hz, 1 H), 3.90 (s, 3 H), 3.24 (s, 3 H), 1.42 (s, 9 H). MS +ESI m/z (rel abundance): 431 (100, M+H), 448 (45, M+NH4).

### Example 18. Preparation of N-(6-(3-tert-butyl-5-(2,4-dioxo-3,4-dihydropyrimidin-1(2H)-yl) phenyl)naphthalen-2-yl)methanesulfonamide (compound IB-L0-2.8).

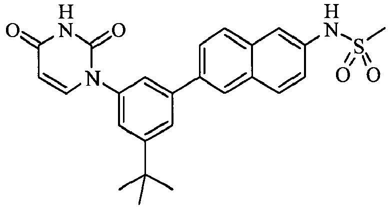

### Part A. Preparation of 2-bromo-4-tert-butyl-6-nitroaniline.

A suspension of 4-tert-butyl-2-nitroaniline (1.033g, 5.32mmol) in glacial acetic acid (7.8mL) was warmed with a heat gun until all solids had dissolved. The solution was then cooled and treated portion wise with pyridinium hydrobromide perbromide (1.96g, 6.12mmol). After addition, the solution was stirred at room temperature for 1h. The mixture was added to water (50mL) and treated with a small amount of sodium sulfite. After stirring for 30min, the precipitate was collected by filtration. The solid obtained was washed with water and dissolved in EtOAc. Washed with water and brine. Dried over Na₂SO₄, filtered and concentrated under vacuum to provide the title compound as a yellow-orange solid (1.36g, 94 %).

### Part B. Preparation of 1-bromo-3-tert-butyl-5-nitrobenzene.

A solution of tert-butyl nitrite (300µL of 90%, 261mg, 2.27mmol) in dry DMF (4mL) was warmed at 50°C and was treated with a solution of the product from **Part A** (414mg, 1.52mmol) in DMF (3.5mL). After a few minutes stirring, the solution began to bubble vigorously. After warming at 50°C for 1h, additional (300µL) tert-butyl nitrite was added followed by warming at 50°C for 1h. After 18h at room temperature, tert-butyl nitrite (1.2mL) was added and the mixture warmed at 50°C for 2h. Cooled and diluted with EtOAc. Washed with water and brine. Dried over Na₂SO₄, filtered and concentrated under vacuum. Purification by silica gel column chromatography eluting with 5-40 % ethyl acetate in hexanes gave the title compound as a light yellow oil (159mg, 41 %).

### Part C. Preparation of 3-bromo-5-tert-butylaniline.

A solution of the product from **Part B** (770mg, 2.98mmol) in 3:3:1 methanol-water-THF (14.9mL) was treated with ammonium chloride (239mg, 4.47mmol) and iron powder (833mg, 14.92mmol) followed by warming at reflux for 8h. Diluted with EtOAc and water and filtered through celite. The filtrate was extracted with water and brine. Dried over Na₂SO₄, filtered and concentrated under vacuum to give the title compound as a yellow oil.

### Part D. Preparation of (E)-N-(3-bromo-5-tert-butylphenylcarbamoyl)-3-methoxy acrylamide.

A solution of the product from **Part C** (681mg, 2.99mmol) in dry DMF (23mL) at -30°C was treated drop wise with a 0.4M solution of (E)-3-methoxyacryloyl isocyanate in benzene (14.9mL, 5.96mmol). The solution was stirred at -30°C for 30min followed by warming gradually to room temperature, and then stirred for 18h. Diluted with EtOAc and washed with water and brine. Dried over Na₂SO₄, filtered and concentrated under vacuum to afford a yellow solid, which was triturated with ether-hexanes and collected by filtration. Dried under vacuum to give the title compound as a light brown powder. (951mg, 90 %).

### Part E. Preparation of 1-(3-bromo-5-tert-butylphenyl)pyrimidine-2,4(1H,3H)-dione.

A suspension of the product from **Part D** (951mg, 2.68mmol) in ethanol (25mL) was treated with a solution of concentrated sulfuric acid (2.60mL, 4.78g, 18.22mmol) in water (13.4mL) followed by warming at 100°C for 1h. Cooled and concentrated to remove ethanol. Cooled to 0°C and the precipitate was collected by filtration and washed with water. Dried under vacuum to give the title compound as an orange solid (619mg, 72 %).

### Part F. Preparation of N-(6-(3-tert-butyl-5-(2,4-dioxo-3,4-dihydropyrimidin-1(2H)-yl)phenyl) naphthalen-2-yl)methanesulfonamide.

In a microwave tube, a suspension of the product from **Part E** (104mg, 0.32mmol), the product from **Example 4A, Part B** (134mg, 0.39mmol), and 1.0M sodium carbonate solution (386µL, 0.39mmol) in 1:1 ethanol-toluene (2.1mL) was degassed by nitrogen sparge for 10min. The solution was treated with 1,1'-bis(di-tert-butylphosphino)ferrocene-palladium (II) dichloride (20mg, 0.031mmol) and degassing was continued for another 5min. The mixture was heated at 100°C in the microwave for 30min. Diluted with EtOAc and washed with water and brine. Dried over Na₂SO₄ and treated with (3-mercapto propyl) silica gel for 30min. Filtered and concentrated under vacuum to afford an amber solid, which was triturated with ether-hexanes. Collected the solid by filtration and dried under vacuum to provide the title compound (81mg, 54 %). ¹H NMR (300 MHz, DMSO-*d₆*): δ 11.46 (s, 1 H) 10.05 (s, 1 H), 8.25 (s, 1 H) 7.98 (dd, J=11.58, 9.01 Hz, 1 H) 7.86 - 7.93 (m, 1 H) 7.78 - 7.85 (m, 2 H) 7.72 (s, 1 H) 7.67 (s, 1 H) 7.31 1 - 7.51 (m, 2 H) 5.70 (dd, J=7.72, 2.21 Hz, 1 H) 3.08 (s, 3 H) 1.39 (s, 9 H).

### Example 19. Preparation of (E)-N'-(5-(3-tert-butyl-5-(2,4-dioxo-3,4-dihydropyrimidin-1(2H)-yl)phenyl)-2,3-dihydro-1H-inden-1-ylidene)methanesulfonohydrazide (compound IB-L0-2.7).

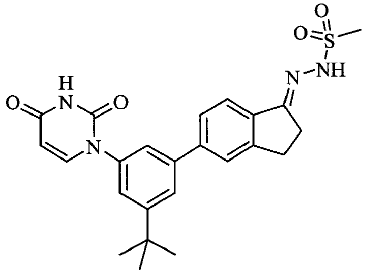

### Part A. Preparation of 1-(3-tert-butyl-5-(1-oxo-2,3-dihydro-1H-inden-5-yl)phenyl) pyrimidine-2,4(1H,3H)-dione.

In a microwave tube, a suspension of the product from **Example 18, Part E,** the product from **Example 6, Part A** (144mg, 0.56mmol), 1.0M sodium carbonate solution (557µL, 0.56mmol) in 1:1 ethanol-toluene (3.0mL) was degassed by nitrogen sparge for 15min. 1,1'-Bis(di-t-butylphosphino) ferrocene palladium (II) chloride complex (15mg, 0.023mmol) was added and degassing was continued for an additional 5 min. The tube was sealed and the mixture was heated at 100°C in the microwave for 30 min. Diluted with EtOAc and water. Washed with 1M citric acid solution, water, and brine. The organic was stirred with (3-mercaptopropyl) silica gel for 1h. Dried over Na₂SO₄, filtered and concentrated under vacuum. Purification by silica gel column chromatography eluting with 10-100 % EtOAc in hexanes gave the title compound as an off-white solid (86mg, 50 %).

### Part B. Preparation of (E)-N'-(5-(3-tert-butyl-5-(2,4-dioxo-3,4-dihydropyrimidin-1(2H)-yl) phenyl)-2,3-dihydro-1H-inden-1-ylidene)methanesulfonohydrazide.

The product from **Part A** (80mg, 0.21mmol) was reacted according to the procedures from **Example 6, Part C** to provide the title compound as a white solid (73mg, 73 %). ¹H NMR (300 MHz, DMSO-d₆): δ 11.44 (s, 1 H) 9.92 (s, 1 H) 7.64 - 7.98 (m, 5 H) 7.57 (s, 1 H) 7.45 (s, 1 H) 5.68 (d, J=7.72 Hz, 1 H) 3.00 - 3.20 (m, 5 H) 2.85 (d, J=12.50 Hz, 2 H) 1.36 (s, 9 H). MS +ESI m/z (rel abundance): 467 (100, M+H).

### Example 20. Preparation of 3-tert-butyl-5-(2,4-dioxotetrahydropyrimidin-1(2H)-yl)-2-hydroxy-N-(4-(methylsulfonamido)phenyl)benzamide (compound IA-L3-1.6).

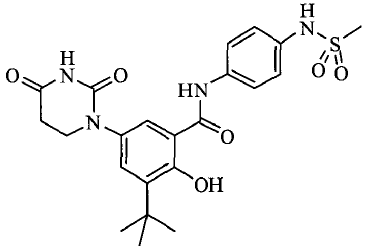

### Part A. Preparation of methyl 3-tert-butyl-2-hydroxy-5-nitrobenzoate.

Methyl 3,5-di-tert-butyl-2-hydroxybenzoate (28.66g, 108.4mmol) was dissolved with stirring in 430mL glacial acetic acid and the resulting mixture was treated drop wise with fuming nitric acid (90%, 179.26mL). When the addition was complete, the resulting mixture was stirred for 2.5h. The reaction mixture was poured into a 2.0L of crushed ice and allowed to stand 30min. Afterwards, 1.0L of water was added and the ice water mixture was allowed to melt. The mixture was then filtered, washed with water and dried to provide the title compound (24.57g, 89%).

### Part B. Preparation of methyl 5-amino-3-tert-butyl-2-hydroxybenzoate.

The product of **Part A** (0.43g, 1.70mmol) was treated with a catalytic amount of Pd/C in THF (10mL) under hydrogen balloon for 3h. The flask was purged with nitrogen and the mixture was filtered, concentrated, and purified by column chromatography on silica gel, eluting with 50% hexane/dichloromethane, followed by dichloromethane to yield 0.37g (98%).

### Part C. Preparation of methyl 5-(3-amino-3-oxopropylamino)-3-tert-butyl-2-hydroxy benzoate.

The product of **Part B** (0.37g , 1.66mmol) and acrylic acid (0.12uL, 1.74mmol) were combined in toluene (10mL) and heated at reflux for 20h. The solution was concentrated to dryness.

### Part D. Preparation of methyl 3-tert-butyl-5-(2,4-dioxotetrahydropyrimidin-1(2H)-yl)-2-hydroxybenzoate.

The product of **Part C** was dissolved in glacial acetic acid (5mL) and treated with urea (0.24g, 4.0mmol) at 120°C for 3h. The solution was diluted with cold water, extracted into ethyl acetate, concentrated, and purified by column chromatography on silica gel, eluting with 1%, then 2%, then 4% methanol/dichloro- methane to give both product (0.25g, 46%) and open dihydrouracil (0.112g, 20%).

### Part E. Preparation of 3-tert-butyl-5-(1-(2-carboxyethyl)ureido)-2-hydroxybenzoic acid.

The products from **Part D** were dissolved in methanol (6mL) and 1M sodium hydroxide solution was added (15mL). After 20h, the solution was adjusted to pH2 with concentrated hydrochloric acid and extracted into ethyl acetate, dried over sodium sulfate, and concentrated to give 0.303g (89%).

### Part F. Preparation of 3-tert-butyl-5-(2,4-dioxotetrahydropyrimidin-1(2H)-yl)-2-hydroxybenzoic acid.

The product from **Part E** (0.303g, 0.93mmol) was taken up into 7mL of concentrated hydrochloric acid and heated in an open flask at 120°C for 1h, during which time the excess acid evaporated off to leave dry product 0.20g (70%).

### Part G. Preparation of 3-tert-butyl-5-(2,4-dioxotetrahydropyrimidin-1(2H)-yl)-2-hydroxy-N-(4-(methylsulfonamido)phenyl)benzamide.

The product from **Part F** (0.13g, 0.42mmol) was heated with thionyl chloride (3mL) at 90°C for 1.5h in an open flask leaving dry acid chloride, which was taken up in dioxane (4mL). N-(4-amino-phenyl)methanesulfonamide.HCl (0.070mg, 0.31mmol) was added and the solution was heated at 90°C for 1h. The mixture was concentrated and then triturated with dichloromethane, filtered, and dried to give 0.071mg (48%) of the title compound. 1H NMR (300 MHz, DMSO-D6) δ ppm 1.39 (s, 9 H), 2.73 (t, J=6.62 Hz, 2 H), 2.99 (s, 3H), 3.78 (t, J=6.62 Hz, 2 H), 7.24 (d, J=8.82 Hz, 2 H), 7.40 (d, J=2.21 Hz, 1 H), 7.60 (d, J=9.19 Hz, 2 H), 7.89 (d, J=2.21 Hz, 1 H), 9.74 (s, 1 H), 10.39 (s, 1 H), 10.44 (s, 1 H),13.30 (s, 1 H).

### Example 21. Preparation of 3-tert-butyl-5-(2,4-dioxotetrahydropyrimidin-1(2H)-yl)-2-hydroxy-N-(4-(2-methoxyethylsulfonamido)phenyl)benzamide (compound IA-L3-1.8).

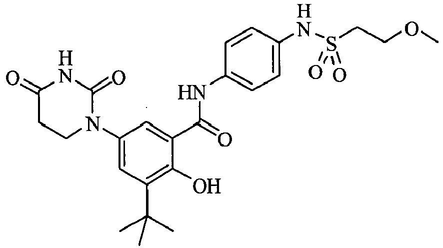

### Part A. Preparation of tert-butyl 4-(vinylsulfonamido)phenylcarbamate.

A solution of tert-butyl 4-aminophenylcarbamate (2.63g, 12.63mmol) and triethylamine (7.04mL, 50.51mmol) were combined in dichloromethane (50mL) and cooled in an ice bath. After drop wise addition of 2-chloroethanesulfonyl chloride (1.45mL, 13.9mmol), the solution was stirred at ambient temperature for 4h then diluted with 0.5M HCl and extracted into dichloromethane. The product was purified by column chromatography on silica gel, eluting with 1% methanol/dichloromethane to give 2.48g (66%).

### Part B. Preparation of tert-butyl 4-(2-methoxyethylsulfonamido)phenylcarbamate.

The product from **Part A** (0.70g, 2.35mmol) was heated at 60°C in a sealed tube with 10mL methanol and 5mL of 25% weight sodium methoxide in methanol for 16h. The solution was diluted with water and adjusted to pH 6 with 1M HCl, then extracted into dichloromethane and concentrated to give 0.582g (75%).

### Part C. Preparation of N-(4-aminophenyl)-2-methoxyethanesulfonamide.

The product from **Part B** (0.582g, 1.76mmol) was taken up in 155mL of 4M HCl in dioxane and stirred at ambient temperature for 20h. The solution was diluted with dichloromethane and the solid product was filtered off and dried to give 0.395g (84%).

### Part D. Preparation of 3-tert-butyl-5-(2,4-dioxotetrahydropyrimidin-1(2H)-yl)-2-hydroxy-N-(4-(2-methoxyethylsulfonamido)phenyl)benzamide.

The product from **Example 20, Part F** (0.05g, 0.163mmol) was treated with thionyl chloride (0.5mL) and the product from **Part C** (0.038g, 0.163mmol) as in **Example 20, Part G** to give 0.038g (45%) of the title compound. ¹H NMR (300 MHz, DMSO-D6): δ ppm 1.39 (s, 9 H), 2.73 (t, J=6.62 Hz, 2 H), 3.20 (s, 3 H), 3.33 - 3.42 (m, 2 H), 3.67 (t, J=6.25 Hz, 2 H), 3.78 (t, J=6.62 Hz, 2 H), 7.23 (d, J=9.19 Hz, 2 H), 7.40 (d, J=2.21 Hz, 1 H), 7.58 (d, J=9.19 Hz, 2 H), 7.89 (d, J=2.21 Hz, 1 H), 9.80 (s, 1 H), 10.39 (s, 1 H), 10.44 (s, 1 H), 13.30 (s, 1 H).

### Example 22. Preparation of 3-tert-butyl-5-(2,4-dioxotetrahydropyrimidin-1(2H)-yl)-2-methoxy-N-(4-methylsulfonamido)phenyl)benzamide (compound IA-L3-1.51).

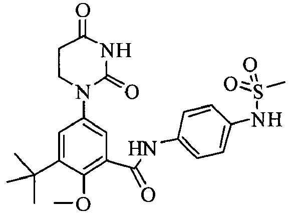

### Part A. Preparation of methyl 3-tert-butyl-2-hydroxy-5-nitrobenzoate.

Methyl 3,5-di-tert-butyl-2-hydroxybenzoate (28.66g, 108.4mmol) was dissolved with stirring in 430mL glacial acetic acid and the resulting mixture was treated drop wise with fuming nitric acid (90%, 179.26mL). When the addition was complete, the resulting mixture was stirred for 2.5h. The reaction mixture was poured into a 2.0L of crushed ice and allowed to stand 30min. Afterwards, 1.0L of water was added and the ice water mixture was allowed to melt. The mixture was then filtered, washed with water and dried to provide the title compound (24.57g, 89%).

### Part B. Preparation of methyl 3-tert-butyl-2-methoxy-5-nitrobenzoate.

The product from **Part A** (11.41g, 45.0mmol), potassium carbonate (9.34g, 67.6mmol), acetone (200mL), and dimethyl sulfate (6.46g, 67.6mmol) were added together. The resultant mixture was then heated to reflux for 16h. The mixture was then filtered and the solid was washed with ethyl acetate. The resulting organic liquid was then concentrated under vacuum to an oil and redissolved in ethyl acetate (600mL). The organic solution was then washed with water, dried, filtered and concentrated under vacuum to an oil that was then subjected to purification via column chromatography (gradient of 5% to 40% EtOAc/Hexanes) to yield the title compound as an oil (10.42, 87%).

### Part C. Preparation of methyl 5-amino-3-tert-butyl-2-methoxybenzoate.

The product from **Part B** (10.42g, 39.0mmol), iron powder (325mesh, 10.89g, 195mmol), ammonium chloride (3.13g, 58.5mmol), water (30mL), and methanol (150mL) were added together. The resultant mixture was then refluxed for 1h. The mixture was then cooled to room temperature, filtered through celite, and the celite washed with methanol. The filtrate was then concentrated under vacuum and dissolved in ethyl acetate (600mL). The resultant solution was then washed with water and brine. The organic extract was then dried, filtered and concentrated under vacuum to yield the title compound as an oil (9.25g, 100%).

### Part D. Preparation of 3-(3-tert-butyl-4-methoxy-5-(methoxycarbonyl)phenylamino) propanoic acid.

The product from **Part C** (16.44g, 69.3mmol) was dissolved in toluene (200mL). This mixture was heated to reflux and acrylic acid added over time (1mL of acrylic acid added every 3h, 5.23mL total, 76.2mmol). The resulting mixture was refluxed for 24h. The mixture was cooled and concentrated to dryness under vacuum to yield the crude title compound as an oil that was used directly in the next reaction.

### Part E. Preparation of methyl 3-tert-butyl-5-(2,4-dioxotetrahydropyrimidin-1(2H)-yl)-2-methoxybenzoate.

The product from **Part D** (21.43g, 69.3mmol), urea (10.4g, 173mmol) and acetic acid (glacial, 200mL) were added together. The mixture was then heated to 120°C for 18.5h followed by concentration under vacuum to dryness to an oil. To this oil was added methanol (13mL), and ethyl acetate (350mL). The resultant mixture was allowed to stand for 24-48h whereby a precipitate formed. The resulting solid was filtered off and washed with a small amount of methanol (10mL) and then air dried to yield the title compound as a solid (15.26g, 66%).

### Part F. Preparation of 3-tert-butyl-5-(2,4-dioxotetrahydropyrimidin-1(2H)-yl)-2-methoxy benzoic acid.

The product from **Part E** (4.52g, 13.52mmol), methanol (70mL), and tetrahydrofuran (70mL) were added together. The mixture was then stirred vigorously until a homogenous solution resulted. Once homogenous, a solution of aqueous sodium hydroxide (1.0M, 68mL) was added. The mixture was then stirred for 12h, the mixture was then concentrated under vacuum to remove the organic solvent, followed by the addition of aqueous hydrochloric acid (1.0M, 80mL) that resulted in solid formation. The mixture was then concentrated under vacuum. To this material was added hydrochloric acid (12M, 100mL) and the resultant material heated to 100°C for 1.5h. The reaction was then cooled and water added. The resulting solid was filtered, washed with water, and dried to yield the title compound as a solid (3.55g, 82%).

### Part G. Preparation of 3-tert-butyl-5-(2,4-dioxotetrahydropyrimidin-1(2H)-yl)-2methoxybenzoyl chloride.

The product from **Part F** (2H)-yl)-2-methoxybenzoic acid (4.07g, 12.71mmol) and thionyl chloride (40.82mL, 559mmol) were added together. The mixture was then refluxed for 2h, followed by concentration under vacuum to provide the product as a light-yellow solid.

### Part H. Preparation of 3-tert-butyl-5-(2,4-dioxotetrahydropyrimidin-1(2H)-yl)-2-methoxy-N-(4-methylsulfonamido)phenyl)benzamide.

The product prepared in **Part G** (0.55g, 1.71mmole) was dissolved in CH₂Cl₂ (35ml) and added drop wise to a suspension in CH₂Cl₂ (40ml) containing N-(4-aminophenyl)methanesulfonamide hydrochloride salt (0.38g, 1.71mmole) and pyridine (0.41ml, 5.1mmole). The reaction mixture was stirred 18h at room temperature. The reaction mixture was filtered and diluted with 400ml CH₂Cl₂. The organic layer was washed with 1N H₃PO₄, 10% NaHCO3 and 10% NaCl and dried over anhydrous solid sodium sulfate. The drying agent was filtered and the organic layer was evaporated in vacuo leaving the title compound as a crème-colored solid (474mg, 57%). ¹H NMR (300 MHz, DMSO-D6) δ ppm 1.37 (s, 9 H) 2.71 (t, *J*=6.62 Hz, 2 H) 2.95 (s, 3 H) 3.77 (s, 3 H) 3.80 (d, 2 H) 7.20 (d, *J*=9.19 Hz, 2 H) 7.28 (d, *J*=2.57 Hz, 1 H) 7.33 (d, *J*=2.94 Hz, 1 H) 7.69 (d, *J*=9.19 Hz, 2 H) 9.59 (s, 1 H) 10.35 (s, 1 H) 10.38 (s, 1 H).

### Example 23. Preparation of 4-(3-tert-butyl-5-(2,4-dioxotetrahydropyrimidin-1(2H)-yl)-2-hydroxybenzamido)phenyl methanesulfonate (compound IA-L3-1.19).

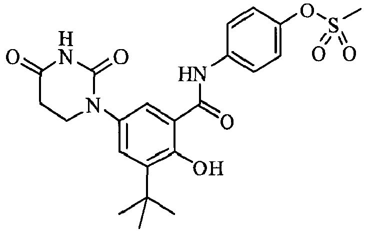

### Part A. Preparation of 4-(tert-butoxycarbonylamino)phenyl methanesulfonate.

Tert-butyl-4-hydroxyphenylcarbamate (1.0g, 4.78mmol) and triethylamine (0.80mL, 5.73mmol) were combined in dichloromethane (50mL), cooled in an ice bath and treated with methanesulfonyl chloride (0.41mL, 5.26mmol). The solution was stirred at ambient temperature for 2h, then was washed with 1M HCl, dried over sodium sulfate, filtered, and concentrated to give 1.2g (87%).

### Part B. Preparation of 4-aminophenyl methanesulfonate hydrochloride.

The product from **Part A** (1.2g, 4.18mmol) was treated with 4 M HCl in dioxane (10mL) at ambient temperature and stirred for 18h. The mixture was concentrated and the solid was triturated with dichloromethane, filtered, and dried to give 0.855g (92%).

### Part C. Preparation of 4-(3-tert-butyl-5-(2,4-dioxotetrahydropyrimidin-1(2H)-yl)-2-hydroxy benzamido)phenyl methanesulfonate.

The product from **Example 20, Part F** (0.055g, 0.18mmol) was treated with thionyl chloride (0.4mL, 5.4mmol) at 80°C for 35min then concentrated to dryness. This acid chloride was dissolved in dioxane (2mL) and treated with the product from **Part B** (0.060g, 0.27mmol) and pyridine (0.037mL, 0.45mmol). The resulting mixture was stirred at 80°C for 1h, diluted with 1M HCl, extracted into ethyl acetate, concentrated and purified by column chromatography on silica gel, eluting with dichloromethane followed by 2% methanol/dichloromethane to give 0.055g (64%) of the title compound. ¹H NMR (300 MHz, DMSO-D6) δ ppm 1.39 (s, 9 H), 2.74 (t, J=6.62 Hz, 2 H) 3.40 (s, 3 H), 3.79 (t, J=6.80 Hz, 2 H), 7.36 - 7.45 (m, 3 H) 7.76 (d, J=9.19 Hz, 2 H), 7.90 (d, J=2.21 Hz, 1 H), 10.40 (s, 1 H), 10.58 (s, 1 H) 13.13 (s, 1 H).

### Example 24. Preparation of 3-tert-butyl-5-(2,4-dioxotetrahydropyrimidin-1(2H)-yl)-2-methoxy-N-methyl-N-(4-(methylsulfonamido)phenyl)benzamide (compound IA-L3-1.27).

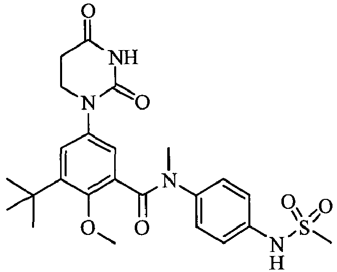

### Part A. Preparation of tert-butyl 4-aminophenyl(methyl)carbamate.

A mixture of *N*-methyl-4-nitroaniline (1.00g, 6.57mmol), di-*tert*-butyl dicarbonate (2.51 g, 11.50mmol), and DMAP (40mg, 0.33mmol) in dichloromethane (35mL) was stirred at reflux for 2h. The reaction mixture was washed with water (20mL), dried over Na₂SO₄, filtered and concentrated under vacuum. The residue was dissolved in a mixture of THF (12mL) and methanol (12mL). To the resulting solution was added iron powder (1.50g, 27.0mmol) and a solution of ammonium chloride (0.54g, 10.11mmol) in water (5mL). The mixture was stirred at 70°C for 3h, cooled to room temperature and filtered through celite, and concentrated under vacuum. The residue was azeotropically dried using toluene (3X) and then triturated with ether to give a solid that was removed by filtration. The filtrate was concentrated under vacuum to give the title compound (1.45g, 99%).

### Part B. Preparation of N-(4-(methylamino)phenyl)methanesulfonamide hydrochloride.

The product prepared in **Part A** (1.45g, 6.52mmol) was dissolved in anhydrous dichloromethane (25mL) and treated with pyridine (1.32mL, 16.31mmol) and methanesulfonyl chloride (0.57mL, 7.18mmol). The resulting solution was stirred at room temperature for 3h, and then poured into 0.5M aq. HCl (25mL). The layers were separated and the aqueous phase was washed with dichloromethane (2 x 25mL). The combined organic layers were dried over Na₂SO₄, filtered and concentrated under vacuum. Column chromatography on silica gel using 1% methanol in chloroform as the eluent gave *tert*-butyl methyl(4-(methylsulfonamido)phenyl) carbamate (1.31g, 67%), which was dissolved in 4N HCl in 1,4-dioxane (20mL). The resulting solution was stirred at 40°C for 1h and concentrated under vacuum. The residue was triturated with dichloromethane to give the title compound as a solid that was collected by filtration and dried under vacuum (0.99 g, 96%).

### Part C. Preparation of 3-tert-butyl-5-(2,4-dioxotetrahydropyrimidin-1(2H)-yl)-2-methoxy-N-methyl-N-(4-(methylsulfonamido)phenyl)benzamide.

The product prepared in **Example 22, Part G** (40mg, 0.13mmol) and thionyl chloride (0.3mL, 4mmol) were refluxed for 30min, followed by concentration under vacuum. The residue was dissolved in anhydrous *N*,*N*-dimethylacetamide (2mL), and to the resulting solution was added the product from **Part B** (30mg, 0.13mmol) and pyridine (0.025mL, 0.31mmol). The mixture was stirred at 80°C for 30min, and was partitioned between 1N HCl (5mL) and ethyl acetate (3 x 5mL). The organic extracts were combined, dried over Na₂SO₄, filtered and concentrated under vacuum to yield a crude product that was purified by column chromatography on silica gel eluting with 19:1 MeOH:CHCl₃ to give the title compound as a colorless solid (45mg, 72%). ¹H NMR (500 MHz, DMSO-D6) δ ppm 1.07 (s, 9 H), 2.69 (t, *J*=6.1 Hz, 2 H), 2.83 (s, 3 H) 3.33 - 3.38 (m, 5 H) 3.73 (s, 3 H) 6.92 (d, *J*=8.5 Hz, 2 H) 7.01 (d, *J*=9.2 Hz, 2 H) 7.06 (d, *J*=2.4 Hz, 1 H) 7.12 (d, *J*=2.4 Hz, 1 H) 9.52 - 9.73 (m, 1 H) 10.28 (s, 1 H).

### Example 25. Preparation of (N-(4-(3-tert-butyl-5-(3-(butyryloxymethyl)-2,4-dioxotetrahydro pyrimidin-1(2H)-yl)-2-methoxybenzamido)phenyl)methylsulfonamido)methyl butyrate (compound IA-L3-1.88).

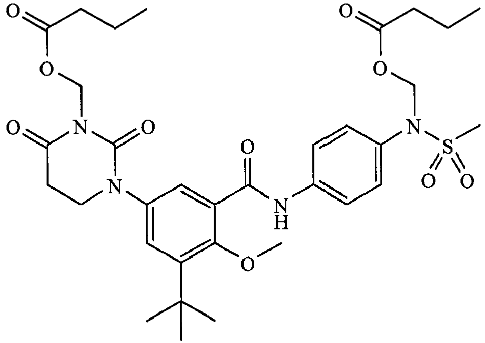

The product from **Example 22, Part G** (0.098g, 0.20mmole) was dissolved in DMSO (2ml) and treated with potassium carbonate (0.166g, 1.20mmole) and chloromethyl butyrate (0.411g, 3.0mmol). The mixture was stirred 20h at room temperature. The reaction mixture was partitioned with ethyl acetate and water. The organic layer was washed with brine and dried over anhydrous solid sodium sulfate. The drying agent was filtered and the solvent evaporated under vacuum. The residue was purified with silica gel eluting with ethyl acetate/hexane (10% to 80%) to give two major fractions. The first fraction was purified with silica gel eluting with methanol/dichloromethane (1% to 3%) to give the title compound as a foam (0.014, 10%). ¹H NMR (300 MHz, DMSO-D6) δ ppm 0.88 (m, 6 H) 1.38 (s, 9 H) 1.55 (m, 4 H) 2.26 (t, J=7.17 Hz, 2 H) 2.39 (t, J=7.17 Hz, 2 H) 2.95 (t, J=6.62 Hz, 2 H) 3.14 (s, 3 H) 3.77 (s, 3 H) 3.81 (t, J=6.62 Hz, 2 H) 5.57 (s, 2 H) 5.68 (s, 2 H) 7.35 (d, J=2.57 Hz, 1 H) 7.38 (d, J=2.94 Hz, 1 H) 7.42 (d, J=8.82 Hz, 2 H) 7.79 (d, J=8.82 Hz, 2 H) 10.60 (s, 1 H).

### Example 26. Preparation of (N-(4-(3-tert-butyl-5-(2,4-dioxotetrahydropyrimidin-1(2H)-yl)-2-methoxybenzamido)phenyl)methylsulfonamido)methyl butyrate (compound IA-L3-1.64).

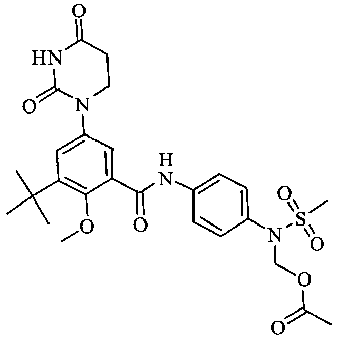

The product from **Example 22, Part G** (0.098g, 0.20mmole) was dissolved in DMSO (1ml) and treated with Cesium carbonate (0.209g, 0.64mmole) and bromomethyl acetate (0.123g, 0.80mmol). The mixture was stirred 4h at room temperature. The reaction mixture was partitioned with ethyl acetate and water. The organic layer was washed with brine and dried over anhydrous solid sodium sulfate. The drying agent was filtered and the solvent evaporated under vacuum. The residue was purified by HTP group by preparative HPLC on a Waters Nova-Pak® HR C18 6um 60Å Prep-Pak® cartridge column (40mm × 100mm). A gradient of acetonitrile (A) and 10mM ammonium acetate in water (B) was used, at a flow rate of 70mL/min (0-0.5min 10% A, 0.5-12.0min linear gradient 10-95% A, 12.0-15.0min 95% A, 15.0-17.0min linear gradient 95-10% A) to give the title compound as a white solid (0.034g, 30%). m.p. 229-230°C. ¹H NMR (300 MHz, DMSO-D6) δ ppm 1.38 (s, 9 H) 2.11 (s, 3 H) 2.72 (t, J=6.80 Hz, 2 H) 3.15 (s, 3 H) 3.78 (m, 5 H) 5.55 (s, 2 H) 7.30 (d, J=2.57 Hz, 1 H) 7.35 (d, J=2.57 Hz, 1 H) 7.42 (d, J=9.19 Hz, 2 H) 7.79 (d, J=8.82 Hz, 2 H) 10.36 (s, 1 H) 10.58 (s, 1 H).

**Example 27.** Preparation of *N*-(3-*tert*-butyl-5-(2,4-dioxotetrahydropyrimidin-1(*2H*)-yl)-2-methoxyphenyl)-4-(methylsulfonamido)benzamide (compound **IA-L4-1.9**).

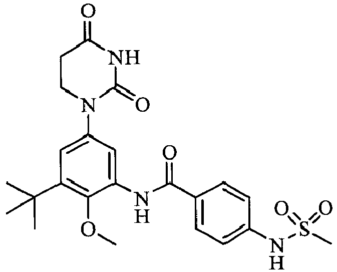

### Part A. Preparation of 2-tert-butyl-1 methoxy-4-nitrobenzene.

A 1:1 mixture of AcOH and fuming HNO₃ (0.6mL) was slowly added to a solution of 2-*tert-*butylphenol (1.0g, 6.6mmol) in cyclohexane (3mL) at 0°C. The resulting dark mixture was stirred at 0°C for 1h, followed by the addition of hexanes (5mL). The resulting solid was collected by filtration and washed with hexanes to give a light greenish solid (0.37g, 29%). The solid was dissolved in acetone (10ml), and to the resulting solution was added K₂CO₃ (0.3g, 2.2mmol), followed by the drop wise addition of Me₂SO₄ (0.27mL, 2.8mmol). The resulting mixture was stirred at room temperature overnight, and then poured into 1N HCl (20mL). The mixture was extracted with EtOAc (3x 20mL), dried over Na₂SO₄, filtered and concentrated to provide the title compound as an oil (0.4g, quant.).

### Part B. Preparation of 1-(3-tert-butyl-4-methoxyphenyl)dihydropyrimidine-2,4(1H,3H)-dione.

The product described in **Part A** was dissolved (0.4g, 1.9mmol) in EtOAc (10mL) and treated with 10% Pd on carbon (50mg). The mixture was stirred at ambient temperature under 1atm H₂ overnight. The mixture was filtered through celite and concentrated under vacuum to give a crude product that was purified on silica gel. The product was eluted using 1:1 EtOAc:hexanes, and isolated as an oil (0.23g, 68%). Acrylic acid (0.1mL, 1.46mmol) and toluene (10mL) were added to the isolated oil and the resulting mixture was heated at 100°C overnight, and then concentrated in vacuo to give a dark oil. The oil was treated with AcOH (5mL) and urea (0.2g, 3.3mmol), and the mixture was heated at 120°C for 6h. The mixture was cooled to ambient temperature, poured into water (20mL) and extracted with EtOAc (3 x 10mL). The combined organic layers were dried over Na₂SO₄, filtered and concentrated under vacuum to give a crude product that was purified by column chromatography on silica gel using 1:1 EtOAc:hexanes. The title compound was obtained as a colorless solid (0.144g, 41%).

### Part C. Preparation of 1-(3-tert-butyl-4-hydroxy-5-nitrophenyl)dihydropyrimidine-2,4(1H,3H)-dione.

The product prepared in **Part B** (1.00g, 3.62mmol) was dissolved in CH₂Cl₂ (25mL) at 0°C and treated with a 1M solution of BBr₃ in CH₂Cl₂ (18mL, 18mmol). The mixture was stirred at reflux overnight and poured into water (50mL). The mixture was extracted with 3:1 CH₂Cl₂:2-PrOH (2 x 50mL), and the combined extracts were dried overmgSO₄, filtered and concentrated under vacuum. The crude product was purified by column chromatography on silica using 2:1 EtOAc:hexanes to elute the product, obtained as a solid (0.60g, 63%). The solid was suspended in AcOH (20mL), to which was added fuming HNO₃ (0.105mL). The resulting solution was stirred at room temperature 1h and poured into ice water (100mL). The mixture was extracted with 3:1 CH₂Cl₂:2-PrOH (2 x 50mL), and the combined extracts were dried overmgSO₄, filtered and concentrated under vacuum. The residue was triturated with ether to give a solid that was collected by filtration (0.40 g, 57%).

### Part D. Preparation of 1-(3-amino-5-tert-butyl-4-methoxyphenyl)dihydropyrimidine-2,4(1H, 3H)-dione.

The product prepared in **Part C** (0.31g, 1.01mmol) was dissolved in 1:1 THF:MeOH (50mL) and treated with a 2M solution of trimethylsilyldiazomethane in THF (1.5mL, 3.0mmol). The resulting solution was stirred at ambient temperature overnight, and concentrated under vacuum. The crude product was purified by column chromatography on silica gel using 1:1 EtOAc:hexanes and a colorless solid (0.235g, 72%) was obtained. The solid was dissolved in 1:1 CH₂Cl₂:MeOH (50mL), treated with 10% Pd/C (25mg), and the mixture was stirred at ambient temperature under 1atm H₂ for 2h. The mixture was filtered through celite and concentrated under vacuum to obtain the title compound (0.215g, quant.).

### Part E. Preparation of N-(3-tert-butyl-5-(2,4-dioxotetrahydropyrimidin-1(2H)-yl)-2-methoxyphenyl)-4-nitrobenzamide.

The product obtained in **Part D** (0.215g, 0.74mmol) was dissolved in anhydrous CH₂Cl₂ (50mL) and treated with 4-nitrobenzoyl chloride (0.164g, 0.88mmol) and pyridine (0.07mL, 0.88mmol). The resulting mixture was stirred at ambient temperature overnight, washed with water (50mL), dried over Na₂SO₄, filtered and concentrated under vacuum. The crude product was purified by column chromatography on silica gel using 1:1 EtOAc:hexanes to give the title compound (0.26g, 80%).

### Part F. Preparation of N-(3-tert-butyl-5-(2,4-dioxotetrahydropyrimidin-1(2H)-yl)-2-methoxyphenyl)-4-(methylsulfonamido)benzamide.

The product obtained in **Part E** (0.26g, 0.59mmol) was dissolved in a 2:1 mixture of CH₂Cl₂:MeOH (6mL), treated with 10% Pd on carbon (30mg) and stirred at ambient temperature under 1atm H₂ for 2h, filtered through celite and concentrated under vacuum. The residue was dissolved in anhydrous CH₂Cl₂ (10mL) treated with methanesulfonyl chloride (0.054mL, 0.70mmol) and pyridine (0.056mL, 0.70mmol). The resulting mixture was stirred at room temperature overnight, partitioned between water (20mL) and 3:1 CH₂Cl₂:2-PrOH (3 x 20mL). The combined organic layers were dried over Na₂SO₄, filtered and concentrated under vacuum. The crude product was purified by column chromatography on silica gel using 19:1 CH₂Cl₂:MeOH to give the title compound as a solid (0.12g, 42%). ¹H NMR (300 MHz, DMSO-D6) δ ppm 1.37 (s, 9 H), 2.70 (t, *J*=6.62 Hz, 2 H), 3.10 (s, 3 H), 3.71 (s, 3 H), 3.76 (t, *J*=6.62 Hz, 2 H), 7.11 (d, *J*=2.57 Hz, 1 H) 7.30 (d, *J*=8.82 Hz, 2 H) 7.37 (d, *J*=2.57 Hz, 1 H), 8.02 (d, *J*=8.82 Hz, 2 H), 9.86 (s, 1 H) 10.20 (s, 1 H) 10.33 (s, 1 H).

### Example 28. Preparation of N-(3-tert-butyl-5-(2,4-dioxotetrahydropyrimidin-1(2H)-yl)-2-methoxyphenyl)-4-(methylsulfonylmethyl)benzamide (compound IA-L4-1.10).

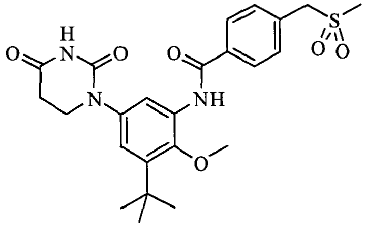

Thionyl chloride (0.31mL, 4.2mmol) and 4-(methylsulfonylmethyl)benzoic acid (0.03g, 0.14mmol) were combined and heated at 85°C for 30min then concentrated to dryness. This acid chloride was dissolved in N, N- dimethylacetamide (2mL) with the product from **Example 27, Part D** (0.041g, 0.14mmol) and pyridine (0.025mL, 2.2mmol) and heated at 100°C for 20min, then cooled to ambient temperature and diluted with 1M HCl. The solid precipitate was isolated by filtration, triturated with methanol and dried to give the title compound) the title compound (0.0175g, 26%). ¹H NMR (300 MHz, DMSO-D6) 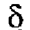 ppm 1.37 (s, 9 H), 2.71 (t, J=6.62 Hz, 2 H), 2.95 (s, 3 H), 3.72 (s, 3 H), 3.77 (t, J=6.62 Hz, 2 H), 4.60 (s, 2 H), 7.13 (d, J=2.94 Hz, 1 H), 7.38 (d, J=2.57 Hz, 1 H), 7.56 (d, J=8.09 Hz, 2 H) 8.05 (d, J=8.09 Hz, 2 H), 10.03 (s, 1 H), 10.33 (s, 1 H).

**Example 29.** Preparation of (E)-N-(4-(3-tert-butyl-5-(2,4-dioxotetrahydropyrimidin-1(2H)-yl)-2-methoxystyryl)phenyl)methanesulfonamide (compound **IA-L1-1.9**).

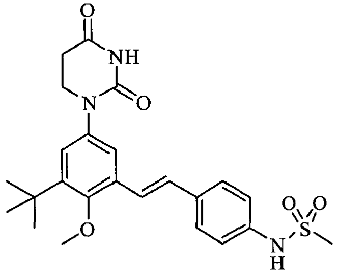

### Part A. Preparation of methyl 3-tert-butyl-2-hydroxy-5-nitrobenzoate.

Methyl 3,5-di-tert-butyl-2-hydroxybenzoate (28.66g, 108.4mmol) was dissolved with stirring in 430mL glacial acetic acid and the resulting mixture was treated drop wise with fuming nitric acid (90%, 179.26mL). When the addition was complete, the resulting mixture was stirred for 2.5h. The reaction mixture was poured into a 2.0L of crushed ice and allowed to stand 30min. Afterwards, 1.0L of water was added and the ice water mixture was allowed to melt. The mixture was then filtered, washed with water and dried to provide the title compound (24.57g, 89%).

### Part B. Preparation of methyl 3-tert-butyl-2-methoxy-5-nitrobenzoate.

Methyl 3-tert-butyl-2-hydroxy-5-nitrobenzoate (11.41g, 45.0mmol), potassium carbonate (9.34g, 67.6mmol), acetone (200mL), and dimethyl sulfate (6.46g, 67.6mmol) were added together. The resultant mixture was then heated to reflux for 16h. The mixture was then filtered and the solid was washed with ethyl acetate. The resulting organic liquid was then concentrated under vacuum to an oil and redissolved in ethyl acetate (600mL). The organic solution was then washed with water, dried, filtered and concentrated under vacuum to an oil that was then subjected to purification via column chromatography (gradient of 5% to 40% EtOAc/Hexanes) to yield the title compound as an oil (10.42, 87%).

### Part C. Preparation of methyl 5-amino-3-tert-butyl-2-methoxybenzoate.

Methyl 3-tert-butyl-2-methoxy-5-nitrobenzoate (10.42g, 39.0mmol), iron powder (325mesh, 10.89g, 195mmol), ammonium chloride (3.13g, 58.5mmol), water (30mL), and methanol (150mL) were added together. The resultant mixture was then refluxed for 1h. The mixture was then cooled to room temperature, filtered through celite, and the celite washed with methanol. The filtrate was then concentrated under vacuum and dissolved in ethyl acetate (600mL). The resultant solution was then washed with water and brine. The organic extract was then dried, filtered and concentrated under vacuum to yield the title compound as an oil (9.25g, 100%).

### Part D. Preparation of 3-(3-tert-butyl-4-methoxy-5-(methoxycarbonyl)phenylamino) propanoic acid.

The product from **Part C** (16.44g, 69.3mmol) was dissolved in toluene (200mL). This mixture was heated to reflux and acrylic acid added over time (1mL of acrylic acid added every 3h, 5.23mL total, 76.2mmol). The mixture was then refluxed for 24h. The mixture was then cooled and concentrated under vacuum to dryness to yield an oil as the crude title compound that was used directly in the next reaction.

### Part E. Preparation of methyl 3-tert-butyl-5-(2,4-dioxotetrahydropyrimidin-1(2H)-yl)-2-methoxybenzoate.

The product from **Part D** (21.43g, 69.3mmol), urea (10.4g, 173mmol) and acetic acid (glacial, 200mL) were added together. The mixture was then heated to 120°C for 18.5h followed by concentration under vacuum to give an oil. To this oil was added methanol (13mL), and ethyl acetate (350mL). The resultant mixture was allowed to stand for 24-48h whereby a precipitate formed. The resulting solid was filtered off and washed with a small amount of methanol (10mL) and then air dried to yield the title compound as a solid (15.26g, 66%).

### Part F. Preparation of 3-tert-butyl-5-(2,4-dioxotetrahydropyrimidin-1(2H)-yl)-2-methoxy benzoic acid.

The product from **Part D** (4.52g, 13.52mmol), methanol (70mL), and tetrahydrofuran (70mL) were added together. The mixture was then stirred vigorously until a homogenous solution resulted. Once homogenous, a solution of aqueous sodium hydroxide (1.0M, 68mL) was added. The mixture was then stirred for 12h, the mixture was then concentrated under vacuum to remove the organic solvent, followed by the addition of aqueous hydrochloric acid (1.0M, 80mL) that resulted in solid formation. The mixture was then concentrated under vacuum. To this material was added hydrochloric acid (12M, 100mL) and the resultant material heated to 100°C for 1.5h. The reaction was then cooled and water added. The resulting solid was filtered, washed with water, and dried to yield the title compound as a solid (3.55g, 82%).

### Part G. Preparation of 3-tert-butyl-5-(2,4-dioxotetrahydropyrimidin-1(2H)-yl)-2-methoxybenzaldehyde.

The product obtained in **Part F** (4.07g, 12.71mmol) and thionyl chloride (40.82mL, 559mmol) were combined and the mixture was refluxed for 2h, followed by concentration under vacuum to provide a light yellow colored solid product. The solid was dissolved in tetrahydrofuran (125mL), the solution cooled to -78°C and LiAlH(O*t*Bu)₃ (1M, 14mL) was added slowly over 10min while maintaining the temperature at -78°C. The mixture was stirred at -78°C for 2h, and the reaction was quenched with hydrochloric acid (aq., 1M, 25mL) at -78°C. The mixture was warmed to room temperature and ethyl acetate was added. The layers were separated and the aqueous layer was washed with ethyl acetate. The organic extracts were combined and washed with half saturated sodium bicarbonate solution. The organic layer was dried, filtered and concentrated under vacuum to yield the title compound as a solid (3.73g, 96%).

### Part H. Preparation of 1-(3-tert-butyl-4-methoxy-5-(4-nitrostyryl)phenyl)dihydro- pyrimidine-2,4(1H,3H)-dione.

The product prepared in **Part G** (1.00g, 3.29mmol) and diethyl 4-nitrobenzyl- phosphonate (0.853g, 3.12mmol) were dissolved in dichloromethane (50mL). Solid potassium tert-butoxide (0.737g, 6.57mmol) was added portion wise at room temperature. The resultant dark red solution was stirred for 1.5h at room temperature. 1N aqueous HCl (50mL) solution was added and the mixture was stirred 30min, and then diluted with dichloromethane (50mL). The resultant organic layer was separated and dried. The material was purified by column chromatography on silica gel using 99/1 dichloromethane/methanol as eluent to obtain the title compound as a solid (1.12g, 80%).

### Part I. Preparation of Preparation of (E)-N-(4-(3-tert-butyl-5-(2,4-dioxotetrahydro- pyrimidin-1(2H)-yl)-2-methoxystyryl)phenyl)methanesulfonamide.

The product obtained in **Part H** (1.1g, 2.60mmol), iron (0.725g, 12.99mmol), and ammonium chloride (0.208g, 3.90mmol) was added to a mixture of tetrahydrofuran (40mL), ethanol (40mL) and water (12mL). The slurry was heated to 90°C for 45min, and then cooled to ambient temperature. The solution was filtered through a pad of celite (10g), washed with ethanol (20mL), and the filtrate concentrated under vacuum to a solid. The resulting solid was dissolved in ethyl acetate (100mL), and the solution was washed with water (50mL) and dried over Na₂SO₄. The drying agent was filtered off and the solvent removed under vacuum to give the aniline adduct as a yellow solid (830mg).

The solid (830mg, 2.109mmol) was dissolved in dichloromethane (50mL), and pyridine (0.512mL, 6.33mmol) and methanesulfonyl chloride (0.181mL, 2.32mmol) were added and the resulting solution was stirred at room temperature 16h. Dichloromethane (100mL) was added followed by extraction with a 1N aq. HCl solution (2 x 50mL). The organic layer was dried, concentrated under vacuum and purified by column chromatography on silica gel using 98/2 CH₂Cl₂/MeOH to provide the title compound as a solid (480mg, 39%, two steps). m.p. = 260-261°C (trans-isomer) ¹H NMR (500 MHz, DMSO-d₆): δ ppm 1.37 (s, 9H), 2.71 (t, J=6.7Hz, 2H), 3.01 (s, 3H), 3.75 (s, 3H), 3.79 (t, J=6.6Hz, 2H), 7.13 (d, J=16.5Hz, 1H), 7.15 (d, J=2.4Hz, 2H), 7.23 (d, J=8.5Hz, 2H), 7.25 (d, J=16.5 Hz, 1H), 7.51 (d, J=2.4Hz, 1H), 7.61 (d, J=8.6Hz, 2H), 9.80(bs, 1H), 10.30 (s, 1H). (trans-isomer).

### Example 30. Preparation of (Z)-N-(4-(2-(3-tert-butyl-5-(2,4-dioxotetrahydropyrimidin-1(2H)-yl)-2-methoxyphenyl)-I-chlorovinyl)phenyl)methanesulfonamide (compound IA-L1-1.3).

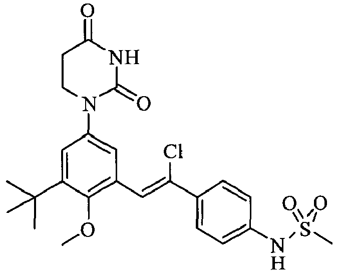

### Part A. Preparation of diethyl hydroxy(4-nitrophenyl)methylphosphonate.

The title compound was prepared as described in Taylor, WP, et. Al, Bioorg. Med. Chem. 4:1515-1520 (1996). 4-Nitrobenzaldehyde (3.0g, 19.85mmol) and diethyl phosphonate (2.74g, 19.85mmol) were combined and treated with a 0.5N solution of sodium methoxide in methanol (0.993mL, 0.496mmol). The resulting red-orange solution was stirred 12h at room temperature. The reaction mixture was extracted with dichloromethane (20mL) followed by half saturated ammonium chloride (20mL). The organic layer was separated, dried and concentrated under vacuum to provide the title compound as a semi-solid (5.1g, 89%).

### Part B. Preparation of diethyl chloro(4-nitrophenyl)methylphosphonate.

The product prepared in **Part A** (500mg, 1.729mmol) was dissolved in dichloromethane (10mL) and treated with triphenylphosphine (998mg, 3.80mmol), followed by N-chlorosuccinimide (462mg, 3.46mmol). The mixture was stirred at room temperature for 18h. The solution was concentrated under vacuum and the residue was purified by column chromatography using silica gel eluting with a 1/1 mixture of hexanes/ethyl acetate to provide the title compound as an oil (262mg, 49%).

### Part C. Preparation of (Z)-N-(4-(2-(3-tert-butyl-5-(2,4-dioxotetrahydropyrimidin-1(2H)-yl)-2-methoxyphenyl)-1-chlorovinyl)phenyl)methanesulfonamide.

The product prepared in **Example 29**, **Part G** (100mg, 0.329mmole) was treated with the product obtained from **Part B** using the procedures described in **Example 29, Part H** and **Example 29**, **Part I** to provide 39mg of the title compound. ¹H NMR (300 MHz, DMSO-d₆): δ ppm 1.36 (s, 9H), 2.71 (t, J=6.8Hz, 2H), 3.06 (s, 3H), 3.71 (s, 3H), 3.78 (t, J=6.8Hz, 2H), 7.23 (d, J=2.6 Hz, 1H), 7.27 (s, 1H), 7.28 (d, J=8.6Hz, 2H), 7.48 (d, J=2.6 Hz, 1H), 7.78 d, J=8.8 Hz, 1H), 10.05(s, 1H), 10.34 (s, 1H).

**Example 31.** Preparation of (E)-1-(3-tert-butyl-5-(4-fluorostyryl)-4-methoxyphenyl) dihydropyrimidine-2,4(1H,3H)-dione (compound **IA-L1-1.12**).

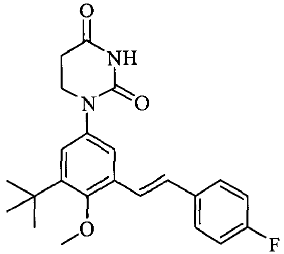

The title compound was prepared according the procedures described in **Example 29**, **Part H** and **Example 29**, **Part I** using the product obtained in **Example 29**, **Part G** (50mg, 0.164mmol) and diethyl 4-fluorobenzylphosphonate (40.5mg, 0.164mmol). The title compound was obtained as a solid (30mg, 46%). ¹H NMR (300 MHz, DMSO-d₆): δ ppm 1.37 (s, 9H), 2.72 (t, J=6.6Hz, 2H), 3.76 (s, 3H), 3.79 (t, =6.6Hz, 2H), 7.21 (m, 4H), 7.30 (d, J=16.3Hz, 1H), 7.53 (d, J=2.6Hz, 1H), 7.73 (m, 2H), 10.35 (s, 1H).

### Example 32. Preparation of (Z)-N-(4-(2-(3-tert-butyl-5-(2,4-dioxotetrahydropyrimidin-1(2H)-yl)-2-methoxyphenyl)-1-fluorovinyl)phenyl)methanesulfonamide (compound IA-L1-1.4).

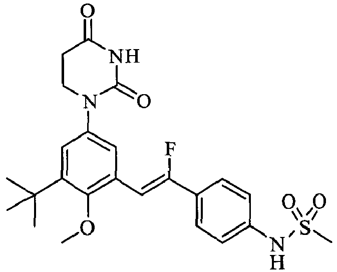

### Part A. Preparation of diethyl fluoro(4-nitrophenyl)methylphosphonate.

The title compound was prepared as described in Taylor, WP, et. Al, Bioorg. Med. Chem. 4:1515-1520 (1996). The product from **Example 30, Part A** (500mg, 1.729mmol) was dissolved in dichloromethane (10mL) and treated by drop wise addition of (diethylamino)sulfur trifluoride (DAST) (2.5mL, 18.9mmol). The mixture was stirred at room temperature for 18h. A solution of half saturated sodium phosphate monobasic (20mL) was added followed by dichloromethane (20mL) addition and separation of the resulting organic phase. The organic solution was dried and concentrated under vacuum, and then subjected to column chromatography using silica gel eluting with a 1/1 mixture of hexanes/ethyl acetate to provide the title compound as an oil (215mg, 43%).

### Part B. Preparation of (Z)-N-(4-(2-(3-tert-butyl-5-(2,4-dioxotetrahydropyrimidin-1(2H)-yl)-2-methoxyphenyl)-1-fluorovinyl)phenyl)methanesulfonamide.

The product prepared as described in **Part A** (100mg, 0.329mmole) was treated with the product prepared in **Example 29**, **Part G** (96mg, 0.329mmole) according to the procedures described in **Example 29, Part H and Example 29, Part I** to provide 53mg of the title compound as a 1/1 mixture of cis/trans isomers. Reverse phase HPLC chromatographic separation using a 40-100% gradient of acetonitrile in 0.1% aqueous trifluoroacetic acid provided the title compound as a solid (20mg). ¹H NMR (300 MHz, DMSO-d₆): δ ppm 1.37 (s, 9H), 2.71 (t, J=6.8Hz, 2H), 3.06 (s, 3H), 3.77 (s, 3H), 3.78 (m, 2H), 6.62 (d, J=40.4Hz, 1H), 7.18 (d, J=2.6Hz, 1H), 7.30 (d, J=8.4Hz, 2H), 7.55 (d, J=2.6Hz, 1H), 7.75 (d, J=8.8Hz, 2H), 10.08 (s, 1H), 10.33 (s, 1H).

### Example 33. Preparation of (E)-N-(4-(2-(3-tert-butyl-5-(2,4-dioxotetrahydropyrimidin-1(2H)-yl)-2-methoxyphenyl)-1-fluorovinyl)phenyl)methanesulfonamide (compound IA-L1-1.5).

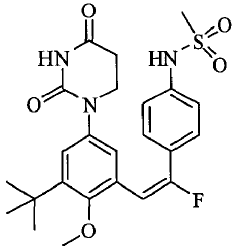

Reverse phase HPLC chromatographic separation of the 1/1 mixture of cis/trans isomeric material (53mg) from **Example 32**, **Part A** using a 40-100% gradient of acetonitrile in 0.1% aqueous trifluoroacetic acid provided the title compound as a solid (16.5mg). ¹H NMR (300 MHz, DMSO-d₆): δ ppm 1.33 (s, 9H), 2.60 (t, J=6.6Hz, 2H), 3.01 (s, 3H), 3.57 (t, J=6.6Hz, 2H) 3.79 (s, 3H), 6.46 (d, J=21.3Hz, 1H), 6.87 (d, J=2.2Hz, 1H), 7.14 (m, 3H), 7.36 (d, J=8.8Hz, 2H), 10.02 (s, 1H), 10.24 (s, 1H).

### Example 34. Preparation of (E)-N-(4-(3-tert-butyl-5-(2,4-dioxotetrahydropyrimidin-1(2H)-yl)-2-methoxystyryl)-2-fluorophenyl)methanesulfonamide (compound IA-L1-1.26)

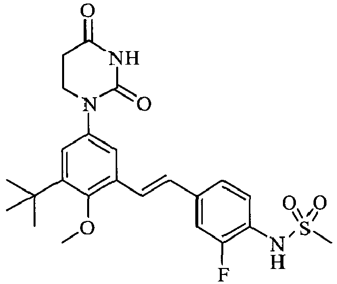

### Part A. Preparation of 4-(bromomethyl)-2-fluoro-1-nitrobenzene.

(3-Fluoro-4-nitrophenol)methanol (1.24g, 7.25mmol) was dissolved in dichloromethane (25mL) and treated with triphenylphosphine (2.281g, 8.70mmol) followed by N-bromosuccinimide (1.548g, 8.70mmol). The mixture was stirred at room temperature for 2h. Water (50mL) and dichloromethane (40mL) were added, and the organic layer was separated and dried. The solution was concentrated under vacuum and purified by column chromatography using silica gel eluting with a 5/1 mixture of hexanes/ethyl acetate to provide the title compound as a solid (1.27g, 75%).

### Part B. Preparation of diethyl 3-fluoro-4-nitrobenzylphosphonate.

The product prepared in **Part A** (1.27g, 5.43mmol) was added to triethyl phosphite (8mL, 54.3mmol) and the solution heated to 120°C for 1hr. After cooling, the excess triethyl phosphite was removed by heating under vacuum and the residue subjected to column chromatography on silica gel using 99/1 dichloromethane/methanol as eluent to obtain the crude title compound as an oil (800mg).

### Part C. Preparation of (E)-N-(4-(3-tert-butyl-5-(2,4-dioxotetrahydropyrimidin-1(2H)-yl)-2-methoxystyryl)-2-fluorophenyl)methanesulfonamide.

The product described in **Example 29, Part G** (533mg, 1.751mmole) was treated with the product described in **Part B** (510mg, 1.751mmole) according to the procedures described in **Example 29**, **Part H** and **Example 29, Part I** to provide 80mg of the title compound. ¹H NMR (300 MHz, DMSO-d₆): δ ppm 1.37 (s, 9H), 2.71 (t, J=6.5Hz, 2H), 3.05 (s, 3H), 3.76 (s, 3H), 3.79 (t, J=6.6Hz, 2H), 7.18 (m, 2H), 7.36 (d, J=16.5Hz, 1H), 7.39 (m, 1H), 7.44 (m, 1H), 7.52 (d, J=2.6Hz, 1H), 7.63 (m, 1H), 9.65 (s, 1H), 10.35 (s, 1H).

### Example 35. Preparation of N-(4-(2-(3-tert-butyl-5-(2,4-dioxotetrahydropyrimidin-1(2H)-yl)-2-methoxyphenyl)cyclopropyl)phenyl)methanesulfonamide (compound IA-L8-1.1).

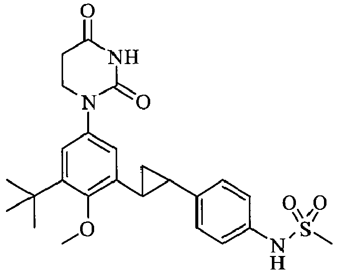

The product obtained as described in **Example 29, Part I** (30mg, 0.064mmol) was dissolved in tetrahydrofuran (2mL) and treated with 0.95mL of a 0.67M ether solution of diazomethane (0.636mmol) followed by palladium acetate (0.7mg, 0.0031mmol). The mixture was stirred for 30 min at room temperature followed by removal of the solid by filtration and concentration of the filtrate. The filtrate was purified by column chromatography on silica gel using 98/2 dichloromethane/methanol as eluent to obtain the title compound as a solid (21.6mg, 70%). m.p. 265-266°C. ¹H NMR (300 MHz, DMSO-d₆): δ ppm 1.33 (s, 9H) 1.50 (m, 2H), 2.13 (m, 1H), 2.27 (m, 1H), 2.69 (t, J=6.6Hz, 2H), 2.94 (s, 3H), 3.63 (s, 3H), 3.74 (t, J=6.6Hz, 2H),6.84 (d, J=2.6Hz, 1H), 7.04 (d, J=2.6Hz, 1H), 7.14 (d, J=8.8Hz, 2H), 7.20 (d, J=8.8Hz, 2H), 9.60 (s, 1H),10.29 (s, 1H).

### Example 36. Preparation of N-(4-(3-tert-butyl-5-(2,4-dioxotetrahydropyrimidin-1(2H)-yl)-2-methoxyphenethyl)phenyl)methanesulfonamide (compound IA-L5-2-1.1).

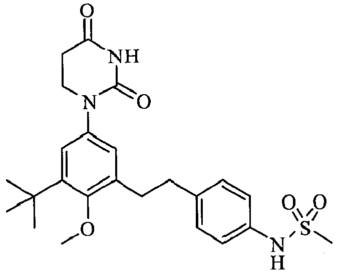

The product obtained as described in **Example 29, Part I** (415mg, 0.88mmol) was dissolved in methanol (30mL) and treated with 50mg of 10% palladium on carbon. The slurry was stirred for 48h at room temperature under 1atm of hydrogen. The reaction mixture was filtered through celite and concentrated in vacuo to provide the title compound as a solid (230mg, 55%). m.p. 233-234°C. ¹H NMR (300 MHz, DMSO-d₆): δ ppm 1.34 (s, 9H), 2.68 (t, J=6.8Hz, 2H), 2.86 (s, 4H), 2.93 (s, 3H), 3.70 (m, 2H), 3.74 (s, 3H), 7.11 (m, 4H), 7.23 (m, 2H), 9.59 (s, 1H), 10.29 (s,).

### Example 37. Preparation of (E)-N-(4-(3-tert-butyl-5-(2,4-dioxotetrahydropyrimidin-1(2H)-yl)styryl)phenyl)methanesulfonamide (compound IA-L1-1.16)

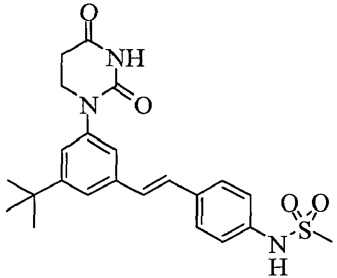

### Part A. Preparation of methyl 3-tert-butyl-5-(chlorocarbonyl)benzoate.

A mixture of 3-tert-butyl-5-(methoxycarbonyl)benzoic acid (9.18g, 38.9mmol, prepared by the method of Carter et. al., WO2005021500A1), thionyl chloride (75mL) and 1 drop of DMF in toluene (200mL) was heated at reflux for 2h, cooled and concentrated. The residue was azeotroped with toluene (3 x 50mL )and dried under high vacuum to give the title compound as an off-white waxy solid (9.9g, quantitative yield).

### Part B. Preparation of methyl 3-(azidocarbonyl)-5-tert-butylbenzoate.

To the product of **Part A** (9.9g, 38.9mmol) in acetone (200ml) was added at a fast drip a solution of sodium azide (10.12g, 156mmol) dissolved in water (20mL). The mixture was stirred for 2h and diluted with EtOAc. The organic layer was washed with H₂O, saturated brine, dried (Na₂SO₄), filtered and concentrated to give the title compound as a white solid (9.9g, 97%).

### Part C. Preparation of methyl 3-amino-5-tert-butylbenzoate.

The product from **Part B** (9.9g, 37.9mmol) in toluene (100mL) was heated at reflux for 1h and concentrated to give the intermediate isocyanate which was dissolved in DME (60mL) treated with 8% HCl (150mL) and stirred for 16h. The mixture was concentrated and the residue was dissolved in water, neutralized with solid sodium bicarbonate and extracted 3 x 100mL with EtOAc. The organics were combined, washed with saturated NaCl, dried (Na₂SO₄), filtered and concentrated. The crude product was chromatographed on silica eluting with 2:1 hexane/EtOAc to give the title compound as an oil (2.7g, 35%).

### Part D. Preparation of methyl 3-tert-butyl-5-(2,4-dioxotetrahydropyrimidin-1(2H)-yl)-2-methoxybenzoate.

A mixture of the product of **Part C** (2.34g, 11.29mmol) and acrylic acid (2.32ml, 33.9mmol) in toluene (60ml) was heated at reflux under nitrogen for 24h, cooled and concentrated. The resulting residue was then treated with urea (2.03g, 33.9mmol) in acetic acid (35ml) and heated at 120°C for 24h, cooled and concentrated. The residue was azeotroped 3 x 50mL with toluene and dissolved in 100mL of EtOAc. The organic layer was washed with dilute aqueous NaHCO₃, H₂O, saturated brine, dried (Na₂SO₄), filtered and concentrated to give the title compound as a white solid (2.1g, 61 %).

### Part E. Preparation of 3-tert-butyl-5-(2,4-dioxotetrahydropyrimidin-1(2H)-yl)benzoic acid.

A mixture of the product from **Part D** (1.8g, 5.91mmol) and 1M NaOH (29.6ml, 29.6mmol) in MeOH (15ml) and THF (15mL) was stirred for 24h and concentrated. The residue was treated with 50mL of 1M HCl and extracted into EtOAc. The EtOAc layer was washed with H₂O, saturated brine, dried (Na₂SO₄), filtered and concentrated to give a white solid. This intermediate urea was combined with 20mL of concentrated HCl and heated at 100°C for 1h, cooled and diluted with 75mL of ice water to give a white powder which was collected by filtration and dried to constant mass to give the title compound (1.6g, 93%).

### Part F. Preparation of (E)-N-(4-(3-tert-butyl-5-(2,4-dioxotetrahydropyrimidin-1(2H)-yl) styryl)phenyl)methanesulfonamide.

The product described in **Part E** was treated with thionyl chloride and lithium tri-tert-butoxyaluminum hydride according to procedures described in **Example 29, Part G** to produce 3-tert-butyl-5-(2,4-dioxotetrahydropyrimidin-1(2H)-yl)benzaldehyde. The aldehyde was treated with diethyl 4-nitrobenzylphosphonate according the procedures described in **Example 29, Part H** and **Example 29, Part I** to provide the title compound (85mg). ¹H NMR (300 MHz, DMSO-d₆): δ ppm 1.32 (s, 9 H) 2.72 (t, J=6.43 Hz, 2 H) 3.01 (s, 3 H) 3.82 (t, J=6.62 Hz, 2 H) 7.18 - 7.25 (m, 5 H) 7.39 (s, 1 H) 7.46 (s, 1 H) 7.58 (d, J=8.46 Hz, 2 H) 9.84 (s, 1 H) 10.37 (s, 1 H).

### Example 38. Preparation of (Z)-N-(4-(2-(3-tert-butyl-5-(2,4-dioxotetrahydropyrimidin-1(2H)-yl)-2-methoxyphenyl)-1-methoxyvinyl)phenyl)methanesulfonamide (compound IA-L1-1.17).

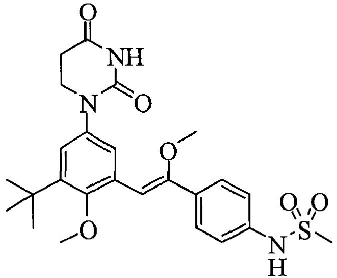

### Part A. Preparation of 1-(dimethoxymethyl)-4-nitrobenzene.

A flask equipped with a magnetic stir bar and vigreux column was charged with 4-nitro-benzaldehyde (5.0g, 33.1mmol), pyridiniump-toluenesulfonate (1.66g, 6.62mmol), trimethoxymethane (3.51g, 33.1mmol) and methanol (100mL). The mixture was heated at 50°C for 12h and was concentrated in vacuo. The residue was redissolved in EtOAc and washed with aq. NaOH (1M), H₂O and brine. The mixture was dried (Na₂SO₄), filtered and concentrated in vacuo to yield the title compound as a clear, light yellow oily product (6.3 6g , 97%).

### Part B. Preparation of diethyl methoxy(4-nitrophenyl)methylphosphonate.

The product from **Part A** (3.0g, 15.2mmol) and triethyl phosphite (2.53g, 15.2mmol) were dissolved in dichloromethane (30mL) under a nitrogen atmosphere, cooled to -20°C and treated with drop wise addition of boron trifluoride etherate (2.27g, 16mmol). The mixture was allowed to slowly warm to room temperature overnight with stirring. Water was added and the resulting mixture was stirred 5min, separated and the organic layer was dried (Na₂SO₄), filtered and concentrated in vacuo to a solid residue. The residue was purified on silica gel (100% EtOAc to 3% CH₃OH/EtOAc) to yield the title compound as a light yellow oily product (3.78 g , 82%).

### Part C. Preparation of (Z)-N-(4-(2-(3-tert-butyl-5-(2,4-dioxotetrahydropyrimidin-1(2H)-yl)-2-methoxyphenyl)-1-methoxyvinyl)phenyl)methanesulfonamide.

The product obtained according to the procedure described in **Example 29, Part G** (400mg, 1.314mmole) was treated with the product obtained in **Part B** (399mg, 1.314mmole) according to the procedures described in **Example 29, Part H** and **Example 29, Part I** to provide the title compound (17mg, 6%). ¹H NMR (300 MHz, DMSO-d₆): 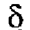 ppm 1.36 (s, 9 H) 2.71 (t, J=6.62 Hz, 2 H) 3.05 (s, 3 H) 3.58 (s, 3 H) 3.75 (s, 3 H) 3.76 - 3.81 (m, 2 H) 6.25 (s, 1 H) 7.11 (d, J=2.57 Hz, 1 H) 7.27 (d, J=8.46 Hz, 2 H) 7.60 (d, J=8.82 Hz, 2 H) 7.67 (d, J=2.57 Hz, 1 H) 9.96 (s, 1 H) 10.32 (s, 1 H).

### Example 39. Preparation of (E)-1-(3-tert-butyl-4-methoxy-5-styrylphenyl)dihydro-pyrimidine-2,4(1H,3H)-dione (compound IA-L1-1.18).

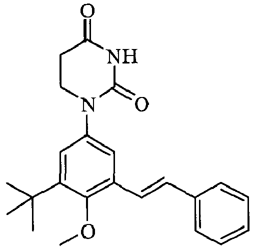

The product obtained according to procedure described in **Example 29, Part G** (50mg, 0.164mmole) was treated with diethyl benzylphosphonate (0.034ml, 0.164mmole) according to the procedure described in **Example 29, Part H** to provide the title compound (13mg, 19%). ¹H NMR (300 MHz, DMSO-d₆): δ ppm 1.37 (s, 9 H) 2.72 (t, J=6.62 Hz, 2 H) 3.76 (s, 3 H) 3.80 (t, J=6.80 Hz, 2 H) 7.16 - 7.18 (m, 1 H) 7.21 - 7.23 (m, 1 H) 7.29 - 7.33 (m, 2 H) 7.36 - 7.43 (m, 2 H) 7.54 (d, J=2.57 Hz, 1 H) 7.64 (d, J=7.35 Hz, 2 H) 10.35 (s, 1 H).

### Example 40. Preparation of (E)-1-(3-tert-butyl-4-methoxy-5-(4-methoxystyryl)phenyl) dihydropyrimidine-2,4(1H,3H)-dione (compound IA-L1-1.14).

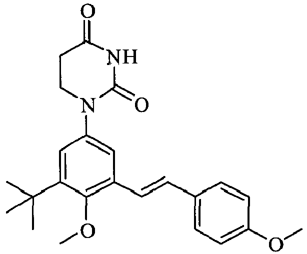

The product obtained according to procedure described in **Example 29, Part G** (50mg, 0.164mmole) was treated with diethyl 4-methoxybenzylphosphonate (0.028ml, 0.164mmole) according to the procedure described in **Example 29, Part H** to provide the title compound (4mg, 4%). ¹H NMR (300 MHz, DMSO-d₆): δ ppm 1.37 (s, 9 H) 2.71 (t, J=6.62 Hz, 2 H) 3.70 - 3.81 (m, 8 H) 6.96 (d, J=8.82 Hz, 2 H) 7.13 (d, J=2.21 Hz, 1 H) 7.15 (d, J=2.57 Hz, 2 H) 7.50 (d, J=2.57 Hz, 1 H) 7.58 (d, J=8.46 Hz, 2 H) 10.34 (s, 1 H).

### Example 41A. Preparation of (E)-N-(4-(3-tert-butyl-5-(2,4-dioxo-3,4-dihydropyrimidin-1(2H)-yl)-2-methoxystyryl)phenyl)methanesulfonamide (compound IB-L1-1.1).

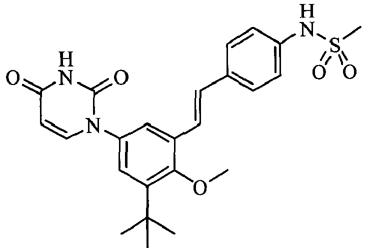

### Part A. Preparation of (E)-methyl 3-tert-butyl-2-methoxy-5-(3-(3-methoxyacryloyl)ureido) benzoate.

The product obtained as described in **Example 29, Part C** (2.0g, 8.43mmol) was dissolved in 30mL of N,N-dimethylacetamide and cooled to -25°C. A 0.5Molar solution of E-3-methoxyacryloyl isocyanate in benzene (21.9mL, 10.96mmol) was added drop wise and the resulting solution was stirred at ambient temperature for 4h, and then poured into water. The product was extracted into dichloromethane, washed with brine, dried over sodium sulfate, filtered and evaporated under vacuum to give the title compound.

### Part B. Preparation of methyl 3-tert-butyl-5-(2,4-dioxo-3,4-dihydropyrimidin-1(2H)-yl)-2-methoxybenzoate.

The product from **Part A** (3.1g, 8.51mmol) was dissolved in ethanol (60mL). To this solution was added a mixture of concentrated sulfuric acid (6mL) and water (60mL). The heterogeneous mixture was heated at 100°C for 3h. The ethanol was removed under vacuum, and then the aqueous solution was extracted with dichloromethane and evaporated to dryness. This residue was purified by column chromatography on silica gel, eluting with 1% methanol/dichloromethane to yield the title compound (1.23g, 44%).

### Part C. Preparation of 3-tert-butyl-5-(2,4-dioxo-3,4-dihydropyrimidin-1(2H)-yl)-2-methoxy benzoic acid.

The product from **Part B** (1.23g, 3.7mmol) was taken up in ethanol (5mL) and 1M sodium hydroxide solution (10mL and stirred at ambient temperature for 18h. The solution was acidified with 1M HCl and the resulting solid was filtered and dried to give the title compound (0.945 g,80%).

### Part D. Preparation of 3-tert-butyl-5-(2,4-dioxo-3,4-dihydropyrimidin-1(2H)-yl)-2-methoxy benzaldehyde.

The product from **Part C** (0.945g, 2.97mmol) was taken up in thionyl chloride (4.5mL) and the mixture was heated at 80°C for 40min. After evaporation to dryness, the acid chloride was dissolved in dry THF (8mL) and cooled to -78°C. A 1 M solution of lithium tri-tert-butoxyaluminum hydride in THF (3.0mL, 3.0mmol) was added drop wise. After 45min the cold reaction was quenched with 1M HCl (5mL), extracted into ethyl acetate, and purified by column chromatography on silica gel, eluting with dichloromethane followed by 1% methanol/dichloromethane to give the title compound (0.635 g, 71%).

### Part E. Preparation of (E)-1-(3-tert-butyl-4-methoxy-5-(4-nitrostyryl)phenyl)pyrimidine-2,4(1H,3H)-dione.

The product of **Part D** (0.634g, 2.1mmol) and diethyl 4-nitrobenzylphosphonate (0.573g, 2.1mmol) were combined in dichloromethane (25mL) at ambient temperature. Potassium tert- butoxide (0.494g, 4.4mmol) was added portion wise and the resulting red/brown heterogeneous mixture was stirred for 1.5h. This mixture was quenched with 1M HCl (155mL), poured into water and extracted into ethyl acetate, and the crude product was purified by column chromatography on silica gel, eluting with 1% methanol/dichloromethane to give the title compound (0.735g, 83%).

### Part F. Preparation of (E)-1-(3-(4-aminostyryl)-5-tert-butyl-4-methoxyphenyl)pyrimidine-2,4(1H,3H)-dione.

The product from **Part E** (0.735g, 1.74mmol), ammonium chloride (0.14g, 2.62mmol), and iron (0.487g, 8.72mmol) were combined in a solution of ethanol (10mL), water (5mL), and THF (10mL) and heated at 75°C for 1h. The mixture was filtered through diatomaceous earth, rinsing well with THF and concentrated to give the title compound.

### Part G. Preparation of (E)-N-(4-(3-tert-butyl-5-(2,4-dioxo-3,4-dihydropyrimidin-1(2H)-yl)-2-methoxystyryl)phenyl)methanesulfonamide.

The product from **Part F** (0.683g, 1.75mmol) and pyridine (0.564mL, 6,98mmol) were combined in dichloromethane (155mL) at ambient temperature. Methane sulfonylchloride (0.163mL, 2.1mmol) was added drop wise and the solution was stirred for 18h. The mixture was poured into 1M HCl and extracted into dichloromethane, concentrated, and purified by column chromatography on silica gel, eluting with 1%, 2% methanol/dichloromethane. Trituration from dichloromethane provided a solid that was filtered and dried to give the title compound as a colorless powder (0.465g, 57%). ¹H NMR (300 MHz, DMSO-D6) δ ppm 1.38 (s, 9 H), 3.01 (s, 3 H), 3.79 (s, 3 H) 5.65 (d, J=7.72 Hz, 1 H), 7.17 - 7.28 (m, 5 H), 7.58 - 7.70 (m, 3 H), 7.75 (d, J=7.72 Hz, 1 H), 9.86 (s, 1 H), 11.42 (s, 1 H).

### Example 41B. Preparation of (E)-N-(4-(3-tert-butyl-5-(2,4-dioxo-3,4-dihydropyrimidin-1(2H)-yl)-2-methoxystyryl)phenyl)methanesulfonamide (compound IB-L1-1.1).

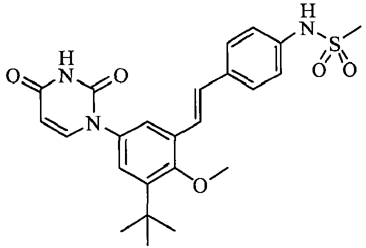

### Part A. Preparation of N-(4-ethynylphenyl)methanesulfonamide.

In a 2L, 3-neck round-bottom flask equipped with an overhead stirrer was added 4-ethynylaniline (30g, 256mmol) and pyridine (42.5ml, 525mmol) in dichloromethane (512ml) to give an orange solution. The mixture was cooled to 5°C and methanesulfonyl chloride (19.96ml, 256mmol) was added drop wise over 15min. The reaction solution was stirred at 5°C for 2h and washed with 1M aqueous HCl (3x250mL). The dichloromethane layer was then washed sequentially with saturated aqueous NaHCO₃, water, and saturated aqueous NaCl. The dichloromethane layer was dried over sodium sulfate and treated simultaneously with decolorizing charcoal for 30min, the solution then filtered through Celite and the filtrate was concentrated. The pink/orange solid was dissolved in a minimal amount of hot ethyl acetate (50-75mL) and slowly diluted with hexanes (500-600ml) to give orange crystals that were collected by filtration and dried to provide the title compound (40.0g, 80%).

### Part B. Preparation of (E)-4-(methylsulfonamido)styrylboronic acid.

(Reference: Org. Prep. Proc. Int., 2004, 36, 573-579) To a flask was added borane-methyl sulfide complex (8.03mL, 85mmol) followed by tetrahydrofuran (16mL) and the mixture then cooled to 0°C. (1R)-(+)-alpha-pinene (26.2mL, 169mmol) was then added drop wise (over 10min) to the ice-cooled solution. The mixture was then stirred at 0°C for 1h followed by stirring 2h at room temperature. The resulting thick white slurry was cooled to -40°C in a dry ice/acetone bath, followed by the addition of the product from **Part A** (15.0g, 77mmol) dissolved in 60mL of THF, drop wise over 30min. After the addition was complete, the mixture was stirred for an additional hour at -35°C, then 1h at room temperature. The light yellow solution was then cooled to 0°C and acetaldehyde (61.4mL, 1088mmol) added, then the mixture refluxed at 50°C for 18h. The solvent was then removed under vacuum to provide an orange syrup, to which water (115mL) was added and the heterogeneous mixture stirred for 3h at room temperature. The light yellow solid generated was collected and washed with water (250mL) then dried in a vacuum oven overnight. The resultant material was then dissolved in boiling acetone (190mL), which provided a homogenous yellow solution, followed by removal of the solution from heating and the addition of hexanes (365ml) over 5min time. A white solid formed in the solution and the mixture was stirred until the solution cooled to room temperature, then the white solid was collected and dried in a vacuum oven for 1hr to provide the title compound (12.1g, 85%).

### Part C. Preparation of 2-tert-butyl-4-nitrophenol.

To a vigorously stirred solution of 2-tert-butylphenol (10g, 66.6mmol) in heptane (67ml) was added at a fast drip a solution of 70% nitric acid (4.25ml, 66.6mmol) diluted with water (4.25ml). The resulting dark red/brown mixture was stirred vigorously for 2h. The suspended solid was collected by filtration washed with hexane (300mL), water (200mL) and once again with hexane (200mL) to give a cocoa colored powder that was dried to constant mass (4.65g, 35.6%).

### Part D. Preparation of 2-bromo-6-tert-butyl-4-nitrophenol.

A solution of the product from **Part C** (1.0g, 5.12mmol) in glacial acetic acid (10.25mL) was treated portion wise with pyridine hydrobromide perbromide (1.80g, 5.63mmol) followed by stirring at room temperature for 2h. Additional pyridinium hydrobromide perbromide (3.6g) was added in two portions and after another 3h of stirring, the reaction was complete. The mixture was poured into ice water, and the mixture treated with a small amount of sodium sulfite. The resulting solid was filtered and dried under vacuum to give the title compound as a brown solid (1.40g, 100 %).

### Part E. Preparation of 1-bromo-3-tert-butyl-2-methoxy-5-nitrobenzene.

A solution of the product from **Part D** (1.40g, 5.11mmol) in 10:1 t-butylmethylether-methanol (25.5mL) was treated with 2.0M trimethylsilyldiazomethane in ether (5.1mL, 10.21mmol), followed by stirring at room temperature for 18h. The mixture was concentrated under vacuum to afford a yellow oil, which was purified by silica gel column chromatography eluting with EtOAc/hexanes to give the title compound as a yellow oil (1.36g, 92 %).

### Part F. Preparation of tert-butyl 3-bromo-5-tert-butyl-4-methoxyphenylcarbamate.

A solution of the product from **Part E** (960mg, 3.33mmol) in methanol (17mL) was treated with 5 % platinum on sulfided carbon (100mg), followed by hydrogenation under balloon pressure for 3h, and then filtered through celite and concentrated under vacuum to afford the 3-bromo-5-tert-butyl-4-methoxyaniline as a yellow oil (860mg, 3.33mmol, 100%). A solution of this material in THF (17mL) was treated with di-tert-butyl dicarbonate (800mg, 3.66mmol) followed by warming at reflux for 2h. Concentration under vacuum afforded a beige solid, which was purified by silica gel column chromatography eluting with EtOAc/hexanes. Solid was triturated with hexanes, collected by filtration, and dried under vacuum to give the title compound as a nearly white solid (890mg, 75 %).

### Part G. Preparation of (E)-N-(3-bromo-5-tert-butyl-4-methoxyphenylcarbamoyl)-3-methoxyacrylamide.

The product from **Part F** (2.0g, 5.58mmo1) was dissolved in dichloromethane (10mL and trifluoroacetic acid (5mL) added. The solution was stirred at room temperature for 1h followed by concentration under vacuum and the addition of 10% aqueous sodium bicarbonate (50mL), followed by extraction with ethyl acetate (3 x 50mL). The combined organic extracts were dried and concentrated to provide a residue that was dissolved in 10mL of N,N-dimethylacetamide and cooled to -25°C. A 0.5 molar solution ofE-3-methoxyacryloyl isocyanate in benzene (20.3mL, 11.16mmol) was added drop wise and the resulting solution was stirred at ambient temperature for 4h, and then poured into water. The product was extracted into dichloromethane, washed with brine, dried over sodium sulfate, filtered and evaporated under vacuum to give the title compound.

### Part H. Preparation of 1-(3-bromo-5-tert-butyl-4-methoxyphenyl)pyrimidine-2,4(1H,3H)-dione.

The product from **Part G** (2.15g, 5.58mmol) was dissolved in ethanol (10mL). To this solution was added a mixture of concentrated sulfuric acid (1mL) and water (10mL). The heterogeneous mixture was heated at 100°C for 2h. The ethanol was removed under vacuum, and then the aqueous solution was extracted with dichloromethane and evaporated to dryness. This residue was purified by column chromatography on silica gel, eluting with 1% methanol/dichloromethane to yield the title compound (1.35g, 69%).

### Part I. Preparation of (E)-N-(4-(3-tert-butyl-5-(2,4-dioxo-3,4-dihydropyrimidin-1(2H)-yl)-2-methoxystyryl)phenyl)methanesulfonamide.

The product from **Part H** (8.0g, 22.65mmol), the product from **Part B** (5.90g, 24.46mmol), 1,1'-bis(di-tert-butylphosphino)ferrocene palladium dichloride (0.73 8g, 1.32mmol), and potassium phosphate (9.62g, 45.3mmo1) were dissolved in a mixture of tetrahydrofuran (128mL) and water (32mL). Nitrogen gas was bubbled through the resultant mixture for 10 min followed by heating the solution at 50°C for 5h in darkness. The reaction was allowed to cool to room temperature followed by the addition of saturated aqueous ammonium chloride (50mL), water (200mL), and the solution extracted with dichloromethane (600mL). To the organic extract was added magnesium sulfate, and 3-mercaptopropyl-functionalized silica gel (20 g) and the resultant solution stirred in darkness for 18h. The solids were then removed by filtration and the filtrate concentrated under vacuum and subjected to silica gel column chromatography using a 99/1 to 99/2 dichloromethane/methanol gradient to provide the title compound (7.4 g, 70%). ¹H NMR (300 MHz, DMSO-D6) δ ppm 1.38 (s, 9 H), 3.01 (s, 3 H), 3.79 (s, 3 H) 5.65 (d, J=7.72 Hz, 1 H), 7.17 - 7.28 (m, 5 H), 7.58 - 7.70 (m, 3 H), 7.75 (d, J=7.72 Hz, 1 H), 9.86 (s, 1 H), 11.42 (s, 1 H).

### Example 42. Preparation of (E)-N-(4-(3-tert-butyl-5-(5-fluoro-2,4-dioxo-3,4-dihydro-pyrimidin-1(2H)-yl)-2-methoxystyryl)phenyl)methanesulfonamide (compound IB-L1-1.2).

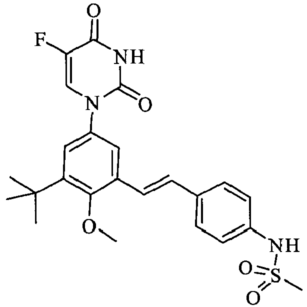

### Part A. Preparation of methyl 3-tert-butyl-5-(5-fluoro-6-methoxy-2,4-dioxotetrahydro-pyrimidin-1 (2H)-yl)-2-methoxybenzoate.

The fluorination procedure was performed as described in Lal, GS, et al. J. Org Chem., 60:7340-7342 (1995). The product from **Example 41A, Part B** (0.42g, 1.26mmol) and Selectfluor^{™} (0.672g, 1.9mmol) were combined in a mixture of acetonitrile (8mL) and methanol (1mL) and heated at 90°C under N₂ for 5h. The solution was diluted with water, extracted into ethyl acetate, washed with sodium bicarbonate solution, concentrated and purified by column chromatography on silica gel to give the title compound (0.138g, 29%).

### Part B. Preparation of methyl 3-tert-butyl-5-(5-fluoro-2,4-dioxo-3,4-dihydropyrimidin-1(2H)-yl)-2-methoxybenzoate.

The product from **Part A** (0.134g, 0.35mmol) and triethylamine (1mL) were combined in methanol (4mL) and stirred at ambient temperature for 18h. The solution was quenched with 1M HCl, extracted into dichloromethane and concentrated to give the title compound (0.113g, 92%).

### Part C. Preparation of 3-tert-butyl-5-(5-fluoro-2,4-dioxo-3,4-dihydropyrimidin-1(2H)-yl)-2-methoxybenzoic acid.

The product from **Part B** (0.113g, 0.32mmol) was treated as described in **Example 41A, Part C** to give the title compound (0.088g, 81%).

### Part D. Preparation of 3-tert-butyl-5-(5-fluoro-2,4-dioxo-3,4-dihydropyrimidin-1(2H)-yl)-2-methoxybenzaldehyde.

The product from **Part C** (0.088g, 0.26mmol) was treated as described in **Example 41A, Part D** to give the title compound (0.075g, 90%).

### Part E. Preparation of (E)-1-(3-tert-butyl-4-methoxy-5-(4-nitrostyryl)phenyl)-5-fluoropyrimidine-2,4(1H,3H)-dione.

The product of **Part D** (0.075g, 0.23mmol) was treated as described in **Example 41A, Part E** to give 0.077g (75%).

### Part F. Preparation of (E)-1-(3-(4-aminostyryl)-5-tert-butyl-4-methoxyphenyl)-5-fluoropyrimidine-2,4(1H,3H)-dione.

The product of **Part E** (0.077g, 0.18mmol) was treated as described in **Example 41A, Part F** to give the title compound (0.071g, 94%).

### Part G. Preparation of (E)-N-(4-(3-tert-butyl-5-(5-fluoro-2,4-dioxo-3,4-dihydropyrimidin-1 (2H)-yl)-2-methoxystyryl)phenyl)methanesulfonamide.

The product of **Part F** (0.071g, 0.17mmol) was treated as described in **Example 41A, Part G** to give the title compound (0.048g, 57%). ¹H NMR (300 MHz, DMSO-D6): δ ppm 1.38 (s, 9 H), 3.01 (s, 3 H), 3.79 (s, 3 H) 7.19 - 7.27 (m, 5 H), 7.62 (d, J=8.82 Hz, 2 H), 7.66 (d, J=2.57 Hz, 1 H), 8.25 (d, J=6.99 Hz, 1H).

### Example 43. Preparation ofN-(4-((3-tert-butyl-5-(2,4-dioxotetrahydropyrimidin-1(2H)-yl)-2-methoxyphenyl)ethynyl)-3-methylphenyl)methanesulfonamide (compound IA-L2-1.9).

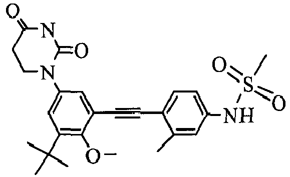

### Part A. Preparation of 2-tert-butyl-4-nitrophenol.

To a vigorously stirred solution of 2-tert-butylphenol (10g, 66.6mmol) in heptane (67ml) was added at a fast drip a solution of 70% nitric acid (4.25ml, 66.6mmol) diluted with water (4.25ml). The resulting dark red/brown mixture was stirred vigorously for 2h. The suspended solid was collected by filtration washed with hexane (300mL), water (200mL) and once again with hexane (200mL) to give the title compound as a cocoa colored powder that was dried to constant mass (4.65g, 35.6%).

### Part B. Preparation of 2-tert-butyl-6-iodo-4-nitrophenol.

To the product from **Part A** (4.5g, 23.05mmol) dissolved in MeOH (120ml) and water (30mL) was added iodine monochloride (1.155ml, 23.05mmol) drop wise over a period of 10min. The mixture was stirred for 2h and diluted into 1L of water and allowed to stand overnight. The solid material was collected by filtration and washed 3 x 50mL with water and dried under vacuum overnight to give the title compound as a tan solid (7.14g, 96%).

### Part C. Preparation of 1-tert-butyl-3-iodo-2-methoxy-5-nitrobenzene.

To an ice bath cooled solution of the product from **Part B** (5.5g, 17.13mmol) in MTBE (15ml) in a 50mL pressure vessel was added 2.0M TMS diazomethane (12.85ml, 25.7mmol) followed by dropwise addition of methanol (1.0mL) resulting in calm bubbling. The vessel was sealed and stirred at room temperature for 16h, cooled and the pressure was released. The solution was partitioned between EtOAc and water. The organic layer was washed with 1.0M HCl, saturated potassium carbonate solution, and saturated NaCl. The organic layer was dried over sodium sulfate, filtered and concentrated to give the title compound as a red oil that was used without purification (5.4g, 84%).

### Part D. Preparation of 3-tert-butyl-5-iodo-4-methoxyaniline.

A mixture of the product from **Part C** (5.80g, 17.31mmol), ammonium chloride (1.389g, 26.0mmol), and iron (4.83g, 87mmol) in THF/MeOH/water (200mL total, 2/2/1) was refluxed for 2h, cooled and filtered through Celite. The filtrate was evaporated and the residue was partitioned between water and EtOAc. The organic layer was washed with saturated brine, dried with sodium sulfate, filtered and evaporated to give the title compound as a brown oil (5.28g, 100% yield).

### Part E. Preparation of 1-(3-tert-butyl-5-iodo-4-methoxyphenyl)dihydropyrimidine-2,4(1H,3H)-dione.

The product from **Part D** (8.2g, 26.9mmol) was treated with acrylic acid (5.53ml, 81mmol) and stirred overnight to give an extremely viscous mixture. The mixture was treated with acetic acid (60mL) and urea (7.3g 120mmol), heated at 120°C for 24h, cooled and concentrated. The residue was azeotroped 3 x 100mL with toluene to give a brown/tan solid. The solid was suspended in a mixture of 50mL EtOAc and 100mL of saturated NaHCO₃ and stirred for 30min to neutralize any remaining acetic acid. The solid was collected by filtration and washed repeatedly with 50mL portions of water and finally with 3:1 hexane/EtOAc (50mL) to give the title compound as an off-white solid that was dried to constant mass (7.1g, 66%).

### Part F. Preparation of N-(4-iodo-3-methylphenyl)methanesulfonamide.

A solution of 4-iodo-3-methylaniline (4.37g, 18.75mmol) in CH₂Cl₂ (25ml) was treated with pyridine (6.07ml, 75mmol) followed by drop wise addition of methanesulfonyl chloride (1.607ml, 20.63mmol) to give a reddish/orange mixture. The mixture was stirred for 2h, concentrated and diluted with EtOAc. The EtOAc layer was washed with 1M HCl, water, saturated NaCl, dried (Na₂SO₄) and filtered. The EtOAc filtrate was treated with activated charcoal for 30min at 50°C and filtered through a 10g silica plug and concentrated to give the title compound as a light yellow solid (5.5g, 94%).

### Part G. Preparation of N-(3-methyl-4-((trimethylsilyl)ethynyl)phenyl)methanesulfonamide.

A mixture of the product from **Part F** (3.11g, 10mmol), copper(I) iodide (0.067g, 0.35mmol), dichlorobis(triphenylphosphine)palladium (II) (0.351g, 0.50mmol), triethylamine (6.97ml, 50.0mmol) and trimethylsilyl acetylene (1.684ml, 12.0mmol) in acetonitrile (50ml) was purged with bubbling N₂ for 5min and heated under N₂ at 80°C for 30min. The reaction mixture was poured into 200mL of EtOAc and partitioned with water adding enough 1M HCl to bring the pH to 1. The mixture was stirred vigorously for 15min and the layers were separated. The EtOAc layer was washed sequentially with 10% aqueous NaHCO₃, water, and saturated NaCl, dried over Na₂SO₄ and filtered. The filtrate was treated with 2.0g of Silicycle Si-thiol silica gel, stirred for 2h and filtered though a 1 inch pad of silica gel. The filtrate was concentrated and the residue was flash chromatographed on silica eluting with 9:1 hexane/EtOAc --> 3:1 hexane/EtOAc to give the title compound as a beige solid (2.7g, 96%).

### Part H. Preparation of N-(4-ethynyl-3-methylphenyl)methanesulfonamide.

The product from **Part G** (1.13g, 4.01mmol) in MeOH (20.07ml) was treated with 1M NaOH (8.43ml, 8.43mmol), stirred for 1h, partitioned into EtOAc/water and carefully acidified to pH 3 with 1M HCl. The EtOAc layer was washed with brine, dried (Na₂SO₄) and concentrated to give the title compound as a tan solid (820mg, 98%).

### Part I. Preparation of N-(4-((3-tert-butyl-5-(2,4-dioxotetrahydropyrimidin-1(2H)-yl)-2-methoxyphenyl)ethynyl)-3-methylphenyl)methanesulfonamide.

A mixture of the products from **Part E** (1.38g, 3.43mmol), **Part H** (0.79g, 3.78mmol), copper(I) iodide (0.023g, 0.12mmol) dichlorobis(triphenylphosphine)palladium (II) (0.12g, 0.172mmol) and triethylamine (2.392ml, 17.16mmol) in acetonitrile (60ml) was purged with bubbling N₂ for 5min and heated in an oil bath under N₂ at 80°C for 20min. The reaction mixture was poured into 400mL of warm EtOAc and partitioned with water adding enough 1M HCl to bring the pH to 1. The mixture was stirred vigorously for 15min and the layers were separated. The EtOAc layer was washed sequentially with 10% NaHCO₃, water, and saturated NaCl. The organic layer was dried (Na₂SO₄), and filtered. The filtrate was treated with 4.0g of Silicycle Si-thiol silica gel, heated at gentle reflux for 2h, cooled and filtered though a linch pad of silica gel. The filtrate was concentrated to a yellow solid that was recrystallized by dissolving in hot EtOAc/MeOH (270mL/30mL), reducing the volume to 100mL and allowing to cool. The resulting precipitate was collected by filtration and recrystallized a second time to give the title compound as a white solid (760mg, 46%). m.p. >280° C. ¹H NMR (300 MHz, DMSO-D6) δ ppm 1.35 (s, 9H) 2.46 (s, 3H) 2.70 (t, *J*=6.62 Hz, 2H) 3.05 (s, 3H) 3.77 (t, *J*=6.62 Hz, 2H) 4.04 (s, 3H) 7.08 (dd, *J*=8.46, 1.84 Hz, 1 H) 7.14 (s, 1 H) 7.25 (d, *J*=2.57 Hz, 1 H) 7.36 (d, *J*=2.57 Hz, 1 H) 7.50 (d, *J*=8.46 Hz, 1 H) 9.99 (s, 1 H) 10.36 (s, 1 H).

### Example 44. Preparation of N-(4-((3-tert-butyl-5-(2,4-dioxotetrahydropyrimidin-1(2H)-yl) phenyl)ethynyl)-3-chlorophenyl)methanesulfonamide (compound IA-L2-1.3).

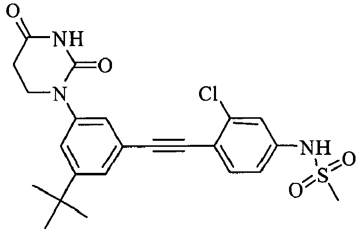

### Part A. Preparation of methyl 3-tert-butyl-5-(chlorocarbonyl)benzoate.

A mixture of 3-tert-butyl-5-(methoxycarbonyl)benzoic acid (9.18g, 38.9mmol, prepared by the method of Carter et. al., WO2005021500A1), thionyl chloride (75mL) and 1 drop of DMF in toluene (200mL) was heated at reflux for 2h, cooled and concentrated. The residue was azeotroped with toluene (3 x 50mL) and dried under high vacuum to give the title compound as an off-white waxy solid (9.9g, quantitative yield).

### Part B. Preparation of methyl 3-(azidocarbonyl)-5-tert-butylbenzoate.

To the product of **Part A** (9.9g, 38.9mmol) in acetone (200ml) was added at a fast drip a solution of sodium azide (10.12g, 156mmol) dissolved in water (20mL). The mixture was stirred for 2h and diluted with EtOAc. The organic layer was washed with H₂O, saturated brine, dried (Na₂SO₄), filtered and concentrated to give the title compound as a white solid (9.9g, 97%).

### Part C. Preparation of methyl 3-amino-5-tert-butylbenzoate.

The product from **Part B** (9.9g, 37.9mmol) in toluene (100mL) was heated at reflux for 1h and concentrated to give the intermediate isocyanate which was dissolved in DME (60mL) treated with 8% HCl (150mL) and stirred for 16h. The mixture was concentrated and the residue was dissolved in water, neutralized with solid sodium bicarbonate and extracted 3 x 100mL with EtOAc. The organics were combined, washed with saturated NaCl, dried (Na₂SO₄), filtered and concentrated. The crude product was chromatographed on silica eluting with 2:1 hexane/EtOAc to give the title compound as an oil (2.7g, 35%).

### Part D. Preparation of methyl 3-tert-butyl-5-(2,4-dioxotetrahydropyrimidin-1(2H)-yl)-2-methoxybenzoate.

A mixture of the product of **Part C** (2.34g, 11.29mmol) and acrylic acid (2.32ml, 33.9mmol) in toluene (60ml) was heated at reflux under nitrogen for 24h, cooled and concentrated. The resulting residue was then treated with urea (2.03g, 33.9mmol) in acetic acid (35ml) and heated at 120°C for 24h, cooled and concentrated. The residue was azeotroped 3 x 50mL with toluene and dissolved in 100mL of EtOAc. The organic layer was washed with dilute bicarbonate, H₂O, saturated brine, dried (Na₂SO₄), filtered and concentrated to give the title compound as a white solid (2.1 g, 61 %).

### Part E. Preparation of 3-tert-butyl-5-(2,4-dioxotetrahydropyrimidin-1(2H)-yl)benzoic acid.

A mixture of the product from **Part D** (1.8g, 5.91mmol) and 1M NaOH (29.6ml, 29.6mmol) in MeOH (15ml) and THF (15mL) was stirred for 24h and concentrated. The residue was treated with 50mL of 1M HCl and extracted into EtOAc. The EtOAc layer was washed with H₂O, saturated brine, dried (Na₂SO₄), filtered and concentrated to give a white solid. This intermediate urea was combined with 20mL of concentrated HCl and heated at 100°C for 1h, cooled and diluted with 75mL of ice water to give a solid that was collected by filtration and dried to constant mass to give the title compound as a colorless powder (1.6g, 93%).

### Part F. Preparation of 3-tert-butyl-5-(2,4-dioxotetrahydropyrimidin-1(2H)-yl)benzaldehyde.

A solution of the product of **Part E** (0.8g, 2.76mmol) in sulfurous dichloride (25mL) was heated at reflux for 1.5h, cooled and concentrated. The residue was azeotroped 3 x 25mL with toluene to give a white powder. This acid chloride was dissolved in anhydrous THF (25mL), cooled to -78°C under nitrogen and treated drop wise with 1M lithium tri-tert-butoxyaluminum hydride (3.03mL, 3.03mmol) in THF. The solution was stirred at -78°C for 3h and quenched cold with 1M HCl, warmed to ambient temperature and extracted 3 x 25mL with EtOAc. The organic extracts were combined, washed with water, 10% bicarbonate, saturated brine and dried with sodium sulfate. The EtOAc was filtered and concentrated to give the title compound as a white solid (0.77g, quantitative yield).

### Part G. Preparation of 1-(3-tert-butyl-5-ethynylphenyl)dihydropyrimidine-2,4(1H,3H)-dione.

A mixture of the product from **Part G** (913mg, 3mmol), dimethyl 1-diazo-2-oxopropyl phosphonate (749mg, 3.90mmol, prepared by the method of Ohira, Syn. Comm. 19 (3&4) 561-564 (1989), and potassium carbonate (829mg, 6.00mmol) in MeOH (20ml) was stirred for 16h and carefully acidified with 1M HCl. The mixture was extracted 2 x 50mL with CH₂Cl₂. The organics were combined, washed with water, saturated NaCl, dried (Na₂SO₄), filtered and concentrated. The crude product was purified via silica gel chromatography eluting with 20:1 (CH₂Cl₂/MeOH) to give the title compound as a white solid (415mg, 46%).

### Part H. Preparation of N-(4-((3-tert-butyl-5-(2,4-dioxotetrahydropyrimidin-1(2H)-yl) phenyl)ethynyl)-3-chlorophenyl)methanesulfonamide.

A mixture of the product from **Part G** (40.5mg, 0.15mmol), copper(I) iodide (1.4mg, 7.5µmol), bis(triphenylphosphine)palladium(II) chloride (5.26mg, 7.50µmol), N-(3-chloro-4-iodophenyl) methanesulfonamide (52.2mg, 0.158mmol, prepared from 3-chloro-4-iodo aniline by the method of **Example 43, Part F**) and triethylamine (0.105ml, 0.750mmol) in acetonitrile (2mL) was combined in a sealed microwave 5mL tube and purged with N₂ bubbling for 5min. The mixture was heated by microwave at 70°C for 5min, cooled and concentrated. The crude material was purified on a 4g silica cartridge eluting with 99.5:0.5 CH₂Cl₂/MeOH --> 97:3 CH₂Cl₂/MeOH. The desired fractions were combined and concentrated. The material was triturated in a minimal amount of EtOAc and the white solid was collected by filtration and dried to give the title compound (28mg, 39%). m.p. 278-280°C. ¹H NMR (300 MHz, DMSO-D6) δ ppm 1.31 (s, 9 H) 2.72 (t, *J*=6.62 Hz, 2 H) 3.12 (s, 3 H) 3.82 (t, *J*=6.62 Hz, 2 H) 7.20 (dd, *J*=8.46, 2.21 Hz, 1 H) 7.35 (s, 2 H) 7.38 - 7.46 (m, 2 H) 7.66 (d, *J*=8.46 Hz, 1 H) 10.31 (s, 1 H) 10.41 (s, 1 H).

### Example 45. Preparation ofN-(6-((3-tert-butyl-5-(2,4-dioxotetrahydropyrimidin-1(2H)-yl)-2-methoxyphenyl)ethynyl)pyridin-3-yl)methanesulfonamide (compound IA-L2-1.25).

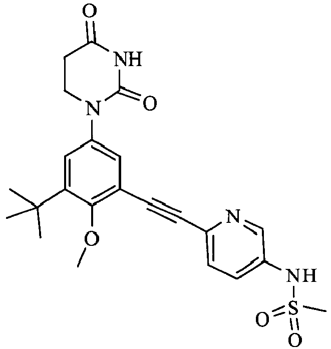

### Part A. Preparation of N-(6-iodopyridin-3-yl)methanesulfonamide.

To a solution of 6-iodopyridin-3-amine (1.077gm, 4.90mmole) in dichloromethane (40ml) and pyridine (1.98ml, 24.49mmole) at ice bath temperature was added methanesulfonyl chloride (0.401ml, 2.444mmole). The mixture was allowed to warm to room temperature and stir four days. The reaction mixture was treated with 5% acetic acid and allowed to stir 20min at room temperature. The organic phase was washed with water (2x50ml), dried (MgSO4) and concentrated in vacuo. The residue was slowly added in portions to rapidly stirred water (100ml) and the resulting solid collected by filtration, washed with water and dried in vacuo to give the title compound (0.7287g, 49.9%).

### Part B. Preparation of N-(6-((trimethylsilyl)ethynyl)pyridin-3-yl)methanesulfonamide.

The product from **Part A** (566mg, 1.899mmole) was combined with copper (I) iodide (17mg, 0.089mmole) and bis(triphenylphosphine) palladium (II) chloride (73mg, 0.104mmole) in a pressure tube. Anhydrous acetonitrile (17ml) added followed by triethylamine (1.323ml, 9.49mmole). Nitrogen was bubbled through the resulting yellow suspension with stirring for 5min then added trimethylsilyl acetylene (0.526ml, 3.80mmole). The vessel was immersed in a pre-heated oil bath at 80°C. The reaction mixture was allowed to stir with heating for 2h then cooled to room temperature and transferred to a round bottom flask. The volatiles were removed in vacuo and the brown residue fractionated by (flash)silica gel chromatography (ethyl acetate/hexanes) to give the title product (0.4539g, 89%) as a tan solid.

### Part C. Preparation of N-(6-ethynylpyridin-3-yl)methanesulfonamide.

The product from **Part B** (0.533gm, 1.984mmole) was dissolved in methanol (17ml) and a 2N sodium hydroxide (2ml, 4.17mmole) solution was added drop wise at room temperature. The mixture was allowed to stir 1h. The reaction mixture was concentrated in vacuo. The residue was partitioned between ethyl acetate and water and the pH adjusted to neutral with glacial acetic acid. The organic phase was diluted with additional ethyl acetate then washed several times with brine and concentrated in vacuo to give the title compound as a tan solid (0.3266g, 84%).

### Part D. Preparation of N-(6-((3-tert-butyl-5-(2,4-dioxotetrahydropyrimidin-1(2H)-yl)-2-methoxyphenyl)ethynyl)pyridin-3-yl)methanesulfonamide.

The product from **Part C** (75mg, 0.382mmole) was combined with the product obtained as described in **Example 43, Part E** (146mg, 0.363mmole), copper (I) iodide (7.42mg, 0.039mmole) and bis(triphenylphosphine) palladium (II) chloride (18.24mg, 0.026mmole) in a heavy wall glass tube and the vessel sealed with a septum crimp cap. Under an atmosphere of nitrogen added anhydrous acetonitrile (10ml) followed by triethylamine (0.266ml, 1.911mmole). Nitrogen was bubbled through the resulting brown suspension for 5min and then the tube was immersed in a pre-heated 80°C oil bath. The reaction was monitored by LC/MS. Three additional aliquots of the alkyne (a total of 71mg, 0.362mmole) each in THF (1ml), were added via syringe over the course of 8h. The reaction mixture was subsequently poured into 150ml of warm (45°C) ethyl acetate, partitioned with brine (75ml) and allowed to stir 15min. The aqueous phase was extracted with ethyl acetate (2x25ml) and the combined organic phase dried (MgSO4) and filtered. The filtrate was treated with a gram of Silicycle Sithiol silica gel and heated under nitrogen with stirring for 90min. The silica gel was removed by filtration and after concentration in vacuo the crude product isolated as a pale orange solid. The title compound (0.1315gm, 73.1 %) was obtained as an off white solid by (flash) silica gel chromatography using a step gradient of ethyl acetate in hexanes followed by methanol in dichloromethane. m.p. 191-192.5°C (d). ¹H NMR (300 MHz, DMSO-D6) δ ppm 1.35 (s, 9 H) 2.70 (t, *J*=6.62 Hz, 2 H) 3.12 (s, 3 H) 3.78 (t, *J*=6.62 Hz, 2 H) 4.07 (s, 3 H) 7.30 (d, *J*=2.94 Hz, 1 H) 7.40 (d, *J*=2.57 Hz, 1 H) 7.65 (d, *J*=1.47 Hz, 2 H) 8.44 (s, 1 H) 10.29 - 10.34 (m, 1 H) 10.38 (s, 1 H).

### Example 46. Preparation of N-(4-((3-tert-butyl-5-(2,4-dioxotetrahydropyrimidin-1(2H)-yl)-2-methoxyphenyl)ethynyl)-3-(trifluoromethyl)phenyl)methanesulfonamide (compound IA-L2-1.18).

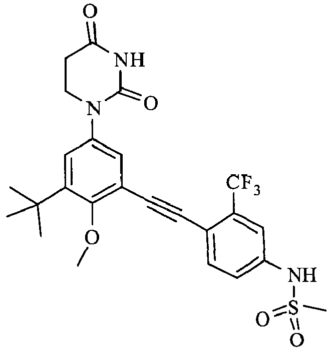

### Part A. Preparation of N-(4-bromo-3-(trifluoromethyl)phenyl)methanesulfonamide.

The title compound was prepared by the reaction of 4-bromo-3-(trifluoromethyl)aniline with methanesulfonyl chloride as described in **Example 45, Part A**.

### Part B. Preparation of N-(3-(trifluoromethyl)-4-((trimethylsilyl)ethynyl)phenyl)methane sulfonamide.

The product from **Part A** (2.00gm, 6.29mmole) was combined with triphenylphosphine (0.211gm, 0.805mmole) and palladium (II) acetate(0.099gm, 0.440mmole) in a 250ml round bottom flask equipped with a condenser and the reaction was performed as described by W.B Austin et al, J. Org. Chem., 46 (11):2280 (1981). Toluene (40ml) was added followed by triethylamine (80ml) and trimethylsilylacetylene (4.41ml, 31.4mmole). The resulting yellow solution was purged with nitrogen for 5min at room temperature. The reaction mixture was heated under nitrogen in an oil bath at 80°C for 24h. Cool to room temperature and filter. The filtrate was concentrated in vacuo and the residue chromatographed on silica gel (ethyl acetate-hexanes) to give the title compound as a tan solid (1.4554gm, 69%).

### Part C. Preparation of N-(4-ethynyl-3-(trifluoromethyl)phenyl)methanesulfonamide.

The product from **Part B** (0.378gm, 1.126mmole) was dissolved in methanol (8ml) and treated with potassium carbonate (0.322gm, 2.331mmole) at room temperature. After 90min the reaction mixture was partitioned between ethyl acetate and dilute HCl. The organic phase was water washed then dried (MgSO4) and concentrated in vacuo. The residue was purified by silica gel chromatography (ethyl acetate-hexane) to give the title compound as a clear oil, which slowly crystallizes on standing (0.2502g, 84%).

### Part D. Preparation ofN-(4-((3-tert-butyl-5-(2,4-dioxotetrahydropyrimidin-1(2H)-yl)-2-methoxyphenyl)ethynyl)-3-(trifluoromethyl)phenyl)methanesulfonamide.

The product from **Part C** (0.2502gm, 0.950mmole) was treated with the product obtained as described in **Example 43, Part E** (0.364gm, 0.905mmole) as detailed in **Example 45, Part D**. The title compound (0.3195g, 65.7%) was obtained as a white solid by trituration of the crude product with ether-dichloromethane. m.p. 257.5-261°C (d). ¹H NMR (300 MHz, DMSO-D6) δ ppm 1.35 (s, 9 H) 2.71 (t, *J*=6.62 Hz, 2 H) 3.14 (s, 3 H) 3.78 (t, *J*=6.43 Hz, 2 H) 4.03 (s, 3 H) 7.29 (d, *J*=2.57 Hz, 1 H) 7.34 (d, *J*=2.57 Hz, 1 H) 7.48 - 7.56 (m, 1 H) 7.60 (s, 1 H) 7.83 (d, *J*=8.09 Hz, 1 H) 10.37 (s, 1 H) 10.45 (s, 1 H).

### Example 47. Preparation of N-[4-(acetyl-methanesulfonyl-amino)-phenyl]-3-tert-butyl-5-(2,4-dioxo-tetrahydro-pyrimidin-1-yl)-2-methoxy-benzamide (compound IA-L3-1.69).

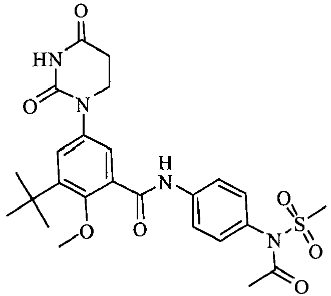

### Part A. Preparation of 3-tert-butyl-5-(2,4-dioxotetrahydropyrimidin-1(2H)-yl)-2-methoxybenzoyl chloride.

The product from **Example 29, Part F,** (2H)-yl)-2-methoxybenzoic acid (4.07g, 12.71 mmol) and thionyl chloride (40.82mL, 559mmol) were added together. The mixture was then refluxed for 2h, followed by concentration under vacuum to provide the product as a light-yellow solid.

### Part B. Preparation of N-[4-(acetyl-methanesulfonyl-amino)-phenyl]-3-tert-butyl-5-(2,4-dioxo-tetrahydro-pyrimidin-1-yl)-2-methoxy-benzamide.

The product obtained from **Part A** (0.073g , 0.15mmole) was dissolved in pyridine (2ml) and treated drop wise with acetic anhydride (0.042mL, 0.45mmol). The mixture was stirred 3h at room temperature. The reaction mixture was concentrated in vacuo and the residue was dissolved in ethyl acetate (25ml). The organic layer was washed with aq. HCl, aq. NaHCO₃, brine, and dried over anhydrous solid sodium sulfate. The drying agent was filtered and the solvent evaporated under vacuum giving the title compound as a white solid (55mg, 68%). mp 228-229°C. 1H NMR (300 MHz, DMSO-D6) δppm 1.38(s,9H) 1.92 (s, 3 H) 2.72 (t, *J*=6.62 Hz, 2 H) 3.52 (s, 3 H) 3.73 - 3.82 (m, 5 H) 7.32 (d, *J*=2.57 Hz, 1 H) 7.36 (d, *J*=2.94 Hz, 1 H) 7.44 (d, *J*=8.82 Hz, 2 H) 7.83 (d, *J*=8.82 Hz, 2 H) 10.37 (s, 1 H) 10.64 (s, 1 H).

### Example 48. Preparation of N-(6-(3-bromo-5-(2,4-dioxo-3,4-dihydropyrimidin-1(2H)-yl)-2-methoxyphenyl)naphthalen-2-yl)methanesulfonamide (compound IB-L0-2.69).

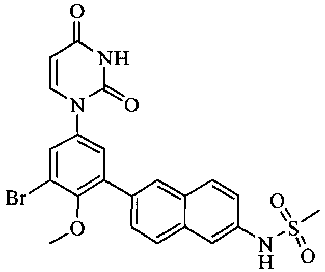

### Part A. Preparation of 2-bromo-4,6-diiodophenol.

A 1L round-bottom flask was charged with 2-bromophenol (Aldrich, 8.65g, 50mmol) and methanol (100ml) to give a colorless solution. Sodium hydroxide (2.40g, 60.0mmol) was added and stirred until the hydroxide pellets had dissolved. The solution was cooled in an ice water bath and sodium iodide (5.6g, 37.4mmol) was added followed by drop-wise addition of sodium hypochlorite (17mL, 27.5mmol) to give a transparent brown/red solution and gradual precipitation of a thick, white solid. The addition of sodium iodide and bleach was repeated 3 times to give an orange mixture that was stirred for 2h, treated with a solution of sodium thiosulfate in water (20g in 100mL), stirred for 15min and treated drop-wise with concentrated HCl to a constant pH of 1. The mixture was stirred for 15min and filtered to collect a white solid that was washed repeatedly with water and dried to constant mass (14.7g, 69%).

### Part B. Preparation of 1-bromo-3,5-diiodo-2-methoxybenzene.

A 500mL round-bottom flask was charged with the product from **Part A** (14.7g, 34.6mmol), iodomethane (2.70ml, 43.3mmol), and sodium hydroxide (2.101ml, 39.8mmol) in acetone (96ml) to give a tan solution. The mixture was stirred for 24h and concentrated. The residue was dissolved in ethyl acetate, washed with water and saturated sodium chloride, dried over sodium sulfate, filtered and concentrated to give a white solid. The solid was recrystallized from hot hexane to give a white solid that was collected by filtration (12.3g, 81 %).

### Part C. Preparation of 1-(3-bromo-5-iodo-4-methoxyphenyl)pyrimidine-2,4(1H,3H)-dione.

A 250mL round-bottom flask was charged with the product from **Part B** (8.09g, 18.44mmol), pyrimidine-2,4(1H,3H)-dione (2.273g, 20.28mmol), N-(2-cyanophenyl)picolinamide (0.823g, 3.69mmol), copper (I) iodide (0.351g, 1.844mmol) and potassium phosphate (8.22g, 38.7mmol) in DMSO (70ml). The mixture was sealed, sparged with nitrogen for 15min and heated at 60°C for 16h. The mixture was partitioned with ethyl acetate and water. The organic layer was washed with 1M HCl, water, brine, dried with sodium sulfate, and filtered. The filtrate was treated with 3-mercaptopropyl functionalized silica gel (Aldrich catalog # 538086), filtered through celite and evaporated to give an off-white solid (3.92g, 50%).

### Part D. Preparation of N-(6-(3-bromo-5-(2,4-dioxo-3,4-dihydropyrimidin-1(2H)-yl)-2-methoxyphenyl)naphthalen-2-yl)methanesulfonamide.

To a 5mL microwave tube was added the product from **Part C** (212mg, 0.50mmol), the product from **Example 4A, Part B** (174mg, 0.50mmol), potassium phosphate (223mg, 1.05mmol), PA-Ph (CAS 97739-46-3, 4.38mg, 0.015mmol) and tris(dibenzylideneacetone)dipalladium(0) (4.58mg, 5.00µmol) in tetrahydrofuran (3.0ml) and water (1.0ml). The tube was sealed and the mixture was sparged with nitrogen for 5min and then stirred for 24h. The reaction mixture was partitioned with ethyl acetate and 1M HCl. The organic layer was washed with saturated sodium bicarbonate, brine, dried with sodium sulfate and filtered. The filtrate was treated with 3-mercaptopropyl functionalized silica gel (Aldrich catalog # 538086), filtered through celite and evaporated. The residue was triturated with methanol/ CH₂Cl₂ to give the title compound as a white solid (256mg, 51%). ¹H NMR (300 MHz, DMSO-D6) δ ppm 3.08 (s, 3 H) 3.43 (s, 3 H) 5.68 (d, J=8.09 Hz, 1 H) 7.43 (dd, J=8.82, 2.21 Hz, 1 H) 7.60 (d, J=2.57 Hz, 1 H) 7.72 (m, 2 H) 7.82 (d, J=3.31 Hz, 1 H) 7.84 (d, J=1.84 Hz, 1 H) 7.96 (m, 2 H) 8.09 (s, 1 H) 10.07 (s, 1 H) 11.49 (s, 1 H). MS (ESI-) m/z 513.9, 515.9 (M-H)⁺.

### Example 49. Preparation of N-(6-(5-(2,4-dioxo-3,4-dihydropyrimidin-1(2H)-yl)-2-methoxy-3-(5-methylfuran-2-yl)phenyl)naphthalen-2-yl)methanesulfonamide (compound IB-L0-2.58).

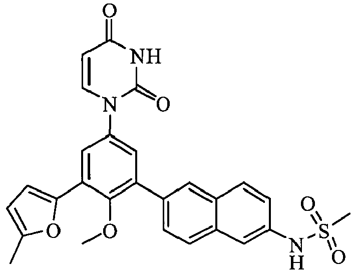

To a 5mL microwave tube was added the product of **Example 48** (52mg, 0.101mmol), 4,4,5,5-tetramethyl-2-(5-methylfuran-2-yl)-1,3,2-dioxaborolane (0.025ml, 0.121mmol), 1,1'-bis(di-tert-butylphosphino)ferrocene palladium dichloride (3.28mg, 5.04µmol) and potassium phosphate (42.8mg, 0.201mmol) in THF (3.0ml) and water (1.0ml). The tube was sealed and the mixture was sparged with nitrogen for 5min and then heated at 50°C for 3h. The cooled mixture was partitioned with ethyl acetate and 1M HCl. The organic layer was washed with saturated sodium bicarbonate, brine, dried with sodium sulfate, filtered and concentrated. The filtrate was treated with 3-mercaptopropyl functionalized silica gel, filtered and evaporated. The residue was purified by reverse phase chromatography to give the desired product as a white solid (23mg, 44%, m.p.174-178⁰C.) ¹H NMR (300 MHz, DMSO-D6) δ ppm 2.38 (s, 3 H) 3.09 (s, 3 H) 3.33 (s, 3 H) 5.69 (dd, J=7.72, 2.21 Hz, 1 H) 6.30 (d, J=3.31 Hz, 1 H) 7.00 (d, J=3.31 Hz, 1 H) 7.43 (m, 2 H) 7.74 (d, J=2.57 Hz, 2 H) 7.78 (dd, J=8.46, 1.84 Hz, 1 H) 7.85 (d, J=8.09 Hz, 1 H) 7.97 (t, J=8.82 Hz, 2 H) 8.12 (s, 1 H) 10.05 (s, 1 H) 11.46 (d, J=2.21 Hz, 1 H). MS (ESI+) *m*/*z* 518 (M+H)⁺.

### Example 50. Preparation of N-(6-(5-(2,4-dioxo-3,4-dihydropyrimidin-1(2H)-yl)-2-methoxy-3-(thiophen-3-yl)phenyl)naphthalen-2-yl)methanesulfonamide (compound IB-L0-2.53).

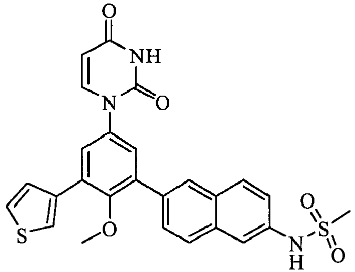

The title compound was prepared according to the procedure **of Example 49** substituting thiophen-3-ylboronic acid for 4,4,5,5-tetramethyl-2-(5-methylfuran-2-yl)-1,3,2-dioxaborolane to give a white solid (12mg, 23%). ¹H NMR (300 MHz, DMSO-D6) δ ppm 3.07 (s, 3 H) 3.22 (s, 3 H) 5.69 (d, J=7.72 Hz, 1 H) 7.41 (dd, J=8.64, 2.02 Hz, 1 H) 7.50 (d, J=2.94 Hz, 1 H) 7.59 (dd, J=5.13, 1.08 Hz, 1 H) 7.69 (m, 3 H) 7.76 (dd, J=8.64, 1.65 Hz, 1 H) 7.89 (d, J=7.72 Hz, 1 H) 7.95 (m, 3 H) 8.09 (s, 1 H) 10.05 (s, 1 H) 11.47 (s, 1 H). MS (ESI+) m/z 520 (M+H)⁺.

### Example 51. Preparation of N-(6-(5-(2,4-dioxo-3,4-dihydropyrimidin-1(2H)-yl)-2-methoxy-3-(thiophen-2-yl)phenyl)naphthalen-2-yl)methanesulfonamide (compound IB-L0-2.61).

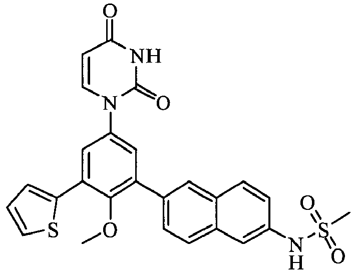

The title compound was prepared according to the procedure of **Example 49** substituting thiophen-2-ylboronic acid for 4,4,5,5-tetramethyl-2-(5-methylfuran-2-yl)-1,3,2-dioxaborolane to give a white solid (8mg, 15%). ¹H NMR (300 MHz, DMSO-D6) δ ppm 3.08 (s, 3 H) 3.30 (s, 3 H) 5.70 (d, J=8.09 Hz, 1 H) 7.19 (dd, J=5.33, 3.86 Hz, 1 H) 7.42 (dd, J=8.82, 2.21 Hz, 1 H) 7.49 (d, J=2.57 Hz, 1 H) 7.69 (dd, J=5.15, 1.20 Hz, 1 H) 7.80 (m, 3 H) 7.88 (d, J=7.72 Hz, 1 H) 7.92 (d, J=2.57 Hz, 1 H) 7.98 (m, 2 H) 8.12 (s, 1 H) 10.06 (s, 1 H) 11.48 (s, 1 H). MS (ESI+) *m*/*z* 520 (M+H)⁺.

### Example 52. Preparation of N-(6-(5-(2,4-dioxo-3,4-dihydropyrimidin-1(2H)-yl)-3-(furan-2-yl)-2-methoxyphenyl)naphthalen-2-yl)methanesulfonamide (compound IB-L0-2.59).

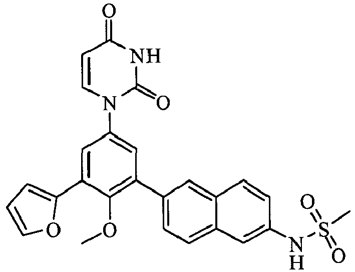

The title compound was prepared according to the procedure of **Example 49** substituting furan-2-ylboronic acid for 4,4,5,5-tetramethyl-2-(5-methylfuran-2-yl)-1,3,2-dioxaborolane to give a white solid (16mg, 32%). ¹H NMR (300 MHz, DMSO-D6) δ ppm 3.09 (s, 3 H) 3.35 (s, 3 H) 5.69 (d, J=7.72 Hz, 1 H) 6.69 (dd, J=3.31, 1.84 Hz, 1 H) 7.11 (d, J=3.31 Hz, 1 H) 7.43 (dd, J=8.82, 2.21 Hz, 1 H) 7.49 (d, J=2.94 Hz, 1 H) 7.80 (m, 5 H) 7.96 (m, 2 H) 8.13 (s, 1 H) 10.06 (s, 1 H) 11.47 (s, 1 H). MS (ESI-) m/z 502.1 (M-H)⁺.

### Example 53. Preparation of N-(6-(5-(2,4-dioxo-3,4-dihydropyrimidin-1(2H)-yl)-3-(furan-3-yl)-2-methoxyphenyl)naphthalen-2-yl)methanesulfonamide (compound IB-L0-2.64).

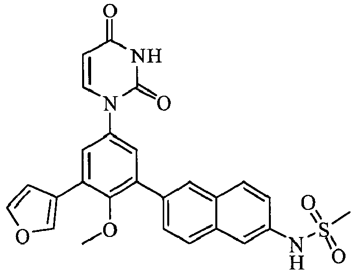

The title compound was prepared according to the procedure of **Example 49** substituting furan-3-ylboronic acid for 4,4,5,5-tetramethyl-2-(5-methylfuran-2-yl)-1,3,2-dioxaborolane to give a white solid (6mg, 12%). ¹H NMR (300 MHz, DMSO-D6) δ ppm 3.09 (s, 3 H) 3.30 (s, 3 H) 5.69 (dd, J=7.71, 1.83 Hz, 1 H) 7.10 (dd, J=1.74, 0.78 Hz, 1 H) 7.42 (dd, J=8.82, 2.21 Hz, 1 H) 7.46 (d, J=2.57 Hz, 1 H) 7.73 (d, J=2.21 Hz, 1 H) 7.76 (d, J=2.57 Hz, 1 H) 7.78 (d, J=1.84 Hz, 1 H) 7.81 (t, J=1.84 Hz, 1 H) 7.86 (d, J=7.72 Hz, 1 H) 7.96 (t, J=8.82 Hz, 2 H) 8.10 (s, 1 H) 8.28 (s, 1 H) 10.05 (s, 1 H) 11.48 (s, 1 H). MS (ESI-) *m*/*z* 502.1 (M-H)⁺.

### Example 54. Preparation of N-(6-(5-(2,4-dioxo-3,4-dihydropyrimidin-1(2H)-yl)-2-methoxy-biphenyl-3-yl)naphthalen-2-yl)methanesulfonamide (compound IB-L0-2.71).

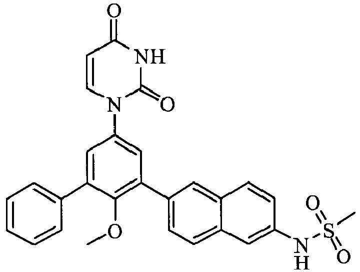

The title compound was prepared according to the procedure of **Example 49** substituting phenylboronic acid for 4,4,5,5-tetramethyl-2-(5-methylfuran-2-yl)-1,3,2-dioxaborolane. The crude product was purified by silica gel chromatography eluting with 3% methanol/ CH₂Cl₂ to give a white solid (10mg, 8%). ¹H NMR (300 MHz, DMSO-D6) δ ppm 3.08 (s, 3 H) 3.12 (s, 3 H) 5.69 (dd, J=7.81, 1.47 Hz, 1 H) 7.36 (m, 5 H) 7.56 (d, J=2.57 Hz, 1 H) 7.64 (m, 2 H) 7.74 (d, J=2.21 Hz, 1 H) 7.78 (dd, J=8.46, 1.84 Hz, 1 H) 7.94 (m, 3 H) 8.11 (s, 1 H) 10.04 (s, 1 H) 11.47 (s, 1 H). MS (ESI-) *m*/*z* 512 (M-H)⁺.

### Example 55. Preparation of N-(6-(3'-chloro-5-(2,4-dioxo-3,4-dihydropyrimidin-1(2H)-yl)-2-methoxybiphenyl-3-yl)naphthalen-2-yl)methanesulfonamide (compound IB-L0-2.74).

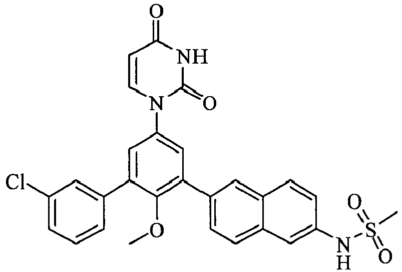

The title compound was prepared according to the procedure of **Example 49** substituting 3-chlorophenylboronic acid for 4,4,5,5-tetramethyl-2-(5-methylfuran-2-yl)-1,3,2-dioxaborolane to give a white solid (38mg, 68%). ¹H NMR (300 MHz, DMSO-D6) δ ppm 3.09 (s, 3 H) 3.13 (s, 3 H) 5.70 (dd, J=8.09, 2.21 Hz, 1 H) 7.43 (dd, J=8.82, 2.21 Hz, 1 H) 7.52 (m, 3 H) 7.62 (m, 2 H) 7.72 (m, 2 H) 7.79 (dd, J=8.46, 1.47 Hz, 1 H) 7.95 (m, 3 H) 8.12 (s, 1 H) 10.05 (s, 1 H) 11.47 (d, J=2.21 Hz, 1 H). MS (ESI-) *m*/*z* 546 (M-H)⁺.

### Example 56. Preparation of N-(6-(5-(2,4-dioxo-3,4-dihydropyrimidin-1(2H)-yl)-2-methoxy-3-(5-methylthiophen-2-yl)phenyl)naphthalen-2-yl)methanesulfonamide (compound IB-L0-2.73).

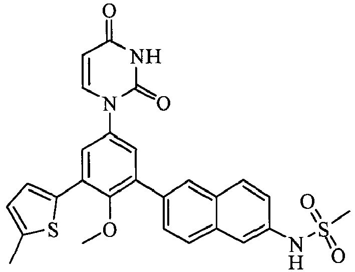

The title compound was prepared according to the procedure of **Example 49** substituting 4,4,5,5-tetramethyl-2-(5-methylthiophen-2-yl)-1,3,2-dioxaborolane for 4,4,5,5-tetramethyl-2-(5-methylfuran-2-yl)-1,3,2-dioxaborolane to give a white solid (22mg, 41%). ¹H NMR (300 MHz, DMSO-D6) δ ppm 2.49 (s, 3 H) 3.09 (s, 3 H) 3.29 (s, 3 H) 5.69 (dd, J=8.09, 2.21 Hz, 1 H) 6.87 (d, J=2.57 Hz, 1 H) 7.43 (m, 2 H) 7.54 (d, J=3.68 Hz, 1 H) 7.76 (m, 2 H) 7.85 (s, 1 H) 7.87 (d, J=5.15 Hz, 1 H) 7.98 (t, J=9.01 Hz, 2 H) 8.11 (s, 1 H) 10.06 (s, 1 H) 11.47 (d, J=2.21 Hz, 1 H). MS (ESI+) m/z 534 (M+H)⁺.

### Example 57. Preparation of N-(6-(5-(2,4-dioxo-3,4-dihydropyrimidin-1(2H)-yl)-3-(1-hydroxy-2-methylpropan-2-yl)-2-methoxyphenyl)naphthalen-2-yl)methanesulfonamide (compound IB-L0-2.54).

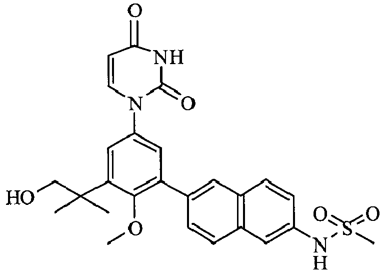

### Part A. Preparation of 2-(2-hydroxy-3,5-diiodophenyl)acetic acid.

To a 250mL round bottom flask was added 2-(2-hydroxyphenyl)acetic acid (Aldrich, 3.04g, 20mmol) in acetonitrile (50ml) to give a colorless solution. N-iodosuccimide (9.00g, 40.0mmol) was added portionwise over 15min to give a red/brown transparent solution that was stirred for 16h. The mixture was concentrated and the resulting solid was triturated in 75mL of water and filtered to collect an orange solid that was dried under vacuum. The crude solid was recrystallized from toluene to give a light orange powder (6.0g, 74%).

### Part B. Preparation of methyl 2-(3,5-diiodo-2-methoxyphenyl)acetate.

To a 250mL round-bottom flask was added the product from **Part A** (6g, 14.85mmol), potassium carbonate (6.16g, 44.6mmol), and dimethyl sulfate (4.12g, 32.7mmol) in acetone (49.5ml) to give a brown suspension. Heated at reflux for 16h, cooled, concentrated and the residue was partitioned between EtOAc and water. The EtOAc layer was washed with brine, dried (Na₂SO₄) and concentrated to a brown oil that was chromatographed on a 40g silica cartridge eluting with 3:1 hexane/EtOAc to give a yellow oil (6.0g, 94%).

### Part C. Preparation of methyl 2-(3,5-diiodo-2-methoxyphenyl)-2-methylpropanoate.

To a 100mL round-bottom flask under nitrogen was added the product from **Part B** (1.728g, 4mmol) in anhydrous THF (20ml) and HMPA (2ml) to give a colorless solution. Methyl iodide (1.251ml, 20.00mmol) was added and the solution was cooled to -40°C. Potassium t-butoxide(12.00ml, 12.00mmol) was added dropwise and the mixture was stirred at -40 to -20°C for 30min and quenched with 1M HCl to a pH of 1. The mixture was extracted 3 X 40ml with EtOAc. The extracts were combined, washed with brine, dried (Na₂SO₄) and concentrated. The crude product was flash chromatographed on a 40g ISCO silica cartridge eluting with 9:1 hexane/EtOAc to give the bis-methylated product as a yellow oil (1.63g, 89%).

### Part D. Preparation of 2-(3,5-diiodo-2-methoxyphenyl)-2-methylpropanoic acid.

A suspension of the product from **Part C** (2.63g, 5.72mmol) in MeOH (40ml) and THF (40ml) was treated with 4.0M sodium hydroxide (28ml, 112mmol) and heated at 80°C for 48 h. The organic solvent was evaporated and the remaining aqueous solution was acidified with 1M HCl producing a solid that was collected by filtration, washed with water and dried to give the desired carboxylic acid (2.46g, 96%).

### Part E. Preparation of 2-(3,5-diiodo-2-methoxyphenyl)-2-methylpropan-1-ol.

A solution of the product from **Part D** (1.00g, 2.242mmol) in THF (40ml) was treated dropwise with borane THF complex 1.0M (20ml, 20mmol) and then heated at 50°C for 24 h. The mixture was treated with methanol (20mL), refluxed for 30 min and concentrated. The resulting residue was washed with water, brine, dried with sodium sulfate, filtered and evaporated. The residue was chromatographed on silica gel eluting with hexane/EtOAc (4:1) to give the desired product (810mg, 84%).

### Part F. Preparation of tert-butyl(2-(3,5-diiodo-2-methoxyphenyl)-2-methylpropoxy)-dimethylsilane.

A solution of the product from **Part E** (432mg, 1.000mmol) in DMF (5ml) was treated with tert-butyldimethylchlorosilane (301mg, 2.000mmol), and imidazole (204mg, 3.00mmol) and stirred for 2h. The mixture was partitioned between 1M HCl and ethyl acetate. The organic layer was washed with saturated sodium bicarbonate, brine, dried with sodium sulfate, filtered and evaporated. The residue was chromatographed on silica gel eluting with hexane/EtOAc (9:1) to give the desired product (522mg, 96%).

### Part G. Preparation of 1-(3-(1-(tert-butyldimethylsilyloxy)-2-methylpropan-2-yl)-5-iodo-4-methoxyphenyl)pyrimidine-2,4(1H,3H)-dione.

To a 50mL round-bottom flask was added the product from **Part F** (520mg, 0.952mmol), pyrimidine-2,4(1H,3H)-dione (117mg, 1.047mmol), N-(2-cyanophenyl)picolinamide (42.5mg, 0.190mmol), copper(I) iodide (18.13mg, 0.095mmol) and potassium phosphate (424mg, 1.999mmol) in DMSO (5ml). The vessel was sealed, sparged with nitrogen and then heated at 60°C for 24h. The mixture was partitioned between 1M HCl and ethyl acetate. The organic layer was washed with saturated sodium bicarbonate, brine, dried with sodium sulfate, and filtered. The filtrate was treated with 3-mercaptopropyl functionalized silica gel, filtered and evaporated. The residue was chromatographed on silica gel eluting with hexane/EtOAc (3:2) to give the product as a solid (285mg, 65%).

### Part H. Preparation of N-(6-(3-(1-(tert-butyldimethylsilyloxy)-2-methylpropan-2-yl)-5-(2,4-dioxo-3,4-dihydropyrimidin-1(2H)-yl)-2-methoxyphenyl)naphthalen-2-yl)methanesulfonamide.

To a 5mL microwave tube was added the product from **Part G** (50mg, 0.094mmol), the product from **Example 4A, Part B** (32.7mg, 0.094mmol), potassium phosphate (42.0mg, 0.198mmol), PA-Ph (CAS 97739-46-3) (0.827mg, 2.83µmol) and tris(dibenzylideneacetone)palladium(0) (0.863mg, 0.943µmol) in THF (3.0ml) and water (1.0ml). The vessel was sealed and the mixture was sparged with nitrogen for 5min and then heated at 50°C for 2h. The mixture was partitioned between 1M HCl and ethyl acetate. The organic layer was washed with saturated sodium bicarbonate, brine, dried with sodium sulfate and filtered. The filtrate was treated with 3-mercaptopropyl functionalized silica gel, filtered and evaporated. The residue was chromatographed on silica gel eluting with hexane/EtOAc (3:7) to give a solid (32mg, 54%).

### Part I. Preparation of N-(6-(5-(2,4-dioxo-3,4-dihydropyrimidin-1(2H)-yl)-3-(1-hydroxy-2-methylpropan-2-yl)-2-methoxyphenyl)naphthalen-2-yl)methanesulfonamide.

The product from **Part H** (31mg, 0.050mmol) in THF (2.0ml) was treated with 1M TBAF (0.3ml, 0.3mmol) in THF and stirred overnight. The mixture was partitioned with water and ethyl acetate. The organic layer was washed with brine three times, dried with sodium sulfate, filtered and evaporated. The residue was chromatographed on silica gel eluting with 2% to 8% methanol in CH₂Cl₂ to give a solid (21mg, 83%). Melting point: 256-257⁰C. ¹H NMR (300 MHz, DMSO-D6) δ ppm 1.35 (s, 6 H) 3.08 (s, 3 H) 3.23 (s, 3 H) 3.67 (d, J=4.78 Hz, 2 H) 4.72 (t, J=4.78 Hz, 1 H) 5.65 (d, J=8.09 Hz, 1 H) 7.36 (m, 3 H) 7.74 (m, 3 H) 7.98 (m, 3 H) 10.04 (s, 1 H) 11.41 (s, 1 H). MS (ESI+) *m*/*z* 527 (M+ NH4)⁺.

### Example 58. Preparation of N-(6-(5-(2,4-dioxo-3,4-dihydropyrimidin-1(2H)-yl)-2-methoxy-3-(1-methoxy-2-methylpropan-2-yl)phenyl)naphthalen-2-yl)methanesulfonamide (compound IB-L0-2.66).

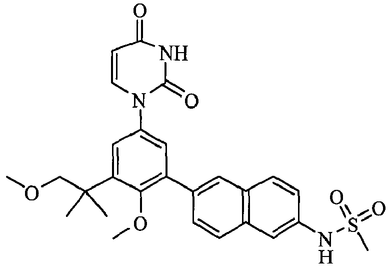

### Part A. Preparation of 1,5-diiodo-2-methoxy-3-(1-methoxy-2-methylpropan-2-yl)benzene.

To a 25mL round-bottom flask was added the product from **Example 57, Part E.** (259mg, 0.6mmol) and sodium hydride (28.8mg, 1.200mmol) in THF (5ml). The mixture was stirred for 30min and iodomethane (0.045 1, 0.720mmol) was added. The mixture was stirred for 16h and partitioned between ethyl acetate and 1M HCl. The organic layer was washed with saturated sodium bicarbonate, brine, dried with sodium sulfate, filtered and evaporated to give an oil (235mg, 88%).

### Part B. Preparation of 1-(3-iodo-4-methoxy-5-(1-methoxy-2-methylpropan-2-yl)phenyl)pyrimidine-2,4(1H,3H)-dione.

In a 25mL round-bottom flask was added the product from **Part A** (230mg, 0.516mmol), pyrimidine-2,4(1H,3H)-dione (63.6mg, 0.567mmol), N-(2-cyanophenyl)picolinamide (23.02mg, 0.103mmol), copper(I) iodide (9.82mg, 0.052mmol) and potassium phosphate (230mg, 1.083mmol) in DMSO (5ml). The vessel was sealed, sparged with nitrogen and heated at 60°C for 16h. The mixture was cooled and partitioned between ethyl acetate and 1M HCl. The organic layer was washed with saturated sodium bicarbonate, brine, dried with sodium sulfate, filtered and evaporated. The residue was chromatographed on silica gel eluting with 2% to 5% methanol in CH₂Cl₂ to give a solid (140mg, 63%).

### Part C. Preparation of N-(6-(5-(2,4-dioxo-3,4-dihydropyrimidin-1(2H)-yl)-2-methoxy-3-(1-methoxy-2-methylpropan-2-yl)phenyl)naphthalen-2-yl)methanesulfonamide.

In a 5ml microwave tube was added the product from **Part B** (43mg, O.l00mmol), the product from **Example 4A, Part B** (34.7mg, 0.100mmol), potassium phosphate (44.6mg, 0.210mmol), PA-Ph (CAS 97739-46-3) (0.876mg, 3.00µmol) and tris(dibenzylideneacetone)palladium(0) (0.915mg, 0.999µmol) in THF (3.0ml) and water (1.0ml). The vessel was sealed, sparged with nitrogen for 5min and heated at 50°C for 2h. The mixture was partitioned with ethyl acetate and 1M HCl. The organic layer was washed with saturated sodium bicarbonate, brine, dried with sodium sulfate, filtered. The filtrate was treated with 3-mercaptopropyl functionalized silica gel, filtered and evaporated. The residue was triturated with methanol/ CH₂Cl₂ (1:1) to give a solid (28mg, 54%). ¹H NMR (300 MHz, DMSO-D6) δ ppm 1.39 (s, 6 H) 3.08 (s, 3 H) 3.23 (s, 3 H) 3.25 (s, 3 H) 3.61 (s, 2 H) 5.65 (d, J=7.72 Hz, 1 H) 7.27 (d, J=2.57 Hz, 1 H) 7.37 (d, J=2.57 Hz, 1 H) 7.42 (dd, J=8.64, 2.02 Hz, 1 H) 7.69 (dd, J=8.46, 1.84 Hz, 1 H) 7.73 (d, J=2.21 Hz, 1 H) 7.78 (d, J=7.72 Hz, 1 H) 7.95 (t, J=8.27 Hz, 2 H) 8.02 (s, 1 H) 10.04 (s, 1 H) 11.41 (s, 1 H). MS (ESI+) m/z 541 (M+ NH4)⁺.

### Example 59. Preparation of methyl 2-(5-(2,4-dioxo-3,4-dihydropyrimidin-1(2H)-yl)-2-methoxy-3-(6-(methylsulfonamido)naphthalen-2-yl)phenyl)-2-methylpropanoate (compound IB-L0-2.70).

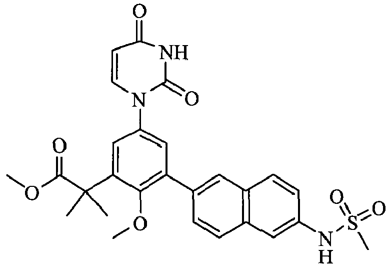

### Part A. Preparation of methyl 2-(5-(2,4-dioxo-3,4-dihydropyrimidin-1(2H)-yl)-3-iodo-2-methoxyphenyl)-2-methylpropanoate.

To a 100mL round-bottom flask under N₂ was added the product from **Example 57, Part C** (410mg, 0.891mmol), 1H-pyrimidine-2,4-dione (120mg, 1.069mmol), and potassium phosphate tribasic (397mg, 1.872mmol) in DMSO (5ml) to give a colorless suspension. N-(2-cyanophenyl) picolinamide (39.8mg, 0.178mmol) was added and the mix was sparged with N₂ for 5min. Copper(I) iodide (16.97mg, 0.089mmol) was added and the mix was sparged once again for 10min, placed under N₂ and heated at 60°C for 18h. The mixture was cooled and partitioned between EtOAc and water adjusting the pH to 1 with HCl. The aqueous layer was extracted 2X with EtOAc. The organics were combined, washed with water, saturated NaHCO₃, and saturated NaCl. The organic layer was dried (Na₂SO₄), treated with 3-mercaptopropyl functionalized silica, filtered and concentrated. The crude product was purified by chromatography on an ISCO 40 g silica cartridge eluting with 3% MeOH in CH₂Cl₂ to give a white foam (269mg, 68%).

### Part B. Preparation of methyl 2-(5-(2,4-dioxo-3,4-dihydropyrimidin-1(2H)-yl)-2-methoxy-3-(6-(methylsulfonamido)naphthalen-2-yl)phenyl)-2-methylpropanoate.

To a 20mL microwave tube was added the product from **Part A** (0.444g, 1.0mmol), the product from **Example 4A, Part B** (0.365g, 1.050mmol), and potassium phosphate tribasic (0.446g, 2.100mmol) in 3:1 tetrahydrofuran-water (12ml) and degassed by nitrogen sparge for 20min. The solution was then treated with PA-Ph (CAS 97739-46-3) (8.77mg, 0.030mmol) and tris(dibenzylidene-acetone)palladium(0) (9.16mg, 10.00µmol) followed by degassing for another 5min. The microwave tube was then sealed and warmed at 50°C for 18h, cooled and partitioned between EtOAc and water adjusting the pH to 1 with 1M HCl. The EtOAc layer was washed with water, saturated NaHCO₃, and saturated NaCl. The organic layer was dried over sodium sulfate, stirred for 1h with 3-mercaptopropyl functionalized silica, filtered and concentrated. The crude product was purified by chromatography on an ISCO 12g silica cartridge eluting with 1-3% MeOH in CH₂Cl₂to give light tan crystals (480mg, 98%). ¹H NMR (300 MHz, DMSO-D6) δ ppm 1.52 (s, 6 H) 3.08 (s, 3 H) 3.14 (s, 3 H) 3.64 (s, 3 H) 5.67 (dd, J=8.09, 1.84 Hz, 1 H) 7.37 - 7.48 (m, 3 H) 7.65 (dd, J=8.46, 1.84 Hz, 1 H) 7.73 (d, J=2.21 Hz, 1 H) 7.83 (d, J=8.09 Hz, 1 H) 7.96 (dd, J=8.64, 5.70 Hz, 2 H) 8.01 (s, 1 H) 10.05 (s, 1 H) 11.45 (s, 1 H). MS (ESI-) *m*/*z* 536 (M-H)⁺.

### Example 60. Preparation of 2-(5-(2,4-dioxo-3,4-dihydropyrimidin-1(2H)-yl)-2-methoxy-3-(6-(methylsulfonamido)naphthalen-2-yl)phenyl)-2-methylpropanoic acid (compound IB-L0-2.77).

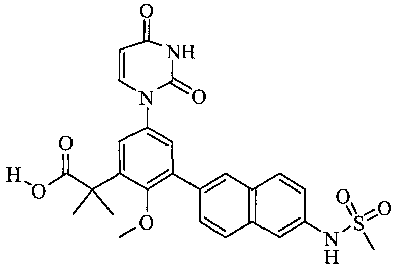

A mixture of the product from **Example 59** (108mg, 0.2mmol) and sodium hydroxide (1mL, 4.00mmol) in methanol, THF, water (3:3:1, 10mL) was heated at 80°C for 18h, cooled and carefully acidified to pH 1 with concentrated HCl resulting in the formation of a white precipitate. The solid was collected by filtration, washed with water and dried. The crude material was triturated in 1mL of 1:1 EtOAc/MeOH, sonicated for 5min and the solid was collected by filtration as a bright white solid (58mg, 54% yield), mp >300°C. ¹H NMR (300 MHz, DMSO-D6) δ ppm 1.50 (s, 6 H) 3.08 (s, 3 H) 3.18 (s, 3 H) 5.66 (d, J=7.72 Hz, 1 H) 7.34 - 7.45 (m, 3 H) 7.67 (dd, J=8.64, 1.65 Hz, 1 H) 7.73 (d, J=1.84 Hz, 1 H) 7.82 (d, J=7.72 Hz, 1 H) 7.96 (dd, J=9.01, 4.60 Hz, 2 H) 8.02 (s, 1 H) 10.04 (s, 1 H) 11.43 (s, 1 H) 12.15 (s, 1 H). MS (ESI-) *m*/*z* 522 (M-H)⁺.

### Example 61. Preparation of methyl 5-(2,4-dioxo-3,4-dihydropyrimidin-1(2H)-yl)-2-methoxy-3-(6-(methylsulfonamido)naphthalen-2-yl)benzoate (compound IB-L0-2.72).

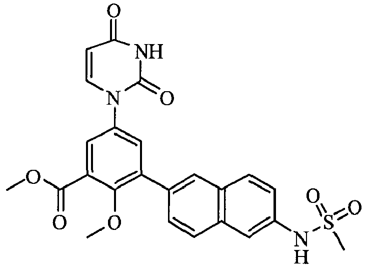

### Part A. Preparation of methyl 3,5-diiodo-2-methoxybenzoate.

A mixture of 2-hydroxy-3,5-diiodobenzoic acid (3.9g, 10.0mmol) potassium carbonate (4.15g, 30.0mmol) and dimethyl sulfate (2.77g, 22.0mmol) in acetone (33ml) was heated at reflux for 16h, cooled and concentrated. The residue was dissolved in EtOAc and washed with water, brine, dried (Na₂SO₄), filtered and concentrated to give an off-white solid (4.2g, quantitative yield).

### Part B. Preparation of methyl 5-(2,4-dioxo-3,4-dihydropyrimidin-1 (2H)-yl)-3-iodo-2-methoxybenzoate.

To a 100mL round-bottom flask under N₂ was added the product from **Part A** (2.09g, 5.0mmol), 1H-pyrimidine-2,4-dione (0.67g, 6.0mmol), and potassium phosphate tribasic (2.2g, 10.5mmol) in DMSO (20ml) to give a colorless suspension. N-(2-cyanophenyl)picolinamide (220mg, 1.0mmol) was added and the mix was sparged with N₂ for 5min. Copper(I) iodide (95mg, 0.5mmol) was added and the mix was sparged once again for 10min, placed under N₂ and heated at 60°C for 18h. The mixture was cooled and partitioned between EtOAc and water adjusting the pH to 1 with HCl. The aqueous layer was extracted 2X with EtOAc. The organics were combined, washed with water, saturated NaHCO₃, and saturated NaCl. The organic layer was dried (Na₂SO₄), treated with 3-mercaptopropyl functionalized silica, filtered and concentrated. The crude product was purified by chromatography on an ISCO 40g silica cartridge eluting with 3% MeOH in CH₂Cl₂ to give a white foam (1.0g, 50 %).

### Part C. Preparation of methyl 5-(2,4-dioxo-3,4-dihydropyrimidin-1(2H)-yl)-2-methoxy-3-(6-(methylsulfonamido)naphthalen-2-yl)benzoate.

A mixture of the product from **Part B** (101mg, 0.25mmol), the product from **Example 4A**, **Part B** (91mg, 0.263mmol), and potassium phosphate tribasic (111mg, 0.525mmol) in 3:1 tetrahydrofuran-water (12mL) was degassed by nitrogen sparge for 20min. The solution was then treated with PA-Ph (CAS 97739-46-3) (2.192mg, 7.50µmol) and tris(dibenzylideneacetone)palladium(0) (2.289mg, 2.500µmol) followed by degassing for another 5min. The microwave tube was then sealed, warmed at 50°C for 18h, cooled and partitioned between EtOAc and water adjusting the pH to 1 with 1M HCl. The EtOAc layer was washed with water, saturated NaHCO3, and saturated NaCl. The organic layer was dried Na₂SO₄, stirred for 1h with 3-mercaptopropyl functionalized silica, filtered and concentrated. The crude product was purified by chromatography on an ISCO 12g silica cartridge eluting with 3% MeOH in CH₂Cl₂ to give an off-white foam (80mg, 63 %). ¹H NMR (300 MHz, DMSO-D6) δ ppm 3.09 (s, 3 H) 3.45 (s, 3 H) 3.89 (s, 3 H) 5.69 (d, J=7.72 Hz, 1 H) 7.43 (dd, J=8.82, 2.21 Hz, 1 H) 7.68 - 7.79 (m, 4 H) 7.84 (d, J=7.72 Hz, 1 H) 7.89 - 8.01 (m, 2 H) 8.09 (s, 1 H) 10.06 (s, 1 H) 11.49 (s, 1 H). MS (ESI-) *m*/*z* 494 (M-H)⁺.

### Example 62. Preparation of N-(6-(5-(2,4-dioxo-3,4-dihydropyrimidin-1(2H)-yl)-3-iodo-2-methoxyphenyl)naphthalen-2-yl)methanesulfonamide (compound IB-L0-2.57).

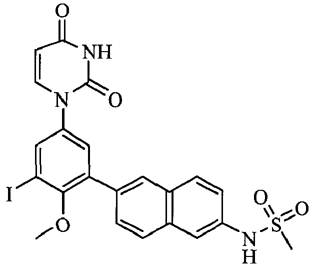

### Part A. Preparation of 1,3,5-triiodo-2-methoxybenzene.

In a 250mL pressure vessel was added 2,4,6-triiodophenol (5g, 10.60mmol) in MTBE (60ml) to give a yellow solution. The solution was cooled in an ice bath and 2.0M trimethylsilyldiazomethane (7.95ml, 15.90mmol) was added at a fast drip followed by dropwise addition of methanol (6mL) resulting in calm bubbling. The vessel was sealed and stirred at room temperature for 4h. The reaction solution was partitioned between EtOAc and water and the organic layer was washed with 1M HCl, saturated NaHCO₃, and saturated NaCl. The EtOAc was dried (MgSO₄), filtered and concentrated to give a tan solid that was used without purification (4.8g, 94 %).

### Part B. Preparation of 1-(3,5-diiodo-4-methoxyphenyl)pyrimidine-2,4(1H,3H)-dione.

To a 100mL round-bottom flask under N₂ was added the product from **Part A** (3.5g, 7.2mmol), 1H-pyrimidine-2,4-dione (0.97g, 8.64mmol), and potassium phosphate tribasic (3.2g, 15.0mmol) in DMSO (50ml) to give a colorless suspension. N-(2-cyanophenyl)picolinamide (320mg, 1.44mmol) was added and the mix was sparged with N₂ for 5min. Copper(I) iodide (137mg, 0.72mmol) was added and the mix was sparged once again for 10min, placed under N₂ and heated at 60°C for 18h. The mixture was cooled and partitioned between EtOAc and water adjusting the pH to 1 with HCl. The aqueous layer was extracted 2X with EtOAc. The organics were combined, washed with water, saturated NaHCO₃, and saturated NaCl, dried (Na₂SO₄), treated with 3-mercaptopropyl functionalized silica, filtered and concentrated. The resulting solid was triturated in 2:1 hexane/EtOAc to give an off white powder (2.2g, 62%).

### Part C. Preparation of N-(6-(5-(2,4-dioxo-3,4-dihydropyrimidin-1(2H)-yl)-3-iodo-2-methoxyphenyl)naphthalen-2-yl)methanesulfonamide.

A mixture of the product from **Part B** 1-(3,5-diiodo-4-methoxyphenyl)pyrimidine-2,4(1H,3H)-dione (118mg, 0.25mmol), the product from **Example 4A, Part B** (87mg, 0.25mmol), 1,1'-bis(diphenylphosphino)ferrocene-palladium(II)dichloride CH₂Cl₂ complex (10.21mg, 0.013mmol) and sodium carbonate (0.250ml, 0.25mmol) in toluene (1.0ml) and ethanol (1.0ml) was sparged with nitrogen for 5min and microwaved at 100°C for 30min. The mixture was cooled and partitioned with ethyl acetate and 1M HCl. The organic layer was washed with saturated sodium bicarbonate, brine, dried with sodium sulfate, filtered and evaporated. The residue was chromatographed on silica eluting with ethyl acetate/hexane (2:3 to 4:1) to give the title compound (16mg, 11%). ¹H NMR (300 MHz, DMSO-D6) δ ppm 3.08 (s, 3 H) 3.35 (s, 3 H) 5.67 (d, J=8.09 Hz, 1 H) 7.42 (dd, J=8.82, 2.21 Hz, 1 H) 7.59 (d, J=2.57 Hz, 1 H) 7.73 (m, 2 H) 7.81 (d, J=8.09 Hz, 1 H) 7.95 (m, 3 H) 8.09 (s, 1 H) 10.06 (s, 1 H) 11.47 (s, 1 H). MS (ESI-) *m*/*z* 562 (M-H)⁺.

### Example 63. Preparation of N-(6-(5-(2,4-dioxo-3,4-dihydropyrimidin-1(2H)-yl)-2-methoxy-3-((trimethylsilyl)ethynyl)phenyl)naphthalen-2-yl)methanesulfonamide (compound IB-L0-2.78).

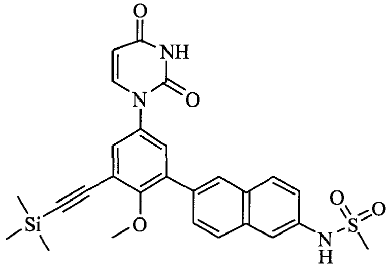

In a 5mL microwave tube were combined ethynyltrimethylsilane (0.044ml, 0.32mmol), the product from **Example 62** (45.1mg, 0.08mmol), copper(I) iodide (0.762mg, 4.0µmol), bis(triphenylphosphine)palladium(II) chloride (2.81mg, 4.0µmol) and triethylamine (0.056ml, 0.40mmol) in acetonitrile (2ml). The mixture was sparged with nitrogen for 5min, sealed and microwaved at 80°C for 20min. The reaction mixture was cooled and partitioned with ethyl acetate and water. The organic layer was washed with brine, dried with sodium sulfate, filtered and evaporated. The residue was chromatographed on silica eluting with 1-4% methanol in CH₂Cl₂ to give a solid, (18mg, 42%) m.p.175-178°C. ¹H NMR (300 MHz, DMSO-D6) δ ppm 0.25 (s, 9 H) 3.07 (s, 3 H) 3.65 (s, 3 H) 5.66 (dd, J=7.91, 2.02 Hz, 1 H) 7.41 (dd, J=8.82, 2.21 Hz, 1 H) 7.58 (m, 2 H) 7.69 (dd, J=8.46, 1.84 Hz, 1 H) 7.72 (d, J=2.21 Hz, 1 H) 7.81 (d, J=7.72 Hz, 1 H) 7.93 (m, 2 H) 8.05 (d, J=1.32 Hz, 1 H) 10.04 (s, 1 H) 11.45 (d, J=2.21 Hz, 1 H). MS (ESI+) *m*/*z* 534 (M+H)⁺.

### Example 64. Preparation of N-(6-(5-(2,4-dioxo-3,4-dihydropyrimidin-1(2H)-yl)-2-methoxy-3-(methylsulfonyl)phenyl)naphthalen-2-yl)methanesulfonamide (compound IB-L0-2.68).

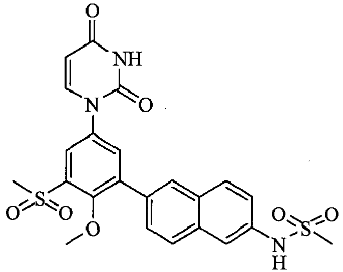

### Part A. Preparation of 4-nitrobenzene-2-diazo-1-oxide.

To a 250mL round-bottom flask was added 2-amino-4-nitrophenol (6.165g, 40.0mmol) in 48% tetrafluoroboric acid (15ml). Sodium nitrite (2.76g, 40.0mmol) in water (6ml) was added dropwise at 0°C and the mixture was stirred at room temperature for 30min. The solid was collected by filtration, washed with tetrafluoroboric acid and water. The solid was suspended in acetone (50ml), filtered and dried to give a solid (3.31g, 50%).

### Part B. Preparation of 2-(methylthio)-4-nitrophenol.

To a 1L beaker was added the product from **Part A** (2.70g, 16.35mmol) in ice water (250g) to give a brown suspension. Copper (0.520g, 8.18mmol) was added, followed by addition of sodium thiomethoxide (2.292g, 32.7mmol) in water (50ml) slowly. The mixture was stirred at room temperature for 24h. The mixture was filtered and the filtrate was acidified with 1M HCl producing a solid that was collected by filtration and dried (2.53g, 84%).

### Part C. Preparation of 2-(methylsulfonyl)-4-nitrophenol.

To a 250mL round-bottom flask was added the product from **Part B** (1.111g, 6.00mmol) in MeOH (20ml) to give a brown suspension. Oxone (7.746g, 12.60mmol) in water (20ml) was added slowly at 0°C. The mixture was warmed to room temperature, stirred for 1h and partitioned with ethyl acetate and 1M HCl. The organic layer was washed with brine, dried with sodium sulfate, filtered and evaporated. The residue was chromatographed on silica gel eluting with 1% to 5% methanol in CH₂Cl₂ to give a solid (0.472g, 36%).

### Part D. Preparation of 2-iodo-6-(methylsulfonyl)-4-nitrophenol.

To a 50mL round-bottom flask was added the product from **Part C** (470mg, 2.164mmol) in MeOH (10ml) and water (2.5ml). Iodine monochloride (0.130ml, 2.60mmol) in CH₂Cl₂ (2.0mL) was added dropwise and the mixture was stirred at room temperature, poured into water (200mL) and stirred for 10min. The resulting solid was collected by filtration and dried (636mg, 86%).

### Part E. Preparation of 1-iodo-2-methoxy-3-(methylsulfonyl)-5-nitrobenzene.

To a 50mL pressure vessel was added the product from **Part D** (630mg, 1.836mmol) in MTBE (6ml) to give a yellow solution. The mixture was cooled in an ice bath and 2M trimethylsilyldiazomethane (1.377ml, 2.75mmol) was added at a fast drip followed by dropwise addition of MeOH (0.4ml) resulting in calm bubbling. The vessel was sealed and stirred at room temperature for 1h. The mixture was partitioned with ethyl acetate and 1M HCl. The organic layer was washed with saturated sodium bicarbonate, brine, dried with sodium sulfate, filtered and evaporated to give an off-white solid (655mg, 100%).

### Part F. Preparation of 3-iodo-4-methoxy-5-(methylsulfonyl)aniline.

To a 250mL round-bottom flask was added the product from **Part E** (0.650g, 1.820mmol), ammonium chloride (0.146g, 2.73mmol), and iron (0.508g, 9.10mmol) in THF/MeOH/water (50ml, 2/2/1). The mixture was refluxed for 2h, cooled and filtered. The filtrate was evaporated and the residue was partitioned with ethyl acetate and water. The organic layer was washed with brine, dried with sodium sulfate, filtered and evaporated to give a solid (590mg, 99%).

### Part G. Preparation of (E)-N-(3-iodo-4-methoxy-5-(methylsulfonyl)phenylcarbamoyl)-3-methoxyacrylamide.

To a 100mL round-bottom flask was added the product from **Part F** (500mg, 1.528mmol) in DMF (15.0ml). The solution was cooled under nitrogen to -20°C and (E)-3-methoxyacryloyl isocyanate (15.28ml, 6.11mmol; prepared as described by Santana, L.; et al. J. Heterocyclic Chem. 1999, 36, 293-295) was added dropwise. The mixture was stirred at this temperature for 15min, then warmed to room temperature and stirred for 45min. The mixture was diluted with ethyl acetate and washed by water (3 x 50ml), brine (3 x 50ml), dried with sodium sulfate, filtered and evaporated. The residue was triturated with ethyl acetate/hexane to give a solid (425mg, 61%).

### Part H. Preparation of 1-(3-iodo-4-methoxy-5-(methylsulfonyl)phenyl)pyrimidine-2,4(1H,3H)-dione.

To a 100mL round-bottom flask was added the product from **Part G** (420mg, 0.925mmol) in ethanol (10ml) to give a suspension. Concentrated sulfuric acid (1mL, 18.76mmol) in water (10ml) was added and the mixture was heated at 110°C for 2h. The reaction mix was cooled, diluted with water (50ml) and stirred for 10min. The solid material was collected by filtration, washed with water and dried to give a white solid (325mg, 83%).

### Part I. Preparation of N-(6-(5-(2,4-dioxo-3,4-dihydropyrimidin-1(2H)-yl)-2-methoxy-3-(methylsulfonyl)phenyl)naphthalen-2-yl)methanesulfonamide.

To a 5mL microwave tube was added the product from **Part H** (63.3mg, 0.15mmol), the product from **Example 4A, Part B** (52.1mg, 0.150mmol), 1,1'-bis(diphenylphosphino)ferrocene-palladium(II)dichloride complex (6.12mg, 7.50µmol) and 1M sodium carbonate (0.150ml, 0.150mmol) in the solvents of toluene (1.0ml) and ethanol (1.0ml). The vessel was sealed and the mixture was sparged with nitrogen for 5min and microwaved at 100°C for 30min. The mixture was partitioned with ethyl acetate and 1M HCl. The organic layer was washed with saturated sodium bicarbonate, brine, dried with sodium sulfate, filtered and evaporated. The residue was purified on silica gel eluting with 1% to 8% methanol in CH₂Cl₂ to give crude product. A final trituration in 1:1 methanol/ethyl acetate afforded pure solid (26mg, 34%). ¹H NMR (300 MHz, DMSO-D6) δ ppm 3.10 (s, 3 H) 3.44 (s, 3 H) 3.45 (s, 3 H) 5.71 (d, J=8.09 Hz, 1 H) 7.44 (dd, J=8.82, 2.21 Hz, 1 H) 7.75 (d, J=1.84 Hz, 1 H) 7.80 (dd, J=8.46, 1.84 Hz, 1 H) 7.86 (d, J=8.09 Hz, 1 H) 7.91 (d, J=2.57 Hz, 1 H) 7.96 (d, J=2.57 Hz, 1 H) 8.00 (m, 2 H) 8.16 (d, J=1.47 Hz, 1 H) 10.10 (s, 1 H) 11.51 (s, 1 H). MS (ESI+) *m*/*z* 533 (M+NH4)⁺.

### Example 65. Preparation of N-(5-(2,4-dioxo-3,4-dihydropyrimidin-1(2H)-yl)-2-methoxy-3-(6-(methylsulfonamido)naphthalen-2-yl)phenyl)methanesulfonamide (compound IB-L0-2.75).

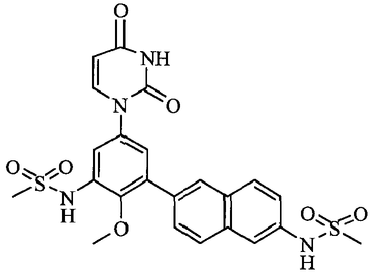

### Part A. Preparation of 2,4-diiodo-6-nitrophenol.

To a solution of 2-nitrophenol (2.78g, 20mmol) in MeOH (120ml) and water (30mL) was added dropwise a solution of iodine monochloride (2.105ml, 42.0mmol) in 10mL CH₂Cl₂. The mixture was stirred for 2h, poured into 600mL water, stirred and sonicated for 30min. The mixture was filtered to collect a yellow solid that was washed 3x with water (50mL each wash) and dried to constant mass (7.3g, 93%). m

### Part B. Preparation of 1,5-diiodo-2-methoxy-3-nitrobenzene.

A 50mL pressure vessel was charged with the product from **Part A** and MTBE (10ml) to give a yellow solution. The solution was cooled in an ice bath and 2M trimethylsilyldiazomethane (2.251ml, 4.50mmol) was added at a fast drop followed by dropwise addition of MeOH (0.6ml) resulting in calm bubbling. The vessel was sealed and stirred allowing warm to room temperature over 4h. The mixture was partitioned with ethyl acetate and 1M HCl. The organic layer was washed with saturated sodium bicarbonate, brine, dried with sodium sulfate, filtered and evaporated to give a yellow solid (1.22g, 100%).

### Part C. Preparation of 3,5-diiodo-2-methoxyaniline.

In a 250 round-bottom flask was added the product from **Part B** (0.98g, 2.420mmol), ammonium chloride (0.194g, 3.63mmol), and iron (0.676g, 12.10mmol) in THF/methanol/water (20ml/20ml/10ml). The mixture was refluxed for 16ho, cooled and filtered. The filtrate was evaporated and the residue was partitioned with water and ethyl acetate. The organic layer was dried with sodium sulfate, filtered and evaporated to give an oil (780mg, 86%).

### Part D. Preparation of 1-(3-amino-5-iodo-4-methoxyphenyl)pyrimidine-2,4(1H,3H)-dione.

In a 25mL round-bottom flask was added the product from **Part C** (650mg, 1.734mmol), pyrimidine-2,4(1H,3H)-dione (214mg, 1.907mmol), N-(2-cyanophenyl)picolinamide (77mg, 0.347mmol), copper(I) iodide (33.0mg, 0.173mmol) and potassium phosphate (773mg, 3.64mmol) in DMSO (5ml). The vessel was sealed and the mixture was sparged with nitrogen for 15min and heated at 60°C for 16h. The mixture was partitioned with ethyl acetate and 1M HCl. The organic layer was washed with saturated sodium bicarbonate, brine, dried with sodium sulfate and filtered. The filtrate was treated with 3-mercaptopropyl functionalized silica gel, filtered and evaporated. The residue was chromatographed on silica eluting with 5:95 methanol/D CH₂Cl₂CM to give a solid (125mg, 20%).

### Part E. Preparation of N-(5-(2,4-dioxo-3,4-dihydropyrimidin-1(2H)-yl)-3-iodo-2-methoxyphenyl)methanesulfonamide.

A solution of the product from **Part D** (110mg, 0.306mmol) in pyridine (2ml) was treated with methanesulfonyl chloride (0.048ml, 0.612mmol) and stirred for 24h. The solvent was evaporated and the residue was partitioned with ethyl acetate and 1M HCl. The organic layer was washed with brine, dried with sodium sulfate, filtered and evaporated. The residue was purified on silica gel eluting with 2% to 5% methanol in CH₂Cl₂ to give a solid (20mg, 15%).

### Part F. Preparation of N-(5-(2,4-dioxo-3,4-dihydropyrimidin-1(2H)-yl)-2-methoxy-3-(6-(methylsulfonamido)naphthalen-2-yl)phenyl)methanesulfonamide.

To a 5mL microwave tube was added the product from **Part E** (18mg, 0.041mmol), **Example 4A, Part B** (14.30mg, 0.041mmol), potassium phosphate (18.35mg, 0.086mmol), PA-Ph (CAS 97739-46-3) (0.361mg, 1.235µmol) and tris(dibenzylideneacetone)dipalladium(0) (0.377mg, 0.412µmol) in THF (3.0ml) and water (1.0ml). The vessel was sealed and the mixture was sparged with nitrogen for 5min and heated at 50°C for 2h. The mixture was partitioned with ethyl acetate and 1M HCl. The organic layer was washed with saturated sodium bicarbonate, brine, dried with sodium sulfate, filtered and evaporated. The residue was purified on silica gel eluting with 2% to 5% methanol in CH₂Cl₂ to give a solid. A final trituration in 1:1 methanol/ CH₂Cl₂ gave the desired product (7mg, 32%). ¹H NMR (300 MHz, DMSO-D6) δ ppm 3.09 (s, 3 H) 3.17 (s, 3 H) 3.37 (s, 3 H) 5.69 (dd, J=7.91, 2.02 Hz, 1 H) 7.34 (d, J=2.57 Hz, 1 H) 7.43 (dd, J=8.82, 2.21 Hz, 1 H) 7.47 (d, J=2.57 Hz, 1 H) 7.73 (m, 2 H) 7.81 (d, J=8.09 Hz, 1 H) 7.94 (d, J=6.25 Hz, 1 H) 7.97 (d, J=6.62 Hz, 1 H) 8.07 (s, 1 H) 9.45 (s, 1 H) 10.05 (s, 1 H) 11.45 (d, J=1.84 Hz, 1 H). MS (ESI-) *m*/*z* 529 (M-H).

### Example 66. Preparation of N-(6-(5-(2,4-dioxo-3,4-dihydropyrimidin-1(2H)-yl)-2-methoxy-3-(trifluoromethyl)phenyl)naphthalen-2-yl)methanesulfonamide (compound IB-L0-2.56).

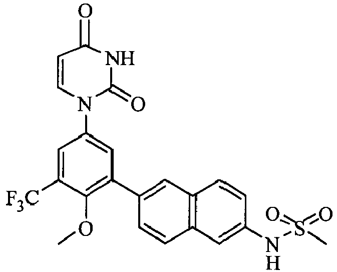

### Part A. Preparation of 4-iodo-2-(trifluoromethyl)phenol.

To a solution of 2-(trifluoromethyl) phenol (3.24g, 20mmol) in MeOH (40ml) was added sodium hydroxide (0.960g, 24.0mmol) and stirred until the hydroxide was dissolved. The mixture was cooled to 0°C and sodium iodide was added (3.0g, 20mmol) followed by dropwise addition of 10% aqueous sodium hypochlorite (9.0ml, 14.6mmol). The addition of sodium iodide followed by sodium hypochlorite was repeated twice more. The mixture was stirred at ambient temperature for 2h and treated dropwise with concentrated HCl to pH 1. The mixture was extracted 3 X with EtOAc. The extracts were combined, washed with brine, dried with sodium sulfate, filtered and evaporated. The residue was purified on silica gel eluting with EtOAc/hexane (1:9) to give the mono-iodo product (5.0 g, 87%).

### Part B. Preparation of 2-bromo-4-iodo-6-(trifluoromethyl)phenol.

In a 250mL round-bottom flask was added the product from **Part A** (5.00g, 17.36mmol) and 1,3-dibromo-5,-dimethylhydantoin (2.73g, 9.55mmol) in CHCl₃ (80ml) to give an orange solution. The mixture was stirred for 2h, washed with water, brine, dried with sodium sulfate, filtered and evaporated. The crude product was purified on silica gel eluting with ethyl acetate/hexane (5:95) to give a solid (3.5g, 54%).

### Part C. Preparation of 1-bromo-5-iodo-2-methoxy-3-(trifluoromethyl)benzene.

A mixture of the product from **Part B** (3.2g, 8.72mmol), iodomethane (1.36ml, 21.8mmol), and 50% sodium hydroxide (0.507ml, 9.59mmol) in acetone (20ml) was stirred for 24h. The solvent was evaporated and the residue was partitioned with ethyl acetate and water. The organic layer was washed with brine, dried with sodium sulfate, filtered and evaporated. The crude material was purified on silica gel eluting with ethyl acetate/hexane (5:95) to give a solid (2.67g, 80%).

### Part D. Preparation of 1-(3-bromo-4-methoxy-5-(trifluoromethyl)phenyl)pyrimidine-2,4 (1H,3H)-dione.

In a 20mL microwave tube was added the product from **Part C** (762mg, 2.0mmol), pyrimidine-2,4(1H,3H)-dione (247mg, 2.2mmol), N-(2-cyanophenyl)picolinamide (89mg, 0.4mmol), copper(I) iodide (38.1mg, 0.2mmol) and potassium phosphate (892mg, 4.2mmol) in DMSO (10ml). The vessel was sealed and the mixture was sparged with nitrogen for 15min and heated at 60°C for 16h. The mixture was partitioned with ethyl acetate and 1M HCl. The organic layer was washed with saturated sodium bicarbonate, brine, dried with sodium sulfate and filtered. The filtrate was treated with 3-mercaptopropyl functionalized silica gel, filtered and evaporated. The residue was purified on silica gel eluting with ethyl acetate/hexane (2:3) to give the desired product (63mg, 9%).

### Part E. Preparation of N-(6-(5-(2,4-dioxo-3,4-dihydropyrimidin-1(2H)-yl)-2-methoxy-3-(trifluoromethyl)phenyl)naphthalen-2-yl)methanesulfonamide.

In a 5mL microwave tube was added the product from **Part D** (60mg, 0.164mmol), the product from **Example 4A, Part B** (62.8mg, 0.181mmol), 1,1'-bis(di-tert-butylphosphino)ferrocene palladium dichloride (5.36mg, 8.22µmol) and potassium phosphate (69.8mg, 0.329mmol) in THF/water (3ml/1ml). The vessel was sealed and the mixture was sparged with nitrogen for 5min and heated at 60°C for 2h. The mixture was partitioned with ethyl acetate and 1M HCl. The organic layer was washed with saturated sodium bicarbonate, brine, dried with sodium sulfate and filtered. The filtrate was treated with 3-mercaptopropyl functionalized silica gel, filtered and evaporated. The residue was purified by reverse phase chromatography to give the title compound as a solid (26mg, 31%). ¹H NMR (300 MHz, DMSO-D6) δ ppm 3.10 (s, 3 H) 3.37 (s, 3 H) 5.71 (dd, J=7.72, 2.21 Hz, 1 H) 7.44 (dd, J=8.82, 2.21 Hz, 1 H) 7.75 (s, 1 H) 7.78 (d, J=1.84 Hz, 1 H) 7.88 (m, 3 H) 7.98 (d, J=3.31 Hz, 1 H) 8.01 (d, J=3.68 Hz, 1 H) 8.15 (s, 1 H) 10.09 (s, 1 H) 11.51 (d, J=2.21 Hz, 1 H). MS (ESI-) *m*/*z* 504.1 (M-H)⁺.

### Example 67. Preparation of N-(6-(5-(2,4-dioxo-3,4-dihydropyrimidin-1(2H)-yl)-2-methoxy-3-(perfluoroethyl)phenyl)naphthalen-2-yl)methanesulfonamide (compound IB-L0-2.60).

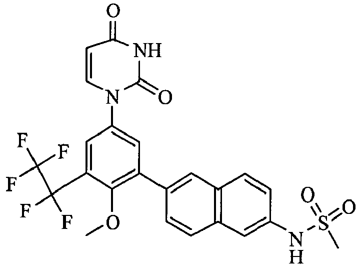

### Part A. Preparation of 1-methoxy-4-nitro-2-(perfluoroethyl)benzene.

To a 250mL round-bottom flask was added 2-bromo-1-methoxy-4-nitrobenzene (3.5g, 15.08mmol), copper(I) iodide (5.75g, 30.2mmol), and sodium 2,2,3,3,3-pentafluoropropanoate (5.25g, 28.2mmol) in DMF (75ml) and toluene (25ml) to give a tan suspension. The mixture was heated at 150°C and toluene was removed by a Dean-Stark trap. The mixture was heated at 155°C for 6h under nitrogen, cooled and poured into 100mL of water and 100mL of ether, filtered through a 1-inch plug of Celite and the plug was rinsed with ether. The filtrate layers were separated. The organic layer was washed with brine, dried (Na₂SO₄) filtered and concentrated. The dark oil was flash chromatographed on an Isco 40g silica cartridge eluting with 4:1 hexane/EtOAc to give a yellow oil that was a (3:1) mix of desired material and starting material (1.5g, 37%).

### Part B. Preparation of 4-nitro-2-(perfluoroethyl)phenol.

In a 100mL round-bottom flask was added the product from **Part A** (1.4g, 5.16mmol) and pyridine hydrochloride (4g, 34.6mmol) neat. The mixture was heated at 210°C for 20min, cooled, and partitioned between EtOAc and water. The organic layer was washed with brine, dried (Na₂SO₄) and concentrated. The crude product was flash chromatographed on an Isco 12g silica cartridge eluting with 3:2 hecxane/EtOAc to give a yellow oil (1.3g, 98%).

### Part C. Preparation of 2-iodo-4-nitro-6-(perfluoroethyl)phenol.

In a 100mL round-bottom flask was added the product from **Part B** (1.3g, 5.06mmol) and N-iodosuccinimide (1.251g, 5.56mmol) in acetonitrile (16.85ml) to give a yellow solution. The solution was stirred for 16h, diluted with 100mL EtOAc and washed 2 x 50ml with 10% sodium thiosulfate, brine, dried (Na₂SO₄) and concentrated to an orange semisolid. The semisolid was flash chromatographed on an Isco 40g silica cartridge eluting with 3:1 hexane EtOAc to give a deep yellow/orange oil (1.3g, 67%).

### Part D. Preparation of 1-iodo-2-methoxy-5-nitro-3-(perfluoroethyl)benzene.

In a 100mL round-bottom flask was added the product from **Part C** (1.04g, 2.72mmol) potassium carbonate (0.563g, 4.07mmol) and dimethyl sulfate (0.411g, 3.26mmol) in acetone (20ml) to give a brown suspension. The mixture was heated at gentle reflux for 16h, cooled, diluted into EtOAc, washed with water and brine. The organic layer was dried Na2SO4, filtered and concentrated to a yellow oil that was purified by flash chromatography on an Isco 40g silica cartridge eluting with 9:1 hexane/EtOAc (600mg, 56%).

### Part E. Preparation of 3-iodo-4-methoxy-5-(perfluoroethyl)aniline.

In a 250mL round-bottom flask was added the product from **Part D** (0.6g, 1.511mmol), iron (0.422g, 7.56mmol), and ammonium chloride (0.121g, 2.267mmol) in a solvent mix of EtOH (9ml), THF (9ml) and water (3ml) to give a brown suspension that was heated at 95-100°C for 2h. The reaction mix was filtered through a plug of Celite and the Celite was rinsed repeatedly with EtOH. The filtrate was concentrated and the residue was dissolved in EtOAc, washed with water, brine, dried (Na₂SO₄), filtered and concentrated to give an oil (560mg, 99%).

### Part F. Preparation of 1,5-diiodo-2-methoxy-3-(perfluoroethyl)benzene.

In a 25mL round-bottom flask under nitrogen was added the product from **Part E** (0.565g, 1.539mmol), tert-butyl nitrite (0.293ml, 2.463mmol), copper(I) iodide (0.293g, 1.539mmol), sodium iodide (0.231g, 1.539mmol) and iodine (0.195g, 0.770mmol) in DME (15.39ml) to give a brown suspension. The mixture was heated at 60°C for 3h, cooled and filtered through Celite washing the Celite pad well with EtOAc. The EtOAc filtrate was treated with 10% sodium thiosulfate, brine, dried (Na₂SO₄), filtered and concentrated to a dark oil. The crude material was purified by flash chromatography on an Isco 40g silica cartridge eluting with 95:5 hexane/EtOAc to give a yellow oil (360mg, 49%).

### Part G. Preparation of 1-(3-iodo-4-methoxy-5-(perfluoroethyl)phenyl)pyrimidine-2,4(1H,3H)-dione.

In a 20mL microwave tube was added the product from **Part F** (0.36g, 0.753mmol), 1H-pyrimidine-2,4-dione (0.101g, 0.904mmol), potassium phosphate tribasic (0.336g, 1.582mmol) N-(2-cyanophenyl)picolinamide (0.034g, 0.151mmol) and copper(I) iodide (0.014g, 0.075mmol in DMSO (7ml). The vessel was sealed and the mixture was sparged with N₂ for 30min, heated at 60°C for 24h, cooled and diluted into EtOAc. The EtOAc layer was washed with 1M HCl, saturated NaHCO3, and saturated NaCl, dried (Na₂SO₄), filtered and concentrated. The residue was flash chromatographed on an Isco 40g silica cartridge eluting with hexane --> 1:1 hexane/EtOAc to give a yellow foam (100mg, 29%).

### Part H. Preparation of N-(6-(5-(2,4-dioxo-3,4-dihydropyrimidin-1(2H)-yl)-2-methoxy-3-(perfluoroethyl)phenyl)naphthalen-2-yl)methanesulfonamide.

In a 5mL microwave tube were combined the product from **Part G** (0.10g, 0.216mmol), **Example 4A, Part B** (0.075g, 0.216mmol), and potassium phosphate tribasic (0.096g, 0.454mmol) in 3:1 tetrahydrofuran-water (5mL) and degassed by nitrogen sparge for 10min. The mixture was then treated with PA-Ph (CAS 97739-46-3) (1.898mg, 6.49µmol) and tris(dibenzylideneacetone)dipalladium(0) (1.982mg, 2.164µmol) followed by degassing for another 5min. The flask was then sealed and stirred at 50°C for 16h and partitioned between EtOAc and water. The EtOAc layer was washed with 0.1M HCl, saturated NaHCO₃, and saturated NaCl. The organic was dried Na₂SO₄, stirred for 0.5h with 3-mercapto-propyl functionalized silica to remove metals, filtered and concentrated. The crude product was purified by chromatography on an Isco 12g silica cartridge eluting with CH₂Cl₂ --> 3% MeOH in CH₂Cl₂ to give a light yellow foam (84mg, 99%) m.p. 162-165°C. ¹H NMR (300 MHz, DMSO-D6) δ ppm 3.10 (s, 3 H) 3.33 (s, 3 H) 5.70 (d, J=7.72 Hz, 1 H) 7.44 (dd, J=8.82, 2.21 Hz, 1 H) 7.70 - 7.76 (m, 2 H) 7.80 (d, J=2.57 Hz, 1 H) 7.86 (d, J=8.09 Hz, 1 H) 7.91 (d, J=2.57 Hz, 1 H) 8.00 (dd, J=8.82, 2.94 Hz, 2 H) 8.12 (s, 1 H) 10.10 (s, 1 H) 11.50 (s, 1 H). MS (ESI-) *m*/*z* 554 (M-H)⁺.

### Example 68. Preparation of (E)-N'-(6-(5-(2,4-dioxo-3,4-dihydropyrimidin-1(2H)-yl)-2-methoxy-3-(thiophen-2-yl)phenyl)-2,3-dihydro-1H-inden-1-ylidene)methanesulfonohydrazide (compound IB-L0-2.51).

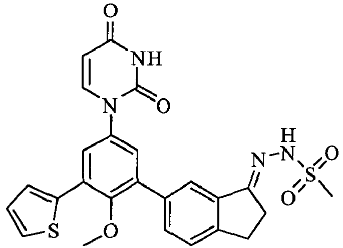

### Part A. Preparation of 1-(3-bromo-4-methoxy-5-(1-oxo-2,3-dihydro-1H-inden-5-yl)phenyl)-pyrimidine-2,4(1H,3H)-dione.

In a 100mL round-bottom flask was added the product from **Example 48, Part C** (846mg, 2.00mmol), **Example 6, Part A** (516mg, 2.000mmol), potassium phosphate (892mg, 4.20mmol), PA-Ph (CAS 97739-46-3) (17.54mg, 0.060mmol) and tris(dibenzylideneacetone)-dipalladium(0) (18.31mg, 0.020mmol) in THF (12.0ml) and water (4.0ml). The vessel was sealed and the mixture was sparged with nitrogen for 5min and stirred at ambient temperature for 72h. The mixture was partitioned with ethyl acetate and 1M HCl. The organic layer was washed with saturated sodium bicarbonate, brine, dried with sodium sulfate and filtered. The filtrate was treated with 3-mercaptopropyl functionalized silica gel, filtered through celite and evaporated. The residue was purified with silica gel eluting with 1 to 4% methanol in CH₂Cl₂ to give a solid (690mg, 81%).

### Part B. Preparation of (E)-N'-(5-(3-bromo-5-(2,4-dioxo-3,4-dihydropyrimidin-1(2H)-yl)-2-methoxyphenyl)-2,3-dihydro-1H-inden-1-ylidene)methanesulfonohydrazide.

In a 50mL round-bottom flask was added the product from **Part A** (685mg, 1.603mmol) and methanesulfonohydrazide (194mg, 1.764mmol) in MeOH (20ml). The mixture was warmed to 40°C and stirred for 24h. The mixture was cooled, filtered and washed with methanol to give a solid (569mg, 68%).

### Part C. Preparation of (E)-N'-(6-(5-(2,4-dioxo-3,4-dihydropyrimidin-1(2H)-yl)-2-methoxy-3-(thiophen-2-yl)phenyl)-2,3-dihydro-1H-inden-1-ylidene)methanesulfonohydrazide.

In a 5mL microwave tube was added the product from **Part B** (52mg, 0.100mmol), thiophen-2-ylboronic acid (12.81mg, 0.100mmol), 1,1'-bis(di-tert-butylphosphino)ferrocene palladium dichloride (3.26mg, 5.01µmol) and potassium phosphate (42.5mg, 0.200mmol) in THF (3.0ml) and water (1.0ml). The mixture was sparged by nitrogen for 5min and heated at 50°C for 3h. The mixture was partitioned with ethyl acetate and 1M HCl. The organic layer was washed with saturated sodium bicarbonate, brine, dried with sodium sulfate and filtered. The filtrate was treated with 3-mercaptopropyl functionalized silica gel, filtered through celite and evaporated. The residue was purified by reverse phase chromatography AA method to give a white solid (27mg, 52%). ¹H NMR (300 MHz, DMSO-D6) δ ppm 2.86 (m, 2 H) 3.09 (s, 3 H) 3.14 (m, 2 H) 3.32 (s, 3 H) 5.69 (d, J=7.72 Hz, 1 H) 7.18 (dd, J=5.15, 3.68 Hz, 1 H) 7.41 (d, J=2.57 Hz, 1 H) 7.63 (m, 3 H) 7.75 (m, 2 H) 7.86 (d, J=8.09 Hz, 1 H) 7.91 (d, J=2.94 Hz, 1 H) 9.96 (s, 1 H) 11.48 (s, 1 H). MS (ESI+) *m*/*z* 523 (M+H)⁺.

### Example 69. Preparation of (E)-N'-(6-(5-(2,4-dioxo-3,4-dihydropyrimidin-1(2H)-yl)-3-(furan-2-yl)-2-methoxyphenyl)-2,3-dihydro-1H-inden-1-ylidene)methanesulfonohydrazide (compound IB-L0-2.55).

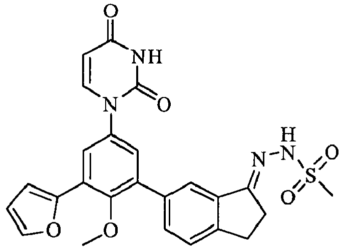

In a 5ml microwave tube was added the product from **Example 68, Part B** (52mg, 0.100mmol), furan-2-ylboronic acid (11.20mg, 0.100mmol), 1,1'-bis(di-tert-butylphosphino)ferrocene palladium dichloride (3.26mg, 5.01µmol) and potassium phosphate (42.5mg, 0.200mmol) in THF (3.0ml) and water (1.0ml). The mixture was sparged by nitrogen for 5min and heated at 50°C for 3h. The mixture was partitioned with ethyl acetate and 1M HCl. The organic layer was washed with saturated sodium bicarbonate, brine, dried with sodium sulfate and filtered. The filtrate was treated with 3-mercaptopropyl functionalized silica gel, filtered through celite and evaporated. The residue was purified by reverse phase chromatography AA method to give a solid (24mg, 47%). ¹H NMR (300 MHz, DMSO-D6) δ ppm 2.86 (m, 2 H) 3.09 (s, 3 H) 3.14 (m, 2 H) 3.36 (s, 3 H) 5.68 (d, J=8.09 Hz, 1 H) 6.69 (dd, J=3.31, 1.84 Hz, 1 H) 7.09 (d, J=3.31 Hz, 1 H) 7.41 (d, J=2.57 Hz, 1 H) 7.62 (m, 2 H) 7.75 (d, J=8.09 Hz, 1 H) 7.80 (d, J=2.57 Hz, 1 H) 7.86 (m, 2 H) 9.97 (s, 1 H) 11.46 (s, 1 H). MS (ESI+) *m*/*z* 507 (M+H)⁺.

### Example 70. Preparation of N-(6-(3-tert-butyl-5-(2,4-dioxo-3,4-dihydropyrimidin-1(2H)-yl)-2-ethoxyphenyl)naphthalen-2-yl)methanesulfonamide (compound IB-L0-2.23).

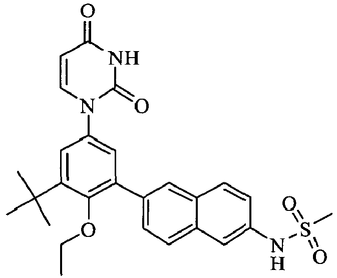

### Part A. Preparation of 2-tert-butyl-4-iodophenol.

To a 250mL round-bottom flask was added 2-tert-butylphenol (3.76g, 25mmol) in MeOH (50.0ml) to give a colorless solution. Sodium hydroxide (1.200g, 30.0mmol) was added and the mix was stirred until the hydroxide was completely dissolved. The solution was cooled to 0°C and treated with sodium iodide (1.75g, 11.6mmol) followed by dropwise addition of 10% sodium hypochlorite solution (7.2ml, 11.6mmol). The addition of sodium iodide followed by sodium hypochlorite was repeated twice and the mixture was stirred at 0°C for 30min. The mixture was treated with 10% w/w solution of sodium thiosulfate, stirred for 30min and treated with concentrated HCl dropwise to a constant pH of 1. The mixture was extracted 3X with EtOAc. The extracts were combined, washed with brine, dried (MgS04), filtered and concentrated. The crude oil was flash chromatographed on an ISCO 80g silica cartridge eluting with hexane --> 4:1 hexane/EtOAc to give a yellow oil (5.2g, 75%).

### Part B. Preparation of 2-bromo-6-tert-butyl-4-iodophenol.

To a 250mL round-bottom flask was added the product from **Part A** (4.8g, 17.38mmol) and 1,3-dibromo-5,5-dimethylhydantoin (2.61g, 9.13mmol) in chloroform (87ml) to give an orange solution. The reaction mixture was stirred for 2h resulting in a black solution that was washed with water, brine, dried (Na₂SO₄) and concentrated. The black oil was flash chromatographed on a 120g Isco silica cartridge eluting with hexane to give a pinkish solid (4.84g, 78%).

### Part C. Preparation of 1-bromo-3-tert-butyl-2-ethoxy-5-iodobenzene.

To a 50mL round-bottom flask was added the product from **Part B** (888mg, 2.5mmol), ethyl iodide (409mg, 2.63mmol), and potassium carbonate (415mg, 3.00mmol) in acetone (12ml) to give a green suspension. The mixture was heated at reflux for 16h, cooled and concentrated. The residue was partitioned between water and EtOAc. The organic layer was washed twice with brine, dried over Na₂SO₄, filtered and concentrated to a red oil. The oil was flash chromatographed on an Isco 40g silica cartridge eluting with hexane to give a clear oil (820mg, 86%).

### Part D. Preparation of 1-(3-bromo-5-tert-butyl-4-ethoxyphenyl)pyrimidine-2,4(1H,3H)-dione.

In a 20mL microwave tube under nitrogen flush was added the product from **Part C** (0.4g, 1.044mmol), 1H-Pyrimidine-2,4-dione (0.140g, 1.253mmol), and potassium phosphate tribasic (0.465g, 2.193mmol) in DMSO (5ml) to give a colorless suspension. N-(2-cyanophenyl)picolinamide (0.047g, 0.209mmol) was added and the mix was sparged with nitrogen for 10min. Copper(I) iodide (0.020g, 0.104mmol) was added and the mix was sparged once again for 10min, placed under nitrogen and heated at 60°C for 18h. The mixture was cooled and partitioned between EtOAc and water adjusting the pH to 1 with HCl. The aqueous layer was extracted 2X with EtOAc. The organics were combined, washed with water, saturated NaHCO₃, and saturated NaCl. The organic layer was dried (Na₂SO₄), stirred with 3-mercaptopropyl functionalized silica for 1h, filtered and concentrated. The crude product was purified by chromatography on an ISCO 12g silica cartridge eluting with 2% MeOH in CH₂Cl₂ to give a white powder (266mg, 69%).

### Part E. Preparation of N-(6-(3-tert-butyl-5-(2,4-dioxo-3,4-dihydropyrimidin-1(2H)-yl)-2-ethoxyphenyl)naphthalen-2-yl)methanesulfonamide.

In a 5mL microwave tube was added the product from **Part D** (55.1mg, 0.15mmol), the product from **Example 4A, Part B** (52.1mg, 0.150mmol), potassium phosphate tribasic (63.7mg, 0.300mmol) and 1,1'-bis(di-tert-butylphosphino)ferrocene palladium dichloride (4.89mg, 7.50µmol) in THF (3ml) water (1ml). The mixture was sparged for 10min with nitrogen, heated sealed at 50°C for 4h, cooled and diluted into EtOAc. The EtOAc layer was washed with 1M HCl, saturated NaHCO₃, saturated NaCl, dried (Na₂SO₄) and treated simultaneously with mercaptopropyl silica gel, filtered and concentrated. The residue was flash chromatographed on a 12g Isco silica cartridge eluting with 2% MeOH in CH₂Cl₂ to give a solid, (16mg, 21%) m.p. 196-202°C. ¹H NMR (300 MHz, DMSO-D6) δ ppm 1.00 (t, J=6.99 Hz, 3 H) 1.44 (s, 9 H) 3.09 (s, 3 H) 3.43 (q, J=7.11 Hz, 2 H) 5.64 (dd, J=7.91, 1.29 Hz, 1 H) 7.32 (d, J=2.94 Hz, 1 H) 7.36 (d, J=2.94 Hz, 1 H) 7.41 (dd, J=8.82, 2.21 Hz, 1 H) 7.72 (s, 1 H) 7.74 (d, J=1.47 Hz, 1 H) 7.80 (d, J=7.72 Hz, 1 H) 7.90 - 8.00 (m, 2 H) 8.05 (s, 1 H) 10.04 (s, 1 H) 11.41 (s, 1 H). MS (ESI-) *m*/*z* 506 (M-H)⁺.

### Example 71. Preparation of N-(6-(3-tert-butyl-2-chloro-5-(2,4-dioxo-3,4-dihydropyrimidin-1 (2H)-yl)phenyl)naphthalen-2-yl)methanesulfonamide (compound IB-L0-2.14).

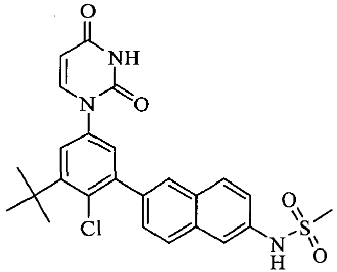

### Part A. Preparation of 2-bromo-6-tert-butyl-4-iodoaniline.

In a 50mL round-bottom flask was added 2-bromo-6-tert-butylaniline [prepared by the method of Onitsuka, et.al. Organometallics, 25(5), 2006, pp 1270-1278] (1.18g, 5.17mmol) and sodium bicarbonate (0.782g, 9.31mmol) in water (5ml). The mixture was cooled in an ice bath and iodine (1.444g, 5.69mmol) was added in several portions. The mixture was warmed to ambient temperature and stirred for 16h. The mixture was treated with aqueous sodium thiosulfate, extracted by ethyl acetate, dried with sodium sulfate, filtered and evaporated. The residue was purified on silica gel eluting with 5% ethyl acetate in hexane to give an oil (1.2g, 65%).

### Part B. Preparation of 1-bromo-3-tert-butyl-2-chloro-5-iodobenzene.

To a mixture oftert-butyl nitrite (0.18ml, 1.5mmol) and copper(II) chloride (161mg, 1.2mmol) in acetonitrile (5mL) was added the product from **Part A** (354mg, 1.0mmol) as a solution in acetonitrile (5mL). The mixture was heated at 60°C for 30min, cooled, partitioned with ethyl acetate and 1M HCl. The organic layer was washed with brine, dried with sodium sulfate, filtered and evaporated. The residue was purified on silica gel eluting with 5% ethyl acetate in hexane to give the product (300mg, 81%).

### Part C. Preparation of 1-(3-bromo-5-tert-butyl-4-chlorophenyl)pyrimidine-2,4(1H,3H)-dione.

In a 20mL microwave tube was added the product from **Part B** (300mg, 0.803mmol), pyrimidine-2,4(1H,3H)-dione (99mg, 0.884mmol), N-(2-cyanophenyl)picolinamide (35.9mg, 0.161mmol), copper(I) iodide (15.30mg, 0.080mmol) and potassium phosphate (358mg, 1.687mmol) in DMSO (5ml). The mixture was sealed, purged with nitrogen and heated at 60°C for 4h. The mixture was partitioned with ethyl acetate and 1M HCl. The organic layer was washed with saturated sodium bicarbonate, brine, dried with sodium sulfate and filtered. The filtrate was treated with 3-mercaptopropyl functionalized silica gel, filtered and evaporated. The residue was purified on silica gel eluting with 10% to 40% ethyl acetate in hexane to give a solid (175mg, 61 %).

### Part D. Preparation of N-(6-(3-tert-butyl-2-chloro-5-(2,4-dioxo-3,4-dihydropyrimidin-1(2H)-yl)phenyl)naphthalen-2-yl)methanesulfonamide.

In a 5mL microwave tube was added the product from **Part C** (35.8mg, 0.10mmol), the product from **Example 4A, Part B** (38.2mg, 0.110mmol), 1,1'-bis(di-tert-butylphosphino)ferrocene palladium dichloride (3.26mg, 5.00µmol) and potassium phosphate (42.5mg, 0.200mmol) in THF/Water (3ml:1ml). The mixture was purged with nitrogen for 5min and heated at 60°C for 2h. The mixture was partitioned with ethyl acetate and 1M HCl. The organic layer was washed with saturated sodium bicarbonate, brine, dried with sodium sulfate and filtered. The filtrate was treated with 3-mercaptopropyl functionalized silica gel, filtered and evaporated. The residue was purified on silica gel eluting with 1:1 ethyl acetate/hexane to give a solid that was triturated with 1% methanol in CH₂Cl₂ to give a white solid (29mg, 55%), melting point: > 280°C. ¹H NMR (300 MHz, DMSO-D6) δ ppm 1.53 (s, 9 H) 3.08 (s, 3 H) 5.69 (d, J=7.72 Hz, 1 H) 7.42 (m, 2 H) 7.52 (dd, J=8.46, 1.84 Hz, 1 H) 7.56 (d, J=2.57 Hz, 1 H) 7.74 (d, J=1.84 Hz, 1 H) 7.84 (d, J=7.72 Hz, 1 H) 7.88 (s, 1 H) 7.91 (d, J=8.82 Hz, 1 H) 7.95 (d, J=9.19 Hz, 1 H) 10.04 (s, 1 H) 11.46 (s, 1 H). MS (ESI-) *m*/*z* 496 (M-H)⁺.

### Example 72. Preparation of N-((6-(3-tert-butyl-5-(2,4-dioxo-3,4-dihydropyrimidin-1(2H)-yl)-2-methoxyphenyl)benzo[d]isoxazol-3-yl)methyl)methanesulfonamide (compound IB-L0-2.45).

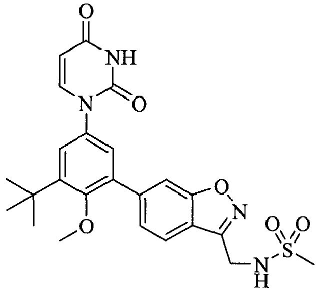

### Part A. Preparation of N-((6-bromobenzo[d]isoxazol-3-yl)methyl)-N-(4-methoxybenzyl)-methanesulfonamide.

To a refluxing solution of 6-bromo-3-methylbenzo[d]isoxazole (1.0g, 4.72mmol) in CCl₄ (25ml) was added 1-bromopyrrolidine-2,5-dione (0.923g, 5.19mmol) and benzoic peroxyanhydride (0.114g, 0.472mmol). The mixture was refluxed for 6h, and then cooled to room temperature, filtered thru celite, and concentrated in vacuo. The crude product was purified by column chromatography on silica gel using CH₂Cl₂ as the eluent to give the dibromide as a solid (0.84g, 43%). To a solution of the dibromide (0.20g, 0.687mmol) and *N*-(4-methoxybenzyl)methanesulfonamide (0.148g, 0.687mmol) in EtOH (3ml) was added 1N aq. NaOH (0.722ml, 0.722mmol), and the resulting mixture was stirred at 80°C for 90min. The mixture was partitioned between 0.1N aq. HCL (10mL) and EtOAc (2 x 10mL) and the combined organic layers were dried over Na₂SO₄, filtered and concentrated in vacuo. The crude product was purified by column chromatography on silica gel using 2:3 EtOAc:hexanes as eluent to give the title compound as an oil (65mg, 22%).

### Part B. Preparation of N-(4-methoxybenzyl)-N-((6-(4,4,5,5-tetramethyl-1,3,2-dioxaborolan-2-yl)benzo[d]isoxazol-3-yl)methyl)methanesulfonamide.

A solution of the product from **Part A** (56mg, 0.132mmol), bis(pinacolato)diboron (37mg, 0.145mmol), and potassium acetate (39mg, 0.395mmol) in 1,4-dioxane (1.3mL) was degassed by bubbling with N₂ gas for 15min. 1,1'-Bis(diphenylphosphino)ferrocene-palladium(II)dichloride dichloromethane complex (3mg, 0.004mmol) was added, and the resulting mixture was stirred at 80°C for 16h, filtered and concentrated in vacuo. The crude product was purified by column chromatography on silica gel using 1:2 EtOAc:hexanes as the eluent to give the title compound as a colorless oil (49mg, 79%).

### Part C. Preparation of N-((6-(3-tert-butyl-5-(2,4-dioxo-3,4-dihydropyrimidin-1(2H)-yl)-2-methoxyphenyl)benzo[d]isoxazol-3-yl)methyl)-N-(4-methoxybenzyl)methanesulfonamide.

A mixture of the product from **Example C** (31.8mg, 0.079mmol), the product from **Part B** (45mg, 0.095mmol) in EtOH (0.5mL), toluene (0.5mL) 1M aq. Na₂CO₃ (0.095mL, 0.095mmol) was degassed by bubbling with N₂ gas for 10min. 1,1'- Bis(diphenylphosphino)ferrocene-palladium(II) dichloride dichloromethane complex (2mg, 2.4µmol) was added, and degassing with N₂ was continued for 5min. The reaction mixture was sealed and heated at 100°C in a microwave reactor for 1h. The mixture was concentrated in vacuo, and the crude product was purified by column chromatography on silica gel using 1:9 *MeOH:CHCl₃ as the eluent. The title compound was obtained as a light brown solid (41mg, 83%).

### Part D. Preparation of N-((6-(3-tert-butyl-5-(2,4-dioxo-3,4-dihydropyrimidin-1(2H)-yl)-2-methoxyphenyl)benzo[d]isoxazol-3-yl)methyl)methanesulfonamide.

A solution of the product from **Part C** (39mg, 0.063mmol) in TFA (0.5mL) was stirred at 40°C for 6h. TFA was removed in vacuo and the crude product was purified by column chromatography on silica gel using 4% MeOH in CHCl₃ as the eluent to give the title compound (13mg, 41%). ¹H NMR (300 MHz, CDCl₃) δ 8.39 (s, 1 H) 7.74 - 7.82 (m, 2 H) 7.57 (dd, *J*=8.27, 1.65 Hz, 1 H) 7.36 (d, *J*=7.72 Hz, 1 H) 7.25 (d, *J*=2.57 Hz, 1 H) 7.19 (d, *J*=2.94 Hz, 1 H) 5.82 (dd, *J*=7.72, 2.21 Hz, 1 H) 5.25 - 5.33 (m, 1 H) 4.70 (d, *J*=6.25 Hz, 2 H) 3.29 (s, 3 H) 3.12 (s, 3 H) 1.45 (s, 9 H).

### Example 73. Preparation of methyl 2-(5-(3-tert-butyl-5-(2,4-dioxo-3,4-dihydropyrimidin-1(2H)-yl)-2-methoxyphenyl)-2,3-dihydro-1H-inden-1-ylidene)hydrazinecarboxylate (compound IB-L0-2.24).

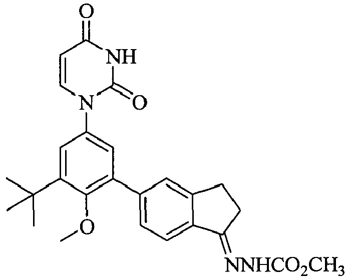

To a solution of the product from **Example 6, Part B** (0.05g, 0.124mmol) in MeOH (1ml) was added methyl carbazate (17mg, 0.185mmol). The mixture was stirred at 60°C for 16h, and then concentrated in vacuo. The crude product was purified by column chromatography on silica gel using 5% MeOH in CH₂Cl₂ as the eluent to give the title compound (44mg, 74%). ¹H NMR (300 MHz, DMSO-d₆) δ 11.40 (s, 1 H) 10.05 (s, 1 H) 7.78 (d, *J*=8.09 Hz, 1 H) 7.69 (d, *J*=7.72 Hz, 1 H) 7.45 - 7.57 (m, 2 H) 7.24 - 7.33 (m, 2 H) 5.64 (d, *J*=8.09 Hz, 1 H) 3.71 (s, 3 H) 3.28 (s, 3 H) 3.06 - 3.16 (m, 2 H) 2.78 - 2.88 (m, 2 H) 1.40 (s, 9 H).

### Example 74. Preparation of 1-(3-tert-butyl-4-methoxy-5-(1-oxoisoindolin-5-yl)phenyl)-pyrimidine-2,4(1H,3H)-dione (compound IB-L0-2.30).

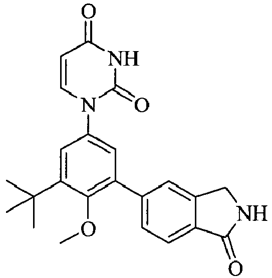

### Part A. Preparation of 5-bromo-2-(2,4-dimethoxybenzyl)isoindolin-1-one.

To a solution of methyl 4-bromo-2-(bromomethyl)benzoate (1.0g, 3.25mmol) and (2,4-dimethoxyphenyl)methanamine (0.65g, 3.90mmol) in THF (16mL) was added triethylamine (0.91mL, 6.5mmol), and the resulting mixture was stirred at room temeprature for 16h. The resulting solid was filtered off, and the filtrate was concentrated in vacuo. The crude product was purified by column chromatography on silica gel using 1:4 EtOAc:hexanes as the eluent to give the title compound as a colorless solid (0.52g, 44%).

### Part B. Preparation of 2-(2,4-dimethoxybenzyl)-5-(4,4,5,5-tetramethyl-1,3,2-dioxaborolan-2-yl)isoindolin-1-one.

The product from **Part A** (100mg, 0.276mmol) was subjected to the conditions described for **Example 72, Part B** to give the title compound as an oil (107mg, 95%).

### Part C. Preparation of 1-(3-tert-butyl-5-(2-(2,4-dimethoxybenzyl)-1-oxoisoindolin-5-yl)-4-methoxyphenyl)pyrimidine-2,4(1H,3H)-dione.

The product from **Part C** (44mg, 0.111mmol) was subjected to the conditions described for **Example 72**, **Part C** to give the title compound (50mg, 81%).

### Part D. Preparation of 1-(3-tert-butyl-4-methoxy-5-(1-oxoisoindolin-5-yl)phenyl) pyrimidine-2,4(1H,3H)-dione.

A solution of the product from **Part C** (48mg, 0.086mmol) in CH₂Cl₂ (0.3ml) and TFA (0.6ml, 7.79mmol) was stirred at room temperature for 16h, and then concentrated in vacuo. The crude product was purified by column chromatography on silica gel using 5% MeOH in CHCl₃ as the eluent to give the title compound as a colorless solid (22mg, 63%). ¹H NMR (300 MHz, DMSO-d₆) δ 11.41 (d, *J*=1.84 Hz, 1 H) 8.61 (s, 1 H) 7.72 - 7.83 (m, 3 H) 7.62 - 7.69 (m, 1 H) 7.29 - 7.36 (m, 2 H) 5.65 (dd, *J*=8.09, 2.21 Hz, 1 H) 4.44 (s, 2 H) 3.25 (s, 3 H) 1.41 (s, 9 H).

### Example 75. Preparation of N-(2-(6-(3-tert-butyl-5-(2,4-dioxo-3,4-dihydropyrimidin-1(2H-yl)-2-methoxyphenyl)-1H-inden-3-yl)propan-2-yl)methanesulfonamide (compound IB-L0-2.41).

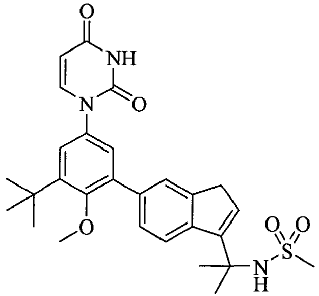

### Part A. Preparation of 6-bromo-1H-indene-3-carbonitrile.

To a solution of 5-bromo-2,3-dihydro-1H-inden-1-one (1g, 4.74mmol) in anhydrous THF (15ml) at -10°C was added 2M lithium diisopropylamide in THF (0.242ml, 0.483mmol) dropwise. The resulting mixture was stirred at -10°C for 15min before diethylcyanophosphonate (0.791ml, 5.21mmol) was added dropwise. Following the addition, the mixture was allowed to warm to room temperature, and was stirred at room temperature for 3h. The mixture was cooled to -78°C and borontrifluoride diethyl etherate (1.196ml, 9.52mmol) was added dropwise. Following the addition, the mixture was stirred at - 78°C for 1h and was then allowed to warm to room temperature and was stirred at room temperature for 16h. The mixture was concentrated in vacuo, and the residue was partitioned between EtOAc (50mL) and H₂O (2 x 50mL). The organic layer was dried over Na₂SO₄, filtered and concentrated in vacuo, and the crude product was purified by column chromatography on silica gel using 9:1 EtOAc:hexanes as the eluent. The title compound was obtained as an tan solid (0.72g, 69%).

### Part B. Preparation of N-(2-(6-bromo-1H-inden-3-yl)propan-2-yl)methanesulfonamide.

Anhydrous cerium(III) chloride (0.224g, 0.909mmol) was flame dried under vacuum and placed under dry N₂. Anhydrous THF (1.5ml) was added, and the resulting mixture was stirred under N₂ at 45°C for 48h. The mixture was cooled to room temperature, and the product from **Part A** (0.1g, 0.454mmol) was added. The resulting mixture was cooled to -78°C, and a 1.5M solution of methyllithium lithium bromide complex (0.757ml, 1.136mmol) in Et₂O was added dropwise over 15min. Following the addition, the mixture was allowed to warm to -20°C and was stirred for 24h. Concentrated aq. NH₄OH (0.3mL) was added dropwise, and the mixture was allowed to warm to room temperature, was stirred for 30min, and was then filtered and washed with THF (2 x 5mL). The filtrate was concentrated in vacuo, and the crude product was purified by column chromatography on silica gel using 5% MeOH in CH₂Cl₂ as the eluent to give a solid (23mg, 20%). To a solution of this solid (23mg, 0.091mmol) in CH₂Cl₂ (1mL) was added methanesulfonyl chloride (0.011mL, 0.137mmol). The mixture was cooled to 0°C and diisopropylethylamine (0.024ml, 0.137mmol) was added dropwise. The resulting mixture was stirred at room temperature for 90min, and was then partitioned between 0.1 N aq. HCl (2mL) and CH₂Cl₂ (3 x 2mL). The combined organic layers were dried over Na₂SO₄, filtered and concentrated in vacuo, and the crude product was purified by column chromatography on silica gel to give the title compound (17mg, 56%).

### Part C. Preparation of N-(2-(6-(4,4,5,5-tetramethyl-1,3,2-dioxaborolan-2-yl)-1H-inden-3-yl)propan-2-yl)methanesulfonamide.

The product from **Part C** (50mg, 0.151mmol) was subjected to the conditions described for **Example 72, Part B** to give the title compound as a colorless solid (37mg, 65%).

### Part D. Preparation of N-(2-(6-(3-tert-butyl-5-(2,4-dioxo-3,4-dihydropyrimidin-1(2H)-yl)-2-methoxyphenyl)-1H-inden-3-yl)propan-2-yl)methanesulfonamide.

The product from **Part C** (35mg, 0.093mmol) was subjected to the conditions described for **Example 72, Part C** to give the title compound as a colorless solid (41mg, 84%). ¹H NMR (300 MHz, DMSO-d₆) δ 11.40 (s, 1 H) 7.94 (d, *J*=8.09 Hz, 1 H) 7.78 (d, *J*=8.09 Hz, 1 H) 7.65 (d, *J*=1.50 Hz, 1 H) 7.56 (s, 1 H) 7.48 (dd, *J*=8.09, 1.47 Hz, 1 H) 7.27 (s, 2 H) 6.48 (s, 1 H) 5.63 (d, *J*=8.09 Hz, 1 H) 3.43 (s, 2 H) 3.25 (s, 3 H) 2.63 (s, 3 H) 1.68 (s, 6 H) 1.41 (s, 9 H).

### Example 76. Preparation of N-((6-(3-tert-butyl-5-(2,4-dioxo-3,4-dihydropyrimidin-1(2H)-yl)-2-methoxyphenyl)benzo[b]thiophen-3-yl)methyl)methanesulfonamide (compound IB-LO-2.11).

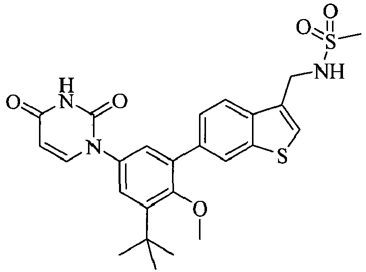

### Part A. Preparation of ethyl 6-bromobenzo[b]thiophene-2-carboxylate.

A solution of ethyl thioglycolate (0.65g, 5.42mmol), 4-bromo-2-fluorobenzaldehyde (1.0g, 4.93mmol) and triethylamine (1.25mL, 12.3mmol) in DMSO (5mL) was heated at 75°C for 2h. The mixture was partitioned between H₂O (50mL) and CH₂Cl₂ (2 x 50mL), and the combined organic layers were dried over Na₂SO₄. The drying agent was filtered off, and the solvent was removed in vacuo to give the title compound as an oil (1.29g, 92%).

### Part B. Preparation of 6-bromobenzo[b]thiophene-2-carboxylic acid.

To a solution of the product from **Part A** (1.21g, 4.24mmol) in THF (10mL) was added a solution of LiOH (0.305g, 12.73mmol) in H₂O (4mL) and the resulting mixture was stirred at 40°C for 2h. The mixture was partitioned between H₂O (50mL) and CH₂Cl₂ (50mL). The aqueous layer was adjusted to pH = 2 using 1N HCl, and extracted with CH₂Cl₂ (2 x 50mL). The combined organic layers were dried over Na₂SO₄, filtered and concentrated in vacuo to give the title compound as an oil (1.04g, 95%).

### Part C. Preparation of 6-bromobenzo[b]thiophene.

The product from **Part B** (0.70g, 2.73mmol) and DBU (1.35mL, 8.94mmol) were combined in DMA (6mL) in a sealed tube and heated at 200°C in a microwave reactor for 70min. The resulting dark solution was diluted with 1 M HCl (20mL) and extracted with CH₂Cl₂ (2 x 20mL). The combined organic layers were dried over Na₂SO₄, filtered and concentrated in vacuo, and the crude product was purified by column chromatography on silica gel using CH₂Cl₂ as the eluent to give the title compound as an oil (0.484g 83%).

### Part D. Preparation of 6-bromo-3-(chloromethyl)benzo[b]thiophene.

To a solution of the product from **Part C** (0.484g, 2.27mmol) in benzene (0.20mL) was added 37% aq. formaldehyde solution (1mL) and concentrated HCl (1mL). The resulting mixture was heated at 70°C for 1h. while HCl gas was bubbled through the mixture. The mixture was partitioned between H₂O (20mL) and CH₂Cl₂ (2 x 20mL), and the combined organic layers were dried over Na₂SO₄, filtered and concentrated in vacuo. The crude product was purified by column chromatography on silica gel using CH₂Cl₂ to give the title compound as a waxy solid (0.49g, 82%).

### Part E. Preparation of N-((6-bromobenzo[b]thiophen-3-yl)methyl)-N-(2,4-dimethoxybenzyl) methanesulfonamide.

To a solution of the product form **Part D** (275mg, 1.05mmol) and *N*-(2.4-dimethoxybenzyl)-methanesulfonamide (284mg, 1.15mmol) in DMA (6mL) was added K₂CO₃ (160mg, 1.15mmol), and the mixture was stirred at room temperature for 3h. The mixture was partitioned between H₂O (20mL) and Et₂O (2 x 20mL), and the combined organic layers were dried over Na₂SO₄, filtered and concentrated in vacuo. The crude product was purified by column chromatography on silica gel using 2% EtOAc in CH₂Cl₂ as the eluent to give the title compound as a waxy solid (316mg, 64%).

### Part F. Preparation of N-(2,4-dimethoxybenzyl)-N-((6-(4,4,5,5-tetramethyl-1,3,2-dioxaborolan-2-yl)benzo[b]thiophen-3-yl)methyl)methanesulfonamide.

The product from **Part E** (300mg, 0.64mmol) was subjected to the conditions described for **Example 72, Part B** to give the title compound as a waxy solid (248mg, 75%).

### Part G. Preparation of N-((6-(3-tert-butyl-5-(2,4-dioxo-3,4-dihydropyrimidin-1(2H)-yl)-2-methoxyphenyl)benzo[b]thiophen-3-yl)methyl)-N-(2,4-dimethoxybenzyl)methanesulfonamide.

The product from **Part F** (214mg, 0.414mmol) was subjected to the conditions described for **Example 72, Part C** to give the title compound as a light yellow solid (238mg, 87%).

### Part H. Preparation of N-((6-(3-tert-butyl-5-(2,4-dioxo-3,4-dihydropyrimidin-1(2H)-yl)-2-methoxyphenyl)benzo[b]thiophen-3-yl)methyl)methanesulfonamide.

To a solution of the product from **Part G** (230mg, 0.34mmol) in CH₂Cl₂ (4mL) was added trifluoroacetic acid (0.5mL), and the mixture was stirred at room temperature for 30min. The solution was diluted with CH₂Cl₂ (10mL) and extracted with saturated aq. NaHCO₃ (2 x 10mL). The organic layer was dried over Na₂SO₄, filtered and concentrated in vacuo, and the crude product was purified by column chromatography on silica gel eluting with 3% MeOH in CH₂Cl₂ to give the title compound as an off-white solid (149mg, 84%). ¹H NMR (300 MHz, DMSO-d₆) δ 11.41 (s, 1 H) 8.16 (d, *J*=1.10 Hz, 1 H) 8.02 (d, *J*=8.46 Hz, 1 H) 7.79 (d, *J*=7.72 Hz, 1 H) 7.71 (s, 1 H) 7.60 - 7.66 (m, 2 H) 7.29 - 7.38 (m, 2 H) 5.65 (d, *J*=7.72 Hz, 1 H) 4.44 (d, *J*=5.88 Hz, 2 H) 3.24 (s, 3 H) 2.95 (s, 3 H) 1.42 (s, 9 H).

### Example 77. Preparation of N-(2-(3-tert-butyl-5-(2,4-dioxo-3,4-dihydropyrimidin-1(2H)-yl)-2-methoxyphenyl)-1,2,3,4-tetrahydroisoquinolin-6-yl)methanesulfonamide (compound IB-L0-2.19).

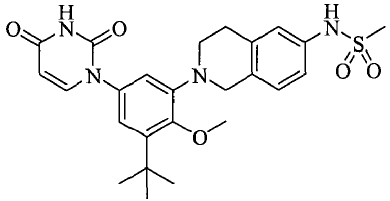

### Part A. Preparation of 1-(3-amino-5-tert-butyl-4-methoxyphenyl)pyrimidine-2,4(1H,3H)-dione.

To a solution of the product from **Example 7**, **Part F** (170mg, 0.534mmol) and triethylamine (223uL, 1.6mmol) in THF (5mL) was added diphenylphosphorylazide (173uL, 0.80mmol). The resulting mixture was stirred at room temperature for 1h, and was then stirred at 45°C for 1h. Water (280uL) was added, and the resulting mixture was stirred at 50°C for 1h, and then stirred at room temperature for 16h. The solution was diluted with H₂O (10mL), and the resulting solid was filtered off. The solid was suspended in 1M aq. HCl and filtered to give the amine product as the HCl salt. This salt was suspended in aq. NaHCO₃ (20mL) and extracted with EtOAc (2 x 20mL). The combined organic layers were dried over Na₂SO₄, filtered and concentrated in vacuo to give the title compound as a colorless solid (55mg, 36%).

### Part B. Preparation of 1-(3-tert-butyl-4-methoxy-5-(6-nitro-3,4-dihydroisoquinolin-2(1H)-yl)phenyl)pyrimidine-2,4(1H,3H)-dione.

A solution of the product from **Part A** (100mg, 0.28mmol) and 2-(2-(methylsulfonyloxy)-ethyl)-4-nitrobenzyl methanesulfonate (196mg, 0.68mmol) were in anhydrous DMA (4mL) was stirred at 80°C for 18h. The cooled mixture was partitioned between H₂O (20mL) and EtOAc (2 x 20mL), and the combined organic layers were dried over Na₂SO₄, filtered and concentrated in vacuo. The residue was suspended in CH₂Cl₂ and filtered to remove unreacted aniline starting material. The filtrate was concentrated in vacuo, and the crude product was purified by column chromatography on silica gel eluting with 1% MeOH in CH₂Cl₂ to give the title compound as a light yellow solid (39.3mg, 31%).

### Part C. Preparation of N-(2-(3-tert-butyl-5-(2,4-dioxo-3,4-dihydropyrimidin-1(2H)-yl)-2-methoxyphenyl)-1,2,3,4-tetrahydroisoquinolin-6-yl)methanesulfonamide.

To a solution of the product from **Part B** (35mg, 0.078mmol) in THF (0.5mL), MeOH (0.5mL) and H₂O (0.25mL) was added Fe powder (17.4mg, 0.41mmol) and NH₄Cl (6.2mg, 0.12mmol, and the resulting mixture was stirred at 70°C for 1h. The hot mixture was filtered through celite and rinsed with THF and MeOH. The filtrate was concentrated and dried in vacuo to give a solid. To a solution of the solid (32mg, 0.076mmol) and pyridine (26uL, 0.32mmol) in CH₂Cl₂ (1.5mL) was added methanesulfonyl chloride (7.7uL, 0.099mmol). The mixture was stirred at room temperature for 1h then concentrated in vacuo. The crude product was purified by column chromatography on silica gel eluting with 5% MeOH in CH₂Cl₂ to give the title compound as a light yellow solid (7mg, 19%). ¹H NMR (300 MHz, DMSO-d₆) δ 7.71 (d, J=8.09 Hz, 1 H) 7.14 - 7.21 (m, 1 H) 7.05 - 7.12 (m, 3 H) 6.98 (d, J=2.57 Hz, 1 H) 5.65 (d, J=7.72 Hz, 1 H) 4.18 (s, 2 H) 3.86 (s, 3 H) 3.03 (t, J=4.23 Hz, 2 H) 2.99 (s, 3 H) 1.38 (s, 9 H).

### Example 78. Preparation of N-(2-(3-tert-butyl-5-(2,4-dioxo-3,4-dihydropyrimidin-1(2H)-yl)-2-methoxyphenyl)isoindolin-5-yl)methanesulfonamide (compound IB-L0-2.79).

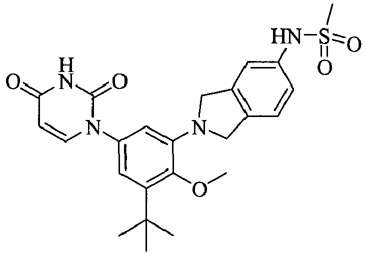

### Part A. Preparation of (4-nitro-1,2-phenylene)bis(methylene)dimethanesulfonate.

To a solution of 4-nitrophthalic acid (500mg, 2.37mmol) in THF (24mL) at room temperature was added a 1M solution of BH₃•THF complex (9.95mL, 9.95mmol) dropwise. This solution was stirred at 65°C for 1h, and then allowed to cool to room temperature. To the mixture was added MeOH (1mL), and the mixture was stirred for 30min and concentrated in vacuo. The residue was partitioned between 1M aq. HCl (20mL) and EtOAc (2 x 20mL), and the combined organic layers were dried over Na₂SO₄, filtered and concentrated in vacuo. The crude product was purified by column chromatography on silica gel eluting with 3% MeOH in CH₂Cl₂ to give an oil (253mg, 58%). To a solution of the oil (250mg, 2.37mmol) and triethylamine (438uL, 3.14mmol) in anhydrous CH₂Cl₂ (30mL) at 0°C was added methanesulfonyl chloride (234uL, 3.0mmol) dropwise. The solution was stirred at room temperature for 18h, and was partitioned between 1M aq. HCl (20mL) and CH₂Cl₂ (2 x 20mL). The combined organic layers were dried over Na₂SO₄, filtered and concentrated in vacuo. The crude product was purified by column chromatography on silica gel eluting with CH₂Cl₂ to give the title compound (150mg, 32%).

### Part B. Preparation of 1-(3-tert-butyl-4-methoxy-5-(5-nitroisoindolin-2-yl)phenyl) pyrimidine-2,4(1H,3H)-dione.

To a solution of the product of **Part A** (110mg, 0.324mmol) and the product of **Example 77**, **Part A** (113mg, 0.389mmol) in anhydrous 1,4-dioxane (4mL) was added sodium bicarbonate (60mg, 0.71mmol) and diisopropylethylamine (142uL, 0.81mmol) and the resulting mixture was stirred at 95°C for 16h. The mixture was partitioned between 0.5M aq. HCl (10mL) and CH₂Cl₂ (2 x 10mL), and the combined organic layers were dried over Na₂SO₄, filtered and concentrated in vacuo. The crude product was purified by column chromatography on silica gel eluting with 1% MeOH in CH₂Cl₂ to give the title compound as a light yellow solid (110mg, 78%).

### Part C. Preparation of N-(2-(3-tert-butyl-5-(2,4-dioxo-3,4-dihydropyrimidin-1(2H)-yl)-2-methoxyphenyl)isoindolin-5-yl)methanesulfonamide.

The product from **Part B** (100mg, 0.25mmol) was subjected to the conditions described for **Example 77**, **Part C** to give the title compound as an off-white solid (53mg, 45%). ¹H NMR (300 MHz, DMSO-d₆) δ 11.37 (s, 1 H) 9.70 (s, 1 H) 7.71 (d, J=7.72 Hz, 1 H) 7.34 (d, J=8.09 Hz, 1 H) 7.23 (d, J=1.84 Hz, 1 H) 7.13 (dd, J=8.09, 1.84 Hz, 1 H) 6.98 (d, J=2.57 Hz, 1 H) 6.81 (d, J=2.21 Hz, 1 H) 5.62 (d, J=7.72 Hz, 1 H) 4.52 (s, 2 H) 4.50 (s, 2 H) 3.63 (s, 3 H) 2.98 (s, 3 H) 1.38 (s, 9 H).

### Example 79. Preparation of N-((6-(3-tert-butyl-5-(2,4-dioxo-3,4-dihydropyrimidin-1(2H)-yl)-2-methoxyphenyl)-1H-inden-3-yl)methyl)methanesulfonamide (compound IB-L0-2.13).

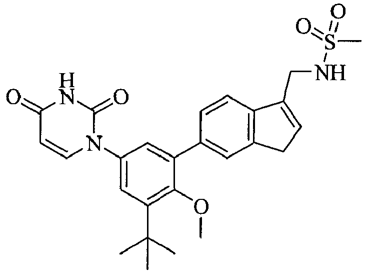

### Part A. Preparation of 5-bromo-1-(trimethylsilyloxy)-2,3-dihydro-1H indene-1-carbonitrile.

To a solution of 5-bromo-2,3-dihydro-1*H*-inden-1-one (10.0g, 47.4mmol) and *N*-methylmorpholine *N*-oxide (1.67g, 14.21mmol) in CH₂Cl₂ (50ml) was added trimethylsilylcyanide (7.05g, 71.1mmol), and the resultant solution was stirred at room temperature for 72h, and then concentrated in vacuo. The crude product was purified by column chromatography on silica gel using 5% EtOAc in hexanes as the eluent to give the title compound as a colorless liquid (12.65g, 86%).

### Part B. Preparation of 1-(aminomethyl)-5-bromo-2,3-dihydro-1H inden-1-ol.

To a solution of the product from **Part A** (18.44g, 59.4mmol) in anhydrous Et₂O (250mL) under N₂ gas at 0°C was added a 1M solution of LiAlH₄ in Et₂O (62.4mL, 62.4mmol) dropwise over 1h. Following the addition, the mixture was allowed to warm to rt and was stirred at room temperature for 2h. The mixture was cooled in an ice bath while H₂O (4.3mL) was added dropwise, followed by the addition of 15% aq. NH₄OH (4.3mL), and then H₂O (13mL). The mixture was stirred at room temperature for 15min, and then filtered through celite and rinsed with EtOAc. The filtrate was concentrated in vacuo, and the residue was suspended in Et₂O (40mL) to give a precipitate that was filtered and dried to give the title compound as a colorless solid (10.0g, 70%).

### Part C. Preparation of (6-bromo-1H-inden-3-yl)methanamine hydrochloride salt.

To a solution of the product from **Part B** (10.0g, 41.3mmol) in MeOH (100mL) was added 6N aq. HCl (125mL) and the mixture was stirred at 70°C for 3h and then allowed to cool to room temperature. MeOH was removed in vacuo to give a precipitate that was collected by filtration, washed with H₂O and dried in vacuo to provide the title compound as a colorless solid (9.89g, 92%).

### Part D. Preparation of N-((6-bromo-1H-inden-3-yl)methyl)methanesulfonamide.

To a suspension of the product from **Part C** (6.46g, 24.8mmol) in anhydrous CH₂Cl₂ (260mL) was added methanesulfonyl chloride (3.86mL, 49.6mmol) and diisopropylethylamine (13.0mL, 74.4mmol), and the resulting mixture was stirred at room temperature for 10h. The solution was washed with 1N aq. HCl (2 x 300mL), and the organic layer was dried over Na₂SO₄, filtered and concentrated in vacuo. The residue was suspended in Et₂O (100mL) to give a precipitate that was collected by filtration and dried to give the title compound as a colorless solid (6.25g, 83%).

### Part E. Preparation of N-((6-(4,4,5,5-tetramethyl-1,3,2-dioxaborolan-2-yl)-1H-inden-3-yl)methyl)methanesulfonamide.

A solution of the product from **Part D** (2.0g, 6.62mmol), bis(pinacolato)diboron (1.85g, 7.28mmol), potassium acetate (1.95g, 19.86mmol) and 1,1'-bis(diphenylphosphino)ferrocenepalladium(II)dichloride dichloromethane complex (0.27g, 0.331mmol) in anhydrous 1,4-dioxane (80mL) under N₂ was stirred at 95°C for 8h. The cooled mixture was filtered through celite, washed with EtOAc (2 x 20mL) and then concentrated in vacuo. The crude product was purified by column chromatography on silica gel using 1:2 EtOAc:hexanes as the eluent to give the title compound as a colorless oil (2.02g, 87%).

### Part F. Preparation of N-((6-(3-tert-butyl-5-(2,4-dioxo-3,4-dihydropyrimidin-1(2H)-yl)-2-methoxyphenyl)-1H-inden-3-yl)methyl)methanesulfonamide.

A mixture of the product from **Part E** (3.14g, 8.99mmol), the product from **Example C** (3.78g, 9.44mmol), tripotassium phosphate (3.82, 17.98mmol), 1,3,5,7-tetramethyl-2,4,8-trioxa-6-phospha-6-phenyl-adamantane (Cytec [97739-46-3]) (105mg, 0.36mmol), and tris(dibenzylidineacetone)-dipalladium(0) (165mg, 0.18mmol) was placed under N₂ gas. To the mixture was added, via canula, a mixture of THF (45mL) and H₂O (15mL) that had been degassed by bubbling Ar gas for 10min. The resulting mixture was further degassed by bubbling with Ar for an additional 15min. The mixture was stirred at 50°C for 1.5h while Ar was continuously bubbled through the solution. Additional tris(dibenzyl idineacetone)dipalladium(0) (55mg, 0.6mmol) in THF (2mL) was added, and the mixture was stirred at 50°C for 1h. The mixture was allowed to cool to rt, and was partitioned between CH₂Cl₂ (300ml) and 1N aq. HCl (150mL). To the orange organic layer was added 3-mercaptopropyl-functionalized silica gel (10g, Aldrich) and mgSO₄, and the mixture was stirred at room temperature for 16h, filtered and concentrated in vacuo. The crude product was purified by column chromatography on silica gel using 3:1 EtOAc:hexanes as the eluent to give the title compound as a colorless solid (2.7g, 61%). ¹H NMR (300 MHz, DMSO-d₆) δ 11.40 (s,1H), 7.78 (d, J=7.4Hz,1H), 7.66 (s,1H), 7.60 (d,J=7.7Hz,1H), 7.50 (m,2H), 7.25 (m,2H), 6.56 (m,1H), 5.64 (dd, J=2.2,7.7Hz,1H), 4.18 (d, J=5.1Hz,2H), 3.46 (s,2H), 3.25 (s,3H), 2.96 (s,3H), 1.41 (s,9H).

### Example 80. Preparation of N'-(5-(3-tert-butyl-5-(2,4-dioxo-3,4-dihydropyrimidin-1(2H-yl)-2-methoxyphenyl)-2,3-dihydro-1H-inden-1-yl)methanesulfonohydrazide (compound IB-L0-2.31).

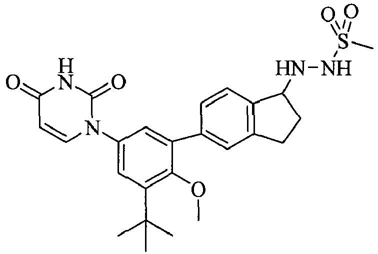

To a solution of the product from **Example 6, Part C** (100mg, 0.201mmol) was in THF (2mL) and MeOH (2mL) was added 2 drops of 10% HCl in MeOH, followed by sodium cyanoborohydride (19mg, 0.302mmol). The mixture was adjusted to pH 4 with the addition of 10% HCl in MeOH, and was then stirred at room temperature for 1h. The resulting mixture was partitioned between saturated aq. sodium bicarbonate (5mL) and CH₂Cl₂ (20mL), and the organic layer was dried over Na₂SO₄, filtered and concentrated. The crude product was purified by column chromatography on silica gel using 3% MeOH in CH₂Cl₂ as the eluent to provide the title compound as a colorless solid (58mg, 58%). ¹H NMR (300 MHz, DMSO-d6) 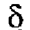 11.39(s,1H), 8.18(d,J=3.7Hz,1H), 7.77(d,J=7.7Hz,1H), 7.51(d,J=8.1Hz,1H), 7.38(m,2H), 7.27(d,J=2.6Hz,1H), 7.21(d,J=2.9Hz,1H), 5.63(d,J=7.7Hz,1H), 5.25(m,1H), 4.39(m,1H), 3.27(s,3H), 2.98(m,1H), 2.83(s,3H), 2.78(m,1H), 2.22(m,1H), 2.07(m,1H), 1.40(s,9H).

### Example 81. Preparation of 1-(3-tert-butyl-5-(1-hydroxy-2,3-dihydro-1H inden-5-yl)-4-methoxyphenyl)pyrimidine-2,4(1H,3H)-dione (compound IB-L0-2.36).

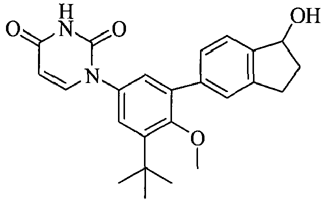

To a solution of the product from **Example 6, Part B** (150mg, 0.371mmol) in MeOH (3mL) and CH₂Cl₂ (3mL) was added sodium borohydride (28mg, 0.742mmol), and the mixture was stirred at room temperature for 1h. The mixture was partitioned between 1N aq. HCl (10mL) and CH₂Cl₂ (20mL), and the organic layer was dried over Na₂SO₄, filtered and concentrated in vacuo. The crude product was purified by column chromatography on silica gel using 5% MeOH in CH₂Cl₂ as the eluent to provide the title compound as a colorless solid (90mg, 60%). ¹H NMR (300MHz, DMSO-d6): 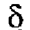 11.39(s,1H), 7.44(d, J= 4.0Hz, 1H), 7.40 (m, 2H), 7.21 (d, J= 2.6 Hz, 1H), 7.26 (d, J= 2.6 Hz, 1H), 5.63 (d, J= 8.1 Hz, 1H), 5.29 (d, J= 5.9 Hz,1H), 5.09 (m, 1H), 3.26 (s, 3H), 2.97 (m, 1H), 2.79 (m, 1H), 2.38 (m, 1H), 1.83 (m, 1H), 1.40 (s, 9H).

### Example 82. Preparation of 1-(3-tert-butyl-5-(2-(2,5-dimethyl-1H-pyrrol-1-yl)benzo[d] thiazol-6-yl)-4-methoxyphenyl)pyrimidine-2,4(1H,3H)-dione (compound IB-L0-2.47).

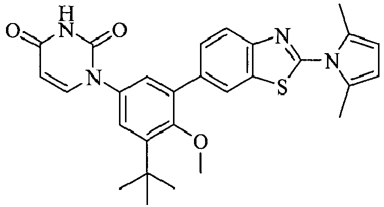

### Part A. Preparation of 6-bromo-2-(2,5-dimethyl-1H-pyrrol-1-yl)benzo[d]thiazole.

A solution 6-bromobenzo[d]thiazol-2-amine (5.75g, 25.1mmol), hexane-2,5-dione (2.95mL, 25.1mmol), and PPTS (0.95g, 3.76mmol) in benzene (100ml) was refluxed for 16h while water was removed with a Dean-Stark trap. The cooled mixture was poured into EtOAc (100mL) and extracted with saturated aq. NaHCO₃ (2 x 100mL) and brine. The organic layer was dried over Na₂SO₄, filtered and concentrated in vacuo. The crude product was purified by column chromatography on silica gel using 9:1 EtOAc:hexanes as the eluent to give the title compound as an orange oil (6.46g, 84%).

### Part B. Preparation of 2-(2,5-dimethyl-1H-pyrrol-1-yl)-6-(4,4,5,5-tetramethyl-1,3,2-dioxaborolan-2-yl)benzo[d]thiazole.

A mixture of the product from **Part A** (3.24g, 10.54mmol), bis(pinacolato)diboron (4.01g, 15.81mmol), bis(di-tert-butyl(hydroxy)phosphino)palladium(II) dichloride (0.264g, 0.527mmol), and potassium acetate (3.10g, 31.6mmol) in anhydrous toluene (25mL) was degassed by bubbling with N₂ gas for 15min, and then heated at reflux under N₂ for 72h. The cooled mixture was filtered through celite and washed with EtOAc, and the filtrate was concentrated in vacuo. The crude product was purified by column chromatography on silica gel using 9:1 EtOAc:hexanes as the eluent to give the title compound (2.77g, 74%).

### Part C. Preparation of 1-(3-tert-butyl-5-(2-(2,5-dimethyl-1H-pyrrol-1-yl)benzo[d]thiazol-6-yl)-4-methoxyphenyl)pyrimidine-2,4(1H,3H)-dione.

The product from **Part B** (405mg, 1.14mmol) was subjected to the conditions described for **Example 72**, **Part C** to give the title compound (430mg, 68%). ¹H NMR (300 MHz, DMSO-d₆) δ 11.43 (d, *J*=2.21 Hz, 1 H) 8.32 (d, *J*=1.47 Hz, 1 H) 8.12 (d, *J*=8.46 Hz, 1 H) 7.80 (d, *J*=7.72 Hz, 1 H) 7.76 (dd, *J*=8.46, 1.84 Hz, 1 H) 7.35 (q, *J*=2.57 Hz, 2 H) 5.97 (s, 2 H) 5.66 (dd, *J*=7.72, 2.21 Hz, 1 H) 3.30 (s, 3 H) 2.30 (s, 6 H) 1.43 (s, 9 H).

### Example 83. Preparation of 1-(3-(2-aminobenzo[d]thiazol-6-yl)-5-tert-butyl-4-methoxyphenyl)pyrimidine-2,4(1H,3H)-dione (compound IB-L0-2.27).

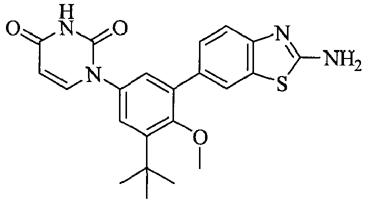

To a solution of the product from **Example 82** (4.0g, 8.0mmol) in trifluoroacetic acid (50mL) was added a few drops of H₂O, and the resulting mixture was stirred at 80°C for 2.5h, and then concentrated in vacuo. A solution of the residue in MeOH was neutralized using conc. NH₄OH, concentrated in vacuo, and the crude product was purified by column chromatography on silica gel using 9:1 CH_{Z}Cl₂:MeOH as the eluent to give the title compound (3.3g, 98%). ¹H NMR (300 MHz, DMSO-d₆) δ 11.40 (s, 1 H) 7.81 (s, 1 H) 7.77 (d, J=8.09 Hz, 1 H) 7.57 (s, 1 H) 7.40 (s, 1 H) 7.33 - 7.38 (m, 1 H) 7.25 (s, 1 H) 5.60 - 5.69 (m, 1 H) 3.26 (s, 3 H) 1.40 (s, 9 H).

### Example 84. Preparation of N-(6-(3-tert-butyl-5-(2,4-dioxo-3,4-dihydropyrimidin-1(2H)-yl)-2-methoxyphenyl)benzo[d]thiazol-2-yl)methanesulfonamide (compound IB-L0-2.28).

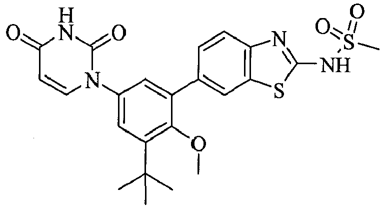

To a solution of the product from **Example 83** (0.35g, 0.83mmol) in anhydrous CH₂Cl₂ (50mL) was added methanesulfonyl chloride (194µL, 2.49mmol) and pyridine (1.34mL, 16.6mmol). The resulting mixture was stirred at room temperature for 16h and concentrated in vacuo. The crude product was purified by C-18 reverse-phase HPLC using an acetonitrile:H₂0 (0.1% TFA) gradient to give the title compound (19mg, 4%). ¹H NMR (300 MHz, DMSO-d₆) δ 13.09 (s, 1 H) 11.41 (d, *J*=1.84 Hz, 1 H) 7.96 (d, *J*=1.47 Hz, 1 H) 7.77 (d, *J*=8.09 Hz, 1 H) 7.57 (dd, 1 H) 7.42 (d, *J*=8.09 Hz, 1 H) 7.25 - 7.32 (m, 2 H) 5.64 (dd, *J*=8.09, 2.21 Hz, 1 H) 3.25 (s, 3 H) 3.02 (s, 3 H) 1.40 (s, 9 H).

### Example 85. Preparation of 1-(3-(benzo[d]thiazol-6-yl)-5-tert-butyl-4-methoxyphenyl) pyrimidine-2,4(1H,3H)-dione (compound IB-L0-2.33).

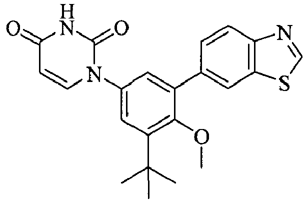

To a solution of the product from **Example 83** (30mg, 0.071mmol) in anhydrous 1,4-dioxane (3mL) under N₂ was added isoamyl nitrite (19µL, 0.142mmol). The resulting mixture was stirred at reflux for 1h, and concentrated in vacuo. The crude product was purified by C-18 reverse-phase HPLC using an acetonitrile:H₂O (0.1% TFA) gradient to give the title compound (14mg, 48%). ¹H NMR (300 MHz, DMSO-d₆) δ 11.42 (d, *J*=1.84 Hz, 1 H) 9.44 (s, 1 H) 8.34 (d, *J*=1.47 Hz, 1 H) 8.19 (d, *J*=8.46 Hz, 1 H) 7.79 (d, *J*=7.72 Hz, 1 H) 7.73 (dd, *J*=8.46, 1.84 Hz, 1 H) 7.32 - 7.37 (m, 2 H) 5.65 (dd, *J*=7.91, 2.39 Hz, 1 H) 3.24 (s, 3 H) 1.42 (s, 9 H).

### Example 86. Preparation of N-(6-(3-tert-butyl-5-(2,4-dioxo-3,4-dihydropyrimidin-1(2H)-yl)-2-methoxyphenyl)benzo[d]thiazol-2-yl)acetamide (compound IB-L0-2.49).

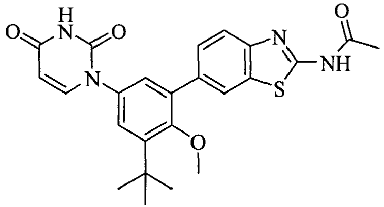

A mixture of the product from **Example 83** (30mg, 0.071mmol) and acetic anhydride (3mL) was stirred at 100°C for 2h, and then allowed to cool to room temperature. The resulting solid was collected by filtration, washed with H₂O, and dried to give the title compound as an off-white solid (29mg, 88%). ¹H NMR (300 MHz, DMSO-d₆) δ 12.42 (s, 1 H) 11.41 (d, J=2.21 Hz, 1 H) 8.12 (d, J=1.47 Hz, 1 H) 7.82 (d, J=8.46 Hz, 1 H) 7.78 (d, J=8.09 Hz, 1 H) 7.61 (dd, J=8.46, 1.84 Hz, 1 H) 7.31 (q, J=2.70 Hz, 2 H) 5.64 (dd, J=8.09, 2.21 Hz, 1 H) 3.24 (s, 3 H) 2.22 (s, 3 H) 1.41 (s, 9 H).

### Example 87. Preparation of 1-(3-tert-butyl-4-methoxy-5-(2-(propylamino)benzo[d]thiazol-6-yl)phenyl)pyrimidine-2,4(1H,3H)-dione (compound IB-L0-2.46).

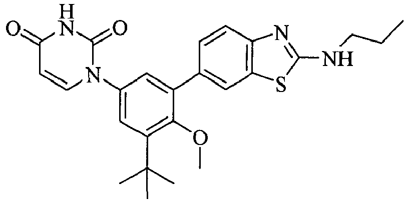

### Part A. Preparation of 1-(3-tert-butyl-5-(2-chlorobenzo[d]thiazol-6-yl)-4-methoxyphenyl) pyrimidine-2,4(1H,3H)-dione.

To a mixture of the product from **Example 83** (50mg, 0.118mmol) and copper(II) chloride (24mg, 0.178mmol) in acetonitrile (3mL) at 0°C was added *tert*-butyl nitrite (21µL, 0.178mmol). The mixture was stirred at 0°C for 1h, and then warmed to 65°C and stirred for 2h. The mixture was concentrated in vacuo and purified by column chromatography on silica gel using 5% MeOH in CH₂Cl₂ to give the title compound as an off-white solid (43mg, 82%).

### Part B. Preparation of 1-(3-tert-butyl-4-methoxy-5-(2-(propylamino)benzo[d]thiazol-6-yl) phenyl)pyrimidme-2,4(1H,3H)-dione.

A mixture of the product from **Part A** (50mg, 0.11mmol), 1-aminopropane (9µL, 0.11mmol), and K₂CO₃ (15.6mg, 0.11 mmol) in anhydrous DMF (5mL) was stirred at 100°C for 24h. The mixture was concentrated in vacuo and purified by column chromatography on silica gel using 2% MeOH in EtOAc as the eluent to give the title compound as an off-white solid (21mg, 40 %). *¹*H NMR (300 MHz, DMSO-d₆) δ 11.39 (d, *J*=1.84 Hz, 1 H) 8.12 (t, *J*=5.52 Hz, 1 H) 7.82 (d, *J*=1.47 Hz, 1 H) 7.77 (d, *J*=7.72 Hz, 1 H) 7.44 (t, *J*=9.01 Hz, 1 H) 7.37 - 7.41 (m, 1 H) 7.25 (s, 2 H) 5.63 (dd, *J*=7.91, 2.02 Hz, 1 H) 3.33 - 3.38 (m, 2 H) 3.26 (s, 3 H) 1.56 - 1.69 (m, 2 H) 1.40 (s, 9 H) 0.94 (t, *J*=7.35 Hz, 3 H).

### Example 88. Preparation of 1-(3-tert-butyl-4-methoxy-5-(3-methylbenzofuran-6-yl)phenyl)-pyrimidine-2,4(1H,3H)-dione (compound IB-L0-2.42).

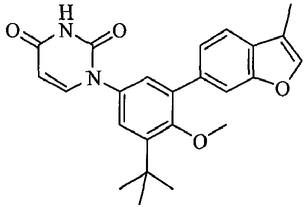

### Part A. Preparation of methyl 2-(2-acetyl-5-bromophenoxy)acetate.

A solution of 1-(4-bromo-2-hydroxyphenyl)ethanone (1.35g, 6.28mmol) in anhydrous DMF (16mL) was treated in several portions with sodium hydride (377mg of 60% in oil, 226mg, 9.42mmol) followed by stirring at room temperature for 30min. The mixture was then treated with methyl bromoacetate (871µL, 1.45g, 9.48mmol) dropwise (solution became warm after addition was complete) followed by stirring at room temperature for 18h. The mixture was diluted with ethyl acetate and extracted with water (4x) and saturated sodium chloride solution. Drying (Na₂SO₄) and concentration in vacuo afforded a nearly colorless solid, which was purified by column chromatography on silica gel, eluting with 20-100% ethyl acetate in hexanes. These procedures afforded the title compound as a colorless solid (1.47g, 82%).

### Part B. Preparation of 2-(2-acetyl-5-bromophenoxy)acetic acid.

A solution of the product from **Part A** (1.47g, 5.12mmol) in tetrahydrofuran (26mL) was treated with 1.0N sodium hydroxide solution (6.7mL, 6.7mmol) followed by stirring at room temperature for 3h, at which point the reaction was complete. The mixture was concentrated in vacuo to remove tetrahydrofuran and then was diluted with water and cooled to 0°C. The mixture was acidified to pH 3 by addition of 1N hydrochloric acid solution, and then the product extracted with ethyl acetate. The organic layer was extracted with saturated sodium chloride solution and dried (Na₂SO₄). Concentration in vacuo afforded the title compound as a colorless solid (1.36g, 97%).

### Part C. Preparation of 6-bromo-3-methylbenzofuran.

A solution of the product from **Part B** (500mg, 1.83mmol) in acetic anhydride (9.2mL) was treated with sodium acetate (300mg, 3.66mmol) followed by warming at reflux for 18h. The mixture was cooled to room temperature and diluted with toluene and concentrated in vacuo to azeotropically remove acetic anhydride. This process was repeated 3x. The mixture was then diluted with ethyl acetate and stirred with saturated sodium bicarbonate solution for 1h. The layers were separated and the organic layer was extracted with saturated sodium chloride solution. Drying (Na₂SO₄) and concentration in vacuo afforded amber oil, which was purified by column chromatography on silica gel, eluting with 8-50 % ethyl acetate in hexanes. These procedures afforded the title compound as a colorless liquid (316mg, 82%).

### Part D. Preparation of 4,4,5,5-tetramethyl-2-(3-methylbenzofuran-6-yl)-1,3,2-dioxaborolane.

In a microwave tube, a mixture of the product from **Part C** (303mg, 1.44mmol), bis(pinacolato)diboron (401mg, 1.58mmol) and potassium acetate (423mg, 4.31mmol) in anhydrous dioxane (5mL) was degassed by nitrogen sparge for 15min. The mixture was treated with 1,1'-bis-(diphenylphosphino)ferrocene palladium (II) chloride dichloromethane complex (24mg, 0.029mmol) followed by degassing for another 5min. The microwave tube was sealed and the mixture was warmed at 90°C for 18h. The mixture was cooled and diluted with ethyl acetate and extracted with water and saturated sodium chloride solution. The organic layer was dried (Na₂SO₄) and stirred with (3-mercapto-propyl) silica gel for 1h. The mixture was filtered and concentrated in vacuo to afford a brown semisolid, which was purified by column chromatography on silica gel, eluting with 8-40% ethyl acetate in hexanes. These procedures afforded the title compound as colorless oil, which slowly solidified upon standing (307mg, 83%).

### Part E. Preparation of 1-(3-tert-butyl-4-methoxy-5-(3-methylbenzofuran-6-yl)phenyl)-pyrimidine-2,4(1H,3H)-dione.

In a microwave tube, a solution of the product from **Part D** (307mg, 1.19mmol), the product from **Example C** (414mg, 1.03mmol), 1,3,5,7-tetramethyl-2,4,8-trioxa-6-phospha-6-phenyl-adamantane (Cytec [97739-46-3]) (15mg, 0.052mmol), and tribasic potassium phosphate (439mg, 2.07mmol) in 3:1 tetrahydrofuran-water (8mL) was degassed by nitrogen sparge for 20min. The mixture was treated with tris(dibenzylideneacetone)dipalladium (0) (12mg, 0.012mmol) followed by degassing for another 10min. During this period, the solution turned from an initially deep maroon color to a greenish brown color. The microwave tube was sealed and the solution warmed at 50°C for 56h. The solution was cooled and diluted with ethyl acetate and acidified with 1M citric acid solution. The organic layer was extracted with saturated sodium chloride solution, dried (Na₂SO₄), and then stirred with (3-mercaptopropyl) silica gel for 1h. After filtration and concentration in vacuo, the residue obtained was purified by column chromatography on silica gel, eluting with 4-20% acetone in dichloromethane, followed by a second column chromatography on silica gel, eluting with 20-100% ethyl acetate in hexanes. These procedures afforded the title compound as a colorless solid (355mg). ¹H NMR (300 MHz, DMSO-*d₆*): δ 11.40 (d, *J*=1.84 Hz, 1 H) 7.74 - 7.92 (m, 2 H) 7.58 - 7.76 (m, 2 H) 7.46 (dd, *J*=8.09, 1.47 Hz, 1 H) 7.30 (q, *J*=2.82 Hz, 2 H) 5.64 (dd, *J*=8.09, 2.21 Hz, 1 H) 3.22 (s, 3 H) 2.25 (s, 3 H) 1.41 (s, 9 H).

### Example 89. Preparation of N-((6-(3-tert-butyl-5-(2,4-dioxo-3,4-dihydropyrimidin-1(2H)-yl)-2-methoxyphenyl)benzofuran-3-yl)methyl)methanesulfonamide (compound IB-L0-2.18).

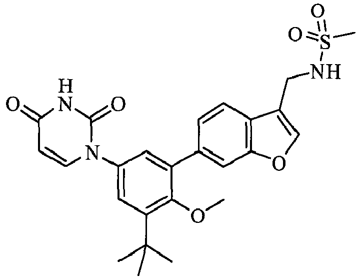

### Part A. Preparation of 6-bromo-3-(bromomethyl)benzofuran.

A solution of the product from **Example 88, Part C** (1.0g, 4.74mmol) and dibenzoyl peroxide (287mg, 1.19mmol) in chlorobenzene (24mL) at reflux was treated in four portions with N-bromosuccinimide (843mg, 4.74mmol) over 30min. The mixture was then stirred at reflux for 2h. The mixture was cooled, filtered and concentrated and purified by column chromatography on silica gel, eluting with 7-30% chloroform in hexanes. The procedures afforded the title compound as a light yellow oil (438mg, 32%).

### Part B. Preparation of N-((6-bromobenzofuran-3-yl)methyl)-N-(4-methoxybenzyl)methanesulfonamide.

A solution of the product from **Part A** (515mg, 1.78mmol), *N*-(4-methoxybenzyl)methanesulfonamide (421mg, 1.95mmol), and potassium carbonate (260mg, 1.95mmol) in anhydrous DMF (8.9mL) was stirred at 70°C for 3h. The mixture was cooled and diluted with ethyl acetate and extracted with water (4x). The organic layer was then extracted with saturated sodium chloride solution and dried (Na₂SO₄). Concentration in vacuo afforded a beige solid. This material was purified by column chromatography on silica gel, eluting with 20-100% ethyl acetate in hexanes. These procedures afforded the title compound as a colorless solid (224mg, 35%).

### Part C. Preparation of N-(4-methoxybenzyl)-N-((6-(4,4,5,5-tetramethyl-1,3,2-dioxaborolan-2-yl)benzofuran-3-yl)methyl)methanesulfonamide.

The product from **Part B** (186mg, 0.44mmol) was subjected to the conditions described for **Example 88, Part D** to afford the title compound as a colorless solid (177mg, 86%).

### Part D. Preparation of N-((6-(3-tert-butyl-5-(2,4-dioxo-3,4-dihydropyrimidin-1(2H)-yl)-2-methoxyphenyl)benzofuran-3-yl)methyl)-N-(4-methoxybenzyl)methanesulfonamide.

In a microwave tube, a suspension of the product from **Part C** (169mg, 0.36mmol), the product from **Example C** (143mg, 0.36mmol), and 1.0M sodium carbonate solution (0.5mL, 0.50mmol) in 1:1 ethanol-toluene (3mL) was degassed by nitrogen sparge for 20min. The solution was treated with 1,1-bis(diphenylphosphino)ferrocene-palladium(II) chloride dichloromethane complex (7mg, 9µmol) followed by degassing for another 5min. The microwave tube was sealed and the mixture heated a 100°C in the microwave oven for 1h. The mixture was diluted with ethyl acetate and water, and acidified with 1M citric acid solution. The organic layer was extracted with saturated sodium chloride solution, dried (Na₂SO₄), and allowed to stand overnight over (3-mercaptopropyl) silica gel. Filtration and concentration in vacuo afforded an off-white foam which was purified by column chromatography on silica gel, eluting with 5-30% ethyl acetate in dichloromethane. The procedures afforded the title compound as a colorless solid (96mg, 43%).

### Part E. Preparation of N-((6-(3-tert-butyl-5-(2,4-dioxo-3,4-dihydropyrimidin-1(2H)-yl)-2-methoxyphenyl)benzofuran-3-yl)methyl)methanesulfonamide.

A solution of the product from **Part D** (88mg, 0.14mmo) in dichloromethane (1.4mL) was treated with trifluoroacetic acid (1.4mL) followed by stirring at room temperature for 18h, and then stirring at 40°C for 2h. The mixture was concentrated in vacuo to afford a dark, purple-brown foam, which was subjected to column chromatography on silica gel, eluting with 5-50% ethyl acetate in methylene chloride to afford an impure material, which was purified by reverse phase chromatography on a C-18 column, eluting with 1% water-TFA/acetonitrile. The procedures afforded the title compound as a solid (3.9mg). ¹H NMR (300 MHz, DMSO-*d₆*): δ 11.31 - 11.48 (m, 1 H) 8.01 (s, 1 H) 7.68 - 7.94 (m, 2 H) 7.40 - 7.65 (m, 2 H) 7.10 - 7.38 (m, 2 H) 5.65 (dd, *J*=7.91, 2.02 Hz, 1 H) 4.33 (d, *J*=5.88 Hz, 2 H) 3.23 (s, 3 H) 2.95 (s, 3 H) 1.41 (s, 9 H).

### Example 90. Preparation of N-((5-(3-tert-butyl-5-(2,4-dioxo-3,4-dihydropyrimidin-1(2H)-yl)-2-methoxyphenyl)-1-methyl-2,3-dihydro-1H-inden-1-yl)methyl)methanesulfonamide (compound IB-L0-2.25).

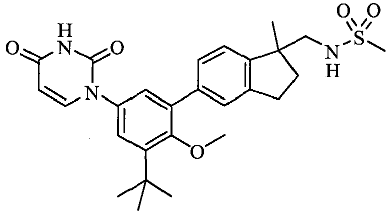

### Part A. Preparation of 5-bromo-1-(1,3-dithian-2-yl)-2,3-dihydro-1H-inden-1-ol.

A solution of 1,3-dithiane (11.96g, 99mmol) in anhydrous tetrahydrofuran (100mL) at -30°C was treated dropwise over 10min with n-butyllithium (2.5M in hexanes, 38.4mL, 96mmol) followed by stirring at -15°C for 2h. The solution was then treated with a solution of 5-bromo-2,3-dihydro-1*H*-inden-1-one (15g, 71.1mmol) in anhydrous tetrahydrofuran (250mL) over 1h, maintaining the temperature between -9°C and 2°C. The mixture was then allowed to set in the refrigerator at 2-8°C for 18h. The solution was concentrated in vacuo to afford a maroon oil, which was treated with 1 N hydrochloric acid solution and extracted with ether. The ether layer was extracted with saturated sodium chloride solution, dried (Na₂SO₄) and concentrated in vacuo to afford an amber oil (23.55g).

### Part B. Preparation of 2-(5-bromo-2,3-dihydro-1H inden-1-ylidene)-1,3-dithiane.

A solution of the product from **Part A** (23.55g, 71.1mmol) in benzene (350mL) was treated with p-toluenesulfonic acid monohydrate (3.0g) followed by stirring at reflux for 1h while removing water by means of a Dean-Stark trap. The mixture was extracted with saturated sodium bicarbonate solution and then with saturated sodium chloride solution. Drying (Na₂SO₄) and concentration in vacuo afforded the product as an amber, oil (22.27g).

### Part C. Preparation of 5-bromo-2,3-dihydro-1H indene-1-carboxylic acid.

A solution of the product from **Part B** (22.27g, 71.1mmol) in glatial acetic acid (375mL) was treated with concentrated hydrochloric acid solution (125mL) followed by stirring at reflux for 3h. The mixture was cooled and concentrated in vacuo by azeotroping off the acetic acid and water with toluene (3x). The brown oil obtained was filtered through a plug of 70-230 mesh silica gel in a 2L sintered glass funnel (volume of silica gel ca. 1800mL) eluting with dichloromethane to remove non-polar impurities (1,3-propanedithiol, *inter alia*) and then with ethyl acetate to elute the title compound, which was obtained as a brown solid (9.85g, 58%).

### Part D. Preparation of methyl 5-bromo-2,3-dihydro-1H-indene-1-carboxylate.

A suspension of the product from **Part C** (9.85g, 40.9mmol) in methanol (400mL) was treated with 4 N hydrogen chloride in 1,4-dioxane (125mL) and the mixture was stirred at reflux for 8h. The mixture was concentrated in vacuo to afford brown oil, which was purified by column chromatography on silica gel, eluting with 0-30% methyl t-butyl ether in chloroform. These procedures afforded the title compound as an amber oil (7.99g, 77%).

### Part E. Preparation of methyl 5-bromo-1-methyl-2,3-dihydro-1H indene-1-carboxylate.

A solution of the product from **Part D** (2.03g, 7.96mmol) in anhydrous tetrahydrofuran (40mL) at -78°C under N₂ was treated dropwise with lithium bis(trimethylsilyl)amide (1.0M in tetrahydrofuran, 9.55mL, 9.55mmol) over 10min. The solution was stirred at -78°C for 45min and then treated with methyl iodide (1.5mL, previously dried by passage through a plug of basic alumina). The mixture was then gradually allowed to warm to rt and was stirred for 18h. The mixture was quenched by addition of saturated ammonium chloride solution (2mL). The mixture was concentrated in vacuo to remove tetrahydrofuran and the residue was diluted with ethyl acetate. The mixture was extracted with saturated ammonium chloride solution and with saturated sodium chloride solution. Drying (Na₂SO₄) and concentration in vacuo afforded the title compound as an amber oil (2.06g, 96%).

### Part F. Preparation of 5-bromo-1-methyl-2,3-dihydro-1H-indene-1-carboxylic acid.

A solution of the product from **Part E** (2.06g, 7.65mmol) and potassium trimethylsilanoate (5.5g of 90%, 4.91g, 38.3mmol) in tetrahydrofuran (40mL) was stirred at reflux for 3h. The mixture was cooled and concentrated in vacuo to remove tetrahydrofuran. The maroon residue was dissolved in water (ca. 175mL) and extracted with methyl t-butyl ether. The aqueous phase was cooled to 0°C and acidified to pH 3 by addition of concentrated hydrochloric acid solution. The mixture was extracted with ethyl acetate (2x) and then with saturated sodium chloride solution. The solution was dried (Na₂SO₄) and treated with Darco G-60, followed by filtration through celite. The filtrate was concentrated in vacuo to afford the title compound as a light yellow solid (1.93g, 99%).

### Part G. Preparation of 5-bromo-1-methyl-2,3-dihydro-1H indene-1-carboxamide.

A solution of the product from **Part F** (1.56g, 6.12mmol) and DMF (473µL, 447mg, 6. 1 2mmol) in hexanes (100mL) was treated with oxalyl chloride (1.61mL, 2.32g, 18.4mmol) followed by stirring at room temperature for 1h. The mixture was treated with celite and then filtered through celite. The filtrate was concentrated in vacuo and dissolved in acetone (75mL) and cooled to 0°C. The solution was treated with 28% aqueous ammonia solution (75mL) followed by stirring at 0°C for 30min and then warming to room temperature. The mixture was concentrated in vacuo and extracted with ethyl acetate. The organic layer was extracted with saturated sodium chloride solution and dried (Na₂SO₄). Concentration in vacuo afforded the title compound as an oil (1.55g, 100%).

### Part H. Preparation of (5-bromo-1-methyl-2,3-dihydro-1H inden-1-yl)methanamine hydrochloride.

In a flask equipped with a vigreaux column and a short path distillation head, a solution of the product from **Part G** (1.21g, 4.76mmol) in anhydrous tetrahydrofuran (8mL) was warmed to a gentle reflux and treated dropwise with borane-dimethylsulfide complex (904µL, 723mg, 9.52mmol). The resulting mixture was stirred at reflux for 2h. The solution was cooled to rt and carefully treated with methanol until bubbling ceased, followed by careful treatment with 4N hydrogen chloride in 1,4-dioxane solution (4mL). The mixture was then concentrated in vacuo. The colorless solid obtained was triturated with ether and collected by filtration. After drying in a vacuum oven at 50°C for 2h, the title compound was obtained as a colorless solid (893mg, 68%).

### Part I. Preparation of tert-butyl (5-bromo-1-methyl-2,3-dihydro-1H-inden-1-yl)methylcarbamate.

A suspension of the product from **Part H** (893mg, 3.23mmo) in tetrahydrofuran (16mL) was treated with di-tert-butyl dicarbonate (846mg, 3.87mmol) and saturated sodium bicarbonate solution (7.2mL, ca. 6.46mmol) followed by stirring at room temperature for 18h. The mixture was diluted with ethyl acetate and extracted with water and saturated sodium chloride solution. The solution was dried (Na₂SO₄) and concentrated in vacuo. The residue was purified by flash chromatography, eluting with 5-40% ethyl acetate in hexanes. These procedures afforded the title compound as a colorless solid (1.03g, 94%).

### Part J. Preparation of tert-butyl (1-methyl-5-(4,4,5,5-tetramethyl-1,3,2-dioxaborolan-2-yl)-2,3-dihydro-1H-inden-1-yl)methylcarbamate.

The product from **Part I** (1.03g, 3.03mmol) was subjected to the conditions described for **Example 88, Part D to** afford the title compound as a colorless solid (977mg, 83%).

### Part K. Preparation of tert-butyl (5-(3-tert-butyl-5-(2,4-dioxo-3,4-dihydropyrimidin-1(2H)-yl)-2-methoxyphenyl)-1-methyl-2,3-dihydro-1H-inden-1-yl)methylcarbamate.

The product from **Part J** (965mg, 2.49mmol) was subjected to the conditions described for **Example 89, Part D** to afford the title compound as a colorless solid (618mg, 47%).

### Part L. Preparation of N-((5-(3-tert-butyl-5-(2,4-dioxo-3,4-dihydropyrimidin-1(2H)-yl)-2-methoxyphenyl)-1-methyl-2,3-dihydro-1H-inden-1-yl)methyl)methanesulfonamide.

The product from **Part K** (446mg, 0.84mmol) was dissolved in 4N hydrogen chloride in dioxane solution (12mL), followed by stirring at room temperature for 18h. The suspension of colorless solid obtained was then concentrated in vacuo. This material was suspended in dichloromethane (5mL) and cooled to 0°C, followed by sequential treatment with triethylamine (280µL, 203mg, 2.01mmol) and methanesulfonyl chloride (81µL, 120mg, 1.05mmol). The mixture was stirred at 0°C for 1h and then warmed to room temperature and diluted with dichloromethane. The mixture was extracted with 1M citric acid solution and then dried (Na₂SO₄) and concentrated in vacuo. The residue was dissolved in 3:1 tetrahydrofuran-water (8mL) and treated with potassium carbonate (231mg, 1.68mmol) followed by stirring at room temperature for 1h. The mixture was concentrated in vacuo and the residue diluted with water and then acidified to ca. pH 2 by addition of 1M citric acid. The product was extracted with ethyl acetate and the organic layer was extracted with saturated sodium chloride solution. Drying (Na₂SO₄) and concentration in vacuo afforded a colorless solid, which was purified by column chromatography on silica gel, eluting with 30-100% ethyl acetate in hexanes. The procedures afforded the title compound as a colorless solid (184mg, 43%). ¹H NMR (300 MHz, DMSO-*d₆*): δ 11.39 (s, 1 H) 7.77 (d, *J*=7.72 Hz, 1 H) 7.14 - 7.48 (m, 5 H) 7.06 (t, *J*=6.62 Hz, 1 H) 5.63 (d, *J*=7.72 Hz, 1 H) 3.18 - 3.33 (m, 3 H) 2.96 - 3.15 (m, 2 H) 2.85 - 3.00 (m, 2 H) 2.70 - 2.87 (m, 3 H) 2.10 - 2.34 (m, 1 H) 1.63 - 1.90 (m, 1 H) 1.40 (s, 9 H) 1.20 - 1.34 (m, 3 H).

### Example 91. Preparation of N-((5-(3-tert-butyl-5-(2,4-dioxo-3,4-dihydropyrimidin-1(2H)-yl)-2-methoxyphenyl)-1-fluoro-2,3-dihydro-1H-inden-1-yl)methyl)methanesulfonamide (compound IB-L0-2.12).

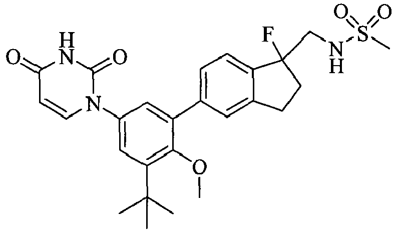

### Part A. Preparation of 5-(5-bromo-2,3-dihydro-1H-inden-1-ylidene)-2,2,3,3,7,7,8,8-octamethyl-4,6-dioxa-3,7-disilanonane.

To a solution of the product from **Example 90, Part C** (1.2g, 4.98mmol) in anhydrous THF (5mL) was added TBSCl (1.726g, 11.45mmol), and the resulting yellow solution was cooled to 0°C in an ice bath. A 1.0M solution of LiHMDS in THF (11.95mL, 11.95mmol) was added dropwise over 5min, and the resulting dark red solution was stirred at 0°C for 90min, and then at room temeprature for 6h. The solvent was removed in vacuo and the oily semi solid residue was treated with pentane (2 x 35mL) to precipitate LiCl. The slurry was filtered and the solvent was removed in vacuo to give the title compound as a brown oil (2.3g).

### Part B. Preparation of 5-bromo-1-fluoro-2,3-dihydro-1H-indene-1-carboxylic acid.

To a mixture of 1-chloromethyl-4-fluoro-1,1-diazoniabicyclo[2.2.2.]octane bis(tetrafluoroborate)(Selectfluor, 2.26g, 6.37mmol in CH₃CN (20mL) was added the product from **Part A** (2.3g, 4.90mmol) in CH₃CN (6mL). The resulting yellow-orange solution was stirred at room temperature overnight. The reaction mixture was poured into 50mL 1N HCl (aqueous), extracted with EtOAc (2 x 35mL). The combined organic extracts are washed with 0.5N NaOH (3 x 30mL). The combined aqueous extracts are washed with EtOAc (2 x 25mL), then adjusted mixture to pH 1 with 5N HCl (10mL) The resulting cloudy brown solution was extracted with EtOAc (2 x 50mL), the combined organic layers were washed with 10% NaCl and then treated with decolorizing carbon and stirred for 1h. The mixture was dried over anhydrous Na₂SO₄(s), filtered through Celite and the solvent removed in vacuo to give the title compound as leaving a yellow oil (0.84g).

### Part C. Preparation of 5-bromo-1-fluoro-2,3-dihydro-1H-indene-1-carbonyl chloride.

To a solution of the product from **Part B** (0.95g, 3.67mmol) in CH₂Cl₂ was added oxalyl chloride (0.96mL, 1.00mmol), followed by DMF (0.28mL). The resulting bubbling solution was stirred at room temperature for 2h, filtered through Celite, and the solvent was removed in vacuo to give the title compound as a brown oil (0.99g).

### Part D. Preparation of 5-bromo-1-fluoro-2,3-dihydro-1H-indene-1-carboxamide.

To a solution of the product from **Part C** (0.99g, 3.57mmol) in acetone (20mL) and at 0°C was added aqueous NH₄OH (28%, 0.28mL, 3.57mmol), and the resulting dark brown mixture was stirred at 0°C for 1h. The reaction mixture was concentrated in vacuo, and the residue was partitioned between water and EtOAc (2 x 50mL). The combined organic extracts were washed with 1N H₃PO₄, 10% NaHCO₃ (aq), 10% NaCl, and dried over anhydrous Na₂SO₄(s), filtered and concentrated in vacuo. The brown solid was purified by column chromatography on silica gel using a solvent gradient of CH₂Cl₂/MeOH (99/1to96/4). The title compound was obtained as a brown solid (0.205g, 22 %).

### Part E. Preparation of tert-butyl (5-bromo-1-fluoro-2,3-dihydro-1H-inden-1-yl)methylcarbamate.

To a solution of the product from **Part D** (0.234g, 0.907mmol) in anhydrous THF (5mL) at 80°C was added borane-DMS complex (0.172mL, 1.813mmol) dropwise. The reaction flask was equipped with a short-path condenser, and the mixture was stirred at reflux for 2h, collecting THF and DMS. The mixture was then cooled to room temperature and MeOH (5mL) was added, followed by 4N HCl in 1,4-dioxane (5mL). The solvent was removed in vacuo to give a colorless solid (0.25g, 98%). The solid was dissolved in THF (5mL), and to the solution was added triethylamine (0.137mL, 0.980mmol), followed by di-*tert*-butyl dicarbonate (0.214g, 0.980mmol). The cloudy mixture was stirred at room temperature for 30min, and 10% aq. NaHCO₃ (1mL) was added. The resulting mixture was stirred at room temperature for 18h and then concentrated in vacuo to an oily residue. The residue was dissolved in EtOAc (50mL), washed with water, 1N H₃PO₄, 10% NaCl, and dried over anhydrous Na₂SO₄(s). The drying agent was filtered off, and the solvent was removed in vacuo to give the title compound as an oil (0.27g, 88%).

### Part F. Preparation of tert-butyl (1-fluoro-5-(4,4,5,5-tetramethyl-1,3,2-dioxaborolan-2-yl)-2,3-dihydro-1H-inden-1-yl)methylcarbamate.

The product from **Part E** (0.27g, 0.784mmol) was subjected to the conditions described for **Example 72, Part B** to give the title compound as a tan solid (0.159g, 52 %).

### Part G. Preparation of tert-butyl (5-(3-tert-butyl-5-(2,4-dioxo-3,4-dihydropyrimidin-1(2H)-yl)-2-methoxyphenyl)-1-fluoro-2,3-dihydro-1H-inden-1-yl)methylcarbamate.

To a solution of the product from **Part F** (0.159g, 0.405mmol), the product from **Example C** (0.162g, 0.405 mol), 1,3,5,7 tetramethyl-2,4,8-trioxa-6-phospha-6-phenyl adamantane (PA-Ph, CAS 97739-46-3) (3.55 g, 0.012mmol) in THF (3mL) was added K₃PO₄ (0.181g, 0.851mmol) and water (1mL), followed by tris(dibenzylideneacetone)dipalladium(0) catalyst (3.71mg, 0.00405mmol). The resulting mixture was degassed by bubbling with N₂ for 20min, and then stirred at room temperature for 12h. The reaction mixture was diluted with EtOAc (50mL), washed with 1N H₃PO₄, 10% NaHCO₃, 10% NaCl, and dried over anhydrous Na₂SO₄(s). The mixture was filtered and solvent was removed in vacuo to give a brown oil, which was purified by column chromatography on silica gel, eluting with 98/2 CH₂Cl₂/MeOH. The title compound was isolated as a colorless solid (0.118g, 54%).

### Part H. Preparation of N-((5-(3-tert-butyl-5-(2,4-dioxo-3,4-dihydropyrimidin-1(2H)-yl)-2-methoxyphenyl)-1-fluoro-2,3-dihydro-1H-inden-1-yl)methyl)methanesulfonamide.

The product from **Part G** (0.118g, 0.219mmol) was dissolved in 4N HCl in 1,4-dioxane (2mL) and stirred at room temperature for 1h. The solvent was removed in vacuo and the residue was suspended in CH₂Cl₂ and evaporated (2 x 4mL) to give a colorless solid (0.10g, 96%). This solid was dissolved in CH₂Cl₂ (1mL) and the resulting slurry was stirred in an ice bath. Triethylamine (0.059mL, 0.422mmol) was added to the slurry resulting in a clear solution and to this was added methanesulfonyl chloride (0.02mL, 0.253mmol). The resulting mixture was stirred in the ice bath for 1h. The reaction mixture was diluted with CH₂Cl₂ 50mL, washed with 1N H₃PO₄, 10% NaHCO₃, 10% NaCl, and dried over anhydrous Na₂SO₄(s). The drying agent was filtered off, and solvent was removed in vacuo leaving a crude product that was purified by column chromatography on silica gel, eluting with a gradient of 1:1 to 3:7 hexane:EtOAc. The title compound was obtained as a colorless solid (64mg, 62 %). ¹H NMR (300 MHz, DMSO-d₆) δ 11.39 (s, 1 H) 7.77 (d, *J*=7.72 Hz, 1 H) 7.30 - 7.48 (m, 3 H) 7.12 - 7.32 (m, 3 H) 5.63 (d, *J*=7.72 Hz, 1 H) 3.27 (s, 3 H) 2.94 - 3.08 (m, 4 H) 2.91 (s, 3 H) 2.17 - 2.38 (m, 1 H) 1.76 - 1.97 (m, 1 H) 1.40 (s, 9 H).

### Example 92. Preparation of N-(6-(3-tert-butyl-5-(2,4-dioxo-3,4-dihydropyrimidin-1(2H)-yl)-2-methoxyphenyl)-3,4-dihydroisoquinolin-2(1H-yl)methanesulfonamide (compound IB-L0-2.43).

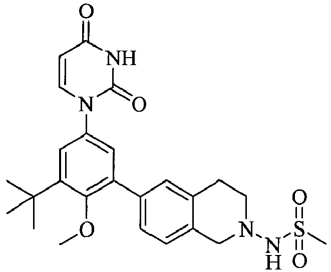

### Part A. Preparation of N-(3-bromophenethyl)-2,2,2-trifluoroacetamide.

To a solution of 2-(3-bromophenyl)ethanamine (10g, 50.0mmol) in dichloromethane (200ml) at 0°C were added 2,6-lutidine (6.40ml, 55.0mmol) and then trifluoroacetic anhydride (7.77ml, 55.0mmol) dropwise, and the reaction was stirred at room temperature overnight. Water was added at 0°C and the reaction was washed with 1M HCl, H₂O, and sat NaHCO₃. The organic was dried overmgSO₄, filtered and concentrated to provide the title compound as a tan solid (14.7g, 99%).

### Part B. Preparation of 1-(6-bromo-3,4-dihydroisoquinolin-2(1H)-yl)-2,2,2-trifluoro-ethanone.

To the product from **Part A** (14.70g, 49.6mmol) and paraformaldehyde (2.39g, 80mmol) was added a mixture of acetic acid (81ml) and sulfuric acid (53.7ml) at room temperature. The suspension was stirred for 60h during which time it became a solution. The reaction was poured into cold water. The reaction was diluted with ethyl acetate and washed with water, sat NaHCO₃, and brine. The organic layer was dried overmgSO₄, filtered and concentrated to provide the title compound, contaminated with the 8-bromo isomer, as a colorless oil (10.5 g, 67%).

### Part C. Preparation of 6-bromo-1,2,3,4-tetrahydroisoquinoline.

To a solution of the product from **Part B** (9.5g, 30.8mmol) in methanol (231ml) and water (77ml) at room temperature was added potassium carbonate (8.52g, 61.7mmol) and the reaction was stirred at room temperature for 30min. The reaction was diluted with water and 25% isopropanol in chloroform and the pH was adjusted to 9 with 1N HCl. The mixture was extracted twice with 25% isopropanol in chloroform. The combined organic layers were dried overmgSO₄, filtered and concentrated to give the title compound, contaminated with the 8-bromo isomer (6.55g, quantitative).

### Part D. Preparation of 6-bromo-2-nitroso-1,2,3,4-tetrahydroisoquinoline.

To a solution of the product from **Part C** (6.55g, 30.9mmol) in acetic acid (61.8ml) and 3N aq. hydrochloric acid (10.29ml, 30.9mmol) at 0°C was added 1.9M sodium nitrite (20.64ml, 39.2mmol) dropwise, and the reaction was stirred at room temperature overnight. The solvent was evaporated and the reaction was diluted with 25% isopropanol in chloroform and sat NaHCO₃. The aqueous layer was extracted twice with 25% isopropanol in chloroform. The combined organic layers were dried overmgSO₄, filtered and concentrated to give the title compound, contaminated with the 8-bromo isomer (6.97 g, 94%).

### Part E. Preparation of 6-bromo-3,4-dihydroisoquinolin-2(1H)-amine.

To a solution of the product from **Part D** (0.5g, 2.074mmol) in methanol (4.15ml) was added zinc (0.542g, 8.30mmol) and the reaction was cooled to 0°C, followed by dropwise addition of AcOH (4.15ml). The reaction was warmed to rt and the reaction was stirred for 2.5h. The reaction was filtered and the solid was washed with methanol. The filtrate was evaporated and the residue was diluted with water and 25% isopropanol in chloroform and saturated NaHCO₃ was added. A white solid was removed by filtration, and the aqueous layer was extracted twice with 25% isopropanol in chloroform. The combined organic layers were dried overmgSO₄, filtered and concentrated to give the title compound, contaminated with the 8-bromo isomer (0.472g, quantitative).

### Part F. Preparation of tert-butyl 6-bromo-3,4-dihydroisoquinolin-2(1H)-ylcarbamate.

A solution of the product from **Part E** (0.472g, 2.078mmol) in THF (20.78ml) was cooled to 0°C followed by addition of di-*tert*-butyl dicarbonate (0.531ml, 2.286mmol), and the reaction was stirred at room temperature overnight. Solvent was removed in vacuo, and the crude product was purified by column chromatography on silica gel (isolated lower R_{f} product) using a gradient starting with dichloromethane and ending with 10% ethyl acetate in dichloromethane to give the title compound (49mg, 73%).

### Part G. Preparation of tert-butyl 6-(4,4,5,5-tetramethyl-1,3,2-dioxaborolan-2-yl)-3,4-dihydroisoquinolin-2(1H)-ylcarbamate.

A solution of the product from **Part F** (100mg, 0.306mmol), bis(pinacolato)diboron (85mg, 0.336mmol), and potassium acetate (57.3µl, 0.917mmol) in 1,4-dioxane (3.0mL) was degassed by bubbling with N₂ gas for 15min. 1,1'-Bis(diphenylphosphino)ferrocene-palladium(II)dichloride dichloromethane complex (11.18mg, 0.015mmol) was added, and the resulting mixture was stirred at 95°C for 16h. The cooled solution was diluted with 25% isopropanol in chloroform and washed with water. The organic layer was dried overmgSO4, filtered and concentrated in vacuo. The product was purified by column chromatography on silica gel eluting with a gradient starting with dichloromethane and ending with 25% ethyl acetate in dichloromethane to give the title compound (70mg, 61%).

### Part H. Preparation of tert-butyl 6-(3-tert-butyl-5-(2,4-dioxo-3,4-dihydropyrimidin-1(2H)-yl)-2-methoxyphenyl)-3,4-dihydroisoquinolin-2(1H)-ylcarbamate.

A mixture of the product from **Example C** (74.8mg, 0.187mmol), the product from **Part G** (70mg, 0.187mmol) in EtOH (1.0mL), toluene (1.0mL) 1M aq. Na₂CO₃ (281µl, 0.281mmol) was degassed by bubbling with N₂ gas for 10min. 1,1'- Bis(diphenylphosphino)ferrocene-palladium(II) dichloride dichloromethane complex (6.84mg, 9.35µmol) was added, and degassing with N₂ was continued for 5min. The reaction mixture was sealed and heated at 78°C for 16h. The reaction was cooled and diluted with 25% isopropanol in chloroform and washed with water. The organic was dried overmgSO4, filtered and concentrated. The crude product was purified by column chromatography on silica gel, eluting with a gradient starting with dichloromethane and ending with ethyl acetate to give the title compound (53mg, 54%).

### Part I. Preparation of N-(6-(3-tert-butyl-5-(2,4-dioxo-3,4-dihydropyrimidin-1(2H)-yl)-2-methoxyphenyl)-3,4-dihydroisoquinolin-2(1H)-yl)methanesulfonamide.

To a solution of the product from **Part H** (25mg, 0.048mmol) in dichloromethane (0.5mL) at room temperature was added TFA (0.5mL) and the reaction was stirred for 30min, and then concentrated in vacuo. The residue was diluted with 25% isopropanol in chloroform and washed with sat NaHCO₃. The organic layer was dried overmgSO₄, filtered and concentrated to give a solid (17.8mg, 88%). To a solution of the solid in pyridine (0.5mL) at 0°C was added methanesulfonyl chloride (12.6µl, 0.162mmol) and the reaction was stirred at room temperature for 90min. Methanol was added and the reaction was stirred for 10min. The residue was diluted with 25% isopropanol in chloroform and washed with sat NaHCO₃. The organic layer was dried overmgSO₄, filtered and concentrated, and the product was purified by column chromatography on silica gel eluting with a gradient starting with dichloromethane and ending with ethyl acetate to give the title compound (11mg, 52%). ¹H NMR (300 MHz, DMSO-d₆) δ 11.39 (s, 1 H) 8.53 (s, 1 H) 7.76 (d, *J*=7.72 Hz, 1 H) 7.11 - 7.42 (m, 5 H) 5.63 (d, *J*=7.72 Hz, 1 H) 4.04 (s, 2 H) 3.28 (s, 3 H) 3.10 (d, *J*=5.52 Hz, 2 H) 2.98 (s, 3 H) 2.90 - 3.05 (m, 2 H) 1.40 (s, 9 H).

### Example 93. Preparation of N-((6-(5-(2,4-dioxo-3,4-dihydropyrimidin-1(2H)-yl)-3-(furan-2-yl)-2-methoxyphenyl)-1H-inden-3-yl)methyl)methanesulfonamide (compound IB-L0-2.65).

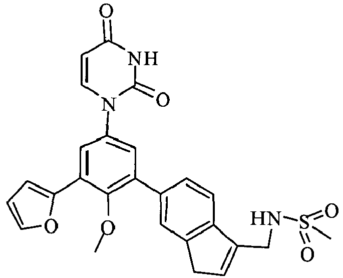

### Part A. Preparation of N-((6-(3-bromo-5-(2,4-dioxo-3,4-dihydropyrimidin-1(2H)-yl)-2-methoxyphenyl)-1H-inden-3-yl)methyl)methanesulfonamide.

The product from **Example 48, Part C** (0.242gm, 0.573mmol) and the product from **Example 79, Part E** (0.200gm, 0.57mmol) was subjected to the conditions described for **Example 79, Part F** to give the title compound as an off-white solid (0.104gm, 35%).

### Part B. Preparation of N-((6-(5-(2,4-dioxo-3,4-dihydropyrimidin-1(2H)-yl)-3-(furan-2-yl)-2-methoxyphenyl)-1H-inden-3-yl)methyl)methanesulfonamide.

A solution of the product from **Part A** (25.2mg, 0.049mmol) in 3:1 v/v THF-water (1.3mL) was combined in a microwave tube at room temperature with furan-2-ylboronic acid (6.91mg, 0.062mmol) and potassium phosphate (16.84mg, 0.097mmol). To this was added 1,1'-bis(di-tert-butyl-phosphino)ferrocene palladium dichloride (1.65mg, 2.53umole). The tube was sealed and the resulting mixture was purged with nitrogen for 4min and then heated for 16.5h in an oil bath at 50°C. The reaction mixture was partitioned between dilute HCl and ethyl acetate, and the organic phase was dried (MgSO4) and concentrated in vacuo. The residue was purified by chromatography on silica gel (ethyl acetate-hexanes) to give the title compound as an off white solid (11.4mg, 46%). ¹H NMR (300 MHz, DMSO-d₆) δ 11.45 (s, 1 H) 7.80 - 7.89 (m, 2 H) 7.73 - 7.79 (m, 2 H) 7.56 - 7.63 (m, 2 H) 7.50 (t, *J*=6.07 Hz, 1 H) 7.38 (d, *J*=2.94 Hz, 1 H) 7.09 (d, *J*=3.31 Hz, 1 H) 6.68 (dd, *J*=3.68, 1.84 Hz, 1 H) 6.58 (s, 1 H) 5.68 (d, *J*=7.72 Hz, 1 H) 4.19 (d, *J*=5.15 Hz, 2 H) 3.48 (s, 2 H) 3.34 (s, 3 H) 2.96 (s, 3 H).

### Example 94. Preparation of -N-((6-(5-(2,4-dioxo-3,4-dihydropyrimidin-1(2H)-yl)-2-methoxy-3-(thiophen-2-yl)phenyl)-1H-inden-3-yl)methyl)methanesulfonamide (compound IB-L0-2.63).

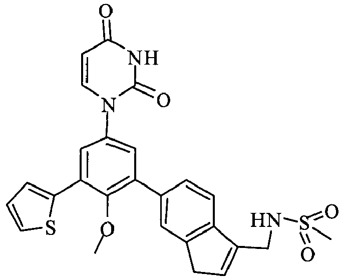

The product from **Example 93, Part A** (26.5mg, 0.051mmol) was reacted with thiophen-2-yl boronic acid (8.3mg, 0.065mmol) as described in **Example 93, Part B** to give the title compound as an off-white solid (8.6mg, 32%). ¹H NMR (300 MHz, DMSO-d₆) δ 11.47 (s, 1 H) 7.86 (d, *J*=7.72 Hz, 2 H) 7.55 - 7.78 (m, 5 H) 7.50 (t, *J*=6.25 Hz, 1 H) 7.38 (d, *J*=2.57 Hz, 1 H) 7.16 - 7.21 (m, 1 H) 6.58 (s, 1 H) 5.69 (d, *J*=7.72 Hz, 1 H) 4.19 (d, *J*=4.78 Hz, 2 H) 3.48 (s, 2 H) 3.30 (s, 3 H) 2.96 (s, 3 H).

### Example 95. Preparation of N-((6-(5-(2,4-dioxo-3,4-dihydropyrimidin-1(2H)-yl)-2-methoxy-3-(thiophen-3-yl)phenyl)-1H-inden-3-yl)methyl)methanesulfonamide (compound IB-L0-2.62).

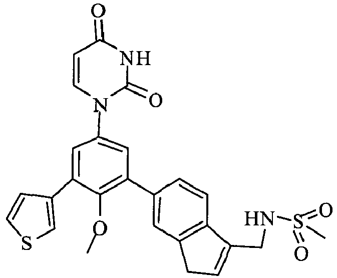

The product from **Example 93**, **Part A** (25.9mg, 0.050mmol) was reacted with thiophen-3-yl boronic acid (8.1mg, 0.063mmol) as described in **Example 93, Part B** to give the title compound as an off-solid (8.6mg, 33%). ¹H NMR (300 MHz, DMSO-d₆) δ 11.45 (d, *J*=1.84 Hz, 1 H) 7.93 (d, *J*=2.94 Hz, 1 H) 7.87 (d, *J*=7.72 Hz, 1 H) 7.53 - 7.75 (m, 6 H) 7.49 (t, *J*=6.25 Hz, 1 H) 7.39 (d, *J*=2.57 Hz, 1 H) 6.57 (s, 1 H) 5.68 (dd, *J*=7.91, 2.02 Hz, 1 H) 4.19 (d, *J*=5.15 Hz, 2 H) 3.47 (s, 2 H) 3.21 (s, 3 H) 2.96 (s, 3 H).

### Example 96. Preparation of N-((6-(5-(2,4-dioxo-3,4-dihydropyrimidin-1(2H)-yl)-3-(furan-3-yl)-2-methoxyphenyl)-1H-inden-3-yl)methyl)methanesulfonamide (compound IB-L0-2.67).

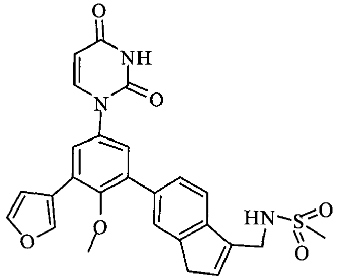

The product from **Example 93, Part A** (25.9mg, 0.050mmol) was reacted with furan-3-yl boronic acid (7.2mg, 0.064mmol) as described in **Example 93, Part B** to give the title compound as an off-white solid (10.6mg, 45%). ¹H NMR (300 MHz, DMSO-d₆) δ 11.46 (s, 1 H) 7.84 (d, *J*=8.09 Hz, 1 H) 7.80 (t, *J*=1.84 Hz, 1 H) 7.68 - 7.75 (m, 2 H) 7.54 - 7.64 (m, 2 H) 7.50 (t, *J*=6.07 Hz, 1 H) 7.35 (d, *J*=2.57 Hz, 1 H) 7.08 (d, *J*=1.47 Hz, 1 H) 6.57 (s, 1 H) 5.68 (d, *J*=8.09 Hz, 1 H) 3.47 (s, 2 H) 3.30 (s, 3 H) 2.96 (s, 3 H).

### Example 97. Preparation of 1-(3-tert-butyl-4-methoxy-5-(1-(methylsulfonyl)indolin-5-yl) phenyl)pyrimidine-2,4(1H,3H)-dione (compound IB-L0-2.32).

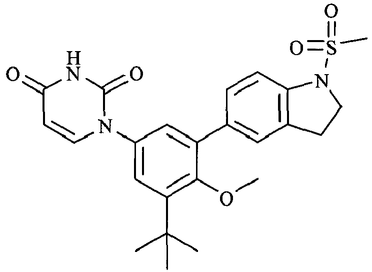

### Part A. Preparation of 5-bromo-1-(methylsulfonyl)indoline.

To DMF (5.0ml) was added sodium hydride (53mg, 1.3mmol) and the solution stirred at room temperature for 30min. 5-Bromoindoline (240mg, 1.2mmol) was added and the solution was stirred at room temperature for 30min. Methanesulfonyl chloride (94ul, 1.2mmol) was added and the solution stirred at room temperature overnight, then concentrated in vacuo. The crude product was purified by column chromatography on silica gel eluting with 2% CH₃OH/CHCl₃ to give the title compound (202mg, 60%).

### Part B. Preparation of 1-(methylsulfonyl)-5-(4,4,5,5-tetramethyl-1,3,2-dioxaborolan-2-yl) indoline.

The product from **Part A** (192mg, 0.70mmol) was subjected to the conditions described for **Example 72, Part B** to give the title compound (114mg, 51%).

### Part C. Preparation of 1-(3-tert-butyl-4-methoxy-5-(1-(methylsulfonyl)indolin-5-yl)phenyl) pyrimidine-2,4(1H,3H)-dione.

The product from **Example C** (58mg, 0.145mmol) and the product from **Part B** (56.2mg, 0.174mmol) were subjected to the conditions described for **Example 72, Part C** to give the title compound as a colorless solid (12mg, 18%). ¹H NMR (300 MHz, DMSO-*d*₆): δ 11.40 (d, *J*=1.84 Hz, 1 H) 7.76 (d, *J*=7.72 Hz, 1 H) 7.53-7.67 (m, 1 H) 7.45 (s, 1 H) 7.32-7.41 (m, 2 H) 7.23 (dd, *J*=13.60, 2.57 Hz, 2 H) 5.63 (dd, *J*=8.09, 2.21 Hz, 1 H) 3.99 (t, *J*=8.46 Hz, 2 H) 3.29 (s, 3 H) 3.18 (t, *J*=8.46 Hz, 2 H) 3.04 (s, 3 H).

### Example 98. Preparation of N-(6-(3-tert-butyl-5-(2,4-dioxo-3,4-dihydropyrimidin-1(2H)-yl)-2-methoxyphenyl)quinoxalin-2-yl)methanesulfonamide (compound IB-L0-2.26).

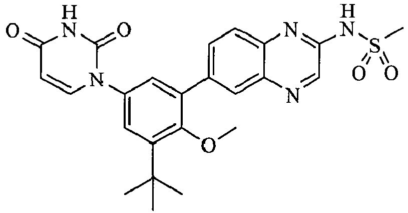

### Part A. Preparation of N-(4-bromo-2-nitrophenyl)-3-oxobutanamide.

A solution of diketene (0.32ml, 4.15mmol) in toluene (2ml) was added to an 80°C solution of 4-bromo-2-nitroaniline (900mg, 4.15mmol) in toluene (7ml) and the solution was heated at reflux for 5h. Triethylamine (0.58ml, 4.15mmol) in toluene (2ml) was added and refluxing was continued for 30min. The cooled solution was concentrated in vacuo and the crude product purified by column chromatography on silica gel eluting with 2:1 hexane/EtOAc to give the title compound as a yellow solid (920mg, 74%).

### Part B. Preparation of 6-bromoquinoxalin-2(1H)-one.

To a solution of sodium hydroxide (337mg, 8.4mmol) in H₂O (2.1ml) was added the product from **Part A** (423mg, 1.4mmol) and stirring was continued at 65°C for 1h. The cooled solution was diluted with H₂O (4ml) and sodium borohydride (31.9mg, 0.84mmol) was added and stirring was continued at room temperature for 1.5h. Ice was added to the solution followed by dropwise addition of 6N HCl until acidic. The resulting solid was collected by filtration, washed with H₂O, and dried in a vacuum oven to give the title compound (273mg, 86%).

### Part C. Preparation of 6-bromo-2-chloroquinoxaline.

To a flask containing phosphorus oxychloride (3.4ml, 36.5mmol) was added the product from **Part B** (255mg, 1.1mmol) and the solution was heated at 60°C overnight. The solution was cooled to room temperature, poured over ice and the resulting solid collected by filtration to give the title compound (239mg, 87%).

### Part D. Preparation of 6-bromo-N-(4-methoxybenzyl)quinoxalin-2-amine.

To a solution of the product from **Part C** (2.8g, 11.5mmol) in ethanol (58ml) was added (4-methoxyphenyl)methanamine (7.5ml, 57.5mmol) and the solution was stirred at room temperature for 1h. Solvent was concentrated in vacuo and the crude product was purified by column chromatography on silica gel eluting with 20% EtOAc/hexane to give the title compound (1.97g, 50%).

### Part E. Preparation of N-(4-methoxybenzyl)-6-(4,4,5,5-tetramethyl-1,3,2-dioxaborolan-2-yl) quinoxalin-2-amine.

The product from **Part D** (500mg, 1.45mmol) was subjected to the conditions described for **Example 72**, **Part B** to give the title compound (378mg, 66%).

### Part F. Preparation of 1-(3-tert-butyl-4-methoxy-5-(2-(4-methoxybenzylamino)quinoxalin-6-yl)phenyl)pyrimidine-2,4(1H,3H)-dione.

The product from **Part E** (133mg, 0.34mmol) was subjected to the conditions described for **Example 72, Part C** to give the title compound (125mg, 82%).

### Part G. Preparation of N-(6-(3-tert-butyl-5-(2,4-dioxo-3,4-dihydropyrimidin-1(2H)-yl)-2-methoxyphenyl)quinoxalin-2-yl)methanesulfonamide.

To a solution of the product from **Part F** (87mg, 0.16mmol) in CH₂Cl₂ (1.6ml) and H₂O (0.07ml) was added DDQ (40.4mg, 0.18mmol) and stirred vigorously at room temperature for 1h. The solution was filtered through Celite and the dark solid collected on the Celite was dissolved in 5ml CH₃OH. The methanol solution was filtered, solvent removed in vacuo and the crude intermediate was dissolved in pyridine (0.6ml). Methanesulfonyl chloride (11ul, 0.14mmol) was added and the solution was heated at 60°C overnight. The cooled solution was concentrated in vacuo and the crude product was purified by column chromatography on silica gel eluting with 2% CH₃OH/CHCl₃ to give the title compound (7.7mg, 12%). ¹H NMR (300 MHz, CDCl₃) δ 8.42 (s, 1 H) 8.29 (s, 1 H) 8.13 (s, 1 H) 7.88 (d, 1 H) 7.54 (s, 1 H) 7.19-7.43 (m, 4 H) 5.83 (dd, *J*=7.91, 2.39 Hz, 1H) 3.32 (s, 3 H) 3.27 (s, 3 H) 1.46 (s, 9 H).

### Example 99. Preparation of N-(5-(3-tert-butyl-5-(2,4-dioxo-3,4-dihydropyrimidin-1(2H)-yl)-2-methoxyphenyl)-2,3-dihydro-1H-inden-1-yl)methanesulfonamide (compound IB-L0-2.44).

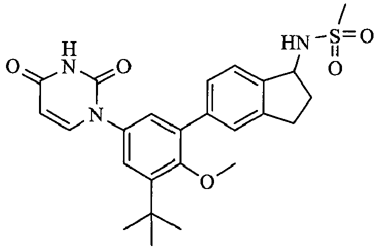

### Part A. Preparation of 5-bromo-2,3-dihydro-1H-inden-1-ol.

A suspension of 5-bromo-2,3-dihydro-1H-inden-1-one (2.07g, 9.81mmol) in ethanol (49mL) was treated with the sodium borohydride (186mg, 4.90mmol) all at once. After a few minutes, the solution warmed slightly and all solids dissolved. After stirring at room temperature for 1h, the mixture was concentrated in vacuo to remove ethanol. The gum obtained was partitioned between ethyl acetate and water. The organic layer was extracted with saturated sodium bicarbonate solution (2 x) and saturated sodium chloride solution. Drying (Na₂SO₄) and concentration in vacuo afforded the title compound (3.05g, 98%) as a colorless oil, which crystallized upon pumping under high vacuum overnight.

### Part B. Preparation of 1-azido-5-bromo-2,3-dihydro-1H-indene.

A solution of the product from **Part A** (1.01g, 4.73mmol) in toluene (8.1mL) was treated with the diphenyl phosphoroyl azide (1.23mL, 1.56g, 5.67mmol) followed by cooling to 0°C. The solution was treated dropwise with DBU (855µL, 863mg, 5.67mmol) followed by stirring at 0°C for 2h, and then warming to room temperature for 48h. The mixture was diluted with ethyl acetate and extracted with water and 1 M citric acid solution, and then with saturated sodium chloride solution. Drying (Na₂SO₄) and concentration in vacuo afforded a brown oil, which was purified by flash chromatography, eluting with 5-50 % ethyl acetate in hexanes. These procedures afforded the title compound (889mg, 79%) as a light yellow oil.

### Part C. Preparation of 5-bromo-2,3-dihydro-1H-inden-1-amine.

To a -15°C solution of 1M lithium aluminum hydride in THF (0.84ml, 0.84mmol) in THF (0.88ml) was added dropwise a solution of the product from **Part B** (200mg, 0.84mmol) and the solution was warmed to room temperature and stirred overnight. The solution was cooled to -10°C and 4:1 THF:H₂O (0.5ml) was added dropwise. The solution was stirred at room temperature for 4h, filtered through Celite and the filtrate concentrated in vacuo to give the title compound (151mg, 85%).

### Part D. Preparation of N-(5-bromo-2,3-dihydro-1H-inden-1-yl)methanesulfonamide.

To a solution of the product from **Part C** (150mg, 0.71mmol) in pyridine (3.5ml) was added methanesulfonyl chloride (61ul, 0.78mmol) and the solution was stirred at room temperature overnight. The solution was concentrated in vacuo and the crude product was purified by column chromatography on silica gel eluting with 20% EtOAc/hexane to give the title compound (111mg, 54%).

### Part E. Preparation of N-(5-(3-tert-butyl-5-(2,4-dioxo-3,4-dihydropyrimidin-1(2H)-yl)-2-methoxyphenyl)-2,3-dihydro-1H-inden-1-yl)methanesulfonamide.

The product from **Part D** (109mg, 0.38mmol) was subjected to the conditions described for **Example 72, Part B** and **Part C** to give the title compound (39mg, 60%). ¹H NMR (300 MHz, DMSO-*d₆)* δ 11.39 (d, *J*=1.84 Hz, 1 H) 7.77 (d, *J*=7.72 Hz, 1 H) 7.58 (d, *J*=8.82 Hz, 1 H) 7.39-7.48 (m, 3 H) 7.27 (d, *J*=2.57 Hz, 1 H) 7.19-7.23 (m, 1 H) 5.63 (dd, *J*=8.09, 2.21 Hz, 1 H) 4.86 (q, *J*=7.97 Hz, 1 H) 3.27 (s, 3 H) 3.04 (s, 3 H) 2.90-3.01 (m, 1 H) 2.71-2.90 (m, 1 H) 2.52-2.62 (m, 1 H) 1.85-1.98 (m, 1 H) 1.40 (s, 9 H).

### Example 100. Preparation of N-((5-(3-tert-butyl-5-(2,4-dioxo-3,4-dihydropyrimidin-1(2H)-yl)-2-methoxyphenyl)-2,3-dihydro-1H-inden-1-yl)methyl)methanesulfonamide (compound IB-L0-2.17).

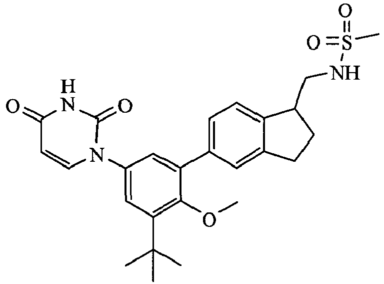

### Part A. Preparation of (E)-5-bromo-1-(methoxymethylene)-2,3-dihydro-1H-indene.

To a suspension of (methoxymethyl)triphenylphosphonium chloride (39.7g, 116mmol) in THF (210ml) at-20°C was added dropwise 1M potassium t-butoxide (95ml, 95mmol) and the solution stirred at -20°C for 20min. To this solution was added dropwise a solution of 5-bromo-2,3-dihydro-1H-inden-1-one (10.0g, 47.4mmol) in THF (230ml) and stirring was continued at -20°C for 30min then warmed to room temperature and stirred for 2h. The solution was filtered through Celite and the filtrate was concentrated in vacuo to give crude product which was purified by chromatography on a silica gel cartridge eluting with CH₂Cl₂/hexane to give the title compound (10.56g, 93%).

### Part B. Preparation of 5-bromo-2,3-dihydro-1H-indene-1-carbaldehyde.

To a solution of the product from **Part A** (1.44g, 6.0mmol) in CH₂Cl₂ (30ml) at -78°C was added dropwise 1M boron tribromide in CH₂Cl₂ (13.8ml, 13.8mmol) and stirring was continued at -78°C for 4h. The solution was poured into an ice-saturated. sodium bicarbonate mixture and stirred vigorously. The layers were separated and the aqueous layer was extracted with CH₂Cl₂ (2x), the organic extracts were combined, dried (Na₂SO₄), and concentrated in vacuo to give crude product which was purified by column chromatography on silica gel eluting with 10% EtOAc/hexane to give the title compound (604mg, 45%).

### Part C. Preparation of 1-(5-bromo-2,3-dihydro-1H-inden-1-yl)-N-(4-methoxybenzyl)-methanamine.

To a solution of the product from **Part B** (300mg, 1.3mmol) in CH₃OH (18.5ml) was added 4-methoxybenzylamine (0.17ml, 1.3mmol) and decaborane (49mg, 0.4mmol) and stirring was continued at room temperature for 1h, solvent was concentrated in vacuo and the crude product was purified by column chromatography on silica gel eluting with 3% CH₃OH/CHCl₃ to give the title compound (264mg, 57%).

### Part D. Preparation of N-((5-bromo-2,3-dihydro-1H-inden-1-yl)methyl)-N-(4-methoxy-benzyl)methanesulfonamide.

To a solution of the product from **Part C** (88mg, 0.25mmol) in CH₂Cl₂ (1.0ml) was added triethylamine (39ul, 0.28mmol) and methanesulfonyl chloride (22ul, 0.28mmol) and stirring was continued at room temperature for 1h, solvent was concentrated in vacuo and the crude product was purified by column chromatography on silica gel eluting with EtOAc/hexane to give the title compound (55mg, 51%).

### Part E. Preparation of N-(4-methoxybenzyl)-N-((5-(4,4,5,5-tetramethyl-1,3,2-dioxaborolan-2-yl)-2,3-dihydro-1H-inden-1-yl)methyl)methanesulfonamide.

The product from **Part D** (1.15g, 2.71mmol) was subjected to the conditions described for **Example 72, Part B** to give the title compound (840mg, 66%).

### Part F. Preparation of N-((5-(3-tert-butyl-5-(2,4-dioxo-3,4-dihydropyrimidin-1(2H)-yl)-2-methoxyphenyl)-2,3-dihydro-1H-inden-1-yl)methyl)methanesulfonamide.

The product from **Part E** (840mg, 2.1mmol) was subjected to the conditions described for **Example 72, Part C** and the isolated material (1.28g, 2.07mmol) was dissolved in CH₂Cl₂ (10ml) and trifluoroacetic acid (10ml) was added slowly. After stirring at room temperature for 1h, solvent was concentrated in vacuo and the crude product was suspended in 10% NaHCO₃, extracted with CH₂Cl₂ (3x), the organic extracts combined, dried (Na₂SO₄), and solvent concentrated in vacuo to give crude product which was purified by column chromatography on silica gel eluting with 2% CH₃OH/CHCl₃ to give title compound (0.84g, 81%). ¹H NMR (300 MHz, DMSO-*d₆*) δ 11.39 (s, 1 H) 7.77 (d, *J*=8.09 Hz, 1 H) 7.29-7.59 (m, 3 H) 7.25 (d, *J*=2.94 Hz, 1 H) 7.10-7.22 (m, 2 H) 5.63 (dd, *J*=7.72, 1.84 Hz, 1 H) 3.93 (s, 3 H) 3.26 (s, 2 H) 3.23-3.40 (m, 1 H) 2.89 (s, 3 H) 2.71-3.09 (m, 2 H) 2.14-2.32 (m, 1 H) 1.75-1.95 (m, 1 H) 1.40 (s, 9 H).

### Example 101. Preparation of 5-(3-tert-butyl-5-(2,4-dioxo-3,4-dihydropyrimidin-1(2H)-yl)-2-methoxyphenyl)-N-(methylsulfonyl)-2,3-dihydro-1H-indene-1-carboxamide (compound IB-L0-2.34).

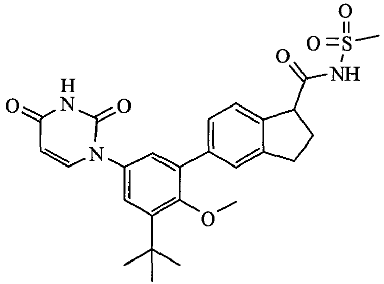

### Part A. Preparation of 5-bromo-2,3-dihydro-1H-indene-1-carboxylic acid.

To a solution of the product from **Example 100, Part B** (300mg, 1.3mmol) and 2-methyl-2-pentene (8ml) in tert-butanol (32ml) was added a solution of sodium chlorite (1.36g, 0.12mmol) in H₂O (12my) containing sodium dihydrogen phosphate (1.07g, 8.9mmol) and the mixture was stirred vigorously for 20min at room temperature. Solvents were concentrated in vacuo and the residue was diluted with H₂O, extracted with EtOAc (3x), extracts combined, dried (Na₂SO₄), and concentrated in vacuo to give the title compound (180mg, 56%).

### Part B. Preparation of 5-bromo-N-(methylsulfonyl)-2,3-dihydro-1H-indene-1-carboxamide.

To a solution of the product from **Part A** (100mg, 0.42mmol) in CH₂Cl₂ (1.7ml) was added carbonyldiimidazole (67.3mg, 0.42mmol) and the reaction was stirred for 2h at room temperature. Methanesulfonamide (39.5mg, 0.42mmol) and DBU (62.5mg, 0.42mmol) were added and stirring was continued at room temperature for 2h. Solution was diluted with CH₂Cl₂, washed 1N HCl, brine, dried (Na₂SO₄), concentrated in vacuo and the crude product was purified by column chromatography on silica gel eluting with 20% EtOAc/hexane to give the title compound (121mg, 92%).

### Part C. Preparation of N-(methylsulfonyl)-5-(4,4,5,5-tetramethyl-1,3,2-dioxaborolan-2-yl)-2,3-dihydro-1H-indene-1-carboxamide.

The product from **Part B** (159mg, 0.5mmol) was subjected to the conditions described for **Example 72, Part B** to give the title compound (144mg, 79%).

### Part D. Preparation of 5-(3-tert-butyl-5-(2,4-dioxo-3,4-dihydropyrimidin-1(2H)-yl)-2-methoxyphenyl)-N-(methylsulfonyl)-2,3-dihydro-1H-indene-1-carboxamide.

The product from **Part C** (134mg, 0.34mmol) was subjected to the conditions described for **Example 72, Part C** to give title compound (14mg, 8%). ¹H NMR (300 MHz, CDCl₃) δ 8.11 (m, 1 H) 7.08-7.57 (m, 7 H) 5.80 (dd, *J*=7.91, 2.39 Hz, 1 H) 4.07 (dd, *J*=9.01, 6.07 Hz, 1 H) 3.33 (s, 3 H) 3.08 (s, 3 H) 2.91-3.22 (m, 1 H) 2.35-2.74 (m, 1 H) 1.44 (s, 9H) 1.17-1.34 (m, 1 H) 0.60-1.00 (m, 1 H).

### Example 102. Preparation of 1-(3-(2-aminobenzo[d]thiazol-6-yl)-5-tert-butyl-4-methoxyphenyl)pyrimidine-2,4(1H,3H)-dione (compound IB-L0-2.39).

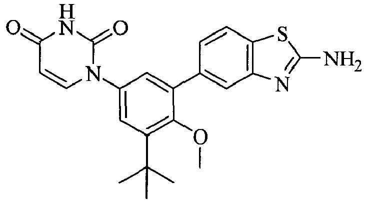

The title compound was prepared using the procedures described for the preparation of **Example 83,** substituting 5-bromo[*d*]thiazol-2-amine for 6-bromobenzo[*d*]thiazol-2-amine. ¹H NMR (300 MHz, DMSO-d₆) δ 11.40 (d, J=1.84 Hz, 1 H) 8.40 (s, 2 H) 7.84 (d, J=8.09 Hz, 1 H) 7.78 (d, J=7.72 Hz, 1 H) 7.54 (d, J=1.47 Hz, 1 H) 7.27 - 7.32 (m, 3 H) 5.64 (dd, J=8.09, 2.21 Hz, 1 H) 3.27 (s, 3 H) 1.41 (s, 9 H).

### Example 103. Preparation of N-(2-(5-(3-tert-butyl-5-(2,4-dioxo-3,4-dihydropyrimidin-1(2H)-yl)-2-methoxyphenyl)-2,3-dihydro-1H-inden-1-yl)propan-2-yl)methanesulfonamide (compound IB-L0-2.29).

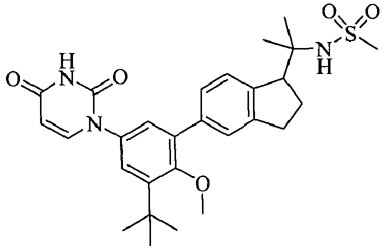

To a solution of the product from **Example 75, Part D** (20mg, 0.038mmol) in 1:1 benzene: MeOH (0.6ml) was added platinum(IV) oxide (1mg). The resulting mixture was stirred under 1 atm H₂ at room temperature for 1h, and then filtered thru celite, and concentrated in vacuo. The crude product was purified by column chromatography on silica gel using 3% MeOH in CHCl₃ as the eluent to give the title compound as a solid (14mg, 70%). ¹H NMR (300 MHz, DMSO-d₆) δ 11.39 (s, 1 H) 7.77 (d, *J*=7.72 Hz, 1 H) 7.58 (d, *J*=8.09 Hz, 1 H) 7.28 - 7.38 (m, 2 H) 7.21 - 7.26 (m, 2 H) 7.07 (s, 1 H) 5.63 (d, *J*=7.72 Hz, 1 H) 3.61 (dd, *J*=8.64, 5.33 Hz, 1 H) 3.25 (s, 3 H) 3.00 (s, 3 H) 2.75 - 2.98 (m, 2 H) 1.97 - 2.21 (m, 2 H) 1.40 (s, 9 H) 1.24 (d, *J*=8.46 Hz, 6 H).

### Example 104. Preparation of (S)-N-(2-(5-(3-tert-butyl-5-(2,4-dioxo-3,4-dihydropyrimidin-1(2H)-yl)-2-methoxyphenyl)-2,3-dihydro-1H-inden-1-yl)propan-2-yl)methanesulfonamide (compound IB-L0-2.22).

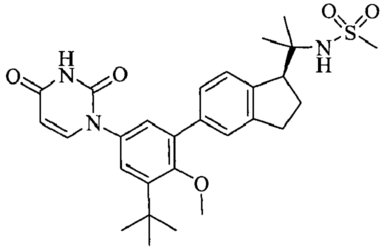

The product from **Example 103** (10mg) was subjected to chiral chromatography (Chiralpak AD-H column; eluting with 1:3 2-PrOH:hexanes(0.1 % TFA)). Isolation of the earlier eluting component gave the title compound (4.4mg). ¹H NMR identical to the product from **Example 103.**

### Example 105. Preparation of (R)-N-(2-(5-(3-tert-butyl-5-(2,4-dioxo-3,4-dihydropyrimidin-1(2H)-yl)-2-methoxyphenyl)-2,3-dihydro-1H-inden-1-yl)propan-2-yl)methanesulfonamide (compound IB-L0-2.37).

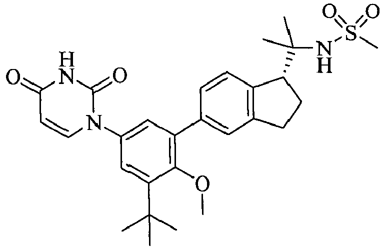

The product from **Example 103** (10mg) was subjected to chiral chromatography (Chiralpak AD-H column; eluting with 1:3 2-PrOH:hexanes(0.1% TFA)). Isolation of the later eluting component gave the title compound (4.2mg). ¹H NMR identical to the product from **Example 103.**

### Example 106. Preparation of (S)-N-((5-(3-tert-butyl-5-(2,4-dioxo-3,4-dihydropyrimidin-1(2H)-yl)-2-methoxyphenyl)-2,3-dihydro-1H-inden-1-yl)methyl)methanesulfonamide (compound IB-L0-2.9).

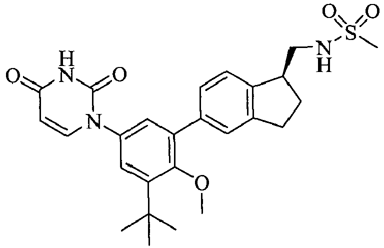

The product from **Example 100, Part F** (20mg) was subjected to chiral chromatography (Chiralpak AD-H column; eluting with 1:4 2-PrOH:hexanes(0.1 % TFA)). Isolation of the earlier eluting component gave the title compound (5.3mg). ¹H NMR identical to the product from **Example A-100,**

### Part F.

### Example 107. Preparation of (R)-N-((5-(3-tert-butyl-5-(2,4-dioxo-3,4-dihydropyrimidin-1(2H)-yl)-2-methoxyphenyl)-2,3-dihydro-1H-inden-1-yl)methyl)methanesulfonamide (compound IB-L0-2.15).

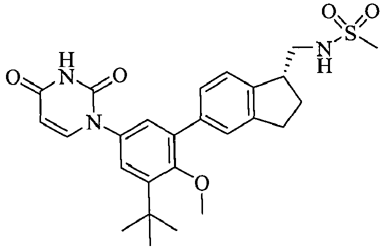

The product from **Example 100, Part F** (20mg) was subjected to chiral chromatography (Chiralpak AD-H column; eluting with 1:4 2-PrOH:hexanes(0.1% TFA)). Isolation of the later eluting component gave the title compound (5.7mg). ¹H NMR identical to product from **Example 100, Part F.**

### Example 108. Preparation of (S)-N-((5-(3-tert-butyl-5-(2,4-dioxo-3,4-dihydropyrimidin-1(2H)-yl)-2-methoxyphenyl)-1-fluoro-2,3-dihydro-1H-inden-1-yl)methyl)methanesulfonamide (compound IB-L0-2.20).

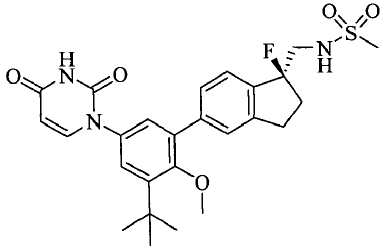

The product from **Example 91, Part H** was subjected to the conditions described in **Example 104** to give the title compound. ¹H NMR identical to the product from **Example 91, Part H.**

### Example 109. Preparation of (R)-N-((5-(3-tert-butyl-5-(2,4-dioxo-3,4-dihydropyrimidin-1(2H)-yl)-2-methoxyphenyl)-1-fluoro-2,3-dihydro-1H-inden-1-yl)methyl)methanesulfonamide (compound IB-L0-2.10).

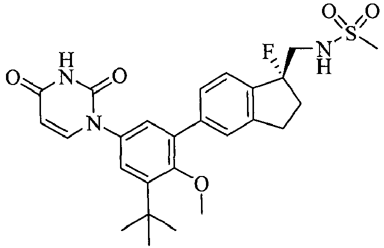

The product from **Example 91, Part H** was subjected to the conditions described in **Example** 104 to give the title compound. ¹H NMR identical to the product from **Example 91, Part H.**

### Example 110. Preparation of N-(6-(5-(2,4-dioxo-3,4-dihydropyrimidin-1(2H)-yl)-2-methoxy-3-tert-pentylphenyl)naphthalen-2-yl)methanesulfonamide (compound IB-L0-2.52).

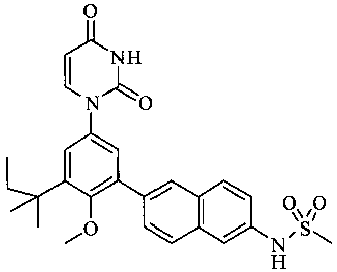

### Part A. Preparation of 1-(3-tert-butyl-5-iodo-4-methoxyphenyl)pyrimidine-2,4(1H,3H)-dione.

2-*tert*-Amylphenol (5.0g, 30mmol) was reacted according to the procedure from **Example C, Part A, Part B,** and **Part C** to provide the title product as a colorless solid.(6.7g, 56% overall yield for 3 steps).

### Part B. Preparation of N-(6-(5-(2,4-dioxo-3,4-dihydropyrimidin-1(2H)-yl)-2-methoxy-3-tert-pentylphenyl)naphthalen-2-yl)methanesulfonamide.

The product from **Part A** (100mg, 0.241mmol), the product from **Example 4A, Part B** (92mg, 0.266mmol), sodium carbonate (38.4mg, 0.362mmol) and 1,1'-bis(diphenylphosphino)ferrocene-palladium(II)dichloride dichloromethane complex (9.9mg, 0.012mmol) were dissolved in a toluene (4mL) and ethanol (4mL) solvent mixture which was sparged with nitrogen for 10min, then the mixture heated to 85°C for 18h. To the solution was then added CH₂Cl₂ (20mL) followed by 1N aqueous HCl (10mL), the organic layer separated 3-mercaptopropyl silica gel (100mg) and magnesium sulfate added. The solution was concentrated and purified by column chromatography on silica gel using 3% MeOH in CH₂Cl₂ as the eluent to provide the title compound as a colorless solid (71mg, 58%). ¹H NMR (300 MHz, DMSO-d₆): δ 11.41(s, 1H), 10.04 (s, 1H), 8.03 (s, 1H), 7.95 (t, *J*=8.7Hz, 2H), 7.79 (d, *J*=7.7Hz, 1H), 7.73(d, *J*=1.8Hz, 1H), 7.69(dd, *J*=8.8,1.6Hz, 1H), 7.42 (dd, *J*=8.8,2.2Hz, 1H), 7.37 (d, *J*=2.6Hz, 1H), 7.25 (d, *J*=2.6Hz, 1H), 5.65 (dd, *J*=8.1,1.6Hz, 1H), 3.22 (s, 3H), 3.08 (s, 3H), 1.84 (m, 2H), 1.38 (s, 6H), 0.73 (t, *J*=7.5Hz, 3H).

### Example 111. Preparation of N-((6-(3-tert-butyl-5-(2,4-dioxo-3,4-dihydropyrimidin-1(2H)-yl)-2-methoxyphenyl)-1H-inden-3-yl)methyl)-N-methylmethanesulfonamide (compound IB-L0-2.16).

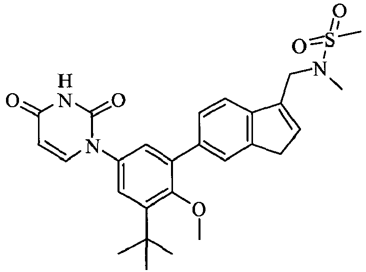

### Part A. Preparation of N-methyl-N-((6-(4,4,5,5-tetramethyl-1,3,2-dioxaborolan-2-yl)-1H-inden-3-yl)methyl)methanesulfonamide.

To a solution of the product from **Example 79, Part E** (210mg, 0.60mmol) in anhydrous THF (5ml) was added a 1.0M solution of lithium bis(trimethylsilyl)amide in toluene (0.60ml, 0.60mmol), and the resulting mixture was stirred at room temperature for 5min. Iodomethane (0.075ml, 1.20mmol) was added and the mixture was stirred at room temperature for 2h, and was partitioned between ethyl acetate and water. The organic layer was washed with brine, dried with sodium sulfate, filtered and concentrated in vacuo. The residue was purified by column chromatography on silica gel eluting with a gradient of ethyl acetate/ in hexane (10% to 25%) to give the title compound as a solid (125mg, 57%).

### Part B. Preparation of N-((6-(3-tert-butyl-5-(2,4-dioxo-3,4-dihydropyrimidin-1(2H)-yl)-2-methoxyphenyl)-1H-inden-3-yl)methyl)-N-methylmethanesulfonamide.

A mixture of the product from **Example C** (60.0mg, 0.15mmol), the product of **Part A** (54.5mg, 0.15mmol), potassium phosphate (66.9mg, 0.315mmol), PA-Ph (CAS 97739-46-3, 1.32mg, 4.5µmol) and tris(dibenzylideneacetone)dipalladium(0) (1.37mg, 1.5µmol) in tetrahydrofuran (3.0ml) and water (1.0ml) was purged with N₂ for 30min. The mixture was stirred at 50°C for 2h, and then partitioned between ethyl acetate and 1M HCl. The organic layer was washed with saturated sodium bicarbonate, brine, dried with sodium sulfate, and filtered. The filtrate was treated with 3-mercaptopropyl functionalized silica gel, filtered through celite and concentrated in vacuo. The crude product was purified by column chromatography on C-18 reversed-phase silica gel using a solvent gradient of 10-100% acetonitrile in water(0.1% TFA) to give the title compound as a solid (19mg, 24%). ¹H NMR (300 MHz, DMSO-d₆) δ 11.40 (d, J=1.84 Hz, 1 H) 7.78 (d, J=7.72 Hz, 1 H) 7.65 (m, 2 H) 7.49 (dd, J=7.72, 1.47 Hz, 1 H) 7.26 (m, 2.57 Hz, 2 H) 6.63 (s, 1 H) 5.64 (dd, J=7.72, 2.21 Hz, 1 H) 4.26 (s, 2 H) 3.51 (s, 2 H) 3.26 (s, 3 H) 3.01 (s, 3 H) 2.72 (s, 3 H) 1.41 (s, 9 H).

### Example 112. Preparation of N-((6-(3-tert-butyl-5-(2,4-dioxo-3,4-dihydropyrimidin-1(2H-yl)-2-methoxyphenyl)benzo[b]thiophen-2-yl)methyl)methanesulfonamide (compound IB-L0-2.40).

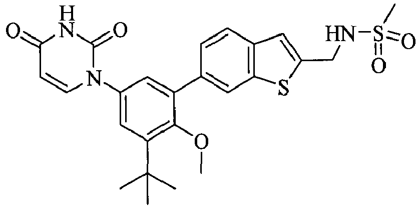

### Part A. Preparation of ethyl 6-bromobenzo[b]thiophene-2-carboxylate.

To a solution of 4-bromo-2-fluorobenzaldehyde (1.02g, 4.83mmol) in DMSO (4mL), was added ethyl 2-mercaptoacetate (0.58mL, 5.31mmol), followed by Et₃N (1.35mL, 9.65mmol), and the mixture was heated at 80°C for 3h. The resulting dark mixture was poured into water (50mL) and extracted with EtOAc (2 x 50mL). The combined organic extracts were washed with 10% NaCl, dried over anhydrous Na₂SO₄, filtered and concentrated in vacuo to give the title compound as a light yellow waxy solid (1.29g, 94%).

### Part B. Preparation of (6-bromobenzo[b]thiophen-2-yl)methanol.

To a solution of the product from **Part A** (0.82g, 2.88mmol) in Et₂O (20mL) at 0°C was added a 1M solution of lithium aluminum hydride in Et₂O (3.16mL, 3.16mmol) dropwise, and the resulting slurry was stirred between 5-10°C for 1h. The slurry was treated with 0.3mL H₂O, 0.3mL 15 % aq NaOH, 0.7mL H₂O stirred 30min, filtered and concentrated in vacuo to give the title compound as a colorless solid (0.58g, 83%).

### Part C. Preparation of 6-bromo-2-(bromomethyl)benzo[b]thiophene.

A mixture of the product from Part B (85mg, 0.35mmol), N-bromosuccinimide (74mg, 0.413mmol) and triphenylphosphine (106mg, 0.403mmol) in CH₂Cl₂ (2mL) was stirred at room temperature for 2h. The reaction mixture was diluted with 50mL CH₂Cl₂, washed with water, 10% NaHCO₃ and 10% NaCl, dried over anhydrousmgSO₄, filtered and concentrated in vacuo. The residue was purified by column chromatography on silica gel eluting with 9:1 hexane:EtOAc to yield the title compound as a white solid (96mg, 89%).

### Part D. Preparation of N-(4-methoxybenzyl)methanesulfonamide.

To a solution of (4-methoxyphenyl)methanamine (1.317g, 9.60mmol) in CH₂Cl₂ (10mL) was added methanesulfonyl chloride (0.34mL, 4.36mmol) dropwise. The mixture was stirred at room temperature for 2h. The reaction mixture was diluted with 50mL CH₂Cl₂ washed with 1N H₃PO₄, 10% NaCl, dried over anhydrousmgSO₄, filtered and concentrated in vacuo to give the title compound as a white solid (0.84g, 89%).

### Part E. Preparation of N-((6-bromobenzo[b]thiophen-2-yl)methyl)-N-(4-methoxybenzyl)-methanesulfonamide.

A solution of the product from **Part D** (0.223g, 1.037mmol) in EtOH (2mL) and 1.0M NaOH (1.1mL, 1.1mmol) was added to a slurry containing the product from **Part C** (0.317g, 1.037mmol) in EtOH (4mL). The resulting slurry was heated at reflux for 1h, and then concentrated in vacuo to give a pasty solid. The residue was partitioned between 40mL water and 40mL EtOAc. The organic layer was washed with 1N H₃PO₄, 10% NaHCO₃, 10% NaCl, dried over anhydrous Na₂SO₄, filtered and concentrated in vacuo leaving a yellow oil. The crude product was purified by column chromatography on silica gel eluting with CH₂Cl₂ to give the title compound as a colorless solid (0.15g, 33%).

### Part F. Preparation of N-(4-methoxybenzyl)-N-((6-(4,4,5,5-tetramethyl-1,3,2-dioxaborolan-2-yl)benzo[b]thiophen-2-yl)methyl)methanesulfonamide.

The product from **Part E** (0.15g, 0.34mmol) was subjected to the conditions described for the preparation of **Example 72, Part B** to give the title compound as a colorless solid (0.121g, 73%).

### Part G. Preparation of N-((6-(3-tert-butyl-5-(2,4-dioxo-3,4-dihydropyrimidin-1-(2H)-yl)-2-methoxyphenyl)benzo[b]thiophen-2-yl)methyl)-N-(4-methoxybenzyl)methanesulfonamide.

The product from **Part F** (24mg, 0.049mmol) was subjected to the conditions described for the preparation of **Example 72, Part C** to give the title compound as a colorless solid (20mg, 65%).

### Part H. Preparation of N-((6-(3-tert-butyl-5-(2,4-dioxo-3,4-dihydropyrimidin-1(2H)-yl)-2-methoxyphenyl)benzo[b]thiophen-2-yl)methyl)methanesulfonamide.

A solution of the product from **Part G** (14mg, 0.022mmol) in CH₂Cl₂ (0.3mL) and TFA (0.3mL) was stirred at room temperature for 4h and then concentrated in vacuo. The residue was partitioned between 10mL CH₂Cl₂ and 2mL 10% aq. NaHCO₃ and the organic layer was concentrated in vacuo. The crude product was purified by column chromatography on silica gel eluting with 99:1 CH₂Cl₂:MeOH to give the title compound as a colorless solid (5mg, 44%). ¹H NMR (300 MHz, DMSO-d₆) δ 11.40 (s, 1 H) 8.09 (s, 1 H) 7.82 - 7.97 (m, 3 H) 7.79 (d, *J*=7.72 Hz, 1 H) 7.47 - 7.63 (m, 1 H) 7.40 (s, 1 H) 7.26 - 7.34 (m, 1 H) 5.64 (d, *J*=7.72 Hz, 1 H) 4.48 (d, *J*=5.88 Hz, 2 H) 3.23 (s, 3 H) 2.95 (s, 3 H) 1.41 (s, 9 H).

### Example 113. Preparation of N-((6-(3-tert-butyl-5-(2,4-dioxo-3,4-dihydropyrimidin-1(2H)-yl)-2-methoxyphenyl)benzo[b]thiophen-3-yl)methyl)-N-methylmethanesulfonamide (compound IB-L0-2.21).

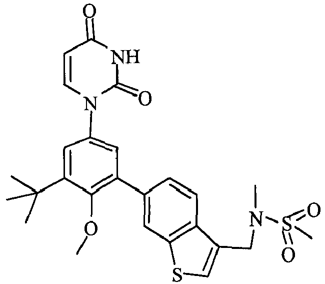

### Part A. Preparation of N-((6-bromobenzo[b]thiophen-3-yl)methyl)-N-methylmethanesulfonamide.

A mixture of the product from **Example 76, Part D** (0.100g, 0.382mmol), *N*-methylmethanesulfonamide (45.9mg, 0.421mmol) and potassium carbonate (0.127g, 0.918mmol) in *N,N*-dimethylacetamide (5mL). The mixture was stirred at 80°C for 11 h, cooled to room temperature and partitioned between diethylether and water (3x), dried overmgSO₄, filtered and concentrated in vacuo to give the title compound as a colorless waxy solid (0.128g, quant.).

### Part B. Preparation of N-methyl-N-((6-(4,4,5,5-tetramethyl-1,3,2-dioxaborolan-2-yl)benzo [b]thiophen-3-yl)methyl)methanesulfonamide.

The product from **Part A** (0.128g, 0.382mmol) was subjected to the conditions described for the preparation of **Example 72, Part B** to give the title compound as a colorless, crystalline solid (0.120g, 82%).

### Part C. Preparation of N-((6-(3-tert-butyl-5-(2,4-dioxo-3,4-dihydropyrimidin-1(2H)-yl)-2-methoxyphenyl)benzo[b]thiophen-3-yl)methyl)-N-methylmethanesulfonamide.

The product from **Part B** (50.6mg, 0.133mmol) was subjected to the conditions described for the preparation of **Example 79, Part F** to give the title compound as a colorless solid (61.5mg, 88%). ¹H NMR (300 MHz, DMSO-d₆) δ 11.41 (s, 1 H) 8.17 (d, *J*=1.47 Hz, 1 H) 8.09 (d, *J*=8.09 Hz, 1 H) 7.74 - 7.85 (m, 2 H) 7.63 (dd, *J*=8.46, 1.47 Hz, 1 H) 7.29 - 7.36 (m, 2 H) 5.65 (d, *J*=7.72 Hz, 1 H) 4.52 (s, 2 H) 3.24 (s, 3 H) 3.03 (s, 3 H) 2.70 (s, 3 H) 1.42 (s, 9 H).

### Example 114. Preparation of (E)-N-(4-(3-bromo-5-(2,4-dioxo-3,4-dihydropyrimidin-1(2H)-yl)-2-methoxystyryl)phenyl)methanesulfonamide (compound IB-L1-1.52).

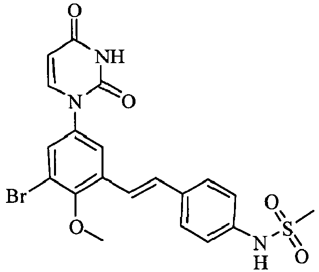

### Part A. Preparation of 2-bromo-4,6-diiodophenol.

A 1L round-bottom flask was charged with 2-bromophenol (8.65g, 50mmol) and methanol (100ml) to give a colorless solution. Sodium hydroxide (2.40g, 60.0mmol) was added and stirred until the hydroxide pellets had dissolved. The solution was cooled in an ice water bath and sodium iodide (5.6g, 37.4mmol) was added followed by drop-wise addition of sodium hypochlorite (17mL, 27.5mmol) to give a transparent brown/red solution and gradual precipitation of a thick, white solid. The addition of sodium iodide and bleach was repeated 3 times to give an orange mixture that was stirred for 2h, treated with a solution of sodium thiosulfate in water (20g in 100mL), stirred for 15min and treated drop-wise with concentrated HCl to a constant pH of 1. The mixture was stirred for 15min and filtered to collect a white solid that was washed repeatedly with water and dried to constant mass (14.7g, 69%).

### Part B. Preparation of 1-bromo-3,5-diiodo-2-methoxybenzene.

A 500mL round-bottom flask was charged with the product from **Part A** (14.7g, 34.6mmol), iodomethane (2.70ml, 43.3mmol), and sodium hydroxide (2.101ml, 39.8mmol) in acetone (96ml) to give a tan solution. The mixture was stirred for 24h and concentrated. The residue was dissolved in ethyl acetate, washed with water and saturated sodium chloride, dried over sodium sulfate, filtered and concentrated to give a white solid. The solid was recrystallized from hot hexane to give a white solid that was collected by filtration (12.3g, 81%).

### Part C. Preparation of 1-(3-bromo-5-iodo-4-methoxyphenyl)pyrimidine-2,4(1H,3H)-dione.

A 250mL round-bottom flask was charged with the product from **Part B** (8.09g, 18.44mmol), pyrimidine-2,4(1H,3H)-dione (2.273g, 20.28mmol), N-(2-cyanophenyl)picolinamide (0.823g, 3.69mmol), copper (I) iodide (0.351g, 1.844mmol) and potassium phosphate (8.22g, 38.7mmol) in DMSO (70ml). The mixture was sealed, sparged with nitrogen for 15min and heated at 60°C for 16h. The mixture was partitioned with ethyl acetate and water. The organic layer was washed with 1M HCl, water, brine, dried with sodium sulfate, and filtered. The filtrate was treated with 3-mercaptopropyl functionalized silica gel (Aldrich catalog #538086), filtered through celite and evaporated to give an off-white solid (3.92g, 50%).

### Part D. Preparation of (E)-N-(4-(3-bromo-5-(2,4-dioxo-3,4-dihydropyrimidin-1(2H)-yl)-2-methoxystyryl)phenyl)methanesulfonamide.

To a 100ml round-bottom flask was added the product from **Part** C (846mg, 2.0mmol), the product from **Example 41B, Part B** (482mg, 2.000mmol), potassium phosphate (892mg, 4.20mmol), 1,3,5,7-tetramethyl-6-phenyl-2,4,8-trioxa-6-phosphaadamante (PA-Ph) (CAS 97739-46-3) (17.54mg, 0.060mmol) and tris(dibenzylideneacetone)dipalladium(0) (18.31mg, 0.020mmol) in THF (12.0ml) and water (4.0ml). The flask was sealed and the mixture was sparged with nitrogen for 5min and stirred at ambient temperature for 72h. The mixture was partitioned with ethyl acetate and 1M HCl. The organic layer was washed with saturated sodium bicarbonate, brine, dried with sodium sulfate and filtered. The filtrate was treated with 3-mercaptopropyl functionalized silica gel, filtered and evaporated. The residue was triturated with a minimal amount of methanol/ CH₂Cl₂ to give the title compound as a white solid (595mg, 60%). ¹H NMR (300 MHz, DMSO-d₆) δ ppm 3.03 (s, 3 H) 3.82 (s, 3 H) 5.69 (dd, J=7.72,1.50 Hz, 1 H) 7.24 (d, J=8.46 Hz, 2 H) 7.35 (m, 2 H) 7.61 (d, J=8.46 Hz, 2 H) 7.69 (d, J=2.21 Hz, 1 H) 7.78 (d, J=8.09 Hz, 1 H) 7.87 (d, J=2.21 Hz, 1 H) 9.90 (s, 1 H) 11.50 (s, 1 H). MS (ESI-) m/z 490,492 (M-H)+.

### Example 115. Preparation of (E)-N-(4-(5-(2,4-dioxo-3,4-dihydropyrimidin-1(2H)-yl)-2-methoxy-3-(thiophen-2-yl)styryl)phenyl)methanesulfonamide (compound IB-L1-1.48).

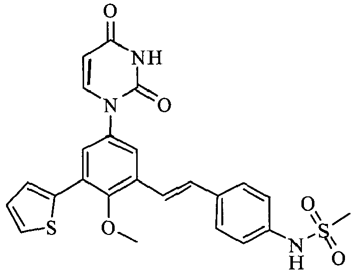

To a 5ml microwave tube was added the product from **Example 114, Part D** (40mg, 0.081mmol), thiophen-2-ylboronic acid (10.40mg, 0.081mmol), 1,1'-bis(di-tert-butylphosphino)ferrocene palladium dichloride (2.65mg, 4.06µmol) and potassium phosphate (34.5mg, 0.162mmol) in THF (3.0ml) and water (1.0ml). The vessel was sealed and the mixture was sparged by nitrogen for 5min and heated at 50°C for 3h. The mixture was partitioned with ethyl acetate and 1M HCl. The organic layer was washed with saturated sodium bicarbonate, brine, dried with sodium sulfate and filtered. The filtrate was treated with 3-mercaptopropyl functionalized silica gel, filtered through celite and evaporated. The residue was purified by reverse phase chromatography to give the title compound as a white solid (20mg, 50%). ¹H NMR (300 MHz, DMSO-d₆) δ ppm 3.03 (s, 3 H) 3.70 (s, 3 H) 5.70 (dd, J=7.72, 2.21 Hz, 1 H) 7.18 (dd, J=5.43, 4.05 Hz, 1 H) 7.25 (d, J=8.82 Hz, 2 H) 7.35 (s, 2 H) 7.63 (d, J=8.82 Hz, 2 H) 7.68 (m, 2 H) 7.77 (m, 2 H) 7.83 (d, J=7.72 Hz, 1 H) 9.89 (s, 1 H) 11.49 (d, J=2.21 Hz, 1 H). MS (ESI+) m/z 496 (M+H)+.

### Example 116. Preparation of (E)-N-(4-(5-(2,4-dioxo-3,4-dihydropyrimidin-1(2H)-yl)-3-(furan-2-yl)-2-methoxystyryl)phenyl)methanesulfonamide (compound IB-L1-1.46).

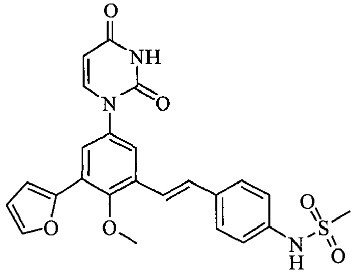

The title compound was prepared according to the procedure of **Example 115** substituting furan-2-ylboronic acid for thiophen-2-ylboronic acid to give a white solid (22mg, 56%). ¹H NMR (300 MHz, DMSO-d₆) δ ppm 3.03 (s, 3 H) 3.76 (s, 3 H) 5.69 (d, J=7.72 Hz, 1 H) 6.69 (dd, J=3.31, 1.84 Hz, 1 H) 7.08 (d, J=2.57 Hz, 1 H) 7.25 (d, J=8.46 Hz, 2 H) 7.36 (m, 2 H) 7.63 (d, J=8.82 Hz, 2 H) 7.67 (d, J=2.57 Hz, 1 H) 7.77 (d, J=2.57 Hz, 1 H) 7.82 (m, J=7.72 Hz, 2 H) 9.88 (s, 1 H) 11.48 (s, 1 H). MS (ESI+) m/z 497 (M+NH4)+.

### Example 117. Preparation of (E)-N-(4-(5-(2,4-dioxo-3,4-dihydropyrimidin-1(2H)-yl)-2-methoxy-3-(pyridin-4-yl)styryl)phenyl)methanesulfonamide (compound IB-L1-1.55).

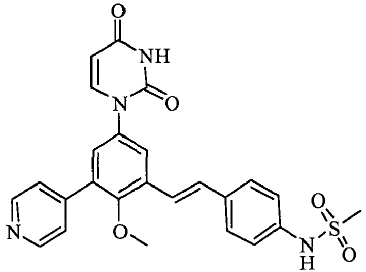

The title compound was prepared according to the procedure of **Example 115** substituting 4-(4,4,5,5-tetramethyl-1,3,2-dioxaborolan-2-yl)pyridine for thiophen-2-ylboronic acid to give a white solid (15mg, 38%). ¹H NMR (300 MHz, DMSO-d₆) δ ppm 3.03 (s, 3 H) 3.49 (s, 3 H) 5.72 (dd, J=7.72, 2.21 Hz, 1 H) 7.25 (d, J=8.46 Hz, 2 H) 7.38 (d, J=4.41 Hz, 2 H) 7.51 (d, J=2.57 Hz, 1 H) 7.63 (d, J=8.82 Hz, 2 H) 7.80 (d, J=5.88 Hz, 2 H) 7.85 (d, J=7.72 Hz, 1 H) 7.97 (d, J=2.57 Hz, 1 H) 8.77 (d, J=6.25 Hz, 2 H) 9.90 (s, 1 H) 11.51 (d, J=2.21 Hz, 1 H). MS (ESI+) m/z 491 (M+H)+.

### Example 118. Preparation of (E)-N-(4-(5-(2,4-dioxo-3,4-dihydropyrimidin-1(2H)-yl)-2-methoxy-3-(pyridin-3- yl)styryl)phenyl)methanesulfonamide (compound IB-L1-1.53).

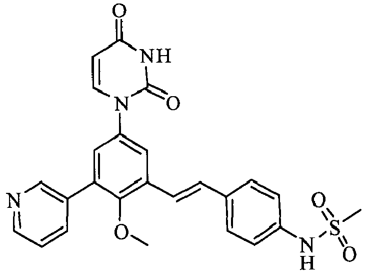

The title compound was prepared according to the procedure of **Example 115** substituting 3-(4,4,5,5-tetramethyl-1,3,2-dioxaborolan-2-yl)pyridine for thiophen-2-ylboronic acid to give a white solid (19mg, 48%). ¹H NMR (300 MHz, DMSO-d₆) δ ppm 3.02 (s, 3 H) 3.45 (s, 3 H) 5.71 (dd, J=8.09, 2.21 Hz, 1 H) 7.24 (d, J=8.46 Hz, 2 H) 7.37 (d, J=2.94 Hz, 2 H) 7.47 (d, J=2.57 Hz, 1 H) 7.63 (m, 3 H) 7.85 (d, J=7.72 Hz, 1 H) 7.93 (d, J=2.57 Hz, 1 H) 8.15 (m, 1 H) 8.68 (dd, J=4.80 Hz, 1.47 Hz, 1 H) 8.86 (d, J=1.84 Hz, 1 H) 9.89 (s, 1 H) 11.50 (d, J=2.21 Hz, 1 H). MS (ESI+) m/z 491 (M+H)+.

### Example 119. Preparation of (E)-N-(4-(5-(2,4-dioxo-3,4-dihydropyrimidin-1(2H)-yl)-2-methoxy-3-(thiophen-3-yl)styryl)phenyl)methanesulfonamide (compound IB-L1-1.47).

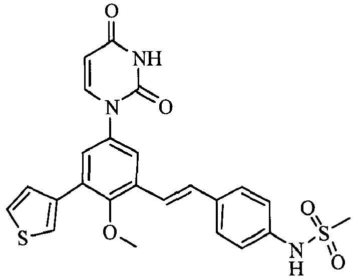

The title compound was prepared according to the procedure of **Example 115** substituting thiophen-3-ylboronic acid for thiophen-2-ylboronic acid to give a white solid (19mg, 38%). ¹H NMR (300 MHz, DMSO-d₆) δ ppm 3.02 (s, 3 H) 3.55 (s, 3 H) 5.69 (d, J=8.09 Hz, 1 H) 7.24 (d, J=8.46 Hz, 2 H) 7.36 (s, 2 H) 7.55 (m, 2 H) 7.61 (d, J=8.46 Hz, 2 H) 7.67 (dd, J=5.15, 2.94 Hz, 1 H) 7.78 (d, J=2.57 Hz, 1 H) 7.83 (d, J=7.72 Hz, 1 H) 7.93 (dd, J=2.57, 0.96 Hz, 1 H) 9.88 (s, 1 H) 11.48 (s, 1 H). MS (ESI-) m/z 494 (M-H)+.

### Example 120. Preparation of (E)-N-(4-(5-(2,4-dioxo-3,4-dihydropyrimidin-1(2H)-yl)-3-(furan-3-yl)-2-methoxystyryl)phenyl)methanesulfonamide (compound IB-L1-1.50).

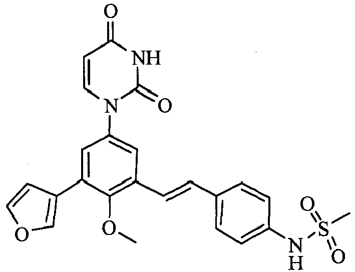

The title compound was prepared according to the procedure of **Example 115** substituting furan-3-ylboronic acid acid for thiophen-2-ylboronic acid to give a white solid (14mg, 29%). ¹H NMR (300 MHz, DMSO-d₆) δ ppm 3.02 (s, 3 H) 3.69 (s, 3 H) 5.69 (d, J=8.09 Hz, 1 H) 7.05 (dd, J=2.57, 0.90 Hz, 1 H) 7.24 (d, J=8.82 Hz, 2 H) 7.34 (s, 2 H) 7.61 (m, 3 H) 7.74 (d, J=2.57 Hz, 1 H) 7.80 (m, 2 H) 8.25 (s, 1 H) 9.88 (s, 1 H) 11.49 (s, 1 H). MS (ESI-) m/z 478 (M-H)+.

### Example 121. Preparation of (E)-N-(4-(5-(2,4-dioxo-3,4-dihydropyrimidin-1(2H)-yl)-3-(1-hydroxy-2-methylpropan-2-yl)-2-methoxystyryl)phenyl)methanesulfonamide (compound IB-L1-1.45).

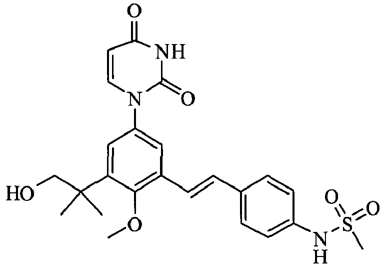

### Part A. Preparation of 2-(2-hydroxy-3,5-diiodophenyl)acetic acid.

To a 250mL round-bottom flask was added 2-(2-hydroxyphenyl)acetic acid (Aldrich, 3.04g, 20mmol) in acetonitrile (50ml) to give a colorless solution. N-iodosuccimide (9.00g, 40.0mmol) was added portionwise over 15min to give a red/brown transparent solution that was stirred for 16h. The mixture was concentrated and the resulting solid was triturated in 75mL of water and filtered to collect an orange solid that was dried under vacuum. The crude solid was recrystallized from toluene to give a light orange powder (6.0g, 74%).

### Part B. Preparation of methyl 2-(3,5-diiodo-2-methoxyphenyl)acetate.

To a 250mL round-bottom flask was added the product from **Part A** (6g, 14.85mmol), potassium carbonate (6.16g, 44.6mmol), and dimethyl sulfate (4.12g, 32.7mmol) in acetone (49.5ml) to give a brown suspension. The suspension was heated at reflux for 16h, cooled, concentrated and the residue was partitioned between EtOAc and water. The EtOAc layer was washed with brine, dried (Na₂SO₄) and concentrated to a brown oil that was chromatographed on a 40g silica cartridge eluting with 3:1 hexane/EtOAc to give a yellow oil (6.0g, 94%).

### Part C. Preparation of methyl 2-(3,5-diiodo-2-methoxyphenyl)-2-methylpropanoate.

To a 100mL round-bottom flask under nitrogen was added the product from **Part B** (1.728g, 4mmol) in anhydrous THF (20ml) and HMPA (2ml) to give a colorless solution. Methyl iodide (1.251ml, 20.00mmol) was added and the solution was cooled to -40°C. Potassium t-butoxide(12.00ml, 12.00mmol) was added drop-wise and the mixture was stirred at -40 to -20°C for 30min and quenched with 1M HCl to a pH of 1. The mixture was extracted 3 X 40ml with EtOAc. The extracts were combined, washed with brine, dried (Na₂SO₄) and concentrated. The crude product was flash chromatographed on a 40g ISCO silica cartridge eluting with 9:1 hexane/EtOAc to give the bis-methylated product as a yellow oil (1.63g, 89%).

### Part D. Preparation of 2-(3,5-diiodo-2-methoxyphenyl)-2-methylpropanoic acid.

A suspension of the product from **Part C** (2.63g, 5.72mmol) in MeOH (40ml) and THF (40ml) was treated with 4.0M sodium hydroxide (28ml, 112mmol) and heated at 80°C for 48h. The organic solvent was evaporated and the remaining aqueous solution was acidified with 1M HCl producing a solid that was collected by filtration, washed with water and dried to give the desired carboxylic acid (2.46g, 96%).

### Part E. Preparation of 2-(3,5-diiodo-2-methoxyphenyl)-2-methylpropan-1-ol.

A solution of the product from **Part D** (1.00g, 2.242mmol) in THF (40ml) was treated dropwise with borane THF complex 1.0M (20ml, 20mmol) and then heated at 50°C for 24h. The mixture was treated with methanol (20mL), refluxed for 30min and concentrated. The resulting residue was washed with water, brine, dried with sodium sulfate, filtered and evaporated. The residue was chromatographed on silica gel eluting with hexane/EtOAc (4:1) to give the desired product (810mg, 84%).

### Part F. Preparation of tert-butyl(2-(3,5-diiodo-2-methoxyphenyl)-2-methylpropoxy)-dimethylsilane.

A solution of the product from **Part E** (432mg, 1.000mmol) in DMF (5ml) was treated with tert-butyldimethylchlorosilane (301mg, 2.000mmol), and imidazole (204mg, 3.00mmol) and stirred for 2h. The mixture was partitioned between 1M HCl and ethyl acetate. The organic layer was washed with saturated sodium bicarbonate, brine, dried with sodium sulfate, filtered and evaporated. The residue was chromatographed on silica gel eluting with hexane/EtOAc (9:1) to give the desired product (522mg, 96%).

### Part G. Preparation of 1-(3-(1-(tert-butyldimethylsilyloxy)-2-methylpropan-2-yl)-5-iodo-4-methoxyphenyl)pyrimidine-2,4(1H,3H)-dione.

To a 50mL round-bottom flask was added the product from **Part F** (520mg, 0.952mmol), pyrimidine-2,4(1H,3H)-dione (117mg, 1.047mmol), N-(2-cyanophenyl)picolinamide (42.5mg, 0.190mmol), copper(I) iodide (18.13mg, 0.095mmol) and potassium phosphate (424mg, 1.999mmol) in DMSO (5ml). The vessel was sealed, sparged with nitrogen and then heated at 60°C for 24h. The mixture was partitioned between 1M HCl and ethyl acetate. The organic layer was washed with saturated sodium bicarbonate, brine, dried with sodium sulfate, and filtered. The filtrate was treated with 3-mercaptopropyl functionalized silica gel, filtered and evaporated. The residue was chromatographed on silica gel eluting with hexane/EtOAc (3:2) to give the product as a solid (285mg, 65%).

### Part H. Preparation of (E)-N-(4-(3-(1-(tert-butyldimethylsilyloxy)-2-methylpropan-2-yl)-5-(2,4-dioxo-3,4-dihydropyrimidin-1(2H)-yl)-2-methoxystyryl)phenyl)methanesulfonamide.

To a 5ml microwave tube was added the product from **Part G** (53mg, 0.1mmol), the product from **Example 41B, Part B** (24mg, 0.1mmol), potassium phosphate (44.0mg, 0.2mmol), PA-Ph (CAS 97739-46-3) (0.87mg, 3.0µmol) and tris(dibenzylideneacetone)palladium(0) (0.9mg, 1µmol) in THF (3.0ml) and water (1.0ml). The vessel was sealed and the mixture was sparged with nitrogen for 5min and then heated at 50°C for 2h. The mixture was partitioned between 1M HCl and ethyl acetate. The organic layer was washed with saturated sodium bicarbonate, brine, dried with sodium sulfate and filtered. The filtrate was treated with 3-mercaptopropyl functionalized silica gel, filtered and evaporated. The residue was chromatographed on silica gel eluting with hexane/EtOAc (1:1) to give a solid (50mg, 83 %).

### Part I. Preparation of (E)-N-(4-(5-(2,4-dioxo-3,4-dihydropyrimidin-1(2H)-yl)-3-(1-hydroxy-2-methylpropan-2-yl)-2-methoxystyryl)phenyl)methanesulfonamide.

A solution of the product from **Part H** (120mg, 0.20mmol) in THF (5.0ml) was treated with 1 M TBAF (0.800ml, 0.800mmol) in THF and stirred for 16h. The mixture was partitioned with water and ethyl acetate. The organic layer was washed (3 X brine), dried with sodium sulfate, filtered and evaporated. The residue was chromatographed on silica gel eluting with 4% methanol in CH₂Cl₂ to give a solid (85mg, 88 %). ¹H NMR (300 MHz, DMSO-d₆) δ ppm 1.30 (s, 6 H) 3.01 (s, 3 H) 3.62 (d, J=5.52 Hz, 2 H) 3.77 (s, 3 H) 4.67 (t, J=5.33 Hz, 1 H) 5.66 (d, J=8.09 Hz, 1 H) 7.21 (m, 5 H) 7.62 (m, 3 H) 7.72 (d, J=8.09 Hz, 1 H) 9.85 (s, 1 H) 11.42 (s, 1 H). MS (ESI+) m/z 503 (M+NH4)+.

### Example 122. Preparation of (E)-N-(4-(5-(2,4-dioxo-3,4-dihydropyrimidin-1(2H)-yl)-3-iodo-2-methoxystyryl)phenyl)methanesulfonamide (compound IB-L1-1.51).

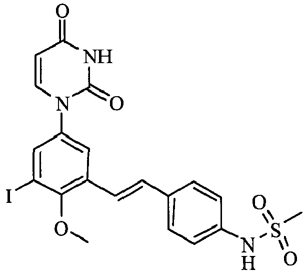

### Part A. Preparation of 1,3,5-triiodo-2-methoxybenzene.

In a 250mL pressure vessel was added 2,4,6-triiodophenol (5g, 10.60mmol) in MTBE (60ml) to give a yellow solution. The solution was cooled in an ice bath and 2.0M trimethylsilyldiazomethane (7.95ml, 15.90mmol) was added at a fast drip followed by dropwise addition of methanol (6mL) resulting in calm bubbling. The vessel was sealed and stirred at room temperature for 4h. The reaction solution was partitioned between EtOAc and water and the organic layer was washed with 1M HCl, saturated NaHCO₃, and saturated NaCl. The EtOAc was dried (MgSO₄), filtered and concentrated to give a tan solid that was used without purification (4.8g, 94 %).

### Part B. Preparation of 1-(3,5-diiodo-4-methoxyphenyl)pyrimidine-2,4(1H,3H)-dione.

To a 100mL round-bottom flask under N₂ was added the product from **Part A** (3.5g, 7.2mmol), 1H-pyrimidine-2,4-dione (0.97g, 8.64mmol), and potassium phosphate tribasic (3.2g, 15.0mmol) in DMSO (50ml) to give a colorless suspension. N-(2-cyanophenyl)picolinamide (320mg, 1.44mmol) was added and the mix was sparged with N₂ for 5min. Copper(I) iodide (137mg, 0.72mmol) was added and the mix was sparged once again for 10min, placed under N₂ and heated at 60°C for 18h. The mixture was cooled and partitioned between EtOAc and water adjusting the pH to 1 with HCl. The aqueous layer was extracted 2X with EtOAc. The organics were combined, washed with water, saturated NaHCO₃, and saturated NaCl, dried (Na₂SO₄), treated with 3-mercaptopropyl functionalized silica, filtered and concentrated. The resulting solid was triturated in 2:1 hexane/EtOAc to give an off white powder (2.2g, 62 %).

### Part C. Preparation of (E)-N-(4-(5-(2,4-dioxo-3,4-dihydropyrimidin-1(2H)-yl)-3-iodo-2-methoxystyryl)phenyl)methanesulfonamide.

In a 5ml microwave tube was mixed the product from **Part B** (141mg, 0.30mmol), the product from **Example 41B, Part B** (72.3mg, 0.300mmol), 1,1'-bis(diphenylphosphino)ferrocene-palladium(II)dichloride CH₂Cl₂ complex (12.25mg, 0.015mmol) and potassium phosphate (70.0mg, 0.330mmol) in THF (3.0ml) and water (1.0ml). The mixture was sparged with nitrogen for 5min and heated at 50°C for 2h. The mixture was partitioned with ethyl acetate and 1M HCl. The organic layer was washed with saturated sodium bicarbonate, brine, dried with sodium sulfate and filtered. The filtrate was treated with 3-mercaptopropyl functionalized silica gel, filtered and evaporated. The residue was chromatographed on silica eluting with 5% methanol in CH₂Cl₂ to give a solid (47mg, 29%). ¹H NMR (300 MHz, DMSO-d₆) δ ppm 3.02 (s, 3 H) 3.77 (s, 3 H) 5.67 (d, J=7.72 Hz, 1 H) 7.28 (m, 4 H) 7.60 (d, J=8.82 Hz, 2 H) 7.76 (d, J=8.09 Hz, 1 H) 7.81 (d, J=2.57 Hz, 1 H) 7.86 (d, J=2.21 Hz, 1 H) 9.90 (s, 1 H) 11.48 (s, 1 H). MS (ESI-) m/z 538 (M-H)+.

### Example 123. Preparation of (E)-N-(4-(5-(2,4-dioxo-3,4-dihydropyrimidin-1(2H)-yl)-2-methoxy-3-(methylsulfonyl)styryl)phenyl)methanesulfonamide (compound IB-L1-1.49).

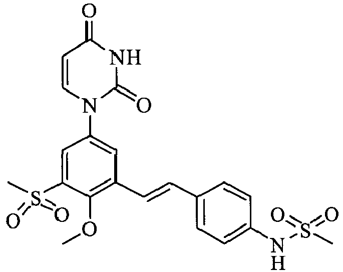

### Part A. Preparation of 4-nitrobenzene-2-diazo-1-oxide.

To a 250mL round-bottom flask was added 2-amino-4-nitrophenol (6.165g, 40.0mmol) in 48% tetrafluoroboric acid (15ml). Sodium nitrite (2.76g, 40.0mmol) in water (6ml) was added dropwise at 0°C and the mixture was stirred at room temperature for 30min. The solid was collected by filtration, washed with tetrafluoroboric acid and water. The solid was suspended in acetone (50ml), filtered and dried to give a solid (3.31g, 50%).

### Part B. Preparation of 2-(methylthio)-4-nitrophenol.

To a 1L beaker was added the product from **Part A** (2.70g, 16.35mmol) in ice water (250g) to give a brown suspension. Copper (0.520g, 8.18mmol) was added, followed by addition of sodium thiomethoxide (2.292g, 32.7mmol) in water (50ml) slowly. The mixture was stirred at room temperature for 24h. The mixture was filtered and the filtrate was acidified with 1M HCl producing a solid that was collected by filtration and dried (2.53g, 84%).

### Part C. Preparation of 2-(methylsulfonyl)-4-nitrophenol.

To a 250mL round-bottom flask was added the product from **Part B** (1.111g, 6.00mmol) in MeOH (20ml) to give a brown suspension. Oxone (7.746g, 12.60mmol) in water (20ml) was added slowly at 0°C. The mixture was warmed to room temperature, stirred for 1h and partitioned with ethyl acetate and 1M HCl. The organic layer was washed with brine, dried with sodium sulfate, filtered and evaporated. The residue was chromatographed on silica gel eluting with 1% to 5% methanol in CH₂Cl₂ to give a solid (0.472g, 36%).

### Part D. Preparation of 2-iodo-6-(methylsulfonyl)-4-nitrophenol.

To a 50mL round-bottom flask was added the product from **Part C** (470mg, 2.164mmol) in MeOH (10ml) and water (2.5ml). Iodine monochloride (0.13ml, 2.60mmol) in CH₂Cl₂ (2.0mL) was added drop-wise and the mixture was stirred at room temperature, poured into water (200mL) and stirred for 10min. The resulting solid was collected by filtration and dried (636mg, 86%).

### Part E. Preparation of 1-iodo-2-methoxy-3-(methylsulfonyl)-5-nitrobenzene.

To a 50mL pressure vessel was added the product from **Part D** (630mg, 1.836mmol) in MTBE (6ml) to give a yellow solution. The mixture was cooled in an ice bath and 2M trimethylsilyldiazomethane (1.377ml, 2.75mmol) was added at a fast drip followed by drop-wise addition of MeOH (0.4ml) resulting in calm bubbling. The vessel was sealed and stirred at room temperature for 1h. The mixture was partitioned with ethyl acetate and 1M HCl. The organic layer was washed with saturated sodium bicarbonate, brine, dried with sodium sulfate, filtered and evaporated to give an off-white solid (655mg, 100%).

### Part F. Preparation of 3-iodo-4-methoxy-5-(methylsulfonyl)aniline.

To a 250mL round-bottom flask was added the product from **Part E** (0.650g, 1.820mmol), ammonium chloride (0.146g, 2.73mmol), and iron (0.508g, 9.10mmol) in THF/MeOH/water (50ml, 2/2/1). The mixture was refluxed for 2h, cooled and filtered. The filtrate was evaporated and the residue was partitioned with ethyl acetate and water. The organic layer was washed with brine, dried with sodium sulfate, filtered and evaporated to give a solid (590mg, 99%).

### Part G. Preparation of (E)-N-(3-iodo-4-methoxy-5-(methylsulfonyl)phenylcarbamoyl)-3-methoxyacrylamide.

To a 100mL round-bottom flask was added the product from **Part F** (500mg, 1.528mmol) in DMF (15.0ml). The solution was cooled under nitrogen to -20°C and (E)-3-methoxyacryloyl isocyanate (15.28ml, 6.11mmol; prepared as described by Santana, L.; et al. J. Heterocyclic Chem. 1999, 36, 293-295) was added dropwise. The mixture was stirred at this temperature for 15min, then warmed to room temperature and stirred for 45min. The mixture was diluted with ethyl acetate and washed by water (3 x 50ml), brine (3 x 50ml), dried with sodium sulfate, filtered and evaporated. The residue was triturated with ethyl acetate/hexane to give a solid (425mg, 61%).

### Part H. Preparation of 1-(3-iodo-4-methoxy-5-(methylsulfonyl)phenyl)pyrimidine-2,4(1H,3H)-dione.

To a 100mL round-bottom flask was added the product from **Part G** (420mg, 0.925mmol) in ethanol (10ml) to give a suspension. Concentrated sulfuric acid (1mL, 18.76mmol) in water (10ml) was added and the mixture was heated at 110°C for 2h. The reaction mix was cooled, diluted with water (50ml) and stirred for 10min. The solid material was collected by filtration, washed with water and dried to give a white solid (325mg, 83%).

### Part I. Preparation of (E)-N-(4-(5-(2,4-dioxo-3,4-dihydropyrimidin-1(2H)-yl)-2-methoxy-3-(methylsulfonyl)styryl)phenyl)methanesulfonamide.

In a 5ml microwave tube was added the product from **Part H** (63.3mg, 0.15mmol), the product from **Example 41B, Part B** (36.2mg, 0.150mmol), potassium phosphate (66.9mg, 0.315mmol), PA-Ph (CAS 97739-46-3) (1.315mg, 4.50µmol) and tris(dibenzylideneacetone)dipalladium(0) (1.374mg, 1.500µmol) in THF (3.0ml) and water (1.0ml). The vessel was sealed and the mixture was sparged with nitrogen for 5min and heated at 50°C for 2h. The mixture was partitioned with ethyl acetate and 1M HCl. The organic layer was washed with saturated sodium bicarbonate, brine, dried with sodium sulfate and filtered. The filtrate was treated with 3-mercaprpropyl functionalized silica gel, filtered and evaporated. The residue was triturated with methanol/ CH₂Cl₂ to give a solid (62mg, 84%). ¹H NMR (300 MHz, DMSO-d₆) δ ppm 3.03 (s, 3 H) 3.37 (s, 3 H) 3.94 (s, 3 H) 5.72 (d, J=7.72 Hz, 1 H) 7.26 (m, 3 H) 7.45 (m, 1 H) 7.65 (d, J=8.46 Hz, 2 H) 7.77 (d, J=2.57 Hz, 1 H) 7.81 (d, J=8.09 Hz, 1 H) 8.21 (d, J=2.57 Hz, 1 H) 9.93 (s, 1 H) 11.52 (s, 1 H). MS (ESI+) m/z 509 (M+NH4)+.

### Example 124. Preparation of (E)-methyl 2-(3-tert-butyl-5-(2,4-dioxo-3,4-dihydropyrimidin-1(2H)-yl)-2-methoxystyryl)-5-(methylsulfonamido)benzoate (compound IB-L1-1.7).

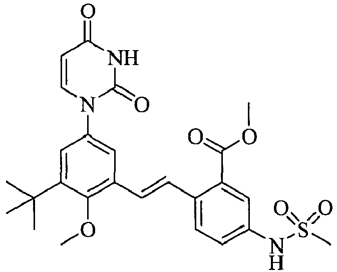

### Part A. Preparation of methyl 2-((diethoxyphosphoryl)methyl)-5-nitrobenzoate.

To a solution of methyl 2-methyl-5-nitrobenzoate (0.40 g, 2.05mmol) in CCl₄ (20ml) was added N-bromosuccinimide (365mg, 2.05mmol) and 2,2'-azobisisobutyronitrile (34mg, 0.21mmol). The resulting mixture was stirred at reflux for 18h, cooled to room temperature and partitioned between EtOAc (50ml) and H₂O (50ml). The organic layer was dried over Na₂SO₄, filtered and concentrated in vacuo. The crude product was purified by column chromatography on silica gel using 1:3 EtOAc:hexanes as the eluent to give the bromide as an oil (345mg, 61%). The oil was placed in triethylphosphite (5ml) and heated with stirring at 120°C for 3h. The mixture was allowed to cool to room temperature, and the crude product was purified by column chromatography on silica gel using 5% MeOH in CH₂Cl₂ as the eluent. The title compound was obtained as an oil (313mg, 75%).

### Part B. Preparation of (E)-methyl2-(3-tert-butyl-5-(2,4-dioxo-3,4-dihydropyrimidin-1(2H)-yl)-2-methoxystyryl)-5-nitrobenzoate.

To a solution of the product from **Part A** (360mg, 1.09mmol) and the product from **Example 41A, Part D** (329mg, 1.09mmol) in anhydrous CH₂Cl₂ (10ml) was added potassium *tert*-butoxide (305mg, 2.72mmol). The resulting dark red solution was stirred at room temperature for 1h, and then poured into 1 N aq. HCl (10ml). The resulting mixture was extracted with CH₂Cl₂ (10ml), dried over Na₂SO₄, filtered and concentrated in vacuo to give a solid. A solution of the solid in thionyl chloride (2.3ml) was heated at 85°C for 30min, and the thionyl chloride was removed in vacuo. The residue was stirred in a 2:1 mixture of CH₂Cl₂ and MeOH (3ml) for 30min, and evaporated to dryness in vacuo. The crude product was purified by column chromatography on silica gel using 3% MeOH in CH₂Cl₂ as the eluent to give the title compound (350mg, 69%).

### Part C. Preparation of (E)-methyl 2-(3-tert-butyl-5-(2,4-dioxo-3,4-dihydropyrimidin-1(2H)-yl)-2-methoxystyryl)-5-(methylsulfonamido)benzoate.

To a solution of the product from **Part B** (465mg, 0.97mmol) in a 2:2:1 mixture of THF:MeOH:H₂O (10ml) was added iron powder (271mg, 4.85mmol), and ammonium chloride (78mg, 1.46mmol). The mixture was heated at 80°C for 45min, filtered through celite, and concentrated to dryness in vacuo. The residue was combined with methanesulfonyl chloride (0.16ml, 2.0mmol) and triethylamine (0.392ml, 4.85mmol) in anhydrous CH₂Cl₂ (10ml) and the resulting mixture was stirred at room temperature for 3h. The mixture was partitioned between 1 N HCl (20ml) and CH₂Cl₂ (20ml), and the organic layer was dried over Na₂SO₄, filtered and concentrated in vacuo. The crude product was purified by column chromatography on silica gel using 3% MeOH in CH₂Cl₂ as the eluent to give the title compound (270mg, 53%). ¹H NMR (300 MHz, DMSO-d₆) δ 11.42 (s, 1 H) 10.07 (s, 1 H) 7.90 (d, *J*=8.82 Hz, 1 H) 7.66 - 7.79 (m, 3 H) 7.52 (d, *J*=2.57 Hz, 1 H) 7.44 (dd, *J*=8.64, 2.39 Hz, 1 H) 7.14 - 7.26 (m, 2 H) 5.65 (dd, *J*=7.72, 1.84 Hz, 1 H) 3.86 (s, 3 H) 3.79 (s, 3 H) 3.04 (s, 3 H) 1.38 (s, 9 H).

### Example 125. Preparation of (E)-2-(3-tert-butyl-5-(2,4-dioxo-3,4-dihydropyrimidin-1(2H)-yl)-2-methoxystyryl)-5-(methylsulfonamido)benzoic acid (compound IB-L1-1.4).

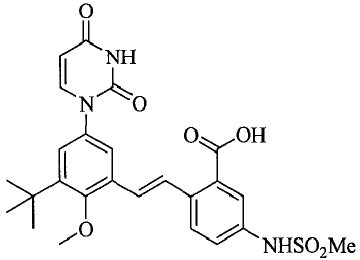

A solution of the product from **Example 124** (55mg, 0.104mmol) in THF (1ml) and 1N aq. NaOH (1ml) was stirred in the dark at room temperature for 1.5h. 1N aqueous HCl was added until pH 3, and the resulting mixture was extracted with EtOAc (2 x 2ml). The combined organic layers were dried over Na₂SO₄, filtered and concentrated to give the title compound (53mg, 99%). ¹H NMR (300 MHz, DMSO-d₆) δ 13.22 (br s, 1 H) 11.40 (d, *J*=2.21 Hz, 1 H) 10.02 (s, 1 H) 7.72 - 7.91 (m, 3 H) 7.68 (d, *J*=2.57 Hz, 1 H) 7.49 (d, *J*=2.57 Hz, 1 H) 7.42 (dd, *J*=8.64, 2.39 Hz, 1 H) 7.21 (d, *J*=2.57 Hz, 1 H) 7.16 (d, *J*=16.18 Hz, 1 H) 5.64 (dd, *J*=7.72, 2.21 Hz, 1 H) 3.79 (s, 3 H) 3.04 (s, 3 H) 1.38 (s, 9 H).

### Example 126. Preparation of (E)-N-(4-(3-tert-butyl-5-(2,4-dioxo-3,4-dihydropyrimidin-1(2H)-yl)-2-methoxystyryl)-3-(morpholine-4-carbonyl)phenyl)methanesulfonamide (compound IB-L1-1.23).

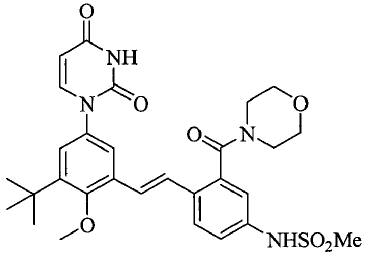

### Part A. Preparation of (E)-2-(3-tert-butyl-5-(2,4-dioxo-3,4-dihydropyrimidin-1(2H)-yl)-2-methoxystyryl)-5-(methylsulfonamido)benzoyl chloride.

A solution of the product from **Example 125** (257mg, 0.50mmol) in thionyl chloride (1.5ml) was heated at 85°C for 40min and then concentrated and dried in vacuo to give the title compound as a solid (0.27 g).

### Part B. Preparation of (E)-N-(4-(3-tert-butyl-5-(2,4-dioxo-3,4-dihydropyrimidin-1(2H)-yl)-2-methoxystyryl)-3-(morpholine-4-carbonyl)phenyl)methanesulfonamide.

To a solution of the product from **Part A** (24mg, 0.045mmol) in anhydrous CH₂Cl₂ (1ml) was added morpholine (0.02ml, 0.226mmol). The mixture was stirred at room temperature for 2h, and then partitioned between 1 N aq. HCl (5ml) and EtOAc (2 x 5ml). The combined organic layers were dried over Na₂SO₄, filtered and concentrated in vacuo. The crude product was purified by column chromatography on silica gel using 4% MeOH in CH₂Cl₂ as the eluent to give the title compound (19mg, 71%). ¹H NMR (300 MHz, DMSO-d₆) δ ppm 11.41 (d, *J*=1.84 Hz, 1 H) 10.04 (s, 1 H) 7.85 (d, *J*=8.46 Hz, 1 H) 7.75 (d, *J*=8.09 Hz, 1 H) 7.52 (d, *J*=2.57 Hz, 1 H) 6.99 - 7.34 (m, 5 H) 5.65 (dd, *J*=7.72, 1.84 Hz, 1 H) 3.76 (s, 3 H) 3.56 - 3.71 (m, 4 H) 3.40 - 3.51 (m, 2 H) 3.11 - 3.22 (m, 2 H) 3.06 (s, 3 H) 1.38 (s, 9 H).

### Example 127. Preparation of (E)-N-(4-(3-tert-butyl-5-(2,4-dioxo-3,4-dihydropyrimidin-1(2H)-yl)-2-methoxystyryl)-3-(hydroxymethyl)phenyl)methanesulfonamide (compound IB-L1-1.10).

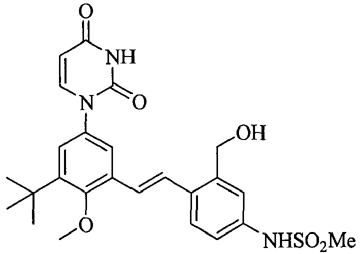

To a solution of the product from **Example 126, Part A** (375mg, 0.705mmol) in anhydrous THF (5ml) at 0°C under N₂ gas was added a 1.0 M solution of lithium *tert*-butoxyaluminiumhydride (1.8ml, 1.8mmol) dropwise. The resulting mixture was stirred at 0°C for 30min, and then allowed to warm to room temperature and was stirred for 1h. The mixture was partitioned between 1 N aq. HCl (10ml) and EtOAc (2 x 10ml). The combined organic layers were dried over Na₂SO₄, filtered and concentrated in vacuo. The crude product was purified by column chromatography on silica gel using 3% MeOH in CH₂Cl₂ as the eluent to give the title compound (220mg, 63%). ¹H NMR (300 MHz, DMSO-d₆) δ ppm 11.41 (s, 1 H) 9.82 (s, 1 H) 7.73 (t, *J*=8.27 Hz, 2 H) 7.66 (d, *J*=2.57 Hz, 1 H) 7.31 - 7.39 (m, 2 H) 7.20 (d, *J*=2.57 Hz, 1 H) 7.12 - 7.19 (m, 2 H) 5.65 (d, *J*=8.09 Hz, 1 H) 5.28 (t, *J*=5.52 Hz, 1 H) 4.65 (d, *J*=5.52 Hz, 2 H) 3.79 (s, 3 H) 3.00 (s, 3 H) 1.38 (s, 9 H).

### Example 128. Preparation of (E)-N-(4-(3-tert-butyl-5-(2,4-dioxo-3,4-dihydropyrimidin-1(2H)-yl)-2-methoxystyryl)-3-(methoxymethyl)phenyl)methanesulfonamide (compound IB-L1-1.13).

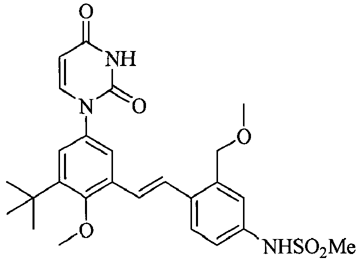

To a solution of the product from **Example 127** (32mg, 0.064mmol) in anhydrous CH₂Cl₂ (1ml) was added thionyl chloride (23µL, 0.32mmol), and the resulting mixture was stirred at room temperature for 30min. The mixture was partitioned between saturated aq. NaHCO₃ (5ml) and CH₂Cl₂ (5ml) and the organic layer was dried over Na₂SO₄, filtered and concentrated. The residue was dissolved in MeOH (1ml), and a solution of 25% NaOMe in MeOH (58µL, 0.254mmol) was added. The resulting mixture was stirred at 50°C for 2h. The mixture was partitioned between 1 N aq. HCl (10ml) and EtOAc (2 x 10ml). The combined organic layers were dried over Na₂SO₄, filtered and concentrated in vacuo. The crude product was purified by column chromatography on silica gel using 3% MeOH in CH₂Cl₂ as the eluent to give the title compound (15mg, 46%). ¹H NMR (300 MHz, DMSO-d₆) δ 11.43 (s, 1 H) 9.86 (s, 1 H) 7.62 - 7.87 (m, 3 H) 7.12 - 7.39 (m, 5 H) 5.66 (d, *J*=7.72 Hz, 1 H) 4.58 (s, 2 H) 3.78 (s, 3 H) 3.35 (s, 3 H) 3.00 (s, 3 H) 1.38 (s, 9 H).

### Example 129. Preparation of (E)-N-(4-(3-tert-butyl-5-(2,4-dioxo-3,4-dihydropyrimidin-1(2H)-yl)-2-methoxystyryl)-3-((isopentylamino)methyl)phenyl)methanesulfonamide (compound IB-L1-1.31).

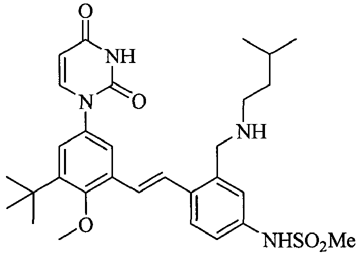

### Part A. Preparation of (E)-N-(4-(3-tert-butyl-5-(2,4-dioxo-3,4-dihydropyrimidin-1(2H)-yl)-2-methoxystyryl)-3-formylphenyl)methanesulfonamide.

To a solution of the product from **Example 127** (0.60 g, 1.20mmol) in anhydrous DMA (15ml) was added 2-iodoxybenzoic acid (336mg, 1.20mmol). The mixture was stirred at room temperature for 1h, and then partitioned between EtOAc (20ml) and H₂O (2x 20ml). The organic layer was dried over Na₂SO₄, filtered and concentrated in vacuo. The crude product was purified by column chromatography on silica gel using 2% MeOH in CH₂Cl₂ as the eluent to give the title compound as a colorless solid (395mg, 66%). ¹H NMR (300 MHz, DMSO-d₆) δ ppm 11.43 (d, *J*=2.21 Hz, 1 H) 10.45 (s, 1 H) 10.15 (s, 1 H) 8.06 (d, *J*=16.18 Hz, 1 H) 7.97 (d, *J*=8.82 Hz, 1 H) 7.73 - 7.78 (m, 2 H) 7.69 (d, *J*=2.57 Hz, 1 H) 7.51 (dd, *J*=8.64, 2.39 Hz, 1 H) 7.30 (d, *J*=16.18 Hz, 1 H) 7.26 (d, *J*=2.57 Hz, 1 H) 5.66 (dd, *J*=7.72, 2.21 Hz, 1 H) 3.81 (s, 3 H) 3.07 (s, 3 H) 1.39 (s, 9 H).

### Part B. Preparation of (E)-N-(4-(3-tert-butyl-5-(2,4-dioxo-3,4-dihydropyrimidin-1(2H)-yl)-2-methoxystyryl)-3-((isopentylamino)methyl)phenyl)methanesulfonamide.

To a solution of the product from **Part A** (50mg, 0.10mmol) and 3-methylbutan-1-amine (12µL, 0.10mmol) in anhydrous THF (3ml) was added sodium triacetoxyborohydride (32mg, 0.15mmol) and AcOH (9µL, 0.15mmol). The resulting mixture was stirred at room temperature for 4 h, and then partitioned between H₂O (10ml) and EtOAc (2 x 10ml). The combined organic layers were dried over Na₂SO₄, filtered and concentrated in vacuo. The crude product was purified by column chromatography on silica gel using 3% MeOH in CH₂Cl₂ as the eluent to give the title compound (37mg, 65%). ¹H NMR (300 MHz, DMSO-d₆) δ 11.45 (d, *J*=1.84 Hz, 1 H) 10.04 (s, 1 H) 8.80 - 8.87 (m, 1 H) 7.88 (d, *J*=8.46 Hz, 1 H) 7.71 - 7.77 (m, 2 H) 7.41 - 7.48 (m, 1 H) 7.37 (d, *J*=2.21 Hz, 1 H) 7.21 - 7.29 (m, 3 H) 5.67 (dd, *J*=7.91, 2.02 Hz, 1 H) 4.30 - 4.38 (m, 2 H) 3.80 (s, 3 H) 3.10 (s, 3 H) 2.95 - 3.04 (m, 2 H) 1.49 - 1.67 (m, 3 H) 1.38 (s, 9 H) 0.86 (d, *J*=6.25 Hz, 6 H).

### Example 130. Preparation of N-(4-(3-tert-butyl-5-(2,4-dioxo-3,4-dihydropyrimidin-1(2H)-yl)-2-methoxystyryl)-3-((E)-(methoxyimino)methyl)phenyl)methanesulfonamide (compound IB-L1-1.19).

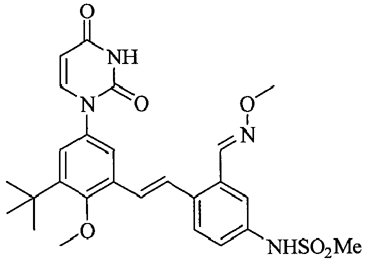

To a solution of the product from **Example 129, Part A** (35mg, 0.070mmol) in EtOH (2ml) was added *O*-methoxylamine hydrochloride (29mg, 0.35mmol) and sodium bicarbonate (30mg, 0.35mmol). The resulting mixture was stirred at 70°C for 2h. To the mixture was added 1 N aq. HCl (1ml) to give a colorless precipitate that was filtered and dried to give the title compound as a colorless solid (24mg, 64%). ¹H NMR (300 MHz, DMSO-d₆) δ ppm 11.43 (d, *J*=2.21 Hz, 1 H) 9.94 (s, 1 H) 8.74 (s, 1 H) 7.79 - 7.85 (m, 2 H) 7.76 (d, *J*=7.72 Hz, 1 H) 7.57 - 7.65 (m, 2 H) 7.32 (dd, *J*=8.64, 2.39 Hz, 1 H) 7.23 (d, J=2.57 Hz, 1 H) 7.18 (d, *J*=16.18 Hz, 1 H) 5.66 (dd, *J*=7.72, 2.21 Hz, 1 H) 3.93 (s, 3 H) 3.79 (s, 3 H) 3.03 (s, 3 H) 1.38 (s, 9 H).

### Example 131. Preparation of (E)-N-(4-(3-tert-butyl-5-(2,4-dioxo-3,4-dihydropyrimidin-1(2H)-yl)-2-methoxystyryl)-3-(oxazol-2-yl)phenyl)methanesulfonamide (compound IB-L1-1.26).

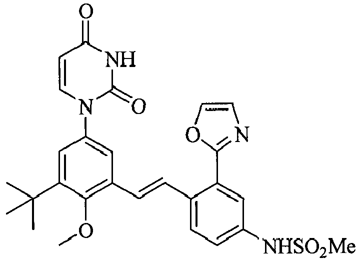

To a solution of the product from **Example 126, Part A** (80mg, 0.15mmol) in tetramethylene sulfone (1.5ml) was added 1*H*-1,2,3-triazole (10µL, 0.17mmol) and potassium carbonate (73mg, 0.53mmol). The mixture was heated for 35min at 130°C in a microwave reactor. After cooling to room temperature, the mixture was partitioned between 1 N aqueous HCl (10ml) and EtOAc (2 x 10ml). The combined organic layers were dried over Na₂SO₄, filtered and concentrated in vacuo. The crude product was purified by column chromatography on silica gel using 3% MeOH in CH₂Cl₂ as the eluent to give the title compound (37mg, 46%). ¹H NMR (300 MHz, DMSO-d₆) δ 11.41 (d, *J*=1.84 Hz, 1 H) 10.10 (s, 1 H) 8.29 (d, *J*=1.10 Hz, 1 H) 8.05 (d, *J*=16.18 Hz, 1 H) 7.95 (d, *J*=8.82 Hz, 1 H) 7.82 (d, *J*=2.21 Hz, 1 H) 7.74 (d, *J*=8.09 Hz, 1 H) 7.51 (d, *J*=2.57 Hz, 1 H) 7.46 (d, *J*=0.74 Hz, 1 H) 7.39 (dd, *J*=8.64, 2.39 Hz, 1 H) 7.20 - 7.30 (m, 2 H) 5.65 (dd, *J*=7.91, 2.02 Hz, 1 H) 3.80 (s, 3 H) 3.07 (s, 3 H) 1.38 (s, 9 H).

### Example 132. Preparation of (E)-N-(4-(3-tert-butyl-5-(2,4-dioxo-3,4-dihydropyrimidin-1(2H)-yl)-2-methoxystyryl)-3-(1H-imidazol-2-yl)phenyl)methanesulfonamide (compound IB-L1-1.16).

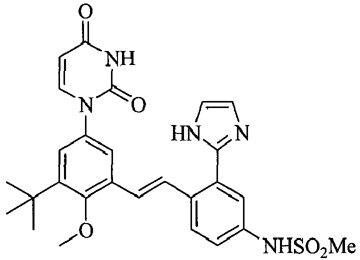

To a solution of the product from **Example 129, Part A** (50mg, 0.10mmol) in EtOH (2ml) was added glyoxal (57uL, 0.50mmol) and concentrated aqueous NH₄OH (70uL, 0.50mmol). The resulting mixture was stirred at room temperature for 16h. To the mixture was added 1 N aq. HCl until pH = 7, and the mixture was partitioned between H₂O (10ml) and EtOAc (2 x 10ml). The combined organic layers were dried over Na₂SO₄, filtered and concentrated in vacuo. The crude product was purified by column chromatography on silica gel using 5% MeOH in CH₂Cl₂ as the eluent to give the title compound (27mg, 50%). ¹H NMR (300 MHz, DMSO-d₆) δ 12.39 (s, 1 H) 11.40 (d, *J*=1.84 Hz, 1 H) 9.98 (s, 1 H) 7.89 (d, *J*=8.82 Hz, 1 H) 7.66 - 7.76 (m, 2 H) 7.38 (t, *J*=2.21 Hz, 2 H) 7.23 - 7.31 (m, 2 H) 7.06 - 7.21 (m, 3 H) 5.63 (dd, *J*=8.09, 1.84 Hz, 1 H) 3.78 (s, 3 H) 3.07 (s, 3 H) 1.37 (s, 9 H).

### Example 133. Preparation of (E)-tert-butyl 2-(3-tert-butyl-5-(2,4-dioxo-3,4-dihydropyrimidin-1(2H)-yl)-2-methoxystyryl)-5-(methylsulfonamido)phenylcarbamate (compound IB-L1-1.32).

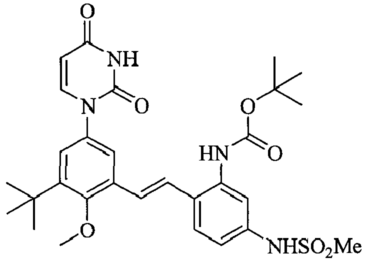

To a solution of the product from **Example 125** (75mg, 0.146mmol) in *tert*-butanol (4ml) was added diphenylphosphoryl azide (47µL 0.219mmol) and triethylamine (31µL, 0.219mmol). The resulting mixture was stirred at 80°C for 18h. The cooled mixture was partitioned between H₂O (10ml) and EtOAc (2 x 10ml). The combined organic layers were dried over Na₂SO₄, filtered and concentrated in vacuo. The crude product was purified by column chromatography on silica gel using 3% MeOH in CH₂Cl₂ as the eluent to give the title compound (16mg, 19%). ¹H NMR (300 MHz, DMSO-d₆) δ 11.45 (d, *J*=1.84 Hz, 1 H) 9.86 (s, 1 H) 9.03 (s, 1 H) 7.75 (d, *J*=7.72 Hz, 2 H) 7.55 (d, *J*=2.57 Hz, 1 H) 7.10 - 7.33 (m, 4 H) 7.04 (dd, *J*=8.64, 2.39 Hz, 1 H) 5.66 (dd, *J*=7.91, 2.02 Hz, 1 H) 3.78 (s, 3 H) 3.02 (s, 3 H) 1.45 (s, 9 H) 1.38(s,9H).

### Example 134. Preparation of (E)-N-(3-amino-4-(3-tert-butyl-5-(2,4-dioxo-3,4-dihydropyrimidin-1(2H)-yl)-2-methoxystyryl)phenyl)methanesulfonamide (compound IB-L1-1.28).

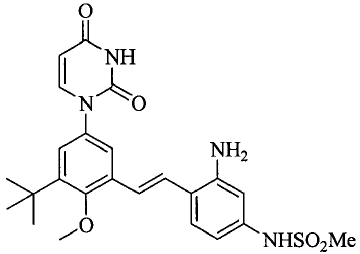

The procedure described for the preparation of **Example 133** provided the title compound, which was purified by column chromatography on silica gel using 5% methanol in CH₂Cl₂ as the eluent (6mg, 9%). ¹H NMR (300 MHz, DMSO-d₆) δ 11.44 (d, *J*=2.21 Hz, 1 H) 9.55 (s, 1 H) 7.77 (d, *J*=2.57 Hz, 1 H) 7.75 (d, *J*=8.09 Hz, 1 H) 7.45 (d, *J*=8.46 Hz, 1 H) 7.33 (d, *J*=15.81 Hz, 1 H) 7.15 (d, *J*=2.57 Hz, 1 H) 7.00 (d, *J*=16.18 Hz, 1 H) 6.56 (d, *J*=2.21 Hz, 1 H) 6.44 (dd, *J*=8.46, 2.21 Hz, 1 H) 5.66 (dd, *J*=7.91, 2.02 Hz, 1 H) 5.56 (s, 2 H) 3.78 (s, 3 H) 2.97 (s, 3 H) 1.37 (s, 9 H).

### Example 135. Preparation of (E)-N-(4-(3-tert-butyl-5-(2,4-dioxo-3,4-dihydropyrimidin-1(2H)-yl)-2-methoxystyryl)-2-fluorophenyl)methanesulfonamide (compound IB-L1-1.5).

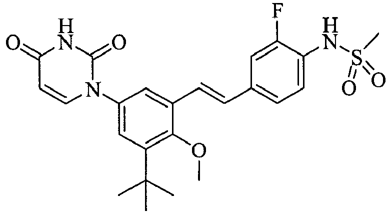

### Part A. Preparation of (3-fluoro-4-nitrophenyl)methanol.

To a solution of 3-fluoro-4-nitrobenzoic acid (2.0 g, 10.8mmol) in THF (50ml) at 0°C was added BH₃·Me₂S complex (2.215ml, 22.15mmol) drop-wise. The mixture was stirred at 0°C for 3h, and was then stirred at 65°C for 18h. To the cooled mixture was added ice (50g), followed by 1 N aq. HCl (100ml), and the resulting mixture was extracted with EtOAc (200ml). The organic layer was dried over Na₂SO₄, filtered and concentrated in vacuo to provide the title compound as a white solid (1.79g, 97%).

### Part B. Preparation of 4-(bromomethyl)-2-fluoro-1-nitrobenzene.

A solution of the product from **Part A** (1.79 g, 10.46mmol), N-bromosuccinimide (2.234 g, 12.55mmol) and triphenylphosphine (3.29 g, 12.55mmol) in CH₂Cl₂ (100ml) and THF (50ml) was stirred at room temperature for 3h. The mixture was partitioned between H₂O (200ml) and EtOAc (400ml), and the organic layer was dried over Na₂SO₄, filtered and concentrated in vacuo. The crude product was purified by column chromatography on silica gel using 1:1 EtOAc:hexanes as the eluent to give the title compound (1.14 g, 47%).

### Part C. Preparation of diethyl 3-fluoro-4-nitrobenzylphosphonate.

The product from **Part B** (1.25 g, 5.34mmol) was subjected to the conditions described for **Example 34, Part B** to provide the title product (0.75 g, 48%).

### Part D. Preparation of (E)-N-(4-(3-tert-butyl-5-(2,4-dioxo-3,4-dihydropyrimidin-1(2H)-yl)-2-methoxystyryl)-2-fluorophenyl)methanesulfonamide.

The product from **Part C** (0.193 g, 0.662mmol) was subjected to the conditions described for **Example 41A, Part E, Part F, and Part G** to provide the title product as a colorless solid (15mg, 5%). ¹H NMR (300 MHz, DMSO-d₆) δ 11.43(s,1H), 9.67(s,1H), 7.76(d,J=8.1Hz,1H), 7.62(m,2H), 7.41(m,2H), 7.38(m,1H), 7.23(m,2H), 5.66(dd,J=8.0,2.0Hz,1H), 3.80(s,3H), 3.05(s,3H), 1.38(s,9H).

### Example 136. Preparation of (E)-N-(4-(3-tert-butyl-5-(2,4-dioxo-3,4-dihydropyrimidin-1(2H)-yl)-2-methoxystyryl)-2-fluoro-5-methylphenyl)methanesulfonamide (compound IB-L1-1.15).

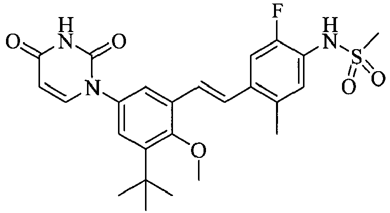

### Part A. Preparation of N-(4-bromo-2-fluoro-5-methylphenyl)methanesulfonamide.

To a solution of 4-bromo-2-fluoro-5-methylaniline (2.04 g, 10.0mmol) in anhydrous CH₂Cl₂ (20ml) and pyridine (3.23ml, 40.0mmol) was added methanesulfonyl chloride (0.86ml, 11.0mmol) and the resulting mixture was stirred at room temperature for 2h. Solvent was removed in vacuo, and the residue was partitioned between EtOAc and 1M aq. HCl. The organic layer was washed with saturated aqueous NaHCO₃, brine and then dried over Na₂SO₄. The drying agent was filtered off, and the filtrate was concentrated to give the title compound as a solid (2.80 g, 99%).

### Part B. Preparation of N-(4-ethynyl-2-fluoro-5-methylphenyl)methanesulfonamide.

A mixture of the product from **Part A** (3.0 g, 10.63mmol), triphenylphosphine (0.279 g, 1.06mmol), trimethylsilylacetate (6.0ml, 42.5mmol) and palladium(II) acetate (0.12 g, 0.53mmol) in triethylamine (30ml) and toluene (15ml) under N₂ was heated at 80°C for 5h. The mixture was allowed to cool to room temperature, and was partitioned between EtOAc and 1M aq. HCl. The organic layer was washed with saturated NaHCO₃ and brine, dried over Na₂SO₄, filtered and concentrated in vacuo. The crude product was purified by column chromatography on silica gel using a solvent gradient of 10% to 35% EtOAc in hexanes to give an oil (3.0 g, 94%). To a solution of the oil (3.0 g, 10.0mmol) in MeOH (50ml) was added 1M aq. NaOH (21ml, 21.0mmol), and the resulting mixture was stirred at room temperature for 45min. The mixture was partitioned between EtOAc and 1M aq. HCl, and the organic layer was washed with brine and dried over Na₂SO₄. The drying agent was filtered off, and the filtrate was concentrated in vacuo to give the title compound as a solid (2.3 g, quant.).

### Part C. Preparation of (E)-5-fluoro-2-methyl-4-(methylsulfonamido)styrylboronic acid.

The product from **Part B** (0.20 g, 0.88mmol) was subjected to the conditions described for the preparation of **Example 41B, Part B** to give the title compound (42mg, 17%).

### Part D. Preparation of (E)-N-(4-(3-tert-butyl-5-(2,4-dioxo-3,4-dihydropyrimidin-1(2H)-yl)-2-methoxystyryl)-2-fluoro-5-methylphenyl)methanesulfonamide.

The product from **Part C** (40mg, 0.15mmol) was subjected to the conditions described for the preparation of **Example 41B, Part I to** give the title compound (51mg, 83%). ¹H NMR (300 MHz, DMSO-d₆) δ 11.42 (d, *J*=2.21 Hz, 1 H) 9.59 (s, 1 H) 7.70 - 7.78 (m, 2 H) 7.66 (d, *J*=11.77 Hz, 1 H) 7.20 - 7.32 (m, 3 H) 5.65 (dd, *J*=7.72, 2.21 Hz, 1 H) 3.79 (s, 3 H) 3.05 (s, 3 H) 2.38 (s, 3 H) 1.38 (s, 9 H).

### Example 137. Preparation of methyl 2-(3-tert-butyl-5-(2,4-dioxo-3,4-dihydropyrimidin-1(2H)-yl)-2-methoxyphenethyl)-5-(methylsulfonamido)benzoate (compound IB-L5-2-1.1).

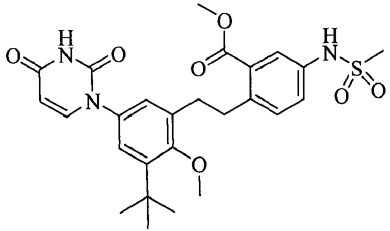

To a solution of the product from **Example 124**(40mg, 0.076mmol) in MeOH (2ml) and THF (2ml) was added 10% Pd/C (20mg) and the resulting mixture was stirred at room temperature under 1 atm H₂ for 16h. The mixture was filtered through celite and concentrated in vacuo to give a solid (27.5mg, 68%). ¹H NMR (300 MHz, DMSO-d₆) 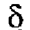 11.39 (s, 1 H) 9.88 (s, 1 H) 7.61 - 7.71 (m, 2 H) 7.28 - 7.36 (m, 2 H) 7.20 (d, *J*=2.57 Hz, 1 H) 7.13 (d, *J*=2.94 Hz, 1 H) 5.64 (d, *J*=7.72 Hz, 1 H) 3.83 (s, 3 H) 3.75 (s, 3 H) 3.14 (dd, *J*=10.30, 5.88 Hz, 2 H) 2.96 (s, 3 H) 2.83 - 2.92 (m, 2 H) 1.34 (s, 9 H).

### Example 138. Preparation of N-(4-(3-tert-butyl-5-(2,4-dioxo-3,4-dihydropyrimidin-1(2H)-yl)-2-methoxyphenethyl)phenyl)methanesulfonamide (compound IB-L5-2-1.2).

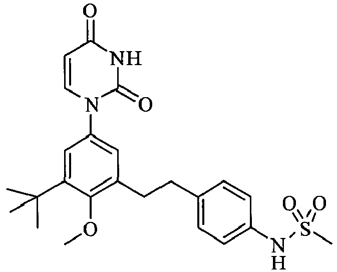

The product from **Example 41B, Part M** (200mg, 0.426mmol) was dissolved in MeOH (10ml) followed by the addition of 10% Palladium on activated Carbon (50mg). The resultant mixture was evacuated and a hydrogen balloon attached then stirred at room temperature for 48h. The mixture was then filtered through celite and the filtrate concentrated under vacuum to an oil which was dissolved in ethanol (4ml) then a 1N solution of aqueous sodium hydroxide (3.8ml, 3.8mmol) was added and the solution stirred at room temperature for 18h. The ethanol was then removed under vacuum and a 1N solution of aqueous hydrochloric acid (4ml) was added to acidify the mixture followed by extraction with EtOAc (2 x 10mL). The organic extracts were combined, dried and purified by column chromatography on silica gel using 5% MeOH in CH₂Cl₂ as the eluent to provide the title compound as a colorless solid (82mg, 41%). ¹H NMR (300MHz, DMSO-d₆) 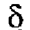 11.39 (s, 1H), 9.60 (s, 1H), 7.65 (d, *J*=8.1Hz, 1H), 7.23 (m, 3H), 7.17 (m, 3H), 5.64 (d, *J*=7.7Hz, 1H), 3.77 (s, 3H), 2.93 (s, 3H), 2.88 (br s, 4H), 1.35 (s, 9H).

### Example 139. Preparation of (E)-N-(4-(3-tert-butyl-5-(2,4-dioxo-3,4-dihydropyrimidin-1(2H)-yl)-2-ethoxystyryl)phenyl)methanesulfonamide (compound IB-L1-1.30).

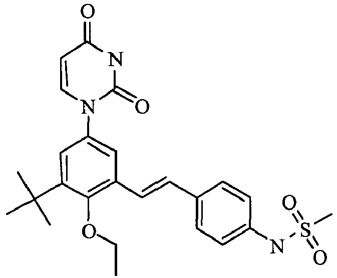

### Part A. Preparation of 2-tert-butyl-4-iodophenol.

To a 250mL round-bottom flask was added 2-tert-butylphenol (3.76g, 25mmol) in MeOH (50.0ml) to give a colorless solution. Sodium hydroxide (1.200g, 30.0mmol) was added and the mix was stirred until the hydroxide was completely dissolved. The solution was cooled to 0°C and treated with sodium iodide (1.75g, 11.6mmol) followed by drop-wise addition of 10% sodium hypochlorite solution (7.2ml, 11.6mmol). The addition of sodium iodide followed by sodium hypochlorite was repeated twice and the mixture was stirred at 0°C for 30min. The mixture was treated with 10% w/w solution of sodium thiosulfate, stirred for 30min and treated with concentrated HCl dropwise to a constant pH of 1. The mixture was extracted 3X with EtOAc. The extracts were combined, washed with brine, dried (MgS04), filtered and concentrated. The crude oil was flash chromatographed on an Isco 80g silica cartridge eluting with hexane to > 4:1 hexane/EtOAc to give a yellow oil (5.2g, 75%).

### Part B. Preparation of 2-bromo-6-tert-butyl-4-iodophenol.

To a 250mL round-bottom flask was added the product from **Part A** (4.8g, 17.38mmol) and 1,3-dibromo-5,5-dimethylhydantoin (2.61g, 9.13mmol) in chloroform (87ml) to give an orange solution. The reaction mixture was stirred for 2h resulting in a black solution that was washed with water, brine, dried (Na₂SO₄) and concentrated. The black oil was flash chromatographed on a 120g Isco silica cartridge eluting with hexane to give a pinkish solid (4.84g, 78%).

### Part C. Preparation of 1-bromo-3-tert-butyl-2-ethoxy-5-iodobenzene.

To a 50mL round-bottom flask was added the product from **Part B** (888mg, 2.5mmol), ethyl iodide (409mg, 2.63mmol), and potassium carbonate (415mg, 3.00mmol) in acetone (12ml) to give a green suspension. The mixture was heated at reflux for 16h, cooled and concentrated. The residue was partitioned between water and EtOAc. The organic layer was washed twice with brine, dried over Na₂SO₄, filtered and concentrated to a red oil. The oil was flash chromatographed on an Isco 40g silica cartridge eluting with hexane to give a clear oil (820mg, 86%).

### Part D. Preparation of 1-(3-bromo-5-tert-butyl-4-ethoxyphenyl)pyrimidine-2,4(1H,3H)-dione.

In a 20mL microwave tube under nitrogen flush was added the product from **Part C** (0.4g, 1.044mmol), 1H-Pyrimidine-2,4-dione (0.140g, 1.253mmol), and potassium phosphate tribasic (0.465g, 2.193mmol) in DMSO (5ml) to give a colorless suspension. N-(2-cyanophenyl)picolinamide (0.047g, 0.209mmol) was added and the mix was sparged with nitrogen for 10min. Copper(I) iodide (0.020g, 0.104mmol) was added and the mix was sparged once again for 10min, placed under nitrogen and heated at 60°C for 18h. The mixture was cooled and partitioned between EtOAc and water adjusting the pH to 1 with HCl. The aqueous layer was extracted 2X with EtOAc. The organics were combined, washed with water, saturated NaHCO₃, and saturated NaCl. The organic layer was dried (Na₂SO₄), stirred with 3-mercaptopropyl functionalized silica for 1h, filtered and concentrated. The crude product was purified by chromatography on an Isco 12g silica cartridge eluting with 2% MeOH in CH₂Cl₂ to give a white powder (266mg, 69%).

### Part E. Preparation of (E)-N-(4-(3-tert-butyl-5-(2,4-dioxo-3,4-dihydropyrimidin-1(2H)-yl)-2-ethoxystyryl)phenyl)methanesulfonamide.

A mixture of the product from **Part D** (55.1mg, 0.15mmol), the product from **Example 41B, Part B** (36.2mg, 0.150mmol), potassium phosphate tribasic (63.7mg, 0.300mmol) and 1,1'-bis(di-tert-butylphosphino)ferrocene palladium dichloride (4.89mg, 7.50µmol) in THF (3ml) water (1ml) was sparged for 10min with nitrogen, and then sealed and heated at 50 °C for 4h. The mixture was cooled to room temperature and diluted into EtOAc. The EtOAc layer was washed with 1M HCl, saturated NaHCO₃, saturated NaCl, dried (Na₂SO₄) and treated simultaneously with mercaptopropyl silica gel, filtered and concentrated. The crude product was purified by column chromatography on silica gel using 2% MeOH in CH₂Cl₂ as the eluent to give the title compound as a solid (40mg, 55%) m.p. 265-266 °C. ¹H NMR (300 MHz, DMSO-d₆) δ 11.42 (s, 1 H) 9.87 (s, 1 H) 7.76 (d, J=8.09 Hz, 1 H) 7.55 - 7.66 (m, 3 H) 7.17 - 7.27 (m, 5 H) 5.65 (dd, J=7.72, 1.47 Hz, 1 H) 3.89 (q, J=6.74 Hz, 2 H) 3.02 (s, 3 H) 1.45 (t, J=6.99 Hz, 3 H) 1.39 (s, 9 H).

### Example 140. Preparation of N-(4-((3-cyclopropyl-5-(2,4-dioxotetrahydropyrimidin-1(2H)-yl)-2-methoxyphenyl)ethynyl)phenyl)methanesulfonamide (compound IA-L2-1.23).

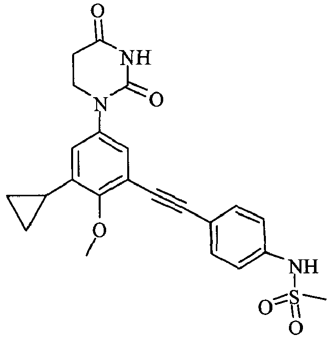

### Part A. Preparation of 3-bromo-2-methoxy-5-nitrobenzaldehyde.

Into an appropriately sized pressure bottle was placed commercially available 3-bromo-2-hydroxy-5-nitrobenzaldehyde (2.46gm, 10.00mmol), iodomethane (6.23mL, 100mmol), silver oxide (2.317g, 10.00mmol) and acetonitrile (50mL). The bottle was sealed and heated with stirring at 50°C for 2h. The reaction mixture was filtered through celite and the filtrate concentrated to give a solid residue that was triturated with hexane, collected by filtration and dried. The resulting solid was purified by silica gel chromatography (hexane-ethylacetate) to give the title compound (1.2g, 46%).

### Part B. Preparation of 3-cyclopropyl-2-methoxy-5-nitrobenzaldehyde.

The product from **Part A** (0.65gm, 2.50mmol) was combined with cyclopropyl boronic acid (0.279g, 3.25mmol), potassium phosphate (1.85g, 8.75mmol), tricyclohexylphosphine teterafluoroborate (0.092gm, 0.250mmol), palladium (II) acetate (0.028gm, 0.125mmol) and toluene-water, 20:1 v/v (12mL) in a microwave tube. The reaction mixture was purged with nitrogen for 5min then heated at 100°C in a microwave reactor for 20min. The mixture was subsequently partitioned with ethyl acetate and water. The organic phase was washed with brine and concentrated in vacuo to give a residue that was purified by chromatography on silica gel (hexane-ethylacetate) to afford the title compound as a white solid (0.47g, 85%).

### Part C. Preparation of 1-cyclopropyl-3-ethynyl-2-methoxy-5-nitrobenzene.

The product from **Part B** (0.20g, 0.904mmol) was reacted with 1-diazo-2-oxopropyl phosphonate (0.182g, 0.949mmol), prepared by the method of Ohira, Syn. Comm. 19 (3&4) 561-564 (1989), as described in **Example 44 Part G.** The crude product was purified by chromatography on silica gel (ethylacetate-hexanes) to give the title compound as a pale yellow oil which crystallizes in standing (0.155 g, 79%).

### Part D. Preparation of N-(4-((3-cyclopropyl-2-methoxy-5-nitrophenyl)ethynyl)phenyl) methanesulfonamide.

The product from **Part C** (0.155g, 0.712mmol) was combined in a round bottom flask with *N-*(4-iodophenyl)methanesulfonamide (0.212gm, 0.712mmol), prepared as in **Example 43, Part F** substituting 4-iodoaniline for 4-iodo-3-methylaniline. To this mixture was added copper (I) iodide (10.31mg, 0.054mmol), bis(triphenylphosphine)palladium(II)chloride (0.034g, 0.048mmol), triethylamine (0.496mL, 3.56mmol) and anhydrous acetonitrile (14mL). A reflux condenser was attached and the mixture was purged with nitrogen for 5min and then heated under nitrogen in an oil bath at 80°C for 1h. The reaction mixture was concentrated in vacuo and the crude product was purified by chromatography on silica gel column eluting with ethylacetate-hexanes to give the title compound as a yellow solid (0.1887g, 68.6%).

### Part E. Preparation of N-(4-((5-amino-3-cyclopropyl-2-methoxyphenyl)ethynyl)phenyl) methanesulfonamide.

The product from **Part D** (0.184gm, 0.475mmol) was combined with iron (0.133gm, 2.38mmol) and ammonium chloride (0.050gm, 0.932mmol) in a round bottom flask. To this was added ethanol (1mL), tetrahydrofuran (THF) (2mL) and water (2mL), and the resulting mixture heated with vigorous stirring under nitrogen to 80°C in an oil bath a total of 4.5h. On completion of heating the reaction mixture was filtered through a pad of sand and celite. The filter pad was washed with THF and the combined filtrates concentrated in vacuo. The residue was partitioned between water and dichloromethane. The organics were washed with water and then dried (MgSO4) and concentrated. The residue was purified by chromatography on silica gel eluting with ethylacetate-hexanes to give the title compound as an amber oil (0.1098g, 65%).

### Part F. Preparation of N-(4-((3-cyclopropyl-5-(2,4-dioxotetrahydropyrimidin-1(2H)-yl)-2-methoxyphenyl)ethynyl)phenyl)methanesulfonamide.

The product from **Part E** (0.1098g, 0.308mmol) was reacted with acrylic acid (1.27mL, 18.48mmol) as described in **Example 43 Part E**. The crude product in acrylic acid was treated with urea (0.094g, 1.558mmol) and glacial acetic acid (1.3mL) then heated under nitrogen to 120°C in an oil bath for eleven hours. The reaction mixture was concentrated in vacuo and diluted with water. The resulting solid was collected by vacuum filtration, washed with water and dried in vacuo to give the title compound as a yellow solid (0.0877g, 63%). Purification by chromatography on silica gel using ethylacetate-hexane then dichloromethane-methanol gave analytical material. ¹H NMR (300 MHz, DMSO-d₆) δ 10.36 (s, 1 H) 10.08 (s, 1 H) 7.53 (d, *J*=8.82 Hz, 2 H) 7.26 (dd, *J*=5.52, 2.94 Hz, 3 H) 6.84 (d, *J*=2.57 Hz, 1 H) 3.94 (s, 3 H) 3.73 (t, *J*=6.62 Hz, 2 H) 3.06 (s, 3 H) 2.68 (t, *J*=6.62 Hz, 2 H) 2.16 (s, 1 H) 0.94 - 1.03 (m, 2 H) 0.63-0.74 (m, 2 H).

The following compounds were prepared utilizing the above discussion:

(E)-N'-(1-(3'-tert-butyl-5'-(2,4-dioxotetrahydropyrimidin-1(2H)-yl)-2'-methoxybiphenyl-4-yl) ethylidene)methanesulfonohydrazide (compound **IA-L0-1.2**). ¹H NMR (300 MHz, DMSO-d₆) δ ppm 1.39 (s, 9 H) 2.26 (s, 3 H) 2.70 (t, J=6.62 Hz, 2 H) 3.10 (s, 3 H) 3.24 (s, 3 H) 3.80 (t, J=6.62 Hz, 2 H) 7.17 (d, J=2.57 Hz, 1 H) 7.24 (d, J=2.57 Hz, 1 H) 7.57 (d, J=8.46 Hz, 2 H) 7.87 (d, J=8.46 Hz, 2 H) 10.12 (s, 1 H) 10.33 (s, 1 H).

(E)-N'-((3'-tert-butyl-5'-(2,4-dioxotetrahydropyrimidin-1(2H)-yl)-3,5-difluoro-2'-methoxy biphenyl-4-yl)methylene)methanesulfonohydrazide (compound **IA-L0-1.3**). ¹H NMR (300 MHz, DMSO-d₆) δ ppm 1.39 (s, 9 H) 2.70 (t, J=6.62 Hz, 2 H) 3.08 (s, 3 H) 3.30 (s, 3 H) 3.80 (t, J=6.43 Hz, 2 H) 7.26 (d, J=2.57 Hz, 1 H) 7.34 (m, 3 H) 8.11 (s, 1 H) 10.36 (s, 1 H) 11.39 (s, 1 H).

(E)-N'-((3'-tert-butyl-5'-(2,4-dioxotetrahydropyrimidin-1(2H)-yl)-3-fluoro-2'-methoxy biphenyl-4-yl)methylene)methanesulfonohydrazide (compound **IA-L0-1.4**). ¹H NMR (300 MHz, DMSO-d₆) δ ppm 1.39 (s, 9 H) 2.70 (t, J=6.62 Hz, 2 H) 3.11 (s, 3 H) 3.28 (s, 3 H) 3.80 (t, J=6.80 Hz, 2 H) 7.22 (d, J=2.57 Hz, 1 H) 7.28 (d, J=2.57 Hz, 1 H) 7.45 (m, 2 H) 7.94 (t, J=8.09 Hz, 1 H) 8.20 (s, 1 H) 10.35 (s, 1 H) 11.32 (s, 1 H).

(E)-N'-((3'-tert-butyl-5'-(2,4-dioxotetrahydropyrimidin-1(2H)-yl)-2',4-dimethoxy-biphenyl-3-yl)methylene)methanesulfonohydrazide (compound **IA-L0-1.5**). ¹H NMR (300 MHz, DMSO-d₆) δ ppm 1.39 (s, 9 H) 2.70 (t, J=6.62 Hz, 2 H) 3.03 (s, 3 H) 3.24 (s, 3 H) 3.78 (t, J=6.80 Hz, 2 H) 3.90 (s, 3 H) 7.12 (d, J=2.57 Hz, 1 H) 7.22 (m, 2 H) 7.54 (dd, J=8.46, 2.21 Hz, 1 H) 7.86 (d, J=2.21 Hz, 1 H) 8.34 (s, 1 H) 10.32 (s, 1 H) 11.05 (s, 1 H).

(E)-N'-((3'-tert-butyl-5'-(2,4-dioxotetrahydropyrimidin-1(2H)-yl)-2,3-difluoro-2'-methoxy biphenyl-4-yl)methylene)methanesulfonohydrazide (compound **IA-L0-1.6**). ¹H NMR (500 MHz, DMSO-d₆) δ ppm 1.38 (s, 9 H) 2.70 (t, J=6.41 Hz, 2 H) 3.11 (s, 3 H) 3.28 (s, 3 H) 3.79 (t, J=6.71 Hz, 2 H) 7.18 (d, J=2.44 Hz, 1 H) 7.35 (m, 2 H) 7.74 (t, J=7.32 Hz, 1 H) 8.19 (s, 1 H) 10.32 (s, 1 H) 11.44 (s, 1 H).

(E)-N'-((3'-tert-butyl-5'-(2,4-dioxotetrahydropyrimidin-1(2H)-yl)-2'-methoxybiphenyl-3-yl) methylene)methanesulfonohydrazide (compound **IA-L0-1.7**). ¹H NMR (300 MHz, DMSO-D6) δ ppm 1.39 (s, 9 H) 2.70 (t, J=6.62 Hz, 2 H) 3.07 (s, 3 H) 3.23 (s, 3 H) 3.80 (t, J=6.43 Hz, 2 H) 7.17 (d, J=2.57 Hz, 1 H) 7.25 (d, J=2.57 Hz, 1 H) 7.54 (m, 2 H) 7.73 (m, 2 H) 8.06 (s, 1 H) 10.33 (s, 1 H) 11.11 (s, 1 H).

(Z)-N'-(1-(3'-tert-butyl-5'-(2,4-dioxotetrahydropyrimidin-1(2H)-yl)-2'-methoxybiphenyl-3-yl) ethylidene)methanesulfonohydrazide (compound **IA-L0-1.8**). ¹H NMR (300 MHz, DMSO-d₆) δ ppm 1.39 (s, 9 H) 2.26 (s, 3 H) 2.70 (t, *J*=6.80 Hz, 2 H) 3.07 (s, 3 H) 3.22 (s, 3 H) 3.80 (t, *J*=6.62 Hz, 2 H) 7.18 (d, *J*=2.57 Hz, 1 H) 7.24 (d, *J*=2.94 Hz, 1 H) 7.46 - 7.59 (m, 2 H) 7.79 (d, *J*=7.35 Hz, 1 H) 7.84 (s, 1 H) 10.11(s, 1 H) 10.33 (s, 1 H).

N-(3-(2-(3-tert-butyl-5-(2,4-dioxotetrahydropyrimidin-1(2H)-yl)-2-methoxyphenyl) oxazol-5-yl)phenyl)methanesulfonamide (compound **IA-L0-1.9**)

1-(3-tert-butyl-4-methoxy-5-(5-(3-nitrophenyl)oxazol-2-yl)phenyl)dihydropyrimidine-2,4(1H,3H)-dione (compound **IA-L0-1.10**)

1-(3-tert-butyl-4-methoxy-5-(5-(4-nitrophenyl)oxazol-2-yl)phenyl)dihydropyrimidine-2,4(1H,3H)-dione (compound **IA-L0-1.11**)

N-(3'-tert-butyl-5'-(2,4-dioxo-3,4-dihydropyrimidin-1(2H)-yl)-2'-methoxybiphenyl-4-yl) methanesulfonamide (compound **IB-L0-1.2**). ¹H NMR (300 MHz, CDCl₃) δ ppm 1.43 (s, 9H) 3.07 (s, 3H) 3.32 (s, 3H) 5.82 (dd, *J*=8.09, 2.21 Hz, 1H) 6.79 (s, 1H) 7.14 (d, *J*=2.94 Hz, 1H) 7.21 (d, *J*=2.57 Hz, 1H) 7.28 (d, 2H) 7.35 (d, *J*=8.09 Hz, 1H) 7.54 (d, *J*=8.46 Hz, 2H) 8.31 (s, 1H).

(E)-N'-((3'-tert-butyl-5'-(2,4-dioxo-3,4-dihydropyrimidin-1(2H)-yl)-3-fluoro-2'-methoxy biphenyl-4-yl)methylene)methanesulfonohydrazide (compound **IB-L0-1.3**). ¹H NMR (300 MHz, DMSO-d₆) δ ppm11.42 (s, 1 H) 11.33 (s, 1 H) 8.21 (s, 1 H) 7.95 (s, 1 H) 7.78 (d, J=7.72 Hz, 1 H) 7.39-7.59 (m, 2 H) 7.35 (s, 2 H) 5.65 (dd, J=7.91, 2.02 Hz, 1 H) 3.20 - 3.44 (m, 3 H) 3.11 (s, 3 H) 1.40 (s, 9 H).

N-(2-(3'-tert-butyl-5'-(2,4-dioxo-3,4-dihydropyrimidin-1(2H)-yl)-2'-methoxybiphenyl-4-yl) ethyl)methanesulfonamide (compound **IB-L0-1.4**). ¹H NMR (300 MHz, CDCl₃) δ ppm 1.43 (s, 9H) 2.86-2.99 (m, 2H) 2.91 (s, 3H) 3.31 (s, 3H) 3.38-3.51 (m, 2H) 5.80 (dd, *J*=8.09, 2.21 Hz, 1H) 7.12 (d, *J*=2.57 Hz, 1H) 7.21 (d, *J*=2.57 Hz, 1H) 7.26-7.32 (m, 1H) 7.35 (d, *J*=8.09 Hz, 1H) 7.52 (d, *J*=8.09 Hz, 2H) 7.78 (d, *J*=7.72 Hz, 1H) 8.15 (s, 1H).

(E)-N'-(1-(3'-tert-butyl-5'-(2,4-dioxo-3,4-dihydropyrimidin-1(2H)-yl)-2'-methoxy biphenyl-4-yl)ethylidene)methanesulfonohydrazide (compound **IB-L0-1.5**). ¹H NMR (300 MHz, DMSO-d₆) δ ppm 11.40 (s, 1 H) 10.13 (s, 1 H) 7.88 (d, J=8.09 Hz, 2 H) 7.78 (d, J=7.72 Hz, 1 H) 7.60 (d, J=8.09 Hz, 2 H) 7.29 (d, J=4.04 Hz, 2 H) 5.64 (dd, J=7.72, 1.84 Hz, 1 H) 3.27 (s, 3 H) 3.10 (s, 3 H) 2.27 (s, 3 H) 1.40 (s, 9 H).

(E)-N'-((3'-tert-butyl-5'-(2,4-dioxo-3,4-dihydropyrimidin-1(2H)-yl)-2',4-dimethoxy biphenyl-3-yl)methylene)methanesulfonohydrazide (compound **IB-L0-1.6**). ¹H NMR (300 MHz, DMSO-d₆) δ ppm 11.38 (s, 1 H) 11.05 (s, 1 H) 8.34 (s, 1 H) 7.88 (s, 1 H) 7.75 (d, J=8.09 Hz, 2 H) 7.41 - 7.66 (m, 1 H) 7.00 - 7.37 (m, 3 H) 5.63 (d, J=7.72 Hz, 1 H) 3.91 (s, 3 H) 3.27 (s, 3 H) 3.03 (s, 3 H) 1.40 (s, 9 H).

N-(3'-tert-butyl-5'-(2,4-dioxo-3,4-dihydropyrimidin-1(2H)-yl)-2'-methoxybiphenyl-4-ylcarbamoyl)methanesulfonamide (compound **IB-L0-1.7**). ¹H NMR (300 MHz, DMSO-d₆) δ ppm 1.39 (s, 9H) 2.50 (s, 3H) 3.26 (s, 3H) 5.63 (dd, *J*=7.91, 2.02 Hz, 1H) 7.23 (d, 2H) 7.38-7.63 (m, 5H) 7.76 (d, *J*=8.09 Hz, 1H) 8.95 (s, 1H) 10.46 (s, 1H) 11.39 (d, *J*=2.21 Hz, 1H).

N'-(3'-tert-butyl-5'-(2,4-dioxo-3,4-dihydropyrimidin-1(2H)-yl)-2'-methoxybiphenyl carbonyl) methanesulfonohydrazide (compound **IB-L0-1.8**). ¹H NMR (300 MHz, DMSO-d₆) δ ppm 1.41 (s, 9H) 3.03 (s, 3H) 3.26 (s, 3H) 5.65 (dd, *J*=7.72, 1.84 Hz, 1H) 7.27-7.36 (m, 2H) 7.73 (dd, *J*=28.31, 8.09 Hz, 4H) 8.01 (d, *J*=8.09 Hz, 2H) 9.65 (s, 1H) 10.82 (s, 1H) 11.41 (s, 1H).

(E)-N'-(1-(3'-tert-butyl-5'-(2,4-dioxo-3,4-dihydropyrimidin-1(2H)-yl)-2'-methoxy biphenyl-3-yl)ethylidene)methanesulfonohydrazide (compound **IB-L0-1.9**). ¹H NMR (300 MHz, DMSO-*D₆*) δ ppm 11.40(s, 1 H) 10.12(s, 1 H) 7.82 - 7.99 (m, 1 H) 7.62 - 7.82 (m, 2 H) 7.35 - 7.71 (m, 2 H) 7.30 (s, 2 H) 5.64 (dd, J=7.91, 2.02 Hz, 1 H) 3.26 (s, 3 H) 3.07 (s, 3 H) 2.27 (s, 3 H) 1.40 (s, 9 H).

(E)-N'-((3'-tert-butyl-5'-(2,4-dioxo-3,4-dihydropyrimidin-1(2H)-yl)-2'-methoxy biphenyl-3-yl)methylene)methanesulfonohydrazide (compound **IB-L0-1.10**). ¹H NMR (300 MHz, CDCl₃) δ ppm 1.44 (s, 9H) 3.19 (s, 3H) 3.31 (s, 3H) 5.44 (s, 1H) 5.82 (d, *J*=7.72 Hz, 1H) 7.17 (d, *J*=2.57 Hz, 1H) 7.24 (d, *J*=2.57 Hz, 1H) 7.3 6 (d, *J*=7.72 Hz, 1H) 7.48 (t, *J*=7.72 Hz, 1 H) 7.63 (d, *J*=7.72 Hz, 1H) 7.70 (d, *J*=7.72 Hz, 1H) 7.80 (s, 1H) 7.88 (s, 1H) 8.32 (s, 1H).

N-(2-(3-tert-butyl-5-(2,4-dioxotetrahydropyrimidin-1(2H)-yl)-2-methoxyphenyl)-1H-benzo[d]imidazol-5-yl)-N-(methylsulfonyl)methanesulfonamide (compound **IA-L0-2.10**) ¹H NMR (300 MHz, DMSO-D6) δ ppm 1.45 (s, 9 H) 2.73 (t, J=6.62 Hz, 2 H) 3.48 (s, 3 H) 3.56 (s, 6 H) 3.83 (t, J=6.80 Hz, 2 H) 4.05 (s, 1 H) 7.38 (dd, J=8.46, 1.84 Hz, 1 H) 7.46 (d, J=2.57 Hz, 1 H) 7.71 (d, J=8.46 Hz, 1 H) 7.76 (d, J=2.57 Hz, 1 H) 7.82 (d, J=1.84 Hz, 1 H) 10.41 (s, 1 H)

N-((6-(3-tert-butyl-2-chloro-5-(2,4-dioxo-3,4-dihydropyrimidin-1(2H)-yl)phenyl)benzo [b]thiophen-3-yl)methyl)methanesulfonamide (compound **IB-L0-2.35**). ¹H NMR (300 MHz, DMSO-d₆) δ ppm 1.52 (s, 9 H) 2.95 (s, 3 H) 4.44 (d, J=5.88 Hz, 2 H) 5.68 (d, J=8.09 Hz, 1 H) 7.40 (d, J=2.57 Hz, 1 H) 7.46 (dd, J=8.09, 1.47 Hz, 1 H) 7.56 (d, J=2.57 Hz, 1 H) 7.62 (t, J=6.07 Hz, 1 H) 7.72 (s, 1 H) 7.83 (d, J=8.09 Hz, 1 H) 8.01 (m, 2 H) 11.46 (s, 1 H).

1-(3-tert-butyl-5-(2-chlorobenzo[d]thiazol-6-yl)-4-methoxyphenyl)pyrimidine-2,4(1H,3H)-dione (compound **IB-L0-2.38**). ¹H NMR (300 MHz, DMSO-D6) δ ppm 1.41 (s, 9 H) 3.24 (s, 3 H) 5.65 (dd, J=8.09, 2.21 Hz, 1 H) 7.34 (s, 2 H) 7.73 (dd, J=8.64, 1.65 Hz, 1 H) 7.79 (d, J=8.09 Hz, 1 H) 8.07 (d, J=8.46 Hz, 1 H) 8.30 (d, J=1.84 Hz, 1 H) 11.42 (d, J=1.84 Hz, 1 H)

N-(2-(3-tert-butyl-5-(2,4-dioxo-3,4-dihydropyrimidin-1(2H)-yl)-2-methoxyphenyl)quinolin-6-yl)methanesulfonamide (compound **IB-L0-2.48**)**.**

1-(3-tert-butyl-4-methoxy-5-(1-oxo-2,3-dihydro-1H-inden-5-yl)phenyl)pyrimidine-2,4(1H,3H)-dione (compound **IB-L0-2.50**).

N,N'-(6,6'-(5-(2,4-dioxo-3,4-dihydropyrimidin-1(2H)-yl)-2-methoxy-1,3-phenylene) bis(naphthalene-6,2-diyl))dimethanesulfonamide (compound **IB-L0-2.76).** ¹H NMR (300 MHz, DMSO-d₆) δ ppm 3.08 (s, 6 H) 3.13 (s, 3 H) 5.72 (d, J=8.18 Hz, 1 H) 7.43 (dd, J=8.46, 1.84 Hz, 2 H) 7.59 (s, 2 H) 7.79 (m, 4 H) 7.96 (m, 5 H) 8.14 (s, 2 H) 10.05 (s, 2 H) 11.48 (s, 1 H).

(E)-N-(4-(1-(3-tert-butyl-5-(2,4-dioxotetrahydropyrimidin-1(2H)-yl)-2-methoxyphenyl) prop-1-en-2-yl)phenyl)methanesulfonamide (compound **IA-L1-1.6**). ¹H NMR (300 MHz, DMSO-d₆) δ 2.14 (s, 3 H) 2.70 (t, J=6.62 Hz, 2 H) 3.01 (s, 3 H) 3.68 (s, 3 H) 3.78 (t, J=6.62 Hz, 2 H) 6.82 (s, 1 H) 7.10 - 7.17 (m, 2 H) 7.23 (d, J=8.46 Hz, 2 H) 7.59 (d, J=8.46 Hz, 2 H) 9.78 (s, 1 H) 10.32 (s, 1 H).

(Z)-N-(4-(3-tert-butyl-5-(2,4-dioxotetrahydropyrimidin-1(2H)-yl)-2-methoxystyryl)phenyl) methanesulfonamide (compound **IA-L1-1.10**). ¹H NMR (300 MHz, DMSO-d₆) δ 10.23 (s, 1 H) 9.74 (s, 1 H) 7.23 (d, J=8.46 Hz, 2 H) 7.13 (d, J=2.57 Hz, 1 H) 7.06 (d, J=8.82 Hz, 2 H) 6.92 (d, J=2.57 Hz, 1 H) 6.54 - 6.67 (m, 2 H) 3.78 (s, 3 H) 3.57 (t, J=6.62 Hz, 2 H) 2.96 (s, 3 H) 2.60 (t, J=6.80 Hz, 2 H) 1.34 (s, 9 H).

(E)-N-(4-(3-tert-butyl-5-(2,4-dioxotetrahydropyrimidin-1(2H)-yl)-2-methoxystyryl) phenyl)-N-(methylsulfonyl)acetamide (compound **IA-L1-1.11**). ¹H NMR (300 MHz, DMSO-d₆) δ 10.36 (s, 1 H) 7.77 (d, J=8.46 Hz, 2 H) 7.56 (d, J=2.21 Hz, 1 H) 7.39 - 7.50 (m, 3 H) 7.25 (d, J=16.55 Hz, 1 H) 7.19 (d, J=2.57 Hz, 1 H) 3.74 - 3.85 (m, 5 H) 3.54 (s, 3 H) 2.72 (t, J=6.62 Hz, 2 H) 1.94 (s, 3 H) 1.38 (s, 9 H).

(E)-1-(3-(4-aminostyryl)-5-tert-butyl-4-methoxyphenyl)dihydropyrimidine-2,4(1H,3H)-dione (compound **IA-L1-1.13**). ¹H NMR (300 MHz, DMSO-d₆) δ 1.36 (s, 9 H) 2.70 (t, J=6.62 Hz, 2 H) 3.74 (s, 3 H) 3.77 (t, J=6.62 Hz, 2 H) 5.34 (s, 1 H) 6.57 (d, J=8.46 Hz, 2 H) 6.98 (s, 1 H) 7.07 (d, J=2.21 Hz, 1 H) 7.17 (s, 2 H) 7.30 (d, J=8.09 Hz, 2 H) 7.45 (d, J=2.21 Hz, 1 H) 10.32 (s, 1 H).

(Z)-N-(4-(3-tert-butyl-5-(2,4-dioxotetrahydropyrimidin-1(2H)-yl)-2-methoxystyryl)phenyl) methanesulfonamide (compound **IA-L1-1.20**). ¹H NMR (500 MHz, DMSO-d₆): δ ppm 1.37 (s, 9H), 2.71 1 (t, J=6.7Hz, 2H), 3.01 (s, 3H), 3.75 (s, 3H), 3.79 (t, J=6.6Hz, 2H), 7.13 (d, J=16.5Hz, 1H), 7.15 (d, J=2.4Hz, 2H), 7.23 (d, J=8.5Hz, 2H), 7.25 (d, J=16.5 Hz, 1H), 7.51 (d, J=2.4Hz, 1H), 7.61 (d, J=8.6Hz, 2H), 9.80(bs, 1H), 10.30 (s, 1H).

N-(4-(2-(3-tert-butyl-5-(2,4-dioxotetrahydropyrimidin-1(2H)-yl)-2-methoxyphenyl)-1-fluorovinyl)phenyl)methanesulfonamide (compound **IA-L1-1.21**). (racemic mixture (1:1) of compounds **IA-L1-1.4** and **IA-L1-1.5**).

(E)-1-(3-tert-butyl-4-methoxy-5-(4-nitrostyryl)phenyl)dihydropyrimidine-2,4(1H,3H)-dione (compound **IA-L1-1.22**).

1-{3-tert-butyl-5-[(Z)-2-chloro-2-(4-nitro-phenyl)-vinyl]-4-methoxy-phenyl}-dihydropyrimidine-2,4-dione (compound **IA-L1-1.23**).

1-{3-tert-butyl-4-methoxy-5-[(E)-2-(4-nitro-phenyl)-propenyl]-phenyl}-dihydro-pyrimidine-2,4-dione (compound **IA-L1-1.24**).

1-{3-tert-Butyl-5-[(E)-2-(4-nitro-phenyl)-vinyl]-phenyl}-dihydro-pyrimidine-2,4-dione (compound **IA-L1-1.25**). ¹H NMR (300 MHz, DMSO-D6) δ ppm 1.33 (s, 9 H) 2.70 - 2.77 (m, 2 H) 3.84 (t, J=6.80 Hz, 2 H) 7.33 (s, 1 H) 7.49 (d, J=4.04 Hz, 2 H) 7.56 (d, J=5.88 Hz, 2 H) 7.89 (d, J=8.82 Hz, 2 H) 8.25 (d, J=8.82 Hz, 2 H) 10.40 (s, 1 H)

N-(4-{(E)-2-[3-tert-Butyl-5-(dioxo-tetrahydro-pyrimidin-1-yl)-2-methoxy-phenyl]-vinyl}-3-methoxy-phenyl)-methanesulfonamide (compound **IA-L1-1.27**). ¹H NMR (300 MHz, DMSO-D6) δ ppm 10.33 (s, 1 H) 9.86 (s, 1 H) 7.64 (d, *J*=8.46 Hz, 1 H) 7.45 (d, *J*=2.21 Hz, 1 H) 7.26 (s, 2 H) 7.12 (d, *J*=2.21 Hz, 1 H) 6.89 (s, 1 H) 6.85 (dd, *J*=8.46, 1.84 Hz, 1 H) 3.84 (s, 3 H) 3.78 (t, *J*=6.80 Hz, 2 H) 3.74 (s, 3 H) 3.04 (s, 3 H) 2.71 (t, *J*=6.62 Hz, 2 H) 1.37 (s, 9 H)

N-(4-{(E)-2-[3-tert-Butyl-5-(2,4-dioxo-3,4-dihydro-2H-pyrimidin-1-yl)-2-methoxy-phenyl]-vinyl}-3-formyl-phenyl)-methanesulfonamide (compound **IB-L1-1.6**). ¹H NMR (300 MHz, DMSO-D6) δ ppm 1.39 (s, 9 H) 3.07 (s, 3 H) 3.81 (s, 3 H) 5.66 (dd, J=7.72, 2.21 Hz, 1 H) 7.26 (d, J=2.57 Hz, 1 H) 7.30 (d, J=16.18 Hz, 1 H) 7.51 (dd, J=8.64, 2.39 Hz, 1 H) 7.69 (d, J=2.57 Hz, 1 H) 7.73 - 7.78 (m, 2 H) 7.97 (d, J=8.82 Hz, 1 H) 8.06 (d, J=16.18 Hz, 1 H) 10.15 (s, 1 H) 10.45 (s, 1 H) 11.43 (d, J=2.21 Hz, 1 H)

N-[4-{(E)-2-[3-tert-Butyl-5-(2,4-dioxo-3,4-dihydro-2H-pyrimidin-1-yl)-2-methoxy-phenyl]-vinyl}-3-(hydroxyimino-methyl)-phenyl]-methanesulfonamide (compound **IB-L1-1.8**). ¹H NMR (300 MHz, DMSO-d₆) δ 1.38 (s, 9 H) 3.03 (s, 3 H) 3.79 (s, 3 H) 5.66 (dd, J=7.91, 2.02 Hz, 1 H) 7.16 (d, J=15.81 Hz, 1 H) 7.22 (d, J=2.57 Hz, 1 H) 7.26 (dd, J=8.64, 2.39 Hz, 1 H) 7.59 (d, J=16.18 Hz, 1 H) 7.63 (d, J=2.21 Hz, 1 H) 7.73 - 7.83 (m, 3 H) 8.64 (s, 1 H) 9.96 (s, 1 H) 11.42 (d, J=2.21 Hz, 1 H) 11.50 (s, 1 H).

2-{(E)-2-[3-tert-Butyl-5-(2,4-dioxo-3,4-dihydro-2H-pyrimidin-1-yl)-2-methoxy-phenyl]-vinyl}-5-methanesulfonylamino-N-(2-methoxy-ethyl)-benzamide (compound **IB-L1-1.9**). ¹H NMR (300 MHz, DMSO-D6) δ 1.38 (s, 9 H) 3.05 (s, 3 H) 3.20 (s, 3 H) 3.37 - 3.49 (m, 4 H) 3.78 (s, 3 H) 5.64 (d, J=7.72 Hz, 1 H) 7.15 (d, J=2.57 Hz, 1 H) 7.20 (d, J=2.57 Hz, 1 H) 7.24 (s, 2 H) 7.28 (dd, J=8.46, 2.21 Hz, 1 H) 7.42 (d, J=2.57 Hz, 1 H) 7.73 (d, J=7.72 Hz, 1 H) 7.87 (d, J=8.82 Hz, 1 H) 8.49 (t, J=5.15 Hz, 1 H) 9.99 (s, 1 H) 11.42 (s, 1 H).

2-{(E)-2-[3-tert-Butyl-5-(2,4-dioxo-3,4-dihydro-2H-pyrimidin-1-yl)-2-methoxy-phenyl]-vinyl}-5-methanesulfonylamino-benzoic acid ethyl ester (compound **IB-L1-1.11**). ¹H NMR (300 MHz, DMSO-d₆) δ 1.31 (t, J=7.17 Hz, 3 H) 1.38 (s, 9 H) 3.05 (s, 3 H) 3.79 (s, 3 H) 4.33 (q, J=7.23 Hz, 2 H) 5.65 (dd, J=7.72, 2.21 Hz, 1 H) 7.15 - 7.25 (m, 2 H) 7.46 (dd, J=8.64, 2.39 Hz, 1 H) 7.52 (d, J=2.57 Hz, 1 H) 7.68 (d, J=2.57 Hz, 1 H) 7.71 - 7.81 (m, 2 H) 7.90 (d, J=8.46 Hz, 1 H) 10.06 (s, 1 H) 11.42 (d, J=1.84 Hz, 1 H).

N-(4-{(E)-2-[3-tert-Butyl-2-chloro-5-(2,4-dioxo-3,4-dihydro-2H-pyrimidin-1-yl)-phenyl]-vinyl}-phenyl)-methanesulfonamide (compound **IB-L1-1.12**). ¹H NMR (300 MHz, DMSO-d₆) δ ppm 1.49 (s, 9 H) 3.02 (s, 3 H) 5.69 (d, J=7.72 Hz, 1 H) 7.22 (m, 3 H) 7.41 (d, J=2.21 Hz, 1 H) 7.51 (d, J=16.18 Hz, 1 H) 7.59 (d, J=8.82 Hz, 2 H) 7.78 (d, J=2.21 Hz, 1 H) 7.80 (d, J=8.09 Hz, 1 H) 9.90 (s, 1 H) 11.47 (s, 1 H).

2-{(E)-2-[3-tert-Butyl-5-(2,4-dioxo-3,4-dihydro-2H-pyrimidin-1-yl)-2-methoxy-phenyl]-vinyl}-5-methanesulfonylamino-N,N-dimethyl-benzamide (compound **IB-L1-1.14**). ¹H NMR (300 MHz, DMSO-d₆) δ 1.37 (s, 9 H) 2.76 (s, 3 H) 3.03 (s, 3 H) 3.05 (s, 3 H) 3.76 (s, 3 H) 5.64 (dd, J=7.91, 1.65 Hz, 1 H) 6.95 (d, J=16.55 Hz, 1 H) 7.02 (d, J=2.21 Hz, 1 H) 7.17 - 7.25 (m, 2 H) 7.27 (dd, J=8.64, 2.39 Hz, 1 H) 7.48 (d, J=2.57 Hz, 1 H) 7.74 (d, J=8.09 Hz, 1 H) 7.82 (d, J=8.82 Hz, 1 H) 10.03 (s, 1 H) 11.39 - 11.43 (m, 1 H).

2-{(E)-2-[3-tert-butyl-5-(2,4-dioxo-3,4-dihydro-2H-pyrimidin-1-yl)-2-methoxy-phenyl]-vinyl}-5-methanesulfonylamino-N-methyl-benzamide (compound **IB-L1-1.17**). ¹H NMR (300 MHz, DMSO-d₆) δ 1.38 (s, 9 H) 2.77 (d, J=4.41 Hz, 3 H) 3.06 (s, 3 H) 3.77 (s, 3 H) 5.64 (dd, J=7.72, 1.84 Hz, 1 H) 7.16 - 7.33 (m, 5 H) 7.43 (d, J=2.21 Hz, 1 H) 7.73 (d, J=7.72 Hz, 1 H) 7.84 (d, J=8.46 Hz, 1 H) 8.37 (q, J=4.41 Hz, 1 H) 10.00 (s, 1 H) 11.40 (d, J=1.84 Hz, 1 H).

2-{(E)-2-[3-tert-butyl-5-(2,4-dioxo-3,4-dihydro-2H-pyrimidin-1-yl)-2-methoxy-phenyl]-vinyl}-N-(1,1-dioxo-tetrahydro-1lambda*6*-thiophen-3-yl)-5-methanesulfonylamino-N-methyl-benzamide (compound **IB-L1-1.18**). ¹H NMR (300 MHz, DMSO-d₆) δ 1.37 (s, 9 H) 2.17 - 2.47 (m, 2 H) 2.70 (s, 3 H) 3.06 (s, 3 H) 3.15 - 3.31 (m, 2 H) 3.36 - 3.51 (m, 2 H) 3.77 (s, 3 H) 5.37 (dt, J=17.74, 8.96 Hz, 1 H) 5.65 (dd, J=7.91, 2.02 Hz, 1 H) 6.93 (d, J=16.18 Hz, 1 H) 7.05 (d, J=2.21 Hz, 1 H) 7.19 - 7.35 (m, 3 H) 7.50 (d, J=2.57 Hz, 1 H) 7.76 (d, J=8.09 Hz, 1 H) 7.87 (d, J=8.82 Hz, 1 H) 10.04 (s, 1 H) 11.38 (d, J=2.21 Hz, 1 H).

N-(4-{(E)-2-[3-tert-butyl-5-(5-chloro-2,4-dioxo-3,4-dihydro-2H-pyrimidin-1-yl)-2-methoxyphenyl]-vinyl}-phenyl)-methanesulfonamide (Compound **IB-L1-1.20**). ¹H NMR (300 MHz, DMSO-D6) δ ppm 11.31 (s, 1 H) 9.77 (s, 1 H) 7.53 (d, *J*=8.09 Hz, 1 H) 7.23 (d, *J*=8.46 Hz, 2 H) 7.17 (d, *J*=2.57 Hz, 1 H) 7.06 (d, *J*=8.82 Hz, 2 H) 7.01 (d, *J*=2.57 Hz, 1 H) 6.53 - 6.71 (m, 2 H) 5.56 (d, *J*=7.72 Hz, 1 H) 3.81 (s,3H)2.96(s,3H)1.35(s,9H)

2-{(E)-2-[3-tert-butyl-5-(2,4-dioxo-3,4-dihydro-2H-pyrimidin-1-yl)-2-methoxy-phenyl]-vinyl}-5-methanesulfonylamino-benzamide (compound **IB-L1-1.21**). ¹H NMR (300 MHz, DMSO-d₆) δ 1.38 (s, 9 H) 3.07 (s, 3 H) 3.78 (s, 3 H) 5.64 (d, J=7.72 Hz, 1 H) 7.18 - 7.34 (m, 5 H) 7.43 (d, J=2.21 Hz, 1 H) 7.54 (s, 1 H) 7.73 (d, J=7.72 Hz, 1 H) 7.84 (d, J=8.46 Hz, 1 H) 7.93 (s, 1 H).

N-(3-(azetidine-1-carbonyl)-4-{(E)-2-[3-tert-butyl-5-(2,4-dioxo-3,4-dihydro-2H-pyrimidin-1-yl)-2-methoxy-phenyl]-vinyl}-phenyl)-methanesulfonamide (compound (compound **IB-L1-1.22**). ¹H NMR (300 MHz, DMSO-d₆) δ 1.38 (s, 9 H) 3.07 (s, 3 H) 3.78 (s, 3 H) 5.64 (d, J=7.72 Hz, 1 H) 7.18 - 7.34 (m, 5 H) 7.43 (d, J=2.21 Hz, 1 H) 7.54 (s, 1 H) 7.73 (d, J=7.72 Hz, 1 H) 7.84 (d, J=8.46 Hz, 1 H) 7.93 (s, 1 H).

2-{(E)-2-[3-tert-butyl-5-(2,4-dioxo-3,4-dihydro-2H-pyrimidin-1-yl)-2-methoxy-phenyl]-vinyl}-5-methanesulfonylamino-N-(2-methoxy-ethyl)-N-methyl-benzamide (compound **IB-L1-1.24**). ¹H NMR (300 MHz, DMSO-d₆) δ 1.40 (s, 9 H) 2.81 (s, 3 H) 3.07 (s, 3 H) 3.23 (s, 3 H) 3.29 (t, J=5.33 Hz, 1 H) 3.39 (t, J=4.96 Hz, 1 H) 3.62 (t, J=4.78 Hz, 2 H) 3.82 (s, 3 H) 5.68 (d, J=8.09 Hz, 1 H) 6.96 - 7.07 (m, 1 H) 7.09 - 7.17 (m, 1 H) 7.23 - 7.38 (m, 3 H) 7.49 (dd, J=16.55, 2.57 Hz, 1 H) 7.71 - 7.76 (m, 1 H) 7.83 - 7.94 (m, 1 H).

N-(4-{(E)-2-[3-tert-butyl-5-(2,4-dioxo-3,4-dihydro-2H-pyrimidin-1-yl)-2-methoxy-phenyl]-vinyl}-3-isopropoxymethyl-phenyl)-methanesulfonamide (compound **IB-L1-1.25**). ¹H NMR (300 MHz, DMSO-d₆) δ 1.16 (d, J=5.88 Hz, 6 H) 1.38 (s, 9 H) 3.01 (s, 3 H) 3.69 (dt, J=12.13, 6.07 Hz, 1 H) 3.79 (s, 3 H) 4.59 (s, 2 H) 5.65 (dd, J=7.91, 2.02 Hz, 1 H) 7.13 - 7.29 (m, 4 H) 7.32 - 7.40 (m, 1 H) 7.59 (d, J=2.57 Hz, 1 H) 7.75 (d, J=8.09 Hz, 2 H) 9.86 (s, 1 H) 11.43 (d, J=1.84 Hz, 1 H).

N-[4-{(E)-2-[3-tert-butyl-5-(2,4-dioxo-3,4-dihydro-2H-pyrimidin-1-yl)-2-methoxy-phenyl]-vinyl}-3-(pyrrolidine-1-carbonyl)-phenyl]-methanesulfonamide (compound **IB-L1-1.27**). ¹H NMR (300 MHz, DMSO-d₆) δ 1.37 (s, 9 H) 1.73 - 1.89 (m, 4 H) 3.03 - 3.12 (m, 5 H) 3.51 (t, J=6.80 Hz, 2 H) 3.76 (s, 3 H) 5.64 (dd, J=7.91, 2.02 Hz, 1 H) 6.99 - 7.06 (m, 1 H) 7.08 (d, J=2.21 Hz, 1 H) 7.19 - 7.31 (m, 3 H) 7.46 (d, J=2.57 Hz, 1 H) 7.75 (d, J=8.09 Hz, 1 H) 7.82 (d, J=8.82 Hz, 1 H) 10.01 (s, 1 H) 11.41 (d, J=2.21 Hz, 1 H).

N-[4-{(E)-2-[3-tert-butyl-5-(2,4-dioxo-3,4-dihydro-2H-pyrimidin-1-yl)-2-methoxy-phenyl]-vinyl}-3-(3-hydroxy-azetidin-1-ylmethyl)-phenyl]-methanesulfonamide (compound **IB-L1-1.29**). ¹H NMR (300 MHz, DMSO-d₆) δ 1.38 (s, 9 H) 2.78 - 2.85 (m, 2 H) 2.99 (s, 3 H) 3.50 - 3.58 (m, 2 H) 3.71 (s, 2 H) 3.79 (s, 3 H) 4.19 (td, J=12.41, 6.07 Hz, 1 H) 5.29 (d, J=6.25 Hz, 1 H) 5.66 (d, J=8.09 Hz, 1 H) 7.10 - 7.18 (m, 2 H) 7.20 (t, J=2.21 Hz, 2 H) 7.35 - 7.42 (m, 1 H) 7.63 (d, J=2.57 Hz, 1 H) 7.69 (d, J=8.46 Hz, 1 H) 7.76 (d, J=7.72 Hz, 1 H) 9.78 (s, 1 H) 11.42 (s, 1 H).

N-(4-{(E)-2-[3-tert-Butyl-5-(2,4-dioxo-3,4-dihydro-2H-pyrimidin-1-yl)-2-methoxy-phenyl]-vinyl}-3-pyrrolidin-1-ylmethyl-phenyl)-methanesulfonamide (compound **IB-L1-1.33**). ¹H NMR (500 MHz, DMSO-d₆) δ 1.40 (s, 9 H) 1.72 - 1.95 (m, 4 H) 2.84 (s, 2 H) 2.88 - 2.98 (m, 2 H) 3.01 (s, 3 H) 3.81 (s, 3 H) 3.86 - 4.23 (m, 2 H) 5.63 (d, J=7.81 Hz, 1 H) 7.17 (d, J=15.63 Hz, 1 H) 7.21 - 7.28 (m, 2 H) 7.32 - 7.38 (m, 1 H) 7.47 (d, J=16.11 Hz, 1 H) 7.53 - 7.59 (m, 1 H) 7.61 (d, J=7.81 Hz, 1 H) 7.70 (d, J=6.35 Hz, 1 H) 9.42 (s, 1 H) 10.88 (s, 1 H).

N-(4-{(Z)-2-[3-tert-Butyl-5-(2,4-dioxo-3,4-dihydro-2H-pyrimidin-1-yl)-2-methoxy-phenyl]-vinyl}-phenyl)-methanesulfonamide (compound **IB-L1-1.34 ¹H** NMR (300 MHz, DMSO-D6) δ ppm 11.31 (s, 1 H) 9.77 (s, 1 H) 7.53 (d, *J*=8.09 Hz, 1 H) 7.23 (d, *J*=8.46 Hz, 2 H) 7.17 (d, *J*=2.57 Hz, 1 H) 7.06 (d, *J*=8.82 Hz, 2 H) 7.01 (d, *J*=2.57 Hz, 1 H) 6.53 - 6.71 (m, 2 H) 5.56 (d, *J*=7.72 Hz, 1 H) 3.81 (s, 3 H) 2.96 (s, 3 H) 1.35 (s, 9 H)

N-(4-((3-tert-butyl-5-(2,4-dioxotetrahydropyrimidin-1(2H)-yl)phenyl)ethynyl)phenyl)-methanesulfonamide (compound **IA-L2-1.1**). ¹H NMR (300 MHz, DMSO-d6) δ ppm 1.30 (s, 9 H) 2.72 (t, J=6.43 Hz, 2 H) 3.06 (s, 3 H) 3.82 (t, J=6.62 Hz, 2 H) 7.24 (d, J=8.82 Hz, 2 H) 7.33 (s, 1 H) 7.39 (d, J=1.47 Hz, 2 H) 7.54 (d, J=8.82 Hz, 2 H) 10.08 (s, 1 H) 10.40 (s, 1 H)

N-(4-((3-tert-butyl-5-(2,4-dioxotetrahydropyrimidin-1(2H)-yl)phenyl)ethynyl)-3-methylphenyl)methanesulfonamide (compound **IA-L2-1.2**). ¹H NMR (300 MHz, DMSO-d6) δ ppm 1.30 (s, 9 H) 2.44 (s, 3 H) 2.72 (t, J=6.62 Hz, 2 H) 3.05 (s, 3 H) 3.82 (t, J=6.62 Hz, 2 H) 7.07 (dd, J=8.46, 1.47 Hz, 1 H) 7.13 (s, 1 H) 7.33 (s, 1 H) 7.39 (d, J=1.47 Hz, 2 H) 7.48 (d, J=8.09 Hz, 1 H) 9.98 (s, 1 H) 10.40 (s, 1 H)

1-(3-tert-butyl-4-methoxy-5-(phenylethynyl)phenyl)dihydropyrimidine-2,4(1H,3H)-dione (compound **IA-L2-1.4**). ¹H NMR (300 MHz, DMSO-d₆) δ ppm 1.36 (s, 9 H) 2.70 (t, *J*=6.62 Hz, 2 H) 3.78 (t, *J*=6.43 Hz, 2 H) 4.07 (s, 3 H) 7.27 (d, *J*=2.57 Hz, 1 H) 7.38 (d, *J*=2.57 Hz, 1 H) 7.41 - 7.49 (m, 3 H) 7.55 - 7.63 (m, 2 H) 10.37 (s, 1 H).

N-(3-((3-tert-butyl-5-(2,4-dioxotetrahydropyrimidin-1(2H)-yl)-2-methoxyphenyl) ethynyl)phenyl)methanesulfonamide (compound **IA-L2-1.7**). ¹H NMR (300 MHz, DMSO-d₆) δ ppm 1.36 (s, 9 H) 2.70 (t, *J*=6.62 Hz, 2 H) 3.04 (s, 3 H) 3.78 (t, *J*=6.62 Hz, 2 H) 4.06 (s, 3 H) 7.23 - 7.35 (m, 3 H) 7.35 - 7.46 (m, 3 H) 9.94 (s, 1 H) 10.37 (s, 1 H).

N-(4-((3-tert-butyl-5-(2,4-dioxotetrahydropyrimidin-1(2H)-yl)-2-methoxyphenyl) ethynyl)-phenyl)methanesulfonamide (compound **IA-L2-1.8**). ¹H NMR (300 MHz, DMSO-d₆) δ ppm 1.35 (s, 9 H) 2.70 (t, *J*=6.62 Hz, 2 H) 3.06 (s, 3 H) 3.77 (t, *J*=6.62 Hz, 2 H) 4.05 (s, 3 H) 7.25 (dd, *J*=5.52, 2.94 Hz, 3 H) 7.35 (d, *J*=2.57 Hz, 1 H) 7.55 (d, *J*=8.46 Hz, 2 H) 10.09 (s, 1 H) 10.37 (s, 1 H).

N-(4-((3-tert-butyl-5-(2,4-dioxotetrahydropyrimidin-1(2H)-yl)-2-methoxyphenyl) ethynyl)-2-methylphenyl)methanesulfonamide (compound **IA-L2-1.10**). ¹H NMR (300 MHz, DMSO-d6) δ 1.35 (s, 9 H) 2.32 (s, 3 H) 2.70 (t, J=6.62 Hz, 2 H) 3.03 (s, 3 H) 3.78 (t, J=6.62 Hz, 2 H) 4.06 (s, 3 H) 7.26 (d, J=2.57 Hz, 1 H) 7.32 - 7.44 (m, 3 H) 7.48 (s, 1 H) 9.23 (s, 1 H) 10.37 (s, 1 H)

N-(4-((3-tert-butyl-5-(2,4-dioxotetrahydropyrimidin-1(2H)-yl)-2-methoxyphenyl) ethynyl)-3-ethylphenyl)methanesulfonamide (compound **IA-L2-1,11**). ¹H NMR (300 MHz, DMSO-d₆) δ ppm 1.15 - 1.28 (m, 3 H) 1.36 (s, 9 H) 2.70 (t, *J*=6.62 Hz, 2 H) 2.81 (q, *J*=7.72 Hz, 2 H) 3.05 (s, 3 H) 3.78 (t, *J*=6.62 Hz, 2 H) 4.04 (s, 3 H) 7.10 (dd, *J*=8.27, 2.02 Hz, 1 H) 7.14 (s, 1 H) 7.25 (d, *J*=2.57 Hz, 1 H) 7.35 (d, *J*=2.21 Hz, 1 H) 7.50 (d, *J*=8.09 Hz, 1 H) 10.01 (s, 1 H) 10.36 (s, 1 H).

N-(4-((3-tert-butyl-5-(2,4-dioxotetrahydropyrimidin-1(2H)-yl)-2-methoxyphenyl) ethynyl)-3-fluorophenyl)methanesulfonamide (compound **IA-L2-1.12**). ¹H NMR (300 MHz, DMSO-d₆) δ ppm 1.35 (s, 9 H) 2.70 (t, *J*=6.62 Hz, 2 H) 3.13 (s, 3 H) 3.78 (t, *J*=6.62 Hz, 2 H) 4.05 (s, 3 H) 6.98 - 7.20 (m, 2 H) 7.28 (d, *J*=2.57 Hz, 1 H) 7.36 (d, *J*=2.57 Hz, 1 H) 7.61 (t, *J*=8.27 Hz, 1 H) 10.37 (s, 2 H).

N-(4-((3-tert-butyl-5-(2,4-dioxotetrahydropyrimidin-1(2H)-yl)-2-methoxyphenyl) ethynyl)-2-fluorophenyl)methanesulfonamide (compound **IA-L2-1.13**). ¹H NMR (300 MHz, DMSO-d₆) δ ppm 1.35 (s, 9 H) 2.70 (t, J=6.62 Hz, 2 H) 3.10 (s, 3 H) 3.78 (t, J=6.62 Hz, 2 H) 4.05 (s, 3 H) 7.28 (d, J=2.57 Hz, 1 H) 7.38 (d, J=2.57 Hz, 1 H) 7.42 - 7.61 (m, 3 H) 9.89 (s, 1 H) 10.38 (s, 1 H).

N-(4-((3-tert-butyl-5-(2,4-dioxotetrahydropyrimidin-1(2H)-yl)-2-methoxyphenyl) ethynyl)-3-chlorophenyl)methanesulfonamide (compound **IA-L2-1.14**). ¹H NMR (300 MHz, DMSO-d₆) δ ppm 1.35 (s, 9 H) 2.70 (t, J=6.62 Hz, 2 H) 3.12 (s, 3 H) 3.78 (t, J=6.80 Hz, 2 H) 4.07 (s, 3 H) 7.21 (dd, J=8.46, 2.21 Hz, 1 H) 7.28 (d, J=2.57 Hz, 1 H) 7.36 (dd, J=4.60, 2.39 Hz, 2 H) 7.67 (d, J=8.46 Hz, 1 H) 10.32 (s, 1 H) 10.37 (s, 1 H).

N-(4-((3-tert-butyl-5-(2,4-dioxotetrahydropyrimidin-1(2H)-yl)-2-methoxyphenyl) ethynyl)-2-chlorophenyl)methanesulfonamide (compound **IA-L2-1.15**). ¹H NMR (300 MHz, DMSO-d6) δ ppm 1.35 (s, 9 H) 2.70 (t, J=6.62 Hz, 2 H) 3.10 (s, 3 H) 3.78 (t, J=6.62 Hz, 2 H) 4.06 (s, 3 H) 7.29 (d, J=2.94 Hz, 1 H) 7.39 (d, J=2.57 Hz, 1 H) 7.48 - 7.59 (m, 2 H) 7.75 (s, 1 H) 9.65 (s, 1 H) 10.38 (s, 1 H)

N-(4-((3-tert-butyl-5-(2,4-dioxotetrahydropyrimidin-1(2H)-yl)-2-methoxyphenyl) ethynyl)-3-methoxyphenyl)methanesulfonamide (compound **IA-L2-1.16**). ¹H NMR (300 MHz, DMSO-d₆) δ ppm 1.35 (s, 9 H) 2.63 - 2.76 (m, 2 H) 3.08 (s, 3 H) 3.77 (t, J=6.80 Hz, 2 H) 3.82 (s, 3 H) 4.08 (s, 3 H) 6.79 - 6.89 (m, 1 H) 6.91 (d, J=1.84 Hz, 1 H) 7.22 (d, J=2.57 Hz, 1 H) 7.30 (d, J=2.94 Hz, 1 H) 7.45 (d, J=8.46 Hz, 1 H) 10.06 (s, 1 H) 10.35 (s, 1 H).

N-(4-((3-tert-butyl-5-(2,4-dioxotetrahydropyrimidin-1(2H)-yl)-2-methoxyphenyl) ethynyl)-2-(trifluoromethoxy)phenyl)methanesulfonamide (compound **IA-L2-1.17**). ¹H NMR (300 MHz, DMSO-d₆) δ ppm 1.35 (s, 9 H) 2.70 (t, J=6.62 Hz, 2 H) 3.13 (s, 3 H) 3.78 (t, J=6.62 Hz, 2 H) 4.05 (s, 3 H) 7.29 (d, J=2.57 Hz, 1 H) 7.40 (d, J=2.57 Hz, 1 H) 7.52 - 7.69 (m, 3 H) 10.06 (s, 1 H) 10.38 (s, 1 H).

N-(4-((3-tert-butyl-5-(2,4-dioxotetrahydropyrimidin-1(2H)-yl)-2-methoxyphenyl) ethynyl)-2-(trifluoromethyl)phenyl)methanesulfonamide (compound **IA-L2-1.19**). ¹H NMR (300 MHz, DMSO-d₆) δ ppm 1.35 (s, 9 H) 2.72 (d, J=6.62 Hz, 2 H) 3.09 (s, 3 H) 3.78 (s, 2 H) 4.06 (s, 3 H) 7.29 (s, 1 H) 7.41 (s, 1 H) 7.57 - 7.73 (m, 1 H) 7.80 - 7.94 (m, 2 H) 9.62 (s, 1 H) 10.38 (s, 1 H).

N-(4-((3-tert-butyl-5-(2,4-dioxotetrahydropyrimidin-1(2H)-yl)-2-methoxyphenyl) ethynyl)-2-fluoro-5-methylphenyl)methanesulfonamide (compound **IA-L2-1.20**). ¹H NMR (300 MHz, DMSO-D6) δ ppm 1.36 (s, 9 H) 2.45 (s, 3 H) 2.71 (t, J=6.43 Hz, 2 H) 3.09 (s, 3 H) 3.78 (t, J=6.62 Hz, 2 H) 4.05 (s, 3 H) 7.28 (d, J=2.57 Hz, 1 H) 7.36 (d, J=8.09 Hz, 1 H) 7.39 (d, J=2.57 Hz, 1 H) 7.47 (d, J=11.03 Hz, 1 H) 9.80 (s, 1 H) 10.37 (s, 1 H)

N-(4-((3-tert-butyl-5-(2,4-dioxotetrahydropyrimidin-1(2H)-yl)-2-methoxyphenyl) ethynyl)-3-chloro-2-fluorophenyl)methanesulfonamide (compound **IA-L2-1.21**). ¹H NMR (300 MHz, DMSO-d₆) δ ppm 1.36 (s, 9 H) 2.71 (t, J=6.43 Hz, 2 H) 3.14 (s, 3 H) 3.78 (t, J=6.62 Hz, 2 H) 4.08 (s, 3 H) 7.31 (d, J=2.94 Hz, 1 H) 7.39 - 7.46 (m, 1 H) 7.48 (d, J=7.72 Hz, 1 H) 7.51 - 7.61 (m, 1 H) 10.15 (s, 1 H) 10.38 (s, 1 H).

N-(4-((3-tert-butyl-5-(2,4-dioxotetrahydropyrimidin-1(2H)-yl)-2-methoxyphenyl) ethynyl)-2-fluoro-5-(trifluoromethyl)phenyl)methanesulfonamide (compound **IA-L2-1.22**). ¹H NMR (300 MHz, DMSO-D6) δ ppm 1.35 (s, 9 H) 2.71 (t, J=6.62 Hz, 2 H) 3.17 (s, 3 H) 3.78 (t, J=6.43 Hz, 2 H) 4.03 (s, 3 H) 7.32 (d, J=2.57 Hz, 1 H) 7.37 (d, J=2.21 Hz, 1 H) 7.83 (d, J=7.72 Hz, 1 H) 7.88 (d, J=10.66 Hz, 1 H) 10.27 (s, 1 H) 10.38 (s, 1 H)

N-(6-((3-tert-butyl-5-(2,4-dioxotetrahydropyrimidin-1(2H)-yl)-2-methoxyphenyl) ethynyl)-5-methylpyridin-3-yl)methanesulfonamide (compound **IA-L2-1.24**). ¹H NMR (300 MHz, DMSO-d₆) δ ppm 1.36 (s, 9 H) 2.71 (t, *J*=6.62 Hz, 2 H) 3.13 (s, 3 H) 3.79 (t, *J*=6.80 Hz, 2 H) 4.07 (s, 3 H) 7.30 (d, *J*=2.57 Hz, 1 H) 7.41 (d, *J*=2.57 Hz, 1 H) 7.55 (s, 1 H) 8.29 (s, 1 H) 10.24 (s, 1 H) 10.38 (s, 1 H).

N-(5-((3-tert-butyl-5-(2,4-dioxotetrahydropyrimidin-1(2H)-yl)-2-methoxyphenyl) ethynyl)-pyridin-2-yl)methanesulfonamide 2,2,2-trifluoroacetate (compound **IA-L2-1.26**). ¹H NMR (300 MHz, DMSO-d₆) δ ppm 1.35 (s, 9 H) 2.70 (t, *J*=6.62 Hz, 2 H) 3.78 (t, *J*=6.80 Hz, 2 H) 4.05 (s, 3 H) 7.01 (d, *J*=8.82 Hz, 1 H) 7.27 (d, *J*=2.57 Hz, 1 H) 7.37 (d, *J*=2.57 Hz, 1 H) 7.93 (dd, *J*=8.82, 2.21 Hz, 1 H) 8.50 (s, 1 H) 10.37 (s, 1 H) 10.93 (s, 1 H).

N-(4-((3-tert-butyl-5-(2,4-dioxotetrahydropyrimidin-1(2H)-yl)-2-methoxyphenyl) ethynyl)-naphthalen-1-yl)methanesulfonamide (compound **IA-L2-2.1**). ¹H NMR (300 MHz, DMSO-d₆) δ ppm 1.39 (s, 9 H) 2.73 (t, J=6.62 Hz, 2 H) 3.08 (s, 3 H) 3.82 (t, J=6.62 Hz, 2 H) 4.13 (s, 3 H) 7.32 (d, J=2.57 Hz, 1 H) 7.55 (d, J=2.57 Hz, 1 H) 7.58 (d, J=7.72 Hz, 1 H) 7.72 (m, 2 H) 7.87 (d, J=7.72 Hz, 1 H) 8.35 (d, J=8.82 Hz, 1 H) 8.41 (d, J=8.09 Hz, 1 H) 9.99 (s, 1 H) 10.39 (s, 1 H).

N-(4-((3-tert-butyl-5-(2,4-dioxo-3,4-dihydropyrimidin-1(2H)-yl)-2-methoxyphenyl) ethynyl)-3-methylphenyl)methanesulfonamide (compound **IB-L2-1.1**). ¹H NMR (300 MHz, DMSO-D6) δ ppm 1.36 (s, 9 H) 2.46 (s, 3 H) 3.05 (s, 3 H) 4.09 (s, 3 H) 5.64 (d, J=7.72 Hz, 1 H) 7.09 (dd, J=8.27, 2.02 Hz, 1 H) 7.14 (s, 1 H) 7.31 (d, J=2.57 Hz, 1 H) 7.51 (m, 2 H) 7.73 (d, J=7.72 Hz, 1 H) 10.00 (s, 1 H) 11.42 (s, 1 H)

N-(4-((3-tert-butyl-5-(2,4-dioxo-3,4-dihydropyrimidin-1(2H)-yl)-2-methoxyphenyl) ethynyl)-3-chlorophenyl)methanesulfonamide (compound **IB-L2-1.2**) ¹H NMR (300 MHz, DMSO-D6) δ ppm 1.36 (s, 9 H) 3.12 (s, 3 H) 4.11 (s, 3 H) 5.65 (dd, J=7.91, 2.02 Hz, 1 H) 7.22 (dd, J=8.46, 2.21 Hz, 1 H) 7.34 (d, J=2.57 Hz, 1 H) 7.36 (d, J=2.21 Hz, 1 H) 7.50 (d, J=2.57 Hz, 1 H) 7.68 (d, J=8.82 Hz, 1 H) 7.74 (d, J=8.09 Hz, 1 H) 10.33 (s, 1 H) 11.42 (d, J=1.84 Hz, 1 H)

N-(4-((3-tert-butyl-5-(2,4-dioxo-3,4-dihydropyrimidin-1(2H)-yl)-2-methoxyphenyl) ethynyl)-2-fluoro-5-methylphenyl)methanesulfonamide (compound **IB-L2-1.3**). ¹H NMR (300 MHz, DMSO-D6) δ ppm 1.36 (s, 9 H) 2.45 (s, 3 H) 3.09 (s, 3 H) 4.09 (s, 3 H) 5.65 (dd, J=7.91, 2.02 Hz, 1 H) 7.36 (m, 2 H) 7.48 (d, J=10.66 Hz, 1 H) 7.53 (d, J=2.57 Hz, 1 H) 7.74 (d, J=7.72 Hz, 1 H) 9.81 (s, 1 H) 11.43 (d, J=1.84 Hz, 1 H)

N-(4-((3-tert-butyl-5-(2,4-dioxo-3,4-dihydropyrimidin-1(2H)-yl)-2-methoxyphenyl) ethynyl)-2,6-difluorophenyl)methanesulfonamide (compound **IB-L2-1.4**). ¹H NMR (300 MHz, DMSO-D6) δ ppm 1.36 (s, 9 H) 3.10 (s, 3 H) 4.10 (s, 3 H) 5.66 (dd, J=7.72, 2.21 Hz, 1 H) 7.37 (d, J=2.57 Hz, 1 H) 7.46 - 7.58 (m, 3 H) 7.74 (d, J=8.09 Hz, 1 H) 9.74 (s, 1 H) 11.44 (d, J=1.84 Hz, 1 H)

N-(4-((3-tert-butyl-5-(2,4-dioxo-3,4-dihydropyrimidin-1(2H)-yl)-2-methoxyphenyl) ethynyl)-2-fluoro-5-(trifluoromethyl)phenyl)methanesulfonamide (compound **IB-L2-1.5**). ¹H NMR (300 MHz, DMSO-D6) δ ppm 1.36 (s, 9 H) 3.17 (s, 3 H) 4.07 (s, 3 H) 5.66 (dd, J=7.91, 2.02 Hz, 1 H) 7.38 (d, J=2.57 Hz, 1 H) 7.50 (d, J=2.57 Hz, 1 H) 7.74 (d, J=8.09 Hz, 1 H) 7.83 (d, J=7.72 Hz, 1 H) 7.89 (d, J=10.66 Hz, 1 H) 10.28 (s, 1 H) 11.43 (d, J=1.84 Hz, 1 H)

N-(4-((3-tert-butyl-5-(2,4-dioxo-3,4-dihydropyrimidin-1(2H)-yl)-2-methoxyphenyl) ethynyl)-3-hydroxyphenyl)fmethanesulfonamide (compound **IB-L2-1.6**). ¹H NMR (300 MHz, DMSO-d₆) δ ppm 1.36 (s, 9 H) 3.04 (s, 3 H) 4.12 (s, 3 H) 5.64 (dd, *J*=7.72, 2.21 Hz, 1 H) 6.68 (dd, *J*=8.46, 2.21 Hz, 1 H) 6.88 (d, *J*=1.84 Hz, 1 H) 7.26 (d, *J*=2.57 Hz, 1 H) 7.35 (d, *J*=8.46 Hz, 1 H) 7.41 (d, *J*=2.57 Hz, 1 H) 7.73 (d, *J*=7.72 Hz, 1 H) 9.97 (s, 1 H) 10.30 (s, 1 H) 11.41 (d, *J*=2.21 Hz, 1 H).

methyl 2-((3-tert-butyl-5-(2,4-dioxo-3,4-dihydropyrimidin-1(2H)-yl)-2-methoxyphenyl)-ethynyl)-5-(methylsulfonamido)benzoate (compound **IB-L2-1.7**). ¹H NMR (300 MHz, DMSO-d₆) δ ppm 1.37 (s, 9 H) 3.09 (s, 3 H) 3.88 (s, 3 H) 4.11 (s, 3 H) 5.65 (dd, J=7.72, 2.21 Hz, 1 H) 7.32 (d, J=2.57 Hz, 1 H) 7.46 (m, 2 H) 7.74 (m, 3 H) 10.30 (s, 1 H) 11.42 (d, J=1.84 Hz, 1 H).

N-(6-((3-tert-butyl-5-(2,4-dioxo-3,4-dihydropyrimidin-1(2H)-yl)-2-methoxyphenyl) ethynyl)-pyridin-3-yl)methanesulfonamide (compound **IB-L2-1.8**). ¹H NMR (300 MHz, DMSO-d₆) δ ppm 1.36 (s, 9 H) 3.13 (s, 3 H) 4.11 (s, 3 H) 5.65 (dd, *J*=7.91, 2.02 Hz, 1 H) 7.35 (d, *J*=2.57 Hz, 1 H) 7.53 (d, *J*=2.57 Hz, 1 H) 7.66 (d, *J*=1.47 Hz, 2 H) 7.75 (d, *J*=7.72 Hz, 1 H) 8.45 (s, 1 H) 10.33 (s, 1 H) 11.43 (d, *J*=1.84 Hz, 1 H).

N-(5-((3-tert-butyl-5-(2,4-dioxo-3,4-dihydropyrimidin-1(2H)-yl)-2-methoxyphenyl) ethynyl)-pyrazin-2-yl)methanesulfonamide (compound **IB-L2-1.9**). ¹H NMR (300 MHz, DMSO-d₆) δ ppm 1.37 (s, 9 H) 3.38 (s, 3 H) 4.12 (s, 3 H) 5.65 (dd, *J*=8.09, 2.21 Hz, 1 H) 7.38 (d, *J*=2.94 Hz, 1 H) 7.55 (d, *J*=2.57 Hz, 1 H) 7.75 (d, *J*=7.72 Hz, 1 H) 8.32 (s, 1 H) 8.62 (d, *J*=1.47 Hz, 1 H) 11.43 (s, 2 H).

N-(3-tert-butyl-4-((3-tert-butyl-5-(2,4-dioxo-3,4-dihydropyrimidin-1(2H)-yl)-2-methoxyphenyl)ethynyl)phenyl)methanesulfonamide (compound **IB-L2-1.10**). ¹H NMR (300 MHz, DMSO-d₆) δ ppm 1.37 (s, 9 H) 1.49 (s, 9 H) 3.06 (s, 3 H) 4.08 (s, 3 H) 5.64 (d, *J*=7.72 Hz, 1 H) 7.13 (dd, *J*=8.27, 2.02 Hz, 1 H) 7.30 (dd, *J*=6.80, 2.39 Hz, 2 H) 7.43 (d, *J*=2.57 Hz, 1 H) 7.56 (d, *J*=8.09 Hz, 1 H) 7.74 (d, *J*=8.09 Hz, 1 H) 10.01 (s, 1 H) 11.41 (s, 1 H).

N-(4-((3-tert-butyl-5-(2,4-dioxo-3,4-dihydropyrimidin-1(2H)-yl)-2-methoxyphenyl) ethynyl)-3-(morpholine-4-carbonyl)phenyl)methanesulfonamide (compound **IB-L2-1.11**). ¹H NMR (300 MHz, DMSO-d₆) δ ppm 1.36 (s, 9 H) 3.11 (s, 3 H) 3.19 (t, J=4.92 Hz, 2 H) 3.50 (t, J=4.95 Hz, 2 H) 3.65 (m, 4 H) 4.01 (s, 3 H) 5.65 (dd, J=7.91, 2.02 Hz, 1 H) 7.13 (d, J=1.84 Hz, 1 H) 7.29 (m, 2 H) 7.42 (d, J=2.57 Hz, 1 H) 7.62 (d, J=8.82 Hz, 1 H) 7.75 (d, J=8.09 Hz, 1 H) 10.28 (s, 1 H) 11.43 (d, J=1.56 Hz, 1 H).

N-(2-((3-tert-butyl-5-(2,4-dioxo-3,4-dihydropyrimidin-1(2H)-yl)-2-methoxyphenyl) ethynyl)-5-(methylsulfonamido)phenyl)acetamide (compound **IB-L2-1.12**). ¹H NMR (300 MHz, DMSO-d₆) δ ppm 1.36 (s, 9 H) 2.10 (s, 3 H) 3.07 (s, 3 H) 4.08 (s, 3 H) 5.66 (dd, J=7.72, 2.21 Hz, 1 H) 7.04 (dd, J=8.46, 2.21 Hz, 1 H) 7.31 (d, J=2.94 Hz, 1 H) 7.53 (m, 2 H) 7.67 (s, 1 H) 7.73 (d, J=8.09 Hz, 1 H) 9.50 (s, 1 H) 10.13 (s, 1 H) 11.44 (d, J=2.21 Hz, 1 H).

N-(4-((5-(2,4-dioxo-3,4-dihydropyrimidin-1(2H)-yl)-3-iodo-2-methoxyphenyl)ethynyl)-3-methylphenyl)methanesulfonamide (compound **IB-L2-1.15**). ¹H NMR (300 MHz, DMSO-d₆) δ ppm 2.46 (s, 3 H) 3.06 (s, 3 H) 3.95 (s, 3 H) 5.65 (dd, J=8.09, 1.47 Hz, 1 H) 7.09 (dd, J=8.46, 2.19 Hz, 1 H)7.15 (d, J=1.84 Hz, 1 H) 7.49 (d, J=8.46 Hz, 1 H) 7.67 (d, J=2.57 Hz, 1 H) 7.73 (d, J=7.72 Hz, 1 H) 7.94 (d, J=2.57 Hz, 1 H) 10.04 (s, 1 H) 11.47 (d, J=1.26 Hz, 1 H).

N-(4-((3-tert-butyl-5-(2,4-dioxo-3,4-dihydropyrimidin-1(2H)-yl)-2-methoxyphenyl) ethynyl)-naphthalen-1-yl)methanesulfonamide (compound **IB-L2-2.1**). ¹H NMR (300 MHz, DMSO-d₆) δ ppm 1.40 (s, 9 H) 3.08 (s, 3 H) 4.17 (s, 3 H) 5.67 (dd, J=7.91, 2.02 Hz, 1 H) 7.37 (d, J=2.57 Hz, 1 H) 7.58 (d, J=7.72 Hz, 1 H) 7.71 (m, 3 H) 7.78 (d, J=8.09 Hz, 1 H) 7.88 (d, J=7.72 Hz, 1 H) 8.35 (d, J=8.46 Hz, 1 H) 8.42 (d, J=8.09 Hz, 1 H) 10.00 (s, 1 H) 11.45 (d, J=1.84 Hz, 1 H).

3-tert-butyl-5-(2,4-dioxotetrahydropyrimidin-1(2H)-yl)-2-hydroxy-N-methyl-N-phenyl-benzamide (compound **IA-L3-1.1**) ¹H NMR (300 MHz, DMSO-d₆) δ 1.27 (s, 9 H) 2.53 - 2.61 (m, 5 H) 3.28 (t, J=6.80 Hz, 2 H) 6.69 (d, J=2.57 Hz, 1 H) 7.06 (d, J=2.57 Hz, 1 H) 7.16 - 7.25 (m, 3 H) 7.26 - 7.34 (m, 2 H) 10.22 (s, 1 H) 10.32 (s, 1 H).

3-tert-butyl-5-(2,4-dioxotetrahydropyrimidin-1(2H)-yl)-2-hydroxy-N-methyl-N-(4-(methyl-sulfonamido)phenyl)benzamide (compound **IA-L3-1.2**). ¹H NMR (300 MHz, DMSO-d₆) δ 1.28 (s, 9 H) 2.57 (t, J=6.62 Hz, 2 H) 2.95 (s, 3 H) 3.33 - 3.45 (m, 5 H) 6.68 (d, J=1.10 Hz, 1 H) 7.08 (d, J=8.82 Hz, 3 H) 7.14 - 7.19 (m, 2 H) 9.76 (s, 1 H) 10.21 (s, 1 H) 10.43 (s, 1 H).

3-tert-butyl-5-(2,4-dioxotetrahydropyrimidin-1(2H)-yl)-2-hydroxy-N-phenylbenzamide (compound **IA-L3-1.3**). ¹H NMR (300 MHz, DMSO d6) δ 1.39 (s, 9 H) 2.74 (t, J=6.80 Hz, 2 H) 3.79 (t, J=6.62 Hz, 2 H) 7.20 (t, J=7.35 Hz, 1 H) 7.35 - 7.47 (m, 3 H) 7.64 (d, J=7.35 Hz, 2 H) 7.91 (d, J=2.21 Hz, 1 H) 10.39 (s, 1 H) 10.45 (s, 1 H) 13.27 (s, 1 H).

3-tert-butyl-5-(2,4-dioxotetrahydropyrimidin-1(2H)-yl)-2-hydroxy-N-(4-(N-methyl methylsulfonamido)phenyl)benzamide (compound **IA-L3-1.4**). ¹H NMR (300 MHz, DMSO-d₆) δ ppm 1.39 (s, 9 H) 2.74 (t, *J*=6.62 Hz, 2 H) 2.96 (s, 3 H) 3.24 (s, 3 H) 3.79 (t, *J*=6.62 Hz, 2 H) 7.38 - 7.49 (m, 3 H) 7.69 (d, *J*=8.82 Hz, 2 H) 7.90 (d, *J*=2.57 Hz, 1 H) 10.39 (s, 1 H) 10.51 (s, 1 H) 13.21 (s, 1 H).

3-tert-butyl-5-(2,4-dioxotetrahydropyrimidin-1(2H)-yl)-2-hydroxy-N-(3-(methylsulfon amido) phenyl)benzamide (compound **IA-L3-1.5**). ¹H NMR (300 MHz, DMSO-d₆) δ 1.39 (s, 9 H) 2.74 (t, J=6.62 Hz, 2 H) 3.02 (s, 3 H) 3.79 (t, J=6.62 Hz, 2 H) 7.03 (d, J=7.72 Hz, 1 H) 7.30 - 7.47 (m, 3 H) 7.54 - 7.62 (m, 1 H) 7.90 (d, J=2.57 Hz, 1 H) 9.86 (s, 1 H) 10.39 (s, 1 H) 10.49 (s, 1 H) 13.20 (s, 1 H).

3-tert-butyl-5-(2,4-dioxotetrahydropyrimidin-1(2H)-yl)-2-hydroxy-N-(4-(2-methyl propylsulfonamido)phenyl)benzamide (compound **IA-L3-1.7**). ¹H NMR (300 MHz, DMSO-d₆) δ 1.00 (d, J=6.62 Hz, 6 H) 1.39 (s, 9 H) 2.14 (ddd, J=19.76, 13.14, 6.80 Hz, 1 H) 2.73 (t, J=6.62 Hz, 2 H) 2.98 (d, J=6.25 Hz, 2 H) 3.78 (t, J=6.62 Hz, 2 H) 7.22 (d, J=8.82 Hz, 2 H) 7.40 (d, J=2.21 Hz, 1 H) 7.59 (d, J=8.82 Hz, 2 H) 7.89 (d, J=2.21 Hz, 1 H) 9.80 (s, 1 H) 10.39 (s, 1 H) 10.43 (s, 1 H) 13.30 (s, 1 H).

3-tert-butyl-5-(2,4-dioxotetrahydropyrimidin-1(2H)-yl)-2-hydroxy-N-(4-(2,2,2-trifluoro ethylsulfonamido)phenyl)benzamide (compound **IA-L3-1.9**). ¹H NMR (300 MHz, DMSO-d₆) δ 1.39 (s, 9 H) 2.73 (t, J=6.62 Hz, 2 H) 3.78 (t, J=6.43 Hz, 2 H) 4.52 (q, J=9.44 Hz, 2 H) 7.25 (d, J=8.82 Hz, 2 H) 7.40 (d, J=1.84 Hz, 1 H) 7.62 (d, J=8.82 Hz, 2 H) 7.89 (d, J=2.21 Hz, 1 H) 10.39 (s, 1 H) 10.45 (d, J=2.57 Hz, 2 H) 13.28 (s, 1 H).

3-tert-butyl-5-(2,4-dioxotetrahydropyrimidin-1(2H)-yl)-2-hydroxy-N-(4-(phenylsulfonamido)phenyl)benzamide (compound **IA-L3-1.10**). ¹H NMR (300 MHz, DMSO-d₆) δ 1.37 (s, 9 H) 2.72 (t, J=6.62 Hz, 2 H) 3.76 (t, J=6.62 Hz, 2 H) 7.11 (d, J=8.82 Hz, 2 H) 7.38 (d, J=2.21 Hz, 1 H) 7.48 (d, J=8.82 Hz, 2 H) 7.52 - 7.64 (m, 3 H) 7.76 (d, J=6.62 Hz, 2 H) 7.83 (d, J=2.21 Hz, 1 H) 10.29 (s, 1 H) 10.37 (d, J=1.84 Hz, 2 H) 13.21 (s, 1 H).

3-tert-butyl-5-(2,4-dioxotetrahydropyrimidin-1(2H)-yl)-2-hydroxy-N-(3-(methylsulfonamidomethyl)phenyl)benzamide (compound **IA-L3-1.11**). ¹H NMR (300 MHz, DMSO-d₆) δ 1.39 (s, 9 H) 2.74 (t, J=6.62 Hz, 2 H) 2.89 (s, 3 H) 3.79 (t, J=6.62 Hz, 2 H) 4.18 (d, J=6.62 Hz, 2 H) 7.18 (d, J=7.72 Hz, 1 H) 7.35 - 7.43 (m, 2 H) 7.57 - 7.66 (m, 3 H) 7.93 (d, J=2.21 Hz, 1 H) 10.39 (s, 1 H) 10.49 (s, 1 H) 13.27 (s, 1 H).

3-tert-butyl-5-(2,4-dioxotetrahydropyrimidin-1(2H)-yl)-2-hydroxy-N-(4-(phenylmethylsulfonamido)phenyl)benzamide (compound **IA-L3-1.1**2). ¹H NMR (300 MHz, DMSO-d₆) δ 1.39 (s, 9 H) 2.74 (t, J=6.80 Hz, 2 H) 3.79 (t, J=6.62 Hz, 2 H) 4.47 (s, 2 H) 7.22 (d, J=8.82 Hz, 2 H) 7.26 - 7.33 (m, 2 H) 7.34 - 7.44 (m, 4 H) 7.60 (d, J=8.82 Hz, 2 H) 7.90 (d, J=1.10 Hz, 1 H) 9.87 (s, 1 H) 10.39 (s, 1 H) 10.44 (s, 1 H) 13.32 (s, 1 H).

3-tert-butyl-N-(4-(3,5-dimethylisoxazole-4-sulfonamido)phenyl)-5-(2,4-dioxotetrahydropyrimidin-1(2H)-yl)-2-hydroxybenzamide (compound **IA-L3-1.13**). ¹H NMR (300 MHz, DMSO-d₆) δ 1.38 (s, 9 H) 2.22 (s, 3 H) 2.44 (s, 3 H) 2.73 (t, J=6.62 Hz, 2 H) 3.77 (t, J=6.43 Hz, 2 H) 7.13 (d, J=8.82 Hz, 2 H) 7.40 (d, J=2.21 Hz, 1 H) 7.59 (d, J=8.82 Hz, 2 H) 7.87 (d, J=1.84 Hz, 1 H) 10.38 (s, 1 H) 10.40 (s, 1 H) 10.45 (s, 1 H) 13.19 (s, 1 H).

3-tert-butyl-5-(2,4-dioxotetrahydropyrimidin-1(2H)-yl)-2-hydroxy-N-(4-(2-morpholino ethylsulfonamido)phenyl)benzamide (compound **IA-L3-1.14**). ¹H NMR (300 MHz, DMSO-d₆) δ 1.39 (s, 9 H) 2.25 - 2.37 (m, 4 H) 2.63 - 2.81 (m, 4 H) 3.20 - 3.29 (m, 2 H) 3.44 - 3.53 (m, 4 H) 3.78 (t, J=6.80 Hz, 2 H) 7.25 (d, J=8.82 Hz, 2 H) 7.40 (d, J=2.21 Hz, 1 H) 7.59 (d, J=9.19 Hz, 2 H) 7.89 (d, J=2.57 Hz, 1 H) 9.83 (s, 1 H) 10.39 (s, 1 H) 10.44 (s, 1 H) 13.30 (s, 1 H).

2-(4-(3-tert-butyl-5-(2,4-dioxotetrahydropyrimidin-1(2H)-yl)-2-hydroxybenzamido) phenyl)-acetic acid (compound **IA-L3-1.15**). ¹H NMR (300 MHz, DMSO-d₆) δ 1.39 (s, 9 H) 2.73 (t, J=6.62 Hz, 2 H) 3.57 (s, 2 H) 3.78 (t, J=6.80 Hz, 2 H) 7.28 (d, J=8.46 Hz, 2 H) 7.39 (d, J=0.74 Hz, 1 H) 7.58 (d, J=8.46 Hz, 2 H) 7.90 (s, 1 H) 10.38 (s, 1 H) 10.44 (s, 1 H) 12.34 (s, 1 H) 13.31 (s, 1 H).

3-tert-butyl-5-(2,4-dioxotetrahydropyrimidin-1(2H)-yl)-2-hydroxy-N-(4-(N-methyl sulfamoylmethyl)phenyl)benzamide (compound **IA-L3-1.16**). ¹H NMR (300 MHz, DMSO-d₆) δ 2.50 (s, 9 H) 2.58 (d, J=5.15 Hz, 3 H) 2.74 (t, J=6.80 Hz, 2 H) 3.79 (t, J=6.62 Hz, 2 H) 4.33 (s, 2 H) 6.95 (q, J=4.78 Hz, 1 H) 7.36 - 7.44 (m, 3 H) 7.66 (d, J=8.82 Hz, 2 H) 7.91 (d, J=2.21 Hz, 1 H) 10.39 (s, 1 H) 10.49 (s, 1 H) 13.25 (s, 1 H).

3-tert-butyl-N-(4-(cyanomethoxy)phenyl)-5-(2,4-dioxotetrahydropyrimidin-1(2H)-yl)-2-hydroxybenzamide (compound **IA-L3-1.17**). ¹H NMR (300 MHz, DMSO-D6) δ ppm 1.39 (s, 9 H) 2.73 (t, J=6.62 Hz, 2 H) 3.79 (t, J=6.80 Hz, 2 H) 5.18 (s, 2 H) 7.08 (m, 2 H) 7.40 (d, J=2.57 Hz, 1 H) 7.60 (m, 2 H) 7.89 (d, J=2.21 Hz, 1 H) 10.39 (s, 1 H) 10.44 (s, 1 H) 13.33 (s, 1 H)

N-(4-(2-amino-2-oxoethoxy)phenyl)-3-tert-butyl-5-(2,4-dioxotetrahydropyrimidin-1(2H)-yl)-2-hydroxybenzamide (compound **IA-L3-1.18**). ¹H NMR (300 MHz, DMSO-D6) δ ppm 1.39 (s, 9 H) 2.73 (t, J=6.62 Hz, 2 H) 3.78 (t, J=6.80 Hz, 2 H) 4.43 (s, 2 H) 6.97 (m, 2 H) 7.40 (m, 2 H) 7.53 (m, 3 H) 7.89 (d, J=2.21 Hz, 1 H) 10.40 (m, 2 H) 13.41 (s, 1 H)

3-tert-butyl-5-(2,4-dioxotetrahydropyrimidin-1(2H)-yl)-2-hydroxy-N-(2-(methyl sulfonyl)phenyl)benzamide (compound **IA-L3-1.20**). ¹H NMR (300 MHz, DMSO-d₆) δ 1.39 (s, 9 H) 2.74 (t, J=6.62 Hz, 2 H) 3.27 (s, 3 H) 3.78 (t, J=6.62 Hz, 2 H) 7.45 (d, J=1.47 Hz, 1 H) 7.57 - 7.66 (m, 1 H) 7.77 (d, J=1.84 Hz, 1 H) 7.81 - 7.89 (m, 2 H) 8.02 (d, J=7.72 Hz, 1 H) 10.40 (s, 1 H) 10.64 (s, 1 H) 12.99 (s, 1 H).

3-tert-butyl-5-(2,4-dioxotetrahydropyrimidin-1(2H)-yl)-2-hydroxy-N-(4-(methyl sulfonyl)phenyl)benzamide (compound **IA-L3-1.21**). ¹H NMR (300 MHz, DMSO-d₆) δ 1.40 (s, 9 H) 2.74 (t, J=6.62 Hz, 2 H) 3.22 (s, 3 H) 3.79 (t, J=6.80 Hz, 2 H) 7.44 (d, J=2.21 Hz, 1 H) 7.92 (d, J=2.21 Hz, 1 H) 7.96 (s, 4 H) 10.41 (s, 1 H) 10.75 (s, 1 H) 12.89 (s, 1 H).

3-tert-butyl-5-(2,4-dioxotetrahydropyrimidin-1(2H)-yl)-2-hydroxy-N-(2-sulfamoyl phenyl)benzamide (compound **IA-L3-1.22**). ¹H NMR (300 MHz, DMSO-d₆) δ 1.40 (s, 9 H) 2.74 (t, J=6.62 Hz, 2 H) 3.78 (t, J=6.62 Hz, 2 H) 7.39 - 7.49 (m, 2 H) 7.61 - 7.74 (m, 4 H) 7.93 (d, J=8.09 Hz, 1 H) 8.01 (d, J=8.09 Hz, 1 H) 10.39 (s, 1 H) 10.42 (s, 1 H) 12.93 (s, 1 H).

3-tert-butyl-5-(2,4-dioxotetrahydropyrimidin-1(2H)-yl)-2-methoxy-N-phenylbenzamide (compound **IA-L3-1.24**). ¹H NMR (300 MHz, DMSO-d₆) δ 1.37 (s, 9 H) 2.71 (t, J=6.80 Hz, 2 H) 3.70 - 3.86 (m, 5 H) 7.10 (t, J=7.35 Hz, 1 H) 7.24 - 7.44 (m, 4 H) 7.73 (d, J=7.35 Hz, 2 H) 10.36 (s, 1 H) 10.39 (s, 1 H).

3-tert-butyl-N-[4-(methanesulfonyl-methyl-amino)-phenyl]-2-methoxy-5-(3-methyl-2,4-dioxotetrahydro-pyrimidin-1-yl)-benzamide (compound **IA-L3-1.25**). ¹H NMR (300 MHz, DMSO-D6) δ ppm 1.37 (s, 9 H) 2.84 (t, J=6.80 Hz, 2 H) 2.94 (s, 3 H) 3.04 (s, 3 H) 3.22 (s, 3 H) 3.71 - 3.81 (m, 5 H) 7.30 (d, J=2.57 Hz, 1 H) 7.35 (d, J=2.57 Hz, 1 H) 7.39 (d, J=8.82 Hz, 2 H) 7.75 (d, J=8.82 Hz, 2 H) 10.51 (s, 1 H)

3-tert-butyl-2-methoxy-5-(3-methyl-2,4-dioxotetrahydropyrimidin-1(2H)-yl)-N-(4-(N-methylmethylsulfonamido)phenyl)benzamide (compound **IA-L3-1.26**). ¹H NMR (300 MHz, DMSO-d₆) δ 1.37 (s, 9 H) 2.84 (t, J=6.80 Hz, 2 H) 2.94 (s, 3 H) 3.04 (s, 3 H) 3.22 (s, 3 H) 3.71 - 3.81 (m, 5 H) 7.30 (d, J=2.57 Hz, 1 H) 7.35 (d, J=2.57 Hz, 1 H) 7.39 (d, J=8.82 Hz, 2 H) 7.75 (d, J=8.82 Hz, 2 H) 10.51 (s, 1 H).

3-tert-butyl-5-(2,4-dioxotetrahydropyrimidin-1(2H)-yl)-N-(2-ethylphenyl)-2-methoxy benzamide (compound **IA-L3-1.28**).

2-(4-(3-tert-butyl-5-(2,4-dioxotetrahydropyrimidin-1(2H)-yl)-2-methoxybenzamido) phenyl)-acetic acid (compound **IA-L3-1.30**). ¹H NMR (300 MHz, DMSO-d₆) δ 1.37 (s, 9 H) 2.71 (t, J=6.62 Hz, 2 H) 3.53 (s, 2 H) 3.73 - 3.82 (m, 5 H) 7.23 (d, J=8.46 Hz, 2 H) 7.28 (d, J=2.57 Hz, 1 H) 7.33 (d, 1 H) 7.66 (d, J=8.46 Hz, 2 H) 10.35 (s, 1 H) 10.37 (s, 1 H) 12.29 (s, 1 H).

3-tert-butyl-5-(2,4-dioxotetrahydropyrimidin-1(2H)-yl)-2-methoxy-N-(4-(N-methyl sulfamoyl methyl)phenyl)benzamide (compound **IA-L3-1.31**).¹H NMR (300 MHz, DMSO-d₆) δ 1.37 (s, 9 H) 2.58 (d, J=4.78 Hz, 3 H) 2.71 (t, J=6.62 Hz, 2 H) 3.72 - 3.84 (m, 5 H) 4.29 (s, 2 H) 6.89 (q, J=4.78 Hz, 1 H) 7.29 (d, J=2.57 Hz, 1 H) 7.31 - 7.40 (m, 3 H) 7.72 (d, J=8.46 Hz, 2 H) 10.36 (s, 1 H) 10.46 (s, 1 H).

3-tert-butyl-5-(2,4-dioxo-tetrahydro-pyrimidin-1-yl)-2-methoxy-N-(4-trifluoromethyl-phenyl)-benzamide (compound **IA-L3-1.32**).

3-tert-butyl-5-(2,4-dioxotetrahydropyrimidin-1(2H)-yl)-N-(4-hydroxyphenyl)-2-methoxybenzamide (compound **IA-L3-1.33**). ¹H NMR (300 MHz, DMSO-d₆) δ ppm 1.37 (s, 9 H) 2.71 (t, J=6.62 Hz, 2 H) 3.76 (s, 3 H) 3.76 - 3.82 (m, 2 H) 6.73 (d, J=8.82 Hz, 2 H) 7.25 (d, J=2.57 Hz, 1 H) 7.31 (d, J=2.57 Hz, 1 H) 7.50 (d, J=9.19 Hz, 2 H) 9.25 (s, 1 H) 10.11 (s, 1 H) 10.35 (s, 1 H).

3-tert-butyl-5-(2,4-dioxo-tetrahydro-pyrimidin-1-yl)-2-methoxy-N-(2-methoxy-phenyl)-benzamide (compound **IA-L3-1.34**).

3-tert-butyl-5-(2,4-dioxo-tetrahydro-pyrimidin-1-yl)-2-methoxy-N-(3-methoxy-phenyl)-benzamide (compound **IA-L3-1.35**).

3-tert-butyl-5-(2,4-dioxo-tetrahydro-pyrimidin-1-yl)-2-methoxy-N-(4-methoxy-phenyl)-benzamide (compound **IA-L3-1.36**).

3-tert-butyl-5-(2,4-dioxo-tetrahydro-pyrimidin-1-yl)-N-(2-ethoxy-phenyl)-2-methoxybenzamide (compound **IA-L3-1.37**).

3-tert-butyl-N-(4-(cyanomethoxy)phenyl)-5-(2,4-dioxotetrahydropyrimidin-1(2H)-yl)-2-methoxybenzamide (compound **IA-L3-1.38**). ¹H NMR (300 MHz, DMSO-D6) δ ppm 1.37 (s, 9 H) 2.71 (t, J=6.62 Hz, 2 H) 3.76 (m, 5 H) 5.15 (s, 2 H) 7.07 (m, 2 H) 7.29 (d, J=2.94 Hz, 1 H) 7.33 (d, J=2.57 Hz, 1 H) 7.69 (m, 2 H) 10.35 (m, 2 H)

N-(4-(2-amino-2-oxoethoxy)phenyl)-3-tert-butyl-5-(2,4-dioxotetrahydropyrimidin-1(2H)-yl)-2-methoxybenzamide (compound **IA-L3-1.39**). ¹H NMR (300 MHz, DMSO-D6) δ ppm 1.37 (s, 9 H) 2.71 (t, J=6.80 Hz, 2 H) 3.78 (m, 5 H) 4.40 (s, 2 H) 6.95 (m, 2 H) 7.27 (d, J=2.57 Hz, 1 H) 7.32 (d, J=2.57 Hz, 1 H) 7.39 (s, 1 H) 7.51 (s, 1 H) 7.64 (m, 2 H) 10.26 (s, 1 H) 10.35 (s, 1 H)

3-tert-butyl-5-(2,4-dioxo-tetrahydro-pyrimidin-1-yl)-2-methoxy-N-(4-trifluoromethoxyphenyl)-benzamide (compound **IA-L3-1.40**).

4-(3-tert-butyl-5-(2,4-dioxotetrahydropyrimidin-1(2H)-yl)-2-methoxybenzamido)phenyl methanesulfonate (compound **IA-L3-1.41**). ¹H NMR (300 MHz, DMSO-d₆) δ 1.37 (s, 9 H) 2.72 (t, J=6.62 Hz, 2 H) 3.43 (s, 3 H) 3.73 - 3.83 (m, 5 H) 7.30 (d, J=2.94 Hz, 1 H) 7.33 - 7.39 (m, 3 H) 7.82 (d, J=8.82 Hz, 2 H) 10.36 (s, 1 H) 10.58 (s, 1 H).

N-(4-acetylphenyl)-3-tert-butyl-5-(2,4-dioxotetrahydropyrimidin-1(2H)-yl)-2-methoxybenzamide (compound **IA-L3-1.42**). ¹H NMR (300 MHz, DMSO d6) δ ppm 1.38 (s, 9 H) 2.71 (t, J=6.62 Hz, 2 H) 3.75 (s, 3 H) 3.78 (t, J=6.62 Hz, 2 H) 6.02 (s, 1 H) 6.55 (d, J=8.82 Hz, 1 H) 7.34 (dd, J=11.77, 2.57 Hz, 2 H) 7.66 (d, J=8.82 Hz, 1 H) 7.83 - 7.92 (m, 2 H) 7.92 - 8.04 (m, 2 H) 10.37 (s, 1 H) 10.75 (s, 1H).

ethyl 3-(4-(3-tert-butyl-5-(2,4-dioxotetrahydropyrimidin-1(2H)-yl)-2-methoxybenzamido) phenyl)-3-oxopropanoate (compound **IA-L3-1.43**). ¹H NMR (300 MHz, DMSO d6) δ ppm 1.19 (t, J=7.17 Hz, 3 H) 1.38 (s, 9 H) 2.71 (t, J=6.62 Hz, 2 H) 3.61 - 3.83 (m, 5 H) 4.06 - 4.20 (m, 4 H) 7.35 (dd, J=12.13, 2.57 Hz, 2 H) 7.84 - 7.91 (m, 2 H) 7.96 - 8.06 (m, 2 H) 10.37 (s, 1 H) 10.80 (s, 1 H).

3-tert-butyl-N-(3-carbamoyl-phenyl)-5-(2,4-dioxo-tetrahydro-pyrimidin-1-yl)-2-methoxybenzamide (compound **IA-L3-1.44**).

3-tert-butyl-5-(2,4-dioxo-tetrahydro-pyrimidin-1-yl)-N-(4-fluoro-phenyl)-2-methoxybenzamide (compound **IA-L3-1.45**).

3-tert-butyl-N-(4-chloro-phenyl)-5-(2,4-dioxo-tetrahydro-pyrimidin-1-yl)-2-methoxybenzamide (compound **IA-L3-1.46**).

N-(4-acetamidophenyl)-3-tert-butyl-5-(2,4-dioxotetrahydropyrimidin-1(2H)-yl)-2-methoxybenzamide (compound **IA-L3-1.47**). ¹H NMR (300 MHz, DMSO d6) δ 10.35 (s, 1 H), 10.31 (s, 1 H), 9.91 (s, 1 H), 7.59 - 7.72 (m, 2 H), 7.41 - 7.59 (m, 2 H), 7.33 (d, J=2.57 Hz, 1 H), 7.28 (d, J=2.94 Hz, 1 H), 3.58 - 3.93 (m, 5 H), 2.71 (t, J=6.62 Hz, 2 H), 2.03 (s, 3 H), 1.37 (s, 9 H).

2-(4-(3-tert-butyl-5-(2,4-dioxotetrahydropyrimidin-1(2H)-yl)-2-methoxybenzamido) phenylamino)-2-oxoethyl acetate (compound **IA-L3-1.48**). ¹H NMR (300 MHz, DMSO d6) δ 10.35 (s, 2 H), 10.06 (s, 1 H), 7.64 - 7.76 (m, 2 H), 7.54 (d, J=9.19 Hz, 1 H), 7.33 (d, J=2.57 Hz, 1 H), 7.28 (d, J=2.94 Hz, 1 H), 4.63 (s, 2 H), 3.60 - 3.90 (m, 5 H), 2.71 (t, J=6.62 Hz, 2 H), 2.12 (s, 3 H), 1.39 (s, 9 H).

Methyl 4-(3-tert-butyl-5-(2,4-dioxotetrahydropyrimidin-1(2H)-yl)-2-methoxybenzamido) phenylcarbamate (compound **IA-L3-1.49**). ¹H NMR (300 MHz, DMSO d6) δ 10.35 (s, 1 H), 10.28 (s, 1 H), 9.59 (s, 1 H), 7.62 (d, J=9.19 Hz, 2 H), 7.41 (d, J=8.82 Hz, 2 H), 7.32 (d, J=2.94 Hz, 1 H), 7.27 (d, J=2.57 Hz, 1 H), 3.69 - 3.88 (m, 5 H), 3.66 (s, 3 H), 2.71 (t, J=6.80 Hz, 2 H), 1.37 (s, 9 H).

Tert-butyl4-(3-tert-butyl-5-(2,4-dioxotetrahydropyrimidin-1(2H)-yl)-2-methoxybenzamido)-phenylcarbamate (compound **IA-L3-1.50**)**.** ¹H NMR (300 MHz, DMSO d6) δ 1.37 (s, 9 H) 1.47 (s, 9 H) 2.71 (t, J=6.62 Hz, 2 H) 3.71 - 3.84 (m, 5 H) 7.27 (d, J=2.57 Hz, 1 H) 7.32 (d, J=2.94 Hz, 1 H) 7.40 (d, J=8.82 Hz, 2 H) 7.59 (d, J=8.82 Hz, 2 H) 9.29 (s, 1 H) 10.25 (s, 1 H) 10.35 (s, 1 H).

3-tert-butyl-5-(2,4-dioxotetrahydropyrimidin-1(2H)-yl)-2-methoxy-N-(4-(2-methylpropylsulfonamido)phenyl)benzamide (compound **IA-L3-1.52**). ¹H NMR (300 MHz, DMSO d6) δ 0.99 (d, J=6.62 Hz, 6 H) 1.37 (s, 9 H) 2.03 - 2.25 (m, 1 H) 2.71 (t, J=6.80 Hz, 2 H) 2.94 (d, J=6.25 Hz, 2 H) 3.69 - 3.84 (m, 5 H) 7.18 (d, J=8.82 Hz, 2 H) 7.28 (d, J=2.57 Hz, 1 H) 7.33 (d, J=2.57 Hz, 1 H) 7.67 (d, J=8.82 Hz, 2 H) 9.66 (s, 1 H) 10.36 (s, 1 H) 10.37 (s, 1 H).

3-tert-butyl-5-(2,4-dioxotetrahydropyrimidin-1(2H)-yl)-N-(4-(2-hydroxyethylsulfonamido) phenyl)-2-methoxybenzamide (compound **IA-L3-1.53**)**.** ¹H NMR (300 MHz, DMSO-D6) δ 1.37 (s, 9 H) 2.71 (t, J=6.80 Hz, 2 H) 3.19 (t, J=6.80 Hz, 2 H) 3.69 - 3.86 (m, 7 H) 4.93 (t, J=5.70 Hz, 1 H) 7.20 (d, J=8.82 Hz, 2 H) 7.28 (d, J=2.94 Hz, 1 H) 7.33 (d, J=2.57 Hz, 1 H) 7.68 (d, J=8.82 Hz, 2 H) 9.60 (s, 1 H) 10.36 (s, 1 H) 10.38 (s, 1 H)

3-tert-butyl-5-(2,4-dioxotetrahydropyrimidin-1(2H)-yl)-2-methoxy-N-(4-(2-methoxyethyl sulfonamido)phenyl)benzamide (compound **IA-L3-1.54**). ¹H NMR (300 MHz, DMSO d6) δ 1.37 (s, 9 H) 2.71 (t, J=6.62 Hz, 2 H) 3.21 (s, 3 H) 3.25 - 3.31 (m, 2 H) 3.66 (t, J=6.07 Hz, 2 H) 3.71 - 3.83 (m, 5 H) 7.19 (d, J=8.82 Hz, 2 H) 7.28 (d, J=2.57 Hz, 1 H) 7.33 (d, J=2.57 Hz, 1 H) 7.67 (d, J=8.82 Hz, 2 H) 9.67 (s, 1 H) 10.35 (s, 1 H) 10.37 (s, 1 H).

3-tert-butyl-5-(2,4-dioxotetrahydropyrimidin-1(2H)-yl)-2-methoxy-N-(4-(2,2,2-trifluoroethylsulfonamido)phenyl)benzamide (compound **IA-L3-1.55**)**.** ¹H NMR (300 MHz, DMSO d6) δ 1.37 (s, 9 H) 2.71 (t, J=6.62 Hz, 2 H) 3.71 - 3.85 (m, 5 H) 4.46 (q, J=9.93 Hz, 2 H) 7.20 (d, J=8.82 Hz, 2 H) 7.28 (d, J=2.57 Hz, 1 H) 7.33 (d, J=2.57 Hz, 1 H) 7.70 (d, J=8.82 Hz, 2 H) 10.32 (s, 1 H) 10.36 (s, 1 H) 10.41 (s, 1 H).

N-(4-(2-(bis(2-hydroxyethyl)amino)ethylsulfonamido)phenyl)-3-tert-butyl-5-(2,4-dioxotetrahydropyrimidin-1(2H)-yl)-2-methoxybenzamide (compound **IA-L3-1.56**). ¹H NMR (300 MHz, DMSO d6) δ 1.37 (s, 9 H) 2.44 - 2.49 (m, 2 H) 2.71 (t, J=6.62 Hz, 2 H) 2.90 - 2.96 (m, 2 H) 3.18 (dd, J=9.01, 5.70 Hz, 2 H) 3.35 - 3.41 (m, 4 H) 3.73 - 3.81 (m, 5 H) 4.41 (s, 2 H) 7.20 (d, J=8.82 Hz, 2 H) 7.28 (d, J=2.57 Hz, 1 H) 7.33 (d, J=2.57 Hz, 1 H) 7.68 (d, J=9.19 Hz, 2 H) 9.65 (s, 1 H) 10.35 (s, 1 H) 10.38 (s, 1 H).

3-tert-butyl-5-(2,4-dioxotetrahydropyrimidin-1(2H)-yl)-2-methoxy-N-(4-(phenylmethylsulfonamido)phenyl)benzamide (compound **IA-L3-1.57**). ¹H NMR (300 MHz, DMSO-d₆) δ 1.38 (s, 9 H) 2.72 (t, J=6.62 Hz, 2 H) 3.72 - 3.84 (m, 5 H) 4.43 (s, 2 H) 7.18 (d, J=8.82 Hz, 2 H) 7.25 - 7.41 (m, 7 H) 7.68 (d, J=8.82 Hz, 2 H) 9.75 (s, 1 H) 10.36 (s, 1 H) 10.38 (s, 1 H).

3-tert-butyl-5-(2,4-dioxotetrahydropyrimidin-1(2H)-yl)-2-methoxy-N-(4-(2-morpholinoethyl sulfonamido)phenyl)benzamide (compound **IA-L3-1.58**). ¹H NMR (300 MHz, DMSO-d₆) δ 1.37 (s, 9 H) 2.28 - 2.35 (m, 4 H) 2.63 - 2.75 (m, 4 H) 3.20 - 3.28 (m, 2 H) 3.46 - 3.54 (m, 4 H) 3.72 - 3.82 (m, 5 H) 7.20 (d, J=8.82 Hz, 2 H) 7.28 (d, J=2.57 Hz, 1 H) 7.33 (d, J=2.57 Hz, 1 H) 7.68 (d, J=8.82 Hz, 2 H) 9.70 (s, 1 H) 10.35 (s, 1 H) 10.37 (s, 1 H).

3-tert-butyl-N-[4-(3,5-dimethyl-isoxazole-4-sulfonylamino)-phenyl]-5-(2,4-dioxo-tetrahydropyrimidin-1-yl)-2-methoxy-benzamide (compound **IA-L3-1.59**). ¹H NMR (300 MHz, DMSO-D6) δ ppm 1.38 (s, 9 H) 2.22 (s, 3 H) 2.44 (s, 3 H) 2.73 (t, J=6.62 Hz, 2 H) 3.77 (t, J=6.43 Hz, 2 H) 7.13 (d, J=8.82 Hz, 2 H) 7.40 (d, J=2.21 Hz, 1 H) 7.59 (d, J=8.82 Hz, 2 H) 7.87 (d, J=1.84 Hz, 1 H) 10.38 (s, 1 H) 10.40 (s, 1 H) 10.45 (s, 1 H) 13.19 (s, 1 H)

3-tert-butyl-5-(2,4-dioxotetrahydropyrimidin-1(2H)-yl)-2-methoxy-N-(4-(phenylsulfonamido) phenyl)benzamide (compound **IA-L3-1.60**). ¹H NMR (300 MHz, DMSO-d₆) δ 1.35 (s, 9 H) 2.70 (t, J=6.62 Hz, 2 H) 3.71 (s, 3 H) 3.75 (t, J=6.62 Hz, 2 H) 7.05 (d, J=8.82 Hz, 2 H) 7.24 (d, J=2.57 Hz, 1 H) 7.31 (d, J=2.57 Hz, 1 H) 7.51 - 7.64 (m, 5 H) 7.70 - 7.80 (m, 2 H) 10.17 (s, 1 H) 10.31 (s, 1 H) 10.34 (s, 1 H).

3-tert-butyl-5-(2,4-dioxotetrahydropyrimidin-1(2H)-yl)-2-methoxy-N-(4-(N-methylmethyl sulfonamido)phenyl)benzamide (compound **IA-L3-1.62**). ¹H NMR (300 MHz, DMSO-d₆) δ ppm 1.37 (s, 9 H) 2.71 (t, *J*=6.62 Hz, 2 H) 2.94 (s, 3 H) 3.22 (s, 3 H) 3.73 - 3.84 (m, 5 H) 7.29 (d, *J*=2.57 Hz, 1 H) 7.34 (d, *J*=2.57 Hz, 1 H) 7.40 (d, *J*=8.82 Hz, 2 H) 7.75 (d, *J*=8.82 Hz, 2 H) 10.36 (s, 1 H) 10.50 (s, 1 H).

3-tert-butyl-5-(2,4-dioxotetrahydropyrimidin-1(2H)-yl)-N-(4-(N-ethylmethylsulfonamido) phenyl)-2-methoxybenzamide (compound **IA-L3-1.63**). 1H NMR (300 MHz, DMSO d6) δ ppm 1.01 (t, J=6.99 Hz, 3 H) 1.38 (s, 9 H) 2.72 (t, J=6.62 Hz, 2 H) 2.97 (s, 3 H) 3.64 (d, J=7.35 Hz, 2 H) 3.74 - 3.83 (m, 5 H) 7.22 - 7.48 (m, 4 H) 7.77 (d, J=8.82 Hz, 2 H) 10.36 (s, 1 H) 10.53 (s, 1 H).

(N-(4-(3-tert-butyl-5-(2,4-dioxotetrahydropyrimidin-1(2H)-yl)-2-methoxybenzamido)phenyl) methylsulfonamido)methyl pivalate (compound **IA-L3-1.65**). ¹H NMR (300 MHz, DMSO-D6) δ ppm 1.19 (s, 9 H) 1.38 (s, 9 H) 2.71 (t, J=6.62 Hz, 2 H) 3.17 (s, 3 H) 3.74 - 3.82 (m, 5 H) 5.56 (s, 2 H) 7.30 (d, J=2.57 Hz, 1 H) 7.35 (d, J=2.57 Hz, 1 H) 7.41 (d, J=8.82 Hz, 2 H) 7.80 (d, J=8.82 Hz, 2 H) 10.36 (s, 1 H) 10.58 (s, 1 H)

3-tert-butyl-N-(4-(N-(cyclopropylmethyl)methylsulfonamido)phenyl)-5-(2,4-dioxotetrahydro pyrimidin-1(2H)-yl)-2-methoxybenzamide(compound **IA-L3-1.66**). ¹H NMR (300 MHz, DMSO-d₆) δ ppm 0.09 (d, *J*=4.78 Hz, 2 H) 0.40 (d, *J*=7.72 Hz, 2 H) 0.84 (d, 1 H) 1.38 (s, 9 H) 2.72 (t, *J*=6.43 Hz, 2 H) 2.97 (s, 3 H) 3.47 (d, *J*=6.62 Hz, 2 H) 3.68 - 3.88 (m, 5 H) 7.19 - 7.54 (m, 4 H) 7.77 (d, *J*=8.82 Hz, 2 H) 10.36 (s, 1 H) 10.53 (s, 1 H).

3-tert-butyl-5-(2,4-dioxotetrahydropyrimidin-1(2H)-yl)-2-methoxy-N-(4-(N-(4-methoxy-benzyl)methylsulfonamido)phenyl)benzamide (compound **IA-L3-1.67**). ¹H NMR (300 MHz, DMSO-d₆) δ ppm 1.36 (s, 9 H) 2.64 - 2.79 (m, 2 H) 3.06 (s, 3 H) 3.69 (s, 3 H) 3.76 (s, 3 H) 3.75 - 3.83 (m, 2 H) 4.75 (s, 2 H) 6.83 (d, J=8.46 Hz, 2 H) 7.16 (d, J=8.46 Hz, 2 H) 7.24 - 7.39 (m, 4 H) 7.65 (d, J=8.82 Hz, 2 H) 10.35 (s, 1 H) 10.45 (s, 1 H).

3-tert-butyl-5-(2,4-dioxotetrahydropyrimidin-1(2H)-yl)-2-methoxy-N-(4-(N-(methylsulfonyl) propionamido)phenyl)benzamide (compound **IA-L3-1.70**). ¹H NMR (300 MHz, DMSO-D6) δ ppm 0.93 (t, J=7.35 Hz, 3 H) 1.38 (s, 9 H) 2.12 (q, J=7.35 Hz, 2 H) 2.72 (t, J=6.62 Hz, 2 H) 3.52 (s, 3 H) 3.78 (m, 5 H) 7.31 (d, J=2.57 Hz, 1 H) 7.36 (d, J=2.57 Hz, 1 H) 7.44 (d, J=8.46 Hz, 2 H) 7.83 (d, J=8.82 Hz, 2 H) 10.37 (s, 1 H) 10.64 (s, 1 H)

3-tert-butyl-5-(2,4-dioxotetrahydropyrimidin-1(2H)-yl)-2-methoxy-N-(4-(N-(methylsulfonyl) butyramido)phenyl)benzamide (compound **IA-L3-1.71**). ¹H NMR (300 MHz, DMSO-D6) δ ppm 0.79 (t, J=7.35 Hz, 3 H) 1.38 (s, 9 H) 1.48 (m, 2 H) 2.09 (t, J=7.17 Hz, 2 H) 2.72 (t, J=6.62 Hz, 2 H) 3.52 (s, 3 H) 3.78 (m, 5 H) 7.32 (d, J=2.57 Hz, 1 H) 7.36 (d, J=2.57 Hz, 1 H) 7.43 (d, J=8.82 Hz, 2 H) 7.83 (d, J=8.82 Hz, 2 H) 10.37 (s, 1 H) 10.65 (s, 1 H)

3-tert-butyl-5-(2,4-dioxotetrahydropyrimidin-1(2H)-yl)-2-methoxy-N-(4-(N-(methylsulfonyl) isobutyramido)phenyl)benzamide (compound **IA-L3-1.72**). ¹H NMR (300 MHz, DMSO-D6) δ ppm 0.99 (d, J=6.62 Hz, 6 H) 1.38 (s, 9 H) 2.29 - 2.41 (m, 1 H) 2.72 (t, J=6.62 Hz, 2 H) 3.51 (s, 3 H) 3.75 - 3.83 (m, 5 H) 7.32 (d, J=2.57 Hz, 1 H) 7.36 (d, J=2.57 Hz, 1 H) 7.48 (d, J=8.82 Hz, 2 H) 7.84 (d, J=8.82 Hz, 2 H) 10.37 (s, 1 H) 10.66 (s, 1 H)

Methyl 4-(3-tert-butyl-5-(2,4-dioxotetrahydropyrimidin-1(2H)-yl)-2-methoxybenzamido) phenyl(methylsulfonyl)carbamate (compound **IA-L3-1.73**). ¹H NMR (300 MHz, DMSO-D6) δ ppm 1.38 (s, 9 H) 2.72 (t, J=6.62 Hz, 2 H) 3.56 (s, 3 H) 3.72 (s, 3 H) 3.74 - 3.84 (m, 5 H) 7.31 (d, J=2.57 Hz, 1 H) 7.35 (dd, J=5.70, 2.76 Hz, 3 H) 7.77 (d, J=8.82 Hz, 2 H) 10.37 (s, 1 H) 10.58 (s, 1 H)

Ethyl 4-(3-tert-butyl-5-(2,4-dioxotetrahydropyrimidin-1(2H)-yl)-2-methoxybenzamido)-phenyl(methylsulfonyl)carbamate (compound **IA-L3-1.74**). ¹H NMR (300 MHz, DMSO-D6) δ ppm 1.16 (t, J=7.17 Hz, 3 H) 1.38 (s, 9 H) 2.71 (t, J=6.62 Hz, 2 H) 3.56 (s, 3 H) 3.73 - 3.84 (m, 5 H) 4.20 (q, J=6.99 Hz, 2 H) 7.26 - 7.41 (m, 4 H) 7.77 (d, J=8.82 Hz, 2 H) 10.37 (s, 1 H) 10.58 (s, 1 H)

Isobutyl 4-(3-tert-butyl-5-(2,4-dioxotetrahydropyrimidin-1(2H)-yl)-2-methoxybenzamido) phenyl(methylsulfonyl)carbamate (compound **IA-L3-1.76**). ¹H NMR (300 MHz, DMSO-D6) δ ppm 0.79 (d, J=6.99 Hz, 6 H) 1.38 (s, 9 H) 1.74 - 1.92 (m, 1 H) 2.71 (t, J=6.62 Hz, 2 H) 3.56 (s, 3 H) 3.73 - 3.83 (m, 5 H) 3.94 (d, J=6.62 Hz, 2 H) 7.29 - 7.41 (m, 4 H) 7.78 (d, J=8.82 Hz, 2 H) 10.36 (s, 1 H) 10.58 (s, 1 H)

2-methoxyethyl 4-(3-tert-butyl-5-(2,4-dioxotetrahydropyrimidin-1(2H)-yl)-2-methoxy benzamido)phenyl(methylsulfonyl)carbamate (compound **IA-L3-1.77**). ¹H NMR (300 MHz, DMSO-D6) δ ppm 1.38 (s, 9 H) 2.71 (t, J=6.62 Hz, 2 H) 3.19 (s, 3 H) 3.46 - 3.52 (m, 2 H) 3.57 (s, 3 H) 3.72 - 3.84 (m, 5 H) 4.28 (dd, J=5.52, 3.68 Hz, 2 H) 7.27 - 7.42 (m, 4 H) 7.78 (d, J=8.82 Hz, 2 H) 10.36 (s, 1 H) 10.58 (s, 1 H)

3-tert-butyl-5-(2,4-dioxotetrahydropyrimidin-1(2H)-yl)-2-methoxy-N-(4-(methylsulfonyl) phenyl)benzamide (compound **IA-L3-1.78**). ¹H NMR (300 MHz, DMSO-d₆) δ 1.38 (s, 9 H) 2.72 (t, J=6.62 Hz, 2 H) 3.19 (s, 3 H) 3.74 (s, 3 H) 3.78 (t, J=6.62 Hz, 2 H) 7.35 (dd, 2 H) 7.95 (dd, 4 H) 10.37 (s, 1 H) 10.86 (s, 1 H).

3-tert-butyl-5-(2,4-dioxotetrahydropyrimidin-1(2H)-yl)-2-methoxy-N-(2-(methylsulfonyl) phenyl)benzamide (compound **IA-L3-1.79**). ¹H NMR (300 MHz, DMSO-d₆) δ 1.39 (s, 9 H) 2.72 (t, J=6.43 Hz, 2 H) 3.29 (s, 3 H) 3.77 - 3.85 (m, 5 H) 7.43 (d, J=2.57 Hz, 1 H) 7.45 - 7.49 (m, 1 H) 7.50 (d, J=2.57 Hz, 1 H) 7.77 - 7.85 (m, 1 H) 7.95 (d, J=8.46 Hz, 1 H) 8.45 (d, J=8.46 Hz, 1 H) 10.39 (s, 2 H).

3-tert-butyl-5-(2,4-dioxotetrahydropyrimidin-1(2H)-yl)-2-methoxy-N-(2-sulfamoylphenyl) benzamide (compound **IA-L3-1.80**). ¹H NMR (300 MHz, DMSO-d₆) δ 1.38 (s, 9 H) 2.72 (t, J=6.62 Hz, 2 H) 3.73 - 3.89 (m, 5 H) 7.34 (t, J=7.72 Hz, 1 H) 7.42 (d, J=2.94 Hz, 1 H) 7.49 (d, J=2.57 Hz, 1 H) 7.66 (t, J=7.17 Hz, 1 H) 7.72 (s, 2 H) 7.91 (d, J=8.09 Hz, 1 H) 8.48 (d, J=8.09 Hz, 1 H) 10.31 (s, 1 H) 10.39 (s, 1 H).

3-tert-butyl-5-(2,4-dioxo-tetrahydro-pyrimidin-1-yl)-N-(3-hydroxy-2-methyl-phenyl)-2-methoxy-benzamide (compound **IA-L3-1.81**).

3-tert-butyl-5-(2,4-dioxo-tetrahydro-pyrimidin-1-yl)-N-(4-fluoro-2-methyl-phenyl)-2-methoxy-benzamide (compound **IA-L3-1.82**).

3-tert-butyl-5-(2,4-dioxotetrahydropyrimidin-1(2H)-yl)-2-methoxy-N-(2-methyl-4-(methyl sulfonamido)phenyl)benzamide (compound **IA-L3-1.83**). ¹H NMR (300 MHz, DMSO-D6) δ ppm 1.38 (s, 9 H) 2.26 (s, 3 H) 2.72 (t, J=6.62 Hz, 2 H) 2.98 (s, 3 H) 3.79 (t, J=6.62 Hz, 2 H) 3.83 (s, 3 H) 7.04 (m, 2 H) 7.33 (m, 2 H) 7.47 (d, J=8.09 Hz, 1 H) 9.65 (s, 1 H) 9.78 (s, 1 H) 10.36 (s, 1 H)

N-(3-tert-butyl-5-(2,4-dioxotetrahydropyrimidin-1(2H)-yl)phenyl)-4-(methylsulfonamido) benzamide (compound **IA-L3-1.84**). ¹H NMR (300 MHz, DMSO-D6) δ ppm 1.38 (s, 9 H) 2.72 (t, J=6.62 Hz, 2 H) 3.09 (s, 3 H) 3.79 (t, J=6.62 Hz, 2 H) 3.84 (s, 3 H) 7.33 (d, J=2.21 Hz, 1 H) 7.37 (d, J=2.57 Hz, 1 H) 7.55 (m, 2 H) 7.67 (d, J=8.82 Hz, 1 H) 10.03 (s, 1 H) 10.18 (s, 1 H) 10.38 (s, 1 H)

3-tert-butyl-5-(2,4-dioxotetrahydropyrimidin-1(2H)-yl)-2-methoxy-N-(2-methoxy-4-(methyl sulfonamido)phenyl)benzamide (compound **IA-L3-1.85**). ¹H NMR (300 MHz, DMSO-d₆) δ 1.39 (s, 9 H) 2.68 - 2.76 (m, 2 H) 3.00 (s, 3 H) 3.75 - 3.87 (m, 8 H) 6.84 (dd, J=8.46, 2.21 Hz, 1 H) 6.94 (d, J=2.21 Hz, 1 H) 7.39 (d, J=2.94 Hz, 1 H) 7.47 (d, J=2.57 Hz, 1 H) 7.93 - 7.99 (m, 1 H) 9.59 (s, 1 H) 9.68 (s, 1 H) 10.37 (s, 1 H).

N-(5-acetylamino-2-methoxy-phenyl)-3-tert-butyl-5-(2,4-dioxo-tetrahydro-pyrimidin-1-yl)-2-methoxy-benzamide (compound **IA-L3-1.86**).

3-tert-butyl-5-(2,4-dioxotetrahydropyrimidin-1(2H)-yl)-2-methoxy-N-(4-(methylsulfon-amido)-3-nitrophenyl)benzamide (compound **IA-L3-1.87**). ¹H NMR (300 MHz, DMSO-d₆) δ 10.84 (s, 1 H) 10.37 (s, 1 H) 9.71 (s, 1 H) 8.52 (d, J=2.57 Hz, 1 H) 7.98 (dd, J=9.01, 2.39 Hz, 1 H) 7.62 (d, J=8.82 Hz, 1 H) 7.35 (dd, J=12.50, 2.57 Hz, 2 H) 3.71 - 3.83 (m, 5 H) 3.11 (s, 3 H) 2.71 (t, J=6.62 Hz, 2 H) 1.38 (s, 9 H).

butyric acid ({4-[3-tert-butyl-5-(3-butyryloxymethyl-2,4-dioxo-tetrahydro-pyrimidin-1-yl)-2-methoxy-benzoylamino]-phenyl}-methanesulfonyl-amino)-methyl ester (compound **IA-L3-1.88**). ¹H NMR (300 MHz, DMSO-D6) δ ppm 0.88 (m, 6 H) 1.38 (s, 9 H) 1.55 (m, 4 H) 2.26 (t, J=7.17 Hz, 2 H) 2.39 (t, J=7.17 Hz, 2 H) 2.95 (t, J=6.62 Hz, 2 H) 3.14 (s, 3 H) 3.77 (s, 3 H) 3.81 (t, J=6.62 Hz, 2 H) 5.57 (s, 2 H) 5.68 (s, 2 H) 7.35 (d, J=2.57 Hz, 1 H) 7.38 (d, J=2.94 Hz, 1 H) 7.42 (d, J=8.82 Hz, 2 H) 7.79 (d, J=8.82 Hz, 2 H) 10.60 (s, 1 H).

3-tert-butyl-5-(2,4-dioxotetrahydropyrimidin-1(2H)-yl)-2-hydroxy-N-(4-(methylsulfonamido methyl)-3-sulfamoylthiophen-2-yl)benzamide (compound **IA-L3-1.89**). ¹H NMR (300 MHz, DMSO-d₆) δ 1.40 (s, 9 H) 2.70 - 2.78 (m, 2 H) 2.97 (s, 3 H) 3.78 (t, J=6.43 Hz, 2 H) 4.36 (d, J=6.99 Hz, 2 H) 7.14 (s, 1 H) 7.42 (d, J=2.21 Hz, 1 H) 7.47 (d, J=2.21 Hz, 1 H) 7.60 (t, J=6.43 Hz, 1 H) 7.82 (s, 2 H) 10.42 (s, 1 H) 11.43 (s, 1 H) 11.93 (s, 1 H).

3-tert-butyl-5-(2,4-dioxotetrahydropyrimidin-1(2H)-yl)-2-methoxy-N-(4-(methylsulfonamide methyl)-3-sulfamoylthiophen-2-yl)benzamide (compound **IA-L3-1.90**). ¹H NMR (300 MHz, DMSO-d₆) δ 1.40 (s, 9 H) 2.72 (t, J=6.62 Hz, 2 H) 2.97 (s, 3 H) 3.73 (s, 3 H) 3.80 (t, J=6.80 Hz, 2 H) 4.36 (d, J=5.52 Hz, 2 H) 7.06 (s, 1 H) 7.47 (d, J=2.57 Hz, 1 H) 7.57 (t, J=6.43 Hz, 1 H 7.61 (d, J=2.57 Hz, 1 H) 7.70 (s, 2 H) 10.40 (s, 1 H) 11.56 (s, 1 H).

3-tert-butyl-5-(2,4-dioxotetrahydropyrimidin-1(2H)-yl)-2-hydroxy-N-(4-(methylsulfonamido methyl)thiophen-2-yl)benzamide (compound **IA-L3-1.91**). ¹H NMR (300 MHz, DMSO-d₆) δ ppm 1.39 (s, 9 H) 2.74 (t, J=6.80 Hz, 2 H) 2.86 (s, 3 H) 3.79 (t, J=6.62 Hz, 2 H) 4.10 (d, J=6.25 Hz, 2 H) 6.98 (s, 1 H) 7.04 (d, J=1.47 Hz, 1 H) 7.42 (d, J=2.57 Hz, 1 H) 7.55 (t, J=6.25 Hz, 1 H) 7.89 (d, J=2.21 Hz, 1 H) 10.41 (s, 1 H) 11.68 (s, 1 H) 12.95 (s, 1 H).

3-tert-butyl-5-(2,4-dioxotetrahydropyrimidin-1(2H)-yl)-2-methoxy-N-(4-(methylsulfonamido methyl)thiophen-2-yl)benzamide (compound **IA-L3-1.94**). ¹H NMR (300 MHz, DMSO-d₆) δ ppm 1.37 (s, 9 H) 2.71 (t, J=6.80 Hz, 2 H) 2.85 (s, 3 H) 3.67 (s, 3 H) 3.78 (t, J=6.62 Hz, 2 H) 4.07 (d, J=5.88 Hz, 2 H) 6.82 (d, J=1.84 Hz, 1 H) 6.87 (s, 1 H) 7.28 - 7.32 (m, 1 H) 7.36 (d, J=2.57 Hz, 1 H) 7.49 (t, J=6.25 Hz, 1 H) 10.37 (s, 1 H) 11.59 (s, 1 H).

3-tert-butyl-5-(2,4-dioxotetrahydropyrimidin-1(2H)-yl)-2-hydroxy-N-(4-((N-methylmethyl sulfonamido)methyl)thiophen-2-yl)benzamide (compound **IA-L3-1.95**). ¹H NMR (300 MHz, DMSO-d₆) δ ppm 1.39 (s, 9 H) 2.70 (s, 3 H) 2.74 (t, J=6.80 Hz, 2 H) 2.92 (s, 3 H) 3.79 (t, J=6.80 Hz, 2 H) 4.18 (s, 2 H) 7.01 (m, 2 H) 7.42 (d, J=2.21 Hz, 1 H) 7.89 (d, J=2.57 Hz, 1 H) 10.41 (s, 1 H) 11.68 (s, 1 H) 12.92 (s, 1 H).

tert-butyl (5-(3-tert-butyl-5-(2,4-dioxotetrahydropyrimidin-1(2H)-yl)-2-methoxybenzamido) thiophen-3-yl)methyl(methylsulfonyl)carbamate (compound **IA-L3-1.96**). ¹H NMR (300 MHz, DMSO-d₆) δ ppm 1.37 (s, 9 H) 1.48 (s, 9 H) 2.71 (t, J=6.62 Hz, 2 H) 3.30 (s, 3 H) 3.67 (s, 3 H) 3.78 (t, J=6.62 Hz, 2 H) 4.66 (s, 2 H) 6.77 - 6.87 (m, 2 H) 7.30 (d, J=2.57 Hz, 1 H) 7.36 (d, J=2.57 Hz, 1 H) 10.37 (s, 1 H) 11.63 (s, 1 H).

3-tert-butyl-5-(2,4-dioxotetrahydropyrimidin-1(2H)-yl)-2-methoxy-N-(4-((N-methylmethyl sulfonamido)methyl)thiophen-2-yl)benzamide (compound **IA-L3-1.97**). ¹H NMR (300 MHz, DMSO-d₆) δ ppm 1.37 (s, 9 H) 2.70 (m, 5 H) 2.91 (s, 3 H) 3.68 (s, 3 H) 3.78 (t, J=6.62 Hz, 2 H) 4.15 (s, 2 H) 6.80 (d, J=1.47 Hz, 1 H) 6.94 (d, J=1.47 Hz, 1 H) 7.31 (d, J=2.57 Hz, 1 H) 7.36 (d, J=2.94 Hz, 1 H) 10.37 (s, 1 H) 11.59 (s, 1 H).

tert-butyl (5-(3-tert-butyl-5-(2,4-dioxotetrahydropyrimidin-1(2H)-yl)-2-hydroxybenzamido) thiophen-3-yl)methyl(methylsulfonyl)carbamate (compound **IA-L3-1.98**). ¹H NMR (300 MHz, DMSO-d₆) δ ppm 1.39 (s, 9 H) 1.47 (s, 9 H) 2.74 (t, J=6.80 Hz, 2 H) 3.35 (s, 3 H) 3.78 (t, J=6.62 Hz, 2 H) 4.68 (s, 2 H) 6.96 (s, 1 H) 7.04 (d, J=1.47 Hz, 1 H) 7.41 (d, J=2.21 Hz, 1 H) 7.88 (d, J=2.21 Hz, 1 H) 10.40 (s, 1 H) 11.70 (s, 1 H) 12.92 (s, 1 H).

3-tert-butyl-5-(2,4-dioxotetrahydropyrimidin-1(2H)-yl)-2-methoxy-N-(6-(methylsulfonamido) pyridin-3-yl)benzamide (compound **IA-L3-1.99**). ¹H NMR (300 MHz, DMSO-d₆) δ ppm 1.37 (s, 9 H) 2.71 (t, J=6.62 Hz, 2 H) 3.28 (s, 3 H) 3.77 (m, 5 H) 7.01 (d, J=8.82 Hz, 1 H) 7.31 (d, J=2.57 Hz, 1 H) 7.35 (d, J=2.57 Hz, 1 H) 8.07 (dd, J=8.82, 2.57 Hz, 1 H) 8.61 (d, J=2.57 Hz, 1 H) 10.36 (s, 1 H) 10.49 (s, br, 1 H) 10.50 (s, 1 H).

N-(6-aminopyridin-3-yl)-3-tert-butyl-5-(2,4-dioxotetrahydropyrimidin-1(2H)-yl)-2-methoxy benzamide (compound **IA-L3-1.100**). ¹H NMR (300 MHz, DMSO-d₆) δ ppm 1.37 (s, 9 H) 2.71 (t, J=6.62 Hz, 2 H) 3.74 (s, 3 H) 3.78 (t, J=6.62 Hz, 2 H) 7.06 (d, J=9.56 Hz, 1 H) 7.31 (d, J=2.57 Hz, 1 H) 7.37 (d, J=2.57 Hz, 1 H) 7.99 (s, 2 H) 8.04 (dd, J=9.56, 2.21 Hz, 1 H) 8.53 (d, J=1.84 Hz, 1 H) 10.38 (s, 1 H) 10.67 (s, 1 H) 13.64 (s, 1 H).

3-tert-butyl-5-(2,4-dioxotetrahydropyrimidin-1(2H)-yl)-2-methoxy-N-(5-(N-(methylsulfonyl) methylsulfonamido)pyridin-2-yl)benzamide (compound **IA-L3-1.101**). ¹H NMR (300 MHz, DMSO-d₆) δ ppm 1.38 (s, 9 H) 2.72 (t, J=6.62 Hz, 2 H) 3.58 (s, 6 H) 3.75 (s, 3 H) 3.80 (t, J=6.62 Hz, 2 H) 7.36 (m, 2 H) 8.06 (dd, J=8.82, 2.57 Hz, 1 H) 8.30 (d, J=8.82 Hz, 1 H) 8.51 (d, J=2.21 Hz, 1 H) 10.37 (s, 1 H) 11.10 (s, 1 H).

3-tert-butyl-5-(2,4-dioxotetrahydropyrimidin-1(2H)-yl)-2-methoxy-N-(5-(methylsulfonamido) pyridin-2-yl)benzamide (compound **IA-L3-1.102**). ¹H NMR (300 MHz, DMSO-d₆) δ ppm 1.37 (s, 9 H) 2.71 (t, J=6.62 Hz, 2 H) 3.02 (s, 3 H) 3.74 (s, 3 H) 3.79 (t, J=6.62 Hz, 2 H) 7.35 (m, 2 H) 7.71 (dd, J=8.82, 2.57 Hz, 1 H) 8.18 (m, 2 H) 9.81 (s, 1 H) 10.36 (s, 1 H) 10.74 (s, 1 H).

3-tert-butyl-5-(2,4-dioxotetrahydropyrimidin-1(2H)-yl)-2-methoxy-N-((1r,4r)-4-(methylsulfon amido)cyclohexyl)benzamide (compound **IA-L3-1.103**). ¹H NMR (300 MHz, DMSO-d₆) δ ppm 1.32 (d, 11 H) 1.91 (s, 2 H) 2.65 - 2.78 (m, 4 H) 2.91 (s, 3 H) 3.07 (s, 2 H) 3.39 (s, 2 H) 3.66 - 3.80 (m, 5 H) 7.03 (d, J=7.35 Hz, 1 H) 7.12 (d, J=2.57 Hz, 1 H) 7.25 (d, J=2.57 Hz, 1 H) 8.19 (d, J=8.09 Hz, 1 H) 10.32 (s, 1 H).

3-tert-butyl-5-(2,4-dioxotetrahydropyrimidin-1(2H)-yl)-2-hydroxy-N-(thiazol-2-yl)benzamide (compound **IA-L3-1.104**). ¹H NMR (300 MHz, DMSO-d₆) δ 1.56 - 1.68 (m, 9 H) 2.92 (t, J=6.62 Hz, 2 H) 3.90 - 4.07 (m, 2 H) 7.27 - 7.70 (m, 2 H) 7.80 (d, J=4.04 Hz, 1 H) 8.03 (s, 1 H) 10.51 (s, 1 H) 14.06 (d, J=116.92 Hz, 1 H).

3-tert-butyl-5-(2,4-dioxotetrahydropyrimidin-1(2H)-yl)-N-(4-(methylsulfonamido)phenyl) benzamide (compound **IA-L3-1.105**). ¹H NMR (300 MHz, DMSO-d₆) δ ppm 1.34 (s, 9 H) 2.74 (t, J=6.62 Hz, 2 H) 2.96 (s, 3 H) 3.86 (t, J=6.62 Hz, 2 H) 7.09 - 7.90 (m, 7 H) 9.62 (s, 1 H) 10.24 (s, 1 H) 10.43 (s, 1 H).

N-(4-aminophenyl)-3-tert-butyl-5-(2,4-dioxotetrahydropyrimidin-1(2H)-yl)-2-methoxy benzamide (compound **IA-L3-1.107**). ¹H NMR (300 MHz, DMSO-d₆) δ 1.37 (s, 9 H) 2.71 (t, J=6.62 Hz, 2 H) 3.71 - 3.83 (m, 5 H) 7.26 - 7.39 (m, 4 H) 7.82 (d, J=8.82 Hz, 2 H) 9.95 (s, 1 H) 10.36 (s, 1 H) 10.57 (s, 1 H).

N-(4-(N-allylmethylsulfonamido)phenyl)-3-tert-butyl-5-(2,4-dioxotetrahydropyrimidin-1 (2H)-yl)-2-methoxybenzamide (compound **IA-L3-1.108**). ¹H NMR (300 MHz, DMSO-d₆) δ ppm 1.37 (s, 9 H) 2.71 (t, J=6.62 Hz, 2 H) 3.01 (s, 3 H) 3.70 - 3.83 (m, 5 H) 4.25 (d, J=5.88 Hz, 2 H) 5.00 - 5.24 (m, 2 H) 5.68 - 5.84 (m, 1 H) 7.29 (d, J=2.57 Hz, 1 H) 7.30 - 7.41 (m, 3 H) 7.74 (d, J=8.82 Hz, 2 H) 10.36 (s, 1 H) 10.51 (s, 1 H).

5-(2,4-dioxotetrahydropyrimidin-1(2H)-yl)-3-isopropyl-2-methoxy-N-(4-(methylsulfonamido) phenyl)benzamide (compound **IA-L3-1.111**). ¹H NMR (500 MHz, DMSO-d₆) δ ppm 1.22 (d, *J*=6.71 Hz, 6 H) 2.72 (t, *J*=6.71 Hz, 2 H) 2.95 (s, 3 H) 3.23 - 3.39 (m, 1 H) 3.75 (s, 3 H) 3.79 (t, *J*=6.71 Hz, 2 H) 7.19 (d, *J*=9.16 Hz, 2 H) 7.31 (d, *J*=2.44 Hz, 1 H) 7.38 (d, *J*=2.44 Hz, 1 H) 7.69 (d, *J*=9.16 Hz, 2 H) 9.55 (s, 1 H) 10.29 (s, 1 H) 10.34 (s, 1H).

5-(2,4-dioxotetrahydropyrimidin-1(2H)-yl)-3-ethyl-2-methoxy-N-(4-(methylsulfonamido) phenyl)benzamide (compound **IA-L3-1.112**). ¹H NMR (300 MHz, DMSO-d₆) δ ppm 1.20 (t, *J*=7.35 Hz, 3 H) 2.60 - 2.78 (m, 4 H) 2.95 (s, 3 H) 3.69 - 3.84 (m, 5 H) 7.19 (d, *J*=9.19 Hz, 2 H) 7.27 - 7.41 (m, 2 H) 7.69 (d, *J*=8.82 Hz, 2 H) 9.59 (s, 1 H) 10.31 (s, 1 H) 10.38 (s, 1 H).

5-(2,4-dioxotetrahydropyrimidin-1(2H)-yl)-3-ethyl-2-hydroxy-N-(4-(methylsulfonamido) phenyl)benzamide (compound **IA-L3-1.113**). ¹H NMR (300 MHz, DMSO-d₆) δ ppm 1.17 (t, *J*=7.54 Hz, 3 H) 2.54 - 2.66 (m, 2 H) 2.73 (t, *J*=6.62 Hz, 2 H) 2.99 (s, 3 H) 3.78 (t, *J*=6.62 Hz, 2 H) 7.24 (d, *J*=8.82 Hz, 2 H) 7.37 (d, *J*=1.84 Hz, 1 H) 7.62 (d, *J*=8.82 Hz, 2 H) 7.87 (d, *J*=2.21 Hz, 1 H) 9.72 (s, 1 H) 10.40 (s, 1 H) 10.43 (s, 1 H) 12.70 (s, 1 H).

5-(2,4-dioxotetrahydropyrimidin-1(2H)-yl)-2-hydroxy-N-(4-(methylsulfonamido)phenyl) benzamide (compound **IA-L3-1.114**). ¹H NMR (300 MHz, DMSO-d₆) δ ppm 2.72 (t, *J*=6.80 Hz, 2 H) 2.97 (s, 3 H) 3.76 (t, *J*=6.80 Hz, 2 H) 6.99 (d, *J*=8.82 Hz, 1 H) 7.22 (d, *J*=9.19 Hz, 2 H) 7.40 (dd, *J*=8.82, 2.57 Hz, 1 H) 7.65 (d, *J*=8.82 Hz, 2 H) 7.87 (d, *J*=2.57 Hz, 1 H) 9.66 (s, 1 H) 10.38 (d, *J*=1.84 Hz, 2 H) 11.83 (s, 1 H).

3-tert-butyl-5-(3-(2-(ethylamino)-2-oxoethyl)-2,4-dioxotetrahydropyrimidin-1(2H)-yl)-N-(4-(N-(2-(ethylamino)-2-oxoethyl)methylsulfonamido)phenyl)-2-methoxybenzamide (compound **IA-L3-1.115**).¹H NMR (300 MHz, DMSO-d₆) δ ppm 0.92 - 1.04 (m, 6 H) 1.37 (s, 9 H) 2.88 (t, J=6.62 Hz, 2 H) 3.01 - 3.08 (m, 4 H) 3.09 (s, 3 H) 3.75 (s, 3 H) 3.81 (t, J=6.62 Hz, 2 H) 4.22 (d, J=5.52 Hz, 4 H) 7.30 (d, J=2.57 Hz, 1 H) 7.31 - 7.37 (m, 1 H) 7.47 (d, J=8.82 Hz, 2 H) 7.74 (d, J=8.82 Hz, 2 H) 7.88 - 8.04 (m, 2 H) 10.53 (s, 1 H).

(3-(3-tert-butyl-4-methoxy-5-(4-(N-(pivaloyloxymethyl)methylsulfonamido)phenylcarbamoyl) phenyl)-2,6-dioxotetrahydropyrimidin-1(2H)-yl)methyl pivalate (compound **IA-L3-1.116**). ¹H NMR (300 MHz, DMSO-d₆) δ ppm 1.11 (s, 9 H) 1.19 (s, 9 H) 1.38 (s, 9 H) 2.95 (t, J=6.62 Hz, 2 H) 3.17 (s, 3 H) 3.77 (s, 3 H) 3.81 (t, J=6.62 Hz, 2 H) 5.56 (s, 2 H) 5.67 (s, 2 H) 7.35 (d, J=2.57 Hz, I H) 7.38 (d, J=2.57 Hz, 1 H) 7.41 (d, J=9.19 Hz, 2 H) 7.80 (d, J=8.82 Hz, 2 H) 10.61 (s, 1 H).

5-(3-((1,3-dioxolan-2-yl)methyl)-2,4-dioxotetrahydropyrimidin-1(2H)-yl)-3-tert-butyl-2-methoxy-N-(4-(methylsulfonamido)phenyl)benzamide (compound **IA-L3-1.117**). ¹H NMR (300 MHz, DMSO-d₆) δ ppm 1.37 (s, 9 H) 2.87 (t, J=6.62 Hz, 2 H) 2.95 (s, 3 H) 3.78 (s, 3 H) 3.76 - 3.85 (m, 4 H) 3.87 - 3.96 (m, 4 H) 5.07 (t, J=4.96 Hz, 1 H) 7.20 (d, J=8.82 Hz, 2 H) 7.32 (dd, J=13.97, 2.57 Hz, 2 H) 7.69 (d, J=8.82 Hz, 2 H) 9.60 (s, 1 H) 10.40 (s, 1 H).

5-(3-allyl-2,4-dioxotetrahydropyrimidin-1(2H)-yl)-N-(4-(N-allylmethylsulfonamido)phenyl)-3-tert-butyl-2-methoxybenzamide (compound **IA-L3-1.118**). ¹H NMR (300 MHz, DMSO-d₆) δ ppm 1.37 (s, 9 H) 2.78 - 2.93 (m, 2 H) 3.01 (s, 3 H) 3.78 (s, 3 H) 3.76 - 3.85 (m, 2 H) 4.25 (d, J=5.88 Hz, 4 H) 4.94 - 5.29 (m, 4 H) 5.67 - 5.96 (m, 1 H) 7.00 - 7.21 (m, 1 H) 7.31 (d, J=2.57 Hz, 1 H) 7.35 - 7.42 (m, 3 H) 7.74 (d, J=8.82 Hz, 2 H) 10.52 (s, 1 H).

3-tert-butyl-5-(2,4-dioxotetrahydropyrimidin-1(2H)-yl)-2-hydroxy-N-(2-methoxy-4-(methyl sulfonamido)phenyl)benzamide (compound **IA-L3-1.119**). ¹H NMR (300 MHz, DMSO-d₆) δ 1.38 (s, 9 H) 2.73 (t, J=6.62 Hz, 2 H) 3.05 (s, 3 H) 3.74 - 3.81 (m, 5 H) 6.84 (dd, J=8.46, 2.21 Hz, 1 H) 6.95 (d, J=2.21 Hz, 1 H) 7.30 (d, J=8.46 Hz, 1 H) 7.39 (d, J=2.21 Hz, 1 H) 7.89 (d, J=2.57 Hz, 1 H) 9.82 (s, 1 H) 10.06 (s, 1 H) 10.37 (s, 1 H) 13.50 (s, 1 H).

3-tert-butyl-5-(2,4-dioxotetrahydropyrimidin-1(2H)-yl)-2-methoxy-N-(3-(methylsulfonamido methyl)phenyl)benzamide (compound **IA-L3-1.120**). ¹H NMR (300 MHz, DMSO-d₆) δ 1.37 (s, 9 H) 2.71 (t, J=6.62 Hz, 2 H) 2.89 (s, 3 H) 3.69 - 3.88 (m, 5 H) 4.15 (d, J=6.25 Hz, 2 H) 7.00 - 7.16 (m, 1 H) 7.26 - 7.40 (m, 3 H) 7.59 (t, J=6.43 Hz, 1 H) 7.65 (d, J=8.82 Hz, 1 H) 7.74 (s, 1 H) 10.36 (s, 1 H) 10.44 (s, 1 H).

3-tert-butyl-N-(4-(2,5-dimethoxyphenylsulfonamido)phenyl)-5-(2,4-dioxotetrahydro-pyrimidin-1(2H)-yl)-2-methoxybenzamide (compound **IA-L3-1.121**). 1H NMR (300 MHz, DMSO d6) δ 1.35 (s, 9 H) 2.69 (t, J=6.80 Hz, 2 H) 3.71 (s, 6 H) 3.75 (t, J=6.62 Hz, 2 H) 3.85 (s, 3 H) 7.06 (d, J=8.82 Hz, 2 H) 7.13 (s, 2 H) 7.22 (dd, J=3.68, 2.21 Hz, 2 H) 7.31 (d, J=2.57 Hz, 1 H) 7.53 (d, J=8.82 Hz, 2 H) 9.87 (s, 1 H) 10.28 (s, 1 H) 10.34 (s, 1 H).

3-tert-butyl-5-(2,4-dioxotetrahydropyrimidin-1(2H)-yl)-N-(4-(2-hydroxyethylsulfonamido) phenyl)-2-methoxybenzamide (compound **IA-L3-1.122**). ¹H NMR (300 MHz, DMSO-d₆) δ 1.37 (s, 9 H) 2.71 (t, J=6.80 Hz, 2 H) 3.19 (t, J=6.80 Hz, 2 H) 3.69 - 3.86 (m, 7 H) 4.93 (t, J=5.70 Hz, 1 H) 7.20 (d, J=8.82 Hz, 2 H) 7.28 (d, J=2.94 Hz, 1 H) 7.33 (d, J=2.57 Hz, 1 H) 7.68 (d, J=8.82 Hz, 2 H) 9.60 (s, 1 H) 10.36 (s, 1 H) 10.38 (s, 1 H).

(N-(4-(3-tert-butyl-5-(2,4-dioxotetrahydropyrimidin-1(2H)-yl)-2-methoxybenzamido)phenyl) methylsulfonamido)methyl butyrate (compound **IA-L3-1.123**). ¹H NMR (300 MHz, DMSO-d6) δ 0.90 (t, J=7.54 Hz, 3 H) 1.38 (s, 9 H) 1.57 (m, 2 H) 2.39 (t, J=7.35 Hz, 2 H) 2.71 (t, J=6.62 Hz, 2 H) 3.14 (s, 3 H) 3.77 (m, 5 H) 5.57 (s, 2 H) 7.30 (d, J=2.57 Hz, 1 H) 7.35 (d, J=2.57 Hz, 1 H) 7.41 (d, J=8.82 Hz, 2 H) 7.79 (d, J=8.82 Hz, 2 H) 10.36 (s, 1 H) 10.57 (s, 1 H)

3-tert-butyl-5-(2,4-dioxotetrahydropyrimidin-1(2H)-yl)-2-hydroxy-N-(quinolin-6-yl) benzamide (compound **IA-L3-2.1**).¹H NMR (300 MHz, DMSO-d₆) δ 1.41 (s, 9 H) 2.75 (t, J=6.62 Hz, 2 H) 3.77 - 3.86 (m, 2 H) 7.45 (d, J=2.21 Hz, 1 H) 7.68 (dd, J=8.46, 4.41 Hz, 1 H) 7.98 (d, J=2.57 Hz, 1 H) 8.11 (s, 2 H) 8.44 (s, 1 H) 8.58 (d, J=8.46 Hz, 1 H) 8.96 (dd, J=4.41, 1.47 Hz, 1 H) 10.41 (s, 1 H) 10.81 (s, 1 H).

3-tert-butyl-5-(2,4-dioxotetrahydropyrimidin-1(2H)-yl)-2-methoxy-N-(2-oxoindolin-5-yl) benzamide (compound **IA-L3-2.2**). ¹H NMR (300 MHz, DMSO-d₆) δ 1.37 (s, 9 H) 2.71 (t, J=6.62 Hz, 2 H) 3.50 (s, 2 H) 3.75 (s, 3 H) 3.77 (t, J=6.62 Hz, 2 H) 6.78 (d, J=8.46 Hz, 1 H) 7.26 (d, J=2.21 Hz, 1 H) 7.32 (d, J=2.57 Hz, 1 H) 7.48 (dd, J=8.46, 1.84 Hz, 1 H) 7.64 (s, 1 H) 10.24 (s, 1 H) 10.34 (s, 1 H) 10.35 (s, 1 H).

3-tert-butyl-N-(2,2-dioxo-1,3-dihydrobenzo[c]thiophen-5-yl)-5-(2,4-dioxotetrahydro pyrimidin-1(2H)-yl)-2-methoxybenzamide (compound **IA-L3-2.3**). ¹H NMR (300 MHz, DMSO-d₆) δ 1.37 (s, 9 H) 2.71 (t, J=6.43 Hz, 2 H) 3.70 - 3.85 (m, 5 H) 4.45 (s, 2 H) 4.53 (s, 2 H) 7.29 (d, J=2.57 Hz, 1 H) 7.32 - 7.40 (m, 2 H) 7.62 (dd, J=8.27, 1.65 Hz, 1 H) 7.86 (s, 1 H) 10.36 (s, 1 H) 10.54 (s, 1 H).

3-tert-butyl-5-(2,4-dioxotetrahydropyrimidin-1(2H)-yl)-2-methoxy-N-(2-oxo-1,2,3,4-tetra hydroquinolin-6-yl)benzamide (compound **IA-L3-2.4**). ¹H NMR (300 MHz, DMSO-d₆) δ 1.37 (s, 9 H) 2.44 (t, 2 H) 2.71 (t, J=6.62 Hz, 2 H) 2.86 (t, J=7.35 Hz, 2 H) 3.77 (t, 2 H) 3.75 (s, 3 H) 6.82 (d, J=8.46 Hz, 1 H) 7.26 (d, J=2.57 Hz, 1 H) 7.32 (d, J=2.57 Hz, 1 H) 7.45 (dd, J=8.46, 2.21 Hz, 1 H) 7.57 (d, 1 H) 10.05 (s, 1 H) 10.24 (s, 1 H) 10.35 (s, 1 H).

3-tert-butyl-N-(2,2-dioxo-1,3-dihydrobenzo[c]thiophen-5-yl)-5-(2,4-dioxotetrahydro pyrimidin-1(2H)-yl)-2-hydroxybenzamide (compound **IA-L3-2.5**).¹H NMR (300 MHz, DMSO-d₆) δ 1.39 (s, 9 H) 2.74 (t, J=6.80 Hz, 2 H) 3.79 (t, J=6.62 Hz, 2 H) 4.49 (s, 2 H) 4.55 (s, 2 H) 7.42 (dd, J=4.96, 2.76 Hz, 2 H) 7.59 (dd, J=8.27, 1.65 Hz, 1 H) 7.74 (s, 1 H) 7.90 (d, J=1.84 Hz, 1 H) 10.40 (s, 1 H) 10.54 (s, 1 H) 13.15 (s, I H).

3-tert-butyl-5-(2,4-dioxo-3,4-dihydropyrimidin-1(2H)-yl)-2-methoxy-N-(4-(methylsulfon amido)phenyl)benzamide (compound **IB-L3-1.1**). ¹H NMR (300 MHz, DMSO-d₆) δ ppm 2.50 (s, 9 H) 2.94 (s, 3 H) 3.79 (s, 3 H) 5.66 (d, J=8.09 Hz, 1 H) 7.14 - 7.25 (m, 2 H) 7.39 (s, 2 H) 7.62 - 7.75 (m, 3 H) 9.60 (s, 1 H) 10.44 (s, 1 H) 11.42 (s, 1 H).

3-tert-butyl-5-(2,4-dioxo-3,4-dihydropyrimidin-1(2H)-yl)-2-hydroxy-N-(4-(methylsulfon-amide) phenyl)benzamide (compound **IB-L3-1.2**). ¹H NMR (300 MHz, DMSO-d6) δ 1.40 (s, 9 H) 2.99 (s, 3 H) 5.70 (dd, J=7.72, 2.21 Hz, 1 H) 7.24 (d, J=8.82 Hz, 2 H) 7.46 (d, J=2.21 Hz, 1 H) 7.61 (d, J=8.82 Hz, 2 H) 7.76 (d, J=7.72 Hz, 1 H) 8.03 (d, J=2.21 Hz, 1 H) 9.75 (s, 1 H) 10.45 (s, 1 H) 11.48 (d, J=2.21 Hz, 1 H) 13.52 (s, 1 H)

3-tert-butyl-2-methoxy-5-(6-methyl-2,4-dioxo-3,4-dihydropyrimidin-1(2H)-yl)-N-(4-(methyl sulfonamido)phenyl)benzamide (compound **IB-L3-1.3**). ¹H NMR (300 MHz, DMSO-d₆) δ ppm 1.37 (s, 9 H) 1.82 (s, 3 H) 2.96 (s, 3 H) 3.80 (s, 3 H) 5.63 (s, 1 H) 7.20 (d, J=8.82 Hz, 2 H) 7.34 (s, 2 H) 7.69 (d, J=8.82 Hz, 2 H) 9.60 (s, 1 H) 10.41 (s, 1 H) 11.27 (s, 1 H).

3-tert-butyl-2-hydroxy-5-(6-methyl-2,4-dioxo-3,4-dihydropyrimidin-1(2H)-yl)-N-(4-(methyl sulfonamido)phenyl)benzamide (compound **IB-L3-1.4**). ¹H NMR (300 MHz, DMSO-d₆) δ ppm 1.39 (s, 9 H) 1.86 (s, 3 H) 2.99 (s, 3 H) 5.68 (s, 1 H) 7.24 (d, *J*=8.82 Hz, 2 H) 7.40 (s, 1 H) 7.61 (d, *J*=8.82 Hz, 2 H) 7.95 (s, 1 H) 9.74 (s, 1 H) 10.39 (s, 1 H) 11.35 (s, 1 H) 13.57 (s, 1 H).

N-(3-tert-butyl-5-(2,4-dioxotetrahydropyrimidin-1(2H)-yl)phenyl)-4-(methylsulfonamido) benzamide (compound **IA-L4-1.1**). ¹H NMR (300 MHz, DMSO-d6) δ 1.29 (s, 9 H) 2.72 (t, *J*=6.62 Hz, 2 H) 3.09 (s, 3 H) 3.79 (t, *J*=6.62 Hz, 2 H) 7.08 (t, *J*=1.84 Hz, 1 H) 7.30 (d, *J*=8.82 Hz, 2 H) 7.67 (dd, *J*=6.99, 1.84 Hz, 2 H) 7.95 (d, *J*=8.82 Hz, 2 H) 10.16 (s, 1 H) 10.19 (s, 1 H) 10.35 (s, 1 H)

N-(3-tert-butyl-5-(2,4-dioxotetrahydropyrimidin-1(2H)-yl)-2-hydroxyphenyl)benzamide (compound *IA-L4-1.2*). ¹H NMR (300 MHz, DMSO-d₆) δ 10.28 (s, 1 H) 10.17 (s, 1 H) 8.99 (s, 1 H) 8.04 (d, J=6.99 Hz, 2 H) 7.49 - 7.67 (m, 3 H) 7.22 (d, J=2.57 Hz, 1 H) 7.06 (d, J=2.57 Hz, 1 H) 3.73 (t, J=6.80 Hz, 2 H) 2.70 (t, J=6.80 Hz, 2 H) 1.40 (s, 9 H).

N-(3-tert-butyl-5-(2,4-dioxotetrahydropyrimidin-1(2H)-yl)-2-hydroxyphenyl)-4-(methylsulfon amido)benzamide (compound **IA-L4-1.3**). ¹H NMR (300 MHz, DMSO-d₆) δ 10.29 (s, 1 H) 10.25 (br s, 1 H) 10.11 (s, 1 H) 9.02 (s, 1 H) 8.02 (d, J=8.82 Hz, 2 H) 7.30 (d, J=8.82 Hz, 2 H) 7.19 (d, J=2.21 Hz, 1 H) 7.06 (d, J=2.57 Hz, 1 H) 3.73 (t, J=6.80 Hz, 2 H) 3.10 (s, 3 H) 2.71 (t, J=6.62 Hz, 2 H) 1.39 (s, 9 H).

N-(3-tert-butyl-5-(2,4-dioxotetrahydropyrimidin-1(2H)-yl)-2-hydroxyphenyl)-4-(methyl sulfonylmethyl)benzamide (compound **IA-L4-1.4**). ¹H NMR (300 MHz, DMSO-d₆) δ 1.40 (s, 9 H) 2.70 (t, J=6.62 Hz, 2 H) 2.95 (s, 3 H) 3.73 (t, J=6.80 Hz, 2 H) 4.61 (s, 2 H) 7.07 (d, J=2.57 Hz, 1 H) 7.21 (d, J=2.57 Hz, 1 H) 7.57 (d, J=8.46 Hz, 2 H) 8.05 (d, J=8.09 Hz, 2 H) 8.95 (s, 1 H) 10.16 (s, 1 H) 10.29 (s, 1 H).

N-(3-tert-butyl-5-(2,4-dioxotetrahydropyrimidin-1(2H)-yl)-2-hydroxyphenyl)-4-nitro benzamide (compound **IA-L4-1.5**). ¹H NMR (300 MHz, DMSO-d₆) δ 10.28 (s, 1 H) 10.26 (s, 1 H) 8.91 (s, 1 H) 8.38 (d, J=8.82 Hz, 2 H) 8.26 (d, J=9.20 Hz, 2 H) 7.19 (d, J=2.57 Hz, 1 H) 7.09 (d, J=2.57 Hz, 1 H) 3.73 (t, J=6.62 Hz, 2 H) 2.70 (t, J=6.80 Hz, 2 H) 1.40 (s, 9 H).

4-amino-N-(3-tert-butyl-5-(2,4-dioxotetrahydropyrimidin-1(2H)-yl)-2-hydroxyphenyl) benzamide (compound **IA-L4-1.6**). ¹H NMR (300 MHz, DMSO-d₆) δ 10.29 (s, 1 H) 9.95 (s, 1 H) 9.46 (s, 1 H) 7.79 (d, J=8.82 Hz, 2 H) 7.16 (d, J=2.57 Hz, 1 H) 7.03 (d, J=2.21 Hz, 1 H) 6.61 (d, J=8.46 Hz, 2 H) 5.90 (s, 2 H) 3.72 (t, J=6.80 Hz, 2 H) 2.70 (t, J=6.62 Hz, 2 H) 1.39 (s, 9 H).

N-(3-tert-butyl-5-(2,4-dioxotetrahydropyrimidin-1(2H)-yl)-2-methoxyphenyl)-N-methyl-4-(methylsulfonamido)benzamide (compound **IA-L4-1.7**). ¹H NMR (300 MHz, DMSO-d₆) δ 10.36 (s, 1 H) 9.81 (s, 1 H) 7.36 (d, J=2.21 Hz, 1 H) 7.15 (d, J=8.46 Hz, 2 H) 7.05 (d, J=2.21 Hz, 1 H) 6.92 (d, J=8.46 Hz, 2 H) 3.65 - 3.90 (m, 2 H) 3.41 (s, 3 H) 3.17 (d, J=5.52 Hz, 3 H) 2.88 (s, 3 H) 2.66 - 2.76 (m, 2 H) 1.03 (s, 9 H).

N-(3-tert-butyl-5-(2,4-dioxotetrahydropyrimidin-1(2H)-yl)-2-methoxyphenyl)benzamide (compound **IA-L4-1.8**). ¹H NMR (300 MHz, DMSO-d₆) δ 10.33 (s, 1 H) 9.98 (s, I H) 8.00 - 8.07 (m, 2 H) 7.49 - 7.64 (m, 3 H) 7.38 (d, J=2.57 Hz, 1 H) 7.13 (d, J=2.57 Hz, 1 H) 3.77 (t, J=6.62 Hz, 2 H) 3.72 (s, 3 H) 2.71 (t, J=6.62 Hz, 2 H) 1.37 (s, 9 H).

N-(3-tert-butyl-5-(2,4-dioxotetrahydropyrimidin-1(2H)-yl)-2-methoxyphenyl)-2-methoxy-4-(methylsulfonamido)benzamide (compound **IA-L4-1.11**). ¹H NMR (300 MHz, DMSO-d₆) δ 10.33 (s, 1 H) 10.31 (s, 1 H) 10.20 (s, 1 H) 8.21 (d, J=2.57 Hz, 1 H) 8.02 (d, J=8.82 Hz, 1 H) 7.01 - 7.07 (m, 2 H) 6.96 (dd, J=8.46, 1.84 Hz, 1 H) 4.03 - 4.07 (m, 3 H) 3.79 - 3.82 (m, 3 H) 3.76 (t, J=6.80 Hz, 2 H) 3.14 (s, 3 H) 2.71 (t, J=6.62 Hz, 2 H) 1.39 (s, 9 H).

N-(3-tert-butyl-5-(2,4-dioxotetrahydropyrimidin-1(2H)-yl)-2-methoxyphenyl)-2-chloro-4-(methylsulfonamido)benzamide (compound **IA-L4-1.12**). ¹H NMR (300 MHz, DMSO-d₆) δ 10.33 (s, 1 H) 10.23 (s, 1 H) 10.02 (s, 1 H) 7.52 - 7.65 (m, 2 H) 7.20 - 7.33 (m, 2 H) 7.09 (d, J=2.57 Hz, 1 H) 3.71 - 3.82 (m, 5 H) 3.11 (s, 3 H) 2.71 (t, J=6.62 Hz, 2 H) 1.35 (s, 9 H).

N-(3-tert-butyl-5-(2,4-dioxo-3,4-dihydropyrimidin-1(2H)-yl)-2-methoxyphenyl)-2-methoxy-4-(methylsulfonamido)benzamide (compound **IB-L4-1.1**). ¹H NMR (300 MHz, DMSO-d₆) δ 1.40 (s, 9 H) 3.14 (s, 3 H) 3.84 (s, 3 H) 4.06 (s, 3 H) 5.65 (dd, J=7.72, 2.21 Hz, 1 H) 6.96 (dd, J=8.46, 1.84 Hz, 1 H) 7.04 (d, J=1.84 Hz, 1 H) 7.09 (d, J=2.94 Hz, 1 H) 7.71 (d, J=7.72 Hz, 1 H) 8.01 (d, J=8.82 Hz, 1 H) 8.28 (d, J=2.57 Hz, 1 H) 10.27 (s, 1 H) 10.32 (s, 1 H) 11.41 (d, J=2.21 Hz, 1 H).

{1-[3-tert-butyl-5-(2,4-dioxo-tetrahydro-pyrimidin-1-yl)-2-hydroxy-benzyl]-piperidin-4-yl}-carbamic acid tert-butyl ester (compound **IA-L5-1-1.1**). ¹H NMR (300 MHz, CDCl₃) δ ppm 7.36 (s, 1 H) 7.03 (d, *J*=2.94 Hz, 1 H) 6.77 (d, *J*=2.57 Hz, 1 H) 4.43 (s, 1 H) 3.79 (t, *J*=6.62 Hz, 2 H) 3.66 (s, 2 H) 3.44-3.61 (m, 1 H) 2.88-3.01 (m, 1 H) 2.81 (t, *J*=6.62 Hz, 2 H) 2.22 (s, 2 H) 1.98 (s, 2 H) 1.44 (s, 9 H) 1.39 (s, 9 H) 1.28-1.71 (m, 2 H)

N-{1-[3-tert-butyl-5-(2,4-dioxo-tetrahydro-pyrimidin-1-yl)-2-hydroxy-benzyl]-piperidin-3-ylmethyl}-methanesulfonamide (compound **IA-L5-1-1.2**). ¹H NMR (300 MHz, CDCl₃) δ ppm 7.45 (s, 1 H) 7.03 (d, *J*=2.57 Hz, 1 H) 6.78 (d, *J*=2.21 Hz, 1 H) 4.37 (s, 1 H) 3.81 (t, *J*=6.80 Hz, 2 H) 3.58-3.73 (m, 2 H) 3.07 (s, 2 H) 2.92 (s, 3 H) 2.81 (t, *J*=6.62 Hz, 2 H) 1.72-1.95 (m, 4 H) 1.49-1.72 (m, 4 H) 1.39 (s, 9 H)

1-[3-tert-butyl-5-(2,4-dioxo-tetrahydro-pyrimidin-1-yl)-2-hydroxy-benzyl]-piperidine-3-carboxylic acid ethyl ester (compound **IA-L5-1-1.3**). ¹H NMR (300 MHz, CDCl₃) δ ppm 7.39 (s, 1 H) 7.03 (d, *J*=2.57 Hz, 1 H) 6.78 (d, *J*=2.57 Hz, 1 H) 4.09-4.22 (m, 2 H) 3.79 (t, *J*=6.62 Hz, 2 H) 3.67 (s, 2 H) 3.05 (s, 1 H) 2.81 (t, *J*=6.80 Hz, 2 H) 2.51-2.69 (m, 1 H) 2.38 (s, 1 H) 2.15 (s, 1 H) 1.88-2.07 (m, 1 H) 1.70-1.85 (m, 1 H) 1.46-1.69 (m, 3 H) 1.39 (s, 9 H) 1.21-1.30 (m, 3 H)

1-[3-tert-butyl-4-hydroxy-5-(3-methyl-piperidin-1-ylmethyl)-phenyl]-dihydro-pyrimidine-2,4-dione; compound with trifluoroacetic acid (compound **IA-L5-1-1.4**).

1-[3-tert-butyl-5-(2,4-dioxo-tetrahydro-pyrimidin-1-yl)-2-hydroxy-benzyl]-piperidine-4-carboxylic acid methyl ester (compound **IA-L5-1-1.5**). ¹H NMR (300 MHz, CDCl₃) δ ppm 7.38 (s, 1 H) 7.03 (d, *J*=2.57 Hz, 1 H) 6.77 (d, *J*=2.57 Hz, 1 H) 3.79 (t, *J*=6.80 Hz, 2 H) 3.69 (s, 3 H) 3.66 (s, 2 H) 2.97 (s, 2 H) 2.80 (t, *J*=6.80 Hz, 2 H) 2.30-2.46 (m, 1 H) 2.17 (s, 2 H) 1.91-2.03 (m, 2 H) 1.83 (s, 2 H) 1.39 (s, 9 H)

1-[3-tert-butyl-4-hydroxy-5-((R)-3-hydroxy-piperidin-1-ylmethyl)-phenyl]-dihydro-pyrimidine-2,4-dione; compound with trifluoroacetic acid (compound **IA-L5-1-1.6**).

1-[3-tert-butyl-5-(2,4-dioxo-tetrahydro-pyrimidin-1-yl)-2-hydroxy-benzyl]-piperidine-3-carboxylic acid diethylamide, trifluoroacetic acid salt (compound **IA-L5-1-1.7**).

1-[3-tert-butyl-5-(2,4-dioxo-tetrahydro-pyrimidin-1-yl)-2-hydroxy-benzyl]-piperidine-3-carboxylic acid amide, trifluoroacetic acid salt (compound **IA-L5-1-1.8**).

4-[3-tert-butyl-5-(2,4-dioxo-tetrahydro-pyrimidin-1-yl)-2-hydroxy-benzyl]-piperazine-1-carboxylic acid tert-butyl ester (compound **IA-L5-1-1.10**). ¹H NMR (300 MHz, CDCl₃) δ ppm 7.37 (s, 1 H) 7.05 (d, *J*=2.57 Hz, 1 H) 6.80 (d, *J*=2.57 Hz, 1 H) 3.79 (t, *J*=6.62 Hz, 2 H) 3.69 (s, 2 H) 3.34-3.61 (m, 2 H) 2.81 (t, *J*=6.62 Hz, 2 H) 2.52 (s, 2 H) 1.56 (s, 4 H) 1.46 (s, 9 H) 1.39 (s, 9 H)

N-{1-[3-tert-butyl-5-(2,4-dioxo-tetrahydro-pyrimidin-1-yl)-2-hydroxy-benzyl]-pyrrolidin-3-yl}-methanesulfonamide (compound **IA-L5-1-1.11**). ¹H NMR (300 MHz, CDCl₃) δ ppm 7.48 (s, 1 H) 7.04 (d, *J*=2.57 Hz, 1 H) 6.81 (d, *J*=2.21 Hz, 1 H) 4.66 (s, 1 H) 4.04-4.17 (m, 2 H) 3.80 (t, *J*=6.62 Hz, 2 H) 2.96 (s, 3 H) 2.85-2.93 (m, 1 H) 2.82 (t, *J*=6.62 Hz, 2 H) 2.65-2.76 (m, 1 H) 2.50-2.64 (m, 1 H) 2.34-2.49 (m, 1 H) 1.73-1.89 (m, 1 H) 1.39 (s, 9 H)

{1-[3-tert-butyl-5-(2,4-dioxo-tetrahydro-pyrimidin-1-yl)-2-hydroxy-benzyl]-pyrrolidin-3-yl}-carbamic acid tert-butyl ester (compound **IA-L5-1-1.12**). ¹H NMR (300 MHz, CDCl₃) δ ppm 7.38 (s, 1 H) 7.01-7.04 (m, 1 H) 6.77-6.80 (m, 1 H) 4.66-4.75 (m, 1 H) 4.16-4.27 (m, 1 H) 3.80 (t, 2 H) 3.68-3.87 (m, 2 H) 2.81 (t, 2 H) 2.26-2.96 (m, 5 H) 1.49-1.74 (m, 2 H) 1.43 (s, 9 H) 1.40 (s, 9 H)

1-(3-tert-butyl-5-((2,6-dimethylmorpholino)methyl)-4-hydroxyphenyl)dihydro pyrimidine-2,4(1H,3H)-dione 2,2,2-trifluoroacetate (compound **IA-L5-1-1.13**).

1-(3-tert-butyl-4-hydroxy-5-(morpholinomethyl)phenyl)dihydropyrimidine-2,4(1H,3H)-dione 2,2,2-trifluoroacetate (compound **IA-L5-1-1.14**).

1-(3-tert-butyl-4-methoxy-5-((1-methyl-1H-indol-3-yl)methyl)phenyl)dihydropyrimidine - 2,4(1H,3H)-dione (compound **IA-L5-1-2.1**). ¹H NMR (300MHz, DMSO-d6): δ 10.23 (s,1H), 7.48 (d, J=8.1Hz,1H), 7.38 (d, J=8.1Hz, 1H), 7.17 (m,1H), 7.08 (d, J=2.6Hz, 1H), 7.04 (s, 1H), 6.98 (m, 2H), 4.05 (s, 2H), 3.76 (s, 3H), 3.72 (s, 3H), 3.66 (t, J=6.6Hz, 1H), 2.62 (t, J=6.6Hz, 1H), 1.37 (s, 9H)

N-(1-(3-tert-butyl-5-(2,4-dioxotetrahydropyrimidin-1(2H)-yl)-2-hydroxybenzyl)-1,2,3,4-tetrahydroquinolin-6-yl)methanesulfonamide (compound **IA-L5-1-2.2**). ¹H NMR (300 MHz, CDCl₃) δ 9.38 (s, 1 H) 7.46 (s, 1 H) 7.09 (d, J=2.21 Hz, 1 H) 6.94 - 7.06 (m, 2 H) 6.91 (d, J=2.57 Hz, 1 H) 6.23 - 6.31 (m, 1 H) 5.37 (d, J=6.99 Hz, 1 H) 3.77 - 3.89 (m, 3 H) 3.04 - 3.12 (m, 2 H) 2.97 (s, 3 H) 2.78 - 2.96 (m, 3 H) 1.94 - 2.04 (m, 2 H) 1.39 (s, 9 H).

N-(4-(3-tert-butyl-5-(2,4-dioxotetrahydropyrimidin-1(2H)-yl)phenethyl)phenyl)methane sulfonamide (compound **IA-L5-2-1.2**). ¹H NMR (300 MHz, DMSO-d₆) δ 1.25 (s, 9 H) 2.69 (t, J=6.62 Hz, 2 H) 2.83 (s, 4 H) 2.91 (s, 3 H) 3.75 (t, J=6.62 Hz, 2 H) 6.99 - 7.21 (m, 7 H) 9.60 (s, 1 H) 10.31 (s, 1 H).

methyl 2-(3-tert-butyl-5-(2,4-dioxo-3,4-dihydropyrimidin-1(2H)-yl)-2-methoxyphen ethyl)-5-(methylsulfonamido)benzoate (compound **IB-L5-2-1.1**). ¹H NMR (300 MHz, DMSO-d6) δ 1.34 (s, 9 H) 2.83 - 2.92 (m, 2 H) 2.96 (s, 3 H) 3.14 (dd, J=10.30, 5.88 Hz, 2 H) 3.75 (s, 3 H) 3.83 (s, 3 H) 5.64 (d, J=7.72 Hz, 1 H) 7.13 (d, J=2.94 Hz, 1 H) 7.20 (d, J=2.57 Hz, 1 H) 7.28 - 7.36 (m, 2 H) 7.61 - 7.71 (m, 2 H) 9.88 (s, 1 H) 11.39 (s, 1 H)

N-(4-(3-tert-butyl-5-(2,4-dioxo-3,4-dihydropyrimidin-1(2H)-yl)-2-methoxyphenethyl)phenyl) methanesulfonamide (compound **IB-L5-2-1.2**). ¹H NMR (300 MHz, DMSO-d6): δ 11.39 (s,1H), 9.60 (s,1H), 7.65 (d, J=8.1Hz,1H), 7.23(m,3H), 7.17(m,3H), 5.64(d,J=7.7Hz,1H), 3.77(s,3H), 2.93(s,3H), 2.88(bs,4H), 1.35(s,9H)

N-(4-(3-tert-butyl-5-(2,4-dioxotetrahydropyrimidin-1(2H)-yl)-2-methoxybenzyloxy)phenyl) methanesulfonamide (compound **IA-L6-1.1**). ¹H NMR (300 MHz, DMSO-d₆) δ ppm 1.36 (s, 9 H) 2.69 (t, J=6.62 Hz, 2 H) 2.89 (s, 3 H) 3.71 - 3.76 (m, 2 H) 3.78 (s, 3 H) 5.05 (s, 2 H) 6.96 - 7.12 (m, 2 H) 7.10 - 7.21 (m, 2 H) 7.23 (d, J=2.94 Hz, 1 H) 7.32 (d, J=2.57 Hz, 1 H) 9.39 (s, 1 H) 10.32 (s, 1 H).

1-(3-tert-butyl-5-((cyclohexyl(ethyl)amino)methyl)-4-hydroxyphenyl)dihydropyrimidine-2,4(1H,3H)-dione 2,2,2-trifluoroacetate (compound **IA-L9-1.1**).

1-(3-tert-butyl-5-((cyclohexyl(methyl)amino)methyl)-4-hydroxyphenyl)dihydro pyrimidine-2,4(1H,3H)-dione 2,2,2-trifluoroacetate (compound **IA-L9-1.2**).

N-(4-(3-tert-butyl-5-(2,4-dioxotetrahydropyrimidin-1(2H)-yl)-2-hydroxybenzylamino)phenyl) methanesulfonamide (compound **IA-L9-1.3**). ¹H NMR (300 MHz, DMSO-d₆) δ 1.37 (s, 9 H) 2.66 (t, J=6.62 Hz, 2 H) 2.82 (s, 3 H) 3.65 (t, J=6.62 Hz, 2 H) 4.24 (d, J=5.15 Hz, 2 H) 6.10 (t, J=5.52 Hz, 1 H) 6.64 (d, J=8.82 Hz, 2 H) 6.98 (d, J=8.82 Hz, 2 H) 7.03 (s, 2 H) 8.79 (s, 1 H) 9.04 (s, 1 H) 10.22 (s, 1 H).

N-(4-(3-tert-butyl-5-(2,4-dioxotetrahydropyrimidin-1(2H)-yl)-2-methoxybenzylamino)phenyl) methanesulfonamide (compound **IA-L9-1.4**). ¹H NMR (500 MHz, DMSO-d₆) δ 1.36 (s, 9H), 2.65 (t,

J=6.7 Hz, 2H), 2.80 (s, 3H), 3.68 (t, 6.7 Hz, 2H), 3.79 (s, 3H), 4.25 (d, J 5.5 Hz, 2H), 6.10 (m, 1H), 6.55 (d, J= 8.5 Hz, 2H), 6.94 (d, J= 8.5 Hz, 2H), 7.13 (d, J= 2.5 Hz, 1H), 7.19 (d, J= 2.4 Hz, 1H), 8.92 (s, 1H), 10.23 (s, 1H).

N-(4-(2-(3-tert-butyl-5-(2,4-dioxotetrahydropyrimidin-1(2H)-yl)-2-methoxyphenyl)-2-oxoethyl)phenyl)methanesulfonamide (compound **IA-L11-1.1**). ¹H NMR (300 MHz, DMSO-d₆) δ 1.36 (s, 9 H) 2.70 (t, J=6.62 Hz, 2 H) 2.96 (s, 3 H) 3.64 (s, 3 H) 3.76 (t, J=6.62 Hz, 2 H) 4.27 (s, 2 H) 7.10-7.26 (m, 4 H) 7.32 - 7.41 (m, 2 H) 9.67 (s, 1 H) 10.37 (s, 1 H).

N-(4-(2-(3-tert-butyl-5-(2,4-dioxotetrahydropyrimidin-1(2H)-yl)-2-methoxyphenyl)acetyl) phenyl)methanesulfonamide (compound **IA-L12-1.1**). ¹H NMR (300 MHz, DMSO-d₆) δ 1.33 (s, 9 H) 2.68 (t, J=6.62 Hz, 2 H) 3.12 (s, 3 H) 3.61 (s, 3 H) 3.72 (t, J=6.62 Hz, 2 H) 4.36 (s, 2 H) 7.01 (d, J=2.94 Hz, 1 H) 7.15 (d, J=2.57 Hz, 1 H) 7.29 (d, J=8.82 Hz, 2 H) 8.04 (d, J=8.82 Hz, 2 H) 10.29 (s, 1 H) 10.35 (s, 1 H).

The following compounds can be prepared utilizing the above discussion:

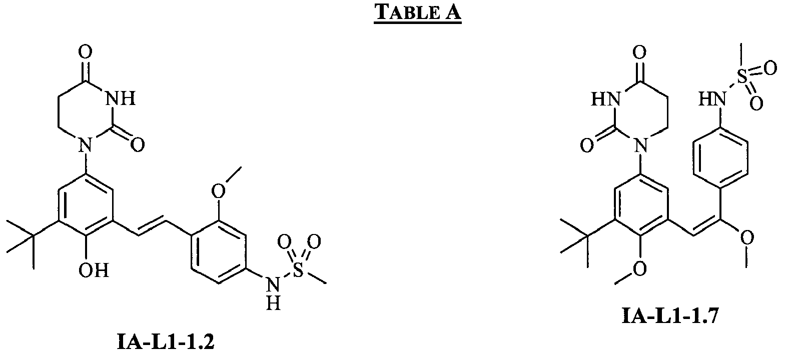

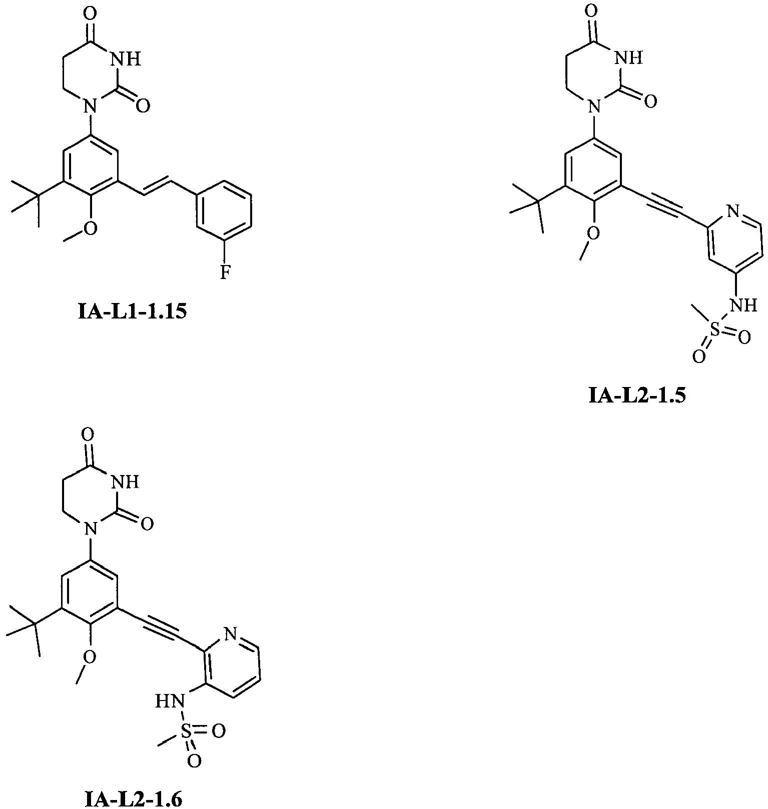

**TABLE B**

| | | |
|---|---|---|
| 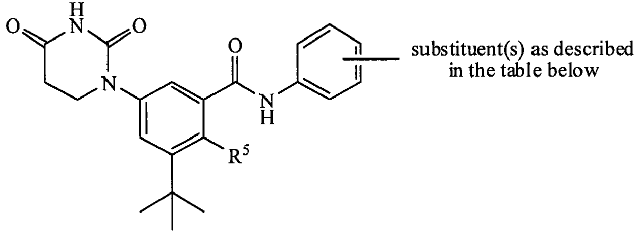 | | |

| **compound** | **R⁵** | **substituent(s)** |
|---|---|---|
| | | |
| **IA-L3-1.23** | -OH | -2-OCH₃- |
| **IA-L3-1.29** | -OCH₃ | -4-C(H)₂N(H)S(O)₂CH₃ |
| **IA-L3-1.61** | -OCH₃ | 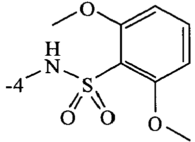 |
| **IA-L3-1.68** | -OCH₃ | -4-N[C(H)₂C(H)=CH₂]S(O)₂CH₃ |
| **IA-L3-1.75** | -OCH₃ | -4-N[C(O)OC(H)₂C(H)₂CH₃]S(O)₂CH₃ |

### HCV Polymerase Inhibition Assay

Either two-fold serial dilutions (fractional inhibition assay) or a narrower range of dilutions spanning the IC₅₀ of the inhibitor (tight binding assay) of the inhibitors were incubated with 20mM Tris-Cl pH 7.4, 2mM MnCl₂, 1mM dithiothreitol, 1mM ethylene diamine tetraacetic acid (EDTA), 60 to 125µM GTP and 20 to 50nM Δ21 NS5B (HCV Strain 1B (BK, Genbank accession number M58335, or H77, Genbank accession number AF011751)) for 15min at room temperature. The reaction was initiated by the addition of 20µM CTP, 20µM ATP, 1µM ³H-UTP (10mCi/umol) 5nM template RNA and 0.1 U/µl RNase inhibitor (RNasin, Promega), and allowed to proceed for 2 to 4h at room temperature. Reaction volume was 50µl. The reaction was terminated by the addition of 1 volume of 4mM spermine in 10mM Tris-Cl pH 8.0, 1mM EDTA. After incubation for at least 15 min at room temperature, the precipitated RNA was captured by filtering through a GF/B filter (Millipore) in a 96 well format. The filter plate was washed three times with 200µl each of 2mM spermine, 10mM Tris-Cl pH 8.0, 1mM EDTA, and 2 times with ethanol. After air-drying, 30µl of Microscint 20 scintillation cocktail (Packard) was added to each well, and the retained cpm were determined by scintillation counting. IC₅₀ values were calculated by a two-variable nonlinear regression equation using an uninhibited control and a fully inhibited control sample to determine the minimum and maximum for the curve. Tight-binding assays were performed on those compounds exhibiting IC₅₀ values less than 0.005µM in the fractional inhibition assay in order to more precisely measure the IC₅₀ values. Retained cpm were plotted vs. inhibitor concentration and fit to equation 1 using non-linear regression (ref. 1) to obtain the IC₅₀ values: $Retained cpm = A ⁢ \left[sqrt\left\{{\left({IC}_{50}+{I}_{t}-{E}_{t}\right)}^{∧}⁢2+4*{IC}_{50}*{E}_{t}\right\}-\left({IC}_{50}+{I}_{t}-{E}_{t}\right)\right]$
where A=Vmax[S]/2(Km+[S]); It=total inhibitor concentration and Et=total active concentration of enzyme.

Ref. Morrison, J. F. and S. R. Stone. 1985. Approaches to the study and analysis of the inhibition of enzymes by slow- and tight-binding inhibitors. Comments Mol. Cell. Biophys. 2: 347-368.

The sequence of the template RNA used was: 5'-GGGCGAAUUG GGCCCUCUAG AUGCAUGCUC GAGCGGCCGC CAGUGUGAUG GAUAUCUGCA GAAUUCGCCC UUGGUGGCUC CAUCUUAGCC CUAGUCACGG CUAGCUGUGA AAGGUCCGUG AGCCGCUUGA CUGCAGAGAG UGCUGAUACU GGCCUCUCUG CAGAUCAAGUC-3'

When tested by the above method, the compounds of this invention inhibit HCV polymerase 1A and/or 1B. The legend in the table below is as follows: A -- IC₅₀ ≤ 0.01 uM; B -- 0.1uM ≥ IC₅₀ > 0.01uM; C -- 1uM ≥ IC₅₀ > 0.1uM; and D -- IC₅₀ > 1uM; ND - not determined.

**Table IC₅₀**

| **compound** | **1a** | **1b** | **compound** | **1a** | **1b** |
|---|---|---|---|---|---|
| **IA-L0-1.1** | A | B | **IA-L0-1.2** | B | B |
| **IA-L0-1.3** | C | C | **IA-L0-1.4** | B | B |
| **IA-L0-1.5** | C | C | **IA-L0-1.6** | B | B |
| **IA-L0-1.7** | D | C | **IA-L0-1.8** | C | C |
| **IA-L0-1.9** | B | C | **IA-L0-1.10** | D | D |
| **IA-L0-1.11** | D | D | **IB-L0-1.1** | A | A |
| **IB-L0-1.2** | C | C | **IB-L0-1.3** | B | B |
| **IB-L0-1.4** | B | B | **IB-L0-1.5** | B | B |
| **IB-L0-1.6** | C | C | **IB-L0-1.7** | B | B |
| **IB-L0-1.8** | C | C | **IB-L0-1.9** | C | C |
| **IB-L0-1.10** | C | C | **IA-L0-2.1** | C | C |
| **IA-L0-2.2** | B | B | **IA-L0-2.3** | C | C |
| **IA-L0-2.4** | B | B | **IA-L0-2.5** | C | C |
| **IA-L0-2.6** | C | C | **IA-L0-2.7** | C | C |
| **IA-L0-2.8** | B | B | **IA-L0-2.9** | A | A |
| **IA-L0-2.10** | D | D | **IB-L0-2.1** | C | C |
| **IB-L0-2.2** | C | C | **IB-L0-2.3** | A | A |
| **IB-L0-2.4** | A | A | **IB-L0-2.5** | B | B |
| **IB-L0-2.6** | B | B | **IB-L0-2.7** | B | B |
| **IB-L0-2.8** | B | B | **IB-L0-2.9** | A | A |
| **IB-L0-2.10** | A | B | **IB-L0-2.11** | A | A |
| **IB-L0-2.12** | A | B | **IB-L0-2.13** | A | B |
| **IB-L0-2.14** | A | A | **IB-L0-2.15** | A | B |
| **IB-L0-2.16** | A | B | **IB-L0-2.17** | A | B |
| **IB-L0-2.18** | A | B | **IB-L0-2.19** | A | B |
| **IB-L0-2.20** | A | B | **IB-L0-2.21** | B | B |
| **IB-L0-2.22** | B | B | **IB-L0-2.23** | B | A |
| **IB-L0-2.24** | B | B | **IB-L0-2.25** | B | B |
| **IB-L0-2.26** | B | B | **IB-L0-2.27** | B | B |
| **IB-L0-2.28** | B | B | **IB-L0-2.29** | B | B |
| **IB-L0-2.30** | B | B | **IB-L0-2.31** | B | B |
| **IB-L0-2.32** | B | B | **IB-L0-2.33** | B | B |
| **IB-L0-2.34** | B | B | **IB-L0-2.35** | B | B |
| **IB-L0-2.36** | B | C | **IB-L0-2.37** | C | C |
| **IB-L0-2.38** | C | B | **IB-L0-2.39** | C | C |
| **IB-L0-2.40** | C | C | **IB-L0-2.41** | C | C |
| **IB-L0-2.42** | C | C | **IB-L0-2.43** | C | C |
| **IB-L0-2.44** | C | C | **IB-L0-2.45** | C | C |
| **IB-L0-2.46** | C | C | **IB-L0-2.47** | D | D |
| **IB-L0-2.48** | D | D | **IB-L0-2.49** | D | D |
| **IB-L0-2.50** | B | B | **IB-L0-2.51** | A | B |
| **IB-L0-2.52** | A | B | **IB-L0-2.53** | A | B |
| **IB-L0-2.54** | A | B | **IB-L0-2.55** | A | B |
| **IB-L0-2.56** | A | B | **IB-L0-2.57** | A | B |
| **IB-L0-2.58** | A | B | **IB-L0-2.59** | A | B |
| **IB-L0-2.60** | A | B | **IB-L0-2.61** | A | B |
| **IB-L0-2.62** | B | B | **IB-L0-2.63** | B | B |
| **IB-L0-2.64** | B | B | **IB-L0-2.65** | B | A |
| **IB-L0-2.66** | B | B | **IB-L0-2.67** | B | B |
| **IB-L0-2.68** | B | B | **IB-L0-2.69** | B | B |
| **IB-L0-2.70** | B | C | **IB-L0-2.71** | C | C |
| **IB-L0-2.72** | C | C | **IB-L0-2.73** | C | C |
| **IB-L0-2.74** | C | C | **IB-L0-2.75** | C | D |
| **IB-L0-2.76** | C | D | **IB-L0-2.77** | D | D |
| **IB-L0-2.78** | D | D | **IB-L0-2.79** | B | B |
| **IA-L1-1.3** | A | A | **IA-L1-1.4** | A | A |
| **IA-L1-1.5** | A | B | **IA-L1-1.6** | A | B |
| **IA-L1-1.9** | A | B | **IA-L1-1.10** | B | B |
| **IA-L1-1.11** | B | B | **IA-L1-1.12** | C | C |
| **IA-L1-1.13** | C | C | **IA-L1-1.14** | D | D |
| **IA-L1-1.16** | A | A | **IA-L1-1.17** | B | B |
| **IA-L1-1.18** | C | C | **IA-L1-1.20** | A | B |
| **IA-L1-1.21** | B | B | **IA-L1-1.22** | C | C |
| **IA-L1-1.23** | C | C | **IA-L1-1.24** | D | D |
| **IA-L1-1.25** | D | D | **IA-L1-1.26** | B | B |
| **IA-L1-1.27** | A | B | **IB-L1-1.1** | A | A |
| **IB-L1-1.2** | B | B | **IB-L1-1.4** | A | A |
| **IB-L1-1.5** | A | A | **IB-L1-1.6** | A | B |
| **IB-L1-1.7** | A | B | **IB-L1-1.8** | A | B |
| **IB-L1-1.9** | A | B | **IB-L1-1.10** | A | B |
| **IB-L1-1.11** | A | B | **IB-L1-1.12** | A | B |
| **IB-L1-1.13** | A | B | **IB-L1-1.14** | A | B |
| **IB-L1-1.15** | A | B | **IB-L1-1.16** | A | B |
| **IB-L1-1.17** | A | B | **IB-L1-1.18** | A | B |
| **IB-L1-1.19** | A | B | **IB-L1-1.20** | A | B |
| **IB-L1-1.21** | A | B | **IB-L1-1.22** | B | B |
| **IB-L1-1.23** | B | B | **IB-L1-1.24** | B | B |
| **IB-L1-1.25** | B | B | **IB-L1-1.26** | B | B |
| **IB-L1-1.27** | B | B | **IB-L1-1.28** | B | B |
| **IB-L1-1.29** | B | B | **IB-L1-1.30** | B | B |
| **IB-L1-1.31** | B | C | **IB-L1-1.32** | C | C |
| **IB-L1-1.33** | C | C | **IB-L1-1.34** | D | D |
| **IB-L1-1.45** | A | B | **ID-L1-1.46** | B | B |
| **IB-L1-1.47** | B | B | **IB-L1-1.48** | B | B |
| **IB-L1-1.49** | B | C | **IB-L1-1.50** | B | B |
| **IB-L1-1.51** | B | B | **IB-L1-1.52** | C | C |
| **IB-L1-1.53** | D | D | **IB-L1-1.55** | D | D |
| **IA-L2-1.1** | B | A | **IA-L2-1.2** | A | A |
| **IA-L2-1.3** | A | A | **IA-L2-1.4** | C | C |
| **IA-L2-1.7** | B | B | **IA-L2-1.8** | A | A |
| **IA-L2-1.9** | A | A | **IA-L2-1.10** | B | B |
| **IA-L2-1.11** | A | B | **IA-L2-1.12** | A | A |
| **IA-L2-1.13** | A | A | **IA-L2-1.14** | A | A |
| **IA-L2-1.15** | B | B | **IA-L2-1.16** | A | B |
| **IA-L2-1.17** | B | B | **IA-L2-1.18** | A | A |
| **IA-L2-1.19** | C | B | **IA-L2-1.20** | A | B |
| **IA-L2-1.21** | A | B | **IA-L2-1.22** | B | B |
| **IA-L2-1.23** | C | C | **IA-L2-1.24** | A | B |
| **IA-L2-1.25** | A | B | **IA-L2-1.26** | B | B |
| **IB-L2-1.1** | A | B | **IB-L2-1.2** | A | B |
| **IB-L2-1.3** | A | B | **IB-L2-1.4** | B | B |
| **IB-L2-1.5** | B | B | **IB-L2-1.6** | A | B |
| **IB-L2-1.7** | A | B | **IB-L2-1.8** | A | B |
| **IB-L2-1.9** | C | B | **IB-L2-1.10** | A | B |
| **IB-L2-1.11** | B | B | **IB-L2-1.12** | B | B |
| **IB-L2-1.15** | C | C | **IA-L2-2.1** | B | B |
| **IB-L2-2.1** | B | B | **IA-L3-1.1** | D | D |
| **IA-L3-1.2** | D | B | **IA-L3-1.3** | D | C |
| **IA-L3-1.4** | C | B | **IA-L3-1.5** | C | B |
| **IA-L3-1.6** | B | A | **IA-L3-1.7** | C | B |
| **IA-L3-1.8** | B | B | **IA-L3-1.9** | C | B |
| **IA-L3-1.10** | B | B | **IA-L3-1.11** | D | C |
| **IA-L3-1.12** | C | B | **IA-L3-1.13** | C | A |
| **IA-L3-1.14** | B | A | **IA-L3-1.15** | D | C |
| **IA-L3-1.16** | D | B | **IA-L3-1.17** | D | ND |
| **IA-L3-1.18** | D | ND | **IA-L3-1.19** | C | B |
| **IA-L3-1.20** | D | B | **IA-L3-1.21** | D | C |
| **IA-L3-1.22** | C | B | **IA-L3-1.24** | C | C |
| **IA-L3-1.25** | D | D | **IA-L3-1.26** | D | D |
| **IA-L3-1.27** | C | C | **IA-L3-1.28** | ND | ND |
| **IA-L3-1.30** | D | C | **IA-L3-1.31** | D | D |
| **IA-L3-1.32** | ND | ND | **IA-L3-1.33** | D | C |
| **IA-L3-1.34** | ND | ND | **IA-L3-1.35** | ND | ND |
| **IA-L3-1.36** | ND | ND | **IA-L3-1.37** | ND | ND |
| **IA-L3-1.38** | D | C | **IA-L3-1.39** | D | C |
| **IA-L3-1.40** | ND | ND | **IA-L3-1.41** | C | D |
| **IA-L3-1.42** | D | D | **IA-L3-1.43** | D | D |
| **IA-L3-1.44** | ND | ND | **IA-L3-1.45** | ND | ND |
| **IA-L3-1.46** | ND | ND | **IA-L3-1.47** | D | D |
| **IA-L3-1.48** | D | D | **IA-L3-1.49** | D | D |
| **IA-L3-1.50** | D | D | **IA-L3-1.51** | B | B |
| **IA-L3-1.52** | C | C | **IA-L3-1.53** | ND | ND |
| **IA-L3-1.54** | B | C | **IA-L3-1.55** | C | C |
| **IA-L3-1.56** | C | C | **IA-L3-1.57** | B | c |
| **IA-L3-1.58** | C | C | **IA-L3-1.59** | C | B |
| **IA-L3-1.60** | B | B | **IA-L3-1.62** | C | C |
| **IA-L3-1.63** | D | D | **IA-L3-1.64** | C | D |
| **IA-L3-1.65** | D | D | **IA-L3-1.66** | D | D |
| **IA-L3-1.67** | D | D | **IA-L3-1.69** | C | C |
| **IA-L3-1.70** | D | D | **IA-L3-1.71** | C | D |
| **IA-L3-1.72** | D | D | **IA-L3-1.73** | D | D |
| **IA-L3-1.74** | D | D | **IA-L3-1.76** | D | D |
| **IA-L3-1.77** | D | D | **IA-L3-1.78** | D | D |
| **IA-L3-1.79** | D | D | **IA-L3-1.80** | D | D |
| **IA-L3-1.81** | ND | ND | **IA-L3-1.82** | ND | ND |
| **IA-L3-1.83** | B | C | **IA-L3-1.84** | C | C |
| **IA-L3-1.85** | C | C | **IA-L3-1.86** | ND | ND |
| **IA-L3-1.87** | D | C | **IA-L3-1.88** | D | D |
| **IA-L3-1.89** | B | B | **IA-L3-1.90** | B | B |
| **IA-L3-1.91** | B | B | **IA-L3-1.94** | C | C |
| **IA-L3-1.95** | C | B | **IA-L3-1.96** | C | D |
| **IA-L3-1.97** | D | D | **IA-L3-1.98** | D | C |
| **IA-L3-1.99** | B | C | **IA-L3-1.100** | D | C |
| **IA-L3-1.101** | D | D | **IA-L3-1.102** | C | C |
| **IA-L3-1.103** | C | D | **IA-L3-1.104** | D | C |
| **IA-L3-1.105** | C | B | **IA-L3-1.107** | D | D |
| **IA-L3-1.108** | D | D | **IA-L3-1.111** | B | C |
| **IA-L3-1.112** | C | C | **IA-L3-1.113** | D | D |
| **IA-L3-1.114** | D | D | **IA-L3-1.115** | D | D |
| **IA-L3-1.116** | D | D | **IA-L3-1.117** | D | D |
| **IA-L3-1.118** | D | D | **IA-L3-1.119** | B | A |
| **IA-L3-1.120** | C | C | **IA-L3-1.121** | C | C |
| **IA-L3-1.122** | B | B | **IA-L3-1.123** | D | D |
| **IB-L3-1.1** | B | B | **IB-L3-1.2** | B | A |
| **IB-L3-1.3** | C | D | **IB-L3-1.4** | C | B |
| **IA-L3-2.1** | D | C | **IA-L3-2.2** | C | C |
| **IA-L3-2.3** | D | D | **IA-L3-2.4** | D | D |
| **IA-L3-2.5** | D | C | **IA-L4-1.1** | C | C |
| **IA-L4-1.2** | D | D | **IA-L4-1.3** | C | C |
| **IA-L4-1.4** | D | D | **IA-L4-1.5** | D | D |
| **IA-L4-1.6** | D | D | **IA-L4-1.7** | C | D |
| **IA-L4-1.8** | D | C | **IA-L4-1.9** | B | B |
| **IA-L4-1.10** | D | D | **IA-L4-1.11** | A | B |
| **IA-L4-1.12** | B | B | **IB-L4-1.1** | A | B |
| **IA-L5-1-1.1** | D | D | **IA-L5-1-1.2** | D | D |
| **IA-L5-1-1.3** | D | D | **IA-L5-1-1.4** | D | D |
| **IA-L5-1-1.5** | D | D | **IA-L5-1-1.6** | D | D |
| **IA-L5-1-1.7** | D | D | **IA-L5-1-1.8** | D | D |
| **IA-L5-1-1.10** | D | D | **IA-L5-1-1.11** | D | D |
| **IA-L5-1-1.12** | D | D | **IA-L5-1-1.13** | D | D |
| **IA-L5-1-1.14** | D | D | **IA-L5-1-2.1** | D | D |
| **IA-L5-1-2.2** | D | D | **IA-L5-2-1.1** | B | B |
| **IA-L5-2-1.2** | B | B | **IB-L5-2-1.1** | A | B |
| **IB-L5-2-1.2** | B | B | **IA-L6-1.1** | B | C |
| **IA-L8-1.1** | C | C | **IA-L9-1.1** | D | D |
| **IA-L9-1.2** | D | D | **IA-L9-1.3** | D | D |
| **IA-L9-1.4** | B | C | **IA-L11-1.1** | B | B |
| **IA-L12-1.1** | B | B | | | |

### HCV Polymerase Replicon Assay

Two stable subgenomic replicon cell lines were used for compound characterization in cell culture: one derived from genotype 1a-H77 and one derived from genotype 1b-Con1 (obtained from Apath, LLC, St. Louis, MO). All replicon constructs were bicistronic subgenomic replicons similar to those described by Bartenschlager and coworkers (Lohmann et al., Replication of Subgenomic Hepatitis C Virus RNAs in a Hepatoma Cell Line, SCIENCE 285:110-3(1999)). The genotype 1a replicon construct contains NS3-NS5B coding region derived from the H77 strain of HCV (1a-H77) (Blight et al., Efficient Replication of Hepatitis C Virus Genotype 1a RNAs in Cell Culture, J. VIROL. 77:3181-90 (2003)). The replicon also has a firefly luciferase reporter and a neomycin phosphotransferase (Neo) selectable marker. These two coding regions, separated by the FMDV 2a protease, comprise the first cistron of the bicistronic replicon construct, with the second cistron containing the NS3-NS5B coding region with addition of adaptive mutations E1202G, K1691R, K2040R and S2204I. The 1b-Con1 replicon construct is identical to the 1a-H77 replicon, except that the NS3-NS5B coding region was derived from the 1b-Con1 strain, and the adaptive mutations are E1202G, T1280I and S2204I. Replicon cell lines were maintained in Dulbecco's modified Eagles medium (DMEM) containing 10% (v/v) fetal bovine serum (FBS), 100 IU/ml penicillin, 100mg/ml streptomycin (Invitrogen), and 200mg/ml G418 (Invitrogen).

The inhibitory effects of compounds on HCV replication were determined by measuring activity of the luciferase reporter gene. Briefly, replicon-containing cells were seeded into 96 well plates at a density of 5000 cells per well in 100ul DMEM containing 5% FBS. 16-24h later, the compounds were diluted in dimethyl sulfoxide (DMSO) to generate a 200x stock in a series of eight half-log dilutions. The dilution series was then further diluted 100-fold in the medium containing 5% FBS. Medium with the inhibitor was added to the overnight cell culture plates already containing 100ul of DMEM with 5% FBS. In assays measuring inhibitory activity in the presence of human plasma, the medium from the overnight cell culture plates was replaced with DMEM containing 40% human plasma and 5% FBS. The cells were incubated for three days in the tissue culture incubators and were then lysed for RNA extraction. For the luciferase assay, 30ul of Passive Lysis buffer (Promega) was added to each well, and then the plates were incubated for 15min with rocking to lyse the cells. Luciferin solution (50 to 100ul, Promega) was added to each well, and luciferase activity was measured with a Victor II luminometer (Perkin-Elmer). The percent inhibition of HCV RNA replication was calculated for each compound concentration and the EC₅₀ value was calculated using nonlinear regression curve fitting to the 4-parameter logistic equation and GraphPad Prism 4 software.

When tested by the above method, the compounds of this invention inhibit HCV polymerase 1A and/or 1B. The legend in the table below is as follows: A -- EC₅₀ ≤ 0.01uM; B -- 0.1uM ≥ EC₅₀ > 0.01uM; C -- 1uM ≥ EC₅₀ > 0.1uM; and D -- EC₅₀ > 1uM; ND - not determined.

**Table EC₅₀**

| **compound** | **1a** | **1b** | **compound** | **1a** | **1b** |
|---|---|---|---|---|---|
| **IA-L0-1.1** | C | A | **IA-L0-1.2** | C | B |
| **IA-L0-1.3** | C | C | **IA-L0-1.4** | C | B |
| **IA-L0-1.5** | D | D | **IA-L0-1.6** | C | B |
| **IA-L0-1.7** | D | D | **IA-L0-1.8** | D | D |
| **IA-L0-1.9** | D | C | **IA-L0-1.10** | ND | ND |
| **IA-LO-1.11** | ND | ND | **IB-L0-1.1** | B | B |
| **IB-L0-1.2** | D | D | **IB-L0-1.3** | C | B |
| **IB-L0-1.4** | C | B | **IB-L0-1.5** | B | B |
| **IB-L0-1.6** | D | C | **IB-L0-1.7** | D | C |
| **IB-L0-1.8** | D | D | **IB-L0-1.9** | D | D |
| **IB-L0-1.10** | D | D | **IA-L0-2.1** | D | D |
| **IA-L0-2.2** | C | B | **IA-L0-2.3** | C | C |
| **IA-L0-2.4** | D | C | **IA-L0-2.5** | D | D |
| **IA-L0-2.6** | D | D | **IA-L0-2.7** | D | C |
| **IA-L0-2.8** | C | B | **IA-L0-2.9** | A | A |
| **IA-L0-2.10** | ND | ND | **IB-L0-2.1** | D | C |
| **IB-L0-2.2** | D | D | **IB-L0-2.3** | A | A |
| **IB-L0-2.4** | ND | A | **IB-L0-2.5** | B | A |
| **IB-L0-2.6** | C | B | **IB-L0-2.7** | C | B |
| **IB-L0-2.8** | ND | B | **IB-L0-2.9** | A | A |
| **IB-L0-2.10** | A | A | **IB-LO-2.11** | B | A |
| **IB-L0-2.12** | B | A | **IB-L0-2.13** | B | A |
| **IB-L0-2.14** | C | B | **IB-L0-2.15** | C | B |
| **IB-L0-2.16** | C | A | **IB-L0-2.17** | B | A |
| **IB-L0-2.18** | C | B | **IB-L0-2.19** | B | B |
| **IB-L0-2.20** | C | B | **IB-L0-2.21** | C | B |
| **IB-L0-2.22** | C | B | **IB-L0-2.23** | C | B |
| **IB-L0-2.24** | B | B | **IB-L0-2.25** | C | B |
| **IB-L0-2.26** | D | C | **IB-L0-2.27** | C | B |
| **IB-L0-2.28** | D | C | **IB-L0-2.29** | C | B |
| **IB-L0-2.30** | C | B | **IB-L0-2.31** | C | B |
| **IB-L0-2.32** | C | B | **IB-L0-2.33** | C | C |
| **IB-L0-2.34** | D | C | **IB-L0-2.35** | D | C |
| **IB-L0-2.36** | C | B | **IB-L0-2.37** | D | C |
| **IB-L0-2.38** | D | D | **IB-L0-2.39** | D | C |
| **IB-L0-2.40** | D | C | **IB-L0-2.41** | C | C |
| **IB-L0-2.42** | C | C | **IB-L0-2.43** | D | C |
| **IB-L0-2.44** | D | D | **IB-L0-2.45** | D | C |
| **IB-L0-2.46** | ND | ND | **IB-L0-2.47** | ND | ND |
| **IB-L0-2.48** | ND | ND | **IB-L0-2.49** | ND | ND |
| **IB-L0-2.50** | C | C | **IB-L0-2.51** | B | A |
| **IB-L0-2.52** | B | A | **IB-L0-2.53** | B | B |
| **IB-L0-2.54** | B | B | **IB-L0-2.55** | B | A |
| **IB-L0-2.56** | C | A | **IB-L0-2.57** | C | B |
| **IB-L0-2.58** | B | A | **IB-L0-2.59** | C | B |
| **IB-L0-2.60** | C | B | **IB-L0-2.61** | C | B |
| **IB-L0-2.62** | C | B | **IB-L0-2.63** | C | B |
| **IB-L0-2.64** | C | A | **IB-L0-2.65** | C | B |
| **IB-L0-2.66** | C | B | **IB-L0-2.67** | C | B |
| **IB-L0-2.68** | D | C | **IB-L0-2.69** | C | B |
| **IB-L0-2.70** | D | C | **IB-L0-2.71** | C | B |
| **IB-L0-2.72** | D | C | **IB-L0-2.73** | C | c |
| **IB-L0-2.74** | D | C | **IB-L0-2.75** | D | D |
| **IB-L0-2.76** | ND | ND | **IB-L0-2.77** | ND | ND |
| **IB-L0-2.78** | ND | ND | **IB-L0-2.79** | C | C |
| **IA-L1-1.3** | B | A | **IA-L1-1.4** | A | A |
| **IA-L1-1.5** | B | A | **IA-L1-1.6** | B | B |
| **IA-L1-1.9** | B | A | **IA-L1-1.10** | B | B |
| **IA-L1-1.11** | A | A | **IA-L1-1.12** | C | c |
| **IA-L1-1.13** | D | C | **IA-L1-1.14** | D | D |
| **IA-L1-1.16** | B | B | **IA-L1-1.17** | B | B |
| **IA-L1-1.18** | C | C | **IA-L1-1.20** | B | B |
| **IA-L1-1.21** | A | A | **IA-L1-1.22** | D | C |
| **IA-L1-1.23** | D | D | **IA-L1-1.24** | D | D |
| **IA-L1-1.25** | ND | ND | **IA-L1-1.26** | B | B |
| **IA-L1-1.27** | B | A | **IB-L1-1.1** | A | A |
| **IB-L1-1.2** | ND | B | **IB-L1-1.4** | B | A |
| **IB-L1-1.5** | B | A | **IB-L1-1.6** | A | A |
| **IB-L1-1.7** | A | A | **IB-L1-1.8** | B | A |
| **IB-L1-1.9** | B | A | **IB-L1-1.10** | A | A |
| **IB-L1-1.11** | B | A | **IB-L1-1.12** | B | B |
| **IB-L1-1.13** | B | A | **IB-L1-1.14** | B | A |
| **IB-L1-1.15** | A | A | **IB-L1-1.16** | C | B |
| **IB-L1-1.17** | B | A | **IB-L1-1.18** | B | B |
| **IB-L1-1.19** | B | A | **IB-L1-1.20** | B | A |
| **IB-L1-1.21** | B | A | **IB-L1-1.22** | B | A |
| **IB-L1-1.23** | C | A | **IB-L1-1.24** | B | A |
| **IB-L1-1.25** | B | A | **IB-L1-1.26** | B | A |
| **IB-L1-1.27** | B | A | **IB-L1-1.28** | A | A |
| **IB-L1-1.29** | C | C | **IBL1-1.30** | C | B |
| **IB-L1-1.31** | D | D | **IB-L1-1.32** | C | B |
| **IB-L1-1.33** | C | B | **IB-L1-1.34** | B | A |
| **IB-L1-1.45** | B | A | **IB-L1-1.46** | C | A |
| **IB-L1-1.47** | C | B | **IB-L1-1.48** | C | A |
| **IB-L1-1.49** | D | D | **IB-L1-1.50** | C | B |
| **IB-L1-1.51** | D | B | **IB-L1-1.52** | D | C |
| **IB-L1-1.53** | ND | ND | **IB-L1-1.55** | ND | ND |
| **IA-L2-1.1** | C | B | **IA-L2-1.2** | B | B |
| **IA-L2-1.3** | B | A | **IA-L2-1.4** | C | C |
| **IA-L2-1.7** | D | C | **IA-L2-1.8** | B | A |
| **IA-L2-1.9** | A | A | **IA-L2-1.10** | C | B |
| **IA-L2-1.11** | A | A | **IA-L2-1.12** | B | A |
| **IA-L2-1.13** | C | B | **IA-L2-1.14** | A | A |
| **IA-L2-1.15** | C | C | **IA-L2-1.16** | B | A |
| **IA-L2-1.17** | C | B | **IA-L2-1.18** | B | A |
| **IA-L2-1.19** | D | D | **IA-L2-1.20** | B | A |
| **IA-L2-1.21** | B | A | **IA-L2-1.22** | C | B |
| **IA-L2-1.23** | D | C | **IA-L2-1.24** | C | B |
| **IA-L2-1.25** | C | B | **IA-L2-1.26** | B | B |
| **IB-L2-1.1** | A | A | **IB-L2-1.2** | ND | A |
| **IB-L2-1.3** | B | A | **IB-L2-1.4** | ND | C |
| **IB-L2-1.5** | C | B | **IB-L2-1.6** | B | A |
| **IB-L2-1.7** | ND | A | **IB-L2-1.8** | B | A |
| **IB-L2-1.9** | D | ND | **IB-L2-1.10** | ND | B |
| **IB-L2-1.11** | C | B | **IB-L2-1.12** | C | B |
| **IB-L2-1.15** | D | C | **IA-L2-2.1** | C | C |
| **IB-L2-2.1** | C | C | **IA-L3-1.1** | ND | ND |
| **IA-L3-1.2** | D | D | **IA-L3-1.3** | D | D |
| **IA-L3-1.4** | D | D | **IA-L3-1.5** | D | D |
| **IA-L3-1.6** | C | B | **IA-L3-1.7** | D | D |
| **IA-L3-1.8** | D | C | **IA-L3-1.9** | D | D |
| **IA-L3-1.10** | C | C | **IA-L3-1.11** | D | D |
| **IA-L3-1.12** | D | D | **IA-L3-1.13** | D | D |
| **IA-L3-1.14** | ND | C | **IA-L3-1.15** | D | D |
| **IA-L3-1.16** | D | D | **IA-L3-1.17** | D | D |
| **IA-L3-1.18** | D | C | **IA-L3-1.19** | D | D |
| **IA-L3-1.20** | D | D | **IA-L3-1.21** | D | D |
| **IA-L3-1.22** | D | D | **IA-L3-1.24** | D | D |
| **IA-L3-1.25** | ND | ND | **IA-L3-1.26** | ND | ND |
| **IA-L3-1.27** | D | D | **IA-L3-1.28** | ND | ND |
| **IA-L3-1.30** | D | D | **IA-L3-1.31** | D | D |
| **IA-L3-1.32** | ND | ND | **IA-L3-1.33** | D | D |
| **IA-L3-1.34** | ND | ND | **IA-L3-1.35** | ND | ND |
| **IA-L3-1.36** | ND | ND | **IA-L3-1.37** | ND | ND |
| **IA-L3-1.38** | D | D | **IA-L3-1.39** | D | D |
| **IA-L3-1.40** | ND | ND | **IA-L3-1.41** | D | C |
| **IA-L3-1.42** | ND | ND | **IA-L3-1.43** | ND | ND |
| **IA-L3-1.44** | ND | ND | **IA-L3-1.45** | ND | ND |
| **IA-L3-1.46** | ND | ND | **IA-L3-1.47** | ND | ND |
| **IA-L3-1.48** | ND | ND | **IA-L3-1.49** | ND | ND |
| **IA-L3-1.50** | ND | ND | **IA-L3-1.51** | C | B |
| **IA-L3-1.52** | D | C | **IA-L3-1.53** | ND | ND |
| **IA-L3-1.54** | ND | B | **IA-L3-1.55** | D | D |
| **IA-L3-1.56** | ND | C | **IA-L3-1.57** | C | C |
| **IA-L3-1.58** | ND | C | **IA-L3-1.59** | D | D |
| **IA-L3-1.60** | C | B | **IA-L3-1.62** | D | C |
| **IA-L3-1.63** | ND | ND | **IA-L3-1.64** | ND | ND |
| **IA-L3-1.65** | ND | ND | **IA-L3-1.66** | ND | ND |
| **IA-L3-1.67** | D | C | **IA-L3-1.69** | C | B |
| **IA-L3-1.70** | C | B | **IA-L3-1.71** | C | B |
| **IA-L3-1.72** | ND | ND | **IA-L3-1.73** | ND | ND |
| **IA-L3-1.74** | ND | ND | **IA-L3-1.76** | ND | ND |
| **IA-L3-1.77** | ND | ND | **IA-L3-1.78** | ND | ND |
| **IA-L3-1.79** | ND | ND | **IA-L3-1.80** | ND | ND |
| **IA-L3-1.81** | ND | ND | **IA-L3-1.82** | ND | ND |
| **IA-L3-1.83** | C | C | **IA-L3-1.84** | D | D |
| **IA-L3-1.85** | C | C | **IA-L3-1.86** | ND | ND |
| **IA-L3-1.87** | ND | D | **IA-L3-1.88** | ND | ND |
| **IA-L3-1.89** | C | B | **IA-L3-1.90** | D | C |
| **IA-L3-1.91** | C | C | **IA-L3-1.94** | D | C |
| **IA-L3-1.95** | D | D | **IA-L3-1.96** | C | D |
| **IA-L3-1.97** | D | D | **IA-L3-1.98** | D | C |
| **IA-L3-1.99** | ND | C | **IA-L3-1.100** | ND | D |
| **IA-L3-1.101** | ND | ND | **IA-L3-1.102** | ND | C |
| **IA-L3-1.103** | D | D | **IA-L3-1.104** | D | D |
| **IA-L3-1.105** | D | D | **IA-L3-1.107** | D | D |
| **IA-L3-1.108** | ND | ND | **IA-L3-1.111** | ND | C |
| **IA-L3-1.112** | D | C | **IA-L3-1.113** | D | D |
| **IA-L3-1.114** | ND | ND | **IA-L3-1.115** | D | D |
| **IA-L3-1.116** | ND | ND | **IA-L3-1.117** | ND | ND |
| **IA-L3-1.118** | ND | ND | **IA-L3-1.119** | C | B |
| **IA-L3-1.120** | D | D | **IA-L3-1.121** | D | C |
| **IA-L3-1.122** | ND | B | **IA-L3-1.123** | C | B |
| **IB-L3-1.1** | C | B | **IB-L3-1.2** | C | B |
| **IB-L3-1.3** | ND | C | **IB-L3-1.4** | D | D |
| **IA-L3-2.1** | D | D | **IA-L3-2.2** | D | D |
| **IA-L3-2.3** | ND | ND | **IA-L3-2.4** | D | D |
| **IA-L3-2.5** | D | D | **IA-L4-1.1** | D | D |
| **IA-L4-1.2** | | | **IA-L4-1.3** | D | D |
| **IA-L4-1.4** | D | D | **IA-L4-1.5** | ND | ND |
| **IA-L4-1.6** | ND | ND | **IA-L4-1.7** | D | D |
| **IA-L4-1.8** | D | C | **IA-L4-1.9** | ND | B |
| **IA-L4-1.10** | ND | ND | **IA-L4-1.11** | C | B |
| **IA-L4-1.12** | C | C | **IB-L4-1.1** | C | B |
| **IA-L5-1-1.1** | ND | ND | **IA-L5-1-1.2** | ND | ND |
| **IA-L5-1-1.3** | ND | ND | **IA-L5-1-1.4** | ND | ND |
| **IA-L5-1-1.5** | ND | ND | **IA-L5-1-1.6** | ND | ND |
| **IA-L5-1-1.7** | ND | ND | **IA-L5-1-1.8** | ND | ND |
| **IA-L5-1-1.10** | ND | ND | **IA-L5-1-1.11** | ND | ND |
| **IA-L5-1-1.12** | ND | ND | **IA-L5-1-1.13** | ND | ND |
| **IA-L5-1-1.14** | ND | ND | **IA-L5-1-2.1** | ND | ND |
| **IA-L5-1-2.2** | ND | ND | **IA-L5-2-1.1** | C | B |
| **IA-L5-2-1.2** | C | C | **IB-L5-2-1.1** | B | A |
| **IB-L5-2-1.2** | C | B | **IA-L6-1.1** | C | B |
| **IA-L8-1.1** | C | C | **IA-L9-1.1** | ND | ND |
| **IA-L9-1.2** | ND | ND | **IA-L9-1.3** | ND | ND |
| **IA-L9-1.4** | D | C | **IA-L11-1.1** | C | B |
| **IA-L12-1.1** | C | B | | | |

All references (patent and non-patent) cited above are incorporated by reference into this patent application. The discussion of those references is intended merely to summarize the assertions made by their authors. No admission is made that any reference (or a portion of ay reference) is relevant prior art (or prior art at all). Applicants reserve the right to challenge the accuracy and pertinence of the cited references.

Embodiments of the present invention include:
1. A compound or salt thereof, wherein:
   the compound corresponds in structure to formula **I**: 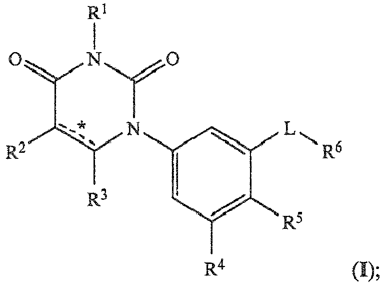
      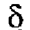 is selected from the group consisting of single carbon-carbon bond and double carbon-carbon bond;
      **R¹** is selected from the group consisting of hydrogen, methyl, and nitrogen-protecting group;
      **R²** is selected from the group consisting of hydrogen, halo, hydroxy, methyl, cyclopropyl, and cyclobutyl;
      **R³** is selected from the group consisting of hydrogen, halo, oxo, and methyl;
      **R⁴** is selected from the group consisting of halo, alkyl, alkenyl, alkynyl, nitro, cyano, azido, alkyloxy, alkenyloxy, alkynyloxy, amino, aminocarbonyl, aminosulfonyl, alkylsulfonyl, carbocyclyl, and heterocyclyl, wherein:
         (**a**) the amino, aminocarbonyl, and aminosulfonyl optionally are substituted with:
            (**1**) one or two substituents independently selected from the group consisting of alkyl, alkenyl, alkynyl, and alkylsulfonyl, or
            (**2**) two substituents that, together with the amino nitrogen, form a single-ring heterocyclyl, and
         (**b**) the alkyl, alkenyl, alkynyl, alkyloxy, alkenyloxy, alkynyloxy, and alkylsulfonyl, optionally are substituted with one or more substituents independently selected from the group consisting of halo, oxo, nitro, cyano, azido, hydroxy, amino, alkyloxy, trimethylsilyl, carbocyclyl, and heterocyclyl, wherein:
            the amino optionally is substituted with:
               (**1**) one or two substituents independently selected from the group consisting of alkyl, alkenyl, alkynyl, alkylcarbonyl, alkylsulfonyl, alkyloxycarbonyl, carbocyclyl, heterocyclyl, carbocyclylalkyl, and heterocyclylalkyl, or
               (**2**) two substituents that, together with the amino nitrogen, form a single-ring heterocyclyl, and
         (**c**) the carbocyclyl and heterocyclyl optionally are substituted with up to three substituents independently selected from the group consisting of alkyl, alkenyl, alkynyl, halo, oxo, nitro, cyano, azido, hydroxy, amino, alkyloxy, trimethylsilyl, carbocyclyl, and heterocyclyl, wherein:
            the amino optionally is substituted with:
               (**1**) one or two substituents independently selected from the group consisting of alkyl, alkenyl, alkynyl, alkylcarbonyl, alkylsulfonyl, alkyloxycarbonyl, carbocyclyl, heterocyclyl, carbocyclylalkyl, and heterocyclylalkyl, or
               (**2**) two substituents that, together with the amino nitrogen, form a single-ring heterocyclyl;
      **R⁵** is selected from the group consisting of hydrogen, hydroxy, alkyl, alkenyl, alkynyl, alkyloxy, alkenyloxy, alkynyloxy, alkylsulfonyloxy, carbocyclylsulfonyloxy, haloalkylsulfonyloxy, and halo;
         as to **L** and **R⁶**;
      **L** is a bond, and **R⁶** is selected from the group consisting of fused 2-ring carbocyclyl and fused 2-ring heterocyclyl, wherein each such substituent optionally is substituted with one or more substituents independently selected from the group consisting of **R^{E}, R^{F}, R^{G}, R^{H}, R^{I}, R^{J},** and **R^{K},** or
         **L** is selected from the group consisting of C(**R^{A}**)=C(**R^{B}**), C≡C, C(O)N(**R^{C}**), N(**R^{D}**)C(O), C₁-C₂-alkylene, C(H)₂O, OC(H)₂, cyclopropyl-1,2-ene, C(H)₂N(**R^{L}**), N(**R^{M}**)C(H)₂, C(O)CH₂, and CH₂C(O), and **R⁶** is selected from the group consisting of C₅-C₆-carbocyclyl and 5-6-membered heterocyclyl, wherein each such substituent optionally is substituted with one or more substituents independently selected from the group consisting **of R^{E}, R^{F}, R^{G}, R^{H}, R^{I}, R^{J},** and **R^{K}**; **R^{A}, R^{B}, R^{L},** and **R^{M}** are independently selected from the group consisting of hydrogen, C₁-C₆-alkyl, C₁-C₆-alkyloxy, C₃-C₈-cycloalkyl, and halo, wherein:
         the C₁-C₆-alkyl optionally is substituted with one or more substituents independently selected from the group consisting of carboxy, halo, hydroxy, nitro, oxo, amino, cyano, alkyloxycarbonyl, alkylcarbonyloxy, alkyloxy, carbocyclyl, and heterocyclyl;
      **R^{C}** is selected from the group consisting of hydrogen and alkyl;
      **R^{D} is** selected from the group consisting of hydrogen and alkyl;
      each **R^{E}** is independently selected from the group consisting of halo, nitro, hydroxy, oxo, carboxy, cyano, amino, imino, azido, and aldehydo, wherein:
      the amino optionally is substituted with one or two substituents independently selected from the group consisting of alkyl, alkenyl, and alkynyl;
         each **R^{F}** is independently selected from the group consisting of alkyl, alkenyl, and alkynyl,
   wherein:
   each such substituent optionally is substituted with one or more substituents independently selected from the group consisting of carboxy, hydroxy, halo, amino, imino, nitro, azido, oxo, aminosulfonyl, alkylsulfonyl, alkyloxycarbonyl, alkenyloxycarbonyl, alkynyloxycarbonyl, alkylcarbonyloxy, alkenylcarbonyloxy, alkynylcarbonyloxy, alkyloxy, alkenyloxy, alkynyloxy, carbocyclyl, heterocyclyl, cyano, and aminocarbonyl, wherein:
      the amino, imino, aminosulfonyl, aminocarbonyl, carbocyclyl, and heterocyclyl optionally are substituted with one or two substituents independently selected from the group consisting of alkyl, alkenyl, alkynyl, alkylsulfonyl, alkenylsulfonyl, alkynylsulfonyl, alkylsulfonylamino, hydroxy, and alkyloxy,
   wherein:
      amino portion of the alkylsulfonylamino optionally is substituted with a substituent selected from the group consisting of alkyl, alkenyl, and alkynyl;
      each **R^{G}** is independently selected from the group consisting of carbocyclyl and heterocyclyl,
   wherein:
   each such substituent optionally is substituted with one or more substituents independently selected from the group consisting of alkyl, alkenyl, alkynyl, carboxy, hydroxy, halo, amino, nitro, azido, oxo, aminosulfonyl, alkyloxycarbonyl, alkenyloxycarbonyl, alkynyloxycarbonyl, alkylcarbonyloxy, alkenylcarbonyloxy, alkynylcarbonyloxy, alkyloxy, alkenyloxy, alkynyloxy, carbocyclyl, heterocyclyl, cyano, and aminocarbonyl, wherein:
      the amino, aminosulfonyl, and aminocarbonyl optionally are substituted with one or two substituents independently selected from the group consisting of alkyl, alkenyl,
      alkynyl, alkylsulfonyl, alkenylsulfonyl, and alkynylsulfonyl;
      each **R^{H}** is independently selected from the group consisting of alkyloxy, alkenyloxy, alkynyloxy, alkylsulfonyloxy, alkenylsulfonyloxy, and alkynylsulfonyloxy, wherein:
   each such substituent optionally is substituted with one or more substituents independently selected from the group consisting of carboxy, hydroxy, halo, amino, nitro, azido, oxo, aminosulfonyl, alkyloxycarbonyl, alkenyloxycarbonyl, alkynyloxycarbonyl, alkylcarbonyloxy, alkenylcarbonyloxy, alkynylcarbonyloxy, alkyloxy, alkenyloxy, alkynyloxy, carbocyclyl, heterocyclyl, cyano, and aminocarbonyl, wherein:
      the amino, aminosulfonyl, and aminocarbonyl optionally are substituted with one or two substituents independently selected from the group consisting of alkyl, alkenyl,
      alkynyl, alkylsulfonyl, alkenylsulfonyl, and alkynylsulfonyl;
      each **R^{I}** is independently selected from the group consisting of alkylcarbonyl, alkenylcarbonyl, alkynylcarbonyl, aminocarbonyl, alkyloxycarbonyl, carbocyclylcarbonyl, and heterocyclylcarbonyl, wherein:
      (**a**) the alkylcarbonyl, alkenylcarbonyl, and alkynylcarbonyl optionally are substituted with one or more substituents independently selected from the group consisting of carboxy, hydroxy, halo, amino, nitro, azido, oxo, aminosulfonyl, alkyloxycarbonyl, alkenyloxycarbonyl, alkynyloxycarbonyl, alkylcarbonyloxy, alkenylcarbonyloxy, alkynylcarbonyloxy, alkyloxy, alkenyloxy, alkynyloxy, carbocyclyl, heterocyclyl, cyano, and aminocarbonyl, and
      (**b**) the aminocarbonyl optionally is substituted with one or two substituents independently selected from the group consisting of alkyl, alkenyl, alkynyl, alkyloxyalkyl, carbocyclyl, heterocyclyl, alkylsulfonyl, and alkylsulfonylamino, wherein:
         the carbocyclyl and heterocyclyl optionally are substituted with one or two substituents independently selected from the group consisting of halo, alkyl, and oxo;
      each **R^{J}** is independently selected from the group consisting of carbocyclylsulfonylamino, heterocyclylsulfonylamino, alkylcarbonylamino, alkenylcarbonylamino, alkynylcarbonylamino, alkyloxycarbonylamino, alkenyloxycarbonylamino, alkynyloxycarbonylamino, alkylsulfonylamino, alkenylsulfonylamino, alkynylsulfonylamino, aminocarbonylamino, alkyloxycarbonylaminoimino, alkylsulfonylaminoimino, alkenylsulfonylaminoimino, and alkynylsulfonylaminoimino, wherein:
      (**a**) the amino portion of such substituents optionally is substituted with a substituent independently selected from the group consisting of carbocyclylalkyl, heterocyclylalkyl, alkylcarbonyloxy, aminocarbonylalkyl, alkyl, alkenyl, alkynyl, alkylcarbonyl, alkenylcarbonyl, alkynylcarbonyl, alkyloxycarbonyl, alkyloxyalkyloxycarbonyl, alkylcarbonyloxyalkyl, and alkylsulfonyl, wherein:
         (**1**) the carbocyclyl portion of the carbocyclylalkyl and the heterocyclyl portion of the heterocyclylalkyl optionally are substituted with one or more substituents independently selected from the group consisting of alkyl, alkenyl, alkynyl, carboxy, hydroxy, alkyloxy, alkenyloxy, alkynyloxy, halo, nitro, cyano, azido, oxo, and amino, and
         (**2**) the amino portion of the aminocarbonylalkyl optionally is substituted with one or two substituents independently selected from the group consisting of alkyl, alkenyl, and alkynyl,
      (b) the alkyl, alkenyl, and alkynyl portion of such substituents optionally is substituted with one or more substituents independently selected from the group consisting of carboxy, halo, oxo, amino, alkyloxycarbonyl, alkylcarbonyloxy, hydroxy, alkyloxy, carbocyclyl, heterocyclyl, and cyano, wherein:
         the amino optionally is substituted with one or two substituents independently selected from the group consisting of alkyl, alkenyl, alkynyl, alkyloxy, alkenyloxy, and alkynyloxy, wherein:
            the alkyl optionally is substituted with one or more hydroxy;
      (c) the carbocyclyl and heterocyclyl portions of such substituents optionally are substituted with one or more substituents independently selected from the group consisting of alkyl, alkenyl, alkynyl, carboxy, hydroxy, alkyloxy, alkenyloxy, alkynyloxy, halo, nitro, cyano, azido, and amino, wherein:
         the amino optionally is substituted with one or two substituents independently selected from the group consisting of alkyl, alkenyl, and alkynyl; and
      each **R^{K}** is independently selected from the group consisting of aminosulfonyl, alkylsulfonyl, alkenylsulfonyl, and alkynylsulfonyl, wherein:
      (**a**) the alkylsulfonyl, alkenylsulfonyl, and alkynylsulfonyl optionally are substituted with one or more substituents independently selected from the group consisting of carboxy, hydroxy, halo, amino, nitro, azido, oxo, aminosulfonyl, alkyloxycarbonyl, alkenyloxycarbonyl, alkynyloxycarbonyl, alkylcarbonyloxy, alkenylcarbonyloxy, alkynylcarbonyloxy, alkyloxy, alkenyloxy, alkynyloxy, carbocyclyl, heterocyclyl, cyano, and aminocarbonyl, wherein:
         the amino, aminosulfonyl, and aminocarbonyl optionally are substituted with one or two substituents independently selected from the group consisting of alkyl, alkenyl, and alkynyl; and
      (**b**) the aminosulfonyl optionally is substituted with one or two substituents independently selected from the group consisting of alkyl, alkenyl, and alkynyl.
2. The compound or salt of 1, wherein:
   **R¹** is selected from the group consisting of hydrogen and methyl;
   **R² is** selected from the group consisting of hydrogen and halo;
   **R³** is selected from the group consisting of hydrogen and halo;
   **R⁴** is selected from the group consisting of C₁-C₄-alkyl, C₃-C₆-carbocyclyl, and 5-6-membered heterocyclyl, wherein:
      (**a**) the C₁-C₄-alkyl optionally is substituted with up to three substituents independently selected from the group consisting of halo, oxo, hydroxy, alkyloxy, and trimethylsilyl, and
      (**b**) the C₃-C₆-carbocyclyl and 5-6-membered heterocyclyl optionally are substituted with one or two substituents independently selected from the group consisting of alkyl, halo, and alkylsulfonylamino;
   **R⁵** is selected from the group consisting of hydrogen, hydroxy, alkyloxy, and halo; as to **L** and **R⁶**:
      **L** is a bond, and **R⁶** is selected from the group consisting of fused 2-ring carbocyclyl and fused 2-ring heterocyclyl, wherein each such substituent is substituted with one, two, three substituents independently selected from the group consisting of **R^{E}**, **R^{F}**, **R^{G}**, **R^{H}**, **R^{I}**, **R^{J}**, and **R^{K}**,
      or
      **L** is selected from the group consisting of C(**R^{A}**)=C(**R^{B}**), C≡C, C(O)N(**R^{C}**), N(**R^{D}**)C(O), C₁-C₂-alkylene, C(H)₂O, OC(H)₂, cyclopropyl-1,2-ene, C(H)₂N(**R^{L}**), N(**R^{M}**)C(H)₂, C(O)CH₂, and CH₂C(O), and **R⁶** is selected from the group consisting of C₅-C₆-carbocyclyl and 5-6-membered heterocyclyl, wherein each such substituent is substituted with one two, or three substituents independently selected from the group consisting of **R^{E}**, **R^{F}**, **R^{G}**, **R^{H}**, **R^{I}**, **R^{J}**, and **R^{K}**; one of **R^{A}** and **R^{B}** is hydrogen, and the other is selected from the group consisting of hydrogen, methyl, methoxy, and halo;
   **R^{C}** is selected from the group consisting of hydrogen and methyl;
   **R^{D}** is selected from the group consisting of hydrogen and methyl;
      each **R^{E}** is independently selected from the group consisting of chloro, fluoro, nitro, hydroxy, oxo, carboxy, amino, imino, aldehydo, and alkylamino;
      each **R^{F}** is an independently selected alkyl optionally substituted with a substituent selected from the group consisting of carboxy, halo, amino, imino, and aminosulfonyl, wherein:
      the amino, imino, and aminosulfonyl, optionally are substituted with one or two substituents independently selected from the group consisting of alkyl, alkylsulfonyl, and alkylsulfonylamino;
   each **R^{I}** is independently selected from the group consisting of alkylcarbonyl and aminocarbonyl, wherein:
   the aminocarbonyl optionally is substituted with a substituent selected from the group consisting of alkyl, alkyloxyalkyl, alkylsulfonyl, and alkylsulfonylamino;
   each **R^{J}** is independently selected from the group consisting of alkylsulfonylamino, alkenylsulfonylamino, alkynylsulfonylamino, and alkylsulfonylaminoimino, wherein:
   (**a**) the amino portion of such substituents optionally is substituted with a substituent independently selected from the group consisting of carbocyclylalkyl, heterocyclylalkyl, alkylcarbonyloxy, aminocarbonylalkyl, alkyl, alkylcarbonyl, alkyloxycarbonyl, alkyloxyalkyloxycarbonyl, alkylcarbonyloxyalkyl, and alkylsulfonyl, wherein:
      (**1**) the carbocyclyl portion of the carbocyclylalkyl and the heterocyclyl portion of the heterocyclylalkyl optionally are substituted with one or two substituents independently selected from the group consisting of alkyl, carboxy, hydroxy, alkyloxy, halo, nitro, cyano, oxo, and amino, and
      (**2**) the amino portion of the aminocarbonylalkyl optionally is substituted with one or two substituents independently selected from the group consisting of alkyl, alkenyl, and alkynyl,
   (**b**) the alkyl, alkenyl, and alkynyl portion of such substituents optionally is substituted with one or two substituents independently selected from the group consisting of carboxy, halo, oxo, amino, alkyloxycarbonyl, alkylcarbonyloxy, hydroxy, alkyloxy, carbocyclyl, heterocyclyl, and cyano, wherein:
      the amino optionally is substituted with one or two substituents independently selected from the group consisting of alkyl and alkyloxy, wherein:
         the alkyl optionally is substituted with one or more hydroxy;
   each **R^{K}** is independently selected from the group consisting of aminosulfonyl and alkylsulfonyl, wherein:
   (**a**) the alkylsulfonyl optionally is substituted with one or two substituents independently selected from the group consisting of carboxy, hydroxy, halo, amino, nitro, oxo, aminosulfonyl, alkyloxycarbonyl, alkylcarbonyloxy, alkyloxy, carbocyclyl, heterocyclyl, cyano, and aminocarbonyl; and
   (**b**) the aminosulfonyl optionally is substituted with one or two substituents independently selected alkyl;
   **R^{L}** is hydrogen; and
   **R^{M}** is hydrogen.
3. The compound or salt of any one of **1** and **2**, wherein:
   **R¹** is hydrogen;
   **R²** is selected from the group consisting of hydrogen and halo;
   **R³** is hydrogen;
   **R⁴** is tert-butyl;
   **R⁵** is selected from the group consisting of hydroxy and methoxy;
   **R^{A}** is hydrogen;
   **R^{B}** is hydrogen;
   **R^{C}** is hydrogen; and
   **R^{D}** is hydrogen.
4. The compound or salt of any one of **1-3**, wherein:
   **L** is a bond; and
   **R⁶** is selected from the group consisting of fused 2-ring heterocyclyl and fused 2-ring carbocyclyl, wherein each such substituent is substituted with one, two, or three substituents independently selected from the group consisting of **R^{E}**, **R^{F}**, and **R^{J}**.
5. The compound or salt of any one of **1-4**, wherein:
   **R⁶** is selected from the group consisting of fused 2-ring heterocyclyl and fused 2-ring carbocyclyl, wherein each such substituent is substituted with a substituent selected from the group consisting of **R^{F}** and **R^{J}**;
   **R^{F}** is alkylsulfonylaminoalkyl; and
   **R^{J}** is alkylsulfonylamino.
6. The compound or salt of any one of **1-5**, wherein the substituted fused 2-ring carbocyclyl is selected from the group consisting of naphthalenyl, dihydronaphthalenyl, tetrahydronaphthalenyl, hexahydronaphthalenyl, octahydronaphthalenyl, decahydronaphthalenyl, indenyl, dihydroindenyl, hexahydroindenyl, octahydroindenyl, pentalenyl, octahydropentalenyl, and hexahydropentalenyl.
7. The compound or salt of any one of **1-5**, wherein the substituted fused 2-ring heterocyclyl is selected from the group consisting of: 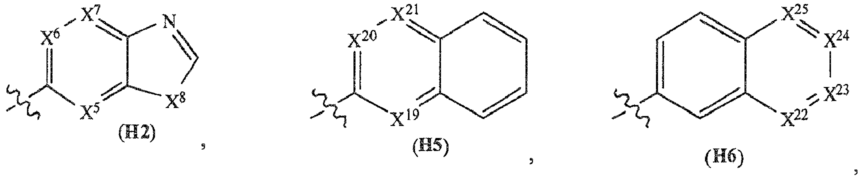 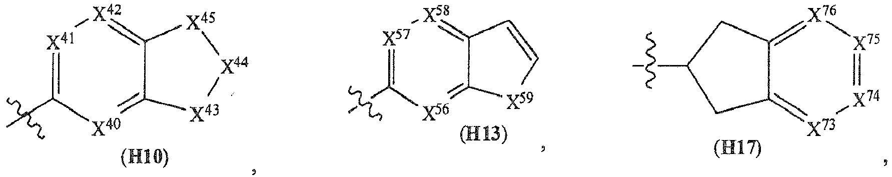 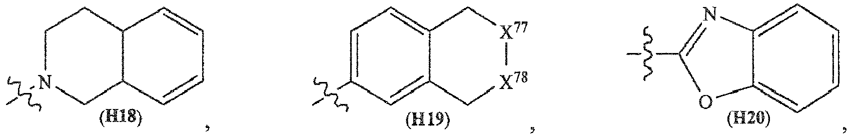 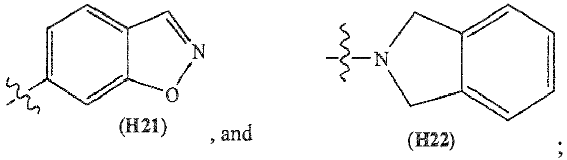
   **X⁵**, **X⁶**, and **X⁷** are independently selected from the group consisting of N and C(H);
   **X⁸** is selected from the group consisting of N(H), O, and S;
   one or more of **X¹⁹**, **X²⁰**, and **X²¹** is N, and the remaining one(s) is/are C(H);
   one or more of **X²²**, **X²³**, **X²⁴**, and **X²⁵** is N, and the remaining one(s) is/are C(H);
   **X⁴⁰**, **X**^{**4**1}, and **X⁴²** are independently selected from the group consisting of N and C(H);
   one of **X⁴³**, **X⁴⁴**, and **X⁴⁵** is selected from the group consisting of N(H), O, and S, and the remaining two are C(H)₂;
   **X⁵⁶**, **X⁵⁷**, and **X⁵⁸** are independently selected from the group consisting of N and C(H);
   one or more of **X⁷³**, **X⁷⁴**, **X⁷⁵**, and **X⁷⁶** is N, and the remaining one(s) is/are C(H); and
   one of **X⁷⁷** and **X⁷⁸** is N(H), and the remaining one is C(H)₂.
8. The compound or salt of any one of **1-3**, wherein:
   **L** is selected from the group consisting of C(**R^{A}**)=C(**R^{B}**), ethylene, and cyclopropyl-1,2-ene; and
   **R⁶** is selected from the group consisting of C₅-C₆-carbocyclyl and 5-6-membered heterocyclyl, wherein each such substituent is substituted with one, two, or three substituents independently selected from the group consisting of **R^{E}**, **R^{F}**, and **R^{J}**.
9. The compound or salt of any one of **1-3**, wherein:
   **L** is selected from the group consisting of C≡C, C(O)N(**R^{C}**), N(**R^{D}**)C(O), C(H)₂O, OC(H)₂, C(H)₂N(**R^{L}**), N(**R^{M}**)C(H)₂, C(O)CH₂, and CH₂C(O); and
   **R⁶** is selected from the group consisting of C₅-C₆-carbocyclyl and 5-6-membered heterocyclyl, wherein each such substituent is substituted with one, two, or three substituents independently selected from the group consisting of **R^{E}**, **R^{F}**, and **R^{J}**.
10. The compound or salt of any one of **1-3** and **9**, wherein **L** is C≡C.
11. The compound or salt of any one of **1-3**, and **8-10**, wherein:
   **R⁶** is phenyl substituted with a substituent selected from the group consisting of **R^{F}** and **R^{J}**;
   **R^{F}** is alkylsulfonylaminoalkyl; and
   **R^{J}** is alkylsulfonylamino.
12. A compound or salt thereof, wherein the compound is selected from the group of compounds shown in Examples **1-140**.
13. N-(6-(3-tert-butyl-5-(2,4-dioxo-3,4-dihydropyrimidin-1(2H)-yl)-2-methoxyphenyl) naphthalen-2-yl)methanesulfonamide crystalline form selected from the group consisting of:
   crystalline N-(6-(3-tert-butyl-5-(2,4-dioxo-3,4-dihydropyrimidin-1(2H)-yl)-2-methoxyphenyl) naphthalen-2-yl)mcthanesulfonamide ethanol solvate having an X-ray powder diffraction pattern comprising one or more peaks selected from the group consisting of 8.3±0.2, 9.7±0.2, 10.6±0.2, 13.6±0.2, 17.2±0.2, 19.2±0.2, 22.7±0.2, 26.9±0.2, and 29.4±0.2 degrees 2θ;
   crystalline N-(6-(3-tert-butyl-5-(2,4-dioxo-3,4-dihydropyrimidin-1(2H)-yl)-2-methoxyphenyl) naphthalen-2-yl)methanesulfonamide ethanol solvate having an X-ray powder diffraction pattern comprising one or more peaks selected from the group consisting of 8.3±0.2, 9.7±0.2, 18.0±0.2, 18.6±0.2, 13.6±0.2, 17.2±0.2, 17.5±0.2, 19.2±0.2, 19.4±0.2, 22.7±0.2, 26.9±0.2, and 29.4±0.2 degrees 2θ;
   crystalline N-(6-(3-tert-butyl-5-(2,4-dioxo-3,4-dihydropyrimidin-1(2H)-yl)-2-methoxy-phenyl)naphthalen-2-yl)methanesulfonamide ethanol solvate having an X-ray powder diffraction pattern substantially as shown in Figure **1**;
   crystalline N-(6-(3-tert-butyl-5-(2,4-dioxo-3,4-dihydropyrimidin-1(2H)-yl)-2-methoxyphenyl) naphthalen-2-yl)methanesulfonamide acetonitrile solvate having an X-ray powder diffraction pattern comprising one or more peaks selected from the group consisting of 5.3±0.2, 8.3±0.2, 9.7±0.2, 10.5±0.2, 13.8±0.2, 17.2±0.2, 19.1±0.2, and 19.5±0.2 degrees 2θ;
   crystalline N-(6-(3-tert-butyl-5-(2,4-dioxo-3,4-dihydropyrimidin-1(2H)-yl)-2-methoxyphenyl) naphthalen-2-yl)methanesulfonamide acetonitrile solvate having an X-ray powder diffraction pattern comprising one or more peaks selected from the group consisting of 5.3±0.2, 8.3±0.2, 9.7±0.2, 10.5±0.2, 13.8±0.2, 17.2±0.2, 17.7±0.2, 19.1±0.2, 19.5±0.2, 22.0±0.2, 22.8±0.2, and 27.2±0.2 degrees 2θ;
   crystalline N-(6-(3-tert-butyl-5-(2,4-dioxo-3,4-dihydropyrimidin-1(2H)-yl)-2-methoxyphenyl) naphthalen-2-yl)methanesulfonamide acetonitrile solvate having an X-ray powder diffraction pattern substantially as shown in Figure **3**;
   crystalline N-(6-(3-tert-butyl-5-(2,4-dioxo-3,4-dihydropyrimidin-1(2H)-yl)-2-methoxyphenyl) naphthalen-2-yl)methanesulfonamide ethyl acetate solvate having an X-ray powder diffraction pattern comprising one or more peaks selected from the group consisting of 7.9±0.2, 9.3±0.2, 9.7±0.2, 10.6±0.2, 18.7±7.2, 38.5±0.2, and 44.7±0.2 degrees 2θ;
   crystalline N-(6-(3-tert-butyl-5-(2,4-dioxo-3,4-dihydropyrimidin-1(2H)-yl)-2-methoxyphenyl) naphthalen-2-yl)methanesulfonamide ethyl acetate solvate having an X-ray powder diffraction pattern comprising one or more peaks selected from the group consisting of 7.9±0.2, 9.3±0.2, 9.7±0.2, 10.6±0.2, 13.7±0.2, 17.4±0.2, 18.7±0.2, 21.7±0.2, 22.0±0.2, 28.2±0.2, 38.5±0.2, and 44.7±0.2 degrees 2θ.
   crystalline N-(6-(3-tert-butyl-5-(2,4-dioxo-3,4-dihydropyrimidin-1(2H)-yl)-2-methoxyphenyl) naphthalen-2-yl)methanesulfonamide ethyl acetate having an X-ray powder diffraction pattern substantially as shown in Figure **4**;
   crystalline N-(6-(3-tert-butyl-5-(2,4-dioxo-3,4-dihydropyrimidin-1(2H)-yl)-2-methoxyphenyl) naphthalen-2-yl)methanesulfonamide 2-propanol solvate having an X-ray powder diffraction pattern comprising one or more peaks selected from the group consisting of 8.2±0.2, 9.3±0.2, 10.1±0.2, 16.3±0.2, 18.1±0.2, 18.6±0.2, 19.4±0.2, 21.6±0.2, and 22.5±0.2 degrees 2θ;
   crystalline N-(6-(3-tert-butyl-5-(2,4-dioxo-3,4-dihydropyrimidin-1(2H)-yl)-2-methoxyphenyl) naphthalen-2-yl)methanesulfonamide 2-propanol solvate having an X-ray powder diffraction pattern comprising one or more peaks selected from the group consisting of 8.2±0.2, 9.3±0.2, 10.1±0.2, 16.3±0.2, 18.1±0.2, 18.6±0.2, 19.4±0.2, 21.6±0.2, 22.5±0.2, 23.8±0.2, 26.0±0.2, and 28.0±0.2 degrees 2θ;
   crystalline N-(6-(3-tert-butyl-5-(2,4-dioxo-3,4-dihydropyrimidin-1(2H)-yl)-2-methoxyphenyl)naphthalen-2-yl)methanesulfonamide 2-propanol solvate having an X-ray powder diffraction pattern substantially as shown in Figure **5**;
   crystalline N-(6-(3-tert-butyl-5-(2,4-dioxo-3,4-dihydropyrimidin-1(2H)-yl)-2-methoxyphenyl) naphthalen-2-yl)methanosulfonamide methanol solvate having an X-ray powder diffraction pattern comprising one or more peaks selected from the group consisting of 8.4±0.2, 9.7±0.2, 10.1±0.2, 13.8±0.2, 17.4±0.2, 19.3±0.2, and 19.6±0.2 degrees 2θ;
   crystalline N-(6-(3-tert-butyl-5-(2,4-dioxo-3,4-dihydropyrimidin-1(2H)-yl)-2-methoxyphenyl) naphthalen-2-yl)methanesulfonamide methanol solvate having an X-ray powder diffraction pattern comprising one or more peaks selected from the group consisting of 8.4±0.2, 9.7±0.2, 10.1±0.2, 13.5±0.2, 13.8±0.2, 17.4±0.2, 19.3±0.2, 19.6±0.2, and 27.1±0.2 degrees 2θ;
   crystalline N-(6-(3-tert-butyl-5-(2,4-dioxo-3,4-dihydropyrimidin-1(2H)-yl)-2-methoxyphenyl) naphthalen-2-yl)methanesulfonamide methanol solvate having an X-ray powder diffraction pattern substantially as shown in Figure **6**;
   crystalline N-(6-(3-tert-butyl-5-(2,4-dioxo-3,4-dihydropyrimidin-1(2H)-yl)-2-methoxyphenyl) naphthalen-2-yl)methanesulfonamide 1-propanol solvate having an X-ray powder diffraction pattern comprising one or more peaks selected from the group consisting of 8.2±0.2, 9.3±0.2, 10.1±0.2, 15.7±0.2, 16.2±0.2, 18.4±0.2, 19.3±0.2, 21.6±0.2, and 22.8±0.2 degrees 2θ;
   crystalline N-(6-(3-tert-butyl-5-(2,4-dioxo-3,4-dihydropyrimidin-1(2H)-yl)-2-methoxyphenyl) naphthalen-2-yl)methanesulfonamide 1-propanol solvate having an X-ray powder diffraction pattern comprising one or more peaks selected from the group consisting of 8.2±0.2, 9.3±0.2, 10.1±0.2, 10.5±0.2, 15.7±0.2, 16.2±0.2, 18.4±0.2, 18.6±0.2, 19.3±0.2, 21.0±0.2, 21.6±0.2, and 22.8±0.2 degrees 2θ;
   crystalline N-(6-(3-tert-butyl-5-(2,4-dioxo-3,4-dihydropyrimidin-1(2H)-yl)-2-methoxyphenyl) naphthalen-2-yl)methanesulfonamide 1-propanol solvate having an X-ray powder diffraction pattern substantially as shown in Figure **7**.
   crystalline solvent free N-(6-(3-tert-butyl-5-(2,4-dioxo-3,4-dihydropyrimidin-1(2H)-yl)-2-methoxyphenyl)naphthalen-2-yl)methanesulfonamide having an X-ray powder diffraction pattern comprising one or more peaks selected from the group consisting of 6.2±0.2, 7.9±0.2, 9.9±0.2, 16.2±0.2, and 18.3±0.2 degrees 2θ;
   crystalline solvent free N-(6-(3-tert-butyl-5-(2,4-dioxo-3,4-dihydropyrimidin-1(2H)-yl)-2-methoxy-phenyl)naphthalen-2-yl)methanesulfonamide having an X-ray powder diffraction pattern comprising one or more peaks selected from the group consisting of 6.2±0.2, 7.9±0.2, 9.9±0.2, 10.1±0.2, 14.9±0.2, 16.2±0.2, 18.3±0.2, 19.11±0.2, and 26.5±0.2 degrees 2θ;
   crystalline solvent free N-(6-(3-tert-butyl-5-(2,4-dioxo-3,4-dihydropyrimidin-1(2H)-yl)-2-methoxy-phenyl)naphthalen-2-yl)methanesulfonamide having an X-ray powder diffraction pattern substantially as shown in Figure **8**;
   crystalline N-(6-(3-tert-butyl-5-(2,4-dioxo-3,4-dihydropyrimidin-1(2H)-yl)-2-methoxyphenyl) naphthalen-2-yl)methanesulfonamide hydrate having an X-ray powder diffraction pattern comprising one or more peaks selected from the group consisting of 5.4±0.2, 12.9±0.2, 17.9±0.2, and 18.9±0.2 degrees 2θ;
   crystalline N-(6-(3-tert-butyl-5-(2,4-dioxo-3,4-dihydropyrimidin-1(2H-yl)-2-methoxyphenyl) naphthalen-2-yl)methanesulfonamide hydrate having an X-ray powder diffraction pattern comprising one or more peaks selected from the group consisting of 6.4±0.2, 12.9±0.2, 17.5±0.2, 17.9±0.2, 18.9±0.2, and 24.4±0.2 degrees 2θ;
   crystalline N-(6-(3-tert-butyl-5-(2,4-dioxo-3,4-dihydropyrimidin-1(2H)-yl)-2-methoxyphenyl) naphthalen-2-yl)methanesulfonamide hydrate having an X-ray powder diffraction pattern comprising one or more peaks selected from the group consisting of 6.4±0.2, 12.7±0.2, 12.9±0.2, 14.1±0.2, 15.7±0.2, 17.2±0.2, 17.5±0.2, 17.9±0.2, 18.9±0.2, 21.2±0.2, 24.4±0.2, and 25.0±0.2 degrees 2θ;
   crystalline N-(6-(3-tert-butyt-5-(2,4-dioxo-3,4-dihydropyrimidin-1(2H)-yl)-2-methoxyphenyl) naphthalen-2-yl)methanesulfonamide hydrate having an X-ray powder diffraction pattern substantially as shown in Figure **9**;

   crystalline pattern A N-(6-(3-tert-butyl-5-(2,4-dioxo-3,4-dihydropyrimidin-1(2H)-yl)-2-methoxy-phenyl)naphthalen-2-yl)methanesulfonamide monosodium salt having an X-ray powder diffraction pattern comprising one or more peaks selected from the group consisting of 4.6±0.2, 10.4±0.2, 12.0±0.2, 15.6±0.2, 18.6±0.2, 22.8±0.2, and 23.9±0.2 degrees 2θ;
   crystalline pattern A N-(6-(3-tert-butyl-5-(2,4-dioxo-3,4-dihydropyrimidin-1(2H)-yl)-2-methoxy-phenyl)naphthalen-2-yl)methanesulfonamide monosodium salt having an X-ray powder diffraction pattern comprising one or more peaks selected from the group consisting of 4.6±0.2, 10.4±0.2, 12.0±0.2, 15.6±0.2, 18.6±0.2, 22.8±0.2, 23.3±0.2, and 23.9±0.2 degrees 2θ;
   crystalline pattern A N-(6-(3-tert-butyl-5-(2,4-dioxo-3,4-dihydropyrimidin-1(2H)-yl)-2-methoxy-phenyl)naphthalen-2-yl)methanesulfonamide monosodium salt having an X-ray powder diffraction pattern comprising one or more peaks selected from the group consisting of 4.6±0.2, 10.4±0.2, 12.0±0.2, 15.6±0.2, 16.0±0.2, 18.6±0.2, 22.8±0.2, 23.3±0.2, 23.9±0.2, and 28.3±0.2 degrees 2θ;
   crystalline pattern A N-(6-(3-tert-butyl-5-(2,4-dioxo-3,4-dihydropyrimidin-1(2H)-yl)-2-methoxyphenyl)naphthalen-2-yl)methanesulfonamide monosodium salt having an X-ray powder diffraction pattern substantially as shown in Figure **10**;
   crystalline pattern B N-(6-(3-tert-butyl-5-(2,4-dioxo-3,4-dihydropyrimidin-1(2H)-yl)-2-methoxy-phenyl)naphthalen-2-yl)methanesulfonamide monosodium salt having an X-ray powder diffraction pattern comprising one or more peaks selected from the group consisting of 5.4±0.2, 10.8±0.2, 14.4±0.2, 16.3±0.2, 17.0±0.2, 21.6±0.2, 22.1±0.2, and 23.7±0.2 degrees 2θ;
   crystalline pattern B N-(6-(3-tert-butyl-5-(2,4-dioxo-3,4-dihydropyrimidin-1(2H)-yl)-2-methoxy-phenyl)naphthalen-2-yl)methanesulfonamide monosodium salt having an X-ray powder diffraction pattern comprising one or more peaks selected from the group consisting of 5.4±0.2, 10.8±0.2, 14.4±0.2, 16.3±0.2, 17.0±0.2, 18.8±0.2, 19.2±0.2, 19.6±0.2, 21.6±0.2, 22.1±0.2, 23.7±0.2, 28.8±0.2, 29.1±0.2, and 31.8±0.2 degrees 2θ;
   crystalline pattern B N-(6-(3-tert-butyl-5-(2,4-dioxo-3,4-dihydropyrimidin-1(2H)-yl)-2-methoxy-phenyl)naphthalen-2-yl)methanesulfonamide monosodium salt having an X-ray powder diffraction pattern comprising three or more peaks selected from the group consisting of 5.4±0.2, 10.8±0.2, 16.3±0.2, 22.1±0.2, and 23.7±0.2 degrees 2θ;
   crystalline pattern B N-(6-(3-tert-butyl-5-(2,4-dioxo-3,4-dihydropyrimidin-1(2H)-yl)-2-methoxy-phenyl)naphthalen-2-yl)methanesulfonamide monosodium salt having an X-ray powder diffraction pattern comprising peaks at 5.4±0.2, 10.8±0.2, 16.3±0.2, and 22.1±0.2 degrees 2θ;
   crystalline pattern C N-(6-(3-tert-butyl-5-(2,4-dioxo-3,4-dihydropyrimidin-1(2H)-yl)-2-methoxy-phenyl)naphthalen-2-yl)methanesulfonamide monosodium salt having an X-ray powder diffraction pattern comprising one or more peaks selected from the group consisting of 5.0±0.2, 12.0±0.2, 17.5±0.2, 18.8±0.2, and 22.7±0.2 degrees 2θ;
   crystalline pattern C N-(6-(3-tert-butyl-5-(2,4-dioxo-3,4-dihydropyrimidin-1(2H)-yl)-2-methoxy-phenyl)naphthalen-2-yl)methanesulfonamide monosodium salt having an X-ray powder diffraction pattern comprising one or more peaks selected from the group consisting of 5.0±0.2, 12.0±0.2, 17.5±0.2, 17.8±0.2, 18.8±0.2, and 22.7±0.2 degrees 2θ;
   crystalline pattern C N-(6-(3-tert-butyl-5-(2,4-dioxo-3,4-dihydropyrimidin-1(2H)-yl)-2-methoxy-phenyl)naphthalen-2-yl)methanesulfonamide monosodium salt having pattern C monosodium salt has an X-ray powder diffraction pattern substantially as shown in Figure **14**;
   crystalline N-(6-(3-tert-butyl-5-(2,4-dioxo-3,4-dihydropyrimidin-1(2H)-yl)-2-methoxyphenyl)-naphthalen-2-yl)mathanesulfonamide disodium salt having an X-ray powder diffraction pattern comprising one or more peaks selected from the group consisting of 4.8±0.2, 9.6±0.2, 10.5±0.2, 13.0±0.2, 14.6±0.2, 15.4±0.2, 16.8±0.2, and 23.0±0.2 degrees 2θ;
   crystalline N-(6-(3-tert-butyl-5-(2,4-dioxo-3,4-dihydropyrimidin-1(2H)-yl)-2-methoxyphenyl) naphthalen-2-yl)methanosulfonamide disodium salt having an X-ray powder diffraction pattern comprising one or more peaks selected from the group consisting of 4.8±0.2, 9.6±0.2, 10.5±0.2, 13.0±0.2, 14.6±0.2, 15.4±0.2, 16.8±0.2, 22.7±0.2, 23.0±0.2, and 23.3±0.2 degrees 2θ;
   crystalline N-(6-(3-tert-butyl-5-(2,4-dioxo-3,4-dihydropyrim)din-1(2H)-yl)-2-methoxyphenyl) naphthalen-2-yl)methanesulfonamide disodium salt having disodium salt has an X-ray powder diffraction pattern substantially as shown in Figure **15**;
   crystalline N-(6-(3-tert-butyl-5-(2,4-dioxo-3,4-dihydropyrimidin-1(2H)-yl)-2-methoxyphenyl) naphthalen-2-yl)methanesulfonamide monopotassium salt having an X-ray powder diffraction pattern comprising one or more peaks selected from the group consisting of 5.0±0.2, 9.9±0.2, 11.3±0.2, 13.3±0.2, 16.9±0.2, 18.1±0.2, 19.1±0.2, 20.0±0.2, 21.1±0.2, 23.5±0.2, 24.8±0.2, and 25.7±0.2 degrees 2θ;
   crystalline N-(6-(3-tert-butyl-5-(2,4-dioxo-3,4-dihydropyrimidin-1(2H)-yl)-2-methoxyphenyl) naphthalen-2-yl)methanesulfonamide monopotassium salt having monopotassium salt has an X-ray powder diffraction pattern comprising one or more peaks selected from the group consisting of 5.0±0.2, 9.9±0.2, 11.3±0.2, 13.3±0.2, 16.9±0.2, 18.1±0.2, 19.1±0.2, 20.0±0.2, 21.1±0.2, 21.5±0.2, 23.5±0.2, 24.8±0.2, and 25.7±0.2 degrees 2θ;
   crystalline N-(6-(3-tert-butyl-3-(2,4-dioxo-3,4-dihydropyrimidin-1(2H)-yl)-2-methoxyphenyl) naphthalen-2-yl)methanesulfonamide monopotassium salt having an X-ray powder diffraction pattern substantially as shown in Figure **17**;
   crystalline pattern A N-(6-(3-tert-butyl-5-(2,4-dioxo-3,4-dihydropyrimidin-1(2H)-yl)-2-methoxy-phenyl)naphthalen-2-yl)methanesulfonamide monocholine salt having an X-ray powder diffraction pattern comprising one or more peaks selected from the group consisting of 10.9±0.2, 12.1±0.2, 13.4±0.2, 15.5±0.2, 17.0±0.2, 17.8±0.2, 18.3±0.2, 19.5±0.2, and 21.9±0.2 degrees 2θ;
   crystalline pattern A N-(6-(3-tert-butyl-5-(2,4-dioxo-3,4-dihydropyrimidin-1(2H)-yl)-2-methoxy phenyl)naphthalen-2-yl)methanesulfonamide monocholine salt having an X-ray powder diffraction pattern comprising one or more peaks selected from the group consisting of 10.9±0.2, 12.1±0.2, 13.0±0.2, 13.4±0.2, 13.6±0.2, 15.5±0.2, 17.0±0.2, 17.8±0.2, 18.3±0.2, 19.5±0.2, 19.7±0.2, and 21.9±0.2 degrees 2θ;
   crystalline pattern A N-(6-(3-tert-butyl-5-(2,4-dioxo-3,4-dihydropyrimidin-1(2H)-yl)-2-methoxyphenyl)naphthalen-2-yl)methanesulfonamide monocholine salt having an X-ray powder diffraction pattern substantially as shown in Figure **19**;
   crystalline pattern B N-(6-(3-tert-butyl-5-(2,4-dioxo-3,4-dihydropyrimidin-1(2H)-yl)-2-methoxyphenyl)naphthalen-2-yl)methanesulfonamide monocholine salt having an X-ray powder diffraction pattern comprising one or more peaks selected from the group consisting of 8.0±0.2, 9.4±0.2, 11.0±0.2, 13.0±0.2, 13.7±0.2, 15.9±0.2, 17.0±0.2, 18.3±0.2, 18.9±0.2, 19.8±0.2, and 22.1±0.2 degrees 2θ;
   crystalline pattern B N-(6-(3-tert-butyl-5-(2,4-dioxo-3,4-dihydropyrimidin-1(2H)-yl)-2-methoxy-phenyl)naphthalen-2-yl)methanesulfonamide monocholine salt having an X-ray powder diffraction pattern comprising one or more peaks selected from the group consisting of 8.0±0.2, 9.4±0.2, 11.0±0.2, 13.0±0.2, 13.3±0.2, 13.7±0.2, 15.9±0.2, 17.0±0.2, 17.4±0.2, 18.3±0.2, 18.9±0.2, 19.8±0.2, 21.8±0.2, and 22.1±0.2 degrees 2θ;
   crystalline pattern B N-(6-(3-tert-butyl-5-(2,4-dioxo-3,4-dihydropyrimidin-1(2H)-yl)-2-methoxyphenyl)naphthalen-2-yl)methanesulfonamide monocholine salt having an X-ray powder diffraction pattern substantially as shown in Figure **21**;
   crystalline N-(6-(3-tert-butyl-5-(2,4-dioxo-3,4-dihydropyrimidin-1(2H)-yl)-2-methoxyphenyl) naphthalen-2-yl)methanesulfonamide dicholine salt having an X-ray powder diffraction pattern comprising one or more peaks selected from the group consisting of 8.6±0.2, 11.0±0.2, 12.9±0.2, 17.0±0.2, 17.5±0.2, 18.9±0.2, 19.8±0.2, and 21.9±0.2 degrees 2θ;
   crystalline N-(6-(3-tert-butyl-5-(2,4-dioxo-3,4-dihydropyrimidin-1(2H)-yl)-2-methoxyphenyl) naphthalen-2-yl)methanesulfonamide dicholine salt having an X-ray powder diffraction pattern comprising one or more peaks selected from the group consisting of 8.6±0.2, 11.0±0.2, 12.9±0.2, 17.0±0.2, 17.5±0.2, 18.9±0.2, 19.8±0.2, 21.9±0.2, and 22.1±0.2 degrees 2θ; and
   crystalline N-(6-(3-tert-butyl-5-(2,4-dioxo-3,4-dihydropyrimidin-1(2H)-yl)-2-methoxy-phenyl)naphthalen-2-yl)methanesulfonamide dicholine salt having an X-ray powder diffraction pattern substantially as shown in Figure **23**.
14. (E)-N-(4-(3-tert-butyl-5-(2,4-dioxo-3,4-dihydropyrimidin-1(2H)-yl)-2-methoxy-styryl)phenyl)methanesulfonamide crystalline form selected from the group consisting of:
   crystalline (E)-N-(4-(3-tert-butyl-5-(2,4-dioxo-3,4-dihydropyrimidin-1(2H)-yl)-2-methoxystyryl) phenyl)methanesulfonamide disodium salt nonahydrate having an X-ray powder diffraction pattern comprising one or more peaks selected from the group consisting of 4.3±0.2, 10.4±0.2, 10.9±0.2, 11.6±0.2, 12.9±0.2, 14.7±0.2, 16.4±0.2, 17.8±0.2, 19.4±0.2, 19.8±0.2, 20.8±0.2, 21.9±0.2, and 23.5±0.2 degrees 2θ;
   crystalline (E)-N-(4-(3-tert-butyl-5-(2,4-dioxo-3,4-dihydropyrimidin-1(2H)-yl)-2-methoxystyryl) phenyl)methanesulfonamide disodium salt nonahydrate having an X-ray powder diffraction pattern comprising one or more peaks selected from the group consisting of 4.3±0.2, 10.4±0.2, 10.9±0.2, 11.6±0.2, 12.9±0.2, 14.7±0.2, 14.9±0.2, 16.4±0.2, 17.8±0.2, 19.4±0.2, 19.7±0.2, 19.8±0.2, 20.8±0.2, 20.9±0.2, 21.9±0.2, 22.1±0.2, and 23.5±0.2 degrees 2θ;
   crystalline (E)-N-(4-(3-tert-butyl-5-(2,4-dioxo-3,4-dihydropyrimidin-1(2H)-yl)-2-methoxystyryl) phenyl)methanesulfonamide disodium salt nonahydrate having an X-ray powder diffraction pattern substantially as shown in Figure **24**;
   crystalline (E)-N-(4-(3-tert-butyl-5-(2,4-dioxo-3,4-dihydropyrimidin-1(2H)-yl)-2-methoxystyryl) phenyl)methanesulfonamide nonahydrate disodium salt having cell unit parameters, wherein a is 8.9Å, b is 9.4Å, and c is 20.7Å;
   crystalline (E)-N-(4-(3-tert-butyl-5-(2,4-dioxo-3,4-dihydropyrimidin-1(2H)-yl)-2-methoxystryl) phenyl)methanesulfonamide disodium salt tetrahydrate having an X-ray powder diffraction pattern comprising one or more peaks selected from the group consisting of 4.8±0.2, 12.1±0.2, 14.0±0.2, 17.0±0.2, 17.5±0.2, 20.9±0.2, 21.6±0.2, 25.0±0.2, and 29.5±0.2 degrees 2θ;
   crystalline (E)-N-(4-(3-tert-butyl-5-(2,4-dioxo-3,4-dihydropyrimidin-1(2H)-yl)-2-methoxystyryl) phenyl)methanesulfonamide disodium salt tetrahydrate having an X-ray powder diffraction pattern comprising one or more peaks selected from the group consisting of 4.8±0.2, 12.1±0.2, 14.0±0.2, 14.4±0.2, 17.0±0.2, 17.5±0.2, 20.9±0.2, 21.6±0.2, 25.0±0.2, 29.5±0.2, and 34.2±0.2 degrees 2θ;
   crystalline (E)-N-(4-(3-tert-butyl-5-(2,4-dioxo-3,4-dihydropyrimidin-1(2H)-yl)-2-methoxystyryl) phenyl)methanesulfonamide disodium salt tetrahydrate having an X-ray powder diffraction pattern substantially as shown in Figure **25**;
   crystalline (E)-N-(4-(3-tert-butyl-5-(2,4-dioxo-3,4-dihydropyrimidin-1(2H)-yl)-2-methoxystyryl) phenyl)methanesulfonamide dipotassium salt tetrahydrate having an X-ray powder diffraction pattern comprising one or more peaks selected from the group consisting of 5.0±0.2, 11.9±0.2, 12.4±0.2, 13.7±0.2, 15.0±0.2, 16.5±0.2, 17.1±0.2, 20.8±0.2, 21.3±0.2, 22.2±0.2, 24.0±0.2, 26.4±0.2, and 29.3±0.2 degrees 2θ;
   crystalline (E)-N-(4-(3-tert-butyl-5-(2,4-dioxo-3,4-dihydropyrimidin-1(2H)-yl)-2-methoxystyryl) phenyl)methanesulfonamide dipotassium salt tetrahydrate having an X-ray powder diffraction pattern comprising one or more peaks selected from the group consisting of 5.0±0.2, 11.9±0.2, 12.4±0.2, 12.6±0.2, 13.7±0.2, 15.0±0.2, 16.5±0.2, 16.7±0.2, 17.1+0.2, 20.7±0.2, 20.8±0.2, 21.3±0.2, 22.2±0.2, 22.4±0.2, 24.0±0.2, 26.4±0.2, and 29.3±0.2 degrees 2θ;
   crystalline (E)-N-(4-(3-tert-butyl-5-(2,4-dioxo-3,4-dihydropyrimidin-1(2H)-yl)-2-methoxystyryl) phenyl)methanesulfonamide dipotassium salt tetrahydrate having an X-ray powder diffraction pattern substantially as shown in Figure **27**;
   crystalline (E)-N-(4-(3-tert-butyl-5-(2,4-dioxo-3,4-dihydropyrimidin-1(2H)-yl)-2-methoxystyryl) phenyl)methanesulfonamide dipotassium salt tetrahydrate having unit cell parameters, wherein a is 14.5Å, b is 10.8Å, and c is 35.8Å;
   crystalline (E)-N-(4-(3-tert-butyl-5-(2,4-dioxo-3,4-dihydropyrimidin-1(2H)-yl)-2-methoxystyryl) phenyl)methanesulfonamide monopotassium salt trihydrate having an X-ray powder diffraction pattern comprising one or more peaks selected from the group consisting of 4.8±0.2, 10.8±0.2, 11.3±0.2, 13.4±0.2, 15.3±0.2, 16.9±0.2, 21.2±0.2, 21.7±0.2, 22.1±0.2, 22.5±0.2, and 23.0±0.2 degrees 2θ;
   crystalline (E)-N-(4-(3-tert-butyl-5-(2,4-dioxo-3,4-dihydropyrimidin-1(2H)-yl)-2-methoxystyryl) phenyl)methanesulfonamide monopotassium salt trihydrate having an X-ray powder diffraction pattern comprising one or more peaks selected from the group consisting of 4.8±0.2, 10.8±0.2, 11.3±0.2, 13.4±0.2, 13.6±0.2, 15.3±0.2, 16.9±0.2, 21.2±0.2, 21.7±0.2, 21.7±0.2, 22.1±0.2, 22.5±0.2, 22.6±0.2, and 23.0±0.2 degrees 2θ;
   crystalline (E)-N-(4-(3-tert-butyl-5-(2,4-dioxo-3,4-dihydropyrimidin-1(2H-yl)-2-methoxystyryl) phenyl)methanesulfonamide monopotassium salt trihydrate having an X-ray powder diffraction pattern comprising five or more peaks selected from the group consisting of 4.8±0.2, 10.8±0.2, 11.3±0.2, 13.4±0.2, 15.3±0.2, 16.9±0.2, 21.2±0.2, 21.7±0.2, 22.1±0.2, 22.5±0.2, and 23.0±0.2 degrees 2θ;
   crystalline (E)-N-(4-(3-tert-butyl-5-(2,4-dioxo-3,4-dihydropyrimidin-1(2H)-yl)-2-methoxystyryl) phenyl)methanesulfonamide monopotassium salt trihydrate having unit cell parameters, wherein a is 9.0Å, b is 8.3Å, and c is 18.6Å;
   crystalline (E)-N-(4-(3-tert-butyl-5-(2,4-dioxo-3,4-dihydropyrimidin-1(2H)-yl)-2-methoxystyryl) phenyl)methanesulfonamide monopotassium salt dihydrate having an X-ray powder diffraction pattern comprising one or more peaks selected from the group consisting of 7.7±0.2, 8.8±0.2, 16.1±0.2, and 19.7±0.2 degrees 2θ;
   crystalline (E)-N-(4-(3-tert-butyl-5-(2,4-dioxo-3,4-dihydropyrimidin-1(2H)-yl)-2-methoxystyryl) phenyl)methanesulfonamide monopotassium salt dihydrate having an X-ray powder diffraction pattern comprising one or more peaks selected from the group consisting of 7.7±0.2, 8.8±0.2, 12.4±0.2, 14.0±0.2, 16.1±0.2, 17.7±0.2, 19.2±0.2, 19.7±0.2, 23.1±0.2, and 29.2±0.2 degrees 2θ;
   crystalline (E)-N-(4-(3-tert-butyl-5-(2,4-dioxo-3,4-dihydropyrimidin-1(2H)-yl)-2-methoxystyryl) phenyl)methanesulfonamide monopotassium salt dihydrate having an X-ray powder diffraction pattern substantially as shown in Figure **29**;
   crystalline (E)-N-(4-(3-tert-butyl-5-(2,4-dioxo-3,4-dihydropyrimidin-1(2H)-yl)-2-methoxystyryl) phenyl)methanesulfonamide 1/7 potassium salt having an X-ray powder diffraction pattern comprising one or more peaks selected from the group consisting of 7.7±0.2, 8.3±0.2, 10.1±0.2, 10.6±0.2, 11.4±0.2, 12.0±0.2, 13.4±0.2, 15.6±0.2, 16.3±0.2, 16.7±0.2, 17.2±0.2, 18.3±0.2, 18.8±0.2, 19.4±0.2, 19.9±0.2, 20.2±0.2, 20.5±0.2, 21.2±0.2, 22.1±0.2, and 22.9±0.2 degrees 2θ;
   crystalline (E)-N-(4-(3-tert-butyl-5-(2,4-dioxo-3,4-dihydropyrimidin-1(2H)-yl)-2-methoxystyryl) phenyl)methanesulfonamide 1/7 potassium salt having an X-ray powder diffraction pattern comprising one or more peaks selected from the group consisting of 7.7±0.2, 8.3±0.2, 10.1±0.2, 10.6±0.2, 1.4±0.2, 12.0±0.2, 13.4±0.2, 15.6±0.2, 16.3±0.2, 16.7±0.2, 17.2±0.2, 18.3±0.2, 18.8±0.2, 19.4±0.2, 19.9±0.2, 20.2±0.2, 20.5±0.2, 20.8±0.2, 21.2±0.2, 22.1±0.2, 22.9±0.2, 24.3±0.2, 24.9±0.2, and 25.1±0.2 degrees 2θ;
   crystalline (E)-N-(4-(3-tert-butyl-5-(2,4-dioxo-3,4-dihydropyrimidin-1(2H)-yl)-2-methoxystyryl) phenyl)methanesulfonamide 1/7 potassium salt having an X-ray powder diffraction pattern substantially as shown in Figure **31**;
   crystalline (E)-N-(4-(3-tert-butyl-5-(2,4-dioxo-3,4-dihydropyrimidin-1(2H)-yl)-2-methoxystyryl) phenyl)methanesulfonamide monodiethylamine salt tetrahydrate having an X-ray powder diffraction pattern comprising one or more peaks selected from the group consisting of 9.5±0.2, 10.0±0.2, 11.8±0.2, 12.1±0.2, 14.4±0.2, 16.8±0.2, 17.6±0.2, 19.8±0.2, 20.8±0.2, 21.4±0.2, 21.8±0.2, and 29.8±0.2 degrees 2θ;
   crystalline (E)-N-(4-(3-tert-butyl-5-(2,4-dioxo-3,4-dihydropyrimidin-(2H)-yl)-2-methoxystyryl) phenyl)methanesulfonamide monodiethylamine salt tetrahydrate having an X-ray powder diffraction pattern comprising one or more peaks selected from the group consisting of 9.5±0.2, 10.0±0.2, 11.8±0.2, 12.1±0.2, 14.4±0.2, 16.8±0.2, 17.6±0.2, 19.4±0.2, 19.8±0.2, 20.8±0.2, 21.4±0.2, 21.8±0.2, 21.9±0.2, and 29.8±0.2 degrees 2θ;
   crystalline (E)-N-(4-(3-tert-butyl-5-(2,4-dioxo-3,4-dihydropyrimidin-1(2H)-yl)-2-methoxystyryl) phenyl)methanesulfonamide monodiethylamine salt tetrahydrate having an X-ray powder diffraction pattern substantially as shown in Figure **32**;
   crystalline pattern A (E)-N-(4-(3-tert-butyl-5-(2,4-dioxo-3,4-dihydropyrimidin-1(2H)-yl)-2-methoxystyryl)phenyl)methanesulfonamide having an X-ray powder diffraction pattern comprising one or more peaks selected from the group consisting of 5.8±0.2, 9.9±0.2, 11.8±0.2, 12.4±0.2, 14.5±0.2, 18.8±0.2, 22.7±0.2, and 29.2±0.2 degrees 2θ;
   crystalline pattern A (E)-N-(4-(3-tert-butyl-5-(2,4-dioxo-3,4-dihydropyrimidin-1(2H)-yl)-2-methoxystyryl)phenyl)methanesulfonamide having an X-ray powder diffraction pattern comprising one or more peaks selected from the group consisting of 5.8±0.2, 9.9±0.2, 11.8±0.2, 12.4±0.2, 14.0±0.2, 14.5±0.2, 15.3±0.2, 18.5±0.2, 18.8±0.2, 22.2±0.2, 22.7±0.2, 23.8±0.2, 26.0±0.2, and 29.2±0.2 degrees 2θ;
   crystalline pattern A (E)-N-(4-(3-tert-butyl-5-(2,4-dioxo-3,4-dihydropyrimidin-1(2H)-yl)-2-methoxystyryl)phenyl)methanesulfonamide having an X-ray powder diffraction pattern substantially as shown in Figure **34**;
   crystalline pattern B (E)-N-(4-(3-tert-butyl-5-(2,4-dioxo-3,4-dihydropyrimidin-1(2H)-yl)-2-methoxystyryl)phenyl)methanesulfonamide having an X-ray powder diffraction pattern comprising one or more peaks selected from the group consisting of 11.5±0.2, 13.3±0.2, 15.4±0.2, 16.4±0.2, 17.1±0.2, 18.6±0.2, 19.4±0.2, 20.4±0.2, 21.6±0.2, 22.4±0.2, 24.0±0.2, 26.8±0.2, and 29.0±0.2 degrees 2θ;
   crystalline pattern B (E)-N-(4-(3-tert-butyl-5-(2,4-dioxo-3,4-dihydropyrimidin-1(2H)-yl)-2-methoxystyryl)phenyl)methanesulfonamide having an X-ray powder diffraction pattern substantially as shown in Figure **36**;
   crystalline pattern C (E)-N-(4-(3-tert-butyl-5-(2,4-dioxo-3,4-dihydropyrimidin-1(2H)-yl)-2-methoxystyryl)phenyl)methanesulfonamide having an X-ray powder diffraction pattern comprising one or more peaks selected from the group consisting of 7.7±0.2, 10.1±0.2, 10.6±0.2, 12.0 ±0.2, 13.4±0.2, 16.2±0.2, 19.4±0.2, 20.5±0.2, 21.4±0.2, 22.0±0.2, 22.6±0.2, 24.3±0.2, and 27.6±0.2 degrees 2θ;
   crystalline pattern C (E)-N-(4-(3-tert-butyl-5-(2,4-dioxo-3,4-dihydropyrimidin-1(2H)-yl)-2-methoxystyryl)phenyl)methanesulfonamide having an X-ray powder diffraction pattern substantially as shown in Figure **37**;
   crystalline pattern D (E)-N-(4-(3-tert-butyl-5-(2,4-dioxo-3,4-dihydropyrimidin-1(2H)-yl)-2-methoxystyryl)phenyl)methanesulfonamide having an X-ray powder diffraction pattern comprising one or more peaks selected from the group consisting of 5.8±0.2, 10.7±0.2, 11.2±0.2, 15.2±0.2, 16.1±0.2, 16.9±0.2, 19.9±0.2, 22.1±0.2, 24.7±0.2, and 26.0±0.2 degrees 2θ;
   crystalline pattern D (E)-N-(4-(3-tert-butyl-5-(2,4-dioxo-3,4-dihydropyrimidin-1(2H)-yl)-2-methoxystyryl)phenyl)methanesulfonamide having an X-ray powder diffraction pattern comprising one or more peaks selected from the group consisting of 5.8±0.2, 10.7±0.2, 11.2±0.2, 15.2±0.2, 16.1±0.2, 16.9±0.2, 17.1±0.2, 19.9±0.2, 20.1+0.2, 22.1±0.2, 24.7±0 .2, and 26.0±0.2 degrees 2θ;
   crystalline pattern D (E)-N-(4-(3-tert-butyl-5-(2,4-dioxo-3,4-dihydropyrimidin-1(2H)-yl)-2-methoxystyryl)phenyl)methanesulfonamide having an X-ray powder diffraction pattern substantially as shown in Figure **38**;
   crystalline pattern A (E)-N-(4-(3-tert-butyl-5-(2,4-dioxo-3,4-dihydropyrimidin-1(2H)-yl)-2-methoxystyryl)phenyl)methanesulfonamide hydrate having an X-ray powder diffraction pattern comprising one or more peaks selected from the group consisting of 5.1±0.2, 7.9±0.2, 9.5±0.2, 10.3±0.2, 13.7±0.2, 16.5±0.2, 17.1±0.2, 17.5±0.2, 18.8±0.2, 19.2±0.2, 20.7±0.2, 21.3±0.2, 21.6±0.2, 25.8±0.2, 26.8±0.2, and 28.4±0.2 degrees 2θ;
   crystalline pattern A (B)-N-(4-(3-tert-butyl-5-(2,4-dioxo-3,4-dihydropyrimidin-1(2H)-yl)-2-methoxystyryl)phenyl)methanesulfonamide hydrate having an X-ray powder diffraction pattern substantially as shown in Figure **39**;
   crystalline pattern A (E)-N-(4-(3-tert-butyl-5-(2,4-dioxo-3,4-dihydropyrimidin-1(2H)-yl)-2-methoxystyryl)phenyl)methanesulfonamide hydrate having an X-ray powder diffraction pattern comprising one or more peaks selected from the group consisting of 6.3±0.2, 7.7±0.2, 10.4:;1:0.2, 12.7±0.2, 13.3±0.2, 14.9±0.2, 15.4±0.2, 16.4±0.2, 18.6±0.2, 18.9±0.2, 19.4±0.2, 22.5±0.2, 23.5±0.2, 24.0±0.2, 26.8±0.2, and 29.0±0.2 degrees 2θ;
   crystalline pattern B (E)-N-(4-(3-tert-butyl-5-(2,4-dioxo-3,4-dihydropyrimidin-1(2H)-yl)-2-methoxystyryl)phenyl)methanesulfonamide hydrate having an X-ray powder diffraction pattern comprising one or more peaks selected from the group consisting of 6.3±0.2, 7.7±0.2, 10.4±0.2, 12.7±0.2, 13.3±0.2, 13.5±0.2, 14.9±0.2, 15.4±0.2, 16.4±0.2, 18.5±0.2, 18.6±0.2, 18.9±0.2, 19.4±0.2, 22.5±0.2, 23.5±0.2, 24.0±0.2, 26.8±0.2, and 29.0±0.2 degrees 2θ;
   crystalline pattern B (E)-N-(4-(3-tert-butyl-5-(2,4-dioxo-3,4-dihydropyrimidin-1(2H)-yl)-2-methoxystyryl)phenyl)methanesulfonamide hydrate having an X-ray powder diffraction pattern substantially as shown in Figure **41**;
   crystalline pattern C (E)-N-(4-(3-tert-butyl-5-(2,4-dioxo-3,4-dihydropyrimidin-1(2H)-yl)-2-methoxystyryl)phenyl)methanesulfonamide hydrate having an X-ray powder diffraction pattern comprising one or more peaks selected from the group consisting of 10.5±0.2, 13.3±0.2, 14.9±0.2, 15.4±0.2, 16.4±0.2, 18.6±0.2, 19.0±0.2, 19.4±0.2, 22.5±0.2, 23.5±0.2, 26.9±0.2, and 29.0±0.2 degrees 2θ;
   crystalline pattern C (E)-N-(4-(3-tert-butyl-5-(2,4-dioxo-3,4-dihydropyrimidin-1(2H)-yl)-2-methoxystyryl)phenyl)methanesulfonamide hydrate having an X-ray powder diffraction pattern comprising one or more peaks selected from the group consisting of 10.5±0.2, 13.3±0.2, 13.5±0.2, 14.9±0.2, 15.4±0.2, 16.4±0.2, 18.6±0.2, 19.0±0.2, 19.4±0.2, 22.5±0.2, 23.5±0.2, 26.9±0.2, and 29.0±0.2 degrees 2θ;
   crystalline pattern C (E)-N-(4-(3-tert-butyl-5-(2,4-dioxo-3,4-dihydropyrimidin-1(2H)-yl)-2-methoxystyryl)phenyl)methanesulfonamide hydrate having an X-ray powder diffraction pattern substantially as shown in Figure **43**;
   crystalline pattern D (E)-N-(4-(3-tert-butyl-5-(2,4-dioxo-3,4-dihydropyrimidin-1(2H)-yl)-2-methoxystyryl)phenyl)methanesulfonamide hydrate having an X-ray powder diffraction pattern comprising one or more peaks selected from the group consisting of 6.6±0.2, 10.0±0.2, 10.5±0.2, 11.1±0.2, 11.6±0.2, 12.2±0.2, 14.2±0.2, 16.6±0.2, 17.1±0.2, 17.7±0.2, 18.5±0.2, 18.8±0.2, 19.3±0.2, 21.4±0.2, 22.7±0.2, 23.1±0.2, 23.6±0.2, 24.6±0.2, 25.2±0.2, 27.2±0.2, 29.1±0.2, and 31.0±0.2 degrees 2θ;
   crystalline pattern D (E)-N-(4-(3-tert-butyl-5-(2,4-dioxo-3,4-dihydropyrimidin-1(2H)-yl)-2-methoxystyryl)phenyl)methanesulfonamide hydrate having an X-ray powder diffraction pattern comprising one or more peaks selected from the group consisting of 6.6±0.2, 10.0±0.2, 10.5±0.2, 11.1±0.2, 11.6±0.2, 12.2±0.2, 12.5±0.2, 14.2±0.2, 16.6±0.2, 17.1±0.2, 17.7±0.2, 18.5±0.2, 18.8±0.2, 19.3±0.2, 21.4±0.2, 22.7±0.2, 22.8±0.2, 23.1±0.2, 23.6±0.2, 24.6±0.2, 24.9±0.2, 25.2±0.2, 27.2±0.2, 29.1±0.2, and 31.0±0.2 degrees 2θ;
   crystalline pattern D (E)-N-(4-(3-tert-butyl-5-(2,4-dioxo-3,4-dihydropyrimidin-1(2H)-yl)-2-methoxystyryl)phenyl)methanesulfonamide hydrate having an X-ray powder diffraction pattern substantially as shown in Figure **45**;
   crystalline pattern D (E)-N-(4-(3-tert-butyl-5-(2,4-dioxo-3,4-dihydropyrimidin-1(2H)-yl)-2-methoxystyryl)phenyl)methanesulfonamide hydrate having unit cell parameters, wherein a is 17.8Å, b is 9.6Å, and c is 27.0Å;
   crystalline pattern E (E)-N-(4-(3-tert-butyl-5-(2,4-dioxo-3,4-dihydropyrimidin-1(2H)-yl)-2-methoxystyryl)phenyl)methanesulfonamide hydrate having an X-ray powder diffraction pattern comprising one or more peaks selected from the group consisting of 6.2±0.2, 7.8±0.2, 10.2±0.2, 10.7±0.2, 12.1±0.2, 16.3±0.2, 19.7±0.2, 20.9±0.2, 21.8±10.2, 24.5±0.2, and 28.0±0.2 degrees 2θ;
   crystalline pattern E (E)-N-(4-(3-tert-butyl-5-(2,4-dioxo-3,4-dihydropyrimidin-1(2H)-yl)-2-methoxystyryl)phenyl)methanesulfonamide hydrate having an X-ray powder diffraction pattern comprising one or more peaks selected from the group consisting of 6.2±0.2, 7.8±0.2, 10.2±0.2, 10.4±0.2, 10.7±0.2, 12.1±0.2, 16.3±0.2, 19.7±0.2, 20.9±0.2, 21.8±0.2, 24.5±0.2, and 28.0±0.2 degrees 2θ;
   crystalline pattern E (E)-N-(4-(3-tert-butyl-5-(2,4-dioxo-3,4-dihydropyrimidin-1(2H)-yl)-2-methoxystyryl)phenyl)methanesulfonamide hydrate having an X-ray powder diffraction pattern substantially as shown in Figure **46**; and
   crystalline pattern E (E)-N-(4-(3-tert-butyl-5-(2,4-dioxo-3,4-dihydropyrimidin-1(2H)-yl)-2-methoxystyryl)phenyl)methanesulfonamide hydrate having unit cell parameters, wherein a is 9.5Å, b is 14.5Å, and c is 17.3Å.
15. A composition comprising (**a**) one or more compounds and/or salts recited in any one of **1-12** or one or more crystalline forms recited in any one of **13** and **14**; (**b**) one or more excipients; and, optionally, (**c**) one or more additional therapeutic agents.
16. A method for inhibiting replication of a ribonucleic acid (RNA) virus, wherein the method comprises exposing the virus to one or more compounds and/or salts recited in any one of **1-12** or one or more crystalline forms recited in any one of **13** and **14**.
17. A method for treating hepatitis C in a mammal in need of such treatment, wherein the method comprises administering to the mammal one or more compounds and/or salts recited in any one of 1-12 or one or more crystalline forms recited in any one of **13** and **14**, and, optionally, one or more additional therapeutic agents.
18. A compound or salt thereof, wherein:
   the compound of formula **II** corresponds in structure to formula **II**: 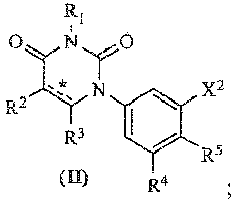
   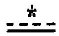 , **R¹**, **R²**, **R³**, **R⁴** and **R⁵** are as defined in **1**; and
   **X²** is halo.
19. The compound or salt of **18**, wherein:
   **R¹**, **R²**, **R³**, **R⁴**, and **R⁵** are as defined in **3**; and
   **X²** is selected from the group consisting of chloro, bromo, and iodo.
20. A process for preparing the compound or salt of **18**, wherein the process comprises reacting a compound of formula **III** (wherein **R¹**, **R²**, and **R³** are as defined in **18**) with a compound, of formula **IV** (wherein **R⁴**, **R⁵**, and **X²** are as defined in **18**; and **X¹** is halo) in the presence of (**i**) copper (I) salt catalyst and (**ii**) nitrogenous heteroaryl ligand 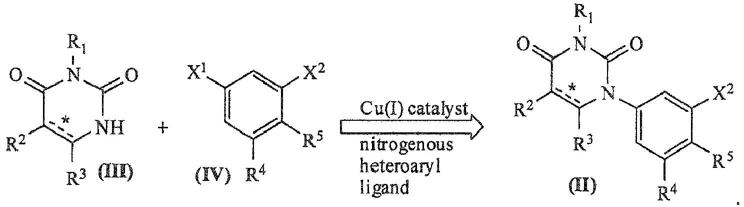
21. The process of 20, wherein **X¹** is selected from the group consisting of chloro, bromo, and iodo.
22. The process of any one of **20** and **21**, wherein the process is conducted in the presence of a base.
23. The process of **22**, wherein the base is selected from the group consisting of potassium salt, sodium salt, and cesium salt.
24. The process of any one of **20-23**, wherein the nitrogenous heteroaryl ligand comprises a picolinamide compound corresponding in structure to formula **V**: 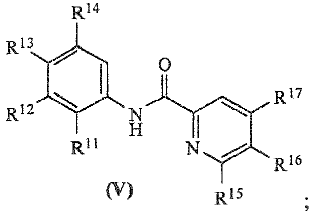 and
   **R¹¹**, **R¹²**, **R¹³**, **R¹⁴**, **R¹⁵**, **R¹⁶**, and **R¹⁷** are independently selected from the group consisting of hydrogen, C₁₋₄-perfluoroalkyl, C₁₋₄-alkyloxy, C₁₋₄-haloalkyl, chloro, and cyano.
25. The process of any one of **20-24**, wherein the nitrogenous heteroaryl ligand is selected from the group consisting of 8-hydroxyquinoline, 2-(2-pyridyl)-benzimidazole, N-(4-cyano-phenyl)picolinamide, and N-(2-cyanophenyl)picolinamide.

## Claims

1. A compound or salt thereof, wherein:
the compound corresponds in structure to formula **I**: 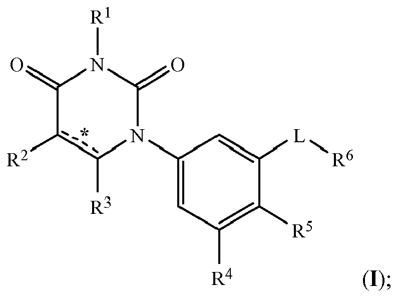
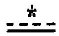 is selected from the group consisting of single carbon-carbon bond and double carbon-carbon bond;
**R¹** is selected from the group consisting of hydrogen, methyl, and nitrogen-protecting group;
**R²** is selected from the group consisting of hydrogen, halo, hydroxy, methyl, cyclopropyl, and cyclobutyl;
**R³** is selected from the group consisting of hydrogen, halo, oxo, and methyl;
**R⁴** is selected from the group consisting of halo, alkyl, alkenyl, alkynyl, nitro, cyano, azido, alkyloxy, alkenyloxy, alkynyloxy, amino, aminocarbonyl, aminosulfonyl, alkylsulfonyl, carbocyclyl, and heterocyclyl, wherein:
(**a**) the amino, aminocarbonyl, and aminosulfonyl optionally are substituted with:
(**1**) one or two substituents independently selected from the group consisting of alkyl, alkenyl, alkynyl, and alkylsulfonyl, or
(**2**) two substituents that, together with the amino nitrogen, form a single-ring heterocyclyl, and
(**b**) the alkyl, alkenyl, alkynyl, alkyloxy, alkenyloxy, alkynyloxy, and alkylsulfonyl, optionally are substituted with one or more substituents independently selected from the group consisting of halo, oxo, nitro, cyano, azido, hydroxy, amino, alkyloxy, trimethylsilyl, carbocyclyl, and heterocyclyl, wherein:
the amino optionally is substituted with:
(**1**) one or two substituents independently selected from the group consisting of alkyl, alkenyl, alkynyl, alkylcarbonyl, alkylsulfonyl, alkyloxycarbonyl, carbocyclyl, heterocyclyl, carbocyclylalkyl, and heterocyclylalkyl, or
(**2**) two substituents that, together with the amino nitrogen, form a single-ring heterocyclyl, and
(**c**) the carbocyclyl and heterocyclyl optionally are substituted with up to three substituents independently selected from the group consisting of alkyl, alkenyl, alkynyl, halo, oxo, nitro, cyano, azido, hydroxy, amino, alkyloxy, trimethylsilyl, carbocyclyl, and heterocyclyl, wherein:
the amino optionally is substituted with:
(**1**) one or two substituents independently selected from the group consisting of alkyl, alkenyl, alkynyl, alkylcarbonyl, alkylsulfonyl, alkyloxycarbonyl, carbocyclyl, heterocyclyl, carbocyclylalkyl, and heterocyclylalkyl, or
(**2**) two substituents that, together with the amino nitrogen, form a single-ring heterocyclyl;
**R⁵** is selected from the group consisting of hydrogen, hydroxy, alkyl, alkenyl, alkynyl, alkyloxy, alkenyloxy, alkynyloxy, alkylsulfonyloxy, carbocyclylsulfonyloxy, haloalkylsulfonyloxy, and halo;
as to **L** and **R⁶**:
**L** is a bond, and **R⁶** is selected from the group consisting of C₅-C₆-carbocyclyl and 5-6-membered heterocyclyl, wherein each such substituent optionally is substituted with one or more substituents independently selected from the group consisting of **R^{E}**, **R^{F}**, **R^{G}**, **R^{H}**, **R^{I}**, **R^{J}**, and **R^{K}**, or
**L** is selected from the group consisting of C(O)N(**R^{C}**), N(**R^{D}**)C(O), C₁-C₂-alkylene, C(H)₂O, OC(H)₂, cyclopropyl-1,2-ene, C(H)₂N(**R^{L}**), N(**R^{M}**)C(H)₂, C(O)CH₂, and CH₂C(O), and
**R⁶** is selected from the group consisting of C₅-C₆-carbocyclyl, 5-6-membered heterocyclyl, fused 2-ring carbocyclyl and fused 2-ring heterocyclyl, wherein each such substituent optionally is substituted with one or more substituents independently selected from the group consisting of **R^{E}**, **R^{F}**, **R^{C}**, **R^{H}**, **R^{I}**, **R^{J}**, and **R^{K}**;
**R^{L}** and **R^{M}** are independently selected from the group consisting of hydrogen, C₁-C₆-alkyl, C₁-C₆-alkyloxy, C₃-C₈-cycloalkyl, and halo, wherein:
the C₁-C₆-alkyl optionally is substituted with one or more substituents independently selected from the group consisting of carboxy, halo, hydroxy, nitro, oxo, amino, cyano, alkyloxycarbonyl, alkylcarbonyloxy, alkyloxy, carbocyclyl, and heterocyclyl;
**R^{C}** is selected from the group consisting of hydrogen and alkyl;
**R^{D}** is selected from the group consisting of hydrogen and alkyl;
each **R^{E}** is independently selected from the group consisting of halo, nitro, hydroxy, oxo, carboxy, cyano, amino, imino, azido, and aldehydo, wherein:
the amino optionally is substituted with one or two substituents independently selected from the group consisting of alkyl, alkenyl, and alkynyl;
each **R^{F}** is independently selected from the group consisting of alkyl, alkenyl, and alkynyl,
wherein:
each such substituent optionally is substituted with one or more substituents independently selected from the group consisting of carboxy, hydroxy, halo, amino, imino, nitro, azido, oxo, aminosulfonyl, alkylsulfonyl, alkyloxycarbonyl, alkenyloxycarbonyl, alkynyloxycarbonyl, alkylcarbonyloxy, alkenylcarbonyloxy, alkynylcarbonyloxy, alkyloxy, alkenyloxy, alkynyloxy, carbocyclyl, heterocyclyl, cyano, and aminocarbonyl, wherein:
the amino, imino, aminosulfonyl, aminocarbonyl, carbocyclyl, and heterocyclyl optionally are substituted with one or two substituents independently selected from the group consisting of alkyl, alkenyl, alkynyl, alkylsulfonyl, alkenylsulfonyl, alkynylsulfonyl, alkylsulfonylamino, hydroxy, and alkyloxy,
wherein:
the amino portion of the alkylsulfonylamino optionally is substituted with a substituent selected from the group consisting of alkyl, alkenyl, and alkynyl;
each **R^{G}** is independently selected from the group consisting of carbocyclyl and heterocyclyl,
wherein:
each such substituent optionally is substituted with one or more substituents independently selected from the group consisting of alkyl, alkenyl, alkynyl, carboxy, hydroxy, halo, amino, nitro, azido, oxo, aminosulfonyl, alkyloxycarbonyl, alkenyloxycarbonyl, alkynyloxycarbonyl, alkylcarbonyloxy, alkenylcarbonyloxy, alkynylcarbonyloxy, alkyloxy, alkenyloxy, alkynyloxy, carbocyclyl, heterocyclyl, cyano, and aminocarbonyl, wherein:
the amino, aminosulfonyl, and aminocarbonyl optionally are substituted with one or two substituents independently selected from the group consisting of alkyl, alkenyl, alkynyl, alkylsulfonyl, alkenylsulfonyl, and alkynylsulfonyl;
each **R^{H}** is independently selected from the group consisting of alkyloxy, alkenyloxy, alkynyloxy, alkylsulfonyloxy, alkenylsulfonyloxy, and alkynylsulfonyloxy, wherein:
each such substituent optionally is substituted with one or more substituents independently selected from the group consisting of carboxy, hydroxy, halo, amino, nitro, azido, oxo, aminosulfonyl, alkyloxycarbonyl, alkenyloxycarbonyl, alkynyloxycarbonyl, alkylcarbonyloxy, alkenylcarbonyloxy, alkynylcarbonyloxy, alkyloxy, alkenyloxy, alkynyloxy, carbocyclyl, heterocyclyl, cyano, and aminocarbonyl, wherein:
the amino, aminosulfonyl, and aminocarbonyl optionally are substituted with one or two substituents independently selected from the group consisting of alkyl, alkenyl, alkynyl, alkylsulfonyl, alkenylsulfonyl, and alkynylsulfonyl;
each **R^{I}** is independently selected from the group consisting of alkylcarbonyl, alkenylcarbonyl, alkynylcarbonyl, aminocarbonyl, alkyloxycarbonyl, carbocyclylcarbonyl, and heterocyclylcarbonyl, wherein:
(**a**) the alkylcarbonyl, alkenylcarbonyl, and alkynylcarbonyl optionally are substituted with one or more substituents independently selected from the group consisting of carboxy, hydroxy, halo, amino, nitro, azido, oxo, aminosulfonyl, alkyloxycarbonyl, alkenyloxycarbonyl, alkynyloxycarbonyl, alkylcarbonyloxy, alkenylcarbonyloxy, alkynylcarbonyloxy, alkyloxy, alkenyloxy, alkynyloxy, carbocyclyl, heterocyclyl, cyano, and aminocarbonyl, and
(**b**) the aminocarbonyl optionally is substituted with one or two substituents independently selected from the group consisting of alkyl, alkenyl, alkynyl, alkyloxyalkyl, carbocyclyl, heterocyclyl, alkylsulfonyl, and alkylsulfonylamino, wherein:
the carbocyclyl and heterocyclyl optionally are substituted with one or two substituents independently selected from the group consisting of halo, alkyl, and oxo;
each **R^{J}** is independently selected from the group consisting of carbocyclylsulfonylamino, heterocyclylsulfonylamino, alkylcarbonylamino, alkenylcarbonylamino, alkynylcarbonylamino, alkyloxycarbonylamino, alkenyloxycarbonylamino, alkynyloxycarbonylamino, alkylsulfonylamino, alkenylsulfonylamino, alkynylsulfonylamino, aminocarbonylamino, alkyloxycarbonylaminoimino, alkylsulfonylaminoimino, alkenylsulfonylaminoimino, and alkynylsulfonylaminoimino, wherein:
(**a**) the amino portion of such substituents optionally is substituted with a substituent independently selected from the group consisting of carbocyclylalkyl, heterocyclylalkyl, alkylcarbonyloxy, aminocarbonylalkyl, alkyl, alkenyl, alkynyl, alkylcarbonyl, alkenylcarbonyl, alkynylcarbonyl, alkyloxycarbonyl, alkyloxyalkyloxycarbonyl, alkylcarbonyloxyalkyl, and alkylsulfonyl, wherein:
(**1**) the carbocyclyl portion of the carbocyclylalkyl and the heterocyclyl portion of the heterocyclylalkyl optionally are substituted with one or more substituents independently selected from the group consisting of alkyl, alkenyl, alkynyl, carboxy, hydroxy, alkyloxy, alkenyloxy, alkynyloxy, halo, nitro, cyano, azido, oxo, and amino, and
(**2**) the amino portion of the aminocarbonylalkyl optionally is substituted with one or two substituents independently selected from the group consisting of alkyl, alkenyl, and alkynyl,
(**b**) the alkyl, alkenyl, and alkynyl portion of such substituents optionally is substituted with one or more substituents independently selected from the group consisting of carboxy, halo, oxo, amino, alkyloxycarbonyl, alkylcarbonyloxy, hydroxy, alkyloxy, carbocyclyl, heterocyclyl, and cyano, wherein:
the amino optionally is substituted with one or two substituents independently selected from the group consisting of alkyl, alkenyl, alkynyl, alkyloxy, alkenyloxy, and alkynyloxy, wherein:
the alkyl optionally is substituted with one or more hydroxy;
(**c**) the carbocyclyl and heterocyclyl portions of such substituents optionally are substituted with one or more substituents independently selected from the group consisting of alkyl, alkenyl, alkynyl, carboxy, hydroxy, alkyloxy, alkenyloxy, alkynyloxy, halo, nitro, cyano, azido, and amino, wherein:
the amino optionally is substituted with one or two substituents independently selected from the group consisting of alkyl, alkenyl, and alkynyl; and
each **R^{K}** is independently selected from the group consisting of aminosulfonyl, alkylsulfonyl, alkenylsulfonyl, and alkynylsulfonyl, wherein:
(**a**) the alkylsulfonyl, alkenylsulfonyl, and alkynylsulfonyl optionally are substituted with one or more substituents independently selected from the group consisting of carboxy, hydroxy, halo, amino, nitro, azido, oxo, aminosulfonyl, alkyloxycarbonyl, alkenyloxycarbonyl, alkynyloxycarbonyl, alkylcarbonyloxy, alkenylcarbonyloxy, alkynylcarbonyloxy, alkyloxy, alkenyloxy, alkynyloxy, carbocyclyl, heterocyclyl, cyano, and aminocarbonyl, wherein:
the amino, aminosulfonyl, and aminocarbonyl optionally are substituted with one or two substituents independently selected from the group consisting of alkyl, alkenyl, and alkynyl; and
(b) the aminosulfonyl optionally is substituted with one or two substituents independently selected from the group consisting of alkyl, alkenyl, and alkynyl.

2. The compound or salt of claim 1, wherein:
**R¹** is selected from the group consisting of hydrogen and methyl;
**R²** is selected from the group consisting of hydrogen and halo;
**R³** is selected from the group consisting of hydrogen and halo;
**R⁴** is selected from the group consisting of C₁-C₄-alkyl, C₃-C₆-carbocyclyl, and 5-6-membered heterocyclyl, wherein:
(**a**) the C₁-C₄-alkyl optionally is substituted with up to three substituents independently selected from the group consisting of halo, oxo, hydroxy, alkyloxy, and trimethylsilyl, and
(**b**) the C₃-C₆-carbocyclyl and 5-6-membered heterocyclyl optionally are substituted with one or two substituents independently selected from the group consisting of alkyl, halo, and alkylsulfonylamino;
**R⁵** is selected from the group consisting of hydrogen, hydroxy, alkyloxy, and halo;
as to **L** and **R⁶**:
**L** is selected from the group consisting of C(O)N(**R^{C}**), N(**R^{D}**)C(O), C₁-C₂-alkylene, C(H)₂O, OC(H)₂, cyclopropyl-1,2-ene, C(H)₂N(**R^{L}**), N(**R^{M}**)C(H)₂, C(O)CH₂, and CH₂C(O), and **R⁶** is selected from the group consisting of C₅-C₆-carbocyclyl and 5-6-membered heterocyclyl, wherein each such substituent is substituted with one two, or three substituents independently selected from the group consisting of **R^{E}**, **R^{F}**, **R^{G}**, **R^{H}**, **R^{I}**, **R^{J}**, and **R^{K}**;
**R^{C}** is selected from the group consisting of hydrogen and methyl;
**R^{D}** is selected from the group consisting of hydrogen and methyl;
each **R^{E}** is independently selected from the group consisting of chloro, fluoro, nitro, hydroxy, oxo, carboxy, amino, imino, aldehydo, and alkylamino;
each **R^{F} is** an independently selected alkyl optionally substituted with a substituent selected from the group consisting of carboxy, halo, amino, imino, and aminosulfonyl, wherein:
the amino, imino, and aminosulfonyl optionally are substituted with one or two substituents independently selected from the group consisting of alkyl, alkylsulfonyl, and alkylsulfonylamino;
each **R¹** is independently selected from the group consisting of alkylcarbonyl and aminocarbonyl, wherein:
the aminocarbonyl optionally is substituted with a substituent selected from the group consisting of alkyl, alkyloxyalkyl, alkylsulfonyl, and alkylsulfonylamino;
each **R^{J}** is independently selected from the group consisting of alkylsulfonylamino, alkenylsulfonylamino, alkynylsulfonylamino, and alkylsulfonylaminoimino, wherein:
(**a**) the amino portion of such substituents optionally is substituted with a substituent independently selected from the group consisting of carbocyclylalkyl, heterocyclylalkyl, alkylcarbonyloxy, aminocarbonylalkyl, alkyl, alkylcarbonyl, alkyloxycarbonyl, alkyloxyalkyloxycarbonyl, alkylcarbonyloxyalkyl, and alkylsulfonyl, wherein:
(**1**) the carbocyclyl portion of the carbocyclylalkyl and the heterocyclyl portion of the heterocyclylalkyl optionally are substituted with one or two substituents independently selected from the group consisting of alkyl, carboxy, hydroxy, alkyloxy, halo, nitro, cyano, oxo, and amino, and
(**2**) the amino portion of the aminocarbonylalkyl optionally is substituted with one or two substituents independently selected from the group consisting of alkyl, alkenyl, and alkynyl,
(**b**) the alkyl, alkenyl, and alkynyl portion of such substituents optionally is substituted with one or two substituents independently selected from the group consisting of carboxy, halo, oxo, amino, alkyloxycarbonyl, alkylcarbonyloxy, hydroxy, alkyloxy, carbocyclyl, heterocyclyl, and cyano, wherein:
the amino optionally is substituted with one or two substituents independently selected from the group consisting of alkyl and alkyloxy, wherein:
the alkyl optionally is substituted with one or more hydroxy;
each **R^{K}** is independently selected from the group consisting of aminosulfonyl and alkylsulfonyl, wherein:
(**a**) the alkylsulfonyl optionally is substituted with one or two substituents independently selected from the group consisting of carboxy, hydroxy, halo, amino, nitro, oxo, aminosulfonyl, alkyloxycarbonyl, alkylcarbonyloxy, alkyloxy, carbocyclyl, heterocyclyl, cyano, and aminocarbonyl; and
(**b**) the aminosulfonyl optionally is substituted with one or two substituents independently selected alkyl;
**R^{L}** is hydrogen; and
**R^{M}** is hydrogen.

3. The compound or salt of any one of claims **1** and **2**, wherein:
**R¹** is hydrogen;
**R²** is selected from the group consisting of hydrogen and halo;
**R³** is hydrogen;
**R⁴** is tert-butyl;
**R⁵** is selected from the group consisting of hydroxy and methoxy;
**R^{C}** is hydrogen; and
**R^{D}** is hydrogen.

4. The compound or salt of any one of claims **1-3**, wherein:
**L** is selected from the group consisting of cyclopropyl-1,2-ene, C(O)N(**R^{C}**), N(**R^{D}**)C(O), C(H)₂O, OC(H)₂, C(H)₂N(**R^{L}**), N(**R^{M}**)C(H)₂, C(O)CH₂, and CH₂C(O); and
**R⁶** is selected from the group consisting of C₅-C₆-carbocyclyl and 5-6-membered heterocyclyl, wherein each such substituent is substituted with one, two, or three substituents independently selected from the group consisting of **R^{E}**, **R^{F}**, and **R^{J}.**

5. The compound or salt of claim **1**, wherein:
**R⁶** is selected from the group consisting of C₅-C₆-carbocyclyl and 5-6-membered heterocyclyl, wherein each such substituent is substituted with one, two, or three substituents independently selected from the group consisting of **R^{E}**, **R^{F}**, and **R^{J}.**

6. The compound or salt of any one of claims **1-4**, wherein:
**R⁶** is phenyl substituted with a substituent selected from the group consisting of **R^{F}** and **R^{J}**;
**R^{F}** is alkylsulfonylaminoalkyl; and
**R^{J}** is alkylsulfonylamino.

7. The compound or salt of claim **1**, wherein the compound is selected from the group consisting of:
1-((3-tert-butyl-4-methoxy-5-(5-(4-nitrophenyl)oxazol-2-yl)phenyl)dihydropyrimidine-2,4(1H,3H)-dione
(E)-N'-((3'-tert-butyl-5'-(2,4-dioxotetrahydropyrimidin-1(2H)-yl)-2'-methoxybiphenyl-3-yl)methylene)methanesulfonohydrazide
(E)-N'-(1-(3'-tert-butyl-5'-(2,4-dioxotetrahydropyrimidin-1(2H)-yl)-2'-methoxybiphenyl-4-yl)ethylidene)methanesulfonohydrazide
(E)-N'-((3'-tert-butyl-5'-(2,4-dioxotetrahydropyrimidin-1(2H)-yl)-3,5-difluoro-2'-methoxybiphenyl-4-yl)methylene)methanesulfonohydrazide
(E)-N'-((3'-tert-butyl-5'-(2,4-dioxotetrahydropyrimidin-1(2H)-yl)-3-fluoro-2'-methoxybiphenyl-4-yl)methylene)methanesulfonohydrazide
N-(3'-tert-butyl-5'-(2,4-dioxo-3,4-dihydropyrimidin-1(2H)-yl)-2'-methoxybiphenyl-4-yl)methanesulfonamide
(E)-N'-((3'-tert-butyl-5'-(2,4-dioxo-3,4-dihydropyrimidin-1(2H)-yl)-3-fluoro-2'-methoxybiphenyl-4-yl)methylene)methanesulfonohydrazide
N-(2-(3'-tert-butyl-5'-(2,4-dioxo-3,4-dihydropyrimidin-1(2H)-yl)-2'-methoxybiphenyl-4-yl)ethyl)methanesulfonamide
(E)-N'-(1-(3'-tert-buty1-5'-(2,4-dioxo-3,4-dihydropyrimidin-1(2H)-yl)-2'-methoxybiphenyl-4-yl)ethylidene)methanesulfonohydrazide
(E)-N'-(1-(3'-tert-butyl-5'-(2,4-dioxo-3,4-dihydropyrimidin-1(2H)-yl)-2'-methoxybiphenyl-3-yl)ethylidene)methanesulfonohydrazide
(E)-N'-((3'-tert-butyl-5'-(2,4-dioxotetrahydropyrimidin-1(2H)-yl)-2,3-difluoro-2'-methoxybiphenyl-4-yl)methylene)methanesulfonohydrazide
(E)-N'-((3'-tert-butyl-5'-(2,4-dioxo-3,4-dihydropyrimidin-1(2H)-yl)-2'-methoxybiphenyl-3-yl)methylene)methanesulfonohydrazide
(E)-N'-((3'-tert-butyl-5'-(2,4-dioxo-3,4-dihydropyrimidin-1(2H)-yl)-2',4-dimethoxybiphenyl-3-yl)methylene)methanesulfonohydrazide
(Z)-N'-(1-(3'-tert-butyl-5'-(2,4-dioxotetrahydropyrimidin-1(2H)-yl)-2'-methoxybiphenyl-3-yl)ethylidene)methanesulfonohydrazide
N-(4-(2-(3-tert-butyl-5-(2,4-dioxotetrahydropyrimidin-(2H)-yl)-2-methoxyphenyl)acetyl)phenyl)methanesulfonamide
1-((3-tert-butyl-4-methoxy-5-(5-(3-nitrophenyl)oxazol-2-yl)phenyl)dihydropyrimidine-2,4(1H,3H)-dione
1-((3-tert-butyl-4-methoxy-5-(5-(3-nitrophenyl)oxazol-2-yl)phenyl)dihydropyrimidine-2,4(1H,3H)-dione
N-(3-(2-(3-tert-butyl-5-(2,4-dioxotetrahydropyrimidin-1(2H)-yl)-2-methoxyphenyl) oxazol-5-yl)phenyl)methanesulfonamide
5-((2,4-dioxotetrahydropyrimidin-1(2H)-yl)-3-isopropyl-2-methoxy-N-(4-(methylsulfonamido)phenyl)benzamide
N-(3'-tert-butyl-5'-(2,4-dioxo-3,4-dihydropyrimidin-1(2H)-yl)-2'-methoxybiphenyl-4-ylcarbamoyl)methanesulfonamide
N'-(3'-tert-butyl-5'-(2,4-dioxo-3,4-dihydropyrimidin-1(2H)-yl)-2'-methoxybiphenylcarbonyl)methanesulfonohydrazide
N-(4-(3-tert-butyl-5-(2,4-dioxotetrahydropyrimidin-1(2H)-yl)-2-methoxybenzyloxy)phenyl)methanesulfonamide
methyl 2-(3-tert-butyl-5-(2,4-dioxo-3,4-dihydropyrimidin-1(2H)-yl)-2-methoxyphenethyl)-5-(methylsulfonamido)benzoate
N-(4-(3-tert-butyl-5-(2,4-dioxo-3,4-dihydropyrimidin-1(2H)-yl)-2-methoxyphenethyl)phenyl)methanesulfonamide
N-(3-tert-butyl-5-(2,4-dioxo-3,4-dihydropyrimidin-1(2H)-yl)-2-methoxyphenyl)-2-methoxy-4-(methylsulfonamido)benzamide
3-tert-butyl-5-(2,4-dioxotetrahydropyrimidin-1(2H)-yl)-2-hydroxy-N-phenylbenzamide
1-[3-tert-butyl-5-(2,4-dioxo-tetrahydro-pyrimidin-1-yl)-2-hydroxy-benzyl]-piperidine-4-carboxylic acid methyl ester
4-[3-tert-butyl-5-(2,4-dioxo-tetrahydro-pyrimidin-1-yl)-2-hydroxy-benzyl]-piperazine-1-carboxylic acid tert-butyl ester
{-[3-tert-butyl-5-(2,4-dioxo-tetrahydro-pyrimidin-1-yl)-2-hydroxy-benzyl]-piperidin-4-yl}-carbamic acid tert-butyl ester
3-tert-butyl-5-(2,4-dioxotetrahydropyrimidin-1(2H)-yl)-2-hydroxy-N-(4-(methylsulfonamido)phenyl)benzamide
3-tert-butyl-5-(2,4-dioxotetrahydropyrimidin-1(2H)-yl)-2-methoxy-N-phenylbenzamide
3-tert-butyl-5-(2,4-dioxotetrahydropyrimidin-1(2H)-yl)-2-methoxy-N-(4-methylsulfonamido)phenyl)benzamide
3-tert-butyl-5-(2,4-dioxotetrahydropyrimidin-1(2H)-yl)-2-hydroxy-N-(2-methoxy-4-(methylsulfonamido)phenyl)benzamide
3-tert-butyl-5-(2,4-dioxotetrahydropyrimidin-1(2H)-yl)-2-hydroxy-N-(4-(methylsulfonamidomethyl)-3-sulfamoylthiophen-2-yl)benzamide
1-[3-tert-butyl-5-(2,4-dioxo-tetrahydro-pyrimidin-1-yl)-2-hydroxy-benzyl]-piperidine-3-carboxylic acid ethyl ester
{1-[3-tert-buty1-5-(2,4-dioxo-tetrahydro-pyrimidin-1-yl)-2-hydroxy-benzy1]-pyrrolidin-3-yl}-carbamic acid tert-butyl ester
3-tert-butyl-5-(2,4-dioxotetrahydropyrimidin-1(2H)-yl)-2-hydroxy-N-methyl-N-phenyl-benzamide
3-tert-butyl-5-(2,4-dioxotetrahydropyrimidin-1(2H)-yl)-2-hydroxy-N-(3-(methylsulfonamido)phenyl)benzamide
3-tert-butyl-5-(2,4-dioxotetrahydropyrimidin-1(2H)-yl)-2-hydroxy-N-(thiazol-2-yl)benzamide
N-{1-[3-tert-buty1-5-(2,4-dioxo-tetrahydro-pyrimidin-1-yl)-2-hydroxy-benzyl]-piperidin-3-ylmethyl}--methanesulfonamide
N-{1-[3-tert-butyl-5-(2,4-dioxo-tetrahydro-pyrimidin-1-yl)-2-hydroxy-benzyl]-pyrrolidin-3-yl} - methanesulfonamide
N-(3-tert-butyl-5-(2,4-dioxotetrahydropyrimidin-1(2H)-yl)-2-methoxyphenyl)benzamide
3-tert-butyl-5-(2,4-dioxotetrahydropyrimidin-1 (2H)-yl)-2-hydroxy-N-(quinolin-6-yl)benzamide
N-(1-(3-tert-butyl-5-(2,4-dioxotetrahydropyrimidin-1(2H)-yl)-2-hydroxybenzyl)-1,2,3,4-tetrahydroquinolin-6-yl)methanesulfonamide
N-(3 -tert-butyl-5 -(2,4-dioxotetrahydropyrimidin-1(2H)-yl)-2-hydroxyphenyl)benzamide
3-tert-butyl-5-(2,4-dioxotetrahydropyrimidin-1(2H)-yl)-2-methoxy-N-(4-(methylsulfonamidemethyl)-3-sulfamoylthiophen-2-yl)benzamide
3-tert-butyl-5-(2,4-dioxotetrahydropyrimidin-1(2H)-yl)-2-methoxy-N-(2-methoxy-4-(methylsulfonamido)phenyl)benzamide
N-(3-tert-butyl-5-(2,4-dioxotetrahydropyrimidin-1(2H)-yl)-2-hydroxyphenyl)-4-nitrobenzamide 4-amino-N-(3-tert-butyl-5-(2,4-dioxotetrahydropyrimidin-1(2H)-yl)-2-hydroxyphenyl)benzamide
N-(3-tert-butyl-5-(2,4-dioxotetrahydropyrimidin-1(2H)-yl)-2-hydroxyphenyl)-4-(methylsulfonamido)benzamide
3-tert-butyl-5-(2,4-dioxotetrahydropyrimidin-1(2H)-yl)-N-(4-(methylsulfonamido)phenyl)benzamide
1-((3-tert-butyl-5-((cyclohexyl(methyl)amino)methyl)-4-hydroxyphenyl)dihydropyrimidine-2,4(1H,3H)-dione 2,2,2-trifluoroacetate
1-((3-tert-butyl-5-((cyclohexyl(ethyl)amino)methyl)-4-hydroxyphenyl)dihydropyrimidine-2,4(1H,3H)-dione 2,2,2-trifluoroacetate
1-[3-tert-butyl-4-hydroxy-5-(3-methyl-piperidin-1-ylmethyl)-phenyl]-dihydro-pyrimidine-2,4-dione; compound with trifluoroacetic acid
1-((3-tert-butyl-4-hydroxy-5-(morpholinomethyl)phenyl)dihydropyrimidine-2,4(1H,3H)-dione 2,2,2-trifluoroacetate
1-[3-tert-butyl-4-hydroxy-5-((R)-3-hydroxy-piperidin-1-ylmethyl)-phenyl]-dihydro-pyrimidine-2,4-dione; trifluoroacetic acid
1-[3-tert-butyl-5-(2,4-dioxo-tetrahydro-pyrimidin-1-yl)-2-hydroxy-benzyl]-piperidine-3-carboxylic acid amide, trifluoroacetic acid salt
1-[3-tert-butyl-5-(2,4-dioxo-tetrahydro-pyrimidin-1-yl)-2-hydroxy-benzyl]-piperidine-3-carboxylic acid diethylamide, trifluoroacetic acid salt
1-((3-tert-butyl-5-((2,6-dimethylmorpholino)methyl)-4-hydroxyphenyl)dihydropyrimidine-2,4(1H,3H)-dione 2,2,2-trifluoroacetate
3-tert-butyl-5-(2,4-dioxotetrahydropyrimidin-1 (2H)-yl)-2-methoxy-N-(4-(2-morpholinoethylsulfonamido)phenyl)benzamide
*N-*(3*-tert*-butyl-5-(2,4-dioxotetrahydropyrimidin-1(2*H*)*-*yl)-2-methoxyphenyl)-4-(methylsulfonamido)benzamide
3-tert-butyl-5-(2,4-dioxotetrahydropyrimidin-1(2H)-yl)-2-methoxy-N-(5-(methylsulfonamido)pyridin-2-yl)benzamide
N-(4-(2-(bis(2-hydroxyethyl)amino)ethylsulfonamido)phenyl)-3-tert-butyl-5-(2,4-dioxotetrahydropyrimidin-1(2H)-yl)-2-methoxybenzamide
3-tert-butyl-5-(2,4-dioxotetrahydropyrimidin-1(2H)-yl)-2-hydroxy-N-(4-(2-morpholinoethylsulfonamido)phenyl)benzamide
3-tert-butyl-5-(2,4-dioxotetrahydropyrimidin-1(2H)-yl)-2-methoxy-N-(5-(N-(methylsulfonyl)methylsulfonamido)pyridin-2-yl)benzamide
3-tert-butyl-5-(2,4-dioxotetrahydropyrimidin-1(2H)-yl)-2-methoxy-N-(4-(2-methoxyethylsulfonamido)phenyl)benzamide
3-tert-butyl-5-(2,4-dioxotetrahydropyrimidin-1(2H)-yl)-N-(4-(2-hydroxyethylsulfonamido)phenyl)-2-methoxybenzamide
N-(6-aminopyridin-3-yl)-3-tert-butyl-5-(2,4-dioxotetrahydropyrimidin-1(2H)-yl)-2-methoxybenzamide
3-tert-butyl-2-methoxy-5-(6-methyl-2,4-dioxo-3,4-dihydropyrimidin-1(2H)-yl)-N-(4-(methylsulfonamido)phenyl)benzamide
3-tert-butyl-5-(2,4-dioxotetrahydropyrimidin-1(2H)-yl)-2-methoxy-N-(6-(methylsulfonamido)pyridin-3-yl)benzamide
3-tert-butyl-5-(2,4-dioxotetrahydropyrimidin-1(2H)-yl)-2-methoxy-N-(4-(methylsulfon-amido)-3-nitrophenyl)benzamide
3-tert-butyl-5-(2,4-dioxotetrahydropyrimidin-1(2H)-yl)-2-methoxy-N-(2-methyl-4-(methylsulfonamido)phenyl)benzamide
N-(3 -tert-butyl-5-(2,4-dioxotetrahydropyrimidin-1(2H)-yl)phenyl)-4-(methylsulfonamido)benzamide
N-(4-(3-tert-butyl-5-(2,4-dioxotetrahydropyrimidin-1(2H)-yl)-2-methoxybenzylamino)phenyl)methanesulfonamide
N-(3-tert-butyl-5-(2,4-dioxotetrahydropyrimidin-1(2H)-yl)phenyl)-4-(methylsulfonamido)benzamide
3-tert-butyl-5-(2,4-dioxo-3,4-dihydropyrimidin-1(2H)-yl)-2-methoxy-N-(4-(methylsulfonamido)phenyl)benzamide
3-tert-butyl-2-hydroxy-5-(6-methyl-2,4-dioxo-3,4-dihydropyrimidin-1(2H)-yl)-N-(4-(methylsulfonamido)phenyl)benzamide
N-(4-(2-amino-2-oxoethoxy)phenyl)-3-tert-butyl-5-(2,4-dioxotetrahydropyrimidin-1(2H)-yl)-2-methoxybenzamide
3-tert-butyl-5-(2,4-dioxotetrahydropyrimidin-1(2H)-yl)-2-hydroxy-N-(4-(2-methoxyethylsulfonamido)phenyl)benzamide
3-tert-butyl-N-(4-(cyanomethoxy)phenyl)-5-(2,4-dioxotetrahydropyrimidin-1(2H)-yl)-2-methoxybenzamide
3-tert-butyl-5-(2,4-dioxo-3,4-dihydropyrimidin-1(2H)-yl)-2-hydroxy-N-(4-(methylsulfonamide)phenyl)benzamide
N-(4-(2-amino-2-oxoethoxy)phenyl)-3-tert-butyl-5-(2,4-dioxotetrahydropyrimidin-1(2H)-yl)-2-hydroxybenzamide
3-tert-butyl-N-(4-(cyanomethoxy)phenyl)-5-(2,4-dioxotetrahydropyrimidin-1(2H)-yl)-2-hydroxybenzamide
4-((3-tert-butyl-5-(2,4-dioxotetrahydropyrimidin-1(2H)-yl)-2-hydroxybenzamido)phenylmethanesulfonate
tert-butyl (5-(3-tert-butyl-5-(2,4-dioxotetrahydropyrimidin-(2H)-yl)-2-hydroxybenzamido)thiophen-3-yl)methyl(methylsulfonyl)carbamate
tert-butyl (5-(3-tert-butyl-5-(2,4-dioxotetrahydropyrimidin-(2H)-yl)-2-methoxybenzamido)thiophen-3-yl)methyl(methylsulfonyl)carbamate
3-tert-butyl-5-(2,4-dioxotetrahydropyrimidin-1(2H)-yl)-2-hydroxy-N-(4-(methylsulfonamidomethyl)thiophen-2-yl)benzamide
3-tert-butyl-5-(2,4-dioxotetrahydropyrimidin-1(2H)-yl)-2-methoxy-N-(4-(methylsulfonamidomethyl)thiophen-2-yl)benzamide
4-((3-tert-butyl-5-(2,4-dioxotetrahydropyrimidin-1(2H)-yl)-2-methoxybenzamido)phenylmethanesulfonate
N-(4-(3-tert-butyl-5-(2,4-dioxotetrahydropyrimidin-1(2H)-yl)-2-hydroxybenzylamino)phenyl)methanesulfonamide
3-tert-butyl-5-(2,4-dioxotetrahydropyrimidin-1(2H)-yl)-2-methoxy-N-(3-(methylsulfonamidomethyl)phenyl)benzamide
3-tert-butyl-5-(2,4-dioxotetrahydropyrimidin-1(2H)-yl)-2-hydroxy-N-(3-(methylsulfonamidomethyl)phenyl)benzamide
3-tert-butyl-5-(2,4-dioxotetrahydropyrimidin-1(2H)-yl)-2-methoxy-N-(4-(N-methylmethylsulfonamido)phenyl)benzamide
methyl 4-(3-tert-butyl-5-(2,4-dioxotetrahydropyrimidin-1(2H)-yl)-2-methoxybenzamido)phenylcarbamate
3-tert-butyl-5-(2,4-dioxotetrahydropyrimidin-1(2H)-yl)-2-hydroxy-N-(4-(methylsulfonyl)phenyl)benzamide
N-(4-acetamidophenyl)-3-tert-butyl-5-(2,4-dioxotetrahydropyrimidin-1(2H)-yl)-2-methoxybenzamide
3-tert-butyl-5-(2,4-dioxotetrahydropyrimidin-1(2H)-yl)-2-methoxy-N-(4-(methylsulfonyl)phenyl)benzamide
3-tert-butyl-5-(2,4-dioxotetrahydropyrimidin-1(2H)-yl)-2-methoxy-N-(4-((N-methylmethylsulfonamido)methyl)thiophen-2-yl)benzamide
3-tert-butyl-5-(2,4-dioxotetrahydropyrimidin-1(2H)-yl)-2-hydroxy-N-(4-((N-methylmethylsulfonamido)methyl)thiophen-2-yl)benzamide
2-((4-(3-tert-butyl-5-(2,4-dioxotetrahydropyrimidin-1(2H)-yl)-2-methoxybenzamido)phenylamino)-2-oxoethyl acetate
N-(3 -tert-butyl-5 -(2,4-dioxotetrahydropyrimidin-1(2H)-yl)-2-methoxyphenyl)-4-(methylsulfonylmethyl)benzamide
N-(4-(2-(3-tert-butyl-5-(2,4-dioxotetrahydropyrimidin-1(2H)-yl)-2-methoxyphenyl)cyclopropyl)phenyl)methanesulfonamide;
5-((2,4-dioxotetrahydropyrimidin-1(2H)-yl)-2-hydroxy-N-(4-(methylsulfonamido)phenyl)benzamide
3-tert-butyl-5-(2,4-dioxotetrahydropyrimidin-1(2H)-yl)-2-hydroxy-N-(4-(N-methylmethylsulfonamido)phenyl)benzamide
3-tert-butyl-2-methoxy-5-(3-methyl-2,4-dioxotetrahydropyrimidin-1(2H)-yl)-N-(4-(N-methylmethylsulfonamido)phenyl)benzamide
N-(4-(3-tert-butyl-5-(2,4-dioxotetrahydropyrimidin-1(2H)-yl)-2-methoxyphenethyl)phenyl)methanesulfonamide
3-tert-butyl-5-(2,4-dioxotetrahydropyrimidin-1(2H)-yl)-2-hydroxy-N-(4-(N-methylsulfamoylmethyl)phenyl)benzamide
3-tert-butyl-5-(2,4-dioxotetrahydropyrimidin-1(2H)-yl)-2-methoxy-N-(4-(N-methylsulfamoylmethyl)phenyl)benzamide
N-[4-(acetyl-methanesulfonyl-amino)-phenyl]-3-tert-butyl-5-(2,4-dioxo-tetrahydro-pyrimidin-1-yl)-2-methoxy-benzamide
3-tert-butyl-N-(2,2-dioxo-1,3-dihydrobenzo[c]thiophen-5-yl)-5-(2,4-dioxotetrahydropyrimidin-1(2H)-yl)-2-hydroxybenzamide
3-tert-butyl-N-(2,2-dioxo-1,3-dihydrobenzo[c]thiophen-5-yl)-5-(2,4-dioxotetrahydropyrimidin-1(2H)-yl)-2-methoxybenzamide
2-((4-(3-tert-butyl-5-(2,4-dioxotetrahydropyrimidin-1(2H)-yl)-2-methoxybenzamido)phenyl)-acetic acid
3-tert-butyl-5-(2,4-dioxotetrahydropyrimidin-1(2H)-yl)-2-methoxy-N-(4-(N-(methylsulfonyl)butyramido)phenyl)benzamide
2-((4-(3-tert-butyl-5-(2,4-dioxotetrahydropyrimidin-(2H)-yl)-2-hydroxybenzamido)phenyl)-acetic acid
3-tert-butyl-5-(2,4-dioxotetrahydropyrimidin-1(2H)-yl)-2-hydroxy-N-(2-sulfamoylphenyl)benzamide
3-tert-butyl-5-(2,4-dioxotetrahydropyrimidin-1(2H)-yl)-2-methoxy-N-(2-sulfamoylphenyl)benzamide
ethyl 4-(3-tert-butyl-5-(2,4-dioxotetrahydropyrimidin-1(2H)-yl)-2-methoxybenzamido)-phenyl(methylsulfonyl)carbamate
1-((3-tert-butyl-4-methoxy-5-((1-methyl-1H-indol-3-yl)methyl)phenyl)dihydropyrimidine-2,4(1H,3H)-dione
3-tert-butyl-5-(2,4-dioxotetrahydropyrimidin-1(2H)-yl)-2-methoxy-N-(4-(N-(methylsulfonyl)propionamido)phenyl)benzamide
isobutyl 4-(3-tert-butyl-5-(2,4-dioxotetrahydropyrimidin-(2H)-yl)-2-methoxybenzamido)phenyl(methylsulfonyl)carbamate
N-(3-tert-butyl-5-(2,4-dioxotetrahydropyrimidin-1(2H)-yl)-2-methoxyphenyl)-2-methoxy-4-(methylsulfonamido)benzamide
N-(3-tert-butyl-5-(2,4-dioxotetrahydropyrimidin-(2H)-yl)-2-methoxyphenyl)-2-chloro-4-(methylsulfonamido)benzamide
3-tert-butyl-5-(2,4-dioxotetrahydropyrimidin-1(2H)-yl)-2-methoxy-N-((1r,4r)-4-(methylsulfonamido)cyclohexyl)benzamide
ethyl 3-(4-(3-tert-butyl-5-(2,4-dioxotetrahydropyrimidin-1(2H)-yl)-2-methoxybenzamido)phenyl)-3-oxopropanoate
3-tert-butyl-5-(2,4-dioxotetrahydropyrimidin-1(2H)-yl)-2-hydroxy-N-(2-(methylsulfonyl)phenyl)benzamide
N-(3-tert-butyl-5-(2,4-dioxotetrahydropyrimidin-1(2H)-yl)-2-hydroxyphenyl)-4-(methylsulfonylmethyl)benzamide
3-tert-butyl-5-(2,4-dioxotetrahydropyrimidin-1(2H)-yl)-2-methoxy-N-(2-(methylsulfonyl)phenyl)benzamide
3-tert-butyl-5-(2,4-dioxotetrahydropyrimidin-1(2H)-yl)-2-methoxy-N-(4-(N-(methylsulfonyl)isobutyramido)phenyl)benzamide
methyl 4-(3-tert-butyl-5-(2,4-dioxotetrahydropyrimidin-1(2H)-yl)-2-methoxybenzamido)phenyl(methylsulfonyl)carbamate
3-tert-butyl-5-(2,4-dioxotetrahydropyrimidin-1(2H)-yl)-2-hydroxy-N-(4-(phenylsulfonamido)phenyl)benzamide
3-tert-butyl-5-(2,4-dioxotetrahydropyrimidin-1(2H)-yl)-2-methoxy-N-(4-(phenylsulfonamido)phenyl)benzamide
(N-4-(3-tert-buty1-5-(2,4-dioxotetrahydropyrimidin-1(2H)-yl)-2-methoxybenzamido)phenyl)methylsulfonamido)methylbutyrate
3-tert-butyl-5-(2,4-dioxotetrahydropyrimidin-1(2H)-yl)-2-hydroxy-N-methyl-N-(4-(methylsulfonamido)phenyl)benzamide
3-tert-butyl-5-(2,4-dioxotetrahydropyrimidin-1(2H)-yl)-N-(4-(N-ethylmethylsulfonamido)phenyl)-2-methoxybenzamide
3-tert-butyl-5-(2,4-dioxotetrahydropyrimidin-1(2H)-yl)-2-methoxy-N-(2-oxoindolin-5-yl)benzamide
(N-(4-(3-tert-butyl-5-(2,4-dioxotetrahydropyrimidin-1(2H)-yl)-2-methoxybenzamido)phenyl)methylsulfonamido)methylpivalate
3-*tert*-butyl-5-(2,4-dioxotetrahydropyrimidin-1(2*H*)-yl)-2-methoxy-*N*-methyl-*N*-(4-(methylsulfonamido)phenyl)benzamide
(N-(4-(3-tert-butyl-5-(3-(butyryloxymethyl)-2,4-dioxotetrahydropyrimidin-1(2H)-yl)-2-methoxybenzamido)phenyl)methylsulfonamido)methylbutyrate
N-(4-(N-allylmethylsulfonamido)phenyl)-3-tert-butyl-5-(2,4-dioxotetrahydropyrimidin-1(2H)-yl)-2-methoxybenzamide
3-tert-butyl-N-(4-(N-(cyclopropylmethyl)methylsulfonamido)phenyl)-5-(2,4-dioxotetrahydropyrimidin-1 (2H)-yl)-2-methoxybenzamide
3-tert-butyl-5-(2,4-dioxotetrahydropyrimidin-1(2H)-yl)-2-methoxy-N-(2-oxo-1,2,3,4-tetrahydroquinolin-6-yl)benzamide
tert-butyl 4-(3-tert-butyl-5-(2,4-dioxotetrahydropyrimidin-1(2H)-yl)-2-methoxybenzamido)-phenylcarbamate
N-(3-tert-butyl-5-(2,4-dioxotetrahydropyrimidin-1(2H)-yl)-2-methoxyphenyl)-N-methyl-4-(methylsulfonamido)benzamide
N-(4-aminophenyl)-3-tert-butyl-5-(2,4-dioxotetrahydropyrimidin-1(2H)-yl)-2-methoxybenzamide (3-(3-tert-butyl-4-methoxy-5-(4-(N-(pivaloyloxymethyl)methylsulfonamido)phenylcarbamoyl)phenyl)-2,6-dioxotetrahydropyrimidin-1(2H)-yl)methylpivalate
(N-(4-(3-tert-butyl-5-(2,4-dioxotetrahydropyrimidin-1(2H)-yl)-2-methoxybenzamido)phenyl)methylsulfonamido)methylbutyrate
N-(4-acetylphenyl)-3-tert-butyl-5-(2,4-dioxotetrahydropyrimidin-1(2H)-yl)-2-methoxybenzamide
3-tert-butyl-5-(2,4-dioxotetrahydropyrimidin-1(2H)-yl)-N-(4-hydroxyphenyl)-2-methoxybenzamide
3-tert-butyl-5-(2,4-dioxotetrahydropyrimidin-1(2H)-yl)-2-methoxy-N-(4-(2,2,2-trifluoroethylsulfonamido)phenyl)benzamide
3-tert-butyl-5-(2,4-dioxotetrahydropyrimidin-1(2H)-yl)-2-methoxy-N-(4-(2-methylpropylsulfonamido)phenyl)benzamide
3-tert-butyl-5-(2,4-dioxotetrahydropyrimidin-1(2H)-yl)-2-methoxy-N-(4-(phenylmethylsulfonamido)phenyl)benzamide
5-((3-allyl-2,4-dioxotetrahydropyrimidin-1(2H)-yl)-N-(4-(N-allylmethylsulfonamido)phenyl)-3-tert-butyl-2-methoxybenzamide
5-((3-((1,3-dioxolan-2-yl)methyl)-2,4-dioxotetrahydropyrimidin-1(2H)-yl)-3-tert-butyl-2-methoxy-N-(4-(methylsulfonamido)phenyl)benzamide
3-tert-butyl-5-(2,4-dioxotetrahydropyrimidin-1(2H)-yl)-2-hydroxy-N-(4-(2-methylpropylsulfonamido)phenyl)benzamide
3-tert-butyl-5-(2,4-dioxotetrahydropyrimidin-1(2H)-yl)-2-hydroxy-N-(4-(phenylmethylsulfonamido)phenyl)benzamide
3-tert-butyl-5-(2,4-dioxotetrahydropyrimidin-1(2H)-yl)-2-hydroxy-N-(4-(2,2,2-trifluoroethylsulfonamido)phenyl)benzamide
2-methoxyethyl4-(3-tert-butyl-5-(2,4-dioxotetrahydropyrimidin-1(2H)-yl)-2-methoxybenzamido)phenyl(methylsulfonyl)carbamate
3-tert-butyl-5-(2,4-dioxotetrahydropyrimidin-1(2H)-yl)-2-methoxy-N-(4-(N-(4-methoxybenzyl)methylsulfonamido)phenyl)benzamide
3-tert-butyl-5-(3-(2-(ethylamino)-2-oxoethyl)-2,4-dioxotetrahydropyrimidin-1(2H)-yl)-N-(4-(N-(2-(ethylamino)-2-oxoethyl)methylsulfonamido)phenyl)-2-methoxybenzamide
3-tert-butyl-N-[4-(3,5-dimethyl-isoxazole-4-sulfonylamino)-phenyl]-5-(2,4-dioxo-tetrahydropyrimidin-1-yl)-2-methoxy-benzamide
3-tert-butyl-N-(4-(2,5-dimethoxyphenylsulfonamido)phenyl)-5-(2,4-dioxotetrahydro-pyrimidin-1(2H)-yl)-2-methoxybenzamide;
3-tert-butyl-N-(4-(3,5-dimethylisoxazole-4-sulfonamido)phenyl)-5-(2,4-dioxotetrahydropyrimidin-1(2H)-yl)-2-hydroxybenzamide
3-tert-butyl-5-(2,4-dioxo-tetrahydro-pyrimidin-1-yl)-2-methoxy-N-(2-methoxy-phenyl)-benzamide
3-tert-butyl-5-(2,4-dioxo-tetrahydro-pyrimidin-1-yl)-2-methoxy-N-(3-methoxy-phenyl)-benzamide
3-tert-butyl-5-(2,4-dioxo-tetrahydro-pyrimidin-1-yl)-2-methoxy-N-(4-methoxy-phenyl)-benzamide
3-tert-butyl-5-(2,4-dioxo-tetrahydro-pyrimidin-1-yl)-N-(4-fluoro-phenyl)-2-methoxy-benzamide
3-tert-butyl-N-(4-chloro-phenyl)-5-(2,4-dioxo-tetrahydro-pyrimidin-1-yl)-2-methoxy-benzamide
3-tert-butyl-5-(2,4-dioxo-tetrahydro-pyrimidin-1-yl)-2-methoxy-N-(4-trifluoromethoxy-phenyl)-benzamide
3-tert-butyl-5-(2,4-dioxo-tetrahydro-pyrimidin-1-yl)-2-methoxy-N-(4-trifluoromethyl-phenyl)-benzamide
3-tert-butyl-5-(2,4-dioxotetrahydropyrimidin-1(2H)-yl)-N-(2-ethylphenyl)-2-methoxybenzamide
3-tert-butyl-5-(2,4-dioxo-tetrahydro-pyrimidin-1-yl)-N-(4-fluoro-2-methyl-phenyl)-2-methoxybenzamide
3-tert-butyl-5-(2,4-dioxo-tetrahydro-pyrimidin-1-yl)-N-(3-hydroxy-2-methyl-phenyl)-2-methoxybenzamide
3-tert-butyl-5-(2,4-dioxo-tetrahydro-pyrimidin-1-yl)-N-(2-ethoxy-phenyl)-2-methoxy-benzamide
N-(5-acetylamino-2-methoxy-phenyl)-3-tert-butyl-5-(2,4-dioxo-tetrahydro-pyrimidin-1-yl)-2-methoxy-benzamide
3-tert-butyl-N-(3-carbamoyl-phenyl)-5-(2,4-dioxo-tetrahydro-pyrimidin-1-yl)-2-methoxybenzamide
N-(4-(3-tert-butyl-5-(2,4-dioxotetrahydropyrimidin-(2H)-yl)phenethyl)phenyl)methanesulfonamide
(E)-N'-((3'-tert-butyl-5'-(2,4-dioxo-3,4-dihydropyrimidin-1(2H)-yl)-2'-methoxybiphenyl-4-yl)methylene)methanesulfonohydrazide
N-(4-(2-(3-tert-butyl-5-(2,4-dioxotetrahydropyrimidin-1(2H)-yl)-2-methoxyphenyl)-2-oxoethyl)phenyl)methanesulfonamide
5-((2,4-dioxotetrahydropyrimidin-1(2H)-yl)-3-ethyl-2-methoxy-N-(4-(methylsulfonamido)phenyl)benzamide
5-((2,4-dioxotetrahydropyrimidin-1(2H)-yl)-3-ethyl-2-hydroxy-N-(4-(methylsulfonamido)phenyl)benzamide
3-tert-butyl-N-[4-(methanesulfonyl-methyl-amino)-phenyl]-2-methoxy-5-(3-methyl-2,4-dioxotetrahydro-pyrimidin-1-yl)-benzamide.

8. A composition comprising (**a**) one or more compounds and/or salts recited in any one of claims **1-7**; (**b**) one or more excipients; and, optionally, (**c**) one or more additional therapeutic agents.

9. A compound or salt recited in any one of claims **1-7** for use in therapy.

10. A product comprising one or more compounds and/or salts recited in any one of claims **1-7** and one or more additional therapeutic agents, preferably selected from the group consisting of interferon agents, ribavirin, HCV inhibitors, and anti-HIV agents, as a combined preparation for simultaneous, sequential or both simultaneous and sequential use in combination therapy.

11. A compound or salt recited in any one of claims **1-7** for use in inhibiting replication of a ribonucleic acid (RNA) virus, preferably hepatitis C virus (HCV).

12. A compound or salt for use as recited in claim **11** wherein the use further comprises the use of one or more additional therapeutic agents, preferably selected from the group consisting of interferon agents, ribavirin, HCV inhibitors, and anti-HIV agents.

13. A compound or salt thereof, wherein:
the compound corresponds in structure to formula **II:** 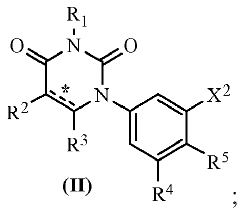
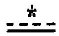 , R**¹, R²**, **R³**, and **R⁵** are as defined in claim **1**;
**R⁴** is selected from the group consisting of C₁-C₄-alkyl, C₂-C₄-alkenyl, C₂-C₄-alkynyl, amino, C₁-C₄-alkylsulfonyl, C₃-C₆-carbocyclyl, and 5-6-membered heterocyclyl, wherein:
(**a**) the amino optionally is substituted with one or two substituents independently selected from the group consisting of alkyl, alkenyl, alkynyl, and alkylsulfonyl,
(**b**) the C₁-C₄-alkyl, C₂-C₄-alkenyl, and C₂-C₄-alkynyl optionally are substituted with one or more substituents independently selected from the group consisting of halo, oxo, hydroxy, alkyloxy, and trimethylsilyl, and
(**c**) the C₃-C₆-carbocyclyl and 5-6-membered heterocyclyl optionally are substituted with up to three substituents independently selected from the group consisting of alkyl, alkenyl, alkynyl, halo, and amino, wherein:
the amino optionally is substituted with one or two substituents independently selected from the group consisting of alkyl, alkenyl, alkynyl, and alkylsulfonyl; and
**X²** is halo.

14. A process for preparing the compound or salt of claim **13**, wherein the process comprises reacting a compound of formula **III** (wherein **R¹**, **R²**, and **R³** are as defined in claim **14**) with a compound of formula **IV** (wherein **R⁴**, **R⁵**, and **X²** are as defined in claim **14;** and **X¹** is halo) in the presence of (**i**) copper (I) salt catalyst and (**ii**) nitrogenous heteroaryl ligand

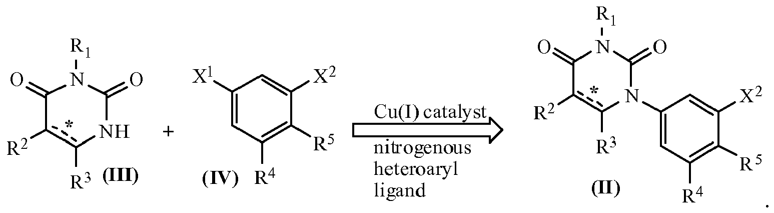

15. The compound or salt of claim **13**, wherein the compound is selected from the group consisting of
